# EUROPEAN PATENT APPLICATION

(11) **EP 4 400 497 A1**
(43) Date of publication of application: **17.07.2024**
(21) Application number: 22866603.8
(22) Date of filing: 06.09.2022
(51) Int. Cl.: C07D 401/02, C07D 401/14, C07D 401/12, C07D 403/02, C07D 403/14, C07D 403/12, A61K 31/472, A61P 31/14

(54) **3CLPRO PROTEASE INHIBITOR**

(30) Priority: 07.09.2021 CN 202111044417; 21.10.2021 CN 202111226591; 03.12.2021 CN 202111470155; 31.12.2021 CN 202111674718; 26.08.2022 CN 202211031927
(71) Applicant: Shanghai Qilu Pharmaceutical Research and Development Centre Ltd., Shanghai 201203 (CN); Qilu Animal Health Products Co., Ltd., Jinan, Shandong 250105 (CN)
(72) Inventor: WANG, Junfei, Shanghai 201203 (CN); CHENG, Cang, Shanghai 201203 (CN); CHI, Bo, Shanghai 201203 (CN); SUN, Daqing, Shanghai 201203 (CN)
(74) Representative: Graf von Stosch Patentanwaltsgesellschaft mbH
(86) International application number: PCT/CN2022/117336
(87) International publication number: WO 2023/036140

(57) **Abstract**

Provided are a 3Clpro protease inhibitor, a pharmaceutical composition containing the compound of formula (I), and a method for treating coronavirus by using the compound.

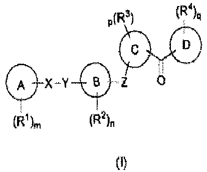

## Description

This application claims the priorities of Chinese patent application No. 202111044417.4, filed on September 7, 2021, Chinese patent application No. 202111226591.0, filed on October 21, 2021, Chinese patent application No. 202111470155.8, filed on December 3, 2021, Chinese patent application No. 202111674718.5, filed on December 31, 2021, and Chinese patent application No. 202211031927.2, filed on August 26, 2022, which are incorporated herein by reference in their entirety.

### TECHNICAL FIELD

The present invention belongs to the field of pharmaceutical chemistry and specifically relates to a compound as a coronavirus 3CLpro protease inhibitor, a pharmaceutical composition containing the compound, and a method for treating coronavirus infections by using the compound of the present invention.

### BACKGROUND

Coronaviruses are a type of positive-sense single-stranded RNA virus. The family of coronaviruses mainly includes novel coronavirus (SARS-CoV-2), SARS coronavirus (SARS-CoV), human coronavirus 229E, human coronavirus NL63, human coronavirus OC43, human coronavirus HKU1, Severe Acute Respiratory Syndrome associated Coronavirus (SARS-CoV), Middle East Respiratory Syndrome Virus (MERS-CoV), wherein SARS-CoV and MERS-CoV are highly pathogenic HCoVs that can cause severe acute respiratory syndrome and have a high mortality rate. Infection with novel coronavirus (2019-nCoV) can cause mild, moderate or severe diseases (including severe pneumonia, sepsis and ARDS, etc.).

3CLPro (3C-like protease) is the main protease produced by novel coronavirus (COVID-19, SARS-CoV-2). Most functional proteins (non-structural proteins) of coronavirus are encoded by ORFlab gene, which is first translated into a polyprotein (7096aa), and then the polyprotein is cleaved into multiple active proteins, such as virus replication protein RdRp, by 3CLPro. In addition, the protease may cleave the intracellular protein NEMO, thereby inhibiting the activation of the interferon signaling pathway. Therefore, inhibition of 3CLPro can effectively inhibit virus infection and replication.

Compared with SARS, diseases caused by novel coronavirus have more complex symptoms and are more transmissible. Up to now, more than 500 million cases have been confirmed worldwide. Since 3C-like protease plays a vital role in the replication of coronavirus, it is of great value and significance to develop inhibitors against 3CLPro as a coronavirus therapeutic medicament.

### SUMMARY

The present application provides a compound represented by formula (I), or an isomer or a pharmaceutically acceptable salt thereof, 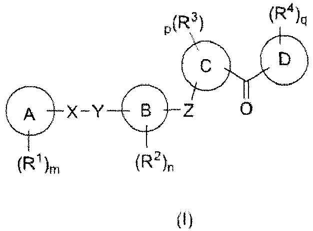
wherein, ring A is selected from the group consisting of C₆₋₁₀ aryl, 5- to 10-membered heteroaryl, C₃₋₁₂ cycloalkyl, and 3- to 12-membered heterocyclyl;
X is selected from the group consisting of -(CH₂)ₛ-C(=O)- and -NH-C(=O)-;
Y is NH or a chemical bond;
ring B is selected from the group consisting of C₃₋₁₂ cycloalkyl, 3- to 12-membered heterocyclyl, and 5- to 10-membered heteroaryl;
Z is selected from the group consisting of -O-, -S-, -CH₂-, -C(=O)-NH- and -(CH₂)ₜNH-;
ring C is selected from the group consisting of C₆₋₁₀ aryl and 5- to 10-membered heteroaryl;
ring D is 3- to 12-membered heterocyclyl or 5- to 6-membered heteroaryl; or ring D is absent; R¹ is selected from the group consisting of halogen, OH, O, cyano, -NR^{1a}R^{1b}, C₁₋₄ alkyl, C₁₋₄ alkoxy, C₃₋₈ cycloalkyl, C₆₋₁₀ aryl, -NR^{1a}COR^{1c}, -SR^{1d}, and -CONR^{1a}R^{1b}, wherein C₁₋₄ alkyl and C₁₋₄ alkoxy can be further substituted by one or more halogens;
R^{1a}, R^{1b}, R^{1c}, and R^{1d} are each independently selected from the group consisting of H, C₁₋₄ alkyl and halo-C₁₋₄ alkyl, wherein C₁₋₄ alkyl can be substituted by one or more deuteriums;
or, two R¹ linked to the same C atom on ring A can together form =CH₂, =S, or C₃₋₅ cycloalkyl;
R² is selected from the group consisting of deuterium, cyano, C₂₋₄ alkynyl, halogen, C₁₋₄ alkyl, hydroxyl, C₁₋₄ alkoxy, deutero-C₁₋₄ alkoxy, -C(=O)-NR^{2a}R^{2b}, -C₂₋₄ alkynyl-R^{2c}, 5- to 6-membered heteroaryl and -O-(CH₂)_{y}-R^{2d};
R^{2c} is selected from the group consisting of 5- to 6-membered heteroaryl, wherein 5- to 6-membered heteroaryl can be further substituted by C₁₋₄ alkyl;
R^{2a} and R^{2b} are each independently selected from the group consisting of H, C₁₋₄ alkyl, phenyl, 5- to 9-membered heteroaryl, 5- to 6-membered heterocyclyl, and C₃₋₅ cycloalkyl, wherein C₁₋₄ alkyl, phenyl, 5- to 9-membered heteroaryl, 5-to 6-membered heterocyclyl, and C₃₋₅ cycloalkyl can be further substituted by one or more substituents selected from the group consisting of methyl, ethyl, cyclopropyl, halogen, CF₃, -CH₂CF₃, phenyl, methoxy, pyridinyl, pyrimidinyl, tetrahydropyranyl and -CH₂CH₂OCH₃, wherein pyridinyl, pyrimidinyl, and tetrahydropyranyl can be further substituted by one or more methyl, CF₃, halogen, -NHCH₃, or -CHF₂;
or, R^{2a}, R^{2b} and their linked N atom together form 4- to 9-membered heterocyclyl or 5- to 9-membered heteroaryl, wherein 4- to 9-membered heterocyclyl and 5- to 9-membered heteroaryl can be further substituted by one or more substituents selected from the group consisting of C₁₋₄ alkyl, halogen, hydroxyl, and -CH₂CH₂OCH₃;
R^{2d} is selected from the group consisting of C₁₋₄ alkyl, C₁₋₄ alkoxy, phenyl, 5- to 6-membered heteroaryl and 5- to 6-membered heterocyclyl, wherein phenyl, 5- to 6-membered heteroaryl and 5- to 6-membered heterocyclyl can be further substituted by one or more substituents selected from the group consisting of halogen, C₁₋₄ alkyl, C₁₋₄ alkoxy, and halo-C₁₋₄ alkyl; y is 0, 1, or 2;
R³ is selected from the group consisting of nitro, halogen, hydroxyl, cyano, C₁₋₄ alkyl, halo-C₁₋₄ alkyl, -NR^{3a}R^{3b} and -N(R^{3a}R^{3b}R^{3c})⁺;
R^{3a}, R^{3b} and R^{3c} are each independently selected from the group consisting of H and C₁₋₄ alkyl;
R⁴ is selected from the group consisting of halogen, hydroxyl, C₁₋₄ alkyl, halo-C₁₋₄ alkyl, 3- to 8-membered heterocyclyl, -C(=O)R^{4a}, -NHC(=O)R^{4a}, -(CH₂)ᵣOR^{4b}, -NR^{4c}R^{4d}, C₁₋₄ alkoxy, -(CH₂)ₓ-C₃₋₆ cycloalkyl, -NR^{4a}-(CH₂)ₓ-C₆₋₁₀ aryl, wherein aryl can be further substituted by halogen;
R^{4a}, R^{4b}, R^{4c}, and R^{4d} are independently selected from the group consisting of H, C₁₋₄ alkyl, halo-C₁₋₄ alkyl, 5- to 6-membered heterocyclyl and C₃₋₆ cycloalkyl;
or R^{4c} and R^{4d} together with their linked N atom form 6-membered heterocyclyl;
x is 0, 1, or 2; r is 0, 1, 2, or 3;
m, n, p, and q are each independently 0, 1, 2, 3, 4, or 5; and
s and t are each independently 0 or 1.

In some embodiments of the present application, ring A is selected from the group consisting of C₆₋₁₀ aryl, 5- to 10-membered heteroaryl, C₃₋₁₂ cycloalkyl, and 3- to 12-membered heterocyclyl;

X is -(CH₂)ₛ-C(=O)- or -NH-C(=O)-;

Y is NH or a chemical bond;
ring B is selected from the group consisting of C₃₋₁₂ cycloalkyl, 3- to 12-membered heterocyclyl, and 5- to 10-membered heteroaryl;
Z is selected from the group consisting of -O-, -S-, -CH₂-, -C(=O)-NH- and -(CH₂)ₜNH-;
ring C is selected from the group consisting of C₆₋₁₀ aryl and 5- to 10-membered heteroaryl;
ring D is 3- to 12-membered heterocyclyl; or ring D is absent;
R¹ is selected from the group consisting of halogen, OH, O, cyano, -NR^{1a}R^{1b}, C₁₋₄ alkyl, C₁₋₄ alkoxy, C₃₋₈ cycloalkyl, C₆₋₁₀ aryl, -NR^{1a}COR^{1c}, -SR^{1d}, and -CONR^{1a}R^{1b}, wherein C₁₋₄ alkyl and C₁₋₄ alkoxy can be further substituted by one or more halogens;
R^{1a}, R^{1b}, R^{1c}, and R^{1d} are each independently selected from the group consisting of H, C₁₋₄ alkyl and halo-C₁₋₄ alkyl;
or, two R¹ linked to the same C atom on ring A can together form =CH₂, =S, or C₃₋₅ cycloalkyl;
R² is selected from the group consisting of halogen, C₁₋₄ alkyl, hydroxyl, and C₁₋₄ alkoxy;
R³ is selected from the group consisting of nitro, halogen, hydroxyl, cyano, C₁₋₄ alkyl, halo-C₁₋₄ alkyl, -NR^{3a}R^{3b} and -N(R^{3a}R^{3b}R^{3c})⁺;
R^{3a}, R^{3b} and R^{3c} are each independently selected from the group consisting of H and C₁₋₄ alkyl;
R⁴ is selected from the group consisting of halogen, hydroxyl, C₁₋₄ alkyl, halo-C₁₋₄ alkyl, 3- to 8-membered heterocyclyl, -C(=O)R^{4a}, -NHC(=O)R^{4a}, -(CH₂)ᵣOR^{4b}, -NR^{4c}R^{4d}, C₁₋₄ alkoxy, -(CH₂)ₓ-C₃₋₆ cycloalkyl, -NR^{4a}-(CH₂)ₓ-C₆₋₁₀ aryl, wherein aryl can be further substituted by halogen;
R^{4a}, R^{4b}, R^{4c}, and R^{4d} are each independently selected from the group consisting of H, C₁₋₄ alkyl, halo-C₁₋₄ alkyl, 5- to 6-membered heterocyclyl and C₃₋₆ cycloalkyl;
or R^{4c}, R^{4d} and their linked N atom together form 6-membered heterocyclyl;
x is 0, 1 or 2;
r is 0, 1, 2, or 3;
m, n, p, and q are each independently 0, 1, 2, 3, 4, or 5; and
s and t are each independently 0 or 1.

In some embodiments of the present application, ring A is selected from the group consisting of C₆₋₁₀ aryl, 5- to 10-membered heteroaryl, C₃₋₁₂ cycloalkyl, and 3- to 12-membered heterocyclyl;

X is -(CH₂)ₛ-C(=O)- or -NH-C(=O)-;

Y is NH or a chemical bond;
ring B is selected from the group consisting of C₃₋₁₂ cycloalkyl, 3- to 12-membered heterocyclyl, and 5- to 10-membered heteroaryl;
Z is selected from the group consisting of -O-, -S-, -CH₂-, -C(=O)-NH- and -(CH₂)ₜNH-;
ring C is selected from the group consisting of C₆₋₁₀ aryl and 5- to 10-membered heteroaryl;
ring D is 3- to 8-membered heterocyclyl; or ring D is absent;
R¹ is selected from the group consisting of halogen, OH, O, cyano, -NR^{1a}R^{1b}, C₁₋₄ alkyl, C₁₋₄ alkoxy, C₃₋₈ cycloalkyl, C₆₋₁₀ aryl, wherein C₁₋₄ alkyl and C₁₋₄ alkoxy can be further substituted by one or more halogens;
R^{1a} and R^{1b} are each independently selected from the group consisting of H and C₁₋₄ alkyl;
R² is selected from the group consisting of halogen and C₁₋₄ alkyl;
R³ is selected from the group consisting of nitro, halogen, hydroxyl, cyano, C₁₋₄ alkyl, halo-C₁₋₄ alkyl, -NR^{3a}R^{3b} and -N(R^{3a}R^{3b}R^{3c})⁺;
R^{3a}, R^{3b} and R^{3c} are each independently selected from the group consisting of H and C₁₋₄ alkyl;
R⁴ is selected from the group consisting of halogen, hydroxyl, C₁₋₄ alkyl, halo-C₁₋₄ alkyl, 3- to 8-membered heterocyclyl, -C(=O)R^{4a}, -NHC(=O)R^{4a}, -(CH₂)ᵣOR^{4b}, and -NR^{4c}R^{4d};
R^{4a}, R^{4b}, R^{4c}, and R^{4d} are each independently selected from the group consisting of H, C₁₋₄ alkyl and halo-C₁₋₄ alkyl,
r is 0, 1, 2, or 3;
m, n, p, and q are each independently 0, 1, 2, or 3; and
s and t are each independently 0 or 1.

When ring D is absent, (R⁴)_{q} is linked to the -C(=O)- bond linked to ring C.

Preferably, when ring A is 9- or 10-membered heteroaryl and ring D is absent, R⁴ is not methylamino.

In some embodiments of the present application,
ring A is C₆₋₁₀ aryl, 5- to 10-membered heteroaryl, or 7- to 10-membered heterocyclyl;
X is -(CH₂)ₛ-C(=O)- or -NH-C(=O)-;
Y is NH or a chemical bond;
ring B is selected from the group consisting of C₃₋₁₂ cycloalkyl, 3- to 12-membered heterocyclyl, and 5- to 10-membered heteroaryl;
Z is selected from the group consisting of -O-, -S-, -CH₂-, -C(=O)-NH- and -(CH₂)ₜNH-;
ring C is selected from the group consisting of C₆₋₁₀ aryl and 5- to 10-membered heteroaryl;
ring D is 3- to 8-membered heterocyclyl;
R¹ is selected from the group consisting of halogen, OH, O, cyano, -NR^{1a}R^{1b}, C₁₋₄ alkyl, C₁₋₄ alkoxy, C₃₋₈ cycloalkyl, C₆₋₁₀ aryl, wherein C₁₋₄ alkyl and C₁₋₄ alkoxy can be further substituted by one or more halogens;
R^{1a} and R^{1b} are each independently selected from the group consisting of H and C₁₋₄ alkyl;
R² is selected from the group consisting of halogen and C₁₋₄ alkyl;
R³ is selected from the group consisting of nitro, halogen, hydroxyl, cyano, C₁₋₄ alkyl, halo-C₁₋₄ alkyl, -NR^{3a}R^{3b} and -N(R^{3a}R^{3b}R^{3c})⁺;
R^{3a}, R^{3b} and R^{3c} are each independently selected from the group consisting of H and C₁₋₄ alkyl;
R⁴ is selected from the group consisting of halogen, hydroxyl, and C₁₋₄ alkyl;
m, n, p, and q are each independently 0, 1, or 2; and
s and t are each independently 0, 1 or 2.

In some embodiments of the present application, ring A is selected from the group consisting of phenyl, cyclohexyl, 5-to 10-membered heteroaryl and 6- to 10-membered heterocyclyl, wherein heteroaryl and heterocyclyl contain 1 to 3 heteroatoms selected from the group consisting of N, O, and S.

In some embodiments of the present application, ring A is selected from the group consisting of phenyl, cyclohexyl, 5-to 6-membered monocyclic heteroaryl, 8- to 10-membered fused heteroaryl, 6-membered monocyclic heterocyclyl, 8-membered spiroheterocyclyl, and 7- to 10-membered fused heterocyclyl, wherein heterocyclyl and heteroaryl contain 1-3 heteroatoms selected from the group consisting of N and O.

In some embodiments of the present application, ring A is selected from the group consisting of pyridinyl, pyrimidinyl, pyrazinyl, isoquinolinyl, cinnolinyl, phthalazinyl, phenyl, imidazolyl, thienyl, isoxazolyl, benzotriazolyl,

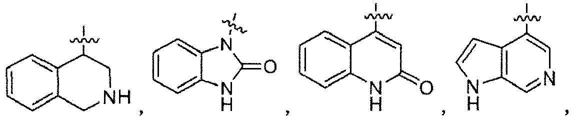

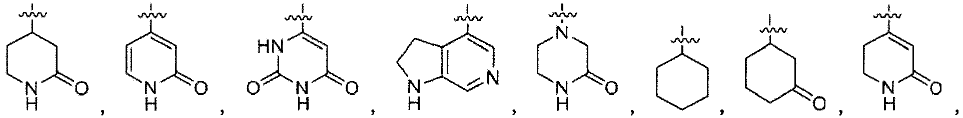

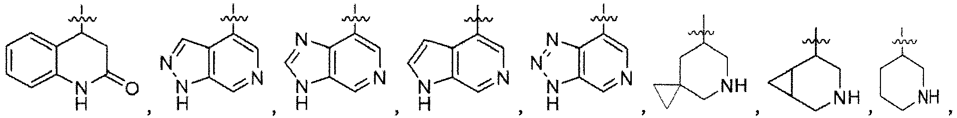

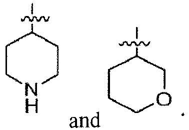

In some embodiments of the present application, ring A is selected from the group consisting of pyridinyl, pyrimidinyl, pyrazinyl, isoquinolinyl, cinnolinyl, phthalazinyl, phenyl, imidazolyl, thienyl, isoxazolyl, benzotriazolyl,

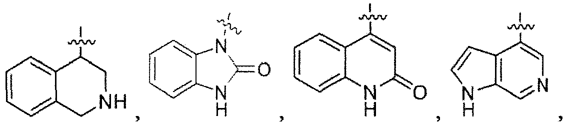

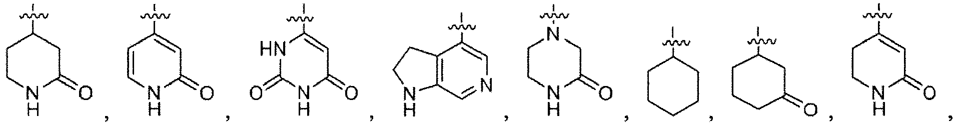

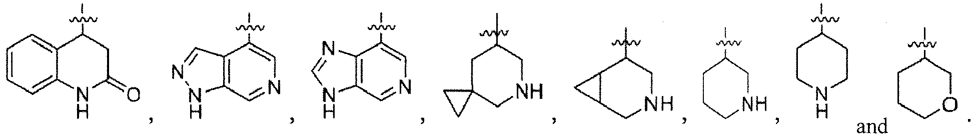

In some embodiments of the present application, ring A is

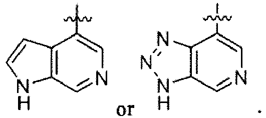

In some embodiments of the present application, ring A is selected from the group consisting of pyridinyl, pyrimidinyl, pyrazinyl, isoquinolinyl, cinnolinyl, phthalazinyl, phenyl, imidazolyl, thienyl, isoxazolyl, benzotriazolyl,

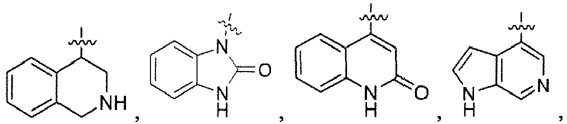

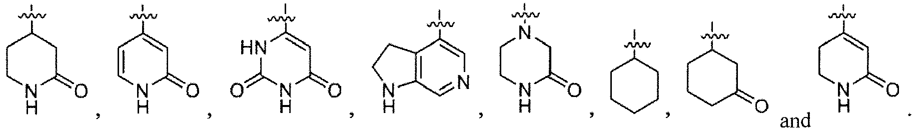

In some embodiments of the present application, ring A is selected from the group consisting of pyridinyl, pyrimidinyl, pyrazinyl, isoquinolinyl, cinnolinyl, phthalazinyl, phenyl, imidazolyl, thienyl, isoxazolyl, benzotriazolyl,

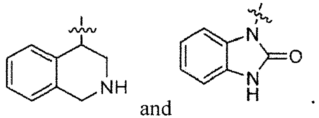

In some embodiments of the present application, ring A is selected from the group consisting of

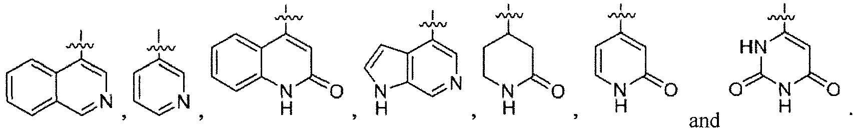

In some embodiments of the present application, R¹ is selected from the group consisting of F, Cl, Br, I, O, hydroxyl, methyl, methoxy, trifluoromethyl, -NH₂, -NHCH₃, -N(CH₃)₂, 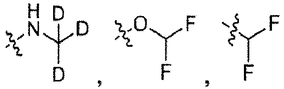 , cyano, cyclopropyl, phenyl, -NHCH₂CH₃, -SCH₃, -CONH₂, -NHCOCF₃ and -NHCOCH₃;
or, two R¹ linked to the same C atom on ring A can together form =CH₂, =S, or cyclopropyl; and
m is 0, 1, 2, or 3.

In some embodiments of the present application, R¹ is selected from the group consisting of F, Cl, Br, I, O, hydroxyl, methyl, methoxy, trifluoromethyl, -NH₂, -NHCH₃, -N(CH₃)₂, 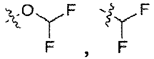 , cyano, cyclopropyl, phenyl, -NHCH₂CH₃, -SCH₃, -CONH₂, -NHCOCF₃ and -NHCOCH₃;
or, two R¹ linked to the same C atom on ring A can together form =CH₂, =S, or cyclopropyl; and
m is 0, 1, 2, or 3.

In some embodiments of the present application, R¹ is selected from the group consisting of F, Cl, Br, I, O, hydroxyl, methyl, methoxy, trifluoromethyl, -NH₂, -NHCH₃, -N(CH₃)₂, 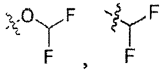 , cyano, cyclopropyl, and phenyl.

In some embodiments of the present application, R¹ is selected from the group consisting of F, Cl, Br, I, O, hydroxyl, methyl, methoxy, trifluoromethyl, -NH₂, -NHCH₃, 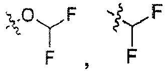 , cyano, cyclopropyl, and phenyl.

In some embodiments of the present application, R¹ is selected from the group consisting of F, Cl, Br, hydroxyl, methyl, -NH₂, -NHCH₃, cyano, and cyclopropyl.

In some embodiments of the present application, the structural unit 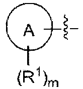 is selected from the group consisting of

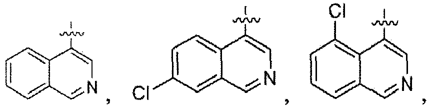

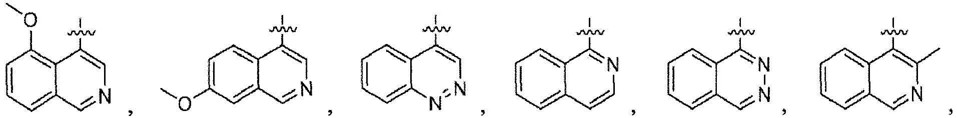

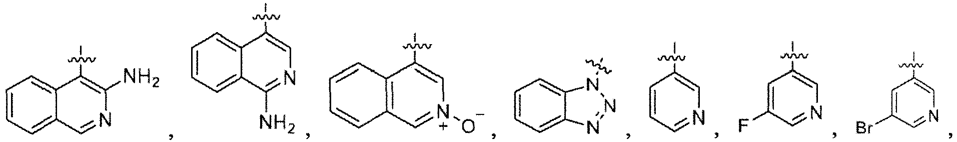

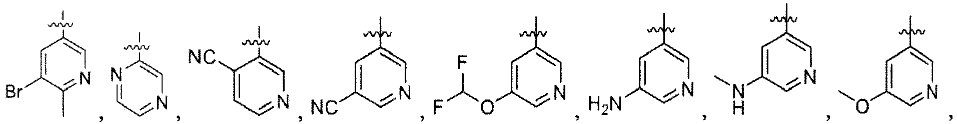

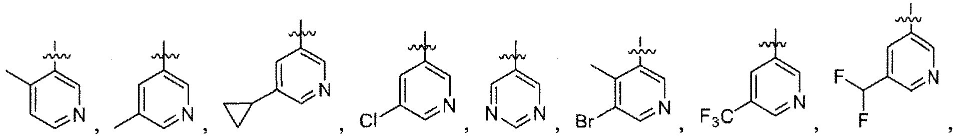

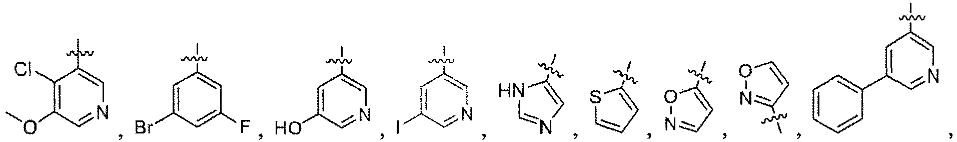

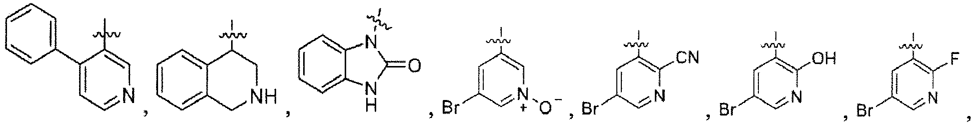

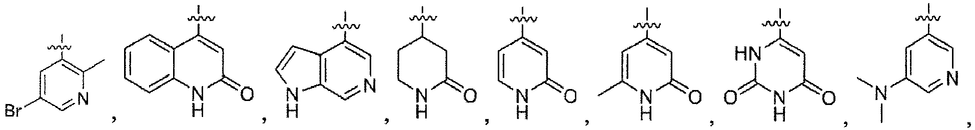

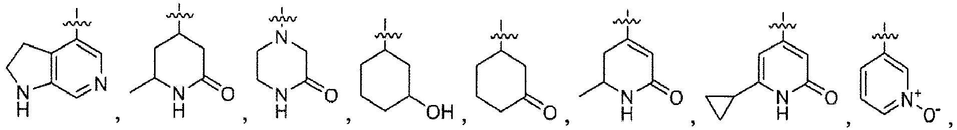

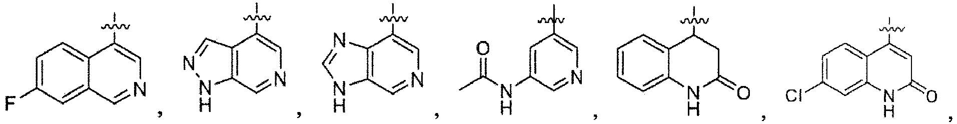

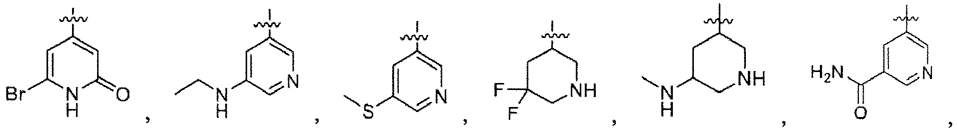

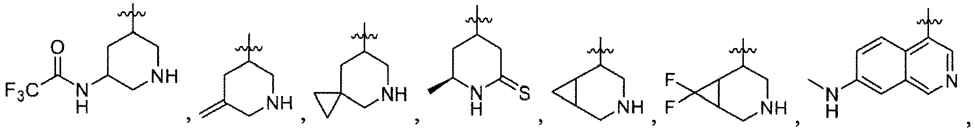

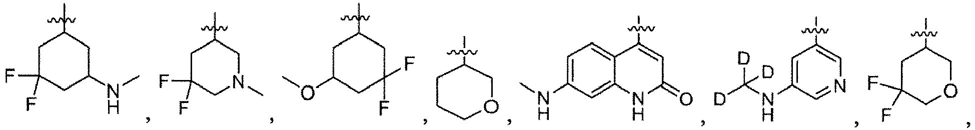

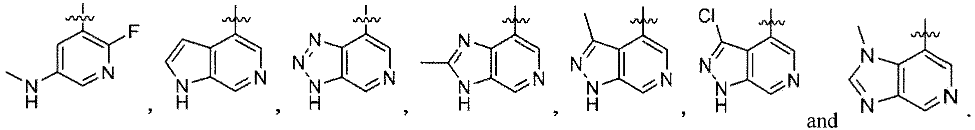

In some embodiments of the present application, the structural unit 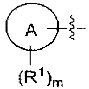 is selected from the group consisting of

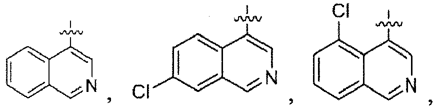

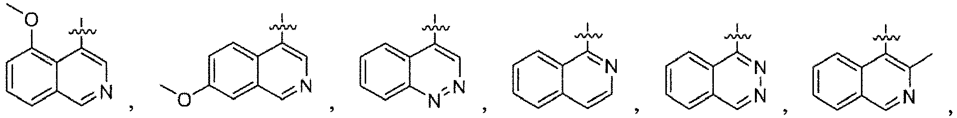

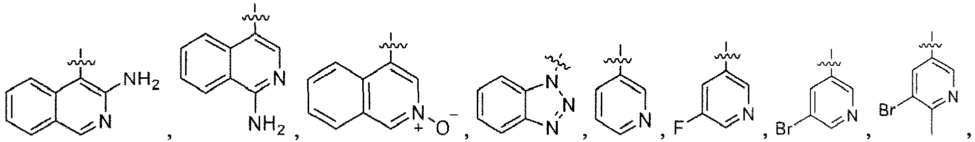

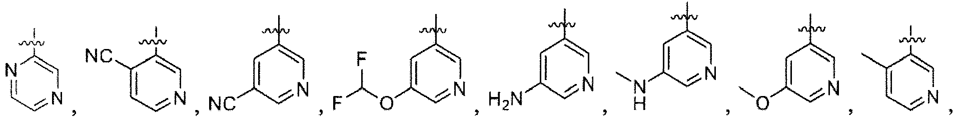

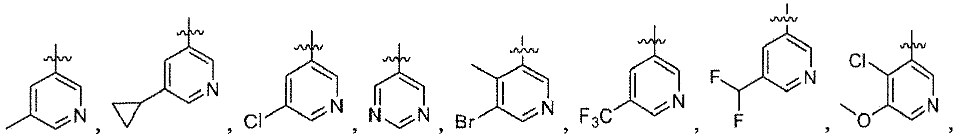

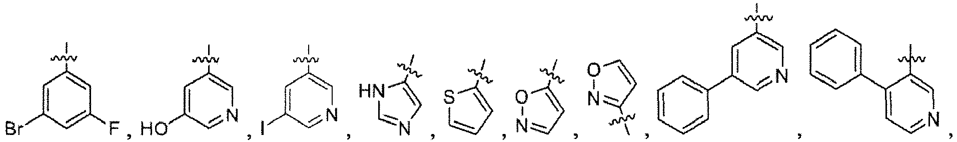

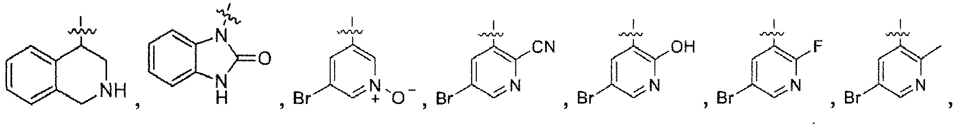

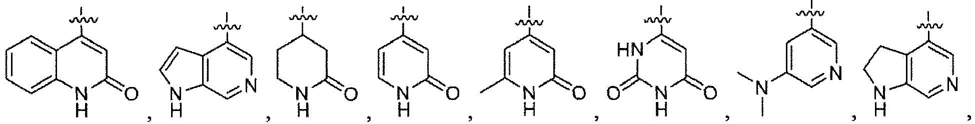

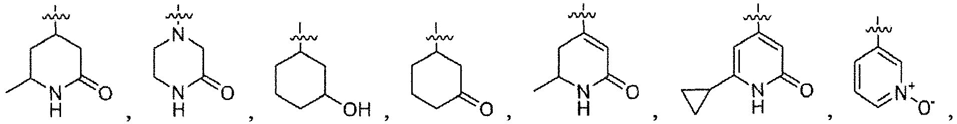

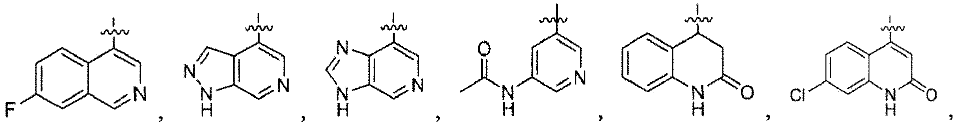

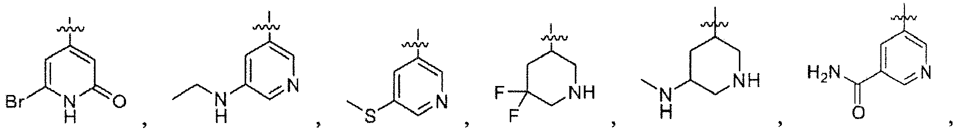

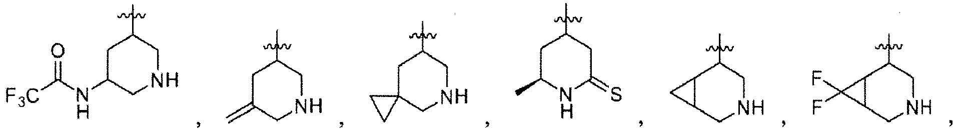

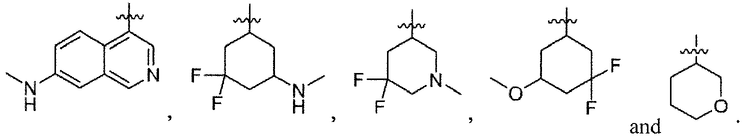

In some embodiments of the present application, the structural unit 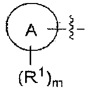 is selected from the group consisting of

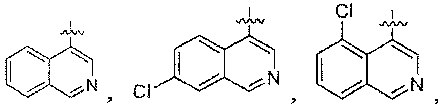

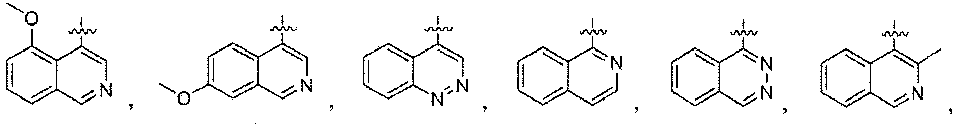

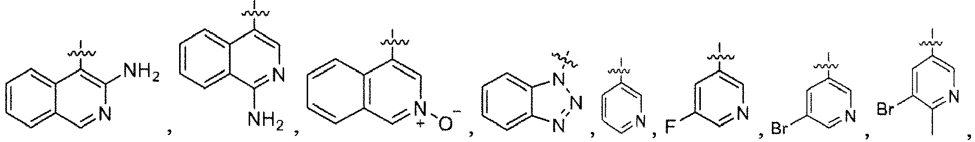

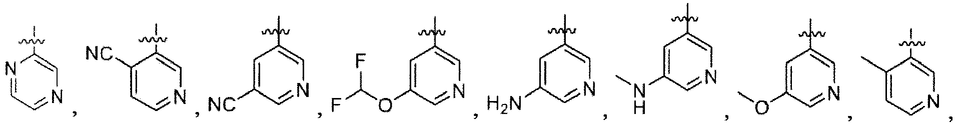

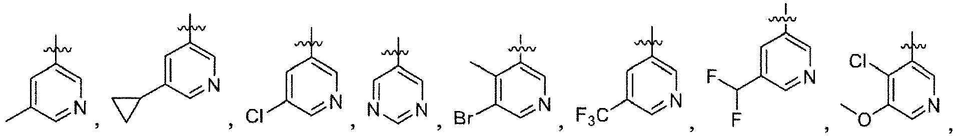

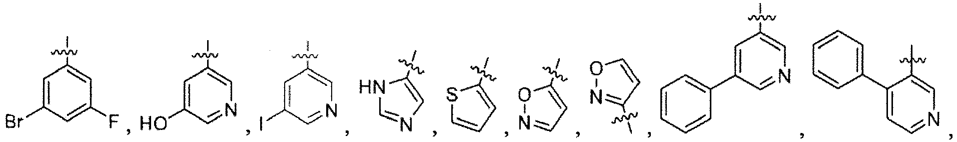

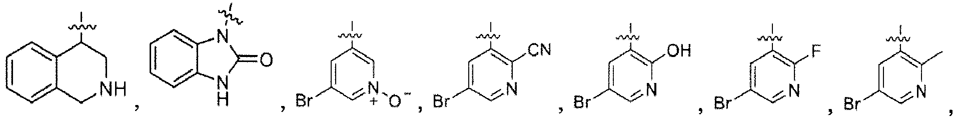

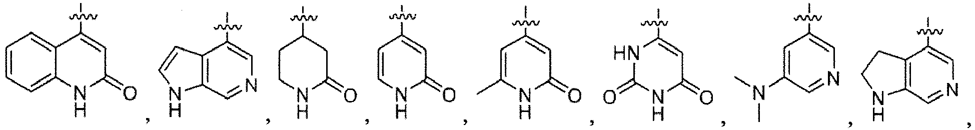

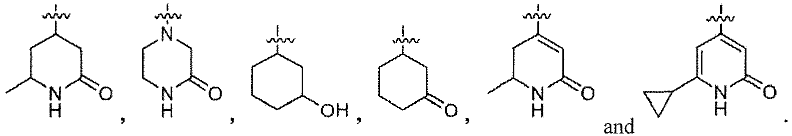

In some embodiments of the present application, the structural unit 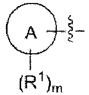 is selected from the group consisting of

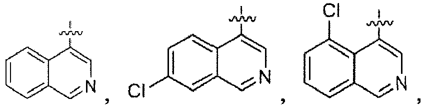

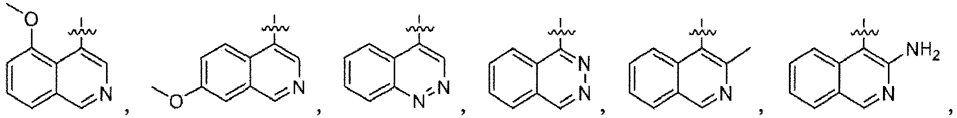

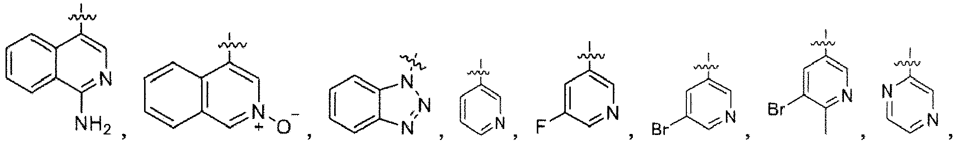

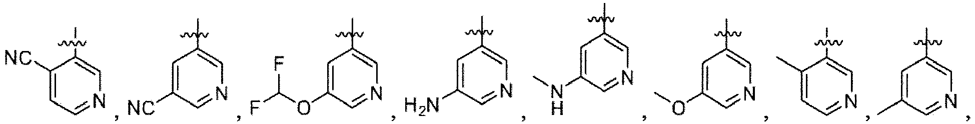

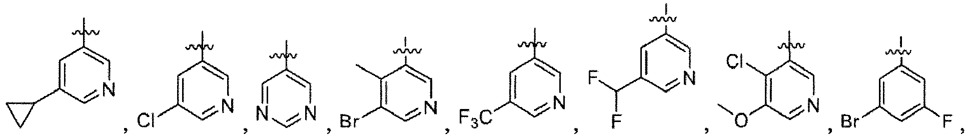

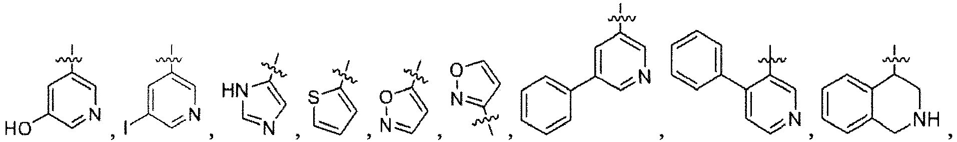

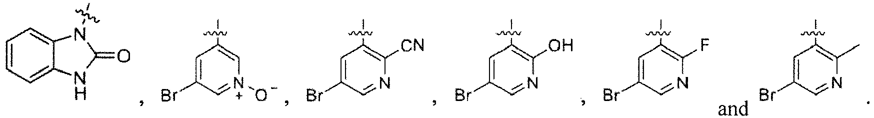

In some embodiments of the present application, the structural unit 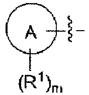 is selected from the group consisting of

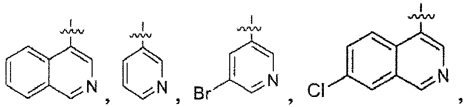

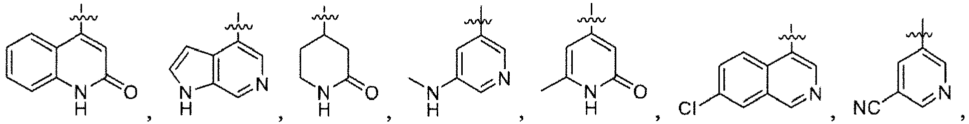

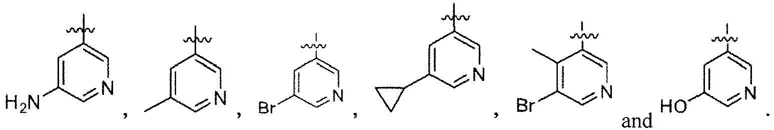

In some embodiments of the present application, X is selected from the group consisting of -CH₂-C(=O)-, -C(=O)-, and -NH-C(=O)-.

In some embodiments of the present application, X is -C(=O)-.

In some embodiments of the present application, ring B is selected from the group consisting of C₅₋₆ cycloalkyl, 4- to 10-membered heterocyclyl, and 5- to 6-membered heteroaryl.

In some embodiments of the present application, ring B is selected from the group consisting of C₅₋₆ cycloalkyl, 4- to 6-membered monocyclic heterocyclyl, 6-membered spiroheterocyclyl, 6- to 9-membered fused heterocyclyl, and 6-membered heteroaryl, wherein heterocyclyl and heteroaryl contain 1 to 2 heteroatoms selected from the group consisting of N and O.

In some embodiments of the present application, ring B is selected from the group consisting of

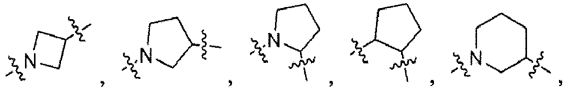

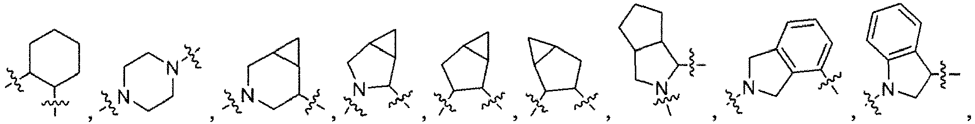

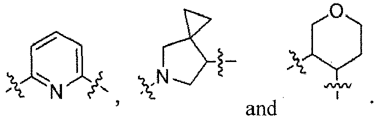

In some embodiments of the present application, ring B is selected from the group consisting of

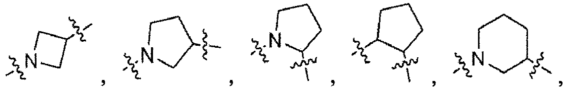

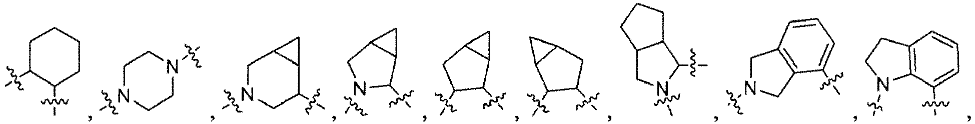

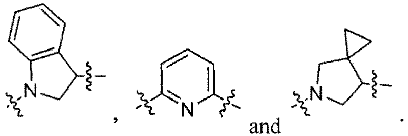

In some embodiments of the present application, ring B is selected from the group consisting of

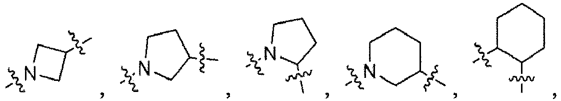

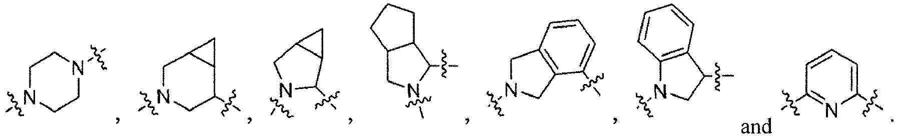

In some embodiments of the present application, ring B is selected from the group consisting of

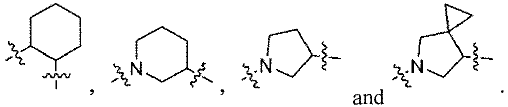

In some embodiments of the present application, R² is selected from the group consisting of F, methyl, hydroxyl, and methoxy; and n is 0, 1, or 2.

In some embodiments of the present application, R² is selected from the group consisting of deuterium, cyano, ethynyl, F, methyl, hydroxyl, methoxy, -CONH₂, -CONHCH₃, 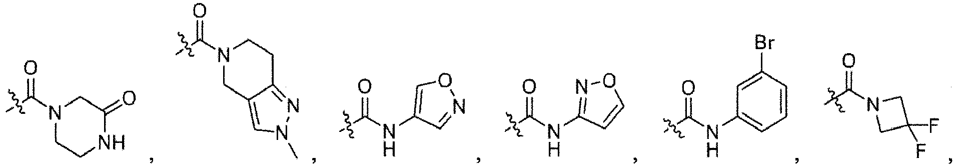 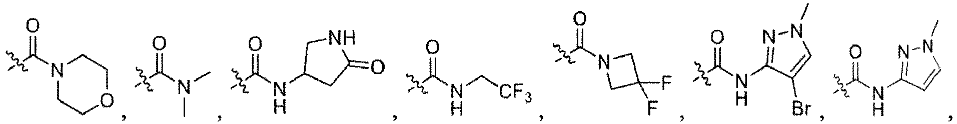 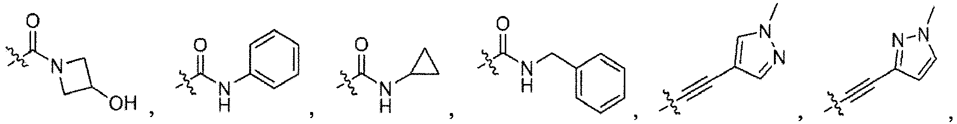 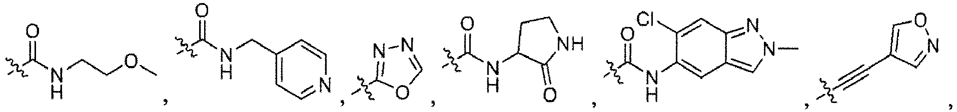 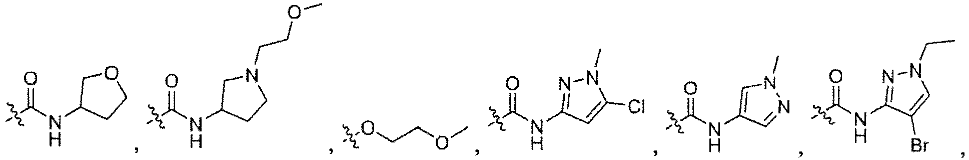 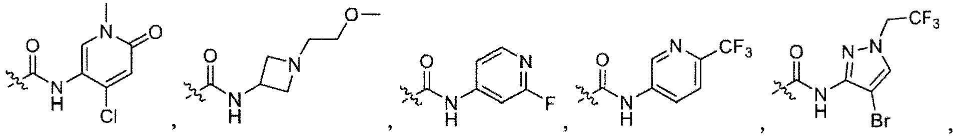 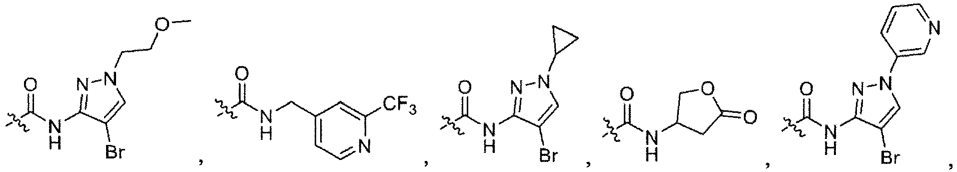 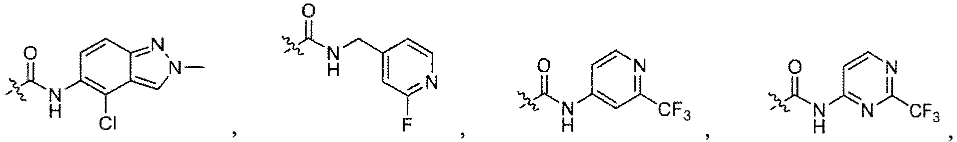 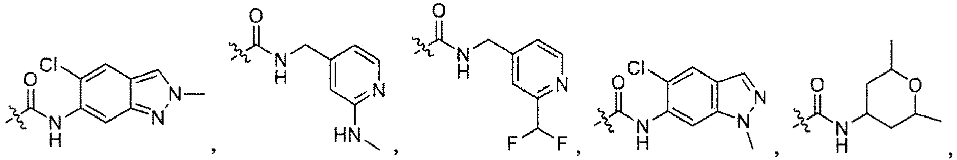 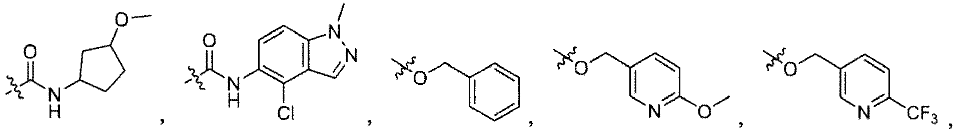 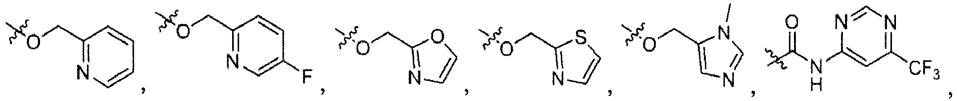 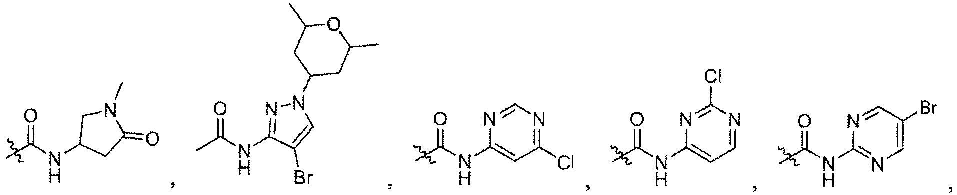 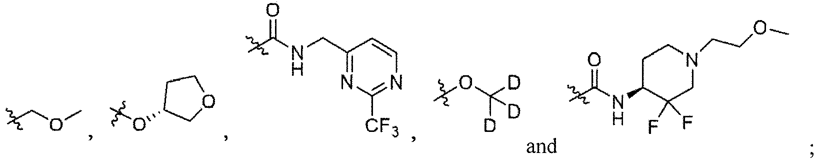 and
n is 0, 1, 2, 3, 4, or 5.

In some embodiments of the present application, R² is selected from the group consisting of F and methyl; and n is 0, 1, or 2.

In some embodiments of the present application, the structural unit 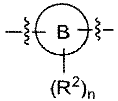 is selected from the group consisting of

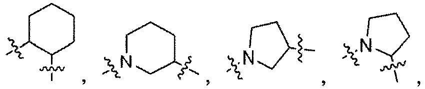

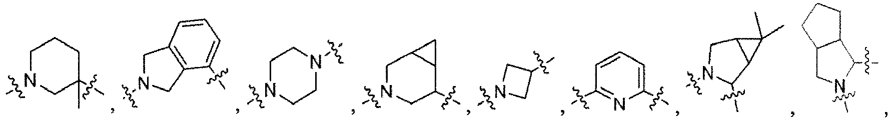

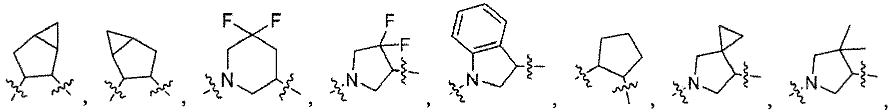

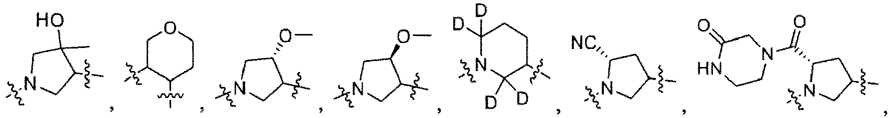

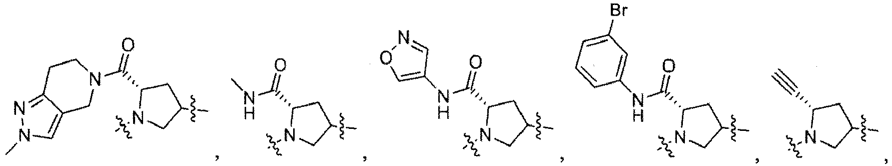

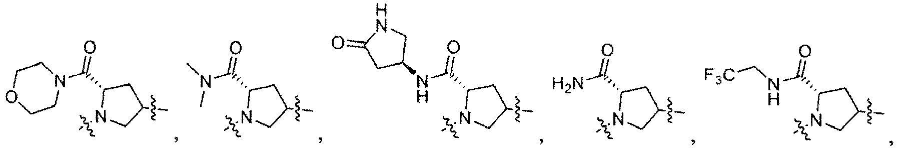

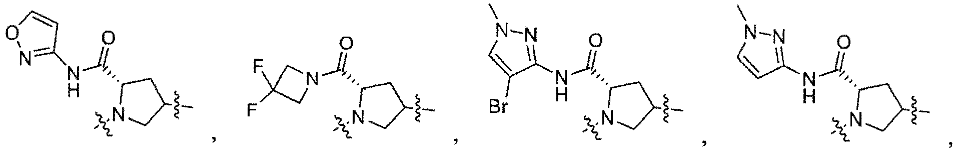

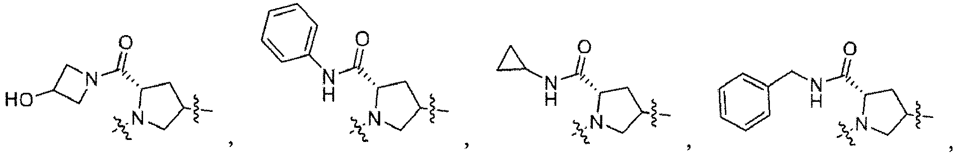

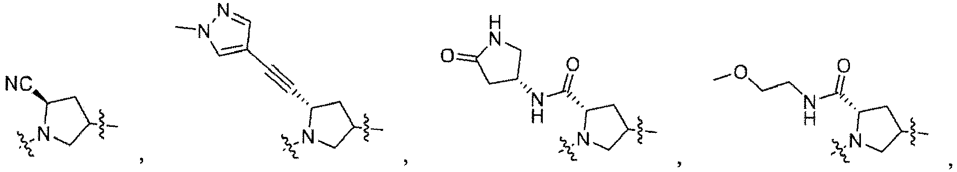

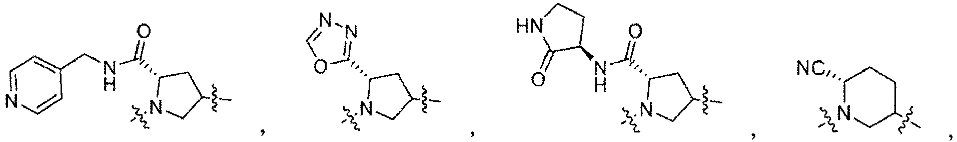

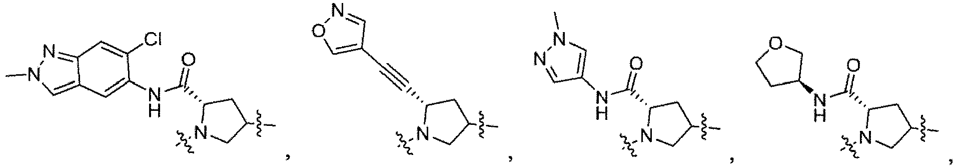

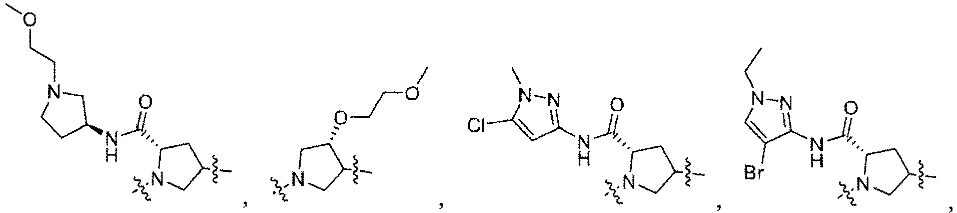

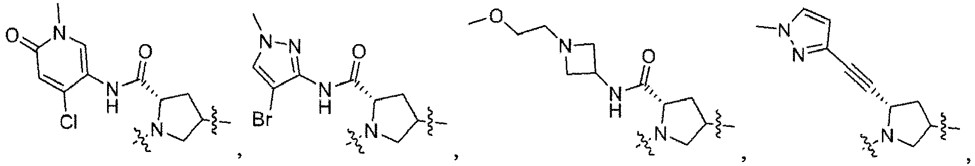

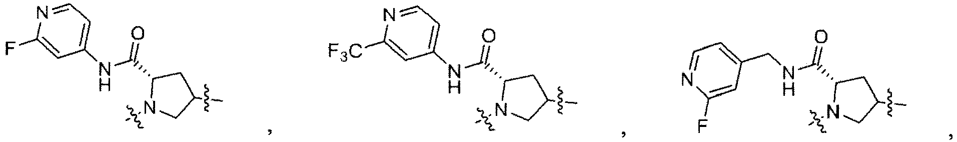

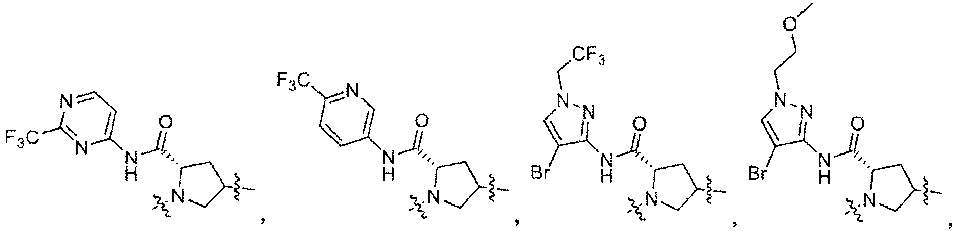

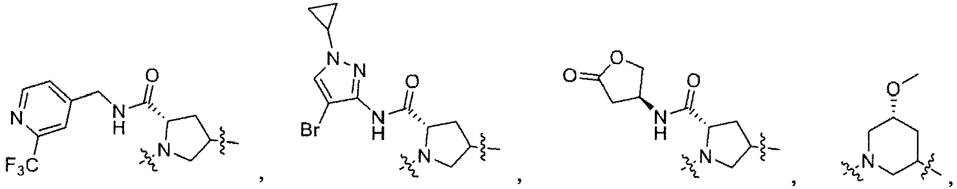

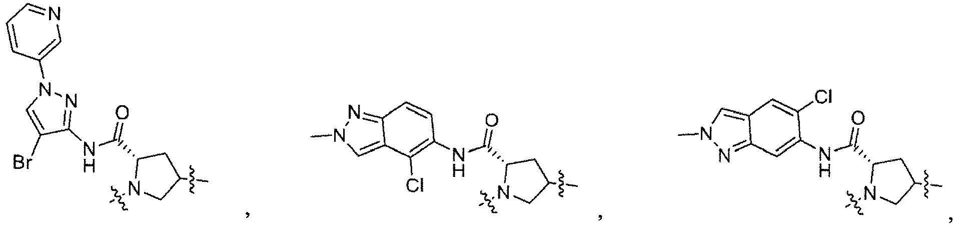

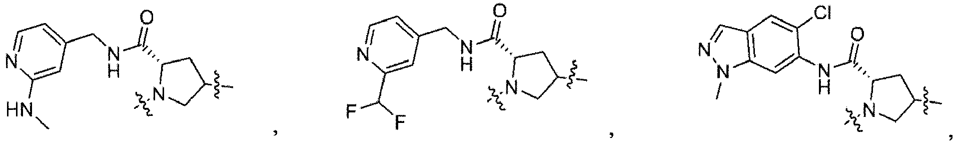

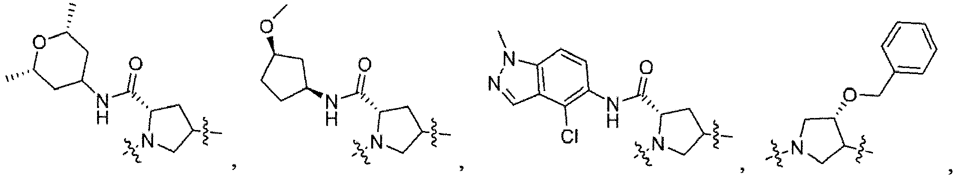

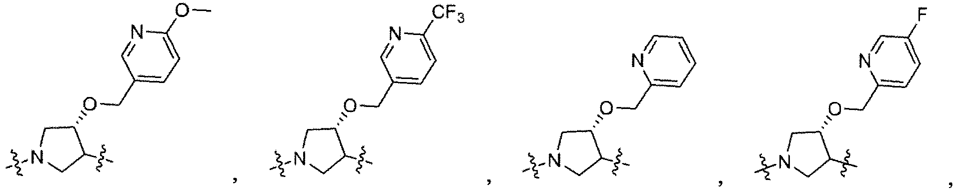

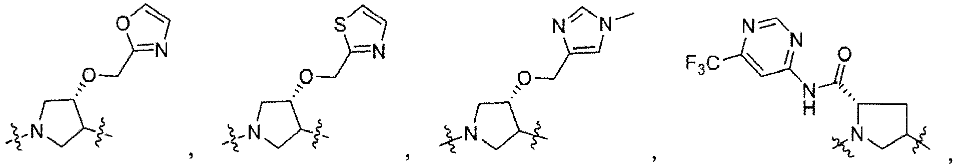

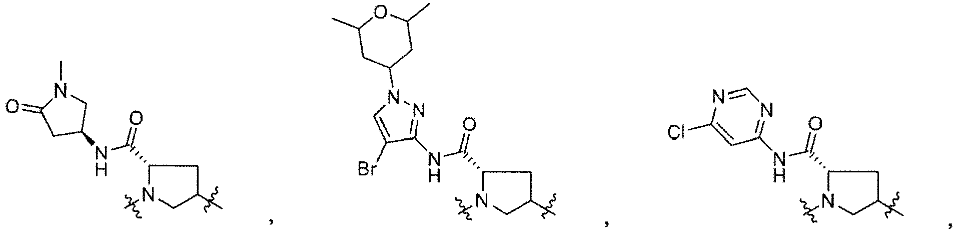

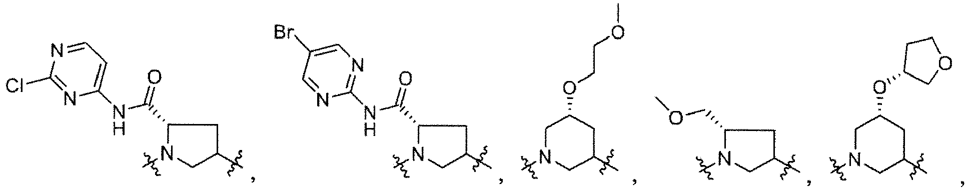

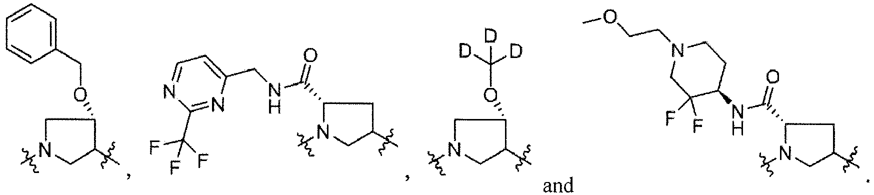

In some embodiments of the present application, the structural unit 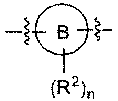 is selected from the group consisting of

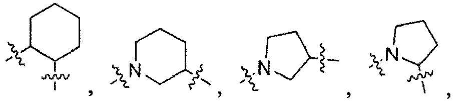

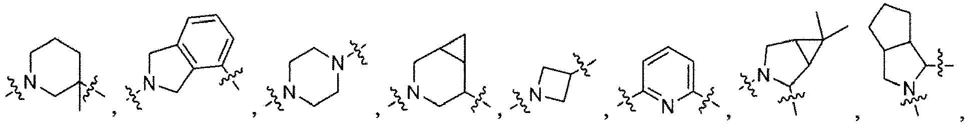

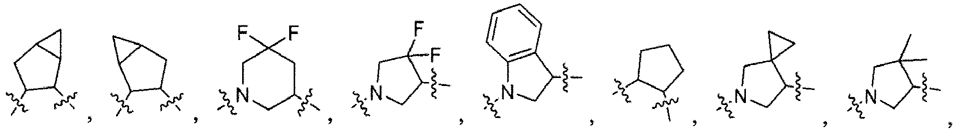

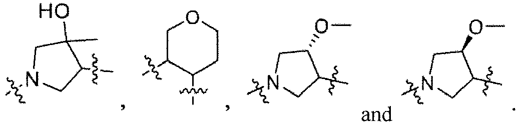

In some embodiments of the present application, the structural unit 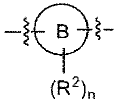 is selected from the group consisting of

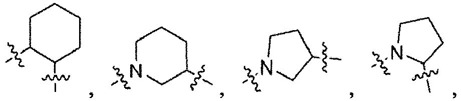

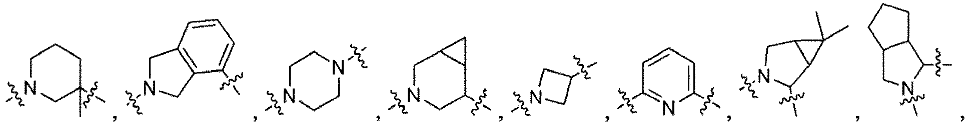

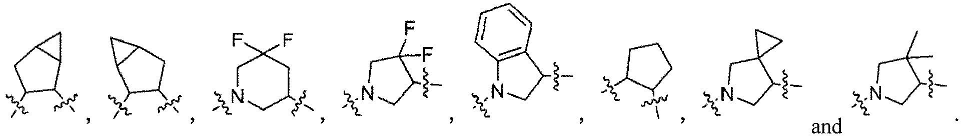

In some embodiments of the present application, the structural unit 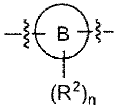 is selected from the group consisting of

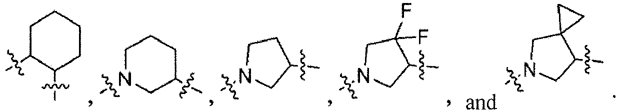

In some embodiments of the present application, Z is selected from the group consisting of -NH-, -O-, -S-, -CH₂-, -CH₂NH-, and -C(=O)-NH-.

In some embodiments of the present application, Z is selected from the group consisting of -NH-, -O-, -CH₂-, -CH₂NH-, and -C(=O)-NH-. In some embodiments of the present application, Z is -NH-.

In some embodiments of the present application, ring C is selected from the group consisting of phenyl, and pyridinyl. In some embodiments of the present application, ring C is phenyl.

In some embodiments of the present application, R³ is selected from the group consisting of nitro, Br, Cl, I, CN, CF₃, and -N(CH₃)₃⁺. In some embodiments of the present application, R³ is selected from the group consisting of nitro, Br, Cl, and CF₃.

In some embodiments of the present application, the structural unit 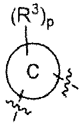 is selected from the group consisting of

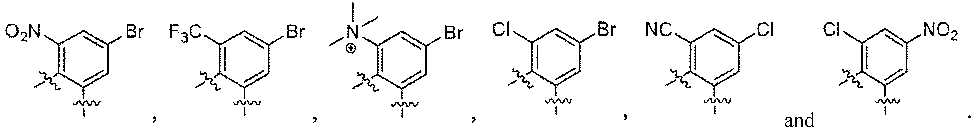

In some embodiments of the present application, the structural unit 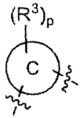 is selected from the group consisting of

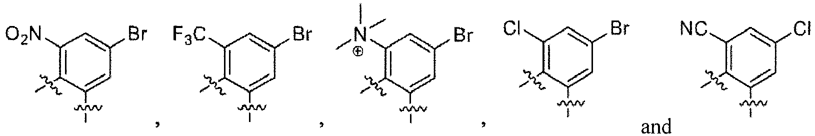

In some embodiments of the present application, the structural unit 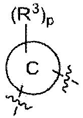 is selected from the group consisting of

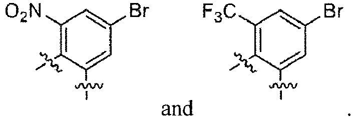

In some embodiments of the present application, ring D is selected from the group consisting of 3- to 12-membered heterocyclyl, wherein heterocyclyl contains 1 to 3 heteroatoms selected from the group consisting of N, O, and S.

In some embodiments of the present application, ring D is selected from the group consisting of 4- to 7-membered monocyclic heterocyclyl, 6- to 9-membered fused heterocyclyl, 7- to 11-membered spiroheterocyclyl, and 7- to 9-membered bridged heterocyclyl, wherein heterocyclyl contains 1 to 3 heteroatoms selected from the group consisting of N, O, and S.

In some embodiments of the present application, ring D is selected from the group consisting of 5- to 6-membered heteroaryl.

In some embodiments of the present application, ring D is selected from the group consisting of 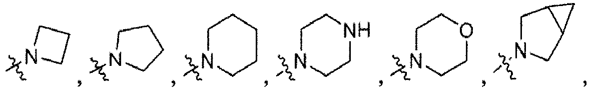 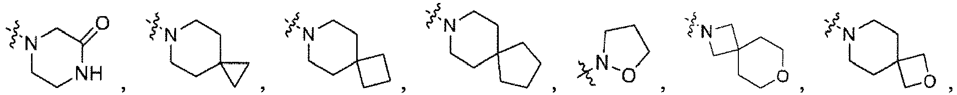 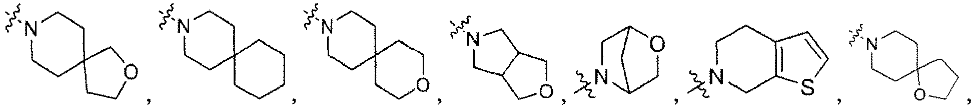 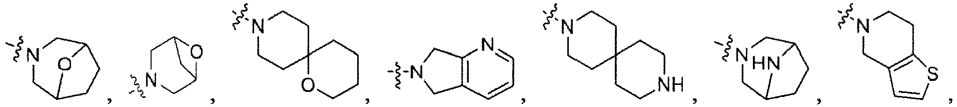 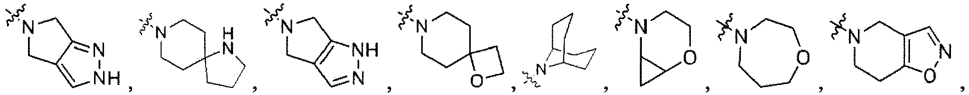 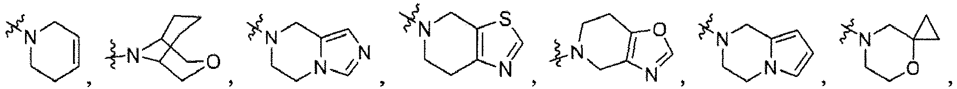 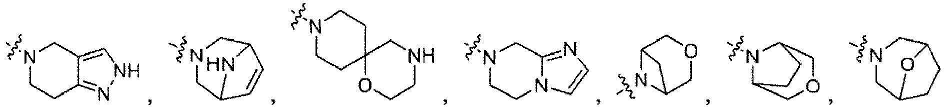 and 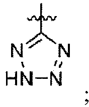 or ring D is absent.

In some embodiments of the present application, ring D is selected from the group consisting of 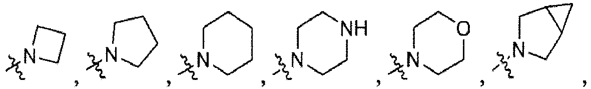 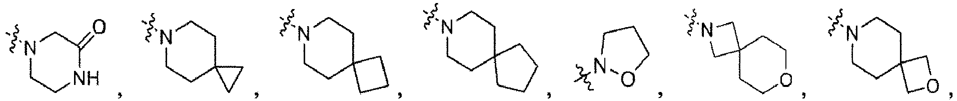 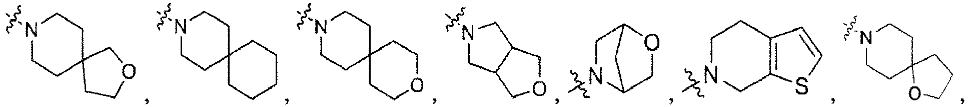 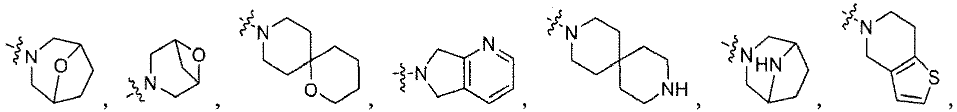 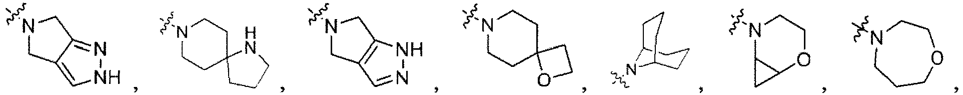 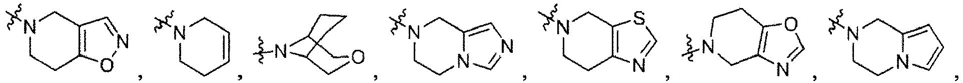 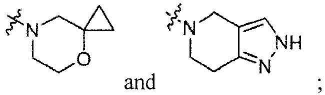 or ring D is absent.

In some embodiments of the present application, ring D is selected from the group consisting of 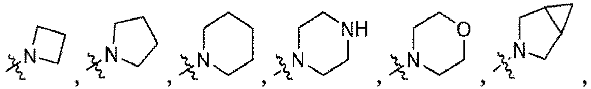 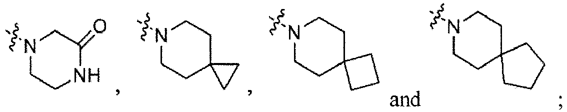 or ring D is absent.

In some embodiments of the present application, ring D is selected from the group consisting of

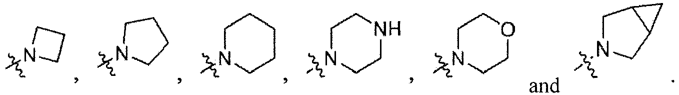

In some embodiments of the present application, ring D is selected from the group consisting of

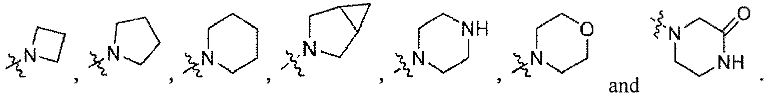

In some embodiments of the present application, R⁴ is selected from the group consisting of deuterium, F, Cl, methyl, tert-butyl, methoxy, amino, hydroxyl, 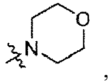 -CH₂CF₃, -CH₂F, -CH₂Cl, vinyl, ethynyl, -CF₃, -CHF₂, 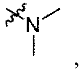 -C(=O)CF₃, -NHC(=O)CF₃, -CH₂CH₂OCH₃, -CH₂CH₂OH, 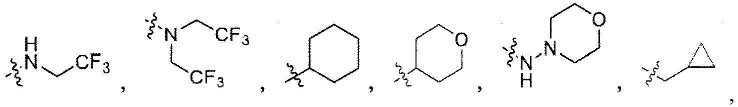 cyclopropyl, 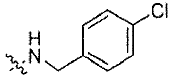 and 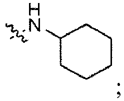 and q is 0, 1, 2, 3, 4, or 5.

In some embodiments of the present application, R⁴ is selected from the group consisting of F, Cl, methyl, tert-butyl, methoxy, amino, hydroxyl, 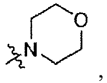 -CH₂CF₃, -CH₂F, -CHF₂, 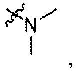 -C(=O)CF₃, -NHC(=O)CF₃, -CH₂CH₂OCH₃, -CH₂CH₂OH, 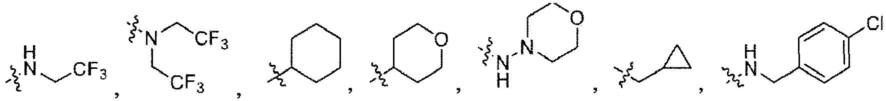 and 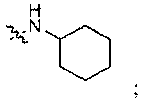 and q is 0, 1, 2, 3, 4, or 5.

In some embodiments of the present application, R⁴ is selected from the group consisting of F, methyl, 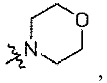 -CH₂CF₃, -CH₂F, -C(=O)CF₃, -NHC(=O)CF₃, -CH₂CH₂OCH₃, 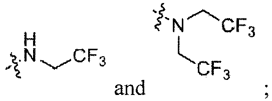 and q is 0, 1, 2, or 3.

In some embodiments of the present application, R⁴ is selected from the group consisting of F, methyl, 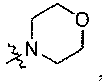 -CH₂CF₃, -C(=O)CF₃, -NHC(=O)CF₃, -CH₂CH₂OCH₃, 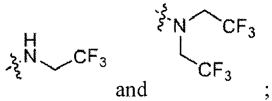 and q is 0, 1,2, or 3.

In some embodiments of the present application, R⁴ is selected from the group consisting of F, methyl, 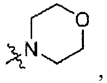 -CH₂CF₃, and -C(=O)CF₃.

In some embodiments of the present application, R⁴ is selected from the group consisting of F and methyl; and q is 0, 1, or 2.

In some embodiments of the present application, the structural unit 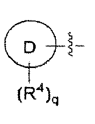 is selected from the group consisting of

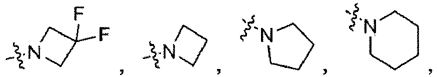

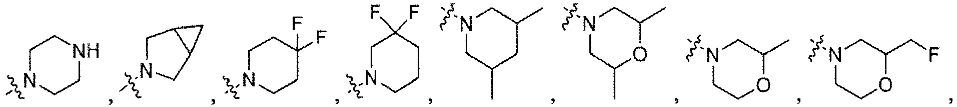

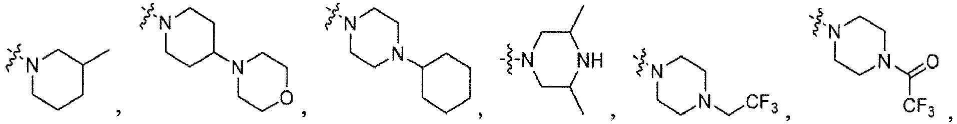

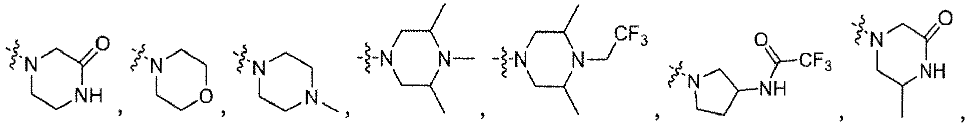

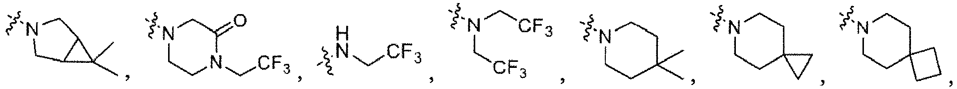

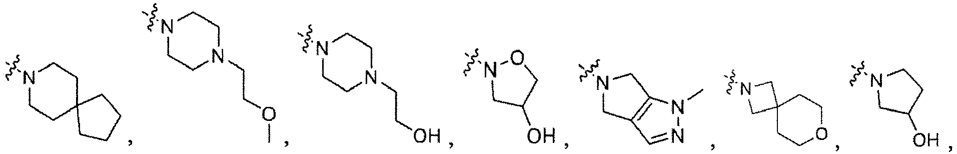

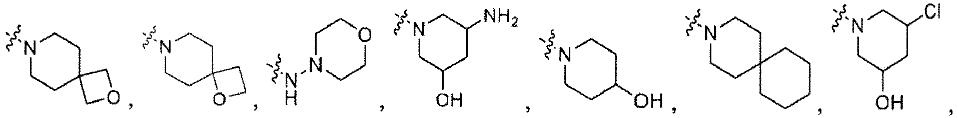

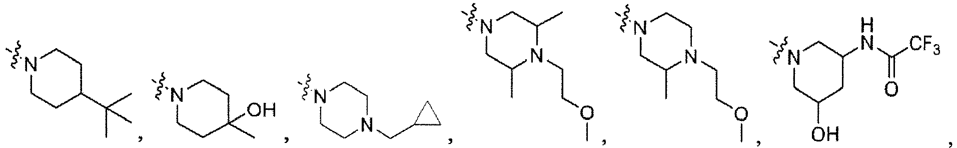

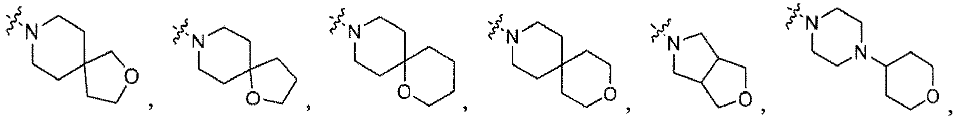

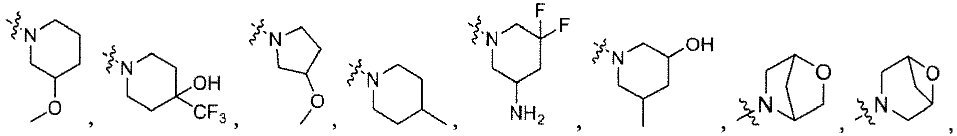

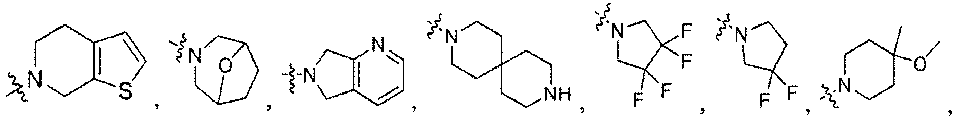

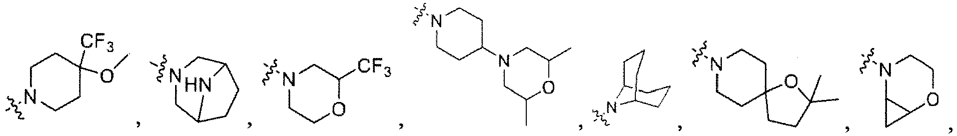

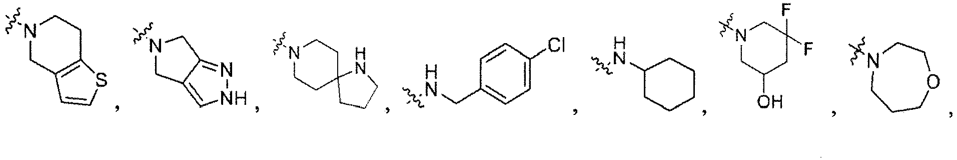

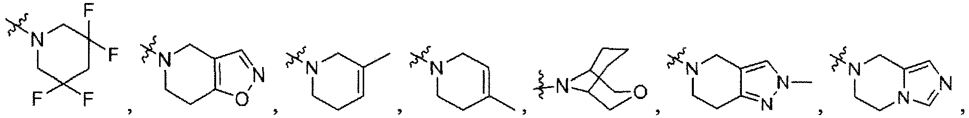

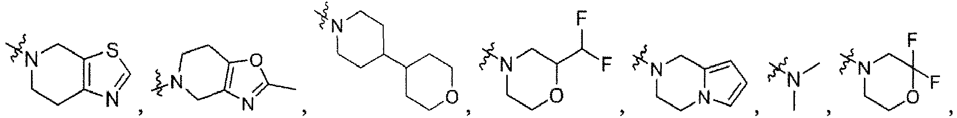

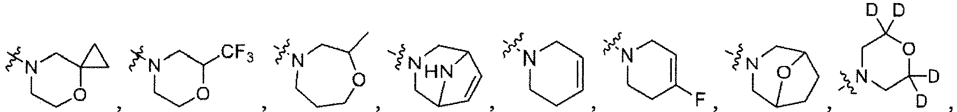

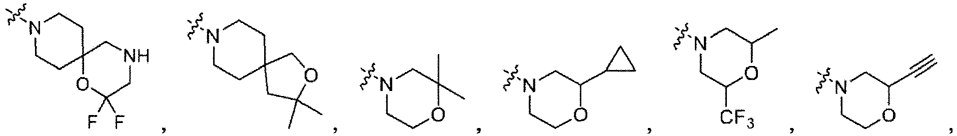

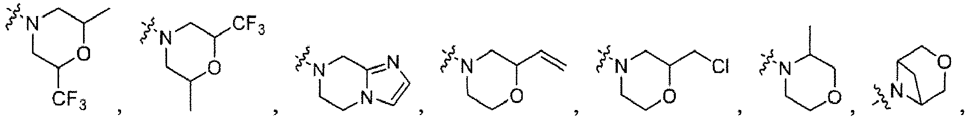

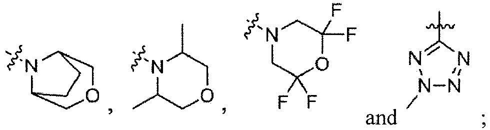

In some embodiments of the present application, the structural unit 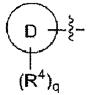 is selected from the group consisting of

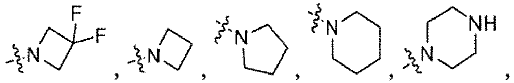

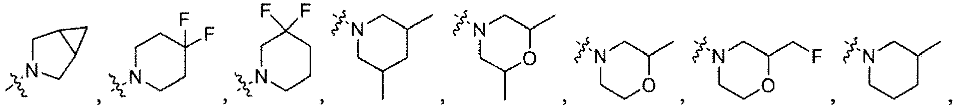

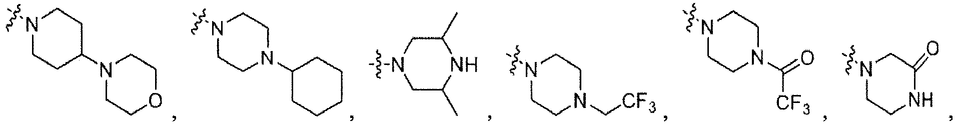

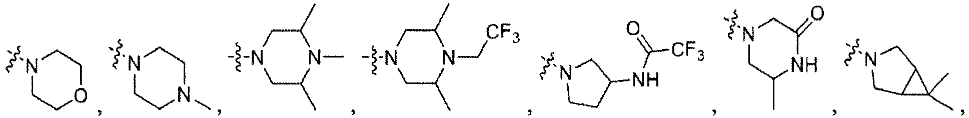

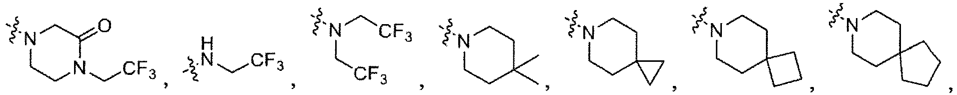

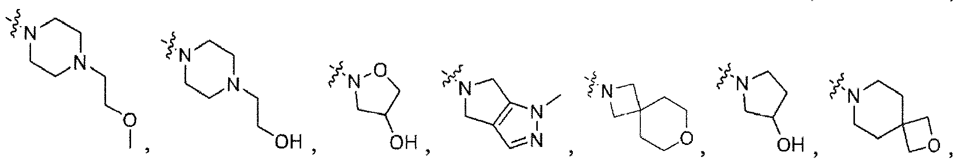

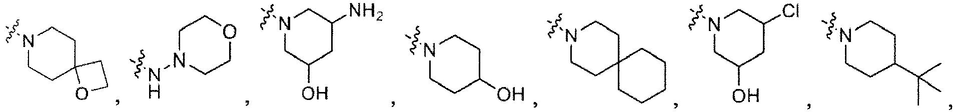

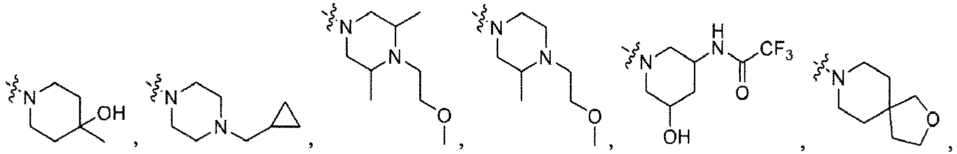

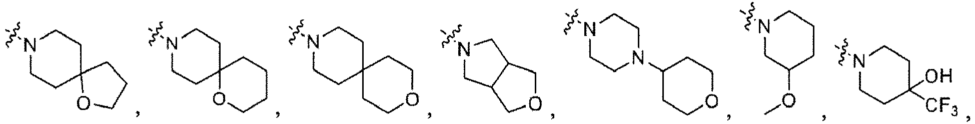

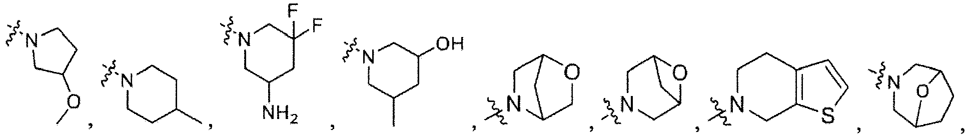

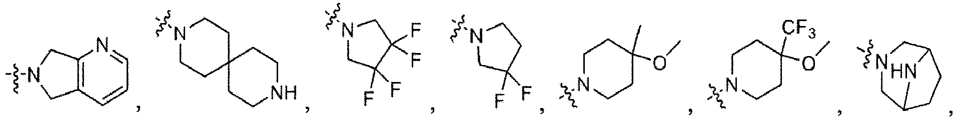

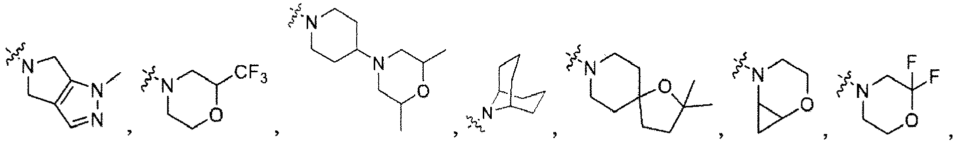

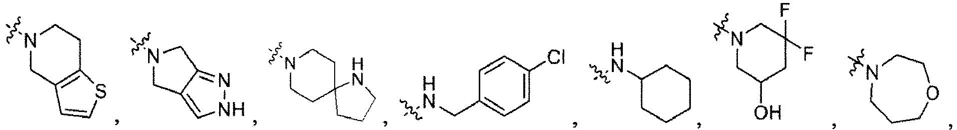

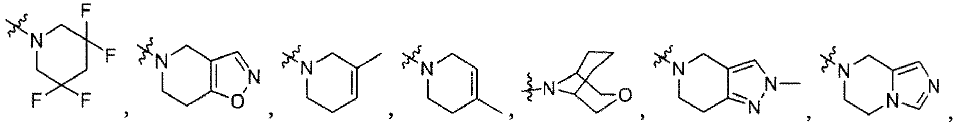

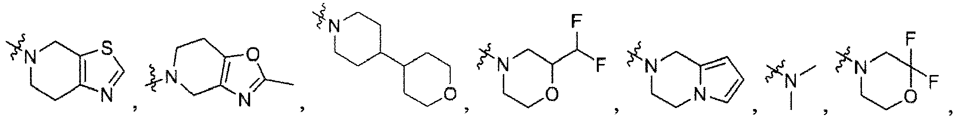

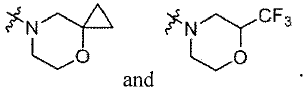

In some embodiments of the present application, the structural unit 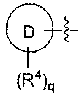 is selected from the group consisting of

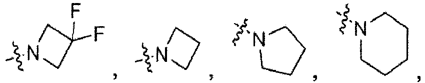

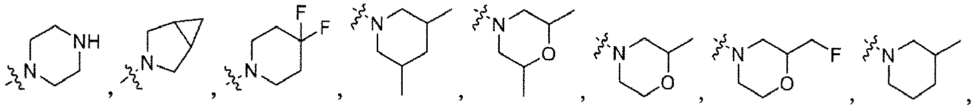

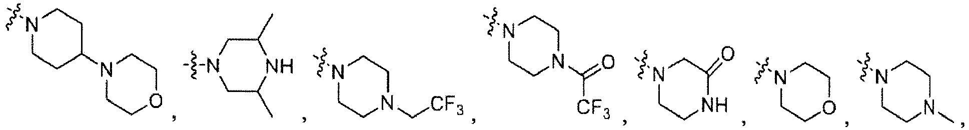

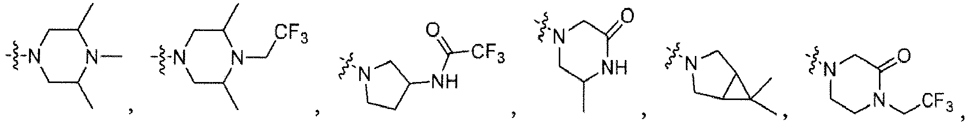

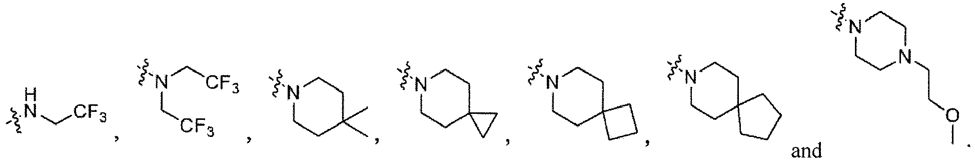

In some embodiments of the present application, the structural unit 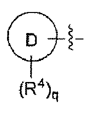 is selected from the group consisting of

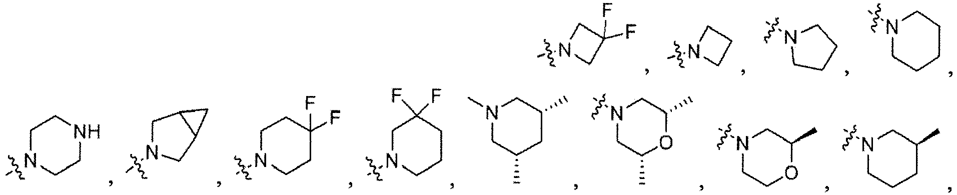

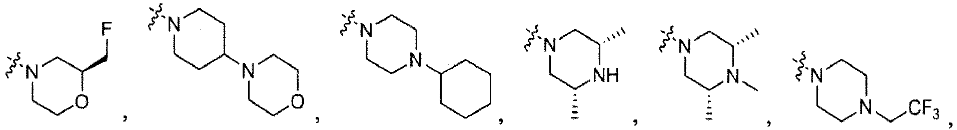

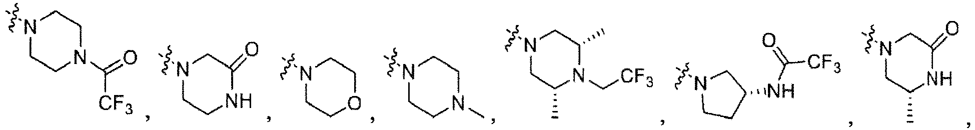

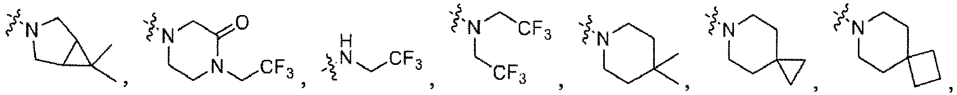

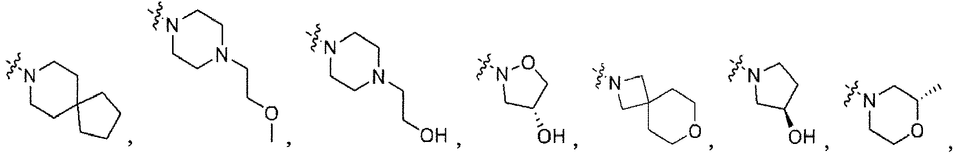

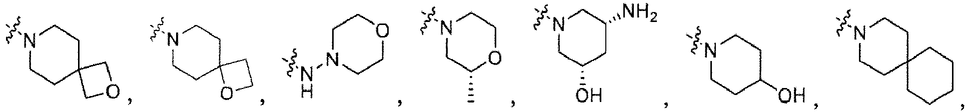

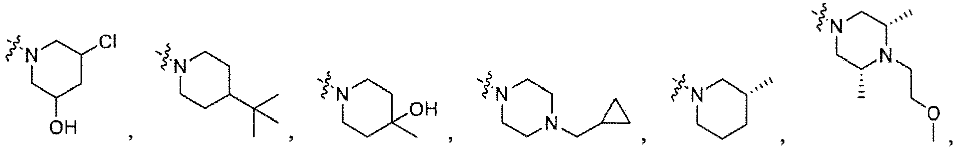

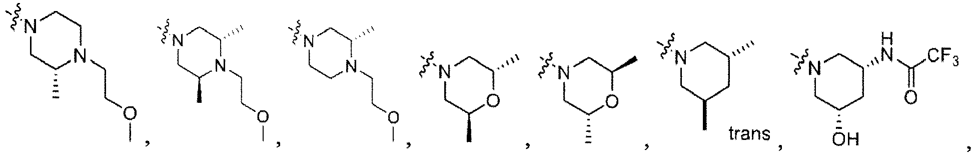

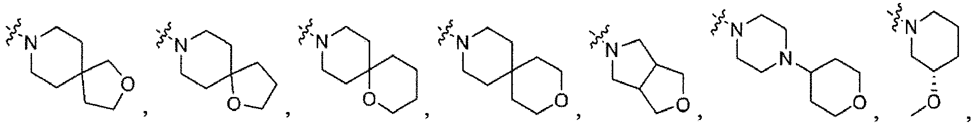

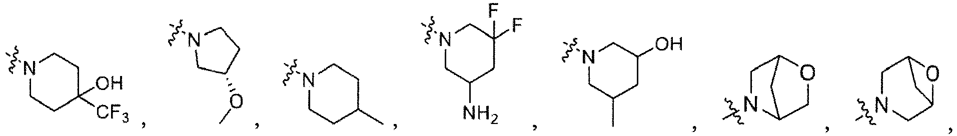

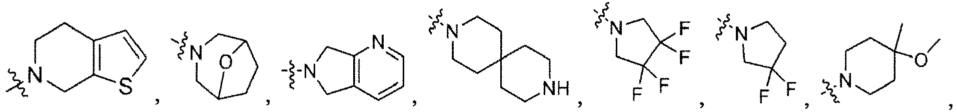

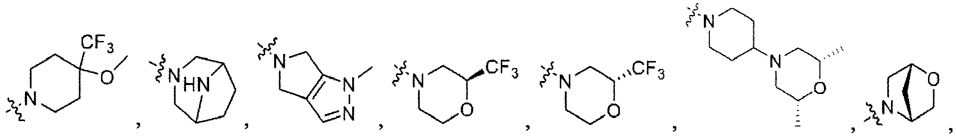

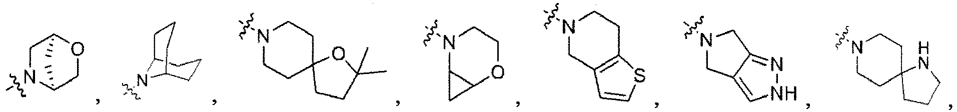

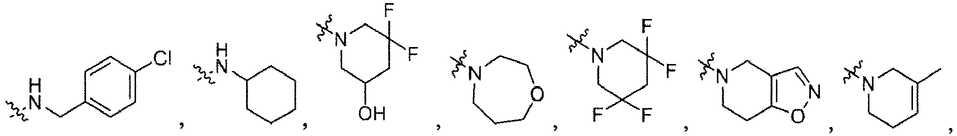

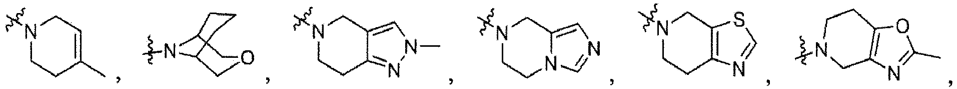

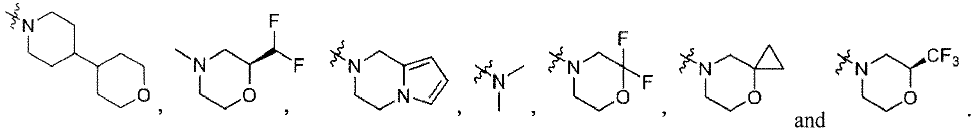

In some embodiments of the present application, the structural unit 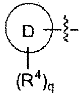 is selected from the group consisting of 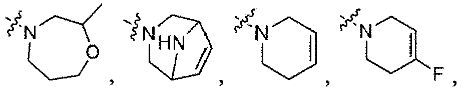 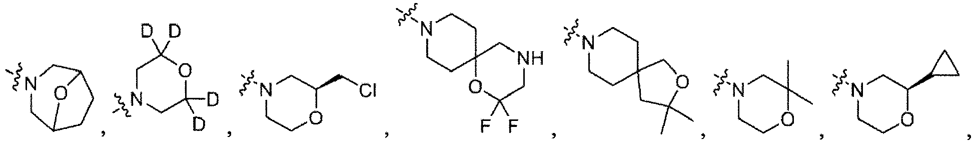 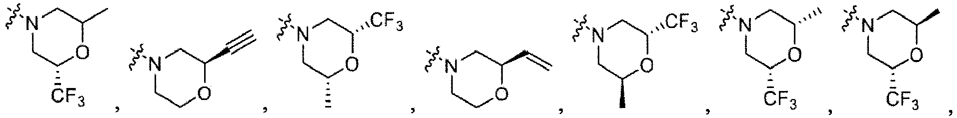 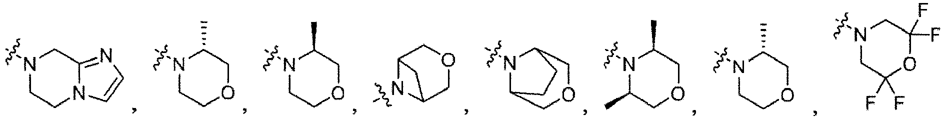 and

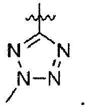

In some embodiments of the present application, the structural unit 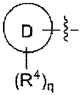 is selected from the group consisting of

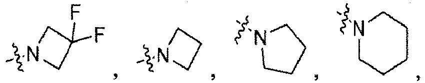

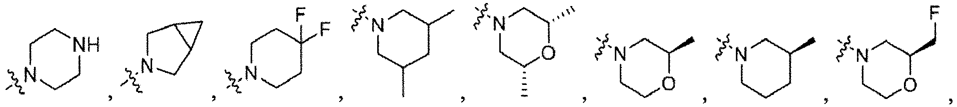

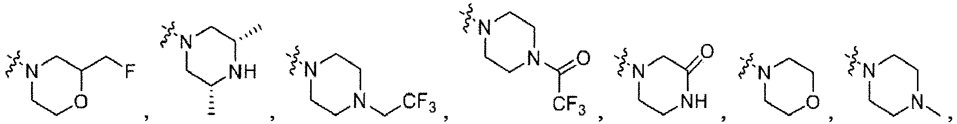

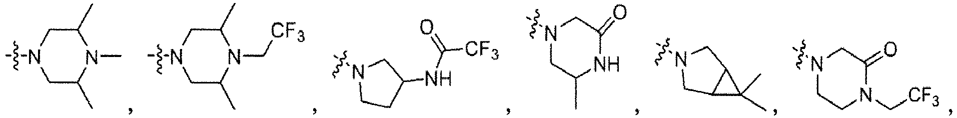

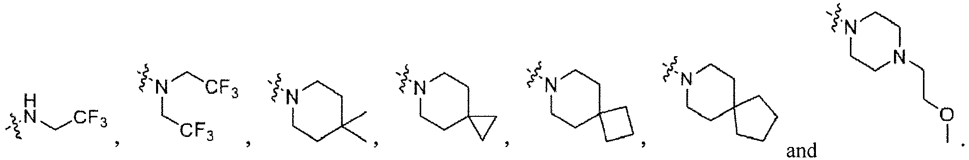

In some embodiments of the present application, the structural unit 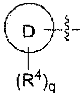 is selected from the group consisting of

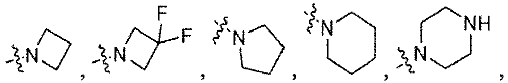

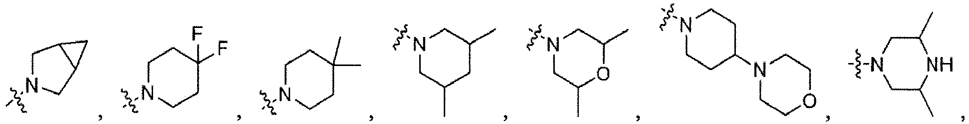

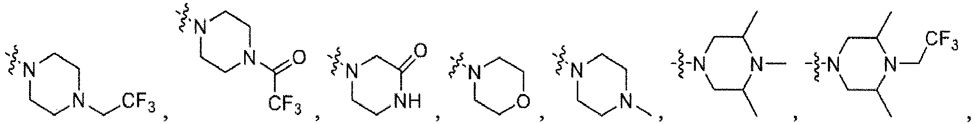

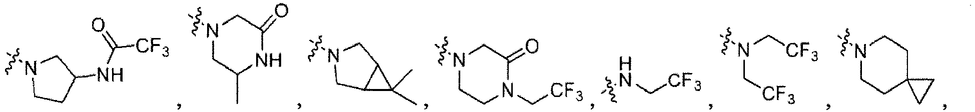

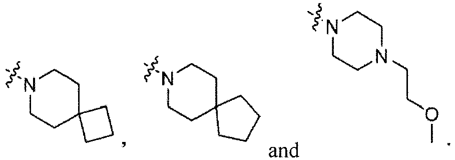

In some embodiments of the present application, the structural unit 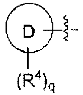 is selected from the group consisting of

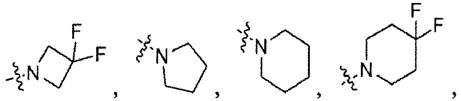

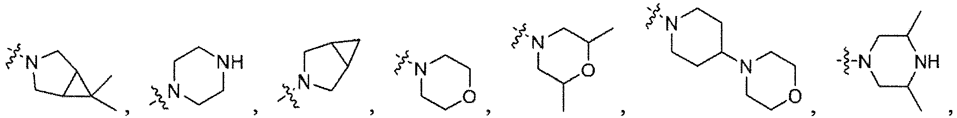

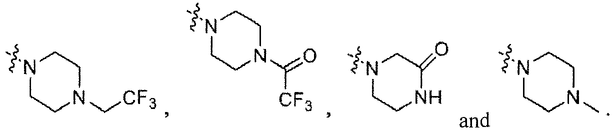

In some embodiments of the present application, when ring D is absent, ring A is pyridinyl or 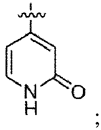 R⁴ is 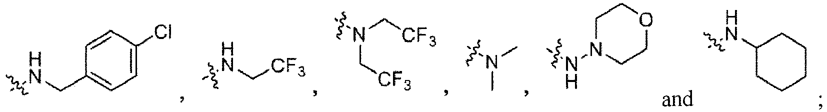 and q is 1.

In some embodiments of the present application, the compound and the isomer or the pharmaceutically acceptable salt thereof are selected from the group consisting of: 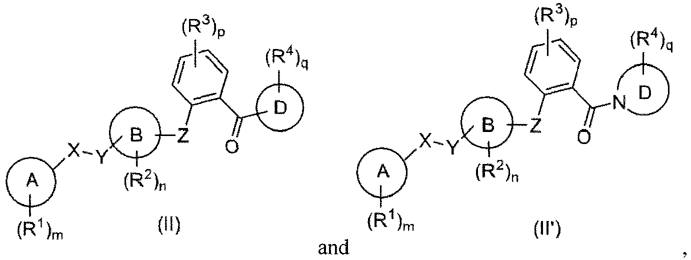 wherein, ring A, ring B, ring D, X, Y, Z, R¹, R², R³, R⁴, m, n, p, and q are as defined in formula (I).

In some embodiments of the present application, the compound is selected from the group consisting of: 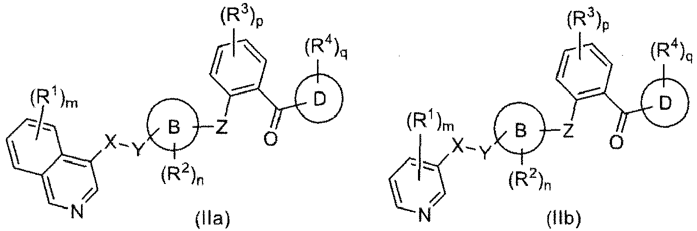 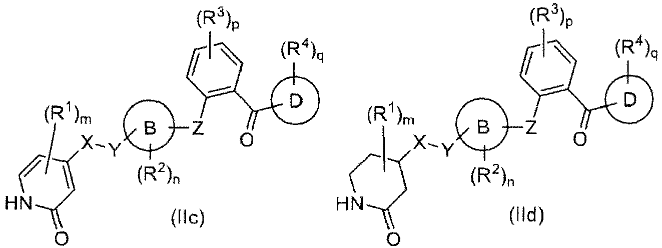 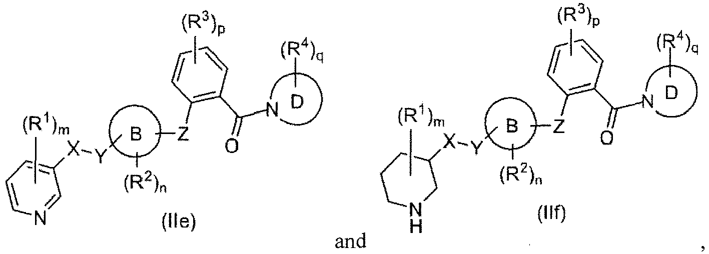 wherein, ring B, ring D, X, Y, Z, R¹, R², R³, R⁴, m, n, p, and q are as defined in formula (I).

In some embodiments of the present application, the compound and the isomer thereof are selected from the group consisting of: 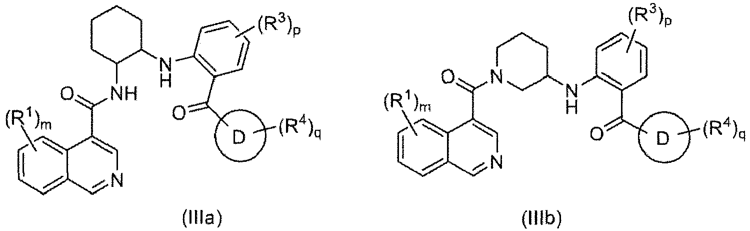 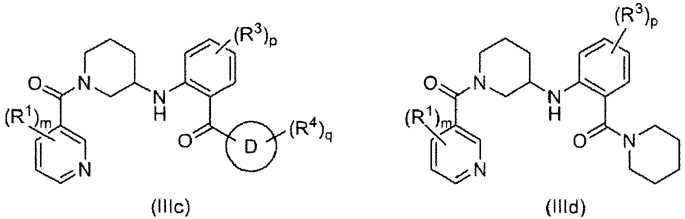 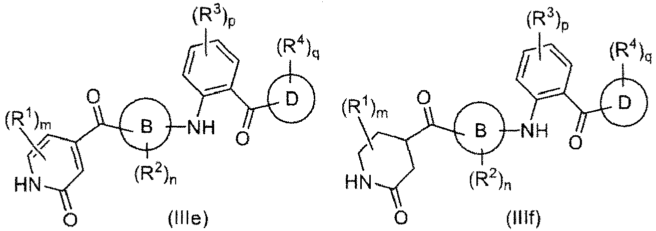 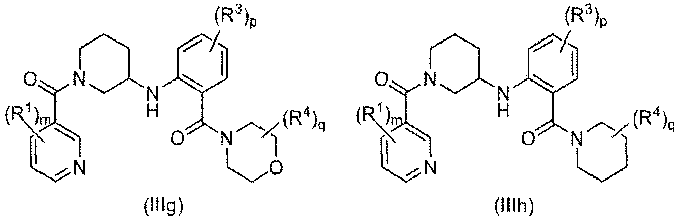 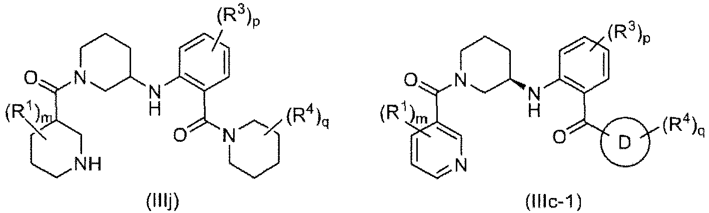 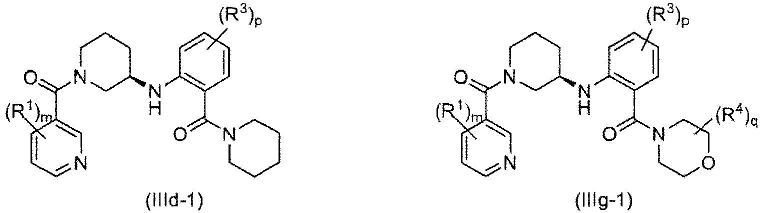 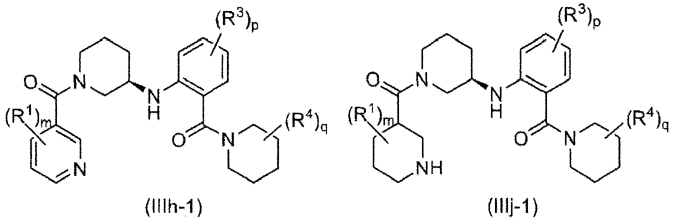 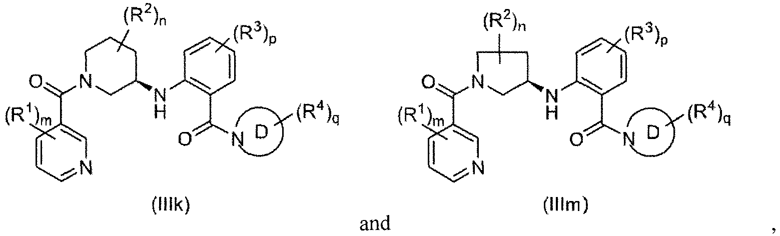 wherein, ring B, ring D, R¹, R², R³, R⁴, m, n, p, and q are as defined in formula (I).

The present application further provides the compound, and the isomer or a pharmaceutically acceptable salt thereof selected from the group consisting of:

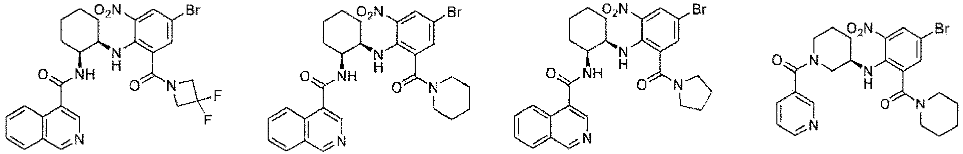

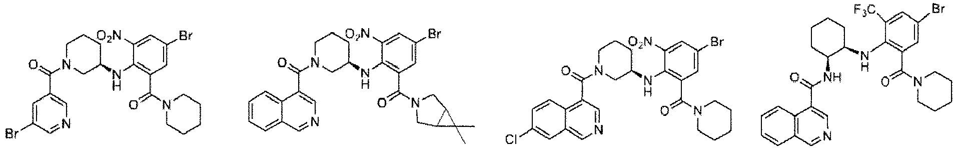

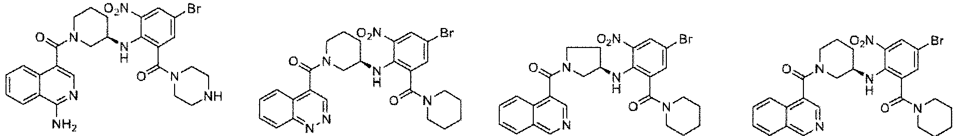

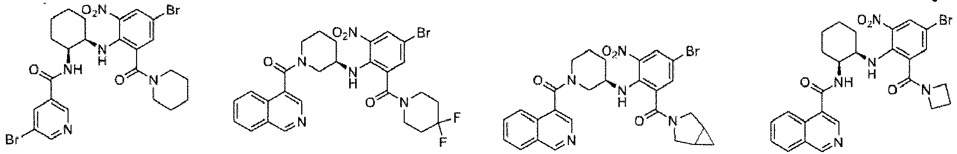

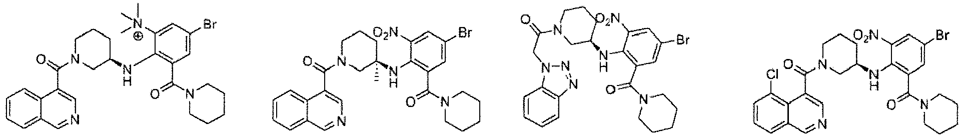

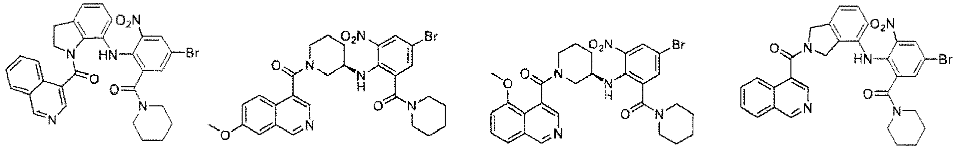

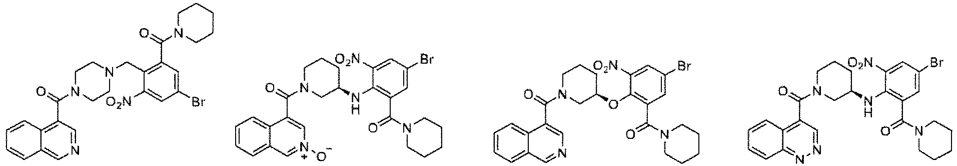

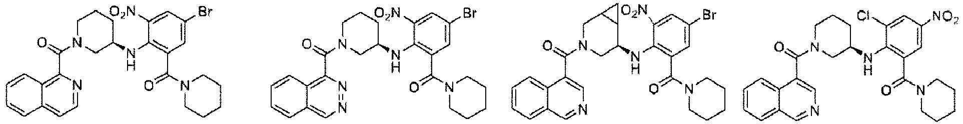

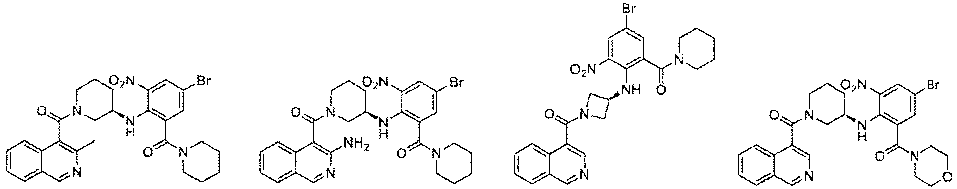

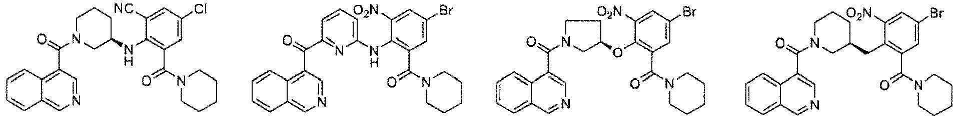

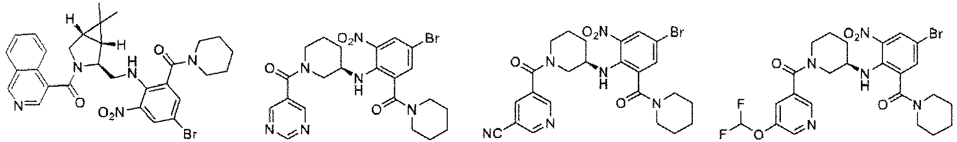

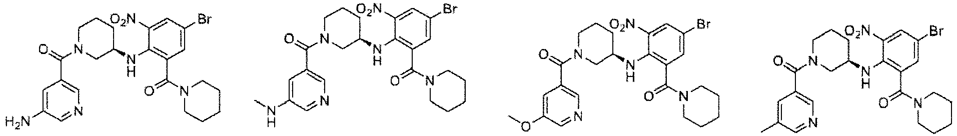

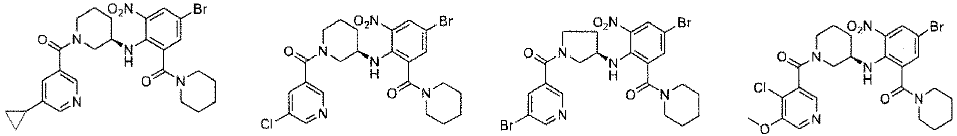

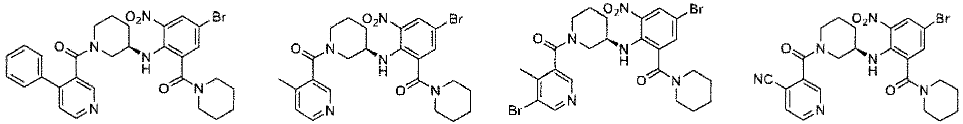

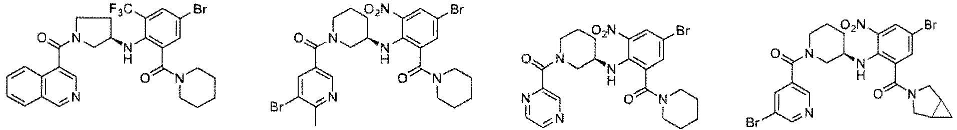

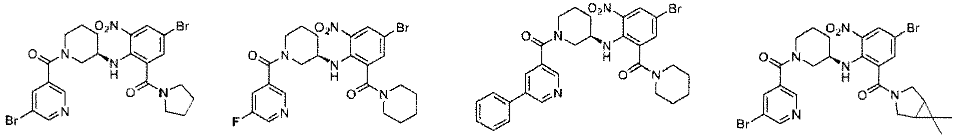

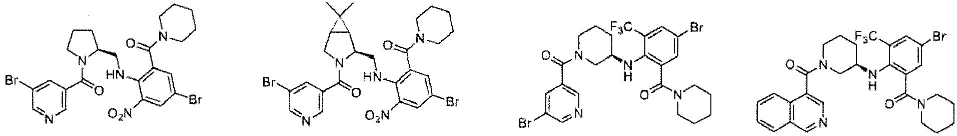

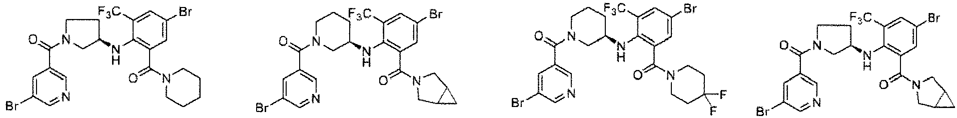

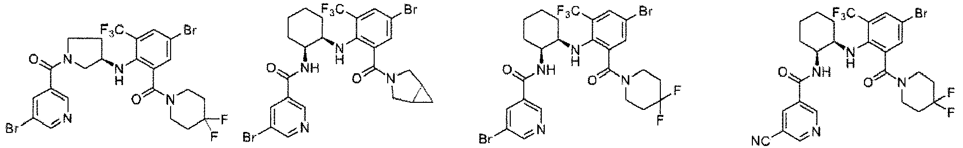

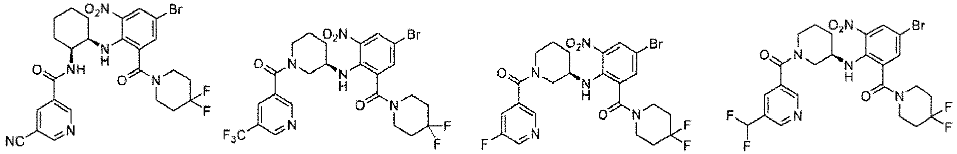

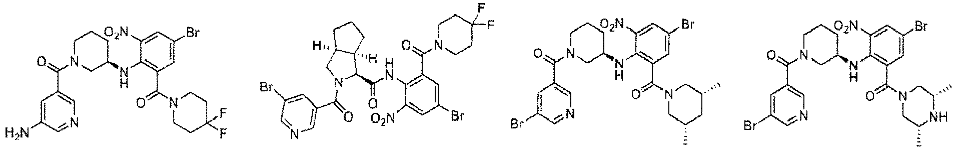

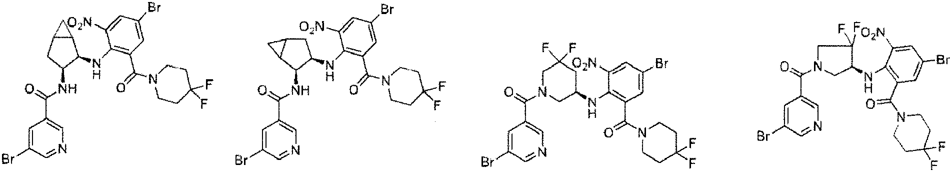

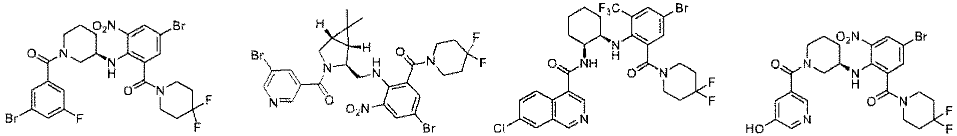

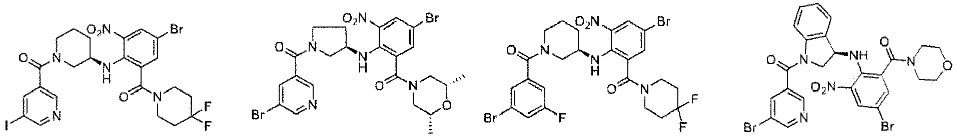

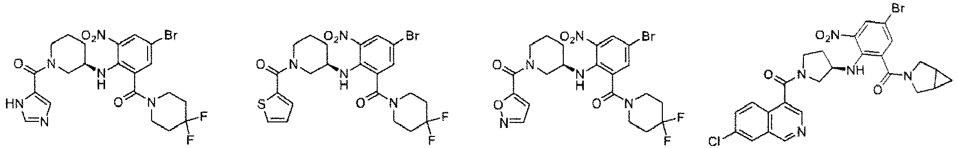

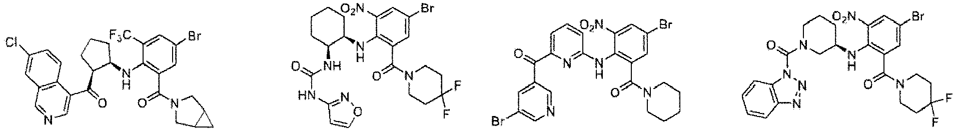

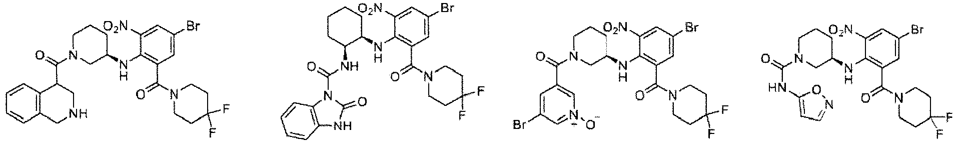

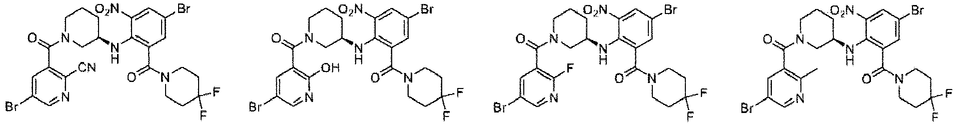

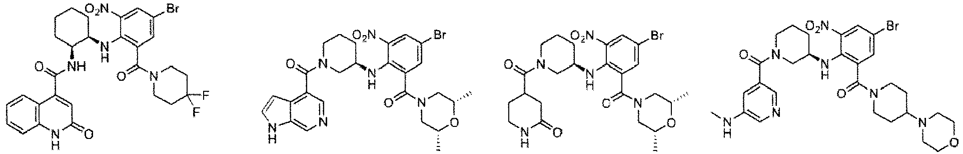

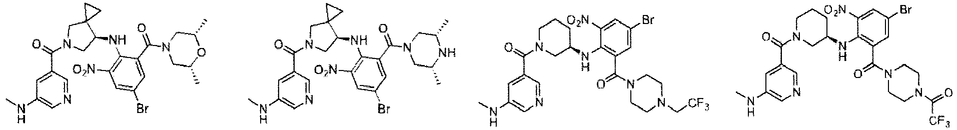

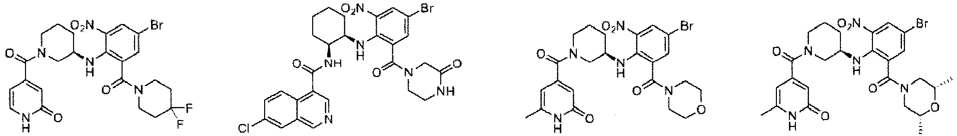

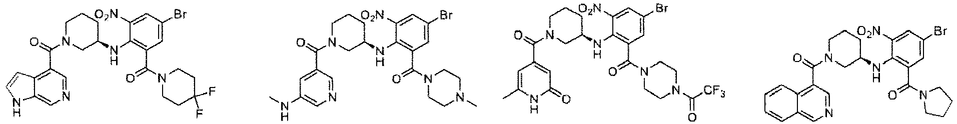

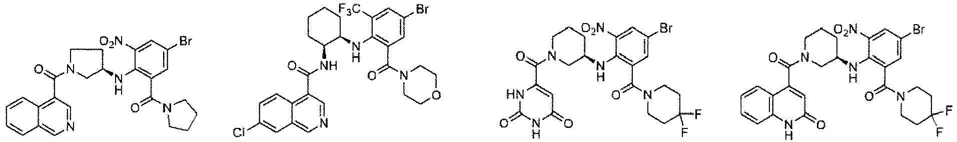

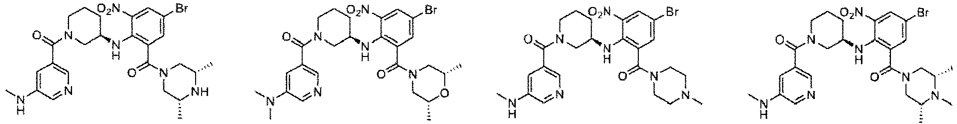

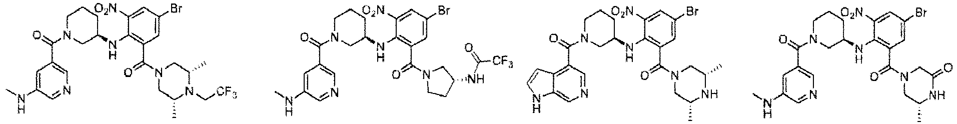

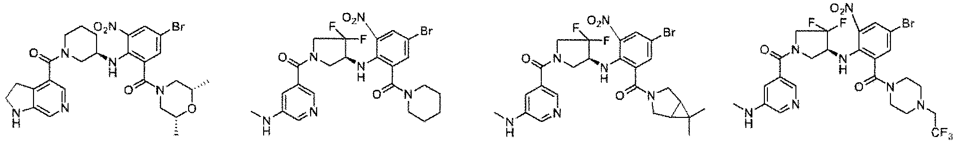

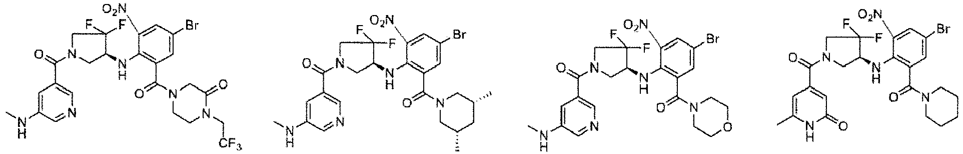

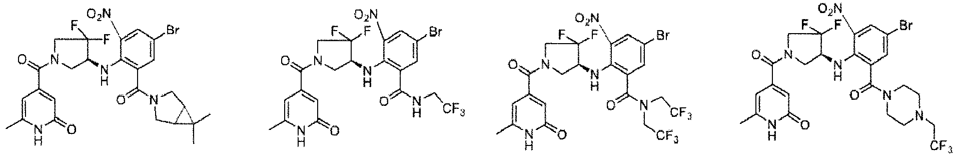

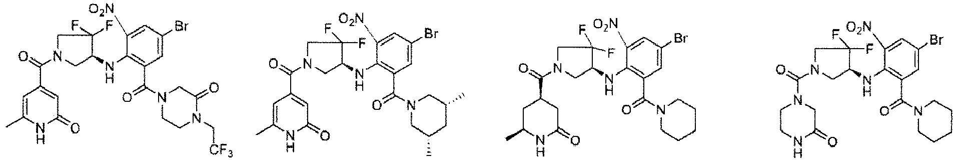

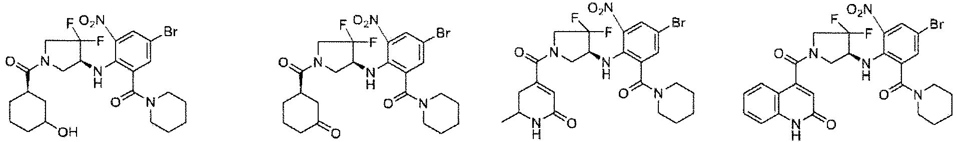

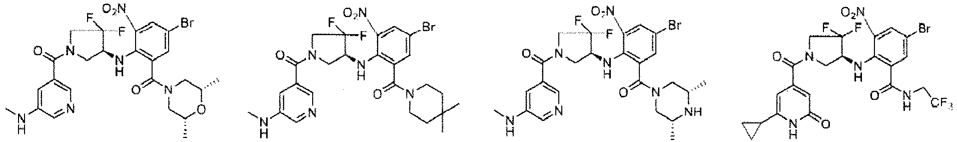

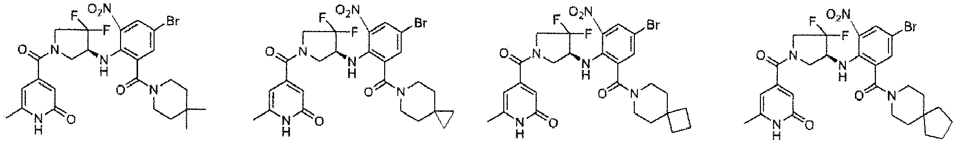

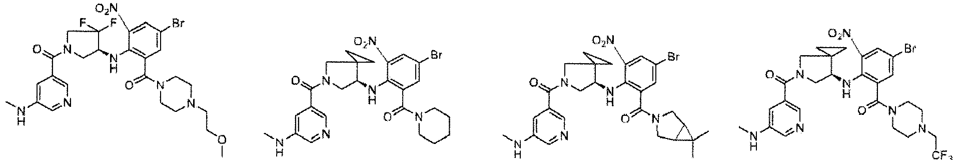

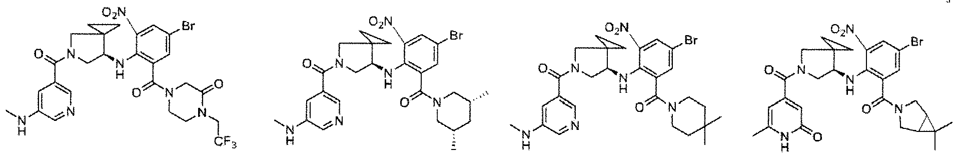

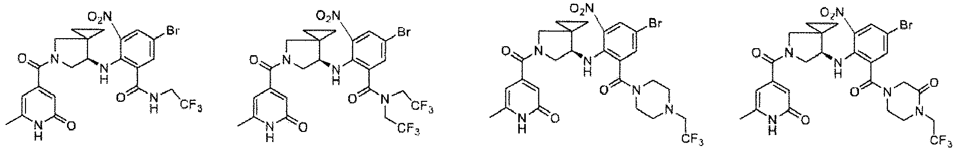

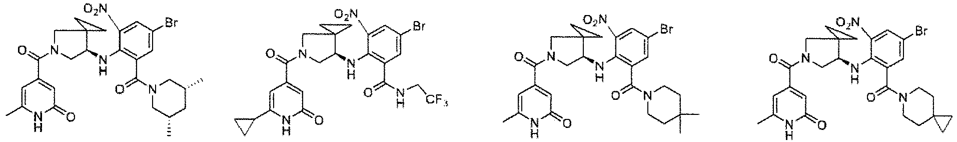

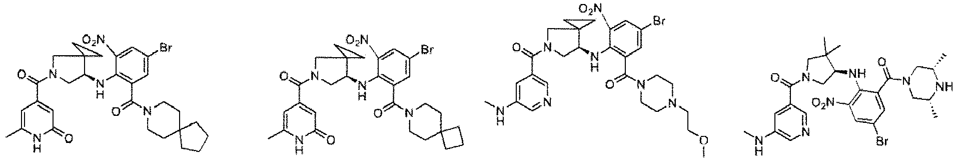

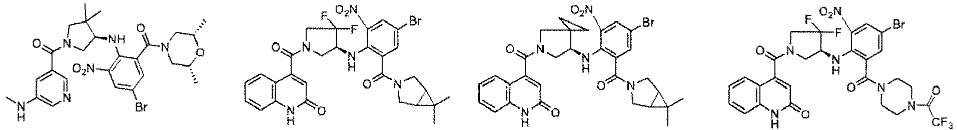

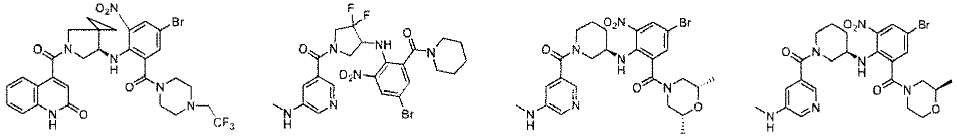

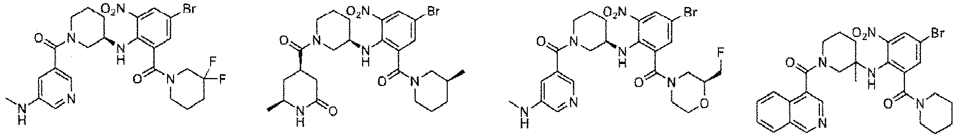

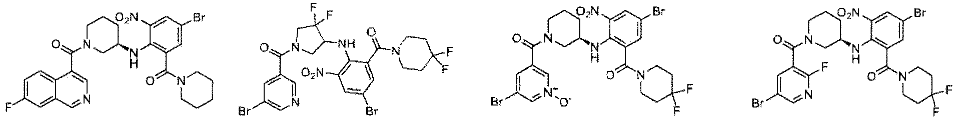

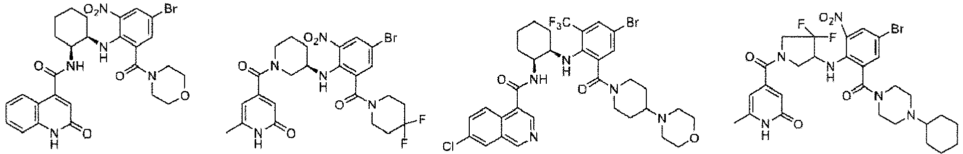

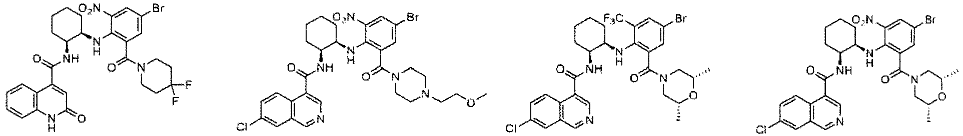

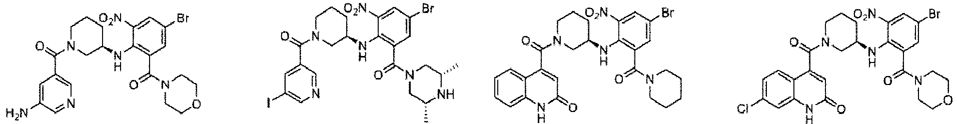

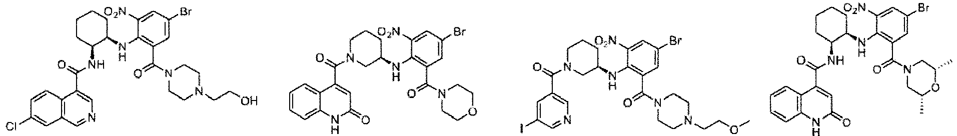

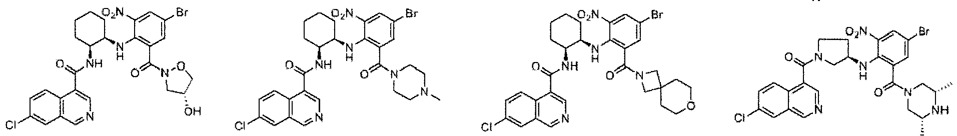

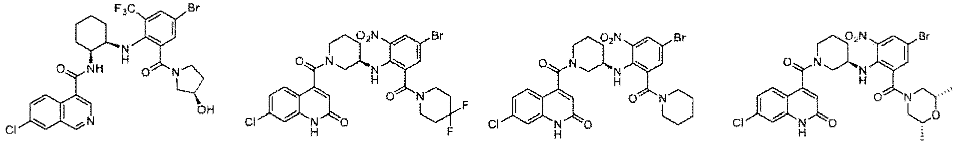

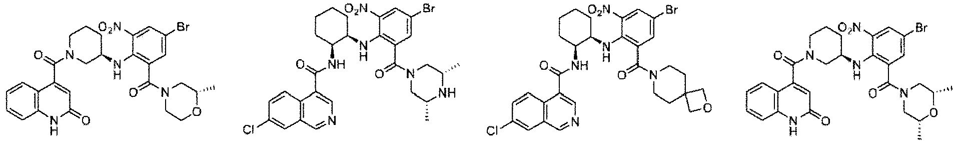

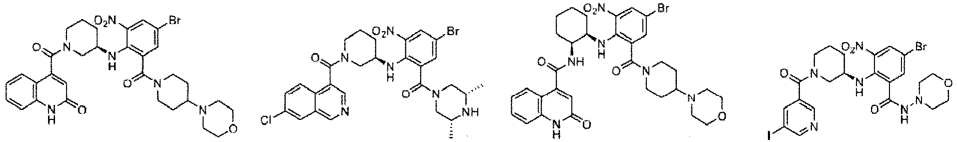

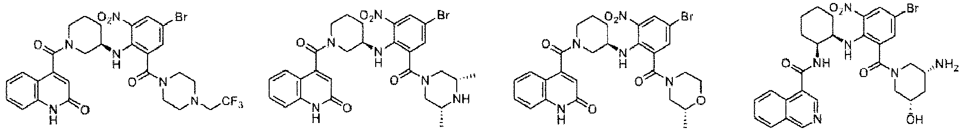

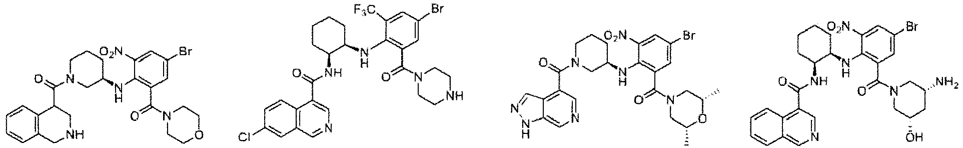

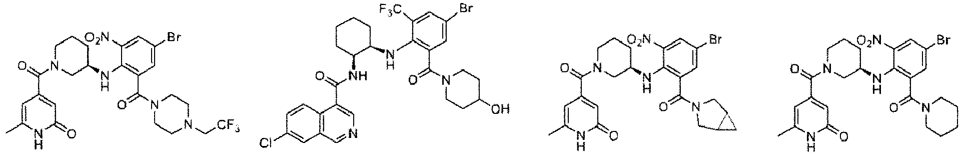

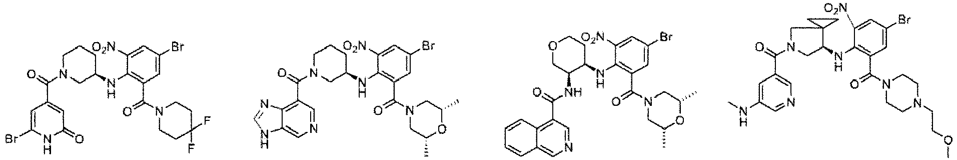

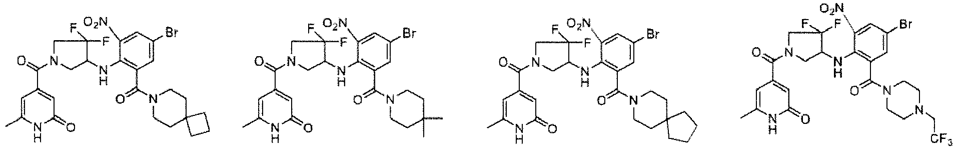

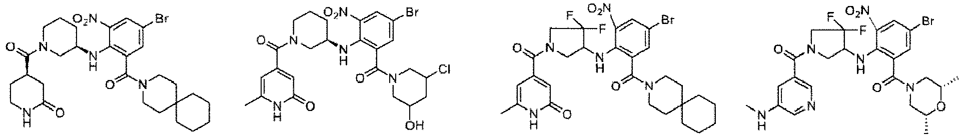

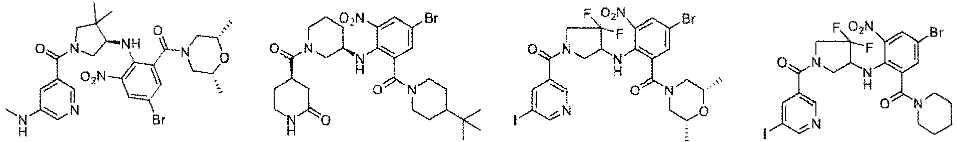

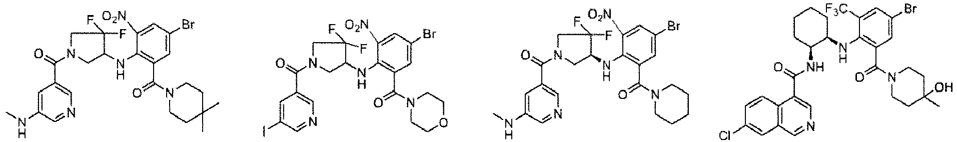

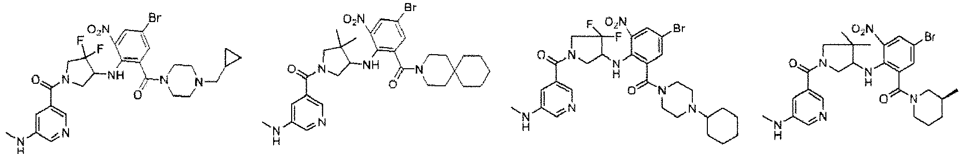

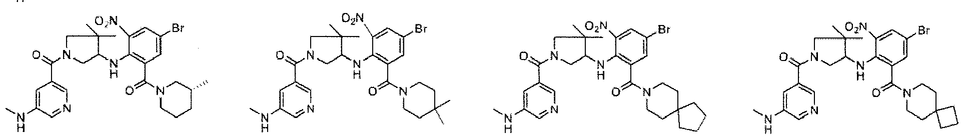

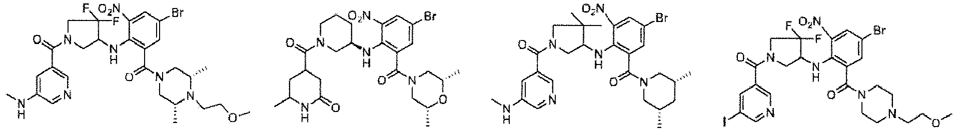

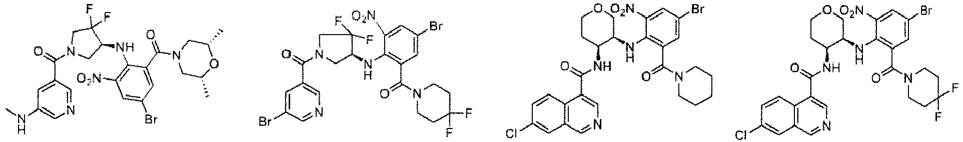

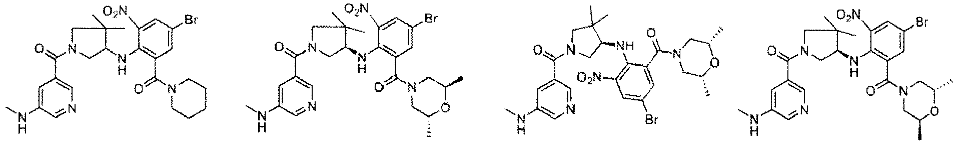

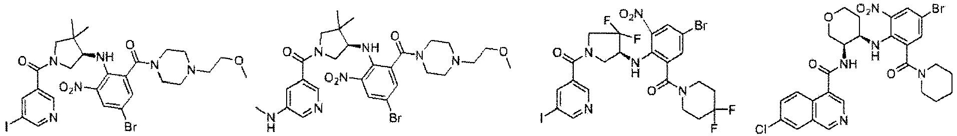

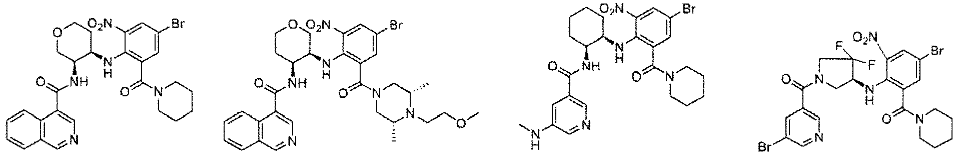

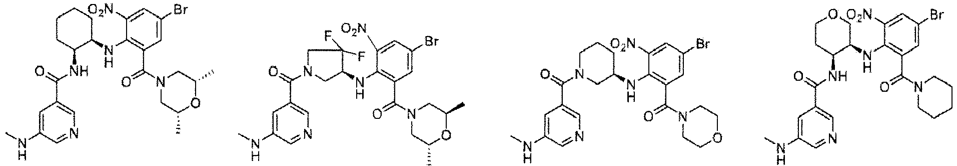

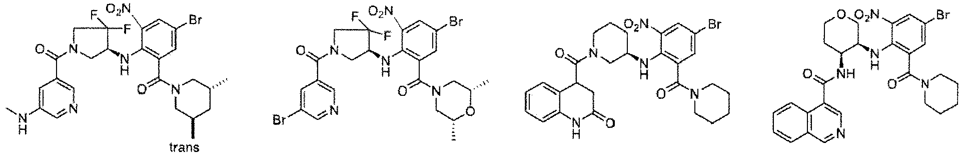

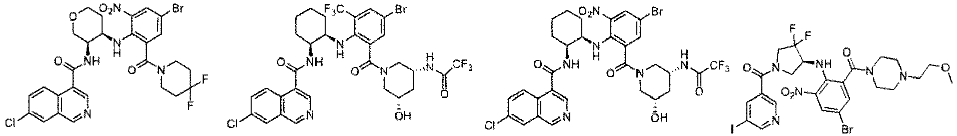

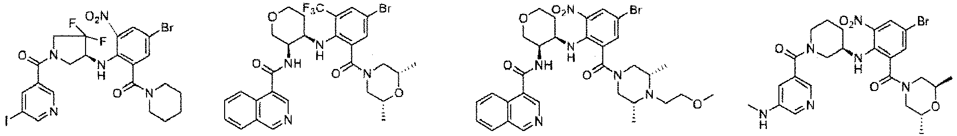

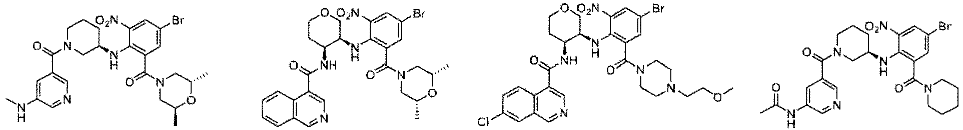

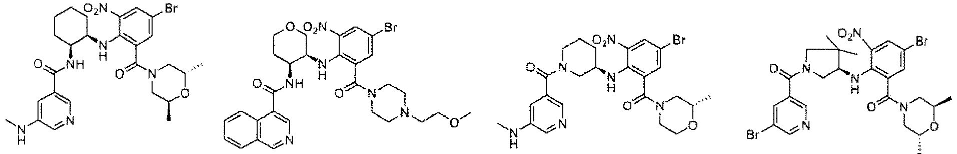

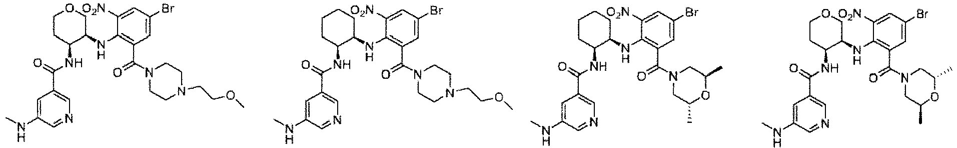

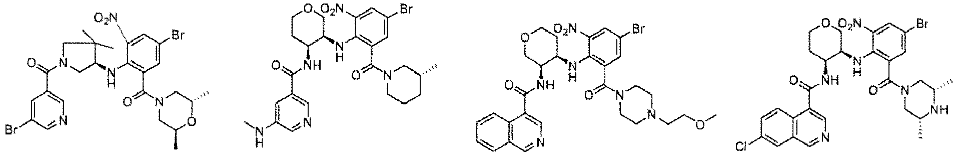

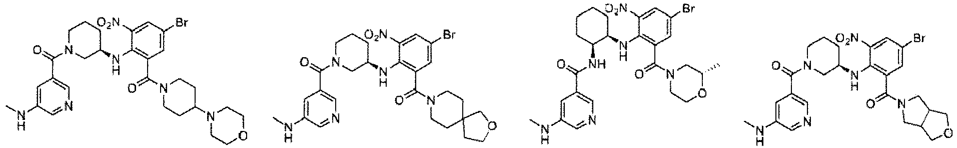

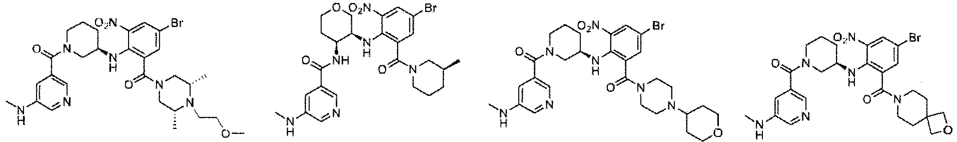

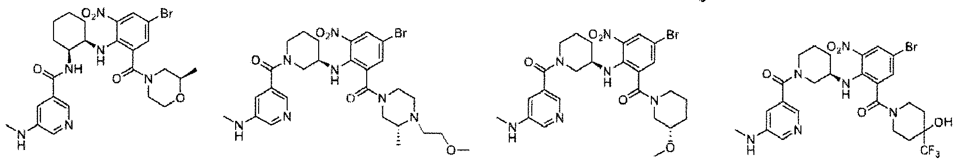

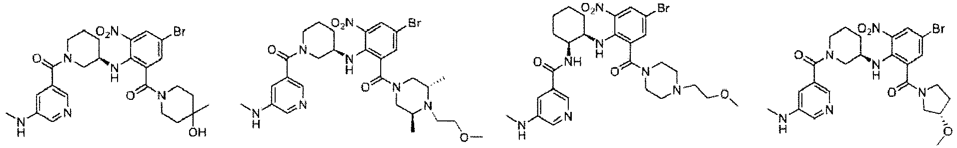

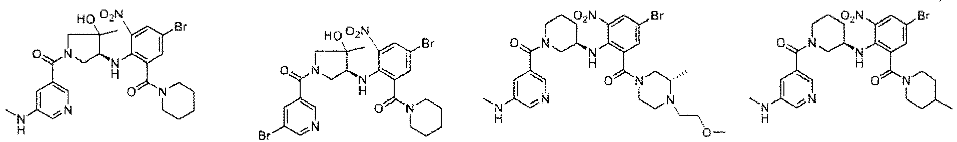

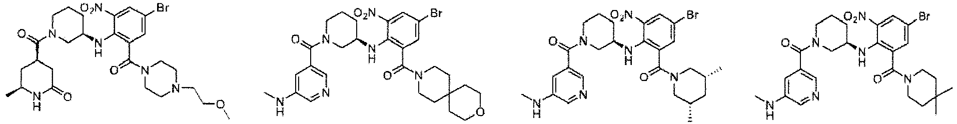

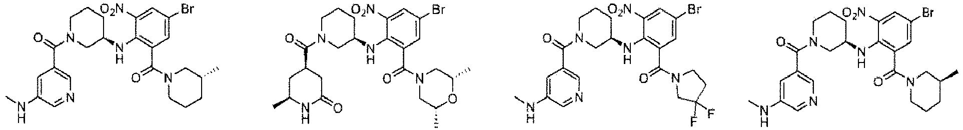

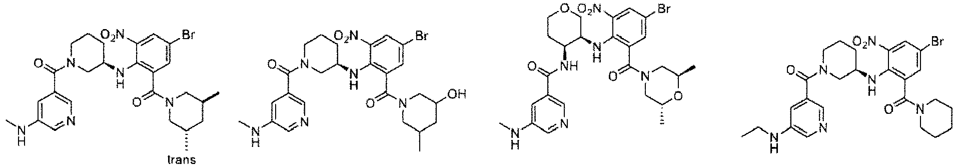

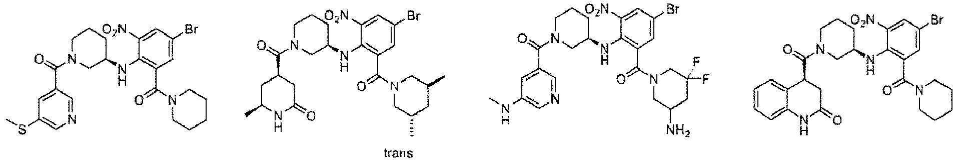

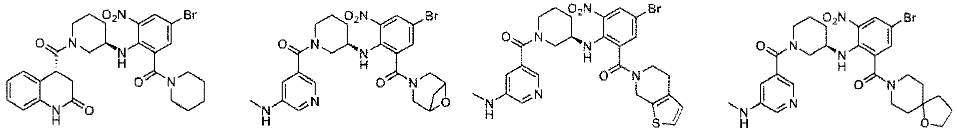

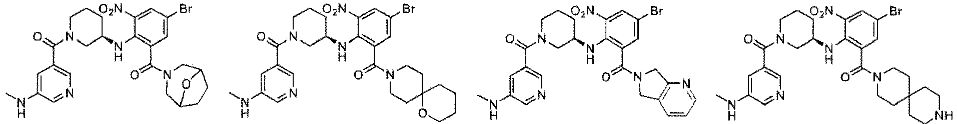

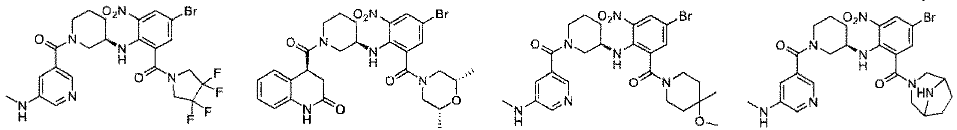

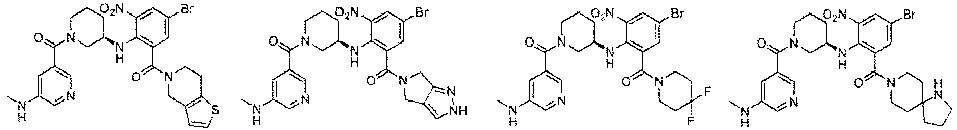

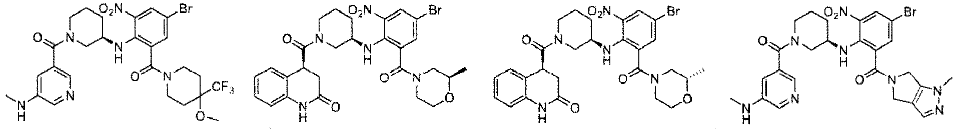

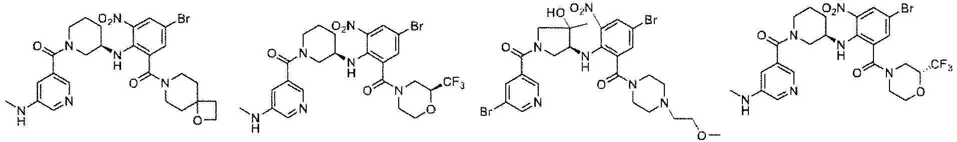

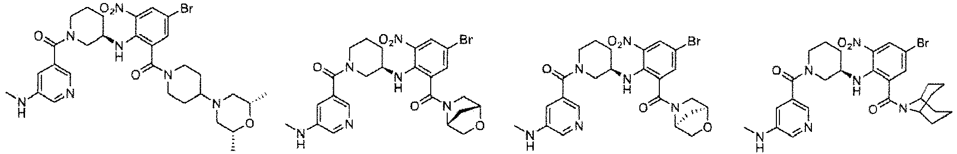

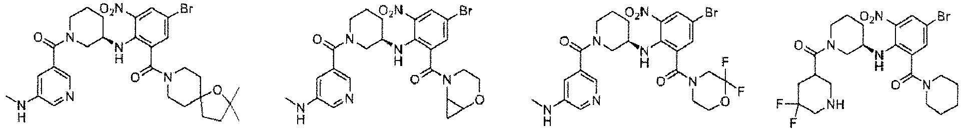

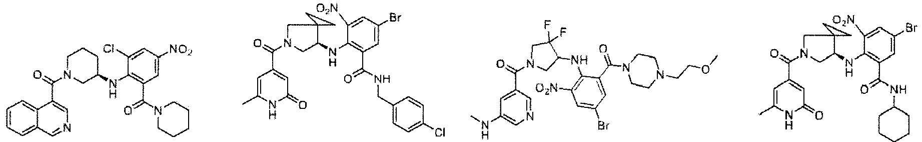

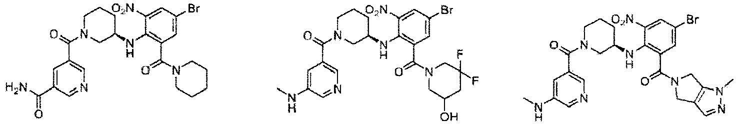

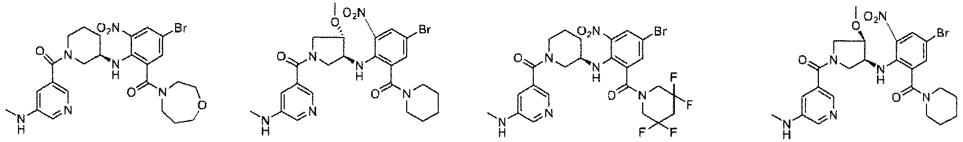

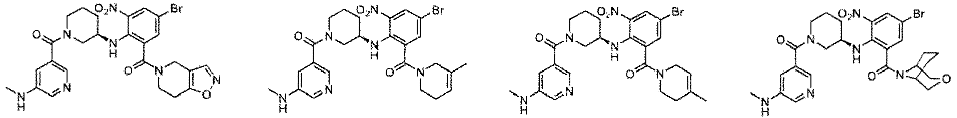

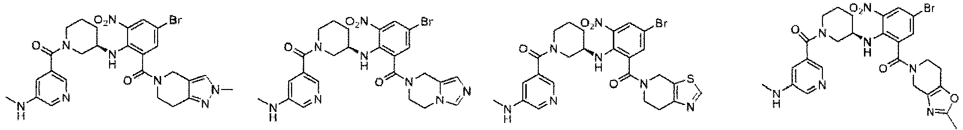

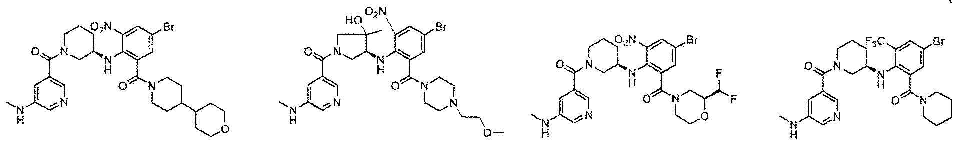

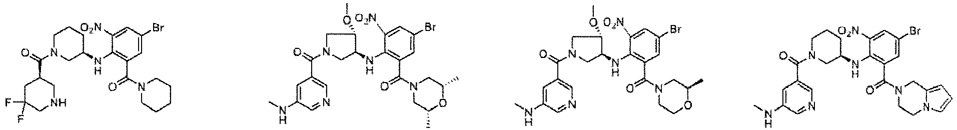

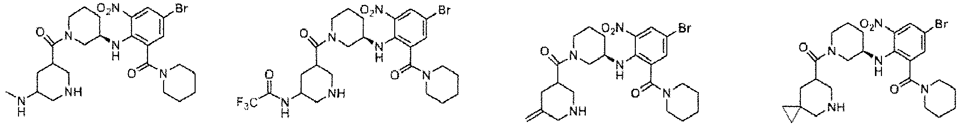

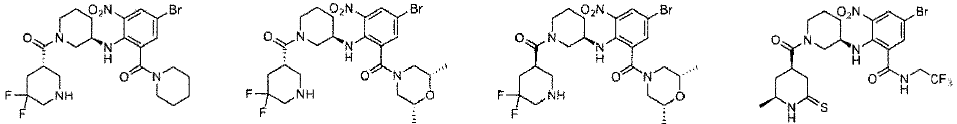

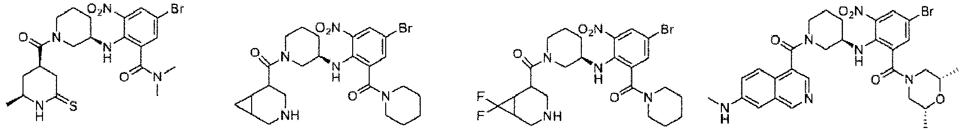

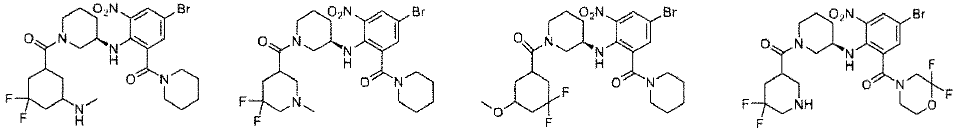

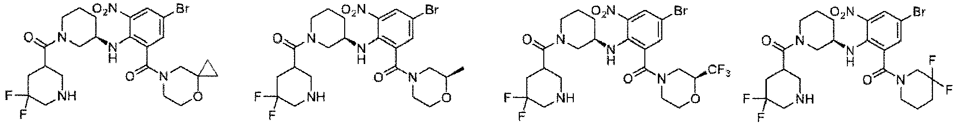

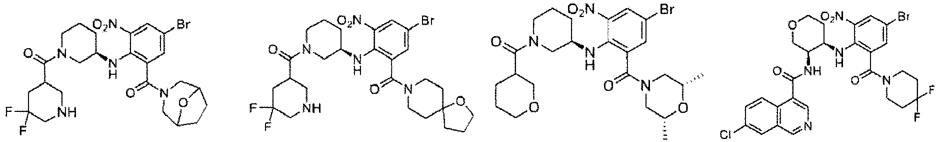

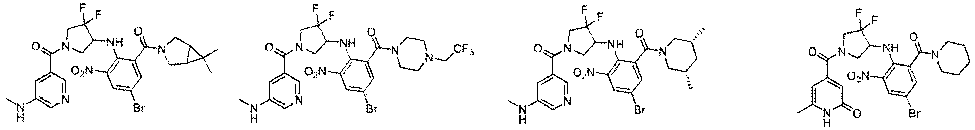

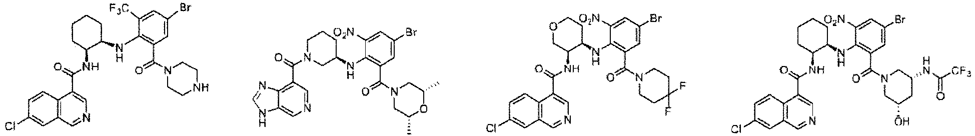

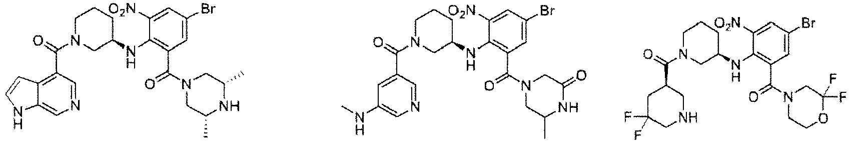

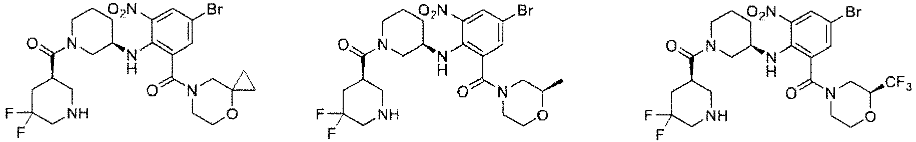

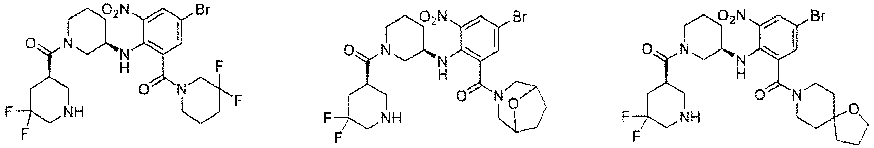

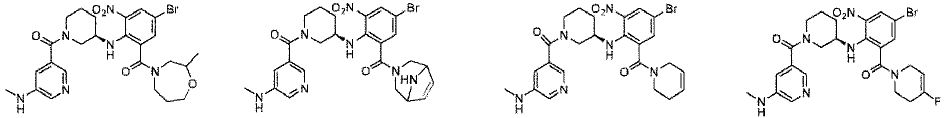

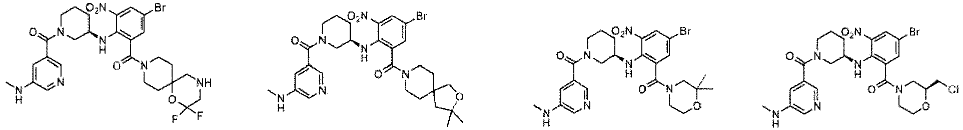

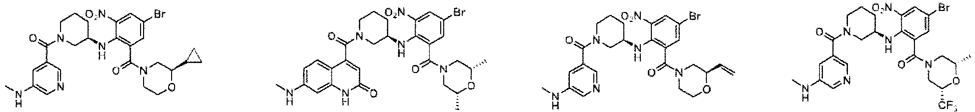

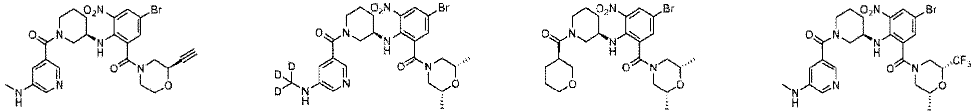

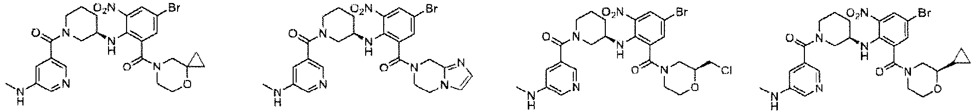

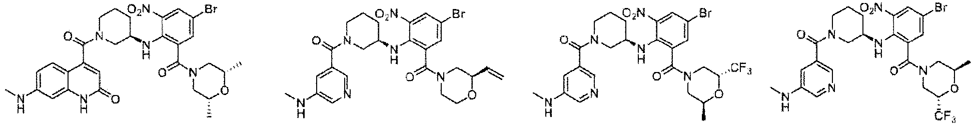

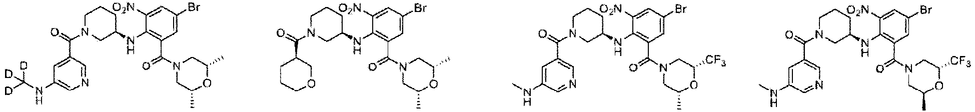

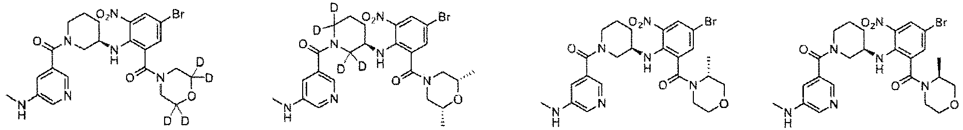

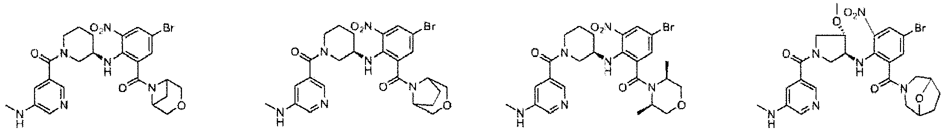

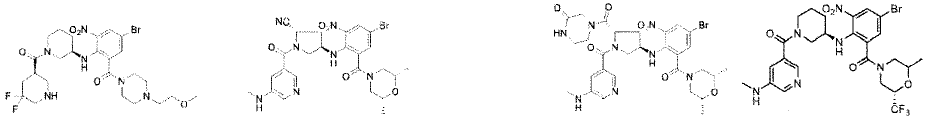

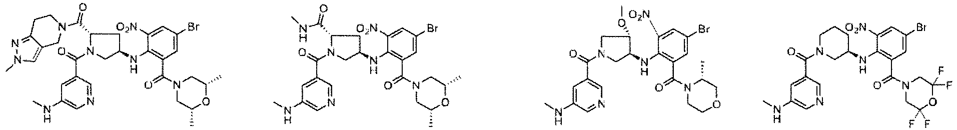

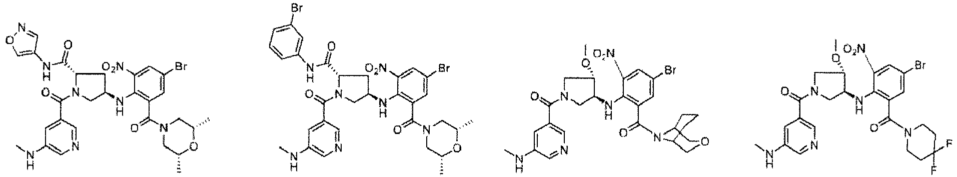

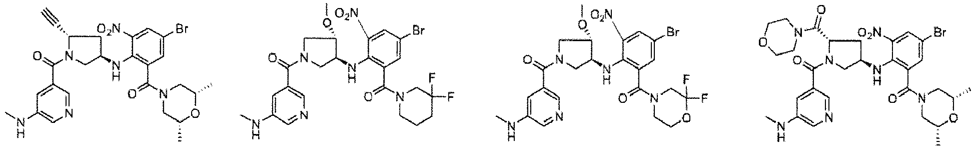

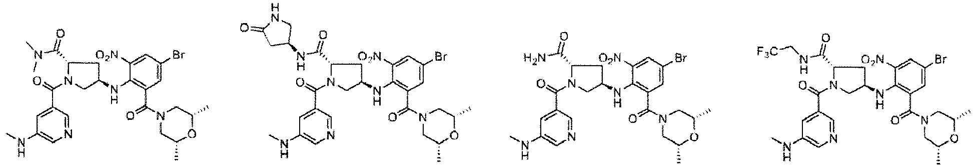

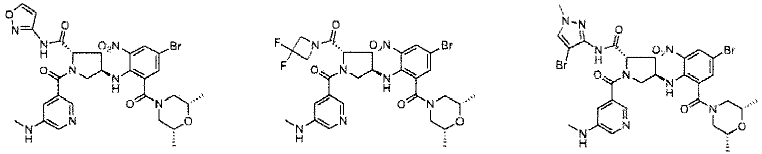

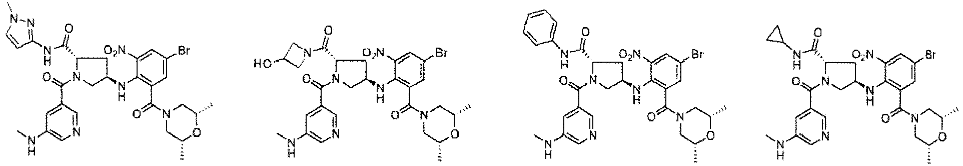

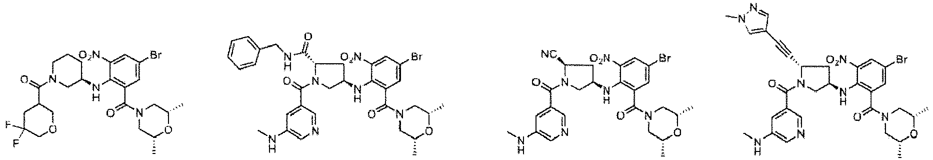

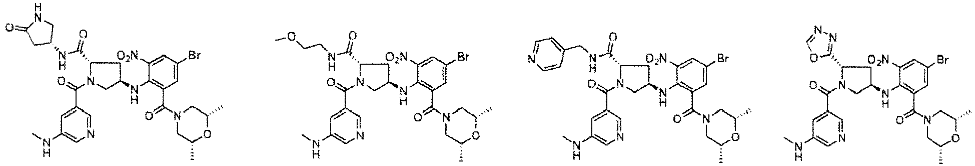

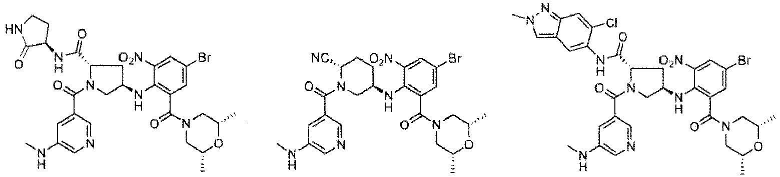

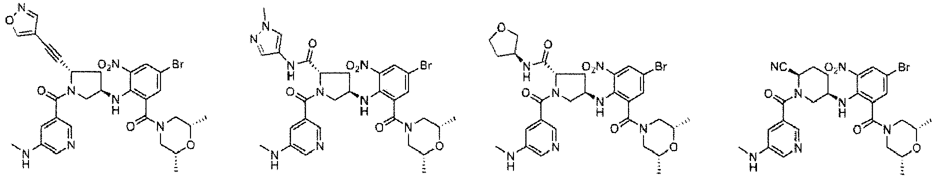

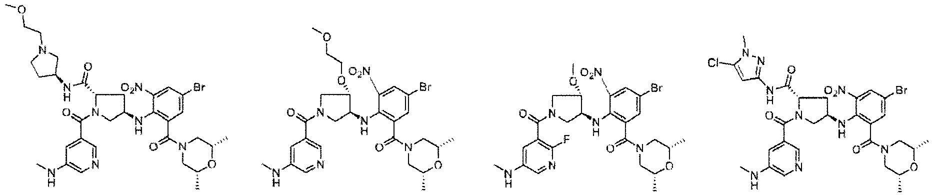

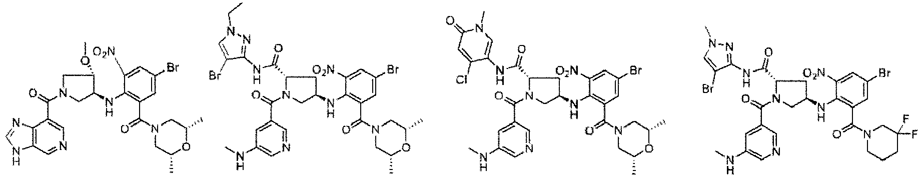

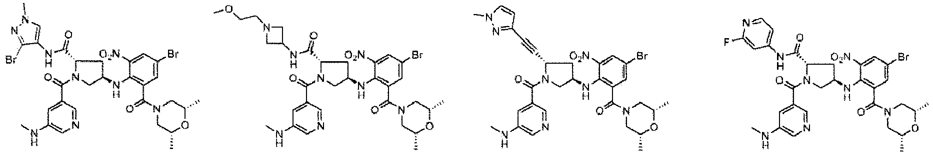

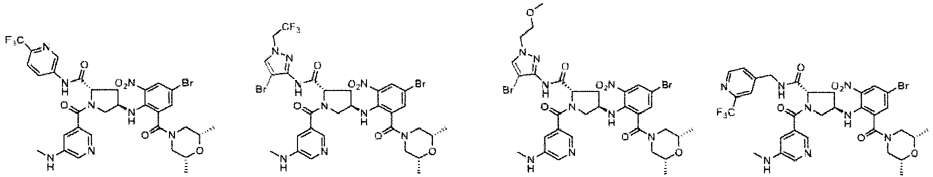

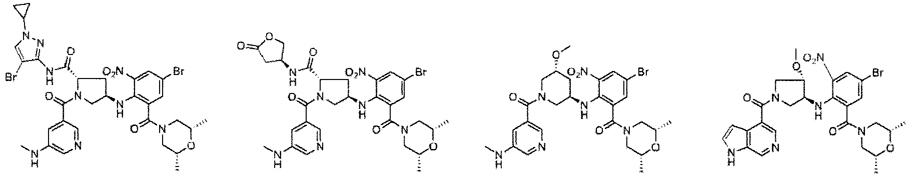

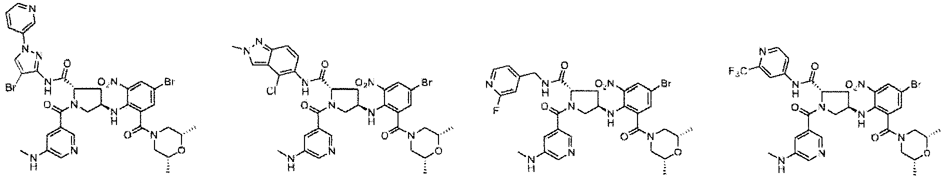

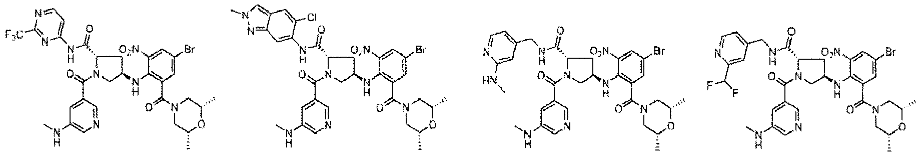

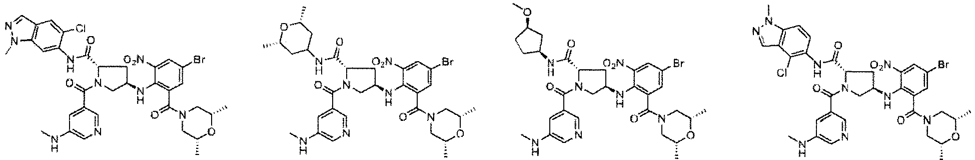

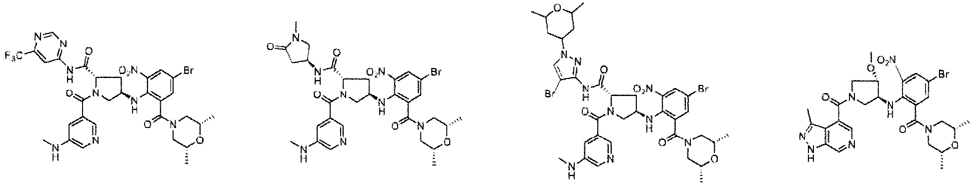

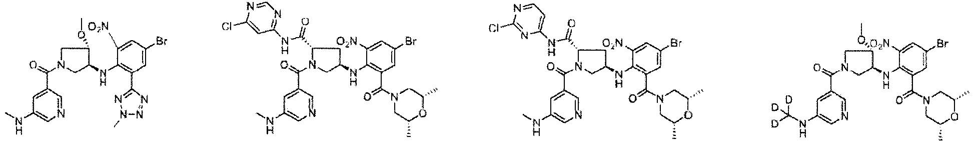

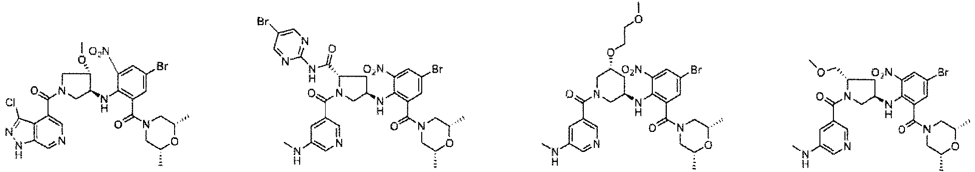

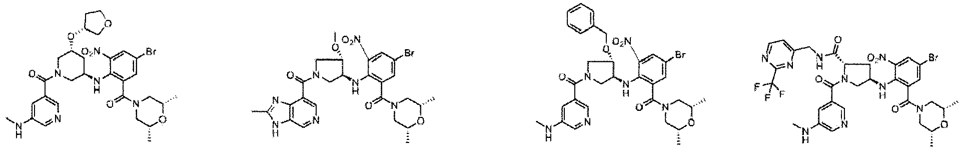

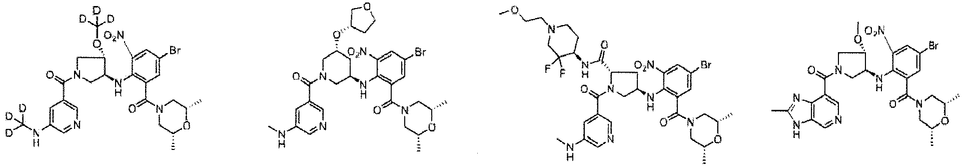

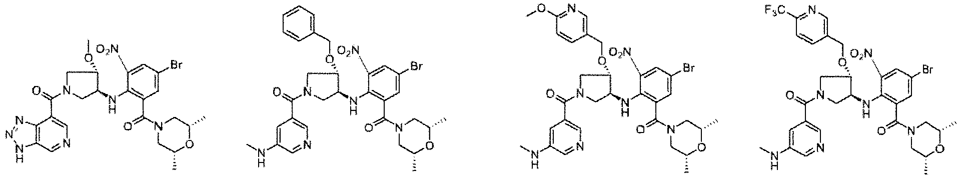

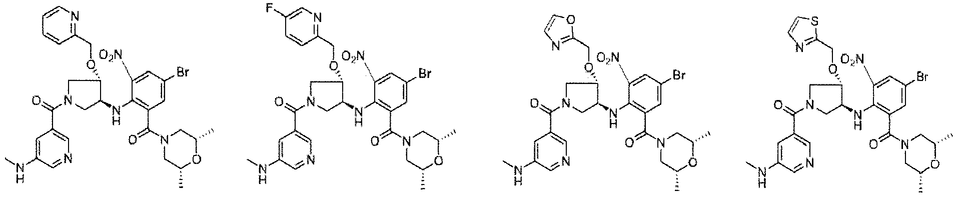

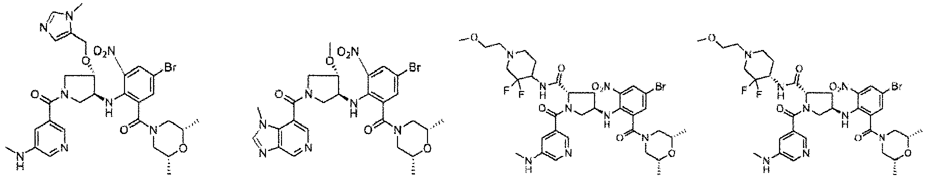

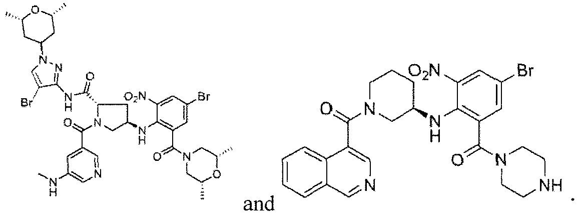

The present application further provides a preparation method for the compound of formula (I): preparing the compound of formula (I) by condensation reaction between compound of formula (I-A) and compound of formula (I-B); 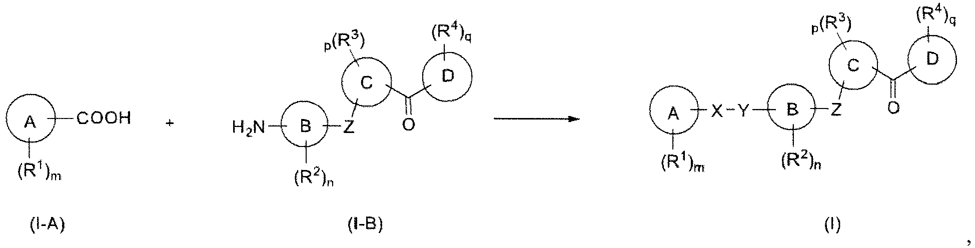 wherein, ring A, ring B, ring C, ring D, X, Y, Z, R¹, R², R³, R⁴, m, n, p, and q are as defined in formula (I).

The present application further relates to a pharmaceutical composition comprising the compound of formula (I), or the isomer or the pharmaceutically acceptable salt thereof, and a pharmaceutically acceptable carrier, diluent or excipient.

The present application further relates to use of the compound, or the isomer or the pharmaceutically acceptable salt thereof, and a pharmaceutically acceptable carrier, diluent or excipient or the pharmaceutical composition in the manufacture of a medicament for treating a disease caused by a coronavirus.

Preferably, the disease is a respiratory infectious disease. Preferably, the respiratory infectious disease is severe acute respiratory syndrome. Preferably, the coronavirus is SARS-CoV-2.

The present application further provides use of the compound of formula (I), or the isomer or the pharmaceutically acceptable salt thereof in the treatment of a disease caused by coronavirus. Preferably, the disease is a respiratory infectious disease. Preferably, the respiratory infectious disease is severe acute respiratory syndrome. Preferably, the coronavirus is SARS-CoV-2.

The present application further provides use of the compound of formula (I), or the isomer or the pharmaceutically acceptable salt thereof, in the manufacture of a 3CLpro protease inhibitor.

The present application further provides a method for treating a disease caused by a coronavirus comprising administering an effective amount of the compound of formula (I), or the isomer or the pharmaceutically acceptable salt thereof. Preferably, the disease is a respiratory infectious disease. Preferably, the respiratory infectious disease is severe acute respiratory syndrome. Preferably, the coronavirus is SARS-CoV-2.

The beneficial effects of the compound of the present application include, but are not limited to, good inhibitory effect on SARS-CoV-2 3CLpro/Mpro protease, improved pharmacokinetic properties, and improved druggability.

### Definition of Terms

Unless otherwise stated, the following terms and phrases used herein are intended to have the following meanings. A particular term or phrase should not be considered uncertain or unclear without a specific definition, but should be understood in its ordinary meaning.

The term "pharmaceutically acceptable" refers to those compounds, materials, compositions and/or dosage forms which, within a reasonable medical judgment, are suitable for use in contact with human and animal tissues without excessive toxicity, irritation, anaphylaxis or other problems or complications and with a reasonable benefit/risk ratio.

The term "pharmaceutically acceptable salt" refers to a derivative prepared from the compound of the present application and a relatively non-toxic acid or base. These salts can be prepared during compound synthesis, separation, or purification, or alone by reacting the purified compound in free form with a suitable acid or base. When a compound contains a relatively acidic functional group, it reacts with an alkali metal hydroxide, an alkaline earth metal hydroxide, or an organic amine to obtain an alkali addition salt, including a cation based on the alkali metal and alkaline earth metal. When a compound contains a relatively basic functional group, it reacts with an organic or inorganic acid to obtain an acid addition salt.

The isomer of the present application includes a geometric isomer and a stereoisomer, such as a cis-trans isomer, an enantiomer, a diastereomer, and a racemic mixture and other mixtures thereof, all of which fall within the scope of the present application.

The term "enantiomer" refers to either of a pair of stereoisomers that are mirror images of each other.

The term "diastereomer" refers to either of stereoisomers of a molecule having two or more chiral centers, and the stereoisomers are not mirror images of each other.

The term "cis-trans isomer" refers to a configuration of a molecule in which a double bond or a single bond of ring carbon atoms cannot rotate freely.

In the present application, 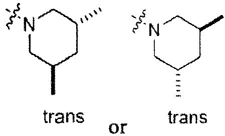 represents a trans isomer, wherein two methyl substituents are on either side of the ring, such as

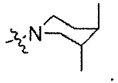

In the present application, 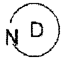 represents that ring D contains N atom.

In the present application, two R¹ linked to the same C atom on ring A can together form =CH₂, =S, e.g.,

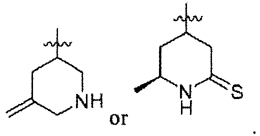

Unless otherwise indicated, the absolute configuration of a stereo-center is indicated by a bond of wedge-shaped solid line 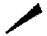 and a bond of wedge-shaped dashed line

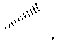

The stereoisomers of the compound of the present application can be prepared by chiral synthesis or from chiral reagents or by other conventional techniques. For example, an enantiomer of the compound of the present application can be prepared by asymmetric catalytic technology or chiral additive derivative technology. Or through a chiral resolution technology, a single spatial configuration of the compound can be obtained from a mixture. Or it can be prepared directly from a chiral starting material. The separation of an optically pure compound in the present application is usually accomplished by preparative chromatography, and a chiral column is adopted to separate the chiral compound.

The present application further includes an isotopically labeled compound comprising isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. Compounds of the present application containing the above isotopes and/or other isotopes of other atoms are all within the scope of the present application. Preferably, the isotope is selected from the group consisting of: ²H, ³H, ¹¹C, and ¹⁸F. More preferably, a radioisotope is ²H. Specifically, a deuterium-substituted compound is intended to be included within the scope of the present application.

Unless otherwise specified, the term "chemical bond" is a mode of bonding between particles in which the particles can be atoms or ions. For example, the structural unit 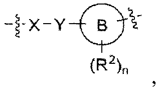 when Y is a chemical bond, represents that X is directly linked to ring B, i.e.

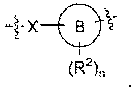

When the number of a linking group is 0, such as -(CH₂)ₛ-C(=O)- with s=0, it means that the linking group is a chemical bond, i.e. -C(=O)-.

When a bond of a substituent can be cross-linked to a ring, the substituent can be bonded to any atom on the ring. For example, the structural unit 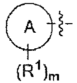 represents that the substituent R¹ can occur at any position on ring A.

In the present application, for the structural unit 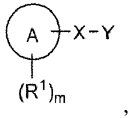 wherein the substituents X and Y are linked from left to right, for example, when X is -(CH2)ₛ-C(=O)-, the structural unit 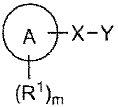 is 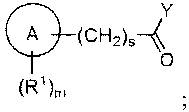 and when X is -NH-C(=O)-, the structural unit 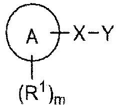 is

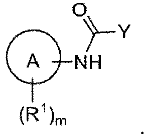

In the present application, when ring D is absent, the structural unit 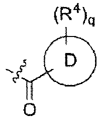 represents that -C(=O) is directly linked to R⁴, i.e. 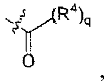 and q is 1.

Preferably, when ring A is 9 - or 10-membered heteroaryl and ring D is absent, R⁴ is not methylamino (-NHCH₃).

In the present application, when an optional substituent in R¹ is O, it indicates that its linked atom is oxidized, for example, when ring A contains an N atom, a nitrogen oxide can be formed, such as

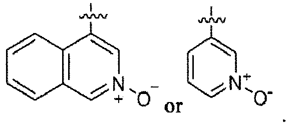

The term "pharmaceutically acceptable carrier" means a medium generally acceptable in the art for the delivery of a biologically active pharmaceutical agent to an animal, particularly a mammal. Depending on the method of administration and the nature of the dosage form, the pharmaceutically acceptable carrier includes an adjuvant, an excipient or a vehicle, such as a diluent, a preservative, a filler, a flow regulator, a disintegrating agent, a wetting agent, an emulsifier, a suspending agent, a sweetener, a flavoring agent, a fragrant agent, an antimicrobial, an antifungal, a lubricant, and a dispersant. The pharmaceutically acceptable carrier is formulated depending on a large number of factors within the knowledge of those skilled in the art. The factors include, but are not limited to: the type and nature of the active agent formulated, the subject to which the composition containing the agent is to be administered, the intended route of administration of the composition, and the target therapeutic indication. The pharmaceutically acceptable carrier includes both aqueous and non-aqueous medium and a variety of solid and semi-solid dosage forms. Such a carrier includes, in addition to an active agent, many different ingredients and additives, and additional ingredients included in the prescription for a variety of reasons (e.g., for stabilizing the active agent, or as an adhesive, etc.) are well known to those skilled in the art. The term "excipient" generally refers to a carrier, diluent, and/or medium required to formulate an effective pharmaceutical composition.

The term "prophylactically or therapeutically effective amount" means an adequate amount of the compound of the present application or the pharmaceutically acceptable salt thereof which is suitable for the treatment of a disorder with a reasonable effect/risk ratio of any medical treatment and/or prevention. However, it should be understood that the total daily dosage of the compound represented by formula (I) of the present application or the pharmaceutically acceptable salt and the composition thereof shall be determined by an attending physician within the scope of reliable medical judgment. For any particular patient, the specific therapeutically effective dosage level must be determined based on multiple factors, including the disorder being treated and the severity of the disorder; the activity of the specific compound used; the specific composition used; age, weight, general health status, gender, and diet of a patient; the specific administration time, administration route, and excretion rate of the compound used; duration of treatment; drugs used in combination or simultaneously with specific compounds used; and similar factors commonly known in the medical field. For example, the practice in the field is to start with a dose of a compound below the level required to achieve the desired therapeutic effect and gradually increase the dose until the desired effect is achieved.

The term "optionally substituted" means that it may or may not be substituted and, unless otherwise specified, the types and numbers of substituents may be optional on the basis of what is chemically achievable, for example, the term "optionally substituted by one or more halogens" means that it may or may not be substituted by one or more halogens.

Unless otherwise specified, "ring" refers to a saturated, partially saturated or unsaturated monocyclic ring and polycyclic ring.

Unless otherwise specified, the term "heterocyclyl" refers to a substituted or unsubstituted saturated or unsaturated non-aromatic ring containing 1 to 3 heteroatoms selected from the group consisting of N, O, and S. The term "heterocyclyl" includes saturated or partially saturated monocyclic heterocyclyl and polycyclic heterocyclyl. The heterocyclyl is independent of the linking position (i.e., can be bonded via a carbon atom or a heteroatom). The polycyclic heterocyclyl includes fused heterocyclyl , spiroheterocyclyl and bridged heterocyclyl.

The heterocyclyl may be substituted by oxo, e.g.,

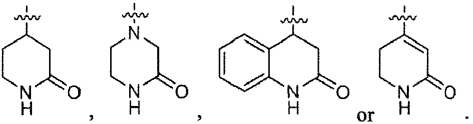

The "fused heterocyclyl" refers to a ring structure formed by two or more rings sharing two adjacent ring atoms with each other, and at least one of the rings is a heterocyclic ring. The fused heterocyclyl includes a ring structure fused by monocyclic heterocyclyl with monocyclic heterocyclyl or cycloalkyl or aryl or heteroaryl, and further includes a ring structure fused by heteroaryl with cycloalkyl or monocyclic heterocyclyl.

The "spiroheterocyclyl" refers to a ring structure formed by two or more rings sharing one ring atom with each other, and at least one ring is a heterocyclic ring.

The "bridged heterocyclyl" refers to a ring structure formed by two or more rings sharing non-adjacent ring atoms with each other, and at least one ring is a heterocyclic ring.

Heterocyclyl can be selected from the group consisting of 4- to 7-member monocyclic heterocyclyl, 6- to 10-membered fused heterocyclyl, 8- to 11-membered spiroheterocyclyl or 7- to 9-membered bridged heterocyclyl.

Examples of "heterocyclyl" include, but are not limited to,

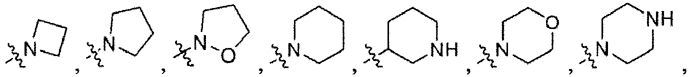

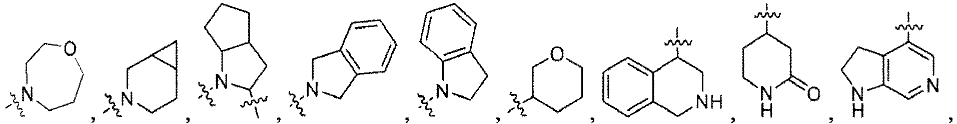

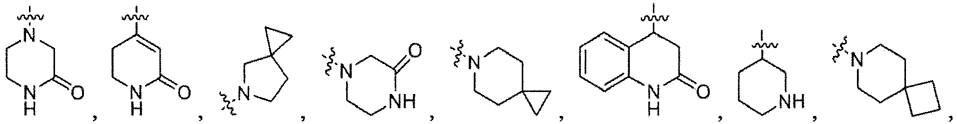

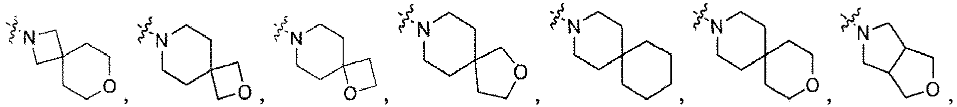

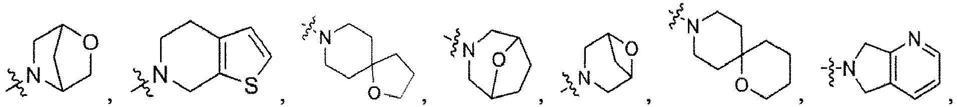

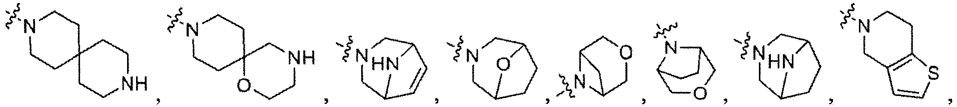

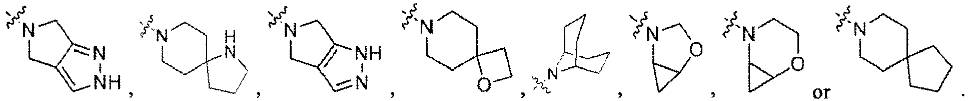

Unless otherwise specified, the term "aryl" refers to unsaturated, usually aromatic hydrocarbyl, which may be a monocyclic ring or a plurality of rings fused together. Examples of C₆₋₁₀ aryl include, but are not limited to, phenyl and naphthyl.

Unless otherwise specified, the term "heteroaryl" refers to stable monocyclic or polycyclic aromatic hydrocarbon, preferably containing carbon atoms and 1, 2, 3 or 4 ring heteroatoms independently selected from the group consisting of N, O and S. The heteroaryl may be substituted by oxo, e.g., 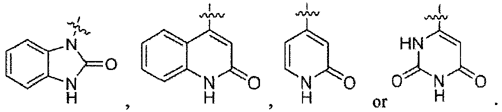 The heteroaryl is preferably 5- to 12-membered heteroaryl, more preferably 5- to 10-membered heteroaryl. Examples of heteroaryl include, but are not limited to, pyrrolyl, pyrazolyl, imidazolyl, pyrazinyl, 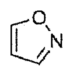 (isoxazolyl), thiazolyl, furanyl, thienyl, pyrimidinyl, pyridinyl, isoquinolinyl, 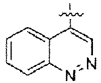 (cinnolinyl), 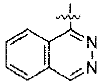 (phthalazinyl),

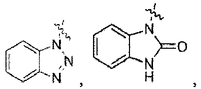

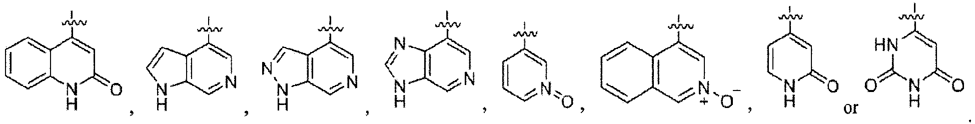

Unless otherwise specified, "cycloalkyl" refers to saturated monocyclic or polycyclic hydrocarbyl. Cycloalkyl is preferably C₃₋₁₂ cycloalkyl, more preferably C₃₋₈ cycloalkyl, and further preferably C₃₋₆ cycloalky. Examples of cycloalkyl include, but are not limited to, cyclopropyl, cyclobutyl, cyclopentyl, cyclohexyl, cycloheptyl, cyclooctyl and 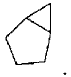 Cycloalkyl can be further substituted by oxo, for example

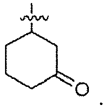

Unless otherwise specified, the term "alkyl" is used to denote straight or branched saturated hydrocarbyl. Alkyl is preferably C₁₋₆ alkyl, more preferably C₁₋₄ alkyl. Examples of alkyl include, but are not limited to, methyl, ethyl, n-propyl, isopropyl, n-butyl, isobutyl, tert-butyl, n-pentyl, isopentyl, neopentyl, n-hexyl, etc.

Unless otherwise specified, the term "alkoxy" is used to denote -O-alkyl. Alkoxy is preferably C₁₋₆ alkoxy, more preferably C₁₋₄ alkoxy. Examples of alkoxy include, but are not limited to, methoxy, ethoxy, n-propoxy, isopropoxy, n-butoxy, sec-butoxy, tert-butoxy, n-pentyloxy, n-hexyloxy, etc.

Unless otherwise specified, the term "halogen" refers to a fluorine, chlorine, bromine or iodine atom.

Unless otherwise specified, the term "haloalkyl" refers to alkyl substituted by one or more halogen atoms. Haloalkyl is preferably halo-C₁₋₄ alkyl. Examples of halo-C₁₋₄alkyl include, but are not limited to, CF₃, CH₂Cl, CH₂F, etc.

In the present application, Severe Acute Respiratory Syndrome, abbreviated as SARS, is a kind of atypical pneumonia. Severe Acute Respiratory Syndrome associated Coronavirus 2 (abbreviated as SARS-CoV-2) is a subtype of severe acute respiratory syndrome associated coronaviruses species in the Coronaviridae family, Betacoronavirus genus. Its gene sequence belongs to the same genealogy as SARS viruses and MERS viruses but a different clade. It is the seventh known coronavirus that can infect humans. The hosts of the virus include mammals and poultry, and it caused the outbreak of coronavisus disease 2019 (COVID-19) at the end of 2019. The virus can invade the human body through the upper respiratory tract and infect with ACE2 as receptors expressed on various cell surfaces. The main infected organs include multiple major organs such as the lungs, heart, and kidneys.

It should be noted that all combinations of substituents and/or their variants herein are only allowed if such combinations produce stable compounds.

### DETAILED DESCRIPTION

The following provides a detailed description of the present application through examples, but does not imply any adverse limitations to the present application. The present application is herein described in detail, and specific embodiments thereof are also disclosed. For those skilled in the art, it will be apparent to make various changes and improvements to the specific embodiments of the present application without departing from the spirit and scope of the present application.

### Summary of experimental instruments:

The compound structure of the present application is determined by nuclear magnetic resonance (NMR) or/and liquid chromatography-mass spectrometry (LC-MS), or ultra high performance liquid chromatography-mass spectrometry (UPLC-MS). NMR chemical shift (δ) is given in parts per million (ppm). NMR was performed with Bruker Neo 400M or Bruker Ascend 400 nuclear magnetic instrument, and with solvents including deuterated dimethyl sulfoxide (DMSO-*d₆*), deuterated methanol (CD₃OD) and deuterated chloroform (CDCl₃), heavy water (D₂O), and the internal standard was tetramethylsilane (TMS).

Liquid chromatography-mass spectrometry, LC-MS, was performed with Agilent 1260-6125B single quadrupole mass spectrometer with a Welch Biomate column (C18, 2.7 µm. 4.6 × 50 mm) or Waters H-Class SQD2 mass spectrometer with Welch Ultimate column (XB-C18, 1.8 µm. 2.1 × 50 mm) (the ion source was electrospray ionization).

Ultra performance liquid chromatography-mass spectrometry (UPLC-MS) was performed with Water UPLC H-class SQD mass spectrometer (the ion source was electrospray ionization).

HPLC was performed with Waters e2695-2998 or Waters ARC and Agilent 1260 or Agilent Poroshell HPH high-performance liquid chromatography.

Thin layer chromatography silica gel plate was performed with GF254 silica gel plate from Yantai Jiangyou Silica gel Development Co., Ltd. or GF254 silica gel plate from Rushan Sanpont New Materials Co., Ltd. The TLC specification was 0.15 mm to 0.20 mm, preparative and 20×20 cm. Generally, 200 to 300 mesh silica gel from Yucheng Chemical was used as the carrier for the column chromatography.

The starting materials in Examples of the present application are known and commercially available or can be synthesized by or according to a method known in the art.

Unless otherwise specified, all reactions of the present application were carried out under continuous magnetic stirring in a dry nitrogen or argon atmosphere, with the solvent being a dry solvent and the reaction temperature in degrees Celsius or °C. Unless otherwise specified, room temperature refers to 25°C ± 5°C.

M in 4 M hydrochloric acid-1,4-dioxane solution is the concentration unit, which represents mol/L.

### Example 1

### N-((1S,2R)-2-((4-bromo-2-(3,3-difluoroazetidin-1-carbonyl)-6-nitrophenyt)amino)cyclohexyl)isoquinolin-4-formamide

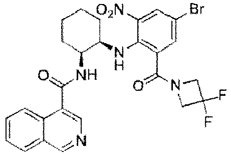

### Reaction route:

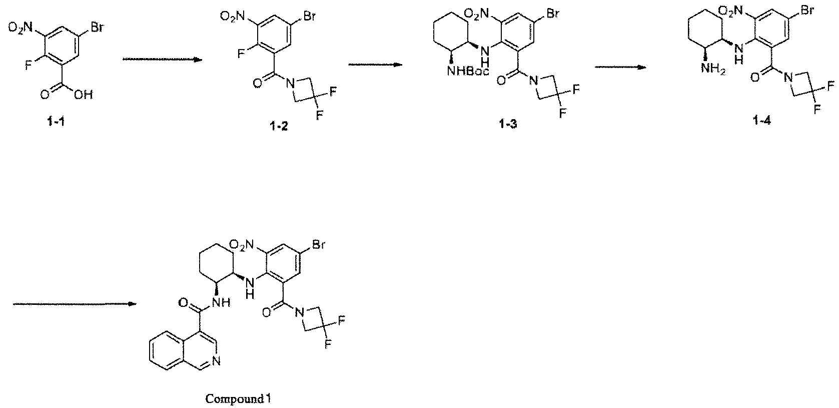

### Operating steps:

Step A: Compound 1-1 (100 mg, 0.38 mmol) was dissolved in *N,N*-dimethylformamide (4 mL) at room temperature under nitrogen protection. Subsequently, the solution was cooled to 0°C, and *N,N*-diisopropylethylamine (147 mg, 1.14 mmol), 2-(7-azabenzotriazol)-*N,N,N',N'*-tetramethyluronium hexafluorophosphate (216 mg, 0.57 mmol) and 3,3-difluoroazetidine hydrochlorid (58.3 mg, 0.46 mmol) were added successively. The reaction system was stirred at 0°C for 15 minutes.

After the depletion of the raw material as monitored by LCMS, the reaction was quenched by adding water (15 mL), and the mixture solution was extracted with ethyl acetate (5 mL × 3 times). The organic phases were combined, and the resultant was washed with aqueous sodium chloride solution (20 mL × 2 times), dried with anhydrous sodium sulfate, filtered, and concentrated under reduced pressure. The resulting residue was purified by silica gel column chromatography to obtain Compound 1-2 (108 mg, 84% yield).

MS (ESI) M/Z: 339.0 [M+H]⁺.

Step B: Compound 1-2 (108 mg, 0.32 mmol) was dissolved in *N,N*-dimethylformamide (3 mL) at room temperature under nitrogen protection. Subsequently, tert-butyl ((*1S*,*2R*)-2-aminocyclohexyl) carbamate (77 mg, 0.36 mmol) and *N,N-*diisopropylethylamine (123.3 mg, 0.96 mmol) were added sequentially to the above solution. The reaction system was stirred at 80°C for 2 hours.

After the depletion of the raw material as monitored by LCMS, the reaction was quenched by adding water (15 mL), and the mixture solution was extracted with ethyl acetate (5 mL × 3 times). The organic phases were combined, and the resultant was washed with aqueous sodium chloride solution (20 mL × 2 times), then dried with anhydrous sodium sulfate, filtered, and concentrated under reduced pressure. The resulting residue was purified by silica gel column chromatography to obtain Compound 1-3 (150 mg, 88.1% yield).

MS (ESI) M/Z: 555.0 [M+Na]⁺.

Step C: Compound 1-3 (150 mg, 0.28 mmol) was dissolved in dichloromethane (2 mL) at room temperature under nitrogen protection. Subsequently, 4 M hydrochloric acid-1,4-dioxane solution (2 mL) was added slowly and dropwise to the reaction system at 0°C. The reaction system was stirred for 1 hour at room temperature.

After the depletion of the raw material as monitored by LCMS, the reaction solution was concentrated by vacuum distillation. Crude Compound 1-4 (140 mg) was obtained.

MS (ESI) M/Z: 433.0 [M+H]⁺.

Step D: Compound 1-4 (140 mg, 0.32 mmol) was dissolved in *N,N-*dimethylformamide (3 mL) at room temperature under nitrogen protection. Subsequently, the above solution was cooled down to 0°C and isoquinoline-4-carboxylic acid (55 mg, 0.32 mmol), 2-(7-azabenzotriazol)-*N,N,N',N'*-tetramethyluronium hexafluorophosphate (182 mg, 0.48 mmol), and *N,N-*diisopropylethylamine (123 mg, 0.96 mmol) were added in sequence. The reaction system was stirred at 0°C for 30 minutes.

After the depletion of the raw material as monitored by LCMS, the reaction was quenched by adding water (15 mL), and the mixture solution was extracted with ethyl acetate (5 mL × 3 times). The organic phases were combined, and the resultant was washed with aqueous sodium chloride solution (20 mL × 2 times), then dried with anhydrous sodium sulfate, filtered and concentrated under reduced pressure. The resulting residue was purified by preparative high performance liquid chromatography to obtain the titled Compound 1 (68.2 mg, 36.3% yield).

MS (ESI) M/Z: 588.0 [M+H]⁺.

¹H NMR (400 MHz, DMSO-*d₆*) δ 9.37 (s, 1H), 8.71 (d, *J* = 8.0 Hz. 1H), 8.34 (s. 1H), 8.27 (d, *J* = 2.4 Hz. 1H), 8.17 (d, *J* = 8.0 Hz. 1H), 8.05 (d, *J* = 10.0 Hz, 1H), 7.94 (d, *J* = 8.4 Hz, 1H), 7.87 (d, *J* = 2.4 Hz. 1H). 7.80 - 7.76 (m. 1H), 7.72 (d, *J* = 7.0 Hz, 1H). 4.73 - 4.43 (m, 4H). 4.33 (brs, 1H). 3.85 (brs, 1H). 1.88 - 1.36 (m. 8H). ¹⁹F NMR (376 MHz. DMSO-*d₆*) δ -100.04 (s).

### Example 2

### N-((1S,2R)-2-((4-bromo-2-nitro-6-(piperidin-1-carbonyl)phenyl)amino)cyclohexyl)isoquinotin-4-formamide

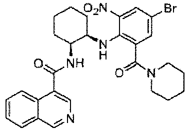

### Reaction route:

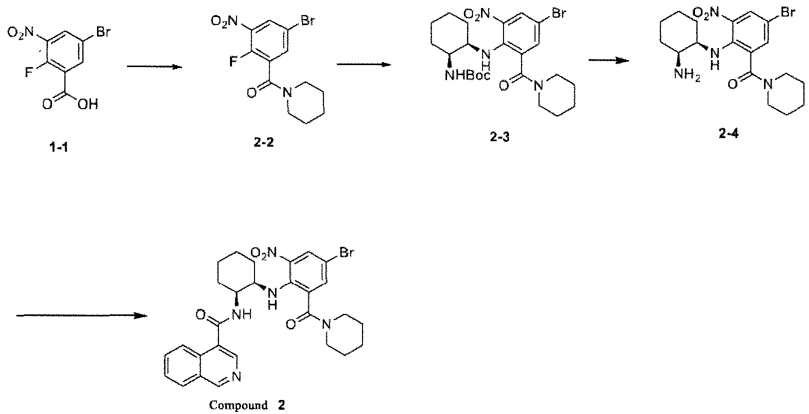

### Operating steps:

Step A: Compound 1-1 (200 mg, 0.76 mmol) was dissolved in *N,N*-dimethylformamide (5 mL) at room temperature under nitrogen protection. Subsequently, the above solution was cooled down to -10°C and *N,N*-diisopropylethylamine (292 mg, 2.3 mmol), 2-(7-azabenzotriazol)-*N,N,N',N'*-tetramethyluronium hexafluorophosphate (381 mg, 1.06 mmol), and piperidine (77 mg, 0.91 mmol) were added in sequence. The reaction system was stirred for 3 min.

After the depletion of the raw material as monitored by TLC, the reaction was quenched by adding water (15 mL) to the reaction solution, and the mixture solution was extracted with ethyl acetate (5 mL × 3 times). The organic phases were combined, and the resultant was washed with aqueous sodium chloride solution (20 mL × 2 times), then dried with anhydrous sodium sulfate, filtered, and concentrated under reduced pressure. The resulting residue was purified by silica gel column chromatography to obtain Compound 2-2 (100 mg, 40% yield).

MS (ESI) M/Z: 331.2 [M+H]⁺.

Step B: Compound 2-2 (100 mg, 0.30 mmol) was dissolved in *N,N*-dimethylformamide (5 mL) at room temperature under nitrogen protection. Subsequently, tert-butyl ((*1S*,*2R*)-2-aminocyclohexyl) carbamate (77 mg, 0.36 mmol) and *N,N-*diisopropylethylamine (96 mg, 0.75 mmol) were sequentially added to the above reaction solution. The reaction system was stirred at 80°C for 2 hours.

After the depletion of the raw material as monitored by LCMS, the reaction was quenched by adding water (15 mL), and the mixture solution was extracted with ethyl acetate (5 mL × 3 times). The organic phases were combined, and the resultant was washed with aqueous sodium chloride solution (20 mL × 2 times), then dried with anhydrous sodium sulfate, filtered, and concentrated under reduced pressure. The resulting residue was purified by silica gel column chromatography to obtain Compound 2-3 (120 mg, 75.5% yield).

MS (ESI) M/Z: 547.4 [M+Na]⁺.

Step C: Compound 2-3 (120 mg, 0.23 mmol) was dissolved in 4 M hydrochloric acid-1,4-dioxane solution (5 mL) at room temperature under nitrogen protection. The reaction system was stirred at room temperature for 1 hour.

After the depletion of the raw material as monitored by LCMS, the reaction solution was concentrated by vacuum distillation. Crude compound 2-4 (100 mg) was obtained.

MS (ESI) M/Z: 425.0 [M+H]⁺.

Step D: Compounds 2-4 (100 mg, 0.24 mmol) were dissolved in *N,N*-dimethylformamide (3 mL) at room temperature under nitrogen protection. Subsequently, the above solution was cooled down to 0°C and isoquinoline-4-carboxylic acid (44 mg, 0.26 mmol), 2-(7-azabenzotriazol)-*N,N,N',N'*-tetramethyluronium hexafluorophosphate (133 mg, 0.35 mmol), and *N,N-*diisopropylethylamine (90 mg, 0.71 mmol) were added in sequence. The reaction system was stirred at 0°C for 30 min.

After the depletion of the raw material as monitored by LCMS, the reaction was quenched by adding water (15 mL), and the mixture solution was extracted with ethyl acetate (5 mL × 3 times). The organic phases were combined, and the resultant was washed with aqueous sodium chloride solution (20 mL × 2 times), then dried with anhydrous sodium sulfate, filtered and concentrated under reduced pressure. The resulting residue was purified by preparative high performance liquid chromatography to obtain the titled Compound 2 (50 mg, 36.8% yield).

MS (ESI) M/Z: 580.0 [M+H]⁺.
¹H NMR (400 MHz, DMSO-*d*₆) δ 9.39 (s, 1H). 8.81 (d. *J* = 4.4 Hz, 0.5H), 8.59 (d. *J* = 5.6 Hz, 0.5H), 8.52 (s, 1H), 8.25 - 8.02 (m. 4H). 7.81 - 7.73 (m. 2H), 7.61 (d, *J* = 2.0 Hz, 1H), 4.45 (brs, 1H), 3.86 - 3.62 (m, 3H), 3.46-3.38 (m, 1H), 3.27 - 3.11 (m, 1H), 1.99 - 127 (m, 14H).

### Example 3

### N-((1S,2R)-2-((4-bromo-2-nitro-6-(pyrrolidin-1-carbonyl)phenyl)amino)cyclohexyl)isoquinolin-4-formamide

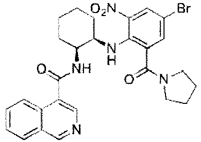

### Reaction route:

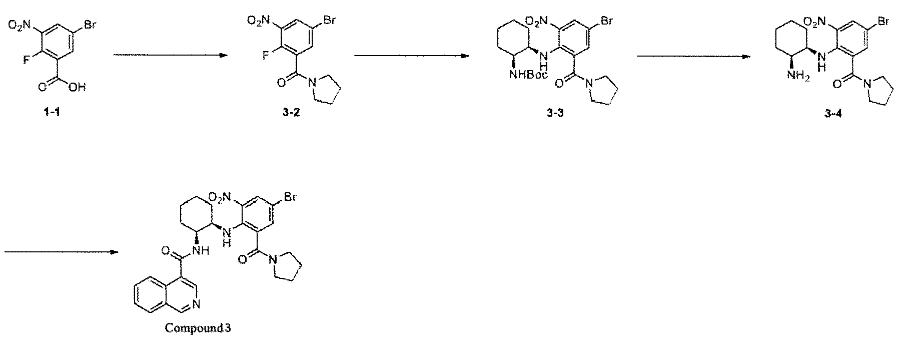

### Operating steps:

Step A: Compound 1-1 (200 mg, 0.76 mmol) was dissolved in *N,N*-dimethylformamide (3.8 mL) at room temperature under nitrogen protection. Subsequently, the above solution was cooled down to -10°C and *N,N*-diisopropylethylamine (292 mg, 2.3 mmol), 2-(7-azabenzotriazol)-*N,N,N',N'*-tetramethyluronium hexafluorophosphate (404 mg, 1.06 mmol), and pyrrolidine (65 mg, 0.91 mmol) were added sequentially. Stirring of the reaction system was kept for 30 minutes.

After the depletion of the raw material as monitored by TLC, the reaction was quenched by adding water (15 mL), and the mixture solution was extracted with ethyl acetate (5 mL × 3 times). The organic phases were combined, and the resultant was washed with saturated saline (20 mL × 2 times), then dried with anhydrous sodium sulfate, filtered, and concentrated under reduced pressure. The resulting residue was purified by silica gel column chromatography to obtain Compound 3-2 (80 mg, 33% yield).

MS (ESI) M/Z: 317.0 [M+H]⁺.

Step B: Compound 3-2 (80 mg, 0.25 mmol) was dissolved in *N,N*-dimethylformamide (1.3 mL) at room temperature under nitrogen protection. Subsequently, tert-butyl ((*1S*,*2R*)-2-aminocyclohexyl) carbamate (64 mg, 0.3 mmol) and *N,N-*diisopropylethylamine (97 mg, 0.75 mmol) were sequentially added to the mixture solution. The reaction system was stirred at 80°C for 2 hours.

After the depletion of the raw material as monitored by LCMS, the above reaction was quenched by adding water (15 mL), and the mixture solution was extracted with ethyl acetate (5 mL × 3 times). The organic phases were combined, and the resultant was washed with saturated saline (20 mL × 2 times), then dried with anhydrous sodium sulfate, filtered, and concentrated under reduced pressure. The crude compound 3-3 (127 mg) was obtained.

MS (ESI) M/Z: 513.0 [M+H]⁺.

Step C: Compound 3-3 (127 mg, 0.25 mmol) was dissolved in a dry dichloromethane solution (1.3 mL) under nitrogen protection at room temperature. Subsequently, 4 M hydrochloric acid-1,4-dioxane solution (0.63 mL) was added slowly and dropwise to the reaction system at 0°C. The reaction system was brought to room temperature and stirring was kept for 1 hour.

After the depletion of the raw material as monitored by LCMS, the reaction solution was concentrated by vacuum distillation. Compound 3-4 (200 mg, 100% yield) was obtained.

MS (ESI) M/Z: 413.0 [M+H]⁺.

Step D: Compound 3-4 (60 mg, 0.15 mmol) was dissolved in *N,N*-dimethylformamide (0.75 mL) at room temperature under nitrogen protection. Subsequently, the above solution was cooled down to 0°C and isoquinoline-4-carboxylic acid (26 mg, 0.15 mmol), 2-(7-azabenzotriazol)-*N,N,N',N'*-tetramethyluronium hexafluorophosphate (86 mg, 0.23 mmol), and *N,N-*diisopropylethylamine (97 mg, 0.75 mmol) were added in sequence. The reaction system was stirred at 0°C for 30 min.

After the depletion of the raw material as monitored by LCMS, the above reaction was quenched by adding water (35 mL), and the mixture solution was extracted with ethyl acetate (10 mL × 3 times). The organic phases were combined, and the resultant was washed with saturated saline (20 mL × 2 times), then dried with anhydrous sodium sulfate, filtered, and concentrated under reduced pressure. The resulting residue was purified by preparative high performance liquid chromatography to obtain the titled Compound 3 (42.4 mg, 50% yield).

MS (ESI) M/Z: 566.0 [M+H]⁺.
¹H NMR (400 MHz, DMSO-*d*₆) δ 9.38 (s, 1H), 8.75 (d, *J* = 8.4 Hz, 1H). 8.44 (s, 1H), 8.26 (d, *J* = 2.4 Hz, 1H), 8.18 (d, *J* = 7.6 Hz, 1H). 8.1,4 (d, *J* = 10.0 Hz, 1H), 8.02 (d, *J* = 8.4 Hz, 1H), 7.79 (t. *J* = 7.0 Hz, 1H), 7.73 (t, *J* = 7.0 Hz, 1H). 7.69 (d, *J* = 2.4 Hz, 1H), 4.36 (brs, 1H), 3.83 (brs, 1H), 3.48 (t, *J* = 6.8 Hz. 2H), 3.31 - 3.27 (m, 2H). 1.97 - 1.73 (m, 6H), 1.71 - 1.29 (m, 6H).

### Example 4

### (R)-(5-bromo-2-((1-nicotinoylpiperidin-3-yl)amino)-3-nitrophenyl)(piperidin-1-yl)methanone

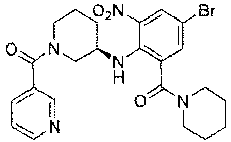

### Reaction route:

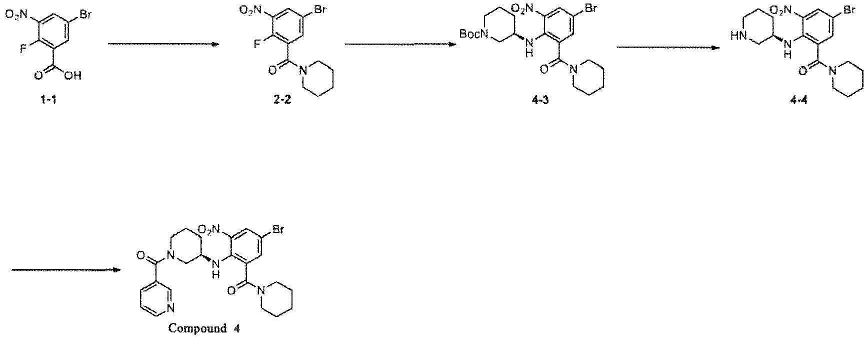

### Operating steps:

Step A: Compound 1-1 (200 mg, 0.76 mmol) was dissolved in *N,N*-dimethylformamide (5 mL) at room temperature under nitrogen protection. Subsequently, the above solution was cooled down to -10°C and *N,N*-diisopropylethylamine (292 mg, 2.3 mmol), 2-(7-azabenzotriazol)-*N,N,N',N'*-tetramethyluronium hexafluorophosphate (381 mg, 1.06 mmol), and piperidine (77 mg, 0.91 mmol) were added in sequence. The reaction system was stirred for 3 min.

After the depletion of the raw material as monitored by TLC, the reaction was quenched by adding water (15 mL), and the mixture solution was extracted with ethyl acetate (5 mL × 3 times). The organic phases were combined, and the resultant was washed with aqueous sodium chloride solution (20 mL × 2 times), then dried with anhydrous sodium sulfate, filtered, and concentrated under reduced pressure. The resulting residue was purified by silica gel column chromatography to obtain Compound 2-2 (100 mg, 40% yield).

MS (ESI) M/Z: 331.2 [M+H]⁺.

Step B: Compound 2-2 (120 mg, 0.60 mmol) was dissolved in *N,N*-dimethylformamide (3 mL) at room temperature under nitrogen protection. Subsequently, tert-butyl (R)-3-aminopiperidin-1-carboxylate (145 mg, 0.72 mmol) and *N,N-*diisopropylethylamine (193 mg, 1.5 mmol) were sequentially added to the above reaction solution. The reaction system was stirred at 80°C for 2 hours.

After the depletion of the raw material as monitored by LCMS, the reaction was quenched by adding water (15 mL), and the mixture solution was extracted with ethyl acetate (5 mL × 3 times). The organic phases were combined, and the resultant was washed with aqueous sodium chloride solution (20 mL × 2 times), then dried with anhydrous sodium sulfate, filtered, and concentrated under reduced pressure. The resulting residue was purified by silica gel column chromatography to obtain Compound 4-3 (160 mg, 80.5% yield).

MS (ESI) M/Z: 411.0 [M-99]⁺.

Step C: Compound 1-2 (160 mg, 0.31 mmol) was dissolved in 4 M hydrochloric acid-1,4-dioxane solution (5 mL) at room temperature under nitrogen protection. The reaction system was stirred at room temperature for 1 hour.

After the depletion of the raw material as monitored by LCMS, the reaction solution was concentrated by vacuum distillation. Compound 4-4 (130 mg, 101.1% yield) was obtained.

MS (ESI) M/Z: 411.0 [M+H]⁺.

Step D: Compound 1-3 (130 mg, 0.31 mmol) was dissolved in *N,N*-dimethylformamide (3 mL) at room temperature under nitrogen protection. Subsequently, the above solution was cooled down to 0°C and nicotinic acid (46 mg, 0.37 mmol), 2-(7-azabenzotriazol)-*N,N,N',N'*-tetramethyluronium hexafluorophosphate (299 mg, 0.78 mmol), and *N,N-*diisopropylethylamine (201 mg, 1.56 mmol) were added in sequence. The reaction system was stirred at 0°C for 30 min.

After the depletion of the raw material as monitored by LCMS, the reaction was quenched by adding water (15 mL), and the mixture solution was extracted with ethyl acetate (5 mL × 3 times). The organic phases were combined, and the resultant was washed with aqueous sodium chloride solution (20 mL × 2 times), then dried with anhydrous sodium sulfate, filtered and concentrated under reduced pressure. The resulting residue was purified by preparative high performance liquid chromatography to obtain the titled Compound 4 (37.5 mg, 23.1% yield).

MS (ESI) M/Z: 516.3 [M+H]⁺.
¹H NMR (400 MHz, DMSO-*d*₆) δ 8.71 - 8.42 (m, 2H), 8.25 (brs, 1H), 7.97 - 7.31 (m, 4H), 3.91 - 3.32 (m, 7H), 3.24 - 3.12 (m, 2H), 2.01 - 1.83 (m, 1H), 1.80 - 1.34 (m, 9H).

### Example 5

### (R)-(5-bromo-2-((1-(5-bromonicotinoyl)piperidin-3-yl)amino)-3-nitrophenyl)(piperidin-1-yl)methanone

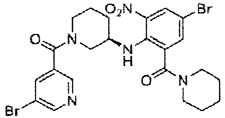

### Reaction route:

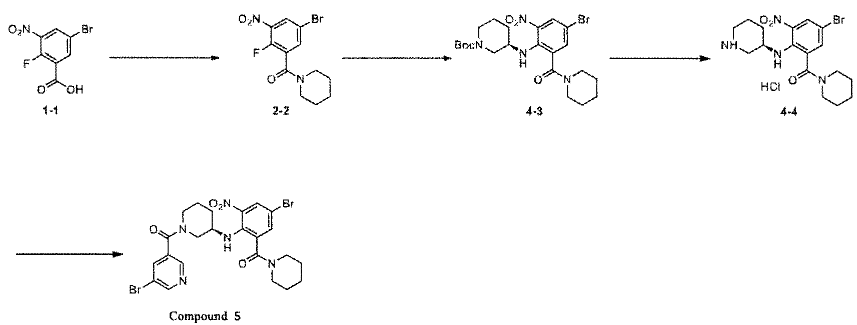

### Operating steps:

**Step A:** Compound 1-1 (2 g, 7.6 mmol) was dissolved in *N,N*-dimethylformamide (38 mL) at room temperature under nitrogen protection. Subsequently, the above solution was cooled down to -10°C and 2-(7-azabenzotriazol)-*N,N,N'*,*N'-*tetramethyluronium hexafluorophosphate (4.05 g, 10.6 mmol), *N,N*-diisopropylethylamine (2.95 g, 23 mmol), and piperidine (777 mg, 9.1 mmol) were added in sequence. The reaction system was stirred for 3 min.

After the depletion of the raw material as monitored by TLC, the reaction was quenched by adding water (120 mL), and the mixture solution was extracted with ethyl acetate (35 mL×3 times). The organic phases were combined, and the resultant was washed with saturated aqueous sodium chloride solution (120 mL×2 times), then dried with anhydrous sodium sulfate, filtered and concentrated under reduced pressure. The resulting residue was purified by silica gel column chromatography (to obtain Compound 2-2 (1.03 g, 41.2% yield)).

MS (ESI) M/Z: 331.0 [M+H]⁺.
¹H NMR (400 MHz, DMSO-*d*₆) δ 8.40 (dd, *J* = 6.4, 2.0 Hz, 1H), 8.10 (dd, *J* = 4.6, 2.2 Hz, 1H), 3.61 (brs, 2H), 3.33 (brs, 2H), 1.76 -1.35 (m. 6H).

**Step B:** Compound 2-2 (1.03 g, 3.1 mmol) was dissolved in *N,N*-dimethylformamide (1.5 mL) at room temperature under nitrogen protection. Subsequently, tert-butyl (R)-3-aminopiperidin-1-carboxylate (744 mg, 3.7 mmol) and *N,N-*diisopropylethylamine (1.2 g, 9.3 mmol) were added sequentially. The reaction system was stirred at 80°C for 2 hours.

After the depletion of the raw material as monitored by LCMS, the reaction was quenched by adding water (30 mL), and the mixture solution was extracted with ethyl acetate (25 mL × 3 times). The organic phases were combined, and the resultant was washed with saturated aqueous sodium chloride solution (60 mL × 2 times), then dried with anhydrous sodium sulfate, filtered, and concentrated under reduced pressure. The crude Compound 4-3 (1.2 g, 76% yield) was obtained.

MS (ESI) M/Z: 411.0 [M-99]⁺.
¹H NMR (400 MHz, DMSO-*d*₆) δ 8.25 (d, *J* = 2.4 Hz, 1H), 7.85 (d, *J* = 9.6 Hz, 1H), 7.60 (d, *J* = 2.4 Hz, 1H), 3.72 - 3.76 (m. 5H), 3.29 - 3.00 (m, 4H), 1.88 - 1.77 (m, 1H), 1.71 - 1.16 (m, 18H),

**Step C:** Compound 4-3 (1.2 g, 2.34 mmol) was dissolved in a dry dichloromethane solution (11.8 mL) at room temperature under nitrogen protection. Subsequently, hydrochloric acid-1,4-dioxane solution (5.9 mL) was added slowly and dropwise to the reaction system. The reaction system was stirred at room temperature for 1 hour.

After the depletion of the raw material as monitored by LCMS, the reaction solution was concentrated by vacuum distillation. The crude Compound 4-4 (1.2 g) was obtained.

MS (ESI) M/Z: 411.0 [M+H]⁺.

**Step D:** Compound 5-bromonicotinic acid (30 mg, 0.15 mmol) was dissolved in *N,N-*dimethylformamide (0.75 mL) at room temperature under nitrogen protection. Subsequently, the above solution was cooled down to 0°C and 2-(7-azabenzotriazol)-*N,N,N',N'*-tetramethyluronium hexafluorophosphate (86 mg, 0.23 mmol), Compound 4-4 (60 mg, 0.15 mmol) and *N,N*-diisopropylethylamine (97 mg, 0.75 mmol) were added sequentially. The reaction system was stirred at 0°C for 30 min.

After the depletion of the raw material as monitored by LCMS, the reaction was quenched by adding water (35 mL), and the mixture solution was extracted with ethyl acetate (15 mL × 3 times). The organic phases were combined, and the resultant was washed with saturated aqueous sodium chloride solution (20 mL × 2 times), then dried with anhydrous sodium sulfate, filtered and concentrated under reduced pressure. The resulting residue was purified by preparative high performance liquid chromatography to obtain the titled Compound 5 (19 mg, 21% yield).

MS (ESI) M/Z: 594.0 [M+H]⁺.
¹H NMR (400 MHz. DMSO-*d*₆) δ 8.86 - 8.71 (m, 1H), 8.65 - 8.54 (m, 1H). 8.42 - 8.25 (m, 1H), 8.15 - 7.35 (m, 3H), 4.02 - 3.39 (m. 7H). 3.25 - 2.95 (m, 2H), 2.01 - 1.29 (m, 10H).

### Example 6

### (R)-(5-bromo-2-((1-(isoquinolin-4-carbonyl)piperidin-3-yl)amino)-3-nitrophenyl)(4,4-difluoropiperidin-1-yl)methanone

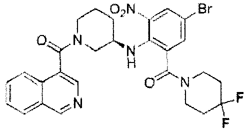

### Reaction route:

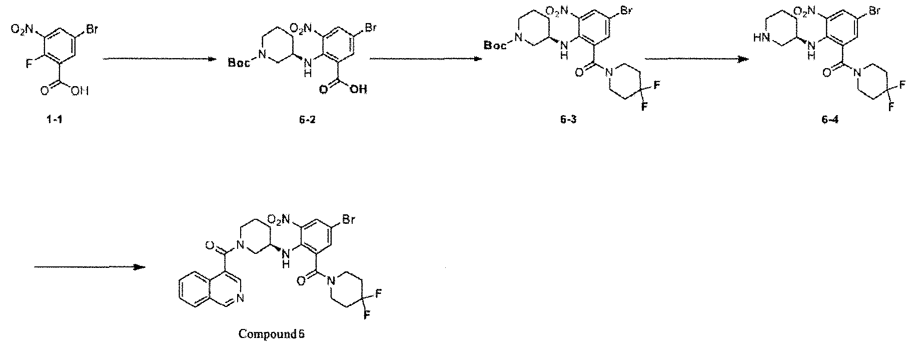

### Operating steps:

Step A: Compound 1-1 (2 g, 7.5 mmol) was dissolved in *N,N*-dimethylformamide (30 mL) at room temperature under nitrogen protection. Subsequently, *N,N*-diisopropylethylamine (2.9 g, 22.5 mmol) and tert-butyl (R)-3-aminopiperidin-1-carboxylate (1.5 g, 7.5 mmol) were added sequentially. The reaction system was stirred overnight.

After the depletion of the raw material as monitored by TLC, the reaction was quenched by adding water (120 mL), and the mixture solution was extracted with ethyl acetate (35 mL × 3 times). The organic phases were combined, and the resultant was washed with saturated saline (120 mL × 2 times), then dried with anhydrous sodium sulfate, filtered, and concentrated under reduced pressure. The crude product Compound 6-2 (3.03 g) was obtained.

MS (ESI) M/Z: 466.0 [M+Na]⁺.

Step B: Compound 6-2 (120 mg, 0.27 mmol) was dissolved in *N,N*-dimethylformamide (2 mL) at room temperature under nitrogen protection. Subsequently, 2-(7-azabenzotriazol)-*N,N,N',N'*-tetramethyluronium hexafluorophosphate (154 mg, 0.41 mmol), *N,N-*diisopropylethylamine (105 mg, 0.81 mmol), and 4,4-difluoropiperidine (33 mg, 0.27 mmol) were added sequentially. The reaction system was raised to 80°C and stirred for 5 hours.

After the depletion of the raw material as monitored by LCMS, the reaction was quenched by adding water (30 mL), and the mixture solution was extracted with ethyl acetate (25 mL × 3 times). The organic phases were combined, and the resultant was washed with saturated saline (60 mL × 2 times), then dried with anhydrous sodium sulfate, filtered, and concentrated under reduced pressure. The resulting residue was purified by silica gel column chromatography to obtain the product 6-3 (58 mg, 39.2% yield).

MS (ESI) M/Z: 447.0 [M-99]⁺.

Step C: Compound 6-3 (58 mg, 0.1 mmol) was dissolved in a dry dichloromethane solution (0.4 mL) at room temperature under nitrogen protection. Subsequently, trifluoroacetic acid (0.1 mL) was added slowly and dropwise to the reaction system. Stirring of the reaction system was kept for 30 min at room temperature.

After the depletion of the raw material as monitored by LCMS, the reaction solution was concentrated by vacuum distillation. The crude Compound 6-4 (50 mg) was obtained.

MS (ESI) M/Z: 447.0 [M+H]⁺.

Step D: The compound isoquinoline-4-carboxylic acid (17 mg, 0.1 mmol) was dissolved in *N,N*-dimethylformamide (1 mL) at room temperature under nitrogen protection. Subsequently, 2-(7-azabenzotriazol)-*N,N,N',N'*-tetramethyluronium hexafluorophosphate (57 mg, 0.15 mmol), *N,N*-diisopropylethylamine (39 mg, 0.3 mmol), and Compound 6-4 (50 mg, 0.1 mmol) were added sequentially. Stirring of the reaction system was kept for 1 hour.

After the depletion of the raw material as monitored by LCMS, the reaction was quenched by adding water (35 mL), and the mixture solution was extracted with ethyl acetate (15 mL × 3 times). The organic phases were combined, and the resultant was washed with saturated saline (20 mL × 2 times), then dried with anhydrous sodium sulfate, filtered, and concentrated under reduced pressure. The resulting residue was purified by preparative high performance liquid chromatography to obtain the titled Compound 6 (3.07 mg, 4.6% yield).

MS (ESI) M/Z: 602.0 [M+H]⁺.
¹H NMR (400 MHz, DMSO-*d*₆) *δ* 9.39 (s, 0.6H), 9.29 (s. 0.4H), 8.47 - 8.10 (m, 3H), 7.97 - 7.47 (m, 5H), 4.38 - 3.37 (m. 7H), 3.24 - 2.88 (m, 2H), 2.25 - 1.22 (m. 8H).

### Example 7

### (5-Bromo-2-((R)-1-(isoquinolin-4-carbonyl)piperidin-3-yl)amino)-3-nitrophenyl)(6,6-dimethyl-3-azabicyclo[3.1.0]hexan-3-yl)methanone

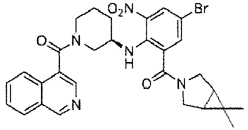

### Reaction route:

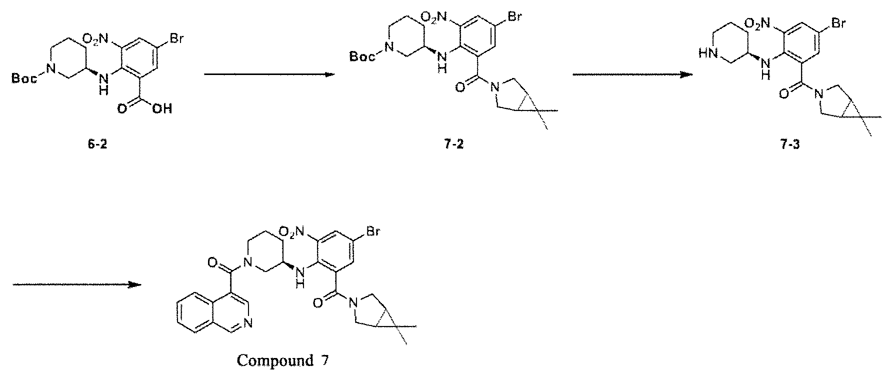

### Operating steps:

Step A: Compound 6-2 (120 mg, 0.27 mmol) was dissolved in *N,N-*dimethylformamide (2 mL) at room temperature under nitrogen protection. Subsequently, 2-(7-azabenzotriazol)-*N,N,N',N'-*tetramethyluronium hexafluorophosphate (154 mg, 0.41 mmol), *N,N*-diisopropylethylamine (105 mg, 0.81 mmol), and 4,4-difluoropiperidine (33 mg, 0.27 mmol) were added in sequence. The reaction system was stirred at 80°C for 5 hours.

After the depletion of the raw material as monitored by LCMS, the reaction was quenched by adding water (30 mL), and the mixture solution was extracted with ethyl acetate (25 mL × 3 times). The organic phases were combined, and the resultant was washed with saturated saline (60 mL × 2 times), then dried with anhydrous sodium sulfate, filtered, and concentrated under reduced pressure. The resulting residue was purified by silica gel column chromatography to obtain Compound 7-2 (77 mg, 53.0% yield).

MS (ESI) M/Z: 437.0 [M-99]⁺.

Step B: Compound 7-2 (77 mg, 0.14 mmol) was dissolved in a dry dichloromethane solution (0.6 mL) at room temperature under nitrogen protection. Subsequently, trifluoroacetic acid (0.15 mL) was added slowly and dropwise to the reaction system. The reaction system was stirred at room temperature for 30 min.

After the depletion of the raw material as monitored by LCMS, the reaction solution was concentrated by vacuum distillation. The crude Compound 7-3 (65 mg) was obtained.

MS (ESI) M/Z: 437.0 [M+H]⁺.

Step C: The compound isoquinoline-4-carboxylic acid (24.3 mg, 0.14 mmol) was dissolved in *N,N-*dimethylformamide (1 mL) at room temperature under nitrogen protection. Subsequently, 2-(7-azabenzotriazol)-*N,N,N',N'*-tetramethyluronium hexafluorophosphate (80 mg, 0.21 mmol), *N,N*-diisopropylethylamine (54 mg, 0.42 mmol), and Compound 7-3 (65 mg, 0.14 mmol) were added sequentially. Stirring of the reaction system was kept for 1 hour.

After the depletion of the raw material as monitored by LCMS, the reaction was quenched by adding water (35 mL), and the mixture solution was extracted with ethyl acetate (15 mL × 3 times). The organic phases were combined, and the resultant was washed with saturated saline (20 mL × 2 times), then dried with anhydrous sodium sulfate, filtered, and concentrated under reduced pressure. The resulting residue was purified by preparative high performance liquid chromatography to obtain the titled Compound 7 (10.8 mg, 12.3% yield).

MS (ESI) M/Z: 592.0 [M+H]⁺.
¹H NMR (400 MHz, DMSO-*d₆*) δ 9.39 - 9.25 (m, 1H), 8.55 - 8.10 (m, 3H), 7.91 - 7.22 (m, 5H). 4.17 - 3.88 (m, 1H), 3.86 - 3.40 (m, 5H), 3.16 - 2.70 (m, 3H), 1.92 - 1.22 (m, 5.7H), 1.11 - 0.30 (m, 6.3H).

### Example 8

### (R)-(5-bromo-2-((1-(7-chloroisoquinolin-4-carbonyl)piperidin-3-yl)amino)-3-nitrophenyl)(piperidin-1-yl)methanone

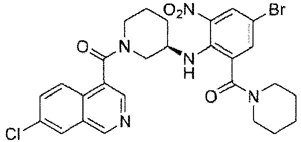

### Reaction route:

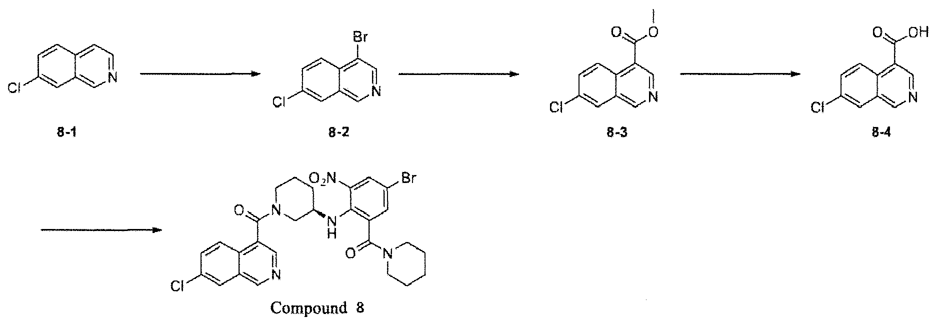

### Operating steps:

Step A: Compound 8-1 (300 mg, 1.84 mmol) was dissolved in nitrobenzene (6 mL) at room temperature under nitrogen protection. Subsequently, the above solution was warmed to 180°C and liquid bromine (315 mg, 2.02 mmol) was added dropwise. Stirring of the reaction system was kept at 180°C for 16 hours.

When the reaction completed, the reaction solution was brought to room temperature. Water (20 mL) was added to the reaction solution, and the mixture solution was extracted with ethyl acetate (10 mL × 3 times). The organic phases were combined, and the resultant was washed with saturated aqueous sodium chloride solution (20 mL × 2 times), then dried with anhydrous sodium sulfate, filtered and concentrated under reduced pressure. The resulting residue was purified by silica gel column chromatography to obtain Compound 8-2 (155 mg, 35% yield).

MS (ESI) M/Z: 241.9 [M+H]⁺.
¹H NMR (400 MHz, DMSO-*d₆*) δ 9.32 (s, 1H), 8.80 (s, 1H), 8.40 (d, *J* = 2.4 Hz, 1H), 8.12 (d, *J* = 9.2 Hz, 1H), 7.99 (dd, *J* = 9.0, 2.2 Hz, 1H).

Step B: Compound 8-2 (155 mg, 0.64 mmol) was dissolved in methanol (6 mL) at room temperature. Subsequently, triethylamine (1.3 g, 12.86 mmol) and [1,1'-bis(diphenylphosphino)ferrocene]palladium dichloride (95 mg, 0.13 mmol) were sequentially added to the above reaction solution. The reaction system was stirred at 90°C for 24 h in carbon monoxide atmosphere.

When the reaction completed, the reaction solution was brought to room temperature. The reaction solution was filtered through diatomaceous earth and the filtrate was collected. Then water (15 mL) was added to the filtrate, and the mixture solution was extracted with ethyl acetate (10 mL × 3 times). The organic phases were combined, and the resultant was washed with saturated aqueous sodium chloride solution (20 mL × 2 times), then dried with anhydrous sodium sulfate, filtered and concentrated under reduced pressure. The resulting residue was purified by silica gel column chromatography to obtain Compound 8-3 (45 mg, 32% yield).

MS (ESI) M/Z: 222.0 [M+H]⁺.

Step C: Compound 8-3 (45 mg, 0.20 mmol) was dissolved in tetrahydrofuran/methanol/water (1mL/0.5 mL/0.1mL) at room temperature. Subsequently, the above solution was cooled down to 0°C and lithium hydroxide monohydrate (42 mg, 1.00 mmol) was added. The reaction system was then warmed to room temperature and stirring was continued for 2 hours.

After the depletion of the raw material as monitored by LCMS, water (10 mL) was added to the reaction solution, and the pH of the mixture solution was adjusted to 3 with dilute hydrochloric acid at 1 mol/L. The mixture solution was extracted with ethyl acetate (15 mL × 3 times), and the organic phases were combined, then dried with anhydrous sodium sulfate, filtered and concentrated under reduced pressure. Compound 8-4 (35 mg, 83% yield) was obtained. The resulting compound was used directly in the next step without further purification.

MS (ESI) M/Z: 208.0 [M+H]⁺.

Step D: Compound 8-4 (30 mg, 0.15 mmol) and (R)-(5-bromo-2-((1-piperidin-3-yl)amino)-3-nitrophenyl)(piperidin-1-yl)methanone (66 mg, 0.16 mmol) (4-4) were dissolved in *N,N*-dimethylformamide (1 mL). Subsequently, the above solution was cooled down to 0°C and 2-(7-azabenzotriazol)-*N,N,N',N'*-tetramethyluronium hexafluorophosphate (55 mg, 0.15 mmol) and *N,N-*diisopropylethylamine (56 mg, 0.45 mmol) were added in sequence. The reaction system was stirred at 0°C for 30 min.

After the depletion of the raw material as monitored by LCMS, the reaction was quenched by adding water (20 mL), and the mixture solution was extracted with ethyl acetate (6 mL × 3 times). The organic phases were combined, and the resultant was washed with saturated aqueous sodium chloride solution (20 mL × 2 times), then dried with anhydrous sodium sulfate, filtered and concentrated under reduced pressure. The resulting residue was purified by reversed-phase chromatographic column to obtain the titled Compound 8 (46.2 mg, 53% yield).

MS (ESI) M/Z: 600.0 [M+H]⁺.
¹H NMR (400 MHz, DMSO-*d*₆) δ 9.39 (s, 0.6H), 9.29 (s, 0.4H). 8.63 - 8.29 (m, 3H), 8.28 - 7.20 (m, 4H). 4.32 - 3.32 (m, 6H), 3.21 - 2.92 (m, 3H), 2.03 - 1.18 (m, 10H).

### Example 9

### N-((1S,2R)-2-((4-Bromo-2-(piperidin-1-carbonyl)-6-(trifluoromethyl)phenyl)amino)cyclohexyl)isoquinolin-4-formamide

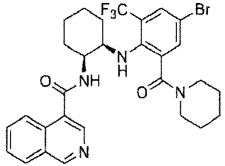

### Reaction route:

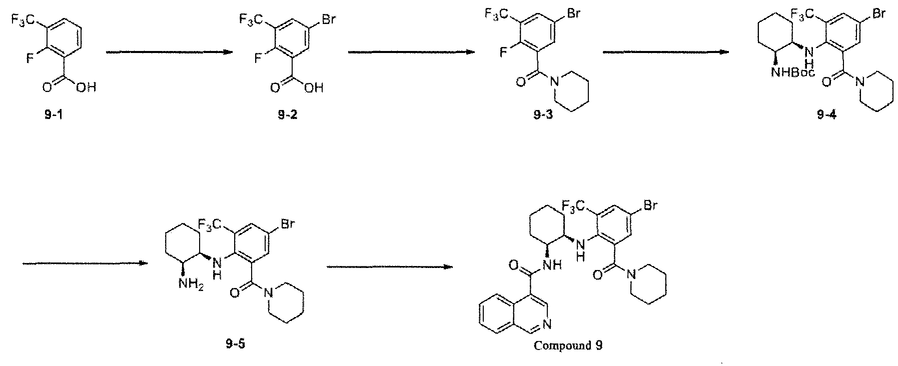

### Operating steps:

Step A: Compound 9-1 (400 mg, 1.92 mmol) was dissolved in a solvent mixture of trifluoroacetic acid (4 mL) / concentrated sulfuric acid (1 mL) at 0°C. Subsequently, *N*-bromosuccinimide (512.6 mg, 2.88 mmol) was added to the above solution. The reaction system was then brought to room temperature and stirring was continued for 18 hours.

After the depletion of the raw material as monitored by LCMS, the reaction was quenched by adding ice water (80 mL), and the mixture solution was extracted with ethyl acetate (15 mL × 3 times). The organic phases were combined, and the resultant was washed with aqueous sodium chloride solution (20 mL × 2 times), then dried with anhydrous sodium sulfate, filtered, and concentrated under reduced pressure. The resulting residue was purified by silica gel column chromatography to obtain Compound 9-2 (390 mg, 71% yield).

HNMR: ¹H NMR (400 MHz, *DMSO-d*₆ ) δ 14.03 (s, 1H), 8.23 - 8.26 (m, 2H).

Step B: Compound 9-2 (390 mg, 1.36 mmol) was dissolved in *N,N*-dimethylformamide (6 mL) at 0°C under nitrogen protection. Subsequently, 2-(7-azabenzotriazol)-*N,N,N',N'*-tetramethyluronium hexafluorophosphate (770 mg, 2.05 mmol), *N,N*-diisopropylethylamine (526 mg, 4.08 mmol), and piperidine (115 mg, 1.36 mmol) were sequentially added to the above reaction solution. Stirring of the reaction system was kept for 15 minutes.

After the depletion of the raw material as monitored by LCMS, the reaction was quenched by adding water (50 mL), and the mixture solution was extracted with ethyl acetate (10 mL × 3 times). The organic phases were combined, and the resultant was washed with aqueous sodium chloride solution (10 mL × 2 times), then dried with anhydrous sodium sulfate, filtered and concentrated under reduced pressure. The resulting residue was purified by silica gel column chromatography to obtain Compound 9-3 (385 mg, 80.2% yield).

MS (ESI) M/Z: 354.0 [M+H]⁺.

Step C: Compound 9-3 (385 mg, 1.1 mmol) and tert-butyl ((*1S*,*2R*)-2-aminocyclohexyl) carbamate (233 mg, 1.1 mmol) were dissolved in *N,N*-dimethylformamide (5 mL) at room temperature under nitrogen protection. Subsequently, *N,N-*diisopropylethylamine (425 mg, 3.3 mmol) was added dropwise to the above reaction solution. The reaction system was raised to 135°C and stirring was continued for 16 hours.

After the conversion from the raw material into product as monitored by LCMS, the reaction was quenched by adding water (40 mL), and the mixture solution was extracted with ethyl acetate (10 mL × 3 times). The organic phases were combined, and the resultant was washed with aqueous sodium chloride solution (20 mL × 2 times), then dried with anhydrous sodium sulfate, filtered, and concentrated under reduced pressure. The resulting residue was purified by silica gel column chromatography to obtain Compound 9-4 (181 mg, 30.1% yield).

MS (ESI) M/Z: 548.1 [M+H]⁺.

Step D: Compound 9-4 (181 mg, 0.33 mmol) was dissolved in dichloromethane (3 mL) at room temperature. Subsequently, the temperature was lowered to 0°C and 4 M hydrochloric acid-1,4-dioxane solution (1.5 mL, 6.6 mmol) was added slowly and dropwise to the reaction solution. The reaction system was then brought to room temperature and stirring was continued for 1 hour.

After the depletion of the raw material as monitored by LCMS, the reaction solution was concentrated by vacuum distillation. Compound 9-5 (161 mg, 109.1% yield) was obtained.

MS (ESI) M/Z: 448.0 [M+H]⁺.

Step E: Compound 9-5 (161 mg, 0.36 mmol) was dissolved in *N*,*N-*dimethylformamide (3 mL) at room temperature under nitrogen protection. Subsequently, the above solution was cooled down to 0°C and isoquinoline-4-carboxylic acid (62 mg, 0.36 mmol), 2-(7-azabenzotriazol)-*N,N,N',N'*-tetramethyluronium hexafluorophosphate (205.2 mg, 0.54 mmol), and *N,N*-diisopropylethylamine (139 mg, 1.08 mmol) were added in sequence. Stirring of the reaction system was kept for 15 minutes.

After the depletion of the raw material as monitored by LCMS, the reaction was quenched by adding water (15 mL), and the mixture solution was extracted with ethyl acetate (5 mL × 3 times). The organic phases were combined, and the resultant was washed with aqueous sodium chloride solution (20 mL × 2 times), then dried with anhydrous sodium sulfate, filtered and concentrated under reduced pressure. The resulting residue was purified by preparative high performance liquid chromatography to obtain the titled Compound 9 (30.5 mg, 14.1% yield).

MS (ESI) M/Z: 603.2 [M+H]⁺.
¹H NMR (400 MHz. DMSO-*d*₆) δ 9.36 (s, 1H), 8.57 (s, 1H), 8.39 (brs, 1H), 8.15 (t, *J* = 7.8 Hz, 2H), 7.79 (t. *J* = 7.6 Hz, 1H), 7.71 (t, *J* = 7.4 Hz. 1H). 7.61 (d, *J* = 2.4 Hz, 1H), 7.44 (d, *J* = 2.0 Hz, 1H), 4.62 (d. *J* = 9.6 Hz, 1H). 4.49 (s, 1H). 3.75 - 3.64 (m, 2H), 3.38 - 3.26 (m, 2H), 1.93 - 1.87 (m, 1H). 1.79 - 1.35 (m. 12H), 1.30 - 1.17 (m, 1H). ¹⁹F NMR (376 MHz, DMSO-*d*₆) δ -59.56 (s). -59.93 (s).

### Example 10

### (R)-(3-bromo-2-(1-(isoquinolin-4-carbonyl)piperidin-3-yl)amino)-5-nitrophenyl)(piperazin-1-yl)methanone

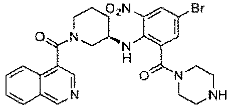

### Reaction route:

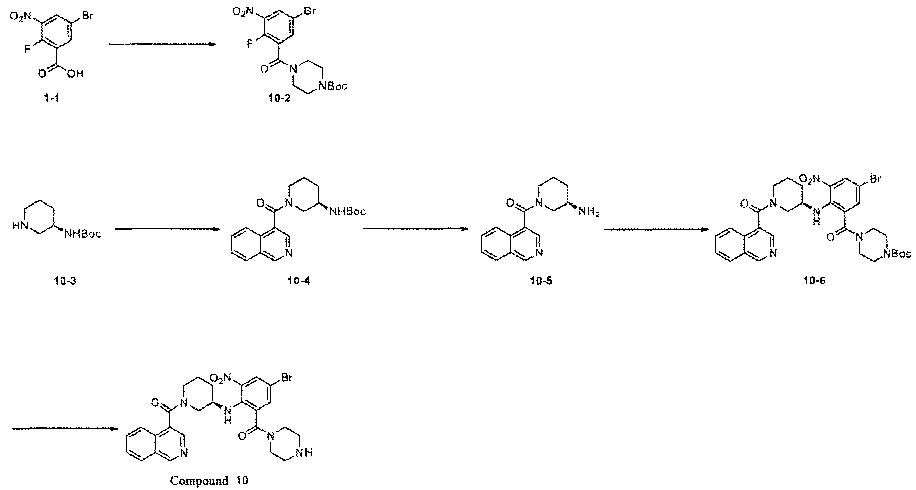

### Operating steps:

Step A: Compound 1-1 (200 mg, 0.76 mmol) was dissolved in *N,N*-dimethylformamide (4 mL) at room temperature under nitrogen protection. Subsequently, the above solution was cooled down to 0°C and 2-(7-azabenzotriazol)-*N,N,N*'*,N*'-tetramethyluronium hexafluorophosphate (403 mg, 1.06 mmol), tert-butyl piperazin-1-carboxylate (169 mg, 0.91 mmol) and *N,N*-diisopropylethylamine (297 mg, 2.28 mmol) were added in sequence. Stirring of the reaction system was kept for 10 min.

After the depletion of the raw material as monitored by TLC, the reaction was quenched by adding water (15 mL), and the mixture solution was extracted with ethyl acetate (10 mL × 3 times). The organic phases were combined, and the resultant was washed with aqueous sodium chloride solution (20 mL × 2 times), dried with anhydrous sodium sulfate, filtered, and concentrated under reduced pressure. The resulting residue was purified by silica gel column chromatography to obtain Compound 10-2 (200 mg, 61.2% yield).

MS (ESI) M/Z: 376.0 [M-55]⁺.

Step B: The compound isoquinoline-4-carboxylic acid (103 mg, 0.6 mmol) was dissolved in *N,N*-dimethylformamide (2.5 mL) at room temperature under nitrogen protection. Subsequently, the above solution was cooled down to 0°C and 2-(7-azabenzotriazol)-*N,N,N*'*,N*'-tetramethyluronium hexafluorophosphate (266 mg, 0.7 mmol), Compound 10-3 (100 mg, 0.5 mmol), and *N*,*N*-diisopropylethylamine (196 mg, 1.50 mmol) were added sequentially. Stirring of the reaction system was kept for 10 minutes.

After the depletion of the raw material as monitored by TLC, the reaction was quenched by adding water (20 mL), and the mixture solution was extracted with ethyl acetate (10 mL × 3 times). The organic phases were combined, and the resultant was washed with aqueous sodium chloride solution (20 mL × 2 times), then dried with anhydrous sodium sulfate, filtered, and concentrated under reduced pressure. The resulting residue was purified by silica gel column chromatography to obtain Compound 10-4 (175 mg, 98.8% yield).

MS (ESI) M/Z: 356.2 [M+H]⁺.

Step C: Compound 10-4 (175 mg, 0.49 mmol) was dissolved in dichloromethane (2.5 mL) at 0°C. Subsequently, 4 M hydrochloric acid-1,4-dioxane solution (2.5 mL) was added slowly and dropwise to the reaction system. The reaction system was then brought to room temperature and stirred for 1.5 hours.

After the depletion of the raw material as monitored by LCMS, the reaction solution was concentrated by vacuum distillation. The crude product Compound 10-5 (144 mg) was obtained.

MS (ESI) M/Z: 256.2 [M+H]⁺.

Step D: Compound 10-5 (125 mg, 0.49 mmol) was dissolved in *N,N*-dimethylformamide (2.5 mL) at room temperature under nitrogen protection. Subsequently, Compound 10-2 (210 mg, 0.32 mmol) and *N,N*-diisopropylethylamine (380 mg, 2.94 mmol) were added sequentially. The reaction system was stirred at 80°C for 2 hours.

After the depletion of the raw material as monitored by LCMS, the reaction was quenched by adding water (25 mL), and the mixture solution was extracted with ethyl acetate (15 mL × 3 times). The organic phases were combined, and the resultant was washed with saturated saline (20 mL × 2 times), then dried over anhydrous sodium sulfate, filtered, and concentrated under reduced pressure to obtain Compound 10-6 (210 mg, 64.4% yield).

MS (ESI) M/Z: 667.2 [M+H]⁺.

Step E: Compound 10-6 (60 mg, 0.09 mmol) was dissolved in dichloromethane (0.46 mL) at 0°C. Subsequently, 4 M hydrochloric acid-1,4-dioxane solution (0.23 mL) was added slowly and dropwise to the reaction system. The reaction system was brought to room temperature and stirring was continued for 1.5 hours.

After the depletion of the raw material as monitored by LCMS, the reaction solution was concentrated by vacuum distillation. The resulting residue was dissolved in water (1.5 mL) and the mixture solution was adjusted to pH = 8 with 10%wt aqueous ammonia. The resulting solution was purified by reversed-phase chromatography column to obtain the titled Compound 10 (22.1 mg, 43.3% yield).

MS (ESI) M/Z: 567.0 [M+H]⁺.
¹H NMR (400 MHz, DMSO-*d*₆) δ 9.39 (s, 0.6H). 9.28 (s, 0.4H), 8.57 - 8.05 (m, 3H), 7.98 - 7.19 (m. 6H), 4.26 - 3.42 (m, 6H), 3.20 - 2.99 (m, 3H), 2.94 - 2.58 (m, 4H), 2.01 - 1.35 (m, 4H).

### Example 11

### (R)-(5-bromo-2-((1-(4-cinnolin-carbonyl)piperidin-3-yl)amino)-3-nitrophenyl)(piperidin-1-yl)methanone

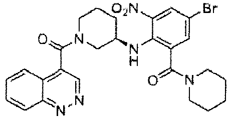

### Reaction route:

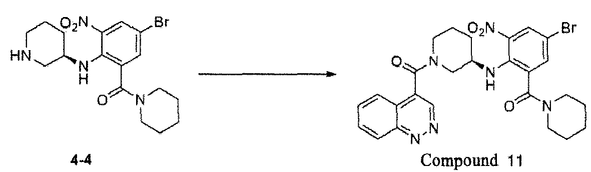

### Operating steps:

Step A: Compound 4-cinnolinecarboxylic acid (26 mg, 0.15 mmol) was dissolved in *N,N*-dimethylformamide (1 mL) at room temperature under nitrogen protection. Subsequently, the above solution was cooled down to 0°C and 2-(7-azabenzotriazol)-*N,N*,*N*',*N*'-tetramethyluronium hexafluorophosphate (86 mg, 0.23 mmol), *N,N*-diisopropylethylamine (97 mg, 0.75 mmol), and (*R*)-(5-bromo-3-nitro-2-(piperidin-3-ylamino)phenyl)(piperidin-1-yl)methanone (60 mg, 0.15 mmol) (4-4) were added in sequence. Stirring of the reaction system was kept for 30 minutes.

After the depletion of the raw material as monitored by LCMS, the reaction was quenched by adding water (15 mL), and the mixture solution was extracted with ethyl acetate (10 mL × 3 times). The organic phases were combined, and the resultant was washed with saturated aqueous sodium chloride solution (60 mL × 2 times), dried with anhydrous sodium sulfate, filtered and concentrated under reduced pressure. The resulting residue was purified by preparative high performance liquid chromatography to obtain the titled Compound 11 (40 mg, 47% yield).

MS (ESI) M/Z: 567.0 [M+H]⁺.
¹H NMR (400 MHz, DMSO-*d*₆) δ 9.31 (s, 0.6H), 9.14 (s, 0.4H), 8.58 (d, *J* = 8.4 Hz, 0.6H), 8.50 (brs, 0.4H), 8.30 (d, *J* = 2.4 Hz, 0.6H). 8.08 - 7.75 (m, 4.4H). 7.67 (d, *J* = 2.4 Hz, 0.6H). 7.44 - 7.18 (m, 0.4H), 4.07 -3.40 (m, 6H), 3.25 - 3.00 (m, 3H). 2.00 -1.28 (m, 10H).

### Example 12

### (R)-(5-bromo-2-((1-(isoquinolin-4-carbonyl)pyrrolidin-3-yl)amino)-3-nitrophenyl)(piperidin-1-yl)methanone

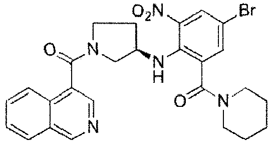

### Reaction route:

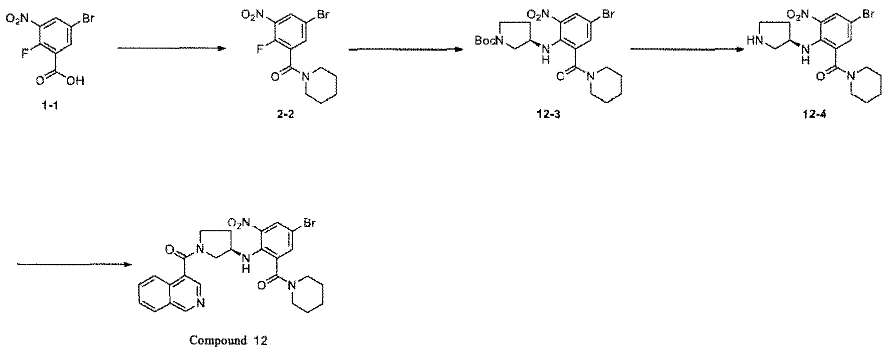

### Operating steps:

Step A: Compound 1-1 (400 mg, 1.5 mmol) was dissolved in *N,N*-dimethylformamide (7.6 mL) at room temperature under nitrogen protection. Subsequently, the above solution was cooled down to -10°C and *N,N*-diisopropylethylamine (587 mg, 4.5 mmol), 2-(7-azabenzotriazol)-*N,N,N',N*'-tetramethyluronium hexafluorophosphate (806 mg, 2.1 mmol), and piperidine (155 mg, 1.8 mmol) were added sequentially. Stirring of the reaction system was kept for 3 min.

After the depletion of the raw material as monitored by TLC, the reaction was quenched by adding water (20 mL), and the mixture solution was extracted with ethyl acetate (20 mL × 3 times). The organic phases were combined, and the resultant was washed with saturated saline (20 mL × 5 times), then dried with anhydrous sodium sulfate, filtered, and concentrated under reduced pressure. The resulting residue was purified by silica gel column chromatography to obtain Compound 2-2 (185 mg, 37% yield).

MS (ESI) M/Z: 331.0 [M+H]⁺.

Step B: Compound 2-2 (85 mg, 0.26 mmol) was dissolved in *N,N*-dimethylformamide (1.3 mL) at room temperature under nitrogen protection. Subsequently, tert-butyl (R)-3-aminopyrrolidin-1-carboxylate (57 mg, 0.31 mmol) and *N,N-*diisopropylethylamine (100 mg, 0.77 mmol) were sequentially added to the above reaction solution. The reaction system was stirred at 80°C for 2 hours.

After the depletion of the raw material as monitored by LCMS, the reaction was quenched by adding water (10 mL), and the mixture solution was extracted with ethyl acetate (20 mL × 3 times). The organic phases were combined, and the resultant was washed with saturated saline (20 mL × 3 times), then dried with anhydrous sodium sulfate, filtered, and concentrated under reduced pressure. Compound 12-3 (150 mg) was obtained, and the crude product was used directly in the next step of the reaction.

MS (ESI) M/Z: 397.0 [M-99]⁺.

Step C: Compound 12-3 (75 mg, 0.15 mmol) was dissolved in 4 M hydrochloric acid-1,4-dioxane solution (0.38 mL) at room temperature under nitrogen protection. The reaction system was stirred at room temperature for 2 hours.

After the depletion of the raw material as monitored by LCMS, the reaction solution was concentrated by vacuum distillation. Compound 12-4 (60 mg) was obtained.

MS (ESI) M/Z: 397.0 [M+H]⁺.

Step D: Compound 12-4 (60 mg, 0.15 mmol) was dissolved in *N,N-*dimethylformamide (0.76 mL) at room temperature under nitrogen protection. Subsequently, the above solution was cooled down to 0°C and isoquinoline-4-carboxylic acid (26 mg, 0.15 mmol), 2-(7-azabenzotriazol)-*N,N,N',N*'-tetramethyluronium hexafluorophosphate (86 mg, 0.23 mmol), and *N,N-*diisopropylethylamine (96 mg, 0.76 mmol) were added in sequence. The reaction system was stirred at 0 °C for 15 min.

After the depletion of the raw material as monitored by LCMS, the reaction was quenched by adding water (10 mL), and the mixture solution was extracted with ethyl acetate (20 mL × 3 times). The organic phases were combined, and the resultant was washed with saturated saline (50 mL × 3 times), then dried with anhydrous sodium sulfate, filtered, and concentrated under reduced pressure. The resulting residue was purified by reversed-phase column to obtain the titled Compound 12 (10.6 mg, 12.8% yield).

MS (ESI) M/Z: 552.0 [M+H]⁺.
¹H NMR (400 MHz. DMSO-*d*₆) δ 9.39 (s, 0.4H), 9.37 (s, 0.6H). 8.50 (s, 0.4H), 8.47 - 8.40 (m, 0.6H), 8.26 - 8.12 (m, 2H), 7.88 - 7.66 (m, 4.4H). 7.44 (d. *J* = 2.4 Hz, 0.6H), 4.21 (s. 0.4H), 4.03 (s, 0.6H), 3.84 - 3.60 (m, 3H), 3.53 - 3.36 (m, 2H). 3.25 - 2.87 (m. 3H), 2.32 - 2.22 (m, 0.6H), 2.18 - 2.06 (m, 1H), 1.98 - 1.89 (m. 0.4H), 1.73 - 1.02 (m, 6H).

### Example 13

### (R)-(5-bromo-2-((1-(isoquinolin-4-carbonyl)piperidin-3-yl)amino)-3-nitrophenyl)(piperidin-1-yl)methanone

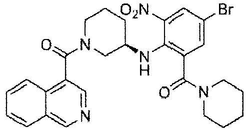

### Reaction route:

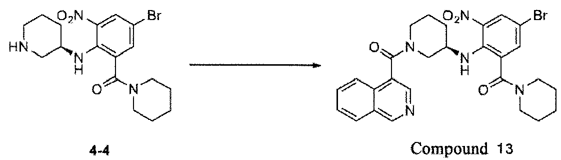

### Operating steps:

Step A: Compound 4-4 (50 mg, 0.12 mmol) and isoquinoline-4-carboxylic acid (23 mg, 0.13 mmol) were dissolved in *N,N*-dimethylformamide (1 mL) at room temperature under nitrogen protection. Subsequently, the above solution was cooled down to 0°C and 2-(7-azabenzotriazol)-*N,N,N',N*'-tetramethyluronium hexafluorophosphate (46 mg, 0.12 mmol) and *N,N*-diisopropylethylamine (47 mg, 0.36 mmol) were added sequentially. The reaction system was stirred at 0°C for 30 min.

After the depletion of the raw material as monitored by LCMS, the reaction was quenched by adding water (20 mL), and the mixture solution was extracted with ethyl acetate (6 mL × 3 times). The organic phases were combined, and the resultant was washed with saturated aqueous sodium chloride solution (20 mL × 2 times), then dried with anhydrous sodium sulfate, filtered and concentrated under reduced pressure. The resulting residue was purified by reversed-phase chromatography column to obtain the titled Compound 13 (11.7 mg, 17% yield).

MS (ESI) M/Z: 566.0 [M+H]⁺.
¹H NMR (400 MHz, DMSO-*d*₆)) δ 9.40 (s. 0.6H). 9.30 (s, 0.4H), 8.59 - 8.06 (m, 3H), 8.01 - 7.27 (m, 5H), 4.37 - 3.42 (m, 6H), 3.26 - 2.96 (m, 3H), 2.04 - 1.16 (m, 10H).

### Example 14

### 5-Bromo-N-((1S,2R))-2-((4-bromo-2-nitro-6-(piperidin-1-carbonyl)phenyl)amino)cyclohexyl)nicotinamide

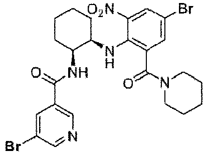

### Reaction route:

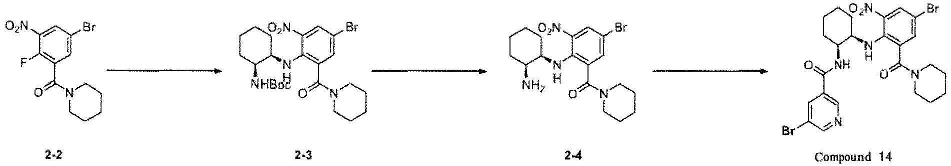

### Operating steps:

Step A: Compound 2-2 (60 mg, 0.18 mmol) was dissolved in *N,N*-dimethylformamide (1 mL) at room temperature under nitrogen protection. Subsequently, tert-butyl (*R*)-3-aminopiperidin-1-carboxylate (47 mg, 0.22 mmol) and *N,N-*diisopropylethylamine (70 mg, 0.54 mmol) were sequentially added to the above reaction solution. The reaction system was stirred at 80°C for 2 hours.

After the depletion of the raw material as monitored by LCMS, the reaction was quenched by adding water (15 mL), and the mixture solution was extracted with ethyl acetate (5 mL × 3 times). The organic phases were combined, and the resultant was washed with aqueous sodium chloride solution (20 mL × 2 times), then dried with anhydrous sodium sulfate, filtered, and concentrated under reduced pressure. The resulting residue was purified by silica gel column chromatography to obtain Compound 2-3 (90 mg, 95% yield).

MS (ESI) M/Z: 547.1 [M+Na]⁺.

Step B: Compound 2-3 (90 mg, 0.17 mmol) was dissolved in 4 M hydrochloric acid-1,4-dioxane solution (0.4 mL) at room temperature under nitrogen protection. The reaction system was stirred at room temperature for 1 hour.

After the depletion of the raw material as monitored by LCMS, the reaction solution was concentrated by vacuum distillation. Compound 2-4 (80 mg) was obtained.

MS (ESI) M/Z: 425.2 [M+H]⁺.

Step D: Compound 2-4 (80 mg, 0.19 mmol) was dissolved in *N,N*-dimethylformamide (1 mL) at room temperature under nitrogen protection. Subsequently, the above solution was cooled down to 0°C and nicotinic acid (32 mg, 0.16 mmol), 2-(7-azabenzotriazol)-*N,N,N',N*'-tetramethyluronium hexafluorophosphate (85 mg, 0.22 mmol), and *N,N-*diisopropylethylamine (103 mg, 0.80 mmol) were added in sequence. The reaction system was stirred at 0°C for 30 minutes.

After the depletion of the raw material as monitored by LCMS, the reaction was quenched by adding water (15 mL), and the mixture solution was extracted with ethyl acetate (5 mL × 3). The organic phases were combined, and the resultant was washed with saturated saline (20 mL × 2), then dried with anhydrous sodium sulfate, filtered, and concentrated under reduced pressure. The resulting residue was purified by reversed-phase chromatography column to obtain the titled Compound 14 (37.5 mg, 23.1% yield).

MS (ESI) M/Z: 610.2 [M+H]⁺.
¹H NMR (400 MHz, DMSO-*d*₆) δ 8.90 - 8.75 (m, 2H), 8.64 (d, *J* = 6.8 Hz, 0.5H), 8.38 - 8.14 (m, 2.5H), 7.97 - 7.76 (m, 1H), 7.56 (d, *J* = 2.0 Hz, 1H). 4.26 (s, 1H). 3.86 - 3.45 (m, 3H). 3.16 (s, 2H), 1.83 - 1.24 (m, 14H).

### Example 15

### ((3R)-3-((2-(3-azabicyclo[3.1.0]hexan-3-carbonyl)-4-bromo-6-nitrophenyl)amino)piperidin-1-yl)(isoquinolin-4-yl)methanone

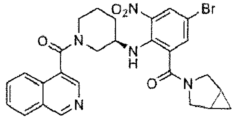

### Reaction route:

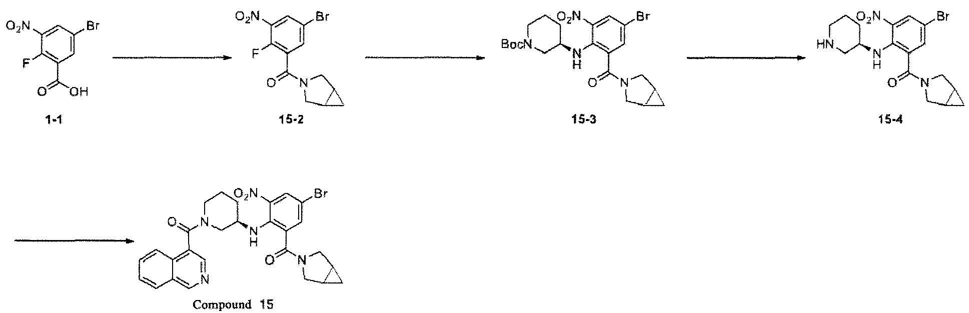

### Operating steps:

Step A: Compound 1-1 (100 mg, 0.38 mmol) was dissolved in *N,N-*dimethylformamide (4 mL) under ice-salt bath and nitrogen protection. Subsequently, 2-(7-azabenzotriazol)-*N,N,N',N'*-tetramethyluronium hexafluorophosphate (216 mg, 0.57 mmol), triethylamine (115 mg, 1.14 mmol), and 3-azabicyclo[3.1.0]hexane hydrochloride (45.3 mg, 0.38 mmol) were added sequentially. Stirring of the reaction system was kept for 10 min.

After the depletion of the raw material as monitored by TLC, the reaction was quenched by adding ice water (30 mL), and the mixture solution was extracted with ethyl acetate (10 mL × 3 times). The organic phases were combined, and the resultant was washed with saturated saline (30 mL × 2 times), then dried with anhydrous sodium sulfate, filtered, and concentrated under reduced pressure. The resulting residue was purified by silica gel column chromatography to obtain Compound 15-2 (70 mg, 56.1% yield).

MS (ESI) M/Z: 329.0 [M+H]⁺.

Step B: Compound 15-2 (70 mg, 0.21 mmol) was dissolved in *N,N*-dimethylformamide (2 mL) at room temperature under nitrogen protection. Subsequently, *N,N*-diisopropylethylamine (82.5 mg, 0.63 mmol) and tert-butyl (*R*)-3-aminopiperidin-1-carboxylate (51 mg, 0.25 mmol) were added sequentially. The reaction system was stirred for 2 hours.

After the depletion of the raw material as monitored by LCMS, the reaction was quenched by adding water (20 mL), and the mixture solution was extracted with ethyl acetate (10 mL×3 times). The organic phases were combined, and the resultant was washed with saturated aqueous sodium chloride solution (20 mL×2 times), dried with anhydrous sodium sulfate, filtered and concentrated under reduced pressure. The crude product Compound 15-3 (70 mg) was obtained.

MS (ESI) M/Z: 409.4 [M-99]⁺.

Step C: Compound 15-3 (70 mg, 0.15 mmol) was dissolved in a dry dichloromethane solution (0.6 mL) at room temperature under nitrogen protection. Subsequently, trifluoroacetic acid (0.15 mL) was added slowly and dropwise to the reaction system. The reaction system was stirred for 30 minutes.

After the depletion of the raw material as monitored by LCMS, the reaction solution was concentrated by vacuum distillation. The crude product Compound 15-4 (90 mg) was obtained.

MS (ESI) M/Z: 409.2 [M+H]⁺.

Step D: The compound isoquinoline-4-carboxylic acid (25 mg, 0.14 mmol) was dissolved in *N,N*-dimethylformamide (1 mL) at room temperature under nitrogen protection. Subsequently, 2-(7-azabenzotriazol)-*N,N,N',N*'-tetramethyluronium hexafluorophosphate (80 mg, 0.21 mmol), *N,N*-diisopropylethylamine (54 mg, 0.42 mmol), and Compound 15-4 (90 mg, 0.1 mmol) were added sequentially. The reaction system was stirred for 1 hour.

After the depletion of the raw material as monitored by LCMS, the reaction was quenched by adding water (35 mL), and the mixture solution was extracted with ethyl acetate (15 mL × 3). The organic phases were combined, and the resultant was washed with saturated aqueous sodium chloride solution (20 mL × 2), then dried with anhydrous sodium sulfate, filtered and concentrated under reduced pressure. The resulting residue was purified by preparative high performance liquid chromatography to obtain the titled Compound 15 (20.5 mg, 25.1% yield).

MS (ESI) M/Z: 564.0 [M+H]⁺.

¹H NMR (400 MHz, DMSO-*d*₆) δ 9.39 - 9.27 (m, 1H), 8.52 - 8.02 (m, 3H), 7.95 - 7.27 (m, 5H), 4.17 - 3.36 (m, 6H), 3.28 - 2.79 (m, 3H), 1.99 - 1.17 (m, 6H), 0.83 - 0.50 (m, 1H), 0.29 - 0.18 (m, 1H).

### Example 16

### N-((1S,2R)-2-((4-bromo-2-(4,4-difluoropiperidin-1-carbonyl)-6-nitrophenyl)amino)cyclohexyl)-2-oxo-1,2-dihydroquinolin-4-formamide

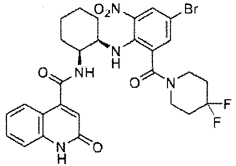

### Reaction route:

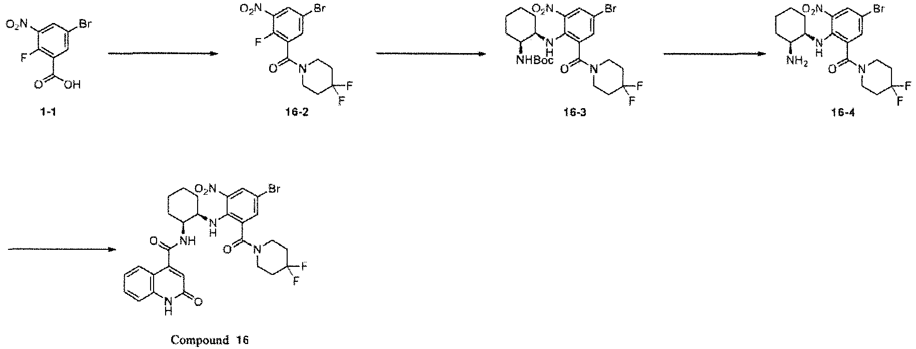

### Operating steps:

Step A: Compound 1-1 (200 mg, 0.76 mmol) was dissolved in *N,N*-dimethylformamide (1 mL) at room temperature. Subsequently, the above solution was cooled down to 0°C and *N,N*-diisopropylethylamine (294 mg, 2.28 mmol), 2-(7-azabenzotriazol)-*N,N*,*N*',*N*'-tetramethyluronium hexafluorophosphate (404 mg, 1.06 mmol), and 4,4-difluoropiperidine (144 mg, 0.91 mmol) were added in sequence. The reaction system was then stirred at 0°C for 5 min.

After the depletion of the raw material as monitored by LCMS, the reaction was quenched by adding water (15 mL), and the mixture solution was extracted with ethyl acetate (10 mL × 3 times). The organic phases were combined, and the resultant was washed with saturated saline (20 mL × 2 times), dried with anhydrous sodium sulfate, filtered, and concentrated under reduced pressure. The resulting residue was purified by silica gel column chromatography to obtain Compound 16-2 (217 mg, 78% yield).

MS (ESI) M/Z: 367.0 [M+H]⁺.

Step B: Compound 16-2 (217 mg, 0.59 mmol) was dissolved in *N,N*-dimethylformamide (1 mL) at room temperature under nitrogen protection. Subsequently, tert-butyl (*R*)-3-aminopiperidin-1-carboxylate (152 mg, 0.71 mmol) and *N,N-*diisopropylethylamine (228 mg, 1.77 mmol) were sequentially added to the above reaction solution. The reaction system was then stirred at 80°C for 2 hours.

After the depletion of the raw material as monitored by LCMS, the reaction was quenched by adding water (15 mL), and the mixture solution was extracted with ethyl acetate (10 mL × 3 times). The organic phases were combined, and the resultant was washed with saturated saline (20 mL × 2 times), then dried with anhydrous sodium sulfate, filtered, and concentrated under reduced pressure. The resulting residue was purified by silica gel column chromatography to obtain Compound 16-3 (300 mg, 90% yield).

MS (ESI) M/Z: 461.0 [M-99]⁺.

Step C: Compound 16-3 (300 mg, 0.53 mmol) was dissolved in 4 M hydrochloric acid-1,4-dioxane solution (1.3 mL) at room temperature under nitrogen protection. The reaction system was then stirred at room temperature for 1 hour.

After the depletion of the raw material as monitored by LCMS, the reaction solution was concentrated by vacuum distillation. Compound 16-4 (250 mg, 100% yield) was obtained.

MS (ESI) M/Z: 461.27 [M+H]⁺.

Step D: Compound 16-4 (60 mg, 0.13 mmol) was dissolved in *N*,*N-*dimethylformamide (1 mL) at room temperature under nitrogen protection. Subsequently, the above solution was cooled down to 0°C and 2-oxo-1,2-dihydroquinolin-4-carboxylic acid (20 mg, 0.11 mmol), 2-(7-azabenzotriazol)-*N,N,N',N*'-tetramethyluronium hexafluorophosphate (58 mg, 0.15 mmol), and *N,N*-diisopropylethylamine (71 mg, 0.55 mmol) were added in sequence. The reaction system was then stirred at 0 °C for 30 min.

After the depletion of the raw material as monitored by LCMS, the reaction was quenched by adding water (15 mL), and the mixture solution was extracted with ethyl acetate (5 mL × 3 times). The organic phases were combined, and the resultant was washed with saturated saline (20 mL × 2 times), then dried with anhydrous sodium sulfate, filtered, and concentrated under reduced pressure. The resulting residue was purified by reversed-phase chromatography column to obtain the titled Compound 16 (21.8 mg, 36% yield).

MS (ESI) M/Z: 632.0 [M+H]⁺.
¹H NMR (400 MHz, DMSO-*d*₆) δ 11.90 (s, 1H), 8.83 (s, 0.5H), 8.66 (s, 0.5H), 8.26 (s, 1H), 7.99 (d, *J* = 10.0 Hz, 1H), 7.88 - 7.75 (m, 1H), 7.59 - 7.40 (m, 2H), 7.33 (d, *J* = 8.4 Hz, 1H). 7.12 (t, *J* = 8.0 Hz, 1H), 6.48 (s, 0.5H), 6.39 (s. 0.5H). 4.31 (s, 1H), 4.06 (s, 1H), 3.74 (s, 1H). 3.63 - 3.45 (m. 3H), 2.30 - 1.84 (m, 4H), 1.83 - 1.24 (m, 8H).

### Example 17

### (2-((R)-1-(1H-pyrrolo[2,3-c]pyridin-4-carbonyl)piperidin-3-yl)amino)-5-bromo-3-nitrophenyl)((2R,6S)-2,6-dimethylmorpholinyl)methanone

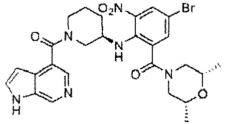

### Reaction route:

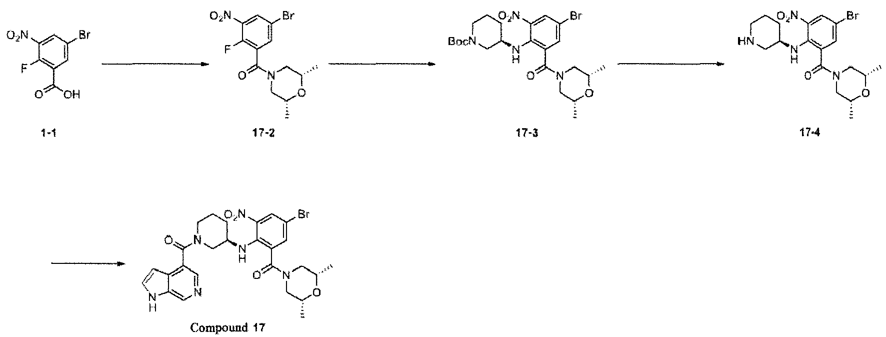

### Procedure.

Step A: Compound 1-1 (1 g, 3.80 mmol) was dissolved in *N,N*-dimethylformamide (19 mL) at room temperature under nitrogen protection. Subsequently, the above solution was cooled down to 0°C and *N,N*-diisopropylethylamine (1.4 g, 11.4 mmol), 2-(7-azabenzotriazol)-*N,N,N',N*'-tetramethyluronium hexafluorophosphate (2 g, 5.32 mmol) and (2*R*,6*S*)-2,6-dimethylmorpholine (526 mg, 4.56 mmol) were added to the above reaction solution in sequence. Stirring of the reaction system was kept at 0°C for 5 min.

After the depletion of the raw material as monitored by LCMS, the reaction was quenched by adding water (100 mL), and the mixture solution was extracted with ethyl acetate (30 mL × 3 times). The organic phases were combined, and the resultant was washed with saturated saline (50 mL × 2 times), then dried with anhydrous sodium sulfate, filtered, and concentrated under reduced pressure. The resulting residue was purified by silica gel column chromatography to obtain Compound 17-2 (600 mg, 44% yield).

MS (ESI) M/Z: 361.0 [M+H]⁺.

Step B: Compound 17-2 (600 mg, 1.67 mmol) was dissolved in *N,N*-dimethylformamide (8.4 mL) at room temperature under nitrogen protection. Subsequently, tert-butyl (*R*)-3-aminopiperidin-1-carboxylate (400 mg, 2.00 mmol) and *N,N-*diisopropylethylamine (649 mg, 5.01 mmol) were sequentially added to the above reaction solution. The reaction system was then stirred at 80°C for 2 hours.

After the depletion of the raw material as monitored by LCMS, the reaction was quenched by adding water (40 mL), and the mixture solution was extracted with ethyl acetate (20 mL × 3 times). The organic phases were combined, and the resultant was washed with saturated saline (50 mL × 2 times), then dried with anhydrous sodium sulfate, filtered, and concentrated under reduced pressure. The resulting residue was purified by silica gel column chromatography to obtain Compound 17-3 (810 mg, 89.8% yield).

MS (ESI) M/Z: 441.0 [M-99]⁺.

Step C: Compound 17-3 (810 mg, 1.50 mmol) was dissolved in 4 M hydrochloric acid-1,4-dioxane solution (2.8 mL) at room temperature under nitrogen protection. The reaction system was then stirred at room temperature for 1 hour.

After the depletion of the raw material as monitored by LCMS, the reaction solution was concentrated by vacuum distillation. Compound 17-4 (720 mg, 100 % yield) was obtained.

### Step D:

Compound 17-1 (20 mg, 0.05 mmol) was dissolved in *N,N*-dimethylformamide (0.5 mL) at room temperature under nitrogen protection. Subsequently, the above solution was cooled down to 0°C and *N,N*-diisopropylethylamine (17 mg, 0.14 mmol), 2-(7-azabenzotriazol)-*N,N,N',N*'-tetramethyluronium hexafluorophosphate (24 mg, 0.06 mmol), and 1H-pyrrolo[2,3-c]pyridin-4-carboxylic acid (9 mg, 0.05 mmol) were added in sequence. Stirring of the reaction system was kept for 2 hours.

After the depletion of the raw material as monitored by LCMS, the reaction was quenched by adding water (5 mL), and the mixture solution was extracted with ethyl acetate (15 mL × 3 times). The organic phases were combined, and the resultant was washed with saturated saline (10 mL × 2 times), dried with anhydrous sodium sulfate, filtered, and concentrated under reduced pressure. The resulting residue was purified by reversed-phase column to obtain the titled Compound 17 (8.4 mg, 31.7% yield).

MS (ESI) M/Z: 585.2 [M+H]⁺.
¹H NMR (400 MHz, DMSO-*d*₆) δ 11.84 (s, 1H), 8.77 (s, 1H), 8.38 - 7.79 (m, 3H). 7.75 - 7.43 (m, 2H), 6.39 (s, 1H), 4.38 (s, 1H), 4.11 - 3.36 (m, 7H), 3.27 - 3.08 (m. 2H), 3.01 -2.77 (m, 1H), 1.96 - 1.79 (m, 1H), 1.77 - 1.37 (m. 3H), 1.20 - 0.91 (m, 6H).

### Example 18

### 4-((R)-3-((4-bromo-2-((2R,6S)-2,6-dimethylmorpholin-4-carbonyl)-6-nitrophenyl)amino)piperidin-1-carbonyl)piperidin-2-one

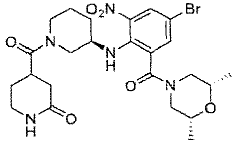

### Reaction route:

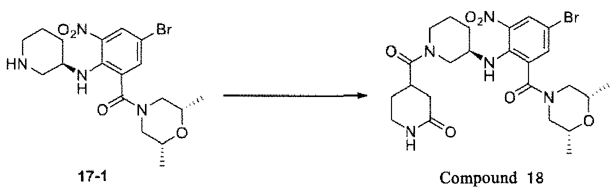

### Operating steps:

Step A: Compound 17-1 (30 mg, 0.068 mmol) was dissolved in *N,N*-dimethylformamide (1 mL) at room temperature under nitrogen protection. Subsequently, the above solution was cooled down to 0°C and 2-oxopiperidin-4-carboxylic acid (10 mg, 0.068 mmol), 2-(7-azabenzotriazol)-*N,N,N',N*'-tetramethyluronium hexafluorophosphate (39 mg, 0.78 mmol) and *N,N*-diisopropylethanamine (44 mg, 0.34 mmol) were added in sequence. The reaction system was stirred at 0°C for 30 min.

After the depletion of the raw material as monitored by LCMS, the reaction was quenched by adding water (10 mL), and the mixture solution was extracted with ethyl acetate (15 mL × 3 times). The organic phases were combined, and the resultant was washed with aqueous sodium chloride solution (10 mL × 2 times), then dried with anhydrous sodium sulfate, filtered and concentrated under reduced pressure. The resulting residue was purified by preparative high performance liquid chromatography to obtain the titled product Compound 18 (12 mg, 38.4% yield).

MS (ESI) M/Z: 566.2 [M+H]⁺.
¹H NMR (400 MHz, DMSO-*d*₆) δ 8.26 - 8.25 (m, 1H), 7.87 - 7.64 (m, 2H), 7.47 - 7.44 (m, 1H). 4.37 - 4.34 (m, 1H), 3.72 - 3.40 (m, 6H), 3.15 - 2.77 (m, 5H), 2.33 - 1.38 (m, 10H), 1.16 - 1.14 (m, 3H), 1.02 (s, 3H).

### Example 19

### (R)-(5-bromo-2-((1-(5-(methylamino)nicotinoyl)piperidin-3-yl)amino)-3-nitrophenyl)(4-morpholinopiperidin-1-yl)methanone

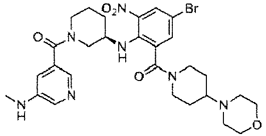

### Reaction route:

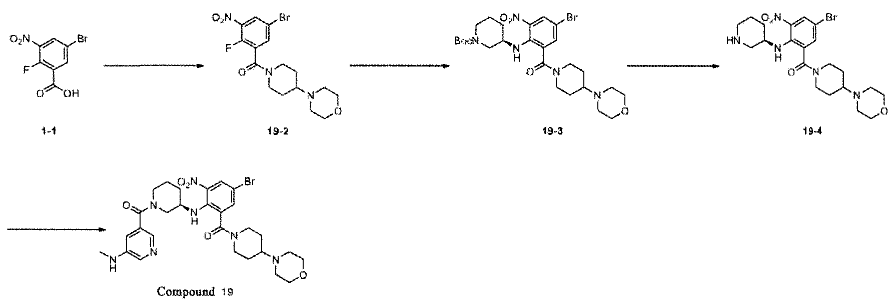

### Operating steps:

Step A: Compound 1-1 (150 mg, 0.57 mmol) was dissolved in *N,N*-dimethylformamide (3 mL) at room temperature. Subsequently, the above solution was cooled down to 0°C and *N,N*-diisopropylethylamine (205 mg, 1.59 mmol), 2-(7-azabenzotriazol)-*N,N*,*N*',*N*'-tetramethyluronium hexafluorophosphate (303 mg, 0.80 mmol), and 4-(4-piperidinyl)morpholine (97 mg, 0.57 mmol) were added in sequence. The reaction system was then stirred at 0°C for 40 min.

After the depletion of the raw material as monitored by LCMS, the reaction was quenched by adding water (15 mL), and the mixture solution was extracted with ethyl acetate (10 mL × 3 times). The organic phases were combined, and the resultant was washed with saturated saline (20 mL × 2 times), then dried with anhydrous sodium sulfate, filtered, and concentrated under reduced pressure. The resulting residue was purified by silica gel column chromatography to obtain Compound 19-2 (200 mg, 85% yield).

MS (ESI) M/Z: 416.0 [M+H]⁺.

Step B: Compound 19-2 (200 mg, 0.48 mmol) was dissolved in *N,N*-dimethylformamide (2 mL) at room temperature under nitrogen protection. Subsequently, tert-butyl (*R*)-3-aminopiperidin-1-carboxylate (116 mg, 0.58 mmol) and *N,N-*diisopropylethylamine (186 mg, 1.44 mmol) were sequentially added to the above reaction solution. The reaction system was then stirred at 100°C for 2 hours.

After the depletion of the raw material as monitored by LCMS, the reaction was quenched by adding water (40 mL), and the mixture solution was extracted with ethyl acetate (20 mL × 3 times). The organic phases were combined, and the resultant was washed with saturated saline (20 mL × 2 times), then dried with anhydrous sodium sulfate, filtered, and concentrated under reduced pressure. The resulting residue was purified by silica gel column chromatography to obtain Compound 19-3 (270 mg, 94% yield).

Step C: Compound 19-3 (270 mg, 0.45 mmol) was dissolved in 4 M hydrochloric acid-1,4-dioxane solution (1.2 mL) at room temperature under nitrogen protection. The reaction system was then stirred at room temperature for 1 hour.

After the depletion of the raw material as monitored by LCMS, the reaction solution was concentrated by vacuum distillation. Compound 19-4 (230 mg, 100 % yield) was obtained.

MS (ESI) M/Z: 496.2 [M+H]⁺.

Step D: Compound 19-4 (60 mg, 0.12 mmol) was dissolved in *N,N-*dimethylformamide (1 mL) at room temperature. Subsequently, the above solution was cooled down to 0°C and 5-methylaminonicotinic acid (15 mg, 0.10 mmol), 2-(7-azabenzotriazol)-*N,N*,*N*',*N*'-tetramethyluronium hexafluorophosphate (53 mg, 0.14 mmol), and *N,N*-diisopropylethylamine (65 mg, 0.50 mmol) were added in sequence. Stirring of the reaction system was then kept at 0°C for 30 min.

After the depletion of the raw material as monitored by LCMS, the reaction was quenched by adding water (15 mL), and the mixture solution was extracted with ethyl acetate (10 mL × 3 times). The organic phases were combined, and the resultant was washed with saturated saline (20 mL × 2 times), then dried with anhydrous sodium sulfate, filtered, and concentrated under reduced pressure. The resulting residue was purified by reversed-phase chromatography column to obtain the titled Compound 19 (12.6 mg, 20% yield).

MS (ESI) M/Z: 630.2 [M+H]⁺.
¹H NMR (400 MHz, DMSO-*d*₆) δ 8.25 (s, 1H), 8.04 - 7.42 (m, 4H), 6.75 (s, 1H), 6.16 (s, 1H). 4.56 - 4.28 (m, 1H), 4.17 - 3.40 (m. 9H), 3.26 - 2.97 (m, 2H). 2.93 - 2.76 (m, 1H), 2.69 (s, 3H), 2.48 - 2.20 (m. 5H), 1.97 - 1.20 (m, 8H).

### Example 20

### (5-Bromo-2-((R)-5-(5-(methylamino)nicotinoyl)-5-azaspiro[2.4]heptan-7-yl)amino)-3-nitrophenyl)((2R,6S)-2,6-dimethylmorpholinyl)methanone

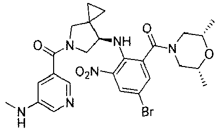

### Reaction route:

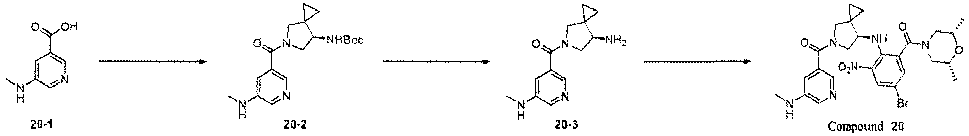

### Operating steps:

Step A: Compound 20-1 (60 mg, 0.39 mmol) was dissolved in *N,N-*dimethylformamide solution (1.0 mL) at room temperature under nitrogen protection. The above solution was cooled down to -10°C and *N,N,N',N'-tetramethyl-O-*(7-azabenzotriazol-1-yl)uronium hexafluorophosphate (187.4 mg, 0.49 mmol), *N*,*N*-diisopropylethylamine (212 mg, 1.64 mmol) and tert-butyl (*R*)-(5-azaspiro[2.4]heptan-7-yl)carbamate (65.1 mg, 0.30 mmol) were added in sequence. The reaction system was then stirred at -10°C for 10 min.

After the depletion of the raw material as monitored by TLC, the reaction was quenched by adding water (35 mL), and the mixture solution was extracted with ethyl acetate (10 mL × 3 times). The organic phases were combined, and the resultant was washed with saturated saline (15 mL × 2 times), then dried with anhydrous sodium sulfate, filtered, and concentrated under reduced pressure. The resulting residue was purified by silica gel column chromatography to obtain Compound 20-2 (100 mg, 94% yield).

MS (ESI) M/Z: 347.2 [M+H]⁺.
¹H NMR (400 MHz, DMSO-*d*₆) δ 7.98 (d, *J* = 2.8 Hz, 0.5H), 7.96 (d, *J=* 2.8 Hz, 0.5H). 7.86 (d, *J* = 1.6 Hz, 0.5H), 7.84 (d, *J* = 1.6 Hz, 0.5H), 7.37 - 7.17 (m, 1H), 7.00 - 6.85 (m, 1H), 6.21 - 6.02 (m, 1H), 3.85 - 3.56 (m, 3H), 3.51 - 3.41 (m. 1H), 3.28 - 3.14 (m, 1H). 2.71 (dd, *J* = 4.8, 2.7 Hz, 3H), 1.40 (s, 5H), 1.34 (s. 4H), 0.85 - 0.34 (m, 4H).

Step B: Compound 20-2 (100 mg, 0.28 mmol) was dissolved in a dry dichloromethane solution (1 mL) at room temperature under nitrogen protection. Subsequently, the above solution was cooled down to 0°C and 4 M hydrochloric acid-1,4-dioxane solution (1 mL) was added slowly and dropwise. The reaction system was then stirred at room temperature for 1 hour.

After the depletion of the raw material as monitored by LCMS, the reaction solution was concentrated under reduced pressure to obtain Compound 20-3 (50 mg, 61 % yield).

MS (ESI) M/Z: 247.2 [M+H]⁺.

Step C: Compound 20-3 (50 mg, 0.17 mmol) was dissolved in *N,N*-dimethylformamide (0.73 mL) at room temperature under nitrogen protection. Subsequently, (5-bromo-2-fluoro-3-nitrophenyl)((2*R*,6*S*)-2,6-dimethylmorpholinyl)methanone (53 mg, 0.14 mmol) and *N,N*-diisopropylethylamine (94.9 mg, 0.73 mmol) were added sequentially to the above reaction solution. The reaction system was then stirred at 80°C for 2 hours.

After the depletion of the raw material as monitored by LCMS, the reaction was quenched by adding water (10 mL), and the mixture solution was extracted with ethyl acetate (15 mL × 3 times). The organic phases were combined, and the resultant was washed with saturated saline (15 mL × 2 times), then dried with anhydrous sodium sulfate, filtered, and concentrated under reduced pressure. The resulting residue was purified by preparative high performance liquid chromatography to obtain the titled Compound 20 (21 mg, 20% yield).

MS (ESI) M/Z: 587.0 [M+H]⁺.
¹H NMR (400 MHz, DMSO-*d₆*) δ 8.33 - 8.17 (m, 1H), 7.98 (s, 1H). 7.90 - 7.45 (m, 3H), 6.93 - 6.64 (m, 1H), 6.12 (s, 1H). 4.44 - 4.16 (m, 1H), 3.93 - 3.38 (m, 7H), 3.23 - 2.61 (m. 6H). 1.23 - 0.50 (m, 10H).

### Example 21

### (5-Bromo-2-((R)-5-(5-(methylamino)nicotinoyl)-5-azaspiro[2.4]heptan-7-yl)amino)-3-nitrophenyl)((3R,5S)-3,5-dimethylpiperazin-1-yl)methanone

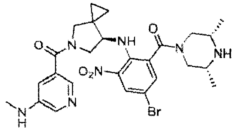

### Reaction route:

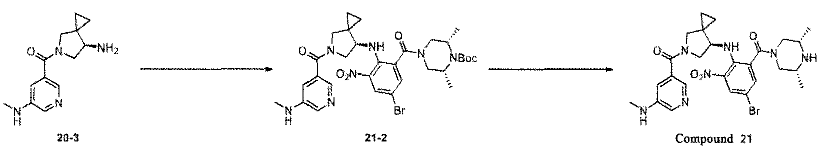

### Operating steps:

Step A: Compound 20-3 (50 mg, 0.18 mmol) was dissolved in *N,N*-dimethylformamide (0.9 mL) at room temperature under nitrogen protection. Subsequently, *N*,*N*-diisopropylethylamine (114 mg, 0.89 mmol) and tert-butyl (2*R*,6*S*)-4-(5-bromo-2-fluoro-3-nitrobenzoyl)-2,6-dimethylpiperazin-1-carboxylate (98 mg, 0.21 mmol) were sequentially added to the above solution. The reaction system was stirred at 80°C for 1 hour.

After the depletion of the raw material as monitored by LCMS, the reaction was quenched by adding water (10 mL). The reaction mixture solution was extracted with ethyl acetate (15 mL × 3). The organic phases were combined, and the resultant was washed with saturated saline (60 mL × 2 times), then dried with anhydrous sodium sulfate, filtered, and concentrated under reduced pressure. The resulting mixture was purified by silica gel column chromatography to obtain Compound 21-2 (100 mg, 82.3% yield).

MS (ESI) M/Z: 686.2 [M+H]⁺.

Step B: Compound 21-2 (100 mg, 0.15 mmol) was dissolved in dichloromethane solution (1.4 mL) at room temperature under nitrogen protection. Subsequently, 4 M hydrochloric acid-1,4-dioxane solution (0.74 mL) was added to the above solution. Stirring of the reaction system was then kept for 30 minutes.

After the depletion of the raw material as monitored by LCMS, the reaction solution was concentrated by vacuum distillation. The resulting crude product was dissolved in water (2 mL) and then adjusted with 25%wt aqueous ammonia to pH = 8. The resulting mixture solution was purified by reversed-phase chromatographic column to obtain the titled Compound 21 (34.6 mg, 40% yield).

MS (ESI) M/Z: 586.2 [M+H]⁺.
¹H NMR (400 MHz, DMSO-*d*₆) δ 8.35 - 8.15 (m, 1H), 8.05 - 7.92 (m, 1H), 7.88 - 7.49 (m, 3H). 6.89 - 6.70 (m, 1H), 6.20 - 6.04 (m. 1H). 4.45 - 4.13 (m, 1H), 3.86 - 3.36 (m, 4H). 3.24 - 2.61 (m, 7H). 2.39 - 1.84 (m, 2H). 1.05 - 0.61 (m, 10H).

### Example 22

### (R)-(5-bromo-2-(1-(5-(methylamino)nicotinoyl)piperidin-3-yl)-3-nitrophenyl)-(4-(2,2,2-trifluoroethyl)piperazin-1-yl)methanone

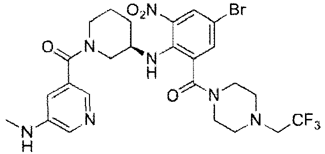

### Reaction route:

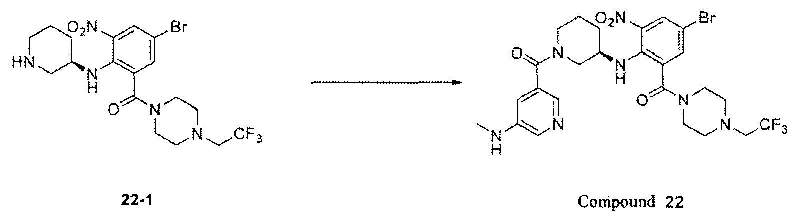

### Operating steps:

**Step A**: Compound 22-1 (30 mg, 0.061 mmol) was dissolved in *N,N*-dimethylformamide (1 mL) at room temperature. Subsequently, the above solution was cooled down to 0°C and 2-(7-azabenzotriazol)-*N,N,N',N*'-tetramethyluronium hexafluorophosphate (33 mg, 0.085 mmol), *N,N*-diisopropylethylamine (26 mg, 0.183 mmol), and 5-(methylamino)nicotinic acid (11 mg, 0.073 mmol) were added in sequence. The reaction system was stirred at 0°C for 2 hours.

After the depletion of the raw material as monitored by LCMS, the reaction was quenched by adding water (5 mL), and the mixture solution was extracted with ethyl acetate (6 mL × 3 times). The organic phases were combined, and the resultant was washed with saturated saline (8 mL × 2 times), then dried with anhydrous sodium sulfate, filtered, and concentrated under reduced pressure. The resulting residue was purified by preparative high performance liquid chromatography to obtain the titled Compound 22 (4.8 mg, 12.6% yield).

MS (ESI) M/Z: 628.0 [M+1]⁺.
¹H NMR (400 MHz, DMSO-*d*₆) δ 8.26 (s, 1H), 8.04 - 7.46 (m, 4H), 6.75 (s, 1H), 6.16 (s, 1H), 4.15 - 3.41 (m. 6H), 3.28 - 3.12 (m, 5H). 2.90 - 2.56 (in, 7H). 1.99 - 1.39 (m, 4H).

### Example 23

### (R)-1-(4-(5-bromo-2-((1-(5-(methylamino)nicotinoyl)piperidin-3-yl)amino)-3-nitrobenzoyl)piperazin-1-yl)-2,2,2-trifluoroethan-1-one

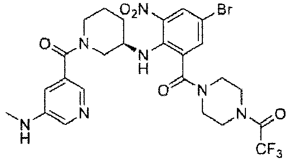

### Reaction route:

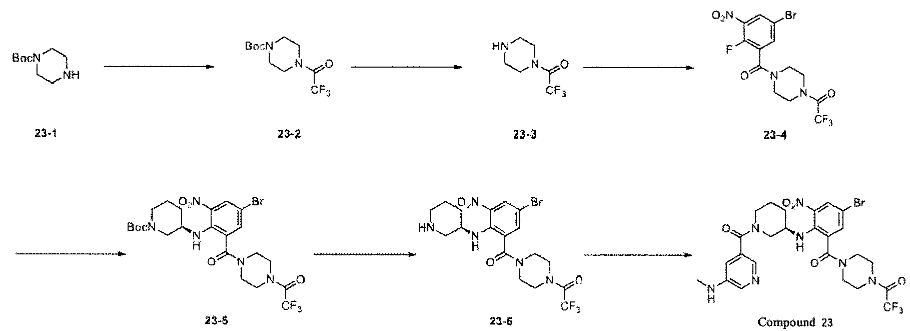

### Operating steps:

Step A: Compound 23-1 (500 mg, 2.69 mmol) was dissolved in dichloromethane (13 mL) at room temperature under nitrogen protection. Subsequently, the above solution was cooled down to 0°C and trifluoroacetic anhydride (846 mg, 4.03 mmol) and triethylamine (815 mg, 8.07 mmol) were added sequentially. The reaction system was then stirred at 0°C for 50 min.

After the depletion of the raw material as monitored by TLC, the reaction was quenched by adding water (15 mL), and the mixture solution was extracted with ethyl acetate (10 mL × 3 times). The organic phases were combined, and the resultant was washed with saturated saline (20 mL × 2 times), then dried with anhydrous sodium sulfate, filtered, and concentrated under reduced pressure. Compound 23-2 (500 mg, 66% yield) was obtained.

Step B: Compound 23-2 (600 mg, 2.12 mmol) was dissolved in 4 M hydrochloric acid-1,4-dioxane solution (5.0 mL) at room temperature under nitrogen protection. The reaction system was then stirred at room temperature for 1 hour.

After the depletion of the raw material as monitored by LCMS, the reaction solution was concentrated by vacuum distillation. Compound 22-3 (400 mg, 100 % yield) was obtained.

MS (ESI) M/Z: 183.2 [M+H]⁺.

Step C: The compound 5-bromo-2-fluoro-3-nitrobenzoic acid (200 mg, 0.75 mmol) was dissolved in *N,N-*dimethylformamide (4 mL) at room temperature. Subsequently, the above solution was cooled down to 0°C and *N,N-*diisopropylethylamine (484 mg, 3.75 mmol), 2-(7-azabenzotriazol)-*N,N*,*N*',*N*'-tetramethyluronium hexafluorophosphate (399 mg, 1.05 mmol), and Compound 23-3 (166 mg, 0.90 mmol) were added in sequence. The reaction system was then stirred at 0°C for 40 min.

After the depletion of the raw material as monitored by LCMS, the reaction was quenched by adding water (15 mL), and the mixture solution was extracted with ethyl acetate (10 mL × 3 times). The organic phases were combined, and the resultant was washed with saturated saline (20 mL × 2 times), then dried with anhydrous sodium sulfate, filtered, and concentrated under reduced pressure. The resulting residue was purified by silica gel column chromatography to obtain Compound 23-4 (160 mg, 50% yield).

MS (ESI) M/Z: 428.06 [M+H]⁺.

Step D: Compound 22-4 (160 mg, 0.37 mmol) was dissolved in *N,N*-dimethylformamide (2 mL) at room temperature under nitrogen protection. Subsequently, tert-butyl (R)-3-aminopiperidin-1-carboxylate (90 mg, 0.45 mmol) and *N,N-*diisopropylethylamine (143 mg, 1.11 mmol) were sequentially added to the above reaction solution. The reaction system was then stirred at 100°C for 2 hours.

After the depletion of the raw material as monitored by LCMS, the reaction was quenched by adding water (40 mL), and the mixture solution was extracted with ethyl acetate (20 mL × 3 times). The organic phases were combined, and the resultant was washed with saturated saline (20 mL × 2 times), then dried with anhydrous sodium sulfate, filtered, and concentrated under reduced pressure. The resulting residue was purified by silica gel column chromatography to obtain Compound 23-5 (200 mg, 94% yield).

MS (ESI) M/Z: 508.23 [M-99]⁺.

Step E: Compound 23-5 (200 mg, 0.33 mmol) was dissolved in 4 M hydrochloric acid-1,4-dioxane solution (0.80 mL) at room temperature under nitrogen protection. The reaction system was then stirred at room temperature for 1 hour.

After the depletion of the raw material as monitored by LCMS, the reaction solution was concentrated by vacuum distillation. Compound 23-6 (180 mg, 100 % yield) was obtained.

MS (ESI) M/Z: 508.0 [M+H]⁺.

Step F: Compound 23-6 (80 mg, 0.16 mmol) was dissolved in *N,N*-dimethylformamide (1 mL) at room temperature under nitrogen protection. Subsequently, the above solution was cooled down to 0°C and 5-methylaminonicotinic acid (20 mg, 0.13 mmol), 2-(7-azabenzotriazol)-*N,N,N',N*'-tetramethyluronium hexafluorophosphate (69 mg, 0.18 mmol), and *N,N-*diisopropylethylamine (84 mg, 0.65 mmol) were added in sequence. The reaction system was then stirred at 0°C for 30 min.

After the depletion of the raw material as monitored by LCMS, the reaction was quenched by adding water (15 mL), and the mixture solution was extracted with ethyl acetate (10 mL × 3 times). The organic phases were combined, and the resultant was washed with saturated saline (20 mL × 2 times), then dried with anhydrous sodium sulfate, filtered, and concentrated under reduced pressure. The resulting residue was purified by reversed-phase chromatography column to obtain the titled Compound 23 (25.7 mg, 25% yield).

MS (ESI) M/Z: 642.0 [M+H]⁺.
¹H NMR (400 MHz, DMSO-*d*₆) δ 8.29 (s, 1H). 8.04 - 7.49 (m, 4H), 6.76 (s, 1H), 6.15 (s, 1H), 4.04 - 3.36 (m, 12H), 3.25 - 3.07 (m, 1H), 2.69 (d, *J* = 1.6 Hz. 3H), 1.97 - 1.67 (m, 2H), 1.66 - 1.24 (m, 2H).

### Example 24

### (R)-4-(3-((4-bromo-2-(4,4-difluoropiperidin-1-carbonyl)-6-nitrophenyl)amino)piperidin-1-carbonyl)pyridin-2(1H)-one

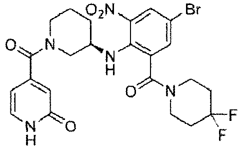

### Reaction route:

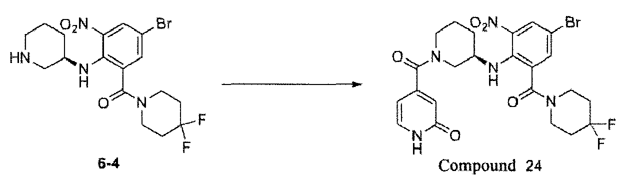

### Operating steps:

**Step A:** Compound 2-oxo-1,2-dihydropyridin-4-carboxylic acid (13 mg, 0.09 mmol) was dissolved in *N,N-*dimethylformamide (0.45 mL) at room temperature under nitrogen protection. Subsequently, the above solution was cooled down to 0°C and 2-(7-azabenzotriazol)-*N,N,N',N*'-tetramethyluronium hexafluorophosphate (48 mg, 0.11 mmol), *N,N-*diisopropylethylamine (35 mg, 0.27 mmol), and Compound 6-4 (50 mg, 0.11 mmol) were added in sequence. The reaction system was then stirred at 0°C for 10 min.

After the depletion of the raw material as monitored by LCMS, the reaction was quenched by adding water (10 mL), and the mixture solution was extracted with ethyl acetate (10 mL × 3 times). The organic phases were combined, and the resultant was washed with saturated aqueous sodium chloride solution (30 mL × 2 times), then dried with anhydrous sodium sulfate, filtered and concentrated under reduced pressure. The resulting residue was purified by preparative high performance liquid chromatography to obtain the titled Compound 24 (11.7 mg, 22.9% yield).

MS (ESI) M/Z: 568.0 [M+H]⁺.
¹H NMR (400 MHz, DMSO-*d*₆) δ 11.75 (s, 1H), 8.44 - 8.18 (in, 1H), 7.89 - 7.61 (m, 2H), 7.48 (d, *J* = 6.4 Hz, 0.7H), 7.32 (s, 0.3H), 6.22 (s, 0.7H), 6.17 - 5.99 (m, 1H), 5.92 (d, *J* = 5.2 Hz, 0.3H), 4.00 - 3.47 (m, 7H), 3.26 - 2.96 (m, 2H), 2.25 - 1.33 (m, 8H).

### Example 25

### N-((1S,2R)-2-((4-bromo-2-nitro-6-(3-oxopiperazin-1-carbonyl)phenyl)amino)cyclohexyl)-7-chloroisoquinolin-4-formamide

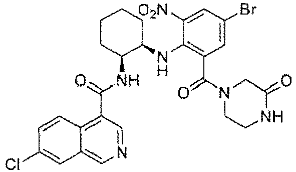

### Reaction route:

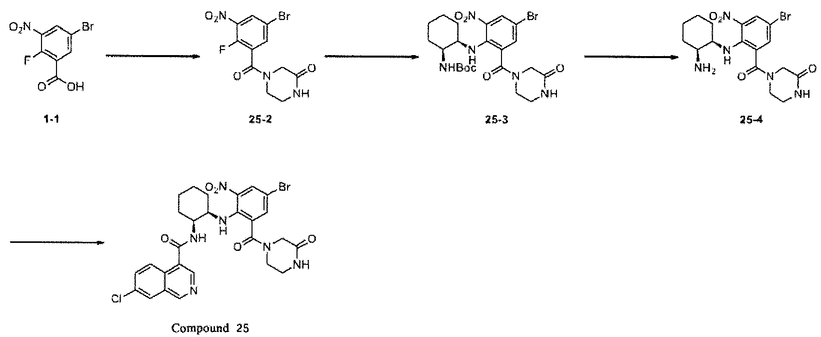

### Operating steps:

Step A: Compound 1-1 (100 mg, 0.39 mmol) and 2-piperazinone (38 mg, 0.39 mmol) were dissolved in *N,N-*dimethylformamide (1 mL) at room temperature under nitrogen protection. Subsequently, the above solution was cooled down to 0°C and 2-(7-azabenzotriazol)-*N,N,N',N*'-tetramethyluronium hexafluorophosphate (216 mg, 0.57 mmol) and *N,N-*diisopropylethylamine (245 mg, 1.89 mmol) were added sequentially. Stirring of the reaction system was then kept at 0°C for 30 min.

After the depletion of the raw material as monitored by LCMS, the reaction was quenched by adding water (20 mL), and the mixture solution was extracted with ethyl acetate (10 mL × 3 times). The organic phases were combined, and the resultant was washed with saturated saline (20 mL × 2 times), then dried with anhydrous sodium sulfate, filtered, and concentrated under reduced pressure. The resulting residue was purified by silica gel column chromatography to obtain Compound 25-2 (30 mg, 22.9% yield).

MS (ESI) M/Z: 346.1 [M+H]⁺.

Step B: Compound 25-2 (30 mg, 0.087 mmol) was dissolved in *N,N*-dimethylformamide (1 mL) at room temperature under nitrogen protection. Subsequently, tert-butyl ((1*S*,2*R*)-2-aminocyclohexyl) carbamate (19 mg, 0.087 mmol) and *N,N-*diisopropylethylamine (56 mg, 0.433 mmol) were added sequentially to the above reaction solution. The reaction system was then raised to 80°C and stirred for 4 hours.

After the depletion of the raw material as monitored by LCMS, the reaction was quenched by adding water (10 mL), and the mixture solution was extracted with ethyl acetate (10 mL × 3 times). The organic phases were combined, and the resultant was washed with saturated saline (20 mL × 2 times), then dried with anhydrous sodium sulfate, filtered, and concentrated under reduced pressure. Compound 25-3 (32 mg, 68.3% yield) was obtained.

MS (ESI) M/Z: 440.3 [M+H]⁺.

Step C: Compound 25-3 (160 mg, 0.27 mmol) was dissolved in dichloromethane (10 mL) at room temperature. Subsequently, the above solution was cooled down to 0°C and added dropwise to a 4 M hydrochloric acid-1,4-dioxane solution (3 mL). The reaction system was then stirred at room temperature for 30 minutes.

After the depletion of the raw material as monitored by LCMS, the reaction solution was concentrated under reduced pressure to obtain Compound 25-4 (130 mg, crude product).

MS (ESI) M/Z: 440.3 [M+H]⁺.

Step D: Compound 25-4 (23 mg, 0.048 mmol) and 7-chloroisoquinolin-4-carboxylic acid (10 mg, 0.048 mmol) were dissolved in *N,N*-dimethylformamide (1 mL) at room temperature under nitrogen protection. Subsequently, the above solution was cooled down to 0°C and 2-(7-azabenzotriazol)-*N,N,N',N*'-tetramethyluronium hexafluorophosphate (27.47 mg, 0.072 mmol) and *N,N*-diisopropylethylamine (32 mg, 0.241 mmol) were added in turn. The reaction system was then stirred at 0°C for 30 min.

After the depletion of the raw material as monitored by LCMS, the reaction was quenched by adding water (20 mL), and the mixture solution was extracted with ethyl acetate (6 mL × 3 times). The organic phases were combined, and the resultant was washed with saturated saline (20 mL × 2 times), then dried with anhydrous sodium sulfate, filtered, and concentrated under reduced pressure. The resulting residue was purified by reversed-phase chromatography column to obtain the titled Compound 25 (11.8 mg, 38.9 % yield).

MS (ESI) M/Z: 629.1 [M+H]⁺.
¹H NMR (400 MHz. DMSO-*d*₆) δ 9.38 (s. 1H). 8.08 - 8.60 (m, 1H), 8.54 (s, 1H), 8.34 (s, 1H). 8.27 (s, 1H), 8.21 (s, 1H), 8.15 - 7.95 (m, 2H), 7.81 (dd, *J* = 9.0, 1.8 Hz, 1H), 7.70 (s, 0.5H). 7.65 (s. 0.5H), 4.45 - 4.33 (m, 1H), 4.28 - 4.04 (m, 1H), 4.03 - 3.64 (m, 3H), 3.61 -3.46 (m, 1H). 3.27 - 3.09 (m. 2H), 1.84 - 1.48 (m. 6H), 1.47 - 1.27 (m, 2H).

### Example 26

### (R)-4-(3-((4-bromo-2-(morpholin-4-carbonyl)-6-nitrophenyl)amino)piperidin-1-carbonyl)-6-methylpyridin-2(1H)-one

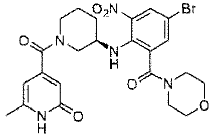

### Reaction route:

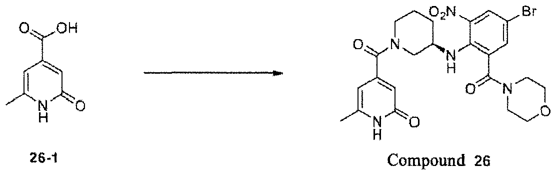

### Operating steps:

**Step A:** Compound 26-1 (15 mg, 0.10 mmol) was dissolved in *N,N*-dimethylformamide (0.50 mL) at room temperature under nitrogen protection. Subsequently, the above solution was cooled down to 0°C and 2-(7-azabenzotriazol)-*N,N,N*'*,N*'-tetramethyluronium hexafluorophosphate (53 mg, 0.14 mmol), *N,N-*diisopropylethylamine (39 mg, 0.30 mmol) and compound (*R*)-(5-bromo-3-nitro-2-(piperidin-3-ylamino)phenyl)(morpholinyl)methanone (50 mg, 0.12 mmol) were added in sequence. The reaction system was then stirred at 0°C for 10 min.

After the depletion of the raw material as monitored by LCMS, the reaction was quenched by adding water (10 mL), and the mixture solution was extracted with ethyl acetate (10 mL × 3 times). The organic phases were combined, and the resultant was washed with saturated aqueous sodium chloride solution (30 mL × 2 times), then dried with anhydrous sodium sulfate, filtered and concentrated under reduced pressure. The resulting residue was purified by reversed-phase chromatographic column to obtain the titled Compound 26 (6.7 mg, 12.2% yield).

MS (ESI) M/Z: 548.0 [M+H]⁺.
¹H NMR (400 MHz, DMSO-*d*₆) δ 11.77 (s, 1H), 8.27 (s, 1H), 7.85 - 7.57 (m, 2H), 6.03 ((s, 0.7H), 5.95 - 5.79 (m, 1H), 5.70 - 5.53 (m, 0.3H), 4.04 - 3.49 (m, 11H). 3.25 - 3.08 (m, 2H), 2.19 (s, 2H), 2.08 (s, 1H). 1.97 - 1.32 (m, 4H).

### Example 27

### 4-((R)-3-((4-bromo-2-((2S,6R)-2,6-dimethylmorpholin-4-carbonyl)-6-nitrophenyl)amino)piperidin-1-carbonyl)-6-methylpyridin-2(1H)-one

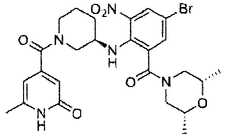

### Reaction route:

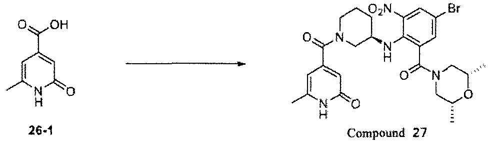

### Operating steps:

Step A: Compound 26-1 (20 mg, 0.13 mmol) was dissolved in *N,N*-dimethylformamide (0.65 mL) at room temperature under nitrogen protection. Subsequently, the above solution was cooled down to 0°C and 2-(7-azabenzotriazol)-*N,N,N*'*,N*'-tetramethyluronium hexafluorophosphate (49 mg, 0.18 mmol), *N,N*-diisopropylethylamine (50 mg, 0.39 mmol) and (5-bromo-3-nitro-2-((*R*)-piperidin-3-yl)amino)phenyl ((2R,6S)-2,6-dimethylmorpholinyl)methanone (48 mg, 0.11 mmol). Stirring of the reaction system was then kept at 0°C for 10 min.

After the depletion of the raw material as monitored by LCMS, the reaction was quenched by adding water (10 mL), and the mixture solution was extracted with ethyl acetate (10 mL × 3 times). The organic phases were combined, and the resultant was washed with saturated saline (30 mL × 2 times), then dried with anhydrous sodium sulfate, filtered, and concentrated under reduced pressure. The resulting residue was purified by reversed-phase chromatography column to obtain the titled Compound 27 (36.2 mg, 57.2% yield).

MS (ESI) M/Z: 576.0 [M+H]⁺.
¹H NMR (400 MHz, DMSO-*d*₆) δ 11.75 (s, 1H), 8.27 (s, 1H), 7.88 - 7.51 (m, 2H), 6.10 - 5.56 (m, 2H), 4.45 - 4.23 (m, 1H), 3.93 - 3.36 (m, 7H), 3.27 - 3.00 (m, 2H), 2.94 - 2.72 (m, 1H), 2.18 (s, 2H), 2.09 (s, 1H), 1.97 - 1.30 (m, 4H). 1.22 - 0.91 (m, 6H).

### Example 28

### (R)-(2-((1-(1-(1hydro-pyrrolo[2,3-c]pyridin-4-carbonyl)piperidin-3-yl)amino)-5-bromo-3-nitrophenyl)(4,4-difluoropiperidin-1-yl)methanone

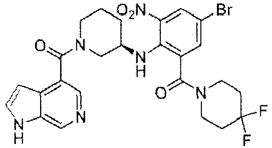

### Reaction route:

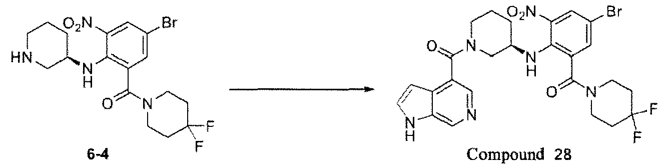

### Operating steps:

Step A: Compound 6-4 (34 mg, 0.077 mmol) was dissolved in *N,N*-dimethylformamide (1 mL) at room temperature under nitrogen protection. Subsequently, the above solution was cooled down to 0°C and 2-oxopiperidin-4-carboxylic acid (12 mg, 0.077 mmol), 2-(7-azabenzotriazol)-*N,N,N',N*'-tetramethyluronium hexafluorophosphate (44 mg, 0.12 mmol) and *N,N*-diisopropylethanamine (50 mg, 0.39 mmol) were added in sequence. The reaction system was stirred at 0°C for 30 min.

After the depletion of the raw material as monitored by LCMS, the reaction was quenched by adding water (15 mL), and the mixture solution was extracted with ethyl acetate (5 mL × 3 times). The organic phases were combined, and the resultant was washed with aqueous sodium chloride solution (20 mL × 2 times), then dried with anhydrous sodium sulfate, filtered and concentrated under reduced pressure. The resulting residue was purified by preparative high performance liquid chromatography to obtain the title product Compound 28 (12.3 mg, 27.0% yield).

MS (ESI) M/Z: 591.0 [M+H]⁺.
¹H NMR (400 MHz, DMSO-*d*₆) δ 11.83 (s, 1H). 8.76 (s, 1H), 8.25 - 7.69 (m, 5H), 6.39 (s, 1H), 4.00 - 3.24 (m, 9H), 2.06 - 1.89 (m, 5H) 1.70 - 1.54 (m, 3H).

### Example 29

### (R)-(5-bromo-2-((1-(5-(methylamino)nicotinoyl)piperidin-3-yl)amino)-3-nitrophenyl)(4-methylpiperazin-1-yl)methanone

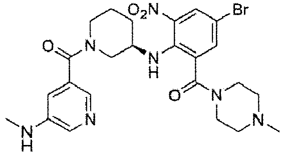

### Reaction route:

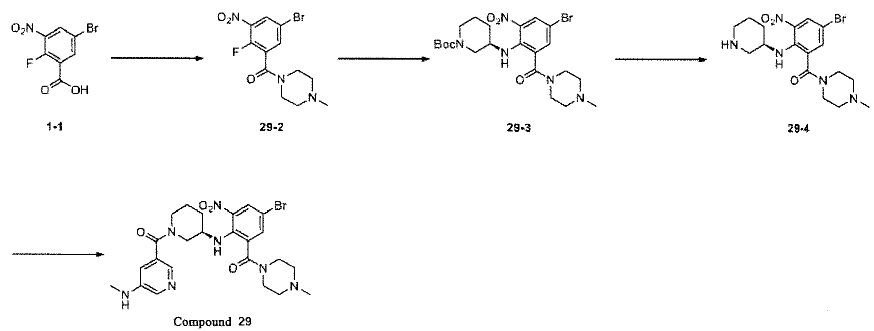

### Operating steps:

Step A: Compound 1-1 (300 mg, 1.14 mmol) and 1-methylpiperazine (125 mg, 1.25 mmol) were dissolved in *N,N-*dimethylformamide (1 mL) at room temperature under nitrogen protection. Subsequently, the above solution was cooled down to 0°C and 2-(7-azabenzotriazol)-*N,N,N',N*'-tetramethyluronium hexafluorophosphate (648 mg, 1.71 mmol) and *N,N-*diisopropylethylamine (587 mg, 4.55 mmol) were added sequentially. Stirring of the reaction system was then kept for 30 min at 0°C.

After the depletion of the raw material as monitored by LCMS, the reaction was quenched by adding water (20 mL), and the mixture solution was extracted with ethyl acetate (10 mL × 3 times). The organic phases were combined, and the resultant was washed with saturated saline (20 mL × 2 times), then dried with anhydrous sodium sulfate, filtered, and concentrated under reduced pressure. The resulting residue was purified by silica gel column chromatography to obtain Compound 29-2 (195 mg, 49.57% yield).

MS(ESI) M/Z: 346.1 [M+H]⁺.

Step B: Compound 29-2 (200 mg, 0.58 mmol) was dissolved in *N,N*-dimethylformamide (1 mL) at room temperature under nitrogen protection. Subsequently, tert-butyl (R)-3-aminopiperidin-1-carboxylate (115.7 mg, 0.58 mmol) and *N,N-*diisopropylethylamine (224 mg, 1.73 mmol) were sequentially added to the above reaction solution. The reaction system was then warmed to 80°C and stirred for 4 hours.

After the depletion of the raw material as monitored by LCMS, the reaction was quenched by adding water (10 mL), and the mixture solution was extracted with ethyl acetate (10 mL × 3 times). The organic phases were combined, and the resultant was washed with saturated saline (20 mL × 2 times), then dried with anhydrous sodium sulfate, filtered, and concentrated under reduced pressure. Compound 29-3 (355 mg, crude product) was obtained.

MS(ESI) M/Z: 526.4 [M+H]⁺.

Step C: Compound 29-3 (355 mg, 0.67 mmol) was dissolved in dichloromethane (10 mL) at room temperature. Subsequently, the above solution was cooled down to 0°C and 4 M hydrochloric acid-1,4-dioxane solution (5 mL) was added slowly and dropwise. The reaction system was then stirred at room temperature for 30 minutes.

After the depletion of the raw material as monitored by LCMS, the reaction solution was concentrated under reduced pressure to obtain Compound 29-4 (320 mg, crude product).

MS (ESI) M/Z: 426.3 [M+H]⁺.

Step D: Compound 29-4 (60 mg, 0.13 mmol) and 5-(methylamino)nicotinic acid (19.7 mg, 0.13 mmol) were dissolved in *N,N*-dimethylformamide (1 mL) at room temperature under nitrogen protection. Subsequently, the above solution was cooled down to 0°C and 2-(7-azabenzotriazol)-*N,N,N',N*'-tetramethyluronium hexafluorophosphate (74 mg, 0.194 mmol) and *N,N*-diisopropylethylamine (83.8 mg, 0.65 mmol) were added in turn. The reaction system was then stirred at 0 °C for 30 min.

After the depletion of the raw material as monitored by LCMS, the reaction was quenched by adding water (20 mL), and the mixture solution was extracted with ethyl acetate (6 mL × 3 times). The organic phases were combined, and the resultant was washed with saturated saline (20 mL × 2 times), then dried with anhydrous sodium sulfate, filtered, and concentrated under reduced pressure. The resulting residue was purified by reversed-phase chromatography column to obtain the titled Compound 29 (27.5 mg, 37.9% yield).

MS (ESI) M/Z: 560.0 [M+H]⁺.
¹H NMR (400 MHz, DMSO-*d*₆) δ 8.26 (s, 1H), 8.07 - 7.43 (m, 4H). 6.75 (s, 1H), 6.16 (s, 1H), 4.07 - 3.46 (m, 7H), 3.29 - 3.11 (m, 2H), 2.70 (s, 3H). 2.44 - 2.00 (m, 6.5H). 1.99 - 1.29 (m, 4.5H).

### Example 30

### (5-Bromo-2-((R)-1-(5-(methyl)nicotinoyl)piperidin-3-yl)amino)-3-nitrobenzoyl)piperazin-1-yl-2,2,2-trifluoroethan-1-one

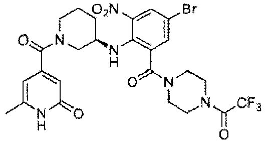

### Reaction route:

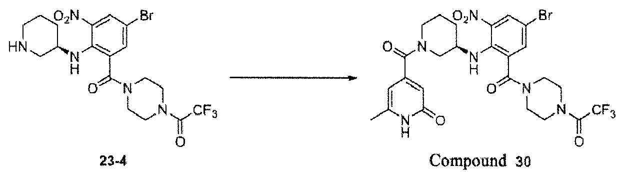

### Operating steps:

Step A: Compound 23-4 (80 mg, 0.16 mmol) was dissolved in *N,N*-dimethylformamide (1 mL) at room temperature. Subsequently, the above solution was cooled down to 0°C, and 6-methyl-2-oxo-1,2-dihydropyridin-4-carboxylic acid (20 mg, 0.13 mmol), 2-(7-azabenzotriazol)-*N,N,N',N*'-tetramethyluronium hexafluorophosphate (69 mg, 0.18 mmol), and *N,N-*diisopropylethylamine (84 mg, 0.65 mmol) were added in sequence. The reaction system was then stirred at 0°C for 30 min.

After the depletion of the raw material as monitored by LCMS, the reaction was quenched by adding water (15 mL), and the mixture solution was extracted with ethyl acetate (5 mL × 3 times). The organic phases were combined, and the resultant was washed with saturated saline (20 mL × 2 times), then dried with anhydrous sodium sulfate, filtered, and concentrated under reduced pressure. The resulting residue was purified by reversed-phase chromatography column to obtain the titled Compound 30 (22.7 mg, 27% yield).

MS (ESI) M/Z: 643.0 [M+H]⁺.
¹H NMR (400 MHz, DMSO-*d*₆) δ 11.77 (s, 1H), 8.34 - 8.22 (m, 1H), 7.87 - 7.59 (m, 2H), 6.04 (s, 0.7H), 5.91 (s, 0.3H). 5.87 (s, 0.7H). 5.63 (s. 0.3H), 3.90 - 3.36 (m, 12H), 3.26 - 3.11 (m, 1H), 2.18 (s. 2H), 2.08 (s, 1H), 1.93 - 1.64 (m, 2H). 1.62 - 1.32 (m, 2H).

### Example 31

### (R)-(5-bromo-2-((1-(5-(methylamino)nicotinoyl)piperidin-3-yl)amino)-3-nitrophenyl)(piperidin-1-yl)methanone

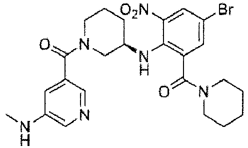

### Reaction route:

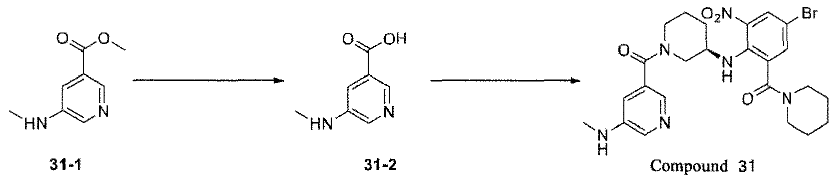

### Operating steps:

Step A: Compound 31-1 (80 mg, 0.48 mmol) was dissolved in a solvent mixture of tetrahydrofuran (2 mL) and water (0.5 mL) at room temperature. Subsequently, lithium hydroxide monohydrate (40 mg, 0.96 mmol) was added to the above reaction solution. The reaction system was stirred at room temperature for 1 hour.

After the depletion of the raw material as monitored by LCMS, the reaction solution was diluted by adding water (2 mL), the mixture solution was adjusted to pH=5 with 1 M dilute hydrochloric acid, and then concentrated under reduced pressure. Compound 31-2 (80 mg, 100% yield) was obtained.

MS (ESI) M/Z: 153.05 [M+H]⁺.

Step B: Compound 31-2 (50 mg, 0.33 mmol) was dissolved in *N,N*-dimethylformamide (1 mL) at room temperature under nitrogen protection. Subsequently, the above solution was cooled down to 0°C and (*R*)-(5-bromo-3-nitro-2-(piperidin-3-ylamino)phenyl)(piperidin-1-yl)methanone (162 mg, 0.26 mmol), 2-(7-azabenzotriazol)-*N,N,N',N*'-tetramethyluronium hexafluorophosphate (108 mg, 0.46 mmol), and *N,N*-diisopropylethylamine (213 mg, 1.65 mmol) were added in sequence. The reaction system was stirred at 0°C for 30 min.

After the depletion of the raw material as monitored by LCMS, the reaction was quenched by adding water (15 mL), and the mixture solution was extracted with ethyl acetate (5 mL × 3 times). The organic phases were combined, and the resultant was washed with saturated saline (20 mL × 2 times), then dried with anhydrous sodium sulfate, filtered, and concentrated under reduced pressure. The resulting residue was purified by reversed-phase chromatography column to obtain the titled Compound 31 (21.5 mg, 12% yield).

MS (ESI) M/Z: 545.1 [M+H]⁺.
¹H NMR (400 MHz, DMSO-*d*₆) δ 8.24 (s, 1H), 8.03 - 7.78 (m, 2H), 7.73 - 7.42 (m, 2H), 6.86 - 6.56 (m. 1H), 6.26 - 5.89 (m, 1H), 4.03 - 3.43 (m, 6H), 3.25 - 3.04 (m, 3H), 2.70 (s. 3H), 1.89 (s, 1H). 1.79 - 1.22 (m, 9H).

### Example 32

### (5-Bromo-2-((4,4-difluoro-1-(5-(methylamino)nicotinoyl)pyrrolidin-3-yl)amino)-3-nitrophenyl)(piperidin-1-yl)methanone

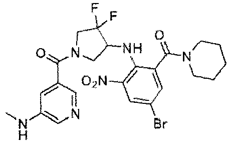

### Reaction route:

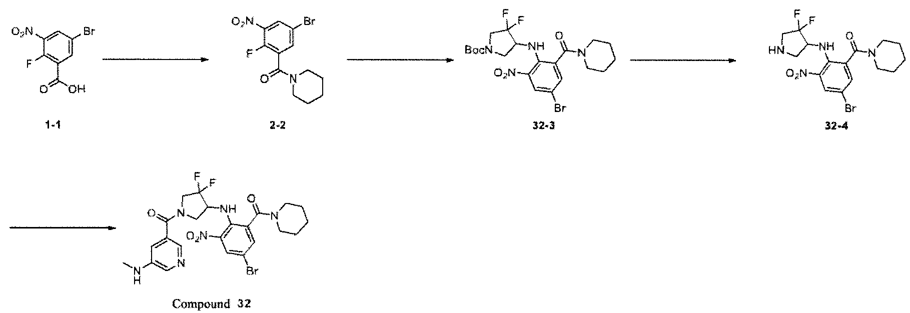

### Operating steps:

Step A: Compound 1-1 (3 g, 11.4 mmol) was dissolved in *N,N*-dimethylformamide (57 mL) under nitrogen protection at room temperature. Then, *N,N*-diisopropylethylamine (4.42 g, 34.2 mmol), 2-(7-azabenzotriazol)-*N,N,N*'*,N*'-tetramethyluronium hexafluorophosphate (6.08 g, 16 mmol) and piperidine (1.17 g, 13.7 mmol) were added to the above solution under an ice-salt bath. Stirring of the reaction system was then kept under ice salt bath for 1 hour.

After the depletion of the raw material as monitored by LCMS, the reaction was quenched by adding water (10 mL), and the mixture solution was extracted with ethyl acetate (15 mL × 3 times). The organic phases were combined, and the resultant was washed with saturated saline (60 mL × 2 times), dried with anhydrous sodium sulfate, filtered, and concentrated under reduced pressure. Compound 2-2 (1.7 g, 45% yield) was obtained.

MS (ESI) M/Z: 332.0 [M+H]⁺.

Step B: Compound 2-2 (350 mg, 1.06 mmol) was dissolved in *N*,*N*-dimethylformamide (5.3 mL) at room temperature under nitrogen protection. Subsequently, tert-butyl 4-amino-3,3-difluoropyrrolidin-1-carboxylate (282.5 mg, 1.27 mmol) and *N,N*-diisopropylethylamine (410.2 mg, 3.18 mmol) were added to the above solution. The reaction system was then stirred at 100°C for 16 hours.

After the depletion of the raw material as monitored by LCMS, the reaction system was quenched by adding water (35 mL), and the mixture solution was extracted with ethyl acetate (10 mL × 3 times). The organic phases were combined, and the resultant was washed with saturated saline (15 mL × 2 times), then dried with anhydrous sodium sulfate, filtered, and finally concentrated under reduced pressure. The resulting residue was purified by silica gel column chromatography to obtain Compound 32-3 (320 mg, 57% yield).

MS (ESI) M/Z: 555.0 [M+23]⁺.

Step C: Compound 32-3 (320 mg, 0.60 mmol) was dissolved in a dry dichloromethane solution (3 mL) under nitrogen protection at room temperature. Subsequently, the above solution was cooled down to 0°C and a 4 M hydrochloric acid-1,4-dioxane solution (1.5 mL) was added slowly and dropwise. The reaction system was then stirred at room temperature for 1 hour.

After the depletion of the raw material as monitored by LCMS, the reaction solution was concentrated by vacuum distillation. Compound 32-4 (260 mg, 100% yield) was obtained.

MS (ESI) M/Z: 433.0 [M+H]⁺.

Step D: Compound 5-(methylamino)nicotinic acid (5.4 mg, 0.03 mmol) was dissolved in *N,N*-dimethylformamide solution (0.2 mL) at room temperature under nitrogen protection. Subsequently, the above solution was cooled down to -10°C and *N,N,N',N*'-tetramethyl-*O*-(7-azabenzotriazol-1-yl)uronium hexafluorophosphate (20 mg, 0.05 mmol), *N,N-*diisopropylethylamine (22.9 mg, 0.17 mmol), and Compound 32-4 (20 mg, 0.04 mmol) were added in sequence. The reaction system was then stirred at -10°C for 1 hour.

After the depletion of the raw material as monitored by LCMS, the reaction was quenched by adding water (10 mL), and the mixture solution was extracted with ethyl acetate (15 mL × 3 times). The organic phases were combined, and the resultant was washed with saturated saline (15 mL × 2 times), then dried with anhydrous sodium sulfate, filtered, and concentrated under reduced pressure. The resulting residue was purified by preparative high performance liquid chromatography to obtain the titled Compound 32 (2.3 mg, 11.5% yield).

MS (ESI) M/Z: 567.0 [M+H]⁺.
¹H NMR (400 MHz, DMSO-*d*₆) δ 8.25 (s, 1H), 8.03 (s, 1H), 7.87 (s, 1H), 7.80 - 7.50 (m, 1.6H), 7.18 (s, 0.4H), 6.93 (s, 1H), 6.18 (s, 1H), 4.46 - 3.51 (m, 6H), 3.27- 2.83 (m, 3H), 2.72 (d, *J* = 5.2 Hz, 3H), 1.71 - 1.07 (m, 6H).

### Example 33

### (4R,6S)-4-((R)-3-((4-bromo-2-((S)-3-methylpiperidin-1-carbonyl)-6-nitrophenyl)amino)piperidin-1-carbonyl)-6-methylpiperidin-2-one

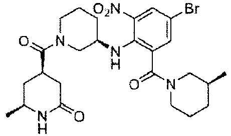

### Reaction route:

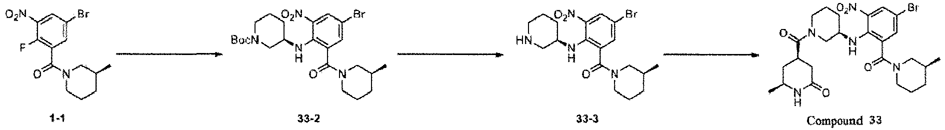

### Operating steps:

**Step A:** Compound 1-1 (37 mg, 0.11 mmol) was dissolved in *N,N*-dimethylformamide (0.54 mL) at room temperature. Subsequently, (*R*)-1-Boc-3-aminopiperidine (26 mg, 0.13 mmol) and *N,N*-diisopropylethylamine (0.05 mL, 0.32 mmol) were added. The reaction system was then stirred at 80°C for 1 hour.

After the depletion of the raw material as monitored by LCMS, the reaction was quenched by adding water (15 mL), and the mixture solution was extracted with ethyl acetate (20 mL × 3 times). The organic phases were combined, and the resultant was washed with saturated aqueous sodium chloride solution (20 mL), then dried with anhydrous sodium sulfate, filtered, and concentrated under reduced pressure. The resulting residue was purified by silica gel column chromatography to obtain Compound 33-2 (50 mg).

**Step B:** Compound 33-2 (50 mg, 0.10 mmol) was dissolved in dichloromethane (0.5 mL) at room temperature. Subsequently, the temperature was lowered to 0°C and 4 M hydrochloric acid-1,4-dioxane solution (0.7 mL, 2.86 mmol) was added slowly and dropwise to the reaction solution. The reaction system was then brought to room temperature and stirring was continued for 1 hour.

After the depletion of the raw material as monitored by LCMS, the reaction solution was concentrated by vacuum distillation. Compound 33-3 was obtained as crude product (45 mg).

MS (ESI) M/Z: 425.0 [M+H]⁺.

**Step C:** (2*S*,4*R*)-2-methyl-6-oxopiperidin-4-carboxylic acid (19 mg, 0.12 mmol) was dissolved in *N,N-*dimethylformamide (0.44 mL) at room temperature under nitrogen protection. Subsequently, *N,N*-diisopropylethylamine (0.06 mL, 0.35 mmol), 2-(7-azabenzotriazol)-*N,N,N',N*'-tetramethyluronium hexafluorophosphate (37 mg, 0.10 mmol), and Compound 33-3 (45 mg, 0.11 mmol) were sequentially added to the above reaction solution. The reaction system was then stirred at room temperature for 20 minutes.

After the depletion of the raw material as monitored by LCMS, the reaction was quenched by adding water (10 mL), and the mixture solution was extracted with ethyl acetate (20 mL × 3 times). The organic phases were combined, and the resultant was washed with saturated aqueous sodium chloride solution (20 mL × 2 times), then dried with anhydrous sodium sulfate, filtered and concentrated under reduced pressure. The resulting residue was purified by reversed-phase column to obtain the titled Compound 33 (16.6 mg).

MS (ESI) M/Z: 564.2 [M+H]⁺.
¹H NMR (400 MHz, DMSO-*d*₆) δ 8.23 (s, 1H), 7.84 - 7.43 (in, 3H), 4.45 - 4.21 (m, 1H), 4.00 - 3.34 (m, 6H), 3.22 - 2.73 (m, 3H). 2.49 - 2.40 (in, 1H), 2.16 - 1.99 (m, 2H), 1.97 - 1.08 (m, 14H), 1.01 - 0.67 (m, 3H).

### Example 34

### (5-Bromo-2-((R)-1-(5-(methylamino)nicotinoyl)piperidin-3-yl)amino)-3-nitrophenyl((2R,6S)-2,6-dimethylmorpholinyl)methanone

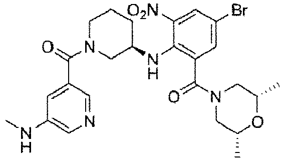

### Reaction route:

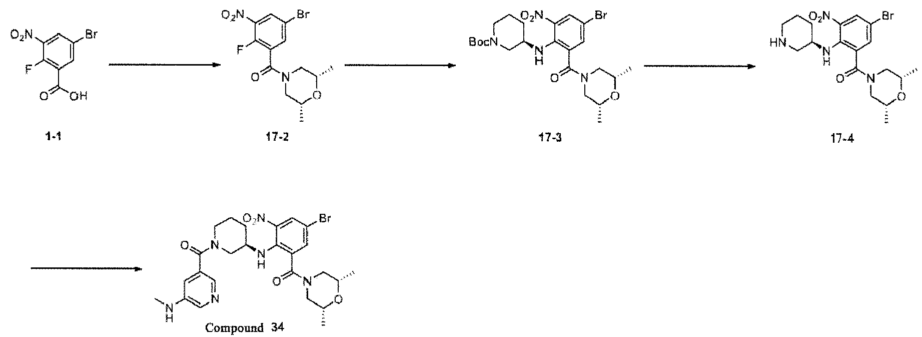

### Operating steps:

Step A: Compound 1-1 (1 g, 3.80 mmol) was dissolved in *N,N*-dimethylformamide (19 mL) at room temperature under nitrogen protection. Subsequently, the above solution was cooled down to 0°C and *N,N-*diisopropylethylamine (1.4 g, 11.4 mmol), 2-(7-azabenzotriazol)-*N,N,N',N*'-tetramethyluronium hexafluorophosphate (2 g, 5.32 mmol) and (2*R*,6*S*)-2,6-dimethylmorpholine (526 mg, 4.56 mmol) were added to the above reaction solution in sequence. Stirring of the reaction system was kept at 0°C for 5 min.

After the depletion of the raw material as monitored by LCMS, the reaction was quenched by adding water (100 mL), and the mixture solution was extracted with ethyl acetate (30 mL × 3 times). The organic phases were combined, and the resultant was washed with saturated saline (50 mL × 2 times), then dried with anhydrous sodium sulfate, filtered, and concentrated under reduced pressure. The resulting residue was purified by silica gel column chromatography to obtain Compound 17-2 (600 mg).

MS (ESI) M/Z: 361.0 [M+H]⁺.

**Step B:** Compound 17-2 (600 mg, 1.67 mmol) was dissolved in *N,N*-dimethylformamide (8.4 mL) at room temperature under nitrogen protection. Subsequently, tert-butyl (*R*)-3-aminopiperidin-1-carboxylate (400 mg, 2.00 mmol) and *N,N-*diisopropylethylamine (649 mg, 5.01 mmol) were sequentially added to the above reaction solution. The reaction system was then stirred at 80°C for 2 hours.

After the depletion of the raw material as monitored by LCMS, the reaction was quenched by adding water (40 mL), and the mixture solution was extracted with ethyl acetate (20 mL × 3 times). The organic phases were combined, and the resultant was washed with saturated saline (50 mL × 2 times), then dried with anhydrous sodium sulfate, filtered, and concentrated under reduced pressure. The resulting residue was purified by silica gel column chromatography to obtain Compound 17-3 (810 mg).

MS (ESI) M/Z:441.0 [M-99]⁺.

**Step C:** Compound 17-3 (810 mg, 1.50 mmol) was dissolved in 4 M hydrochloric acid-1,4-dioxane solution (2.8 mL) at room temperature under nitrogen protection. The reaction system was then stirred at room temperature for 1 hour.

After the depletion of the raw material as monitored by LCMS, the reaction solution was concentrated by vacuum distillation. Compound 17-4 was obtained as crude product (720 mg).

MS (ESI) M/Z: 441.0 [M+H]⁺.

**Step D:** Compound 5-(methylamino)nicotinic acid (23 mg, 0.15 mmol) was dissolved in *N,N*-dimethylformamide (1 mL) at room temperature under nitrogen protection. Subsequently, the above solution was cooled down to 0°C, and (5-bromo-3-nitro-2-((*R*)-piperidin-3-yl)amino)phenyl((2*R*,6*S*)-2,6-dimethylmorpholinyl)methanone (80 mg, 0.18 mmol), 2-(7-azabenzotriazol)-*N,N*,*N*',*N*'-etramethyluronium hexafluorophosphate (108 mg, 0.46 mmol), and *N,N*-diisopropylethylamine (79 mg, 0.21 mmol) were added in sequence. The reaction system was stirred at 0°C for 30 min.

After the depletion of the raw material as monitored by LCMS, the reaction was quenched by adding water (15 mL), and the mixture solution was extracted with ethyl acetate (10 mL × 3 times). The organic phases were combined, and the resultant was washed with saturated saline (20 mL × 2 times), then dried with anhydrous sodium sulfate, filtered, and concentrated under reduced pressure. The resulting residue was purified by reversed-phase chromatography column to obtain the titled Compound 34 (21.5 mg).

MS (ESI) M/Z: 575.0 [M+H]⁺.
¹H NMR (400 MHz, DMSO-*d*₆) δ 8.26 (s, 1H), 8.06 - 7.45 (m, 4H), 6.74 (s, 1H), 6.16 (s, 1H), 4.52 - 4.16 (m, 1H), 4.04 - 3.34 (m, 7H), 3.30 - 2.98 (m, 2H). 2.91 - 2.77 (m, 1H), 2.70 (s, 3H). 2.02 - 1.25 (in. 4H). 1.15 (brs, 3H). 1.03 (brs, 3H).

### Example 35

### (5-Bromo-2-((R)-1-(5-(methylamino)nicotinoyl)piperidin-3-yl)amino)-3-nitrophenyl)((R)-2-methylmorpholinyl)methanone

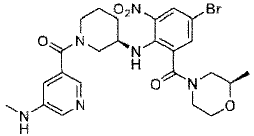

### Reaction route:

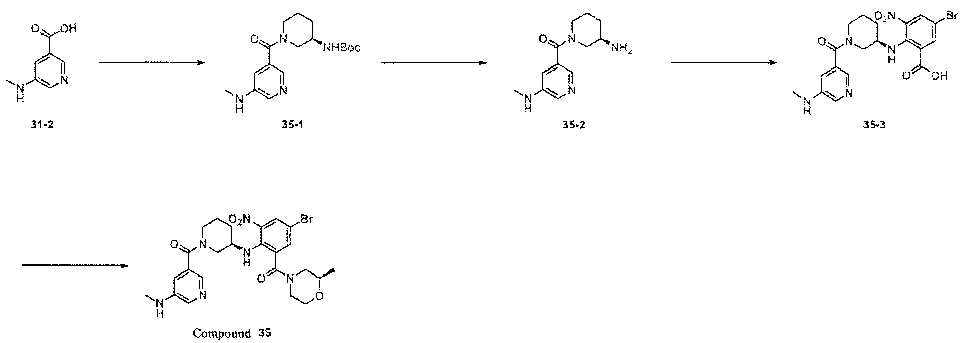

### Operating steps:

**Step A:** Compound 31-2 (500 mg, 3.29 mmol) was dissolved in *N,N-*dimethylformamide (16 mL) at room temperature under nitrogen protection. Subsequently, the above solution was cooled down to 0°C and *N,N*-diisopropylethylamine (1.3 g, 9.87 mmol), 2-(7-azabenzotriazol)-*N,N,N',N*'-tetramethyluronium hexafluorophosphate (1.4 g, 3.62 mmol) and tert-butyl(*R*)-piperidin-3-yl carbamate (725 mg, 3.62 mmol) were added to the above reaction solution in sequence. Stirring of the reaction system was then kept at 0°C for 10 min.

After the depletion of the raw material as monitored by LCMS, the reaction was quenched by adding water (20 mL), and the mixture solution was extracted with ethyl acetate (10 mL × 3 times). The organic phases were combined, and the resultant was washed with saturated saline (40 mL × 2 times), then dried with anhydrous sodium sulfate, filtered, and concentrated under reduced pressure. The resulting residue was purified by silica gel column chromatography to obtain Compound 35-1 (788 mg).

MS (ESI) M/Z: 335.2 [M+H]⁺.

**Step B:** Compound 35-1 (788 mg, 2.27 mmol) was dissolved in 4 M hydrochloric acid-1,4-dioxane solution (11 mL) at room temperature under nitrogen protection. The reaction system was then stirred at room temperature for 1 hour.

After the depletion of the raw material as monitored by LCMS, the reaction solution was concentrated by vacuum distillation. Compound 35-2 was obtained as crude product (682 mg).

MS (ESI) M/Z: 235.2 [M+H]⁺.

**Step C:** Compound 35-2 (682 mg, 2.78 mmol) was dissolved in *N,N-*dimethylformamide (11.6 mL) at room temperature under nitrogen protection. Subsequently, 5-bromo-2-fluoro-3-nitrobenzoic acid (610 mg, 2.32 mmol) and *N,N-*diisopropylethylamine (1.2 mL, 6.96 mmol) were sequentially added to the above reaction solution. The reaction system was then stirred at 80°C for 1 hour.

After the depletion of the raw material as monitored by LCMS, the reaction solution was concentrated by vacuum distillation, then diluted with dichloromethane (10 mL), adjusted to pH = 5-6 with 3M dilute hydrochloric acid and finally concentrated under reduced pressure. The resulting residue was purified by silica gel column chromatography to obtain the titled Compound 35-3 (1.02 g).

MS (ESI) M/Z: 478.0 [M+H]⁺.

**Step D:** Compound 35-3 (200 mg, 0.41 mmol) was dissolved in *N,N*-dimethylformamide solution (2.0 mL) at room temperature under nitrogen protection. Subsequently, the above solution was cooled down to -10°C and *N,N,N',N'-*tetramethyl-*O*-(7-azabenzotriazol-1-yl)uronium hexafluorophosphate (191.2 mg, 0.50 mmol), *N,N-*diisopropylethylamine (162.2 mg, 1.25 mmol), and (*R*)-2-methylmorpholine (70 mg, 0.50 mmol) were added in sequence. The reaction system was then stirred at -10°C for 30 min.

After the depletion of the raw material as monitored by LCMS, the reaction was quenched by adding water (10 mL), and the mixture solution was extracted with ethyl acetate (15 mL × 3 times). The organic phases were combined, and the resultant was washed with saturated saline (15 mL × 2 times), then dried with anhydrous sodium sulfate, filtered, and concentrated under reduced pressure. The resulting residue was purified by preparative high performance liquid chromatography to obtain the titled Compound 35 (89.1 mg).

MS (ESI) M/Z: 561.2 [M+H]⁺.
¹H NMR (400 MHz, DMSO-*d₆*) δ 8.26 (s, 1H). 8.08 - 7.34 (m, 4H). 6.75 (s, 1H), 6.15 (s, 1H), 4.50 - 3.35 (m. 8H). 3.28 - 2.51 (m, 7H), 1.98 - 1.31 (m, 4H), 1.22 - 0.96 (m, 3H).

### Example 36

### (R)-(5-bromo-2-((1-(5-(methylamino)nicotinoyl)piperidin-3-yl)amino)-3-nitrophenyl)(3,3-difluoropiperidin-1-yl)methanone

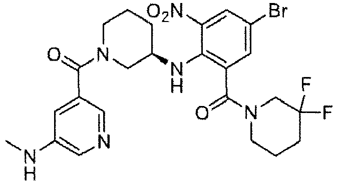

### Reaction route:

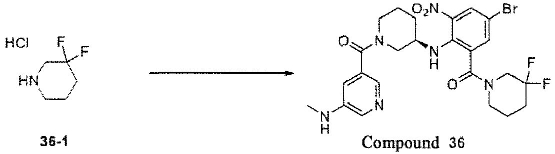

### Operating steps:

Step A: Compound 36-1 (15 mg, 0.084 mmol) and (*R*)-5-bromo-2-((1-(5-(methylamino)nicotinoyl)piperidin-3-yl)amino)-3-nitrobenzoic acid (40 mg, 0.084 mmol, Compound 35-3) were dissolved in *N,N*-dimethylformamide (1.0 mL) at room temperature. Subsequently, the above solution was cooled down to 0°C and 2-(7-azabenzotriazol)-*N,N,N*'*,N*'-tetramethyluronium hexafluorophosphate (32 mg, 0.084 mmol) and *N,N-*diisopropylethylamine (32 mg, 0.252 mmol) were added in turn. The reaction system was then stirred at 0 °C for 30 min.

After the depletion of the raw material as monitored by LCMS, the reaction was quenched by adding water (30 mL), and the mixture solution was extracted with ethyl acetate (6 mL × 3 times). The organic phases were combined, and the resultant was washed with saturated saline (20 mL × 2 times), then dried with anhydrous sodium sulfate, filtered, and concentrated under reduced pressure. The resulting residue was purified by reversed-phase chromatography column to obtain the titled Compound 36 (14.2 mg).

MS (ESI) M/Z: 581.0 [M+H]⁺.
¹H NMR (400 MHz, DMSO-*d*₆) δ 8.28 (s, 1H), 8.08 - 7.33 (m, 4H). 6.73 (s, 1H), 6.14 (s, 1H), 4.49 - 3.35 (m, 7H), 3.27- 2.87 (m, 2H), 2.70 (s, 3H), 2.24 - 1.95 (m, 2H). 1.94 - 1.29 (m. 6H).

### Example 37

### (5-Bromo-2-((R)-1-(5-(methylamino)nicotinoyl)piperidin-3-yl)amino)-3-nitrophenyl)((S)-2-(fluoromethyl)morpholinyl)methanone

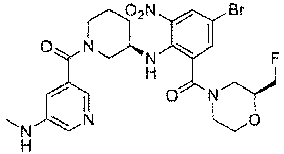

### Reaction route:

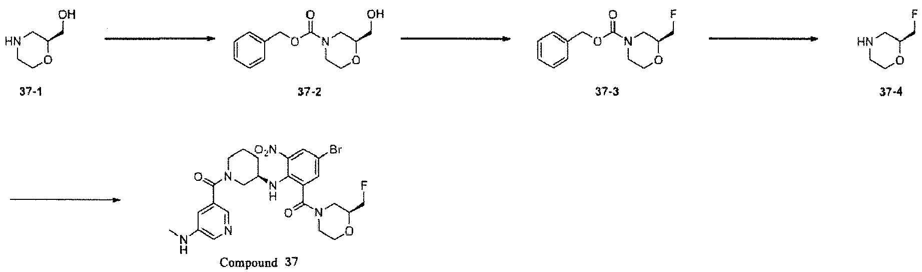

### Operating steps:

Step A: Compound 37-1 (300 mg, 1.96 mmol) was dissolved in a solvent mixture of acetonitrile (1 mL) and water (1 mL) at room temperature. Subsequently, benzyl chloroformate (503 mg, 2.94 mmol) and sodium bicarbonate (412 mg, 4.90 mmol) were sequentially added to the above reaction solution. Stirring of the reaction system was then kept for 2 hours at room temperature.

After the depletion of the raw material as monitored by LCMS, the reaction was quenched by adding water (40 mL), and the mixture solution was extracted with ethyl acetate (20 mL × 3 times). The organic phases were combined, and the resultant was washed with saturated saline (30 mL × 2 times), then dried with anhydrous sodium sulfate, filtered, and concentrated under reduced pressure. The resulting residue was purified by silica gel column chromatography to obtain Compound 37-2 (350 mg).

MS (ESI) M/Z: 252.2 [M+H]⁺.

Step B: Compound 37-2 (300 mg, 1.20 mmol) was dissolved in dichloromethane (6 mL) under an ice bath under nitrogen protection. Subsequently, diethylaminosulfur trifluoride (503 mg, 2.94 mmol) was added slowly and dropwise to the above reaction solution. The reaction system was then brought to room temperature and stirring was kept for 16 hours.

After the depletion of the raw material as monitored by LCMS, the reaction was quenched by adding water (20 mL), and the mixture solution was extracted with ethyl acetate (10 mL × 3 times). The organic phases were combined, and the resultant was washed with saturated saline (30 mL × 2 times), then dried with anhydrous sodium sulfate, filtered, and concentrated under reduced pressure. The resulting residue was purified by silica gel column chromatography to obtain Compound 37-3 (70 mg).

MS (ESI) M/Z: 276.0 [M+23]⁺.

Step C: Compound 37-3 (70 mg, 0.28 mmol) was dissolved in methanol (5 mL) at room temperature. Subsequently, 10% palladium/carbon (10 mg) and concentrated hydrochloric acid (5 mg) were added sequentially to the above reaction solution. The reaction system was then hydrogenated at room temperature for 1 hour.

After the depletion of the raw material as monitored by LCMS, the reaction solution was filtered and the filtrate was concentrated under reduced pressure. Compound 37-4 was obtained as crude product (60 mg).

MS (ESI) M/Z: 120.2 [M+H]⁺.

Step D: Compound 37-4 (20 mg, 0.14 mmol) was dissolved in *N,N-*dimethylformamide (1 mL) at room temperature under nitrogen protection. Subsequently, the above solution was cooled down to 0°C and (R)-5-bromo-2-((1-(5-(methylamino)nicotinoyl)piperidin-3-yl)amino)-3-nitrobenzoic acid (40 mg, 0.08 mmol), 2-(7-azabenzotriazol)-*N,N,N*'*,N*'-tetramethyluronium hexafluorophosphate (43 mg, 0.11 mmol), and *N,N*-diisopropylethylamine (52 mg, 0.14 mmol) were added in sequence. Stirring of the reaction system was then kept at 0°C for 30 min.

After the depletion of the raw material as monitored by LCMS, the reaction was quenched by adding water (20 mL), and the mixture solution was extracted with ethyl acetate (10 mL × 3 times). The organic phases were combined, and the resultant was washed with saturated saline (10 mL × 2 times), then dried with anhydrous sodium sulfate, filtered, and concentrated under reduced pressure. The resulting residue was purified by reversed-phase chromatographic column to obtain the titled Compound 37 (13 mg).

MS (ESI) M/Z: 579.0 [M+H]⁺.
¹H NMR (400 MHz, DMSO-*d*₆) δ 8.28 (s, 1H), 8.14 - 7.40 (m. 4H), 6.75 (s, 1H), 6.16 (s, 1H). 5.13 - 3.44 (m, 11H), 3.24 - 2.61 (m. 6H), 2.12 - 1.29 (m, 4H).

The following target compounds were prepared with reference to the synthetic methods of the above Examples:

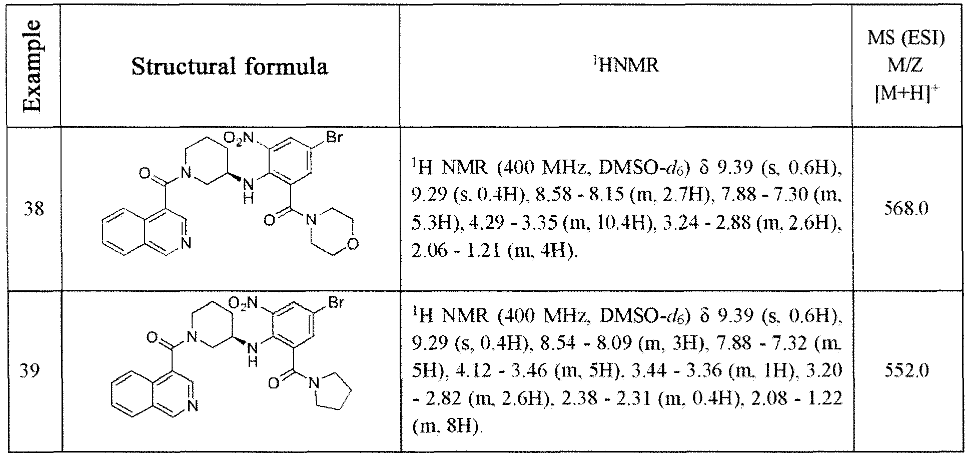

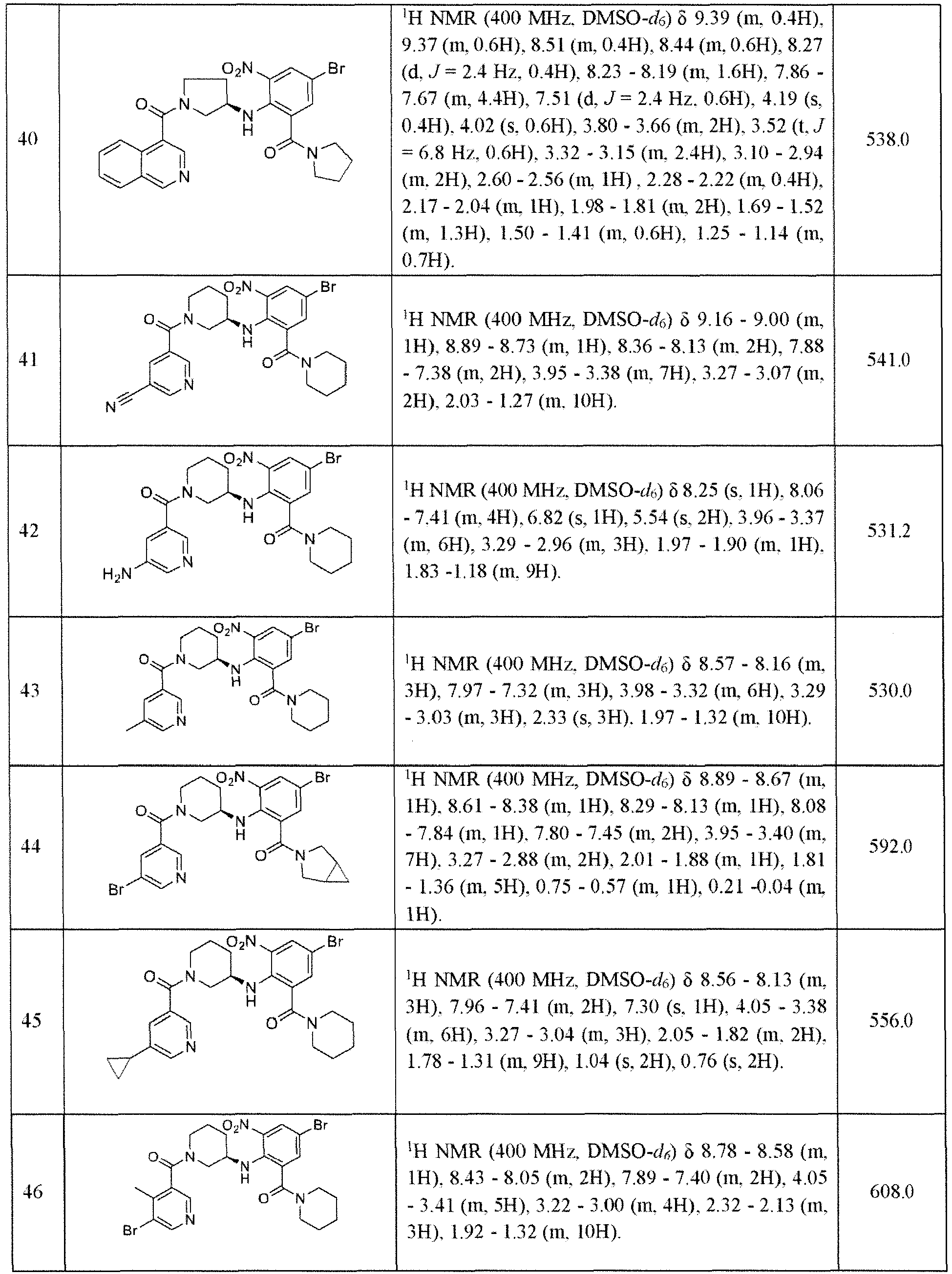

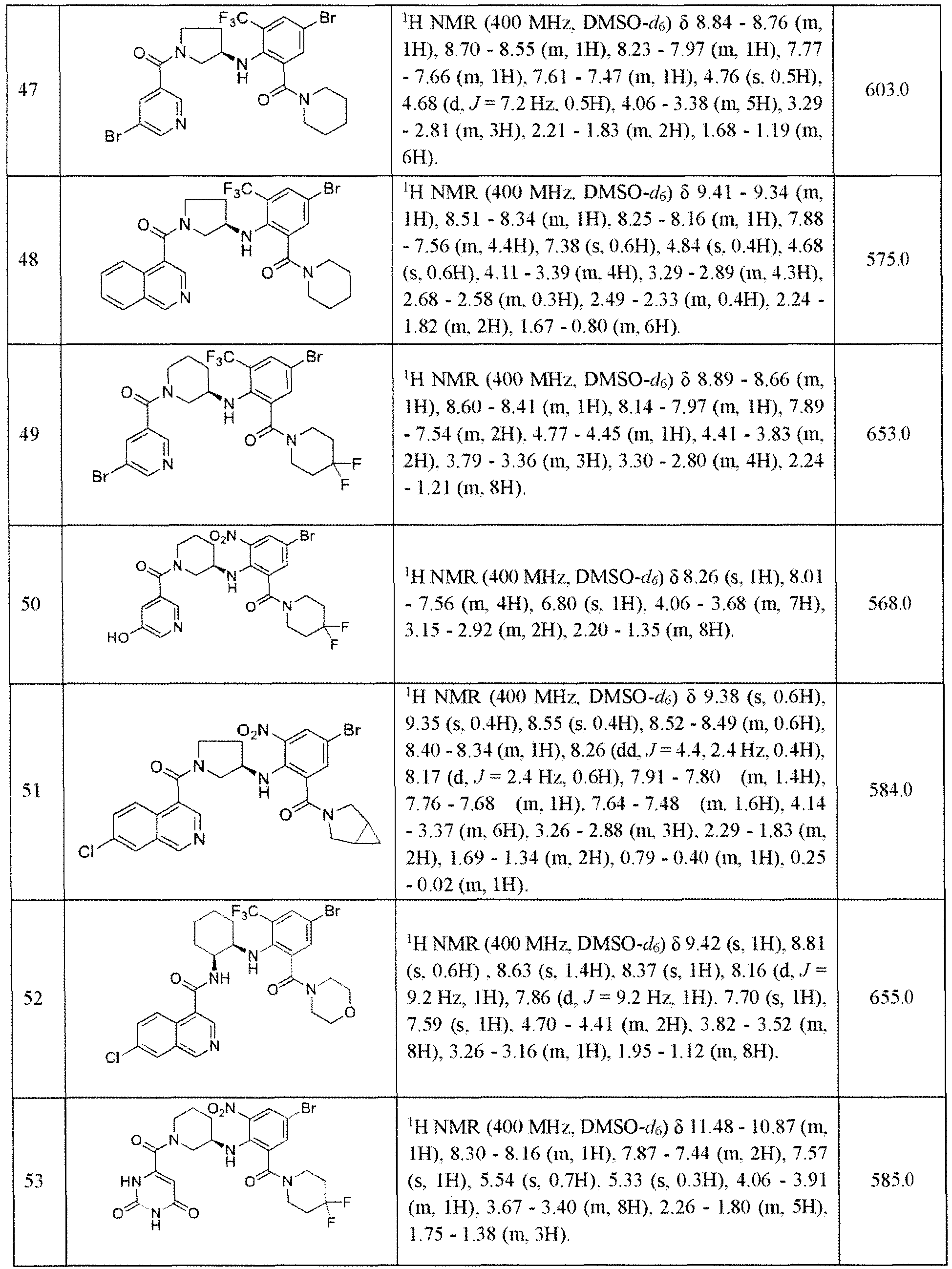

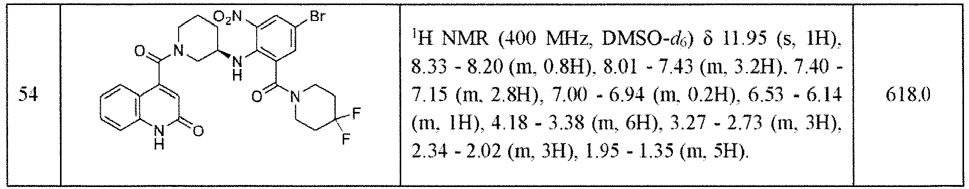

### Example 55

### (5-Bromo-2-((R)-1-(5-(methylamino)nicotinoyl)piperidin-3-yl)amino)-3-nitrophenyl)((S)-2-(difluoromethyl)morpholinyl)methanone

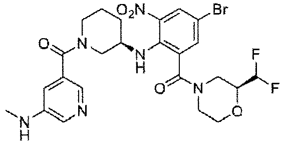

### Reaction route:

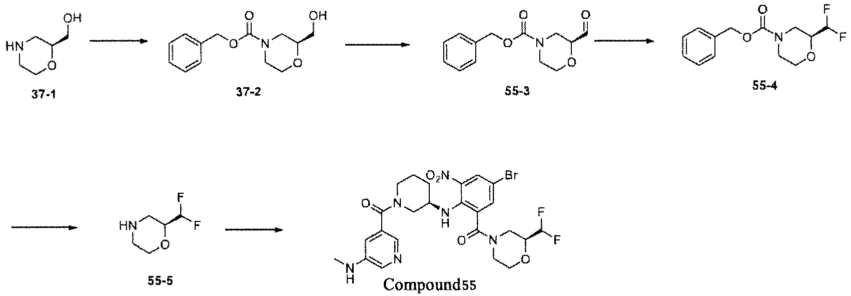

### Operating Steps:

Step A: Compound 37-1 (500 mg, 3.27 mmol) was added to a mixture of acetonitrile (5.0 mL) and water (5.0 mL) at room temperature. Subsequently, benzyl chloroformate (833 mg, 4.90 mmol) and sodium bicarbonate (1.13 g, 8.2 mmol) were then added in turn to the reaction solution. The reaction system was then stirred for 15 h.

After the depletion of the raw material as monitored by LCMS, the reaction was quenched by addition of water (10 mL), and the mixture solution was extracted with ethyl acetate (10 mL × 3 times). The organic phases were combined and washed with saturated aqueous sodium chloride solution (8 mL × 2 times), then dried with anhydrous sodium sulfate, filtered, and concentrated under reduced pressure. The resulting residue was purified by silica gel column chromatography to obtain Compound 37-2 (750 mg).

MS (ESI) M/Z: 252.2 [M+H]⁺.

¹H NMR (400 MHz. DMSO-*d*₆) δ 7.41 - 7.27 (m, 5H), 5.09 (s, 2H), 4.77 (t, *J* = 5.4 Hz, 1H), 3.93 (d, *J* = 13.2 Hz, 1H). 3.86 - 3.72 (m, 2H), 3.48 - 3.34 (m, 2H), 2.92 (brs, 1H). 2.50 (brs. 1H).

Step B: Oxalyl chloride (760 mg, 5.98 mmol) was added to anhydrous dichloromethane (5.0 mL) under the protection of nitrogen. Subsequently, the above solution was cooled to -78°C, followed by dropwise addition of anhydrous dimethyl sulfoxide (700 mg, 8.96 mmol), and stirring was kept for 30 min. A solution of Compound 37-2 (600 mg, 2.39 mmol) in dichloromethane (5.0 mL) was then added slowly and dropwise to the above solution, and the stirring was continued for 1 h. Finally, triethylamine (1.93 g, 19.1 mmol) was added dropwise to the mixture solution at this temperature, followed by raising the temperature of the reaction system to room temperature, and the stirring was continued for 1 h.

After the depletion of the raw material as monitored by LCMS, dichloromethane (20 mL) was added to the reaction solution for dilution, and the mixture solution was washed with water (15 mL), IN dilute hydrochloric acid (15 mL), saturated aqueous sodium bicarbonate (15 mL), and saturated aqueous sodium chloride (15 mL) in turn. Then the washed mixture solution was dried with anhydrous sodium sulfate, filtered, and concentrated under reduced pressure. Compound 55-3 (630 mg) was then obtained.

Step C: Compound 55-3 (220 mg, 0.88 mmol) was dissolved in anhydrous dichloromethane (5.0 mL) under the protection of nitrogen. Subsequently, the above solution was cooled to -78°C, followed by dropwise addition of diethylaminosulfur trifluoride (500 mg, 3.09 mmol). The temperature of the reaction system was then raised to room temperature, and the reaction system was continued to be stirred for 15 h.

After the depletion of the raw material as monitored by LCMS, the reaction solution was diluted by addition of dichloromethane (20 mL), and the mixture solution was extracted with saturated aqueous sodium bicarbonate solution (10 mL). The organic phases were collected and washed with saturated aqueous sodium chloride solution (10 mL × 2 times), then dried with anhydrous sodium sulfate, filtered, and concentrated under reduced pressure. The resulting residue was purified by silica gel column chromatography to obtain Compound 55-4 (55 mg).

Step D: Compound 55-4 (50 mg, 0.18 mmol) and 10% palladium/carbon (10 mg) were dissolved in methanol (3 mL) at room temperature and the reaction system was then subjected to hydrogenation reaction for 16 h at room temperature.

After the depletion of the raw material as monitored by LCMS, the reaction solution was filtered and the resulting filtrate was concentrated under reduced pressure to obtain Compound 55-5 (22 mg).

MS (ESI) M/Z: 138.2 [M+H]⁺.

Step E: Compound (*R*)-5-bromo-2-((1-(5-(methylamino)nicotinoyl)piperidin-3-yl)amino)-3-nitrobenzoic acid (30 mg, 0.06 mmol) was dissolved in *N*,*N*-dimethylformamide (1.0 mL) at room temperature. Subsequently, 2-(7-azabenzotriazolyl)-*N,N,N',N*'-tetramethyluronium hexafluorophosphate (28 mg, 0.07 mmol), *N,N*-diisopropylethylamine (16 mg, 0.12 mmol), and Compound 1-5 (10 mg, 0.07 mmol) were added in turn to the above solution. The reaction system was then continued to be stirred for 1 h.

After the depletion of the raw material as monitored by LCMS, the reaction was quenched by addition of water (15 mL), and the mixture solution was extracted with ethyl acetate (5 mL × 3 times). The organic phases were combined and washed with aqueous sodium chloride solution (20 mL × 2 times), then dried with anhydrous sodium sulfate, filtered, and concentrated under reduced pressure. The resulting residue was purified by preparative high performance liquid chromatography to obtain the titled Compound 55 (10.7 mg).

MS (ESI) M/Z: 597.0 [M+H]⁺.

¹H NMR (400 MHz, DMSO-*d*₆) δ 8.28 (s, 1H), 8.09 - 7.40 (m, 4H), 6.88 - 6.47 (m, 1H). 6.42 - 5.83 (m, 2H). 4.58 - 4.15 (m, 1H), 4.13 - 3.36 (m, 8H), 3.29 - 2.86 (m, 3H), 2.70 (brs. 3H). 2.03 - 1.30 (m, 4H).

The following target compounds were prepared with reference to the synthetic method of the above examples:

| **Example** | **Structural Formula** | ¹HNMR | MS (ESI) MlZ [M+H]⁺ |
|---|---|---|---|
| 56. | 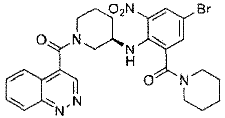 | ¹H NMR (400 MHz, DMSO-*d*₆) δ 9.31 (s, 0.6H), 9.14 (s, 0.4H), 8.58 (d, *J* = 8.4 Hz, 0.6H), 8.50 (brs, 0.4H), 8.30 (d, *J* = 2.4 Hz. 0.6H), 8.08 - 7.75 (m, 4.4H), 7.67 (d, *J* = 2.4 Hz, 0.6H), 7.44 - 7.18 (m, 0.4H), 4.07 -3.40 (m, 6H), 3.25 - 3.00 (m, 3H), 2.00 -1.28 (m, 10H), | 567.5 |
| 57. | 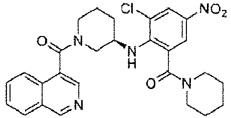 | ¹H NMR (400 MHz, DMSO-*d*₆) δ 9.40 (s, 0.6H), 9.29 (s, 0.4H), 8.55 - 7.56 (m, 7H), 6.16 (s, 0.6H), 5.85 (s, 0.4H), 4.47 - 3.38 (m, 6H), 3.26 - 2.85 (m, 3H), 2.07 - 1.38 (m, 10H). | 522.0 |
| 59. | 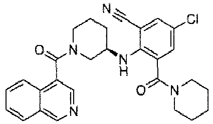 | ¹H NMR (400 MHz, DMSO-*d*₆): δ 9.39 (s, 0.6H), 9.31 (s, 0.4H), 8.16 (s, 0.4H), 8.39 (s, 0.6H), 8.23 (d, *J* = 8.0 Hz, 0.6H), 8.16 (d, *J* = 8.0 Hz. 0.4H), 7.94 - 7.66 (m, 3.6H), 7.60 (s, 0.4H), 7.50 (d, *J* = 2.0 Hz, 0.6H), 7.41 - 7.15 (m, 0.4H), 5.56 (brs, 0.6H), 5.35 - 5.07 (m, 0.4H), 4.34 - 3.39 (m, 5H), 3.28 - 2.94 (m, 3H), 2.89 -2.58 (m, 1H), 1.94 - 1.01 (m, 10H). | 502.2 |
| 60. | 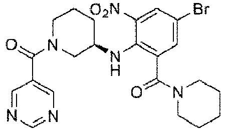 | ¹H NMR (400 MHz, DMSO-*d*₆) δ 9.35 - 9.12 (m, 1H), 8.92 - 8.68 (m, 2H), 8.34 - 8.10 (m, 1H), 7.90 - 7.38 (m, 2H), 4.11 - 3.38 (m, 7H), 3.24 - 2.95 (m, 2H), 2.00 - 1.30 (m, 10H). | 517.0 |
| 61. | 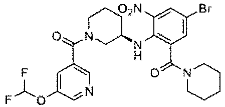 | ¹H NMR (400 MHz, DMSO-*d*₆): δ 8.67 - 8.33 (m, 2H), 8.32 - 8.12 (m, 1H), 7.91 - 6.51 (m, 4H), 4.25 - 3.54 (m, 6H), 3.30 - 3.00 (m, 3H), 1.99 - 1.26 (m, 10H). | 582.0 |
| 62. | 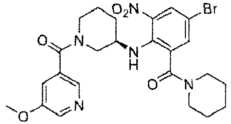 | ¹H NMR (400 MHz, DMSO-*d*₆): δ 8.44 - 8.00 (m, 3H), 7.97 - 7.72 (m, 1H), 7.68 - 7.42 (m, 1H), 7.37 - 7.11 (m, 1H), 4.16 - 3.52 (m, 8H), 3.31 - 2.86 (m, 4H), 1.99 - 1.26 (m, 10H). | 546.0 |
| 63. | 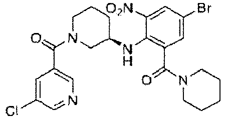 | ¹H NMR (400 MHz, DMSO-*d*₆): δ 8.84 - 8.59 (m, 1H), 8.56 - 8.38 (m, 1H), 8.34 - 8.12 (m, 1H), 8.00 - 7.36 (m, 3H), 4.17 - 3.37 (m, 6H), 3.26 - 2.80 (m, 3H), 2.00 - 1.31 (m, 10H). | 550.0 |
| 64. | 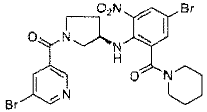 | ¹H NMR (400 MHz, DMSO-*d₆*) δ 8.85 - 8.76 (m, 1H), 8.67 (s, 0.5H), 8.63 (s, 0.5H), 8.22 (d, *J* = 2.4 Hz. 0.5H), 8.21 - 8.00 (m, 1.5H), 7.64 (d, *J* = 2.4 Hz. 0.5H), 7.61 - 7.47 (m, 1.5H), 4.22 - 3.98 (m, 1H), 3.63 - 3.52 (m, 5.5H), 3.51 - 3.34 (m, 0.5H), 3.25 - 2.88 (m, 2H), 2.27 - 2.08 (m, 1H), 2.05 - 1.90 (m, 1H), 1.68 -1.09 (m, 6H). | 580.0 |
| 65. | 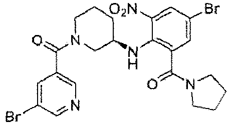 | ¹H NMR (400 MHz, DMSO-*d*₆) δ 8.86 - 8.67 (m, 1H), 8.56 - 8.39 (m, 1H), 8.33 - 8.17 (m, 1H), 8.08 - 7.89 (m, 1H), 7.84 - 7.52 (m, 2H), 3.87 - 3.35 (m, 7H), 3.27 - 2.84 (m, 2H), 1.96 - 1.37 (m, 8H). | 580.0 |
| 66. | 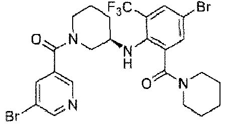 | ¹H NMR (400 MHz, DMSO-*d*₆): δ 8.87 - 8.67 (m, 1H), 8.59 - 8.43 (m, 1H), 8.10 - 7.91 (m, 1H), 7.75-7.39 (m, 2H), 4.76 - 4.39 (m, 1H), 4.10 (brs, 1H), 3.91 - 3.42 (m, 3H), 3.24 - 2.89 (m, 4H), 1.96 - 1.18 (m, 10H). | 617.0 |
| 67. | 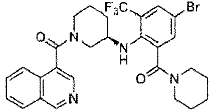 | ¹H NMR (400 MHz, DMSO-*d*₆) δ 9.45 - 9.24 (m, 1H), 8.53 - 8.10 (m, 2H), 7.91 - 7.27 (m, 5H), 4.78 - 4.17 (m, 2H), 4.06 - 3.39 (m, 2.6H), 3.27 - 2.83 (m, 5.4H), 1.97 - 1.24 (m, 8.6H), 1.18 - 0.91 (m, 1.4H). | 589.0 |
| 68. | 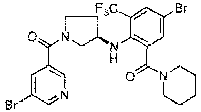 | ¹H NMR (400 MHz, DMSO-*d*₆) δ 8.84 - 8.76 (m, 1H), 8.70 - 8.55 (m, 1H), 8.23 - 7.97 (m, 1H), 1.77 - 7.66 (m, 1H), 7.61 - 7.47 (m, 1H), 4.76 (s, 0.5H), 4.68 (d, *J* = 7.2 Hz, 0.5H), 4.06 - 3.38 (m, 5H), 3.29 - 2.81 (m, 3H), 2.21 - 1.83 (m, 2H), 1.68 - 1.19 (m, 6H). | 603.0 |
| 69. | 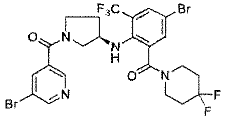 | ¹M NMR (400 MHz, DMSO-*d*₆) δ 8.87 - 8.73 (m, 1H), 8.70 - 8.53 (m, 1H), 8.20 - 7.99 (m, 1H), 7.83 - 7.61 (m, 2H), 4.78 (d, *J* = 8.0 Hz, 0.5H), 4.69 (s, 0.5H), 4.11 - 3.79 (m, 2H), 3.69 - 3.35 (m, 5.5H), 3.27 - 2.99 (m, 1.5H), 2.23 - 1.71 (m, 6H). | 639.0 |
| 70. | 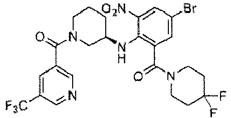 | ¹H NMR (400 MHz, DMSO-*d*₆) δ 9.14 - 8.958 (m, 1H), 8.94 - 8.76 (m, 1H), 8.34 - 8.05 (m, 2H), 7.90 - 7.54 (m, 2H), 4.04 - 3.38 (m, 9H), 2.25 - 1.78 (m, 5H), 1.73 - 1.35 (m, 3H). | 620.0 |
| 71. | 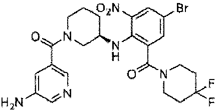 | ¹H NMR (400 MHz, CDCl₃) δ 8.36 (s, 1H), 8.13 (s, 0.8H), 8.21 - 7.63 (m, 2.4H), 7.47 (s, 1H), 7.10 (s, 0.8H), 4.34 - 2.94 (m, 9H), 2.27 - 1.77 (m, 7H), 1.56 - 1.34 (m, 1H). | 567.0 |
| 72. | 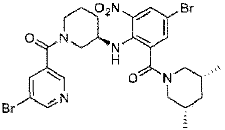 | ¹H NMR (400 MHz, DMSO-*d*₆) δ 8.86 -8.64 (m, 1H), 8.59 - 8.38 (m, 1H), 8.33 - 8.15 (m, 1H), 8.10 - 7.39 (m, 3H), 4.58 - 4.16 (m, 1H), 4.02 - 3.37 (m, 5H), 3.27 - 3.20 (m, 1H), 2.70 - 2.53 (m, 1H), 2.29 - 2.10 (m, 1H), 2.03 - 1.31 (m, 8H), 0.97 -0.65 (m, 6H). | 622.0 |
| 73. | 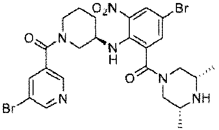 | ¹H NMR (400 MHz, DMSO-*d*₆): δ 8.89 - 8.65 (m, 1H), 8.62 - 8.44 (m, 1H), 8.33 - 8.14 (m, 1H), 8.09 - 7.41 (m, 3H), 4.44 - 4.15 (m, 1H), 3.96 - 3.38 (m, 4H), 3.28 - 2.98 (m, 2H), 2.86 - 2.54 (m, 3H), 2.45 - 2.16 (m, 2H), 2.02 - 1.32 (m, 4H), 1.02 (brs, 3H), 0.88 (brs, 3H). | 623.0 |
| 74. | 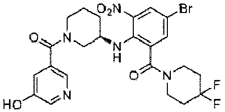 | ¹H NMR (400 MHz, DMSO-*d₆*) δ 8.26 (s, 1H), 8.01 - 7.56 (m, 4H), 6.80 (s, 1H), 4.06 - 3.68 (m, 7H), 3.15 - 2.92 (m, 2H), 2.20 - 1.35 (m, 8H). | 568.0 |
| 75. | 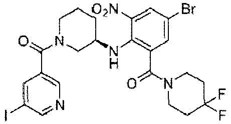 | ¹H NMR (400 MHz, DMSO-*d₆*) δ 8.90 - 8.68 (m, 1H), 8.62 - 8.37 (m, 1H), 8.34 - 7.92 (m, 2H), 7.87 - 7.54 (m, 2H), 4.07 - 3.36 (m, 7H), 3.28 - 2.83 (m, 2H), 2.25 - 1.80 (m, 5H), 1.78 - 1.32 (m, 3H). | 678.0 |
| 76. | 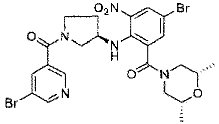 | ¹H NMR (400 MHz, DMSO-*d*₆) δ 8.81 (s, 1H), 8.74 - 8.58 (m, 1H), 8.30 - 8.03 (m, 2H), 7.74 - 7.45 (m, 2H), 4.47 - 3.82 (m, 2H), 3.77 - 3.42 (m, 6H), 3.29 - 2.69 (m, 2H), 2.47 - 2.36 (m, 1H), 2.31- 2.11 (m, 1H), 2.08 - 1.85 (m, 1H), 1.25 -0.83 (m, 6H). | 610.0 |
| 77. | 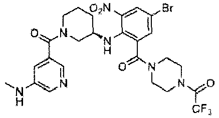 | ¹H NMR (400 MHz, DMSO-*d*₆) δ 8.29 (s, 1H), 8.04 - 7.49 (m, 4H), 6.76 (s, 1H), 6.15 (s, 1H), 4.04 - 3.36 (m, 12H), 3.25 - 3.07 (m, 1H), 2.69 (d, *J* = 1.6 Hz, 3H), 1.97 - 1.67 (m, 2H), 1.66 - 1.24 (m, 2H). | 642.0 |
| 78. | 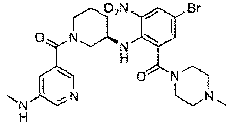 | ¹H NMR (400 MHz, DMSO-*d*₆) δ 8.26 (s, 1H), 8.07 - 7.43 (m, 4H), 6.75 (s, 1H), 6.16 (s, 1H), 4.07 - 3.46 (m, 7H), 3.29 - 3.11 (m, 2H), 2.70 (s, 3H), 2.44 - 2.00 (m, 6.5H), 1.99 - 1.29 (m, 4.5H). | 560.0 |
| 79. | 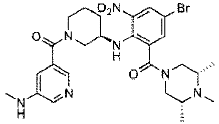 | ¹H NMR (400 MHz, DMSO-*d*₆): δ 8.26 (s, 1H), 8.03 - 7.46 (m, 4H), 6.74 (s, 1H), 6.15 (brs, 1H), 4.31 (s, 1H), 4.03 - 3.37 (m, 4H), 3.26 - 2.79 (m, 3H), 2.75 - 2.63 (m, 3H), 2.62 - 2.51 (m, 1H), 2.16 (s, 3H), 2.10 - 2.12 (m, 6H), 1.16 - 0.81 (m, 6H). | 588.1 |
| 80. | 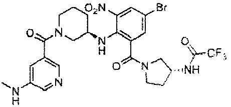 | ¹H NMR (400 MHz, DMSO-*d*₆): δ 9.73 - 9.52 (m, 1H), 8.27 (s, 1H), 8.06 - 7.52 (m, 4H), 6.77 (s, 1H), 6.16 (brs, 1H), 4.51 - 4.17 (m, 1H), 4.03 -3.37 (m, 7H), 3.29 - 3.06 (m, 2H), 2.70 (brs, 3H), 2.30 - 2.07 (m, 1H), 2.02 - 1.66 (m, 3H), 1.64 - 1.35 (m, 2H). | 584.1 |
| 81. | 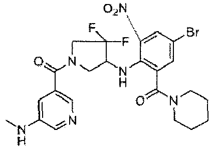 | ¹H NMR (400 MHz, DMSO-*d₆*) δ 8.25 (s, 1H), 8.03 (s, 1H), 7.87 (s, 1H), 7.80 - 7.50 (m, 1.6H), 7.18 (s, 0.4H), 6.93 (s, 1H), 6.18 (s, 1H), 4.46 - 3.51 (m, 6H), 3.27-2.83 (m, 3H), 2.72 (d, *J* = 5.2 Hz, 3H), 1.71 -1.07 (m, 6H) | 567.0 |
| 82. | 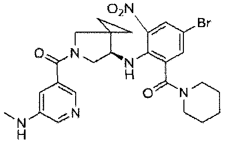 | ¹H NMR (400 MHz, DMSO-*d₆*) δ 8.26 (d, *J* = 2.4 Hz, 0.5H), 8.22 (s, 0.5H), 8.01 - 7.91 (m, 1H), 7.88 - 7.49 (m, 3H), 6.93 - 6.66 (m, 1H), 6.18 - 6.06 (m, 1H), 3.89 - 3.35 (m, 6H), 3.29 - 2.92 (m, 3H), 2.75 - 2.60 (m, 3H), 1.70 - 1.22 (m, 6H), 0.89 - 0.49 (m, 4H). | 557.2 |
| 83. | 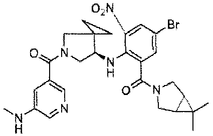 | ¹H NMR (400 MHz, DMSO-*d₆*) δ 8.31 - 8.16 (m, 1H), 8.00 - 7.91 (m, 1H), 7.81 - 7.39 (m, 3H), 6.89 - 6.71 (m, 1H), 6.17 - 6.04 (m, 1H), 3.87 - 3.35 (m, 7H), 3.28 - 3.17 (m, 1H), 3.11 - 2.84 (m, 1H), 2.75 - 2.63 (m, 3H), 1.54 - 1.12 (m, 2H), 1.05 - 0.53 (m, 10H). | 583.0 |
| 84. | 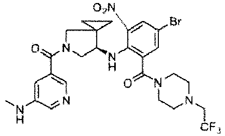 | ¹H NMR (400 MHz, DMSO-*d₆*) δ 8.28 (d, *J* = 2.0 Hz, 0.5H), 8.24 (s, 0.5H), 7.98 (d, *J* = 2.4 Hz, 0.5H), 7.95 (d, .7= 2.4 Hz, 0.5H), 7.87 - 7.51 (m, 3H), 6.93 - 6.65 (m, 1H), 6.16 - 6.06 (m, 1H), 3.92 - 3.41 (m, 6H), 3.29 - 3.01 (m, 5H), 2.96 - 2.58 (m, 6H), 2.47 - 2.10 (m, 1H), 0.91 - 0.51 (m, 4H). | 640.1 |
| 85. | 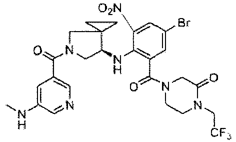 | ¹H NMR (400 MHz, DMSO-*d*₆) δ 8.31 (s, 0.5H), 8.26 (s, 0.5H), 7.98 (d, *J* = 2.4 Hz, 0.5H), 7.94 (d, *J* = 2.0 Hz, 0.5H), 7.86 - 7.47 (m, 3H), 6.85 (s, 0.5H), 6.75 (s, 0.5H), 6.18 - 6.03 (m, 1H), 4.39 - 3.36 (m, 11H), 3.27 - 3.11 (m, 2H), 2.74 - 2.61 (m, 3H), 0.88 - 0.52 (m, 4H). | 654.0 |
| 86. | 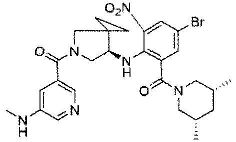 | ¹H NMR (400 MHz, DMSO-*d₆*) δ 8.33 - 8.17 (m, 1H), 8.05 - 7.47 (m, 4H), 6.95 - 6.67 (m, 1H), 6.25 - 6.04 (m, 1H), 4.58 - 4.23 (m, 1H), 3.96 - 3.48 (m, 4H), 3.34 - 2.87 (m, 2H), 2.85 - 2.51 (m, 4H), 2.47 - 2.06 (m, 1H), 1.91 - 1.29 (m, 3H), 1.03 - 0.47 (m, 11H). | 585.2 |
| 87. | 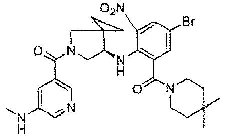 | ¹H NMR (400 MHz, DMSO-*d₆*) δ 8.33 - 8.15 (m, 1H), 8.04 - 7.92 (m, 1H), 7.89 - 7.47 (m, 3H), 6.92 - 6.70 (m, 1H), 6.19 - 6.04 (m, 1H), 3.89 - 3.44 (m, 5H), 3.27 -2.93 (m, 4H), 2.76 - 2.60 (m, 3H), 1.56 - 1.08 (m, 4H), 1.05 - 0.52 (m, 11H). | 585.2 |
| 88. | 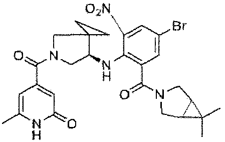 | ¹H NMR (400 MHz, DMSO-*d₆*) δ 11.76 (s, 1H), 8.30 - 8.22 (m, 1H), 7.67 - 7.36 (m, 2H), 6.13 - 5.68 (m, 2H), 3.76 - 3.34 (m, 7H), 3.26 - 3.17 (m, 1H), 3.09 - 2.84 (m, 1H), 2.21 - 2.10 (m, 3H), 1.54 - 1.25 (m, 2H), 1.04 - 0.96 (m, 3H), 0.94 - 0.53 (m, 7H). | 584.1 |
| 89. | 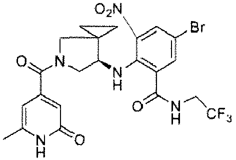 | ¹H NMR (400 MHz, DMSO-*d₆*) δ 11.75 (s, 1H), 9.57 (t, *J* = 6.4 Hz, 0.5H), 9.49 (t*, J* = 5.8 Hz, 0.5H), 8.29 (d, *J* = 2.0 Hz, 0.5H), 8.24 (d, *J* = 2.4 Hz, 0.5H), 8.01 (d, *J* = 7.2 Hz, 0.5H), 7.90 (d, *J* = 7.6 Hz. 0.5H), 7.81 (d, *J* = 2.4 Hz, 0.5H), 7.78 (d, *J* = 2.4 Hz, 0.5H), 6.09 (s, (0.5H), 6.02 (s, 0.5H), 5.91 (s, 0.5H), 5.71 (s, 0.5H), 4.18 - 4.00 (m, 2H), 3.72 - 3.44 (m, 4H), 3.25 - 3.15 (m, 1H), 2.17 (s, 1.5H), 2.14 (s, 1.5H), 0.79 - 0.55 (m, 4H). | 572.0 |
| 90. | 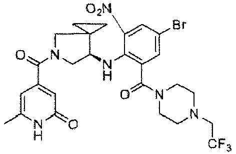 | ¹H NMR (400 MHz, DMSO-*d₆*) δ 11.76 (s, 1H), 8.27 (d, *J* = 2.4 Hz, 0.5H), 8.26 (d, *J* = 2.4 Hz, 0.5H), 7.86 - 7.49 (m, 2H), 6.17 - 5.60 (m, 2H), 3.93 - 3.42 (m, 6H), 3.28 - 3.01 (m, 5H), 2.86 - 2.55 (m, 3H), 2.47 - 2.33 (m, 1H), 2.17 (s, 1.5H), 2.12 (s, 1.5H), 0.89 -0.53 (m, 4H). | 641.0 |
| 91. | 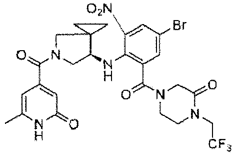 | ¹H NMR (400 MHz, DMSO-*d₆*) δ 11.74 (s, 1H), 8.32 - 8.26 (m, 1H), 7.83 - 7.48 (m, 2H), 6.17 - 5.54 (m, 2H), 4.35 - 4.13 (m, 3H), 4.08 - 3.35 (m, 9H), 3.26 - 3.15 (m, 1H), 2.17 (s, 1.5H), 2.13 (s, 1.5H), 0.85 - 0.57 (m, 4H). | 655.0 |
| 92. | 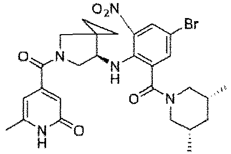 | ¹H NMR (400 MHz, DMSO-*d*₆) δ 11.78 (s, 1H), 8.41 - 8.20 (m, 1H), 8.13 - 7.44 (m, 2H), 6.22 - 5.64 (m, 2H), 4.56 - 4.25 (m, 1H), 3.87 - 3.46 (m, 4H), 3.28 - 2.93 (m, 2H), 2.75 - 2.53 (m, 1H), 2.28 - 1.97 (m, 4H), 1.84 - 1.26 (m, 3H), 1.00 - 0.52 (m, 11H). | 586.0 |
| 93. | 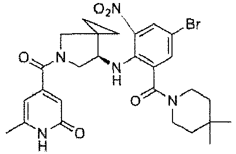 | ¹H NMR (400 MHz, DMSO-*d₆*) δ 11.76 (s, 1H), 8.29 - 8.21 (m, 1H), 7.89 - 7.52 (m, 2H), 6.16 - 5.63 (m, 2H), 3.85 - 3.35 (m, 6H), 3.29 - 2.88 (m, 3H), 2.17 (s, 1.3H), 2.12 (s, 1.7H), 1.54 - 1.05 (m, 4H), 0.98 - 0.56 (m, 10H). | 586.0 |
| 94. | 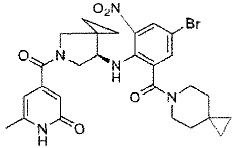 | ¹H NMR (400 MHz, DMSO-*d₆*) δ 11.76 (s, 1H), 8.29 - 8.20 (m, 1H), 7.92 - 7.54 (m, 2H), 6.18 - 5.66 (m, 2H), 3.78 - 3.35 (m, 6H), 3.27 - 2.84 (m, 3H), 2.18 (s, 1.3H), 2.13 (s, 1.7H), 1.57 - 1.09 (m, 4H), 1.01 - 0.51 (m, 4H), 0.44 - 0.22 (m, 4H). | 584.0 |
| 95. | 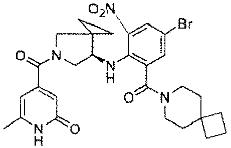 | ¹H NMR (400 MHz, DMSO-*d₆*) δ 11.77 (s, 1H), 8.27 -8.20 (m, 1H), 7.85 - 7.53 (m, 2H), 6.15 - 5.67 (m, 2H), 3.78 - 3.41 (m, 6H), 3.26 - 2.74 (m, 3H), 2.17 (s, 1.3H), 2.13 (s, 1.7H), 1.93 - 1.24 (m, 10H), 0.85 - 0.53 (m, 4H). | 598.1 |
| 96. | 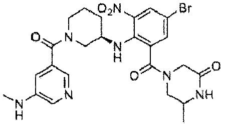 | ¹H NMR (400 MHz, DMSO-*d₆*) δ 8.36 - 8.14 (m, 2H), 8.02 - 7.50 (m, 4H), 6.75 (s, 1H), 6.14 (brs, 1H), 4.48 - 3.40 (m, 8H), 3.25 - 2.83 (m, 2H), 2.70 (brs, 3H), 1.94 - 1.30 (m, 4H), 1.15 (brs, 1H), 1.01 (brs, 2H). | 574.0 |
| 97. | 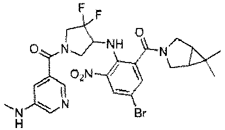 | ¹H NMR (400 MHz, DMSO-*d₆*) δ 8.30 - 8.21 (m, 1H), 8.07 - 7.97 (m, 1H), 7.91 - 7.83 (m, 1H), 7.80 - 7.63 (m, 1H), 7.48 (d, *J* = 9.2 Hz. 0.3H), 7.20 (d, *J* = 8.0 Hz. 0.2H), 7.09 - 6.83 (m, 1.5H), 6.25 - 6.09 (m, 1H), 4.48 - 4.14 (m, 1H), 4.10 - 3.49 (m, 7H), 3.08 - 2.89 (m, 1H), 2.77 - 2.63 (m, 3H), 1.58 - 1.18 (m, 2H), 1.08 - 0.71 (m, 6H). | 593.0 |
| 98. | 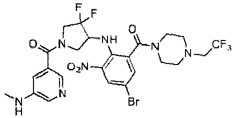 | ¹H NMR (400 MHz, DMSO-*d₆*) δ 8.26 (s, 1H), 8.03 (d, *J* = 2.8 Hz. 1H), 7.88 (s, 1H), 7.82 - 7.48 (m, 1.5H), 7.09 - 6.80 (m, 1.5H), 6.17 (brs, 1H), 4.41 - 4.19 (m, 1H), 4.16 - 3.43 (m, 6H), 3.29 - 2.93 (m, 4H), 2.83 - 2.51 (m, 7H). | 650.0 |
| 99. | 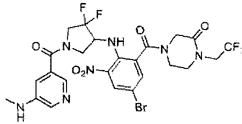 | ¹H NMR (400 MHz, DMSO-*d₆*) δ 8.28 (s, 1H), 8.02 (d, *J* = 2.0 Hz. 1H), 7.90 - 7.77 (m, 2H), 7.60 -7.06 (m, 1H), 6.91 (s, 1H), 6.16 (q, *J* = 4.8 Hz, 1H), 4.46 - 3.77 (m, 9H), 3.72 - 3.39 (m, 4H), 2.72 (d, *J* = 5.2 Hz, 3H). | 664.0 |
| 100. | 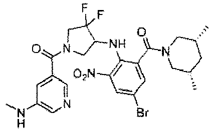 | ¹H NMR (400 MHz, DMSO-*d₆*) δ 8.32 - 8.18 (m, 0.85H), 8.04 (s, 0.85H), 7.88 (s, 0.85H), 7.78 - 7.44 (m, 1.45H), 7.08 - 6.72 (m, 1H), 6.23 (brs, 1H), 4.65 - 3.50 (m, 6H), 3.23 - 2.81 (m, 3H), 2.73 (brs, 3H), 2.28 - 1.36 (m, 4H), 1.00 - 0.50 (m, 6H). | 595.0 |
| 101. | 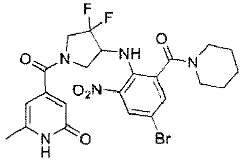 | ¹H NMR (400 MHz, DMSO-*d₆*) δ 11.85 (s, 1H), 8.27 - 8.22 (m, 1H), 7.82 - 7.66 (m, 1H), 7.58 (s, 0.5H), 7.12 (s, 0.3H), 6.91 (s, 0.2H), 6.20 (s, 1H), 6.00 (s, 1H), 4.45 - 3.40 (m, 7H), 3.28 - 2.92 (m, 2H), 2.19 (s, 3H), 1.72 - 1.20 (m, 6H). | 568.0 |
| 102. | 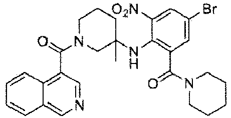 | ¹H NMR (400 MHz, DMSO-*d₆*) δ 9.38 (s, 1H), 8.51- 8.29 (m, 1H), 8.26 - 8.13 (m, 1.4H), 8.07 - 7.39 (m, 4.6H), 5.59 - 5.12 (m, 0.4H), 4.85 - 4.16 (m, 1H), 3.98 - 3.42 (m, 2H), 3.29 - 2.70 (m, 5H), 2.49 - 2.33 (m, 0.6H), 1.64 - 1.28 (m, 9H), 1.20 - 0.54 (m, 4H). | 580.0 |
| 103. | 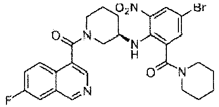 | ¹H NMR (400 MHz, CDCl₃) δ 9.48 - 9.23 (m, 1H), 8.68 - 8.25 (m, 2H), 8.17 - 7.44 (m, 5H), 4.31 - 2.89 (m, 9H), 2.40 - 2.11 (m, 5H), 2.03 - 1.91 (m, 1H), 1.71 - 1.32 (m, 4H). | 584.0 |
| 104. | 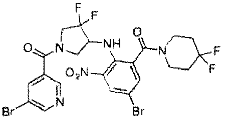 | ¹H NMR (400 MHz, DMSO-*d₆*) δ 8.84 (s, 1H), 8.72 (s, 1H), 8.36 - 8.16 (m, 2H), 7.98 - 7.83 (m, 1H), 7.46 (s, 0.5H), 6.92 (s, 0.5H), 4.41 - 3.66 (m, 9H), 2.31 - 1.74 (m, 4H). | 652.0 |
| 105. | 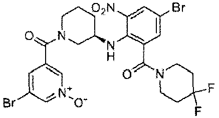 | ¹H NMR (400 MHz, DMSO-*d*₆) δ 8.70 - 8.52 (m, 1H), 8.32 - 8.19 (m, 2H), 7.82 - 7.35 (m, 3H), 4.05 - 3.35 (m, 8H), 3.27 - 3.20 (m, 1H), 2.33 - 1.80 (m, 5H), 1.77 - 1.37 (m, 3H), | 645.9 |
| 106. | 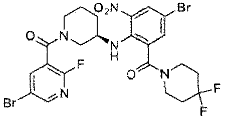 | ¹H NMR (400 MHz, DMSO-*d₆*) δ 8.56 - 8.36 (m, 1H), 8.31 - 8.12 (m, 1.8H), 7.89 - 7.40 (m, 2.2H), 4.10 - 3.41 (m, 7H), 3.27 - 3.13 (m 2H), 2.27 - 2.12 (m, 1.5H), 2.04 - 1.83 (m, 3H), 1.80 - 1.32 (m, 3.5H). | 648.0 |
| 107. | 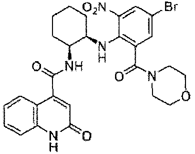 | ¹H NMR (400 MHz, DMSO-*d*₆) δ 11.84 (s, 1H), 8.84 (s, 0.5H), 8.66 (s, 0.5H), 8.25 (s, 1H), 8.00 (d, *J* = 9.6 Hz. 1H), 7.69 (s, 1H), 7.56 - 7.40 (m, 2H), 7.33 (d, *J* = 8.0 Hz. 1H), 7.12 (t, *J* = 7.6 Hz. 1H), 6.52 - 6.36 (m, 1H), 4.35 (s, 1H), 3.91 - 3.50 (m, 9H), 1.82 - 1.24 (m, 8H). | 598.0 |
| 108. | 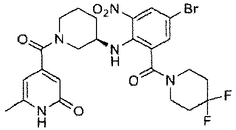 | ¹H NMR (400 MHz, DMSO-*d*₆) δ 11.67 (s, 1H), 8.27 (s, 1H), 7.86 - 7.64 (m, 2H), 6.03 (s, 0.6H), 5.96 - 5.81 (m, 1H), 5.71 - 5.57 (m, 0.4H), 4.07 - 3.35 (m, 6H), 3.24 - 2.95 (m, 3H) , 2.27 - 1.88 (m, 8H), 1.80 - 1.29 (m, 3H). | 582.0 |
| 109. | 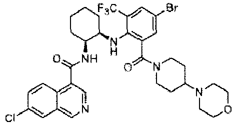 | ¹H NMR (400 MHz, DMSO-*d₆*) δ 9.42 (s, 1H), 8.82 - 8.76 (m, 0.6H), 8.70 - 8.53 (m, 1.4H), 8.37 (s, 1H), 8.20 - 8.12 (m, 1H), 7.87 (d, *J* = 8.8 Hz, 1H), 7.68 (s, 1H), 7.68 - 7.45 (m, 1H), 4.72 - 4.28 (m, 3H), 3.72 - 3.51 (m, 5H), 3.18 - 2.74 (m, 3H), 2.47 - 2.23 (m, 5H), 2.00 - 1.32 (m, 12H). | 722.0 |
| 110. | 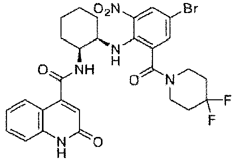 | ¹H NMR (400 MHz, DMSO-*d*₆) δ 11.90 (s, 1H), 8.83 (s, 0.5H), 8.66 (s, 0.5H), 8.26 (s, 1H), 7.99 (d, *J* = 10.0 Hz. 1H), 7.88 -7.75 (m, 1H), 7.59 - 7.40 (m, 2H), 7.33 (d, *J* = 8.4 Hz, 1H), 7.12 (t, *J* = 8.0 Hz. 1H), 6.48 (s, 0.5H), 6.39 (s, 0.5H), 4.31 (s, 1H), 4.06 (s, 1H), 3.74 (s, 1H), 3.63 - 3.45 (m, 3H), 2.30 - 1.84 (m, 4H), 1.83 - 1.24 (m, 8H). | 632.0 |
| 111. | 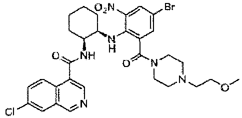 | ¹H NMR (400 MHz, DMSO-*d₆*) δ 9.39 (s, 1H), 8.89 - 8.78 (m, 0.5H), 8.69 - 8.60 (m, 0.5H), 8.57 (s, 1H), 8.35 (s, 1H), 8.25 (s, 1H), 8.13 - 7.99 (m, 2H), 7.81 (d, *J* = 8.4 Hz, 1H), 7.63 (s, 1H), 4.43 (s, 1H), 3.93 - 3.69 (m, 2H), 3.63 - 3.51 (m, 1H), 3.49 - 3.36 (m, 3H), 3.25 - 3.18 (m, 4H), 2.68 - 2.51 (m, 3H), 2.46 - 2.24 (m, 3H), 1.88 - 1.17 (m, 8H). | 673.0 |
| 112. | 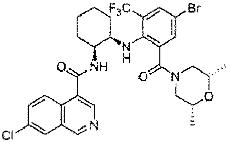 | ¹H NMR (400 MHz, DMSO-*d₆*) δ 9.41 (s, 1H), 8.93 - 8.54 (m, 2H), 8.37 (s, 1H), 8.27 - 8.02 (m, 1H), 7.87 - 7.84 (m, 1H), 7.75 - 7.62 (m, 1H), 7.60 - 7.47 (m, 1H), 4.74 - 4.33 (m, 3H), 3.92 - 3.56 (m, 2H), 3.55 - 3.42 (m, 2.4H), 3.19 - 3.02 (m, 0.6H), 2.96 - 2.71 (m, 1H), 1.99 - 1.36 (m, 7H), 1.32 - 1.09 (m, 4H), 1.07 - 0.79 (m, 3H). | 667.0 |
| 113. | 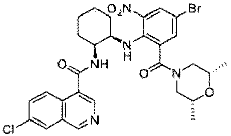 | ¹H NMR (400 MHz, DMSO-*d₆*) δ 9.43 - 9.34 (m, 1H), 8.98 - 8.79 (m, 0.6H), 8.74 - 8.48 (m, 1H), 8.42 - 7.91 (m, 4.4H), 7.87 - 7.76 (m, 1H), 7.71 - 7.60 (m. 1H), 4.61 - 4.26 (m, 2H), 4.05 - 3.51 (m, 5H), 3.01 - 2.69 (m, 1H), 1.93 - 1.29 (m, 8H), 1.26 - 1.10 (m, 3H), 1.09 - 0.68 (m, 3H). | 644.0 |
| 114. | 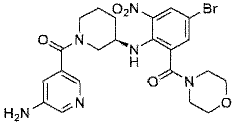 | ¹H NMR (400 MHz, DMSO-*d₆*) δ 8.26 (s, 1H), 8.08 - 7.40 (m, 4H), 6.83 (s, 1H), 5.53 (s, 2H), 3.99 - 3.46 (m, 10.5H), 3.27 - 3.01 (m, 2H), 1.99 - 1.30 (m, 4H). | 533.0 |
| 115. | 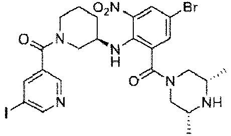 | ¹H NMR (400 MHz, DMSO-*d₆*) δ 8.97 - 8.74 (m, 1H), 8.63 - 8.37 (m, 1H), 8.34 - 7.92 (m, 2H), 7.89 - 7.39 (m, 2H), 4.47 - 4.13 (m, 1H), 4.06 - 3.38 (m, 4H), 3.27 - 2.92 (m, 2H), 2.80 - 2.54 (m, 3H), 2.43 - 2.11 (m, 2H), 2.01 - 1.43 (m, 4H), 1.03 (brs, 3H), 0.88(brs, 3H). | 671.0 |
| 116. | 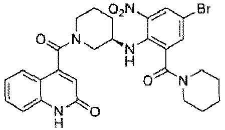 | ¹H NMR (400 MHz, DMSO-*d₆*) δ 11.95 (s, 1H), 8.32 - 8.13 (m, 0.8H), 8.00 - 7.76 (m, 1.2H), 7.69 - 7.13 (m, 5H), 6.57 - 6.09 (m, 1H), 4.13 - 3.39 (m, 5H), 3.25 - 3.02 (m, 4H), 1.95 - 1.24 (m, 10H). | 582.0 |
| 117. | 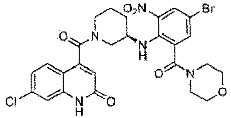 | ¹H NMR (400 MHz, DMSO-*d₆*) δ 12.04 (s, 1H), 8.30 (d, *J* = 2.4 Hz. 0.6H), 8.22 (s, 0.2H), 8.00 - 7.86 (m, 0.4H), 7.82 (d, *J* = 10.0 Hz, 0.5H), 7.73 (d, *J* = 2.4 Hz, 0.8H), 7.63 - 7.42 (m, 0.5H), 7.39 - 7.13 (m, 2.8H), 7.03 - 6.93 (m, 0.2H), 6.55 - 6.34 (m, 0.8H), 6.22 (s, 0.2H), 4.29 - 3.35 (m, 10.5H), 3.24 - 2.97 (m, 2.5H), 2.02 - 1.17 (m, 4H). | 618.0 |
| 118. | 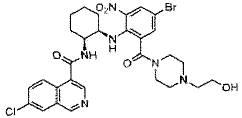 | ¹H NMR (400 MHz, DMSO-*d₆*) δ 9.39 (s, 1H), 8.88 - 8.81 (m, 0.5H), 8.70 - 8.59 (m, 0.5H), 8.57 (s, 1H), 8.35 (s, 1H), 8.25 (s, 1H), 8.11-8.01 (m, 2H), 7.85 - 7.77 (m, 1H), 7.63 (s, 1H), 4.45 (brs, 2H), 3.90 - 3.46 (m, 6H), 2.45 - 2.33 (m, 6H), 1.84 - 1.31 (m, 8H). | 659.0 |
| 119. | 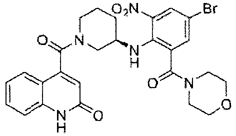 | ¹H NMR (400 MHz, DMSO-*d*₆) δ 12.02 - 11.79 (m, 1H), 8.30 (d, *J* = 2.0 Hz, 0.65H), 8.23 (s, 0.15H), 8.01 - 7.69 (m, 1.85H), 7.63 - 7.25 (m, 3.15H), 7.20 (t, *J* = 7.6 Hz, 1H), 6.99 (t, *J* = 8. 2. 0.15H), 6.55 - 6.30 (m, 0.85H), 6.15 (s, 0.15H), 4.22 - 3.42 (m, 9H), 3.28 - 2.84 (m, 4H), 2.04 - 1.36 (m, 4H). | 584.0 |
| 120. | 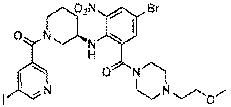 | ¹H NMR (400 MHz, DMSO-*d*₆) δ 8.97 - 8.73 (m, 1H), 8.63 - 8.41 (m, 1H), 8.32 - 7.93 (m, 2H), 7.86 - 7.42 (m, 2H), 3.98 - 3.39 (m, 8H), 3.29 - 3.08 (m, 6H), 2.72 -2.51 (m, 3H), 2.45 - 2.07 (m, 3H), 2.04 - 1.40 (m, 4H). | 701.0 |
| 121. | 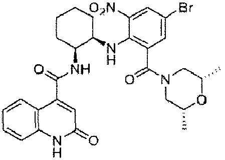 | ¹H NMR (400 MHz, DMSO-*d*₆) δ 11.91 (s, 1H), 8.99 - 8.54 (m, 1H), 8.25 (s, 1H), 8.13 - 7.92 (m, 1H), 7.76 - 7.43 (m, 2.7H), 7.40 - 7.25 (m, 1.3H), 7.20 - 7.05 (m, 1H), 6.57 - 6.25 (m, 1H), 4.50 - 4.20 (m, 2H), 4.04 - 3.49 (m, 4H), 3.04 - 2.72 (m, 1H), 2.62 - 2.52 (m, 1H), 1.95 - 1.11 (m, 11H), 1.09 -0.84 (m, 3H). | 626.1 |
| 122. | 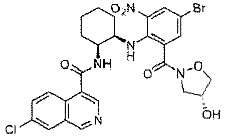 | ¹H NMR (400 MHz, DMSO-*d*₆) δ 9.36 (s, 1H), 8.70 (d, *J* = 8.4 Hz, 1H), 8.45 (s, 1H), 8.33 (d, *J* = 2.0 Hz, 1H), 8.23 (d, *J* = 2.4 Hz. 1H), 8.14 (d, *J* = 10.0 Hz, 1H), 8.06 (d, *J* = 9.2 Hz, 1H), 7.77 (dd, *J* = 9.2, 2.0 Hz, 1H), 7.68 (d, *J* = 1.6 Hz, 1H), 5.62 (d, *J* = 4.0 Hz, 1H), 4.74 (s, 1H), 4.34 (s, 1H), 4.07 - 3.79 (m, 4H), 3.63 (d, *J* = 11.6 Hz, 1H), 1.88 - 1.77 (m, 1H), 1.75 - 1.58 (m, 4H), 1.56 - 1.36 (m, 3H). | 618.0 |
| 123. | 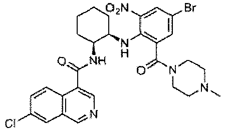 | ¹H NMR (400 MHz, DMSO-*d*₆) δ 9.39 (s, 1H), 8.85 (brs, 0.5H), 8.63 (brs, 0.5H), 8.56 (s, 1H), 8.35 (s, 1H), 8.25 (s, 1H), 8.13 - 8.42 (m, 2H), 7.81 (d, *J* = 8.8 Hz, 1H), 7.64 (s, 1H), 4.44 (s, 1H), 3.92 - 3.69 (m, 2H), 3.59 (brs, 1H), 3.26 - 3.16 (m, 2H), 2.43 - 2.27 (m, 2H), 2.20 (s, 3H), 1.87 - 1.35 (m, 10H). | 629.0 |
| 124. | 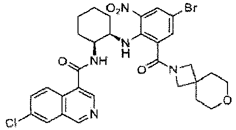 | ¹H NMR (400 MHz, DMSO-*d*₆) δ 9.37 (s, 1H), 8.75 (d, *J* = 8.4 Hz, 1H), 8.39 (s, tH), 8.34 (d, *J* = 2.4 Hz, 1H), 8.24 (d, *J* = 2.4 Hz, 1H), 8.09 (d, *J* = 10.0 Hz, 1H), 8.03 (d, *J* = 8.8 Hz. 1H), 7.80 (dd, *J* = 9.2, 2.4 Hz, 1H), 7.77 (d, *J* = 2.8 Hz, 1H), 4.35 (s, 1H), 3.91 (s, 1H), 3.84 (s, 2H), 3.76 (s, 2H), 3.55 - 3.35 (m, 3H), 3.30 - 3.26 (m, 1H), 1.93 - 1.82 (m, 1H), 1.80 - 1.59 (m, 8H), 1.57 - 1.35 (m, 3H). | 656.0 |
| 125. | 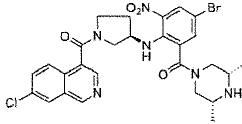 | ¹H NMR (400 MHz, DMSO-*d*₆) δ 9.43 - 9.33 (m, 1H), 8.60 - 8.43 (m, 1H), 8.41 - 8.33 (m, 1H), 8.30 - 8.13 (m, 1H), 7.90 - 7.40 (m, 4H), 4.49 - 3.64 (m, 6H), 3.22 - 2.87 (m, 2H), 2.85 - 2.6 (m, 2H), 2.44 - 2.08 (m, 3H), 2.00 - 1.83 (m, 0.6H), 1.79 - 1.58 (m, 0.4H), 1.13 - 0.84 (m, 5H), 0.71 - 0.50 (m, 1H). | 615.2 |
| 126. | 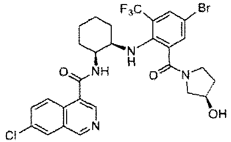 | ¹H NMR (400 MHz, DMSO-*d*₆) δ 9.41 (d, *J* = 1.6 Hz, 1H), 8.68 (d, *J* = 8.0 Hz, 1H), 8.62 (s, 0.5H), 8.60 (s, 0.5H), 8.37 (d, *J* = 1.6 Hz, 1H), 8.16 (d, *J* = 9.2 Hz, 0.5H), 8.10 (d, *J* = 9.2 Hz, 0.5H), 7.87 (t, *J* = 2.0 Hz, 0.5H), 7.85 (t, *J* = 2.0 Hz, 0.5H), 7.69 (d, *J* = 2.4 Hz, 0.5H), 7.66 (d, *J* = 2.4 Hz, 0.5H), 7.54 (d, *J* = 2.4 Hz, 1H), 5.08 - 5.04 (m, 1H), 4.77 (d, *J* = 10.0 Hz. 0.5H), 4.66 (d, *J* = 10.4 Hz, 0.5H), 4.45 - 4.24 (m, 2H), 3.65 - 3.35 (m, 4.5H), 3.17 (d, J= 11.2 Hz, 0.5H), 2.00 -1.36 (m, 9H), 1.31 - 1.16 (m, 1H), | 639.0 |
| 127. | 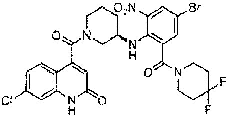 | ¹H NMR (400 MHz, DMSO-*d*₆) δ 12.02 (s, 1H), 8.30 (d, *J* = 2.4 Hz, 0.6H), 8.23 (s, 0.2H), 8.00 - 7.47 (m, 2.2H), 7.45 - 7.10 (m, 2.8H), 6.96 (s, 0.2H), 6.55 - 6.14 (m, 1H), 4.26 - 3.38 (m, 6H), 3.30 - 2.87 (m, 3H), 2.25 - 1.24 (m, 8H). | 652.0 |
| 128. | 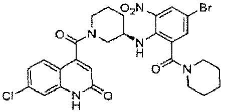 | ¹H NMR (400 MHz, DMSO-*d*₆) δ 12.16 - 11.75 (m, 1H), 8.28 (d, *J* = 2.4 Hz, 0.5H), 8.19 (s, 0.2H), 7.99 - 7.75 (m, 1H), 7.68 - 7.61 (m, 0.5H), 7.61 - 6.93 (m, 3.8H), 6.57 - 6.14 (m, 1H), 4.25 - 3.38 (m, 5H), 3.27 - 2.79 (m, 4H), 1.99 - 1.23 (m, 10H). | 616.0 |
| 129. | 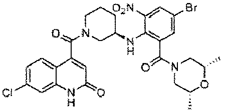 | ¹H NMR (400 MHz, DMSO-*d*₆) δ 12.02 (s, 1H), 8.30 (s, 0.6H), 8.23 - 6.91 (m, 5.4H), 6.59 - 6.10 (m, 1H), 4.50 - 3.38 (m, 8H), 3.27 - 2.70 (m, 3H), 1.95 - 1.28 (m, 4H), 1.24 - 0.80 (m, 6H). | 646.0 |
| 130. | 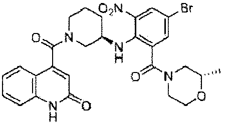 | ¹H NMR (400 MHz, DMSO-*d*₆) δ 11.96 (s, 1H), 8.34 - 7.98 (m, 1H), 7.92 - 7.52 (m, 3H), 7.49 - 6.96 (m, 3H), 6.53 - 6.20 (m, 1H), 4.45 - 3.44 (m, 7H), 3.25 - 2.56 (m, 5H), 2.03 - 0.79 (m, 8H). | 598.0 |
| 131. | 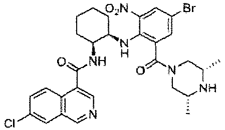 | ¹H NMR (400 MHz, DMSO-*d*₆) δ 9.39 (s, 1H), 8.93 - 8.47 (m, 2H), 8.44 - 7.93 (m, 4H), 7.86 -7.73 (m, 1H), 7.66 - 7.53 (m, 1H), 4.56 - 4.28 (m, 2H), 4.01 - 3.51 (m, 1H), 3.28 - 3.14 (m, 1H), 2.82 -2.51 (m, 3.5H), 2.37 - 2.20 (m, 1.5H), 1.87 - 1.29 (m, 8H), 1.11 - 0.97 (m, 3H), 0.93 - 0.60 (m, 3H). | 643.0 |
| 132. | 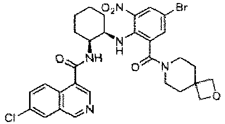 | ¹H NMR (400 MHz, DMSO-*d*₆) δ 9.45 - 9.33 (m, 1H), 8.84 (d, *J* = 7.2 Hz. 0.5H), 8.63 (d, *J* = 7.6 Hz. 0.5H), 8.56 (s, 1H), 8.39 - 8.30 (m, 1H), 8.29 - 8.20 (m, 1H), 8.14 - 7.99 (m, 2H), 7.87 - 7.76 (m, 1H), 7.63 (d, *J* = 2.0 Hz, 1H), 4.47 - 4.04 (m, 5H), 3.91 - 3.46 (m, 3H), 3.26 - 3.12 (m, 2H), 1.89 - 1.22 (m, 12H). | 656 |
| 133. | 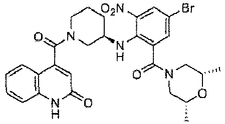 | ¹H NMR (400 MHz, DMSO-*d*₆) δ 12.05 - 11.70 (m, 1H), 8.35 - 8.19 (m, 1H), 8.11 - 6.97 (m, 6H), 6.56 - 6.09 (m, 1H), 4.49 - 4.18 (m, 1H), 4.16 - 3.38 (m, 7H), 3.29 - 2.70 (m, 3H), 1.96 - 1.46 (m, 3H), 1.23 - 0.95 (m, 7H). | 612 |
| 134. | 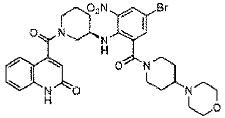 | ¹H NMR (400 MHz, DMSO-*d*₆) δ 11.93 (s, 1H), 8.38 - 8.07 (m, 1H), 8.05 - 7.42 (m, 3H), 7.40 - 6.94 (m, 3H), 6.57 - 5.92 (m, 1H), 4.64 - 3.62 (m, 4H), 3.60 - 3.38 (m, 6H), 3.26 - 2.73 (m, 4H), 2.47 - 2.35 (m, 4H), 1.99 - 1.19 (m, 8H). | 667.1 |
| 135. | 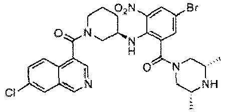 | ¹H NMR (400 MHz, DMSO-*d*₆) δ 9.42 - 9.22 (m, 1H), 8.59 - 8.15 (m, 2.7H), 7.99 - 7.56 (m, 4H), 7.35 - 7.21 (m, 0.3H), 4.59 - 3.39 (m, 5H), 3.20 - 2.93 (m, 2H), 2.88 - 2.52 (m, 2H), 2.49 - 2.07 (m, 2H), 2.02 - 1.12 (m, 5H), 1.05 - 0.73 (m, 6H). | 629.0 |
| 136. | 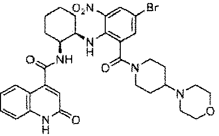 | ¹H NMR (400 MHz, DMSO-*d*₆) δ 11.98 - 11.81 (m, 1H), 8.93 - 8.73 (tn, 0.5H), 8.73 - 8.50 (m, 0.5H), 8.30 -8.17 (m, 1H), 8.13 -7.91 (m, 1H), 7.72 - 7.57 (m, 1H), 7.56 - 7.37 (m, 2H), 7.34 - 7.27 (m, 1H), 7.18 - 7.07 (m, 1H), 6.51 - 6.39 (m, 1H), 4.59 - 4.17 (m, 2H), 3.91 - 3.38 (m, 6H), 3.27 - 2.63 (m, 3H), 2.49 - 2.39 (m, 4H), 1.95 - 1.25 (m, 12H). | 681.2 |
| 137. | 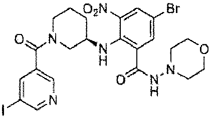 | ¹H NMR (400 MHz, DMSO-*d*₆) δ 9.96 - 9.73 (m, 1H), 8.95 - 8.75 (m, 1H), 8.63 - 8.43 (m, 1H), 8.30 - 8.06 (m, 2H), 8.03 - 7.86 (m, 1H), 7.79 - 7.60 (m, 1H), 4.10 - 3.88 (m, 1H), 3.87 - 3.58 (m, 5H), 3.56 - 3.36 (m, 2H), 3.27 - 3.12 (m, 2H), 2.86 (brs, 4H), 1.92 - 1.66 (m, 2H), 1.63 - 1.38 (m, 2H). | 659.0 |
| 138. | 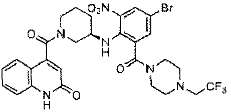 | ¹H NMR (400 MHz, DMSO-*d*₆) δ 11.95 (s, 1H), 8.34 -8.18 (m, 1H), 7.99 - 7.16 (m, 6H), 6.56 -6.14 (m, 1H), 4.12 - 3.61 (m, 5H), 3.26 - 3.08 (m, 4H), 2.85 - 2.62 (m, 4H), 2.10 - 1.24 (m, 6H). | 665.1 |
| 139. | 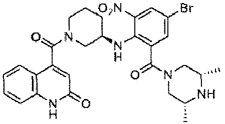 | ¹H NMR (400 MHz, DMSO-*d*₆) δ 12.03 - 11.78 (m, 1H), 8.32 - 8.00 (m, 1H), 7.97 - 7.72 (m, 1H), 7.68 - 7.45 (m, 2H), 7.42 - 7.15 (m, 3H), 6.55 -6.26 (m, 1H), 4.53 -4.14 (m, 1H), 4.09 - 3.38 (m, 4H), 3.28 - 2.84 (m, 3H), 2.78 - 2.70 (m, 1H), 2.67 - 2.58 (m, 1H), 2.42 - 2.13 (m, 2H), 2.01 - 1.38 (m, 4H), 1.22 - 0.96 (m, 3H), 0.95 - 0.62 (m, 3H). | 611.1 |
| 140. | 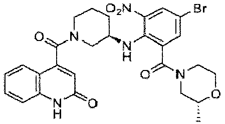 | ¹H NMR (400 MHz, DMSO-*d*₆) δ 11.95 (s, 1H), 8.35 - 8.17 (m, 1H), 8.13 - 7.09 (m, 6H), 6.58 - 6.03 (m, 1H), 4.49 - 3.40 (m, 7H), 3.27 - 2.56 (m, 5H), 2.06 - 1.29 (m, 4H), 1.24 - 0.74 (m, 3H). | 598.0 |
| 141. | 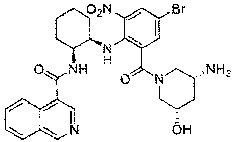 | ¹H NMR (400 MHz, DMSO-*d*₆) δ 9.39 - 9.37 (m, 1H), 8.82 - 8.47 (m, 2H), 8.29 - 7.94 (m, 4H), 7.79 - 7.63 (m, 3H), 5.19 - 5.04 (m, 1H), 4.52 - 4.38 (m, 2H), 3.76 - 3.53 (m, 2H), 2.76 - 2.33 (m, 4H), 2.08 - 1.12 (m, 12H) | 611.0 |
| 142. | 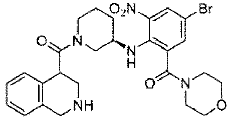 | ¹H NMR (400 MHz, DMSO-*d*₆) δ 8.31 - 8.21 (m, 1H), 7.91 -7.73 (m, 1H), 7.67 (brs, 1H), 7.18 - 6.98 (m, 3H), 6.93 - 6.76 (m, 1H), 4.26 - 4.08 (m, 1H), 4.00 - 3.44 (m, 13H), 3.18 - 2.74 (m, 4H), 2.01 -1.88 (m, 1H), 1.76 - 1.34 (m, 3H). | 572.1 |
| 143. | 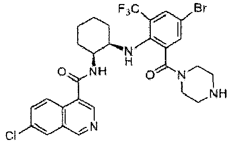 | ¹H NMR (400 MHz, DMSO-*d*₆) δ 9.42 (s, 1H), 8.79 (brs, 0.6H), 8.63 (brs, 1.4H), 8.37 (s, 1H), 8.16 (d, *J* = 8.8 Hz, 1H), 7.87 (d, *J* = 8.0 Hz, 1H), 7.69 (s, 1H), 7.58 (s, 1H), 5.46 - 5.27 (m, 0.6H), 4.69 - 4.40 (m, 2.4H), 3.81 - 3.51 (m, 5H), 3.00 - 2.71 (m, 4H), 2.06 - 1.72 (m, 2H), 1.69 - 1.37 (m, 6H). | 638.0 |
| 144. | 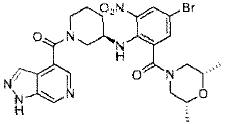 | ¹H NMR (400 MHz, DMSO-*d*₆) δ 13.95 (s, 1H), 9.11 (s, 1H), 8.34 - 7.54 (m, 5H), 4.54 - 3.41 (m, 10H), 2.99 - 2.71 (m, 1H), 2.03 - 1.43 (m. 4H), 1.21 -0.89 (m, 6H). | 586.0 |
| 145. | 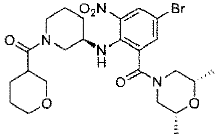 | ¹H NMR (400 MHz, DMSO-*d*₆) δ 8.25 (s, 1H), 7.75 - 7.63 (m, 2H), 4.37 - 4.34 (m, 1H), 3.79 - 3.25 (m, 10H), 2.83 - 2.80 (m, 2H), 2.49 - 2.45 (m, 1H), 1.92 - 1.40 (m, 8H), 1.15 (d, *J* = 6.1 Hz, 3H), 1.02 (br. 3H). | 553.0 |
| 146. | 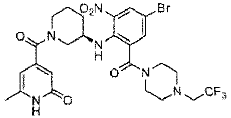 | ¹H NMR (400 MHz, DMSO-*d*₆) δ 11.77 (s, 1H), 8.27 (s, 1H), 7.79 - 7.70 (m, 1H), 7.67 - 7.54 (m, 1H), 6.04 (s, 0.65H), 5.94 - 5.78 (m, 1H), 5.76 - 5.50 (m, 0.35H), 3.91 - 3.43 (m, 4H), 3.27 - 3.11 (m, 5H), 2.85 - 2.56 (m, 4H), 2.18 (s, 2H), 2.08 (s, 1H), 2.04 - 1.94 (m, 2H), 1.91 - 1.65 (m, 2H), 1.61 - 1.42 (m, 2H). | 629.0 |
| 147. | 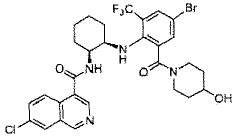 | ¹H NMR (400 MHz, DMSO-*d*₆) δ 9.41 (s, 1H), 8.84 - 8.72 (m, 0.6H), 8.69 - 8.54 (m, 1.4H), 8.39 - 8.32 (m, 1H), 8.16 (d, *J* = 8.8 Hz, 1H), 7.90 - 7.82 (m, 1H), 7.71 -7.63 (m, 1H), 7.54 -7.48 (m, 1H), 4.91 - 4.78 (m, 1H), 4.66 - 4.39 (m, 2H), 4.18 - 3.92 (m, 1H), 3.87 - 3.66 (m, 1H), 3.60 - 3.43 (m, 2H), 3.14 - 3.04 (m, 2H), 1.92 - 1.37 (m, 12H). | 653.0 |
| 148. | 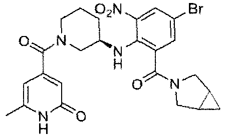 | ¹H NMR (400 MHz, DMSO-*d*₆) δ 11.76 (s, 1H), 8.27 - 8.17 (m, 1H), 7.79 - 7.57 (m, 2H), 6.07 - 5.58 (m, 2H), 3.86 - 3.66 (m, 2H), 3.63 - 3.35 (m, 5H), 3.26 - 2.89 (m, 2H), 2.17 (s, 2H), 2.12 - 2.03 (m, 1H), 1.95 - 1.77 (m, 1H), 1.75 - 1.35 (m, 5H), 0.78 - 0.62 (m, 1H), 0.18 - 0.04 (m, 1H). | 544.0 |
| 149. | 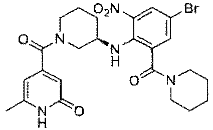 | ¹H NMR (400 MHz, DMSO-*d*₆) δ 11.75 (s, 1H), 8.25 (s, 1H), 7.88 - 7.70 (m, 1H), 7.66 - 7.48 (m, 1H), 6.02 (s, 0.6H), 5.94 - 5.78 (m, 1H), 5.64 (m, 0.4H), 3.98 - 3.36 (m, 6H), 3.27 - 2.83 (m, 3H), 2.19 (s, 1.8H), 2.07 (s, 1.2H), 1.95 - 1.82 (m, 1H), 1.80 - 1.31 (m, 9H). | 546.0 |
| 150. | 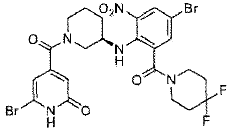 | ¹H NMR (400 MHz, DMSO-*d*₆) δ 8.27 (s, 1H), 7.98 - 7.54 (m, 2H), 6.68 - 5.80 (m, 2H), 4.08 - 3.41 (m, 6H), 3.21 - 2.77 (m, 3H), 2.23 - 1.82 (m, 5H), 1.79 - 1.39 (m, 3H). | 646.0 |
| 151. | 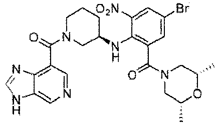 | ¹H NMR (400 MHz, DMSO-*d*₆): δ 13.10 (s, 1H), 8.97 (s, 1H), 8.57 - 8.04 (m, 3H), 7.96 - 7.29 (m, 2H), 4.55 - 3.34 (m, 8H), 3.24 - 2.73 (m, 3H), 2.08 - 1.51 (m, 4H), 1.34 - 0.65 (m, 6H). | 586.1 |
| 152. | 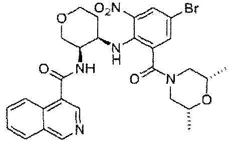 | ¹H NMR (400 MHz, DMSO-*d*₆) δ 9.40 (s, 1H), 9.00 - 8.79 (m, 1H), 8.67 - 8.57 (m, 1H), 8.28 (d, *J* = 2.4 Hz, 1H), 8.20 - 8.13 (m, 1H), 8.07 - 7.63 (m, 5H), 4.47 - 4.44 (m, 2H), 4.01 - 3.34 (m, 8H), 3.00 - 2.80 (m, 1H), 2.67 - 2.49 (m, 1H), 2.07 - 1.74 (m, 2H), 1.20 - 1.19 (m, 3H), 1.03 - 1.01 (m, 3H). | 612.1 |
| 153. | 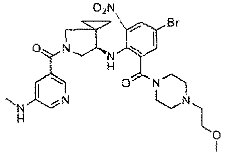 | ¹H NMR (400 MHz, DMSO-*d*₆) δ 8.29 (s, 0.5H), 8.24 (s, 0.5H), 7.98 (d, *J* = 2.4 Hz, 0.5H), 7.95 (d, *J* = 2.4 Hz, 0.5H), 7.91 - 7.51 (m, 3H), 6.94 - 6.62 (m, 1H), 6.25 - 5.96 (m, 1H), 3.91 - 3.41 (m, 8H), 3.28 - 3.06 (m, 6H), 2.86 - 2.54 (m, 7H), 2.45 - 2.06 (m, 2H), 0.85 - 0.62 (m, 4H). | 616.2 |
| 154. | 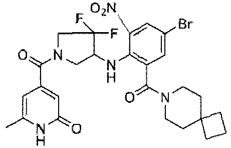 | ¹H NMR (400 MHz, DMSO-*d*₆) δ 11.85 (s, 1H), 8.25 (s, 1H), 7.86 - 7.46 (m, 1.5H), 7.10 (s, 0.3H), 6.92 (s, 0.2H), 6.19 (s, 1H), 6.00 (s, 1H), 4.44 - 4.18 (m, 1H), 4.08 - 3.84 (m, 3H), 3.81 -3.54 (m, 3H), 3.21 -2.83 (m, 2H), 2.20 (s, 3H), 1.96 - 1.21 (m, 10H). | 608 |
| 155. | 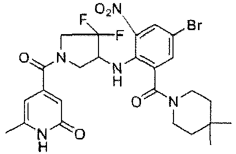 | ¹H NMR (400 MHz, DMSO-*d*₆) δ 11.83 (s, 1H), 8.30 - 8.22 (m, 1H), 7.79 - 7.46 (m, 1.5H), 7.09 - 6.88 (m, 0.5H), 6.19 (s, 1H), 6.00 (s, 1H), 4.47 - 3.39 (m, 7H), 3.29 - 2.87 (m, 2H), 2.18 (s, 3H), 1.49 - 1.08 (m, 4H), 1.02 - 0.74 (m, 6H). | 596 |
| 156. | 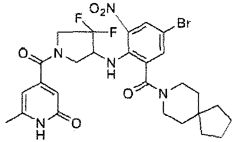 | ¹H NMR (400 MHz, DMSO-*d*₆) δ 11.85 (s, 1H), 8.25 (s, 1H), 7.84 - 7.44 (m, 1.5H), 7.15 - 6.87 (m, 0.5H), 6.20 (s, 1H), 6.00 (s, 1H), 4.49 - 3.42 (m, 7H), 3.25 - 2.92 (m, 2H), 2.19 (s, 3H), 1.49 - 1.08 (m, 4H), 1.68 - 1.13 (m, 12H). | 622.0 |
| 157. | 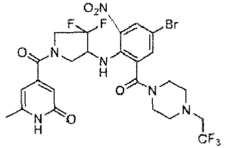 | ¹H NMR (400 MHz, DMSO-*d*₆) δ 11.59 (brs, 1H), 8.33 - 8.22 (s, 1H), 7.82 - 7.68 (m, 1H), 7.58 (s, 0.5H), 6.97 (brs, 0.5H), 6.21 (s, 1H), 6.00 (s, 1H), 4.42 - 4.22 (m, 1H), 4.16 - 3.40 (m, 6.5H), 3.30 - 3.02 (m, 3.5H), 2.86 - 2.55 (m, 3.5H), 2.47 - 2.31 (m, 0.5H), 2.19 (s, 3H). | 651.0 |
| 158. | 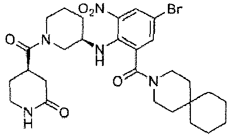 | ¹H NMR (400 MHz, DMSO-*d*₆) δ 8.23 (d, *J* = 2.5 Hz, 1H), 7.80 - 7.73 (m, 1H), 7.60 (s, 1H), 7.45 (s, 1H), 3.78 - 3.45 (m, 6H), 3.31 - 3.12 (m, 6H), 2.14 - 2.07 (m, 2H), 1.89 - 1.79 (m, 2H), 1.62 - 1.31 (m, 18H). | 604.2 |
| 159. | 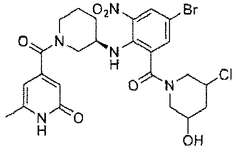 | ¹H NMR (400 MHz, DMSO-*d*₆) δ 11.77 (s, 1H), 8.28 - 8.25 (m, 1H), 7.88 - 7.45 (m, 2H), 6.05 - 5.69 (m, 2H), 5.12 (br. 1H), 4.54 - 3.61 (m, 7H), 3.19 - 3.16 (m, 3H), 2.19 - 1.55 (m, 10H). | 596.1 |
| 160. | 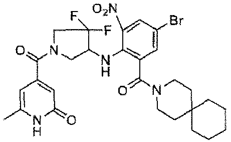 | ¹H NMR (400 MHz, DMSO-*d*₆) δ 11.85 (s, 1H), 8.24 (d, *J* = 2.4 Hz, 1H), 7.81 - 7.68 (m, 1H), 7.66 - 7.45 (m, 0.5H), 7.15 - 6.90 (m, 0.5H), 6.19 (s, 1H), 5.99 (s, 1H), 4.44 - 4.14 (m, 1H), 4.11 - 3.52 (m, 6H), 3.27 - 2.89 (m, 2H), 2.18 (s, 3H), 1.60 - 1.24 (m, 13H), 1.19-1.02 (m, 1H). | 636.1 |
| 161. | 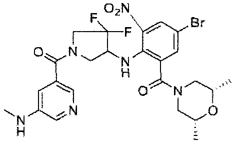 | ¹H NMR (400 MHz, DMSO-*d*₆) δ 8.27 (s, 1H), 8.03 (s, 1H), 7.87 (s, 1H), 7.81 - 7.69 (m, 1H), 7.66 - 7.41 (m, 0.5H), 7.26 - 6.80 (s, 1.5H), 6.18 (brs, 1H), 4.56 - 3.36 (m, 8H), 3.23 - 2.59 (m, 5H), 2.45 - 2.32 (m, 1H), 1.30 - 0.79 (m, 6H). | 597.0 |
| 162. | 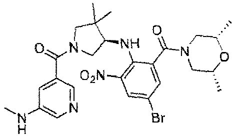 | ¹H NMR (400 MHz, DMSO-*d*₆) δ 8.31 - 8.19 (m, 1H), 8.04 - 7.94 (m, 1H), 7.91 - 7.81 (m, 1H), 7.74 - 7.47 (m, 2H), 6.89 (m, 1H), 6.20 - 6.06 (s, 1H), 4.49 - 4.32 (m, 0.5H), 4.27 - 4.11 (m, 0.5H), 4.05 - 3.37 (m, 6H), 3.28 - 2.77 (m, 3H), 2.72 - 2.66 (m, 3H), 2.64 - 2.53 (m, 0.5H), 2.46 - 2.31 (m, 0.5H), 1.18 - 0.77 (m, 12H). | 589.2 |
| 163. | 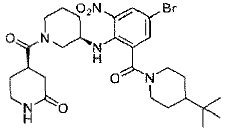 | ¹H NMR (400 MHz, DMSO-*d*₆) δ 8.24 (d, *J* = 2.4 Hz, 1H), 7.77 - 7.58 (m, 2H), 7.46 (br, 1H), 4.58 - 4.53 (m, 1H), 3.94 - 3.32 (m, 4H), 3.13 - 3.12 (m, 5H), 2.67 - 2.66 (m, 1H), 2.13 - 1.00 (m, 14H), 0.84 (s, 9H). | 592.1 |
| 164. | 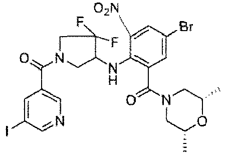 | ¹H NMR (400 MHz, DMSO-*d*₆) δ 8.95 (s, 1H), 8.72 (s, 1H), 8.39 - 8.20 (m, 2H), 7.88 - 7.64 (m, 1H), 7.61 - 7.30 (m, 0.5H), 7.19 - 6.99 (m, 0.5H), 4.76 - 4.10 (m, 5H), 3.87 - 2.70 (m, 6H), 1.22 - 0.80 (m, 6H). | 693.9 |
| 165. | 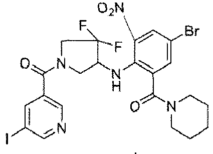 | ¹H NMR (400 MHz, DMSO-*d*₆) δ 8.95 (s, 1H), 8.72 (s, 1H), 8.42 - 8.29 (m, 1H), 8.24 (s, 1H), 7.81 - 7.65 (m, 1H), 7.55 (s, 0.5H), 7.14 - 6.90 (m, 0.5H), 4.48 - 3.86 (m, 4H), 3.83 - 3.42 (m, 3H), 3.26 - 2.83 (m, 2H), 1.72 - 1.11 (m, 6H). | 664.0 |
| 166. | 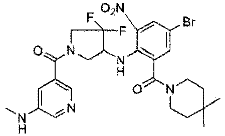 | ¹H NMR (400 MHz, DMSO-*d*₆): δ 8.25 (s, 1H), 8.02 (d, *J* = 2.4 Hz. 1H), 7.87 (s, 1H), 7.81 - 7.52 (m, 1.5H), 7.16 - 6.97 (m, 0.5H), 6.92 (s, 1H), 6.17 (brs, tH), 4.49 - 4.17 (m, 1H), 4.16 - 3.51 (m, 5H), 3.26 - 2.83 (m, 3H), 2.72 (d, *J* = 4.8 Hz. 3H), 1.53 - 1.10 (m, 4H), 1.05 - 0.66 (m, 6H). | 595.1 |
| 167. | 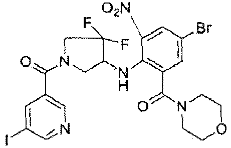 | ¹H NMR (400 MHz, DMSO-*d*₆): δ 8.94 (s, 1H), 8.72 (s, 1H), 8.33 (s, 1H), 8.30 - 8.19 (m, 1H), 7.83 (s, 0.5H), 7.77 (s, 0.5H), 7.47 (s, 0.5H), 6.93 (s, 0.5H), 4.38 - 3.47 (m, 13H). | 665.9 |
| 168. | 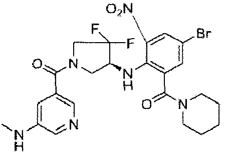 | ¹H NMR (400 MHz, DMSO-*d*₆) δ 8.25 (s, 1H), 8.03 (s, 1H), 7.87 (s, 1H), 7.81 - 7.52 (m, 1.5H), 7.18 (s, 0.3H), 6.93 (s, 1.2H), 6.18 (brs, 1H), 4.46 - 3.36 (m, 7H), 3.28 - 2.81 (m, 2H), 2.72 (d, *J* = 4.8 Hz, 3H), 1.75 - 1.08 (m, 6H). | 567.0 |
| 169. | 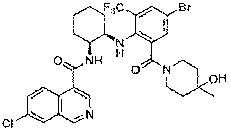 | ¹H NMR (400 MHz, DMSO-d₆): δ 9.41 (s, 1H), 8.86 - 8.71 (m, 0.6H), 8.68 - 8.52 (m, 1.4H), 8.37 (s, 1H), 8.21 - 8.10 (m, 1H), 7.86 (d, *J* = 8.8 Hz, 1H), 7.67 (s, 1H), 7.53 (d, *J* = 10.8 Hz, 1H), 4.73 - 4.31 (m, 3H), 4.22 - 4.04 (m, 1H), 3.69 -3.49 (m, 1H), 3.29 - 3.06 (m, 3H), 1.96 - 1.83 (m, 1H), 1.80 - 1.32 (m, 11H), 1.20 - 1.09 (m, 3H). | 667.0 |
| 170. | 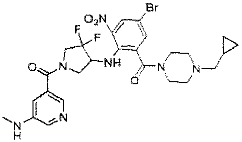 | ¹H NMR (400 MHz, DMSO-*d*₆): δ 8.32 - 8.22 (m, 1H), 8.06 - 7.98 (m, 1H), 7.87 (brs, 1H),7.82 - 7.46 (m, 1.6H), 7.06 - 6.82 (m, 1.4H), 6.17 (brs, 1H), 4.47 - 3.43 (m, 7H), 3.22 - 2.91 (m, 2H), 2.72 (d, *J* = 4.8 Hz, 3H), 2.67 -2.55 (m, 1H), 2.46 -2.36 (m, 1H), 2.32 - 1.77 (m, 4H), 0.91 - 0.59 (m, 1H), 0.55 - 0.31 (m, 2H), 0.16 - 0.01 (m, 2H). | 622.2 |
| 171. | 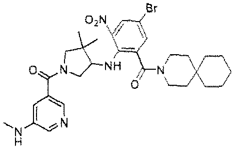 | ¹H NMR (400 MHz, DMSO-*d*₆): δ 8.25 (d, *J* = 2.0 Hz, 0.4H), 8.22 (d, *J* = 2.4 Hz, 0.6H), 7.99 (d, *J* = 2.8 Hz, 1H), 7.86 (s, 0.6H), 7.83 (s, 0.4H), 7.73 - 7.46 (m, 2H), 6.88 (s, 1H), 6.18 - 6.06 (m, 1H), 3.98 - 3.42 (m, 5H), 3.30 - 3.11 (m, 3H), 3.06 - 2.80 (m, 1H), 2.71 (d, *J* = 4.8 Hz. 3H), 1.62 - 1.09 (m, 15H), 1.07 - 0.84 (m, 5H). | 627.2 |
| 172. | 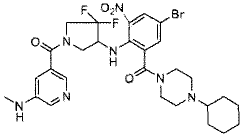 | ¹H NMR (400 MHz, DMSO-d₆) δ 8.25 (s, 1H), 8.03 (d, *J* = 2.4 Hz, 1H), 7.86 (s, 1H), 7.80 - 7.54 (m, 1.6H), 6.92 (brs, 1.4H), 6.24 - 6.06 (m, 1H), 4.46 - 3.38 (m, 7H), 3.27 - 2.97 (m, 2H), 2.72 (d, *J* = 4.0 Hz. 3H), 2.67 - 2.54 (m, 1H), 2.45 - 1.96 (m, 4H), 1.78 - 1.50 (m, 5H), 1.27 - 0.95 (m, 5H). | 650.2 |
| 173. | 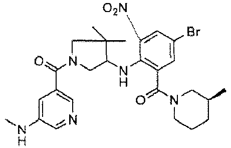 | ¹H NMR (400 MHz, DMSO-*d*₆): δ 8.31 - 8.18 (m, 1H), 8.03 - 7.95 (m, 1H), 7.91 - 7.81 (m, 1H), 7.78 - 7.47 (m, 2H), 6.89 (brs, 1H), 6.11 (brs, 1H), 4.44 - 3.52 (m, 6H), 3.16 - 2.90 (m, 3H), 2.73 - 2.69 (m, 3H), 1.85 - 1.35 (m, 4H), 1.18 - 0.57 (m, 10H). | 573.2 |
| 174. | 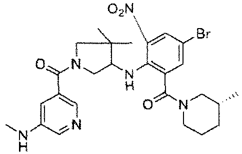 | ¹H NMR (400 MHz, DMSO-*d*₆): δ 8.30 - 8.19 (m, 1H), 8.04 - 7.94 (m, 1H), 7.90 - 7.78 (m, 1H), 7.75 - 7.50 (m, 2H), 6.89 (brs, 1H), 6.12 (brs, 1H), 4.53 - 4.07 (m, 111). 4.06 - 3.37 (m, 511), 3.28 - 2.95 (m, 3H), 2.72 - 2.67 (m, 3H), 1.87 - 1.26 (m, 4H), 1.20 - 0.51 (m, 10H). | 573.1 |
| 175. | 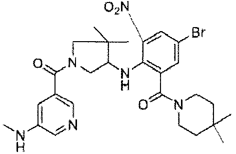 | ¹H NMR (400 MHz, DMSO-*d*₆) δ 8.26 (d, *J* = 2.4 Hz, 0.4H), 8.22 (d, *J* = 2.4 Hz, 0.6H), 8.02 - 7.95 (m, 1H), 7.86 (s, 0.6H), 7.84 (s, 0.4H), 7.74 - 7.46 (m, 2H), 6.88 (s, 1H), 6.17 - 6.06 (m, 1H), 3.99 - 3.45 (m, 5H), 3.27 - 2.86 (m, 4H), 2.78 - 2.71 (m, 3H), 1.54 - 0.69 (m, 16H). | 587.2 |
| 176. | 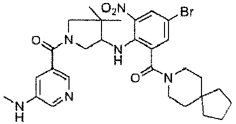 | ¹H NMR (400 MHz, DMSO-*d*₆) δ 8.26 (d, *J* = 2.0 Hz. 0.4H), 8.23 (d, *J* = 2.4 Hz, 0.6H), 7.99 (s, 1H), 7.86 (s, 0.6H), 7.83 (s, 0.4H), 7.75 - 7.47 (m, 2H), 6.88 (s, 1H), 6.21 - 6.02 (m, 1H), 4.01 - 3.45 (m, 6H), 3.26 - 3.14 (m, 2H), 3.07 - 2.81 (m, 1H), 2.71 (brs, 3H), 1.77 - 1.30 (m, 11H), 1.20 - 0.80 (m, 7H). | 613.2 |
| 177. | 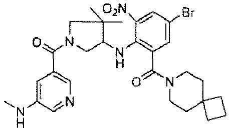 | ¹H NMR (400 MHz, DMSO-*d*₆): δ 8.26 (d, *J* = 2.4 Hz, 0.4H), 8.22 (d, *J* = 2.0 Hz. 0.6H), 7.99 (d, *J* = 2.4 Hz, 1H), 7.85 (s, 0.6H), 7.82 (s, 0.4H), 7.75 - 7.51 (m, 2H), 7.88 (s, 1H), 6.13 (brs, 1H), 4.00 - 3.41 (m, 5H), 3.29 - 2.91 (m, 4H), 2.71 (d, *J* = 4.8 Hz, 3H), 1.94 - 1.39 (m, 10H), 1.18 - 0.80 (m, 6H). | 599.2 |
| 178. | 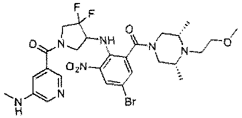 | ¹H NMR (400 MHz, DMSO-*d*₆): δ 8.27 (s, 1H), 8.03 (s, 1H), 7.88 (s, 1H), 7.82 - 7.60 (m, 1.3H), 7.51 (brs, 0.2H), 7.20 (brs, 0.2H), 7.07 - 6.80 (m, 1,3H), 6.19 (brs, 1H), 4.55 - 3.83 (m, 5H), 3.80 - 3.38 (m, 3H), 3.28 - 3.06 (m, 4H), 3.03 - 2.51 (m, 8H), 2.46 - 2.25 (m, 1H), 1.11 - 0.82 (m, 6H). | 654.2 |
| 179. | 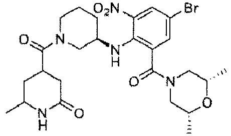 | ¹H NMR (400 MHz, DMSO-*d*₆): δ 8.31 - 8.20 (m, 1H), 7.94 - 7.41 (m, 3H), 4.46 - 4.24 (m, 1H), 3.90 - 3.34 (m, 8H), 3.27 - 2.96 (m, 2H), 2.93 - 2.70 (m, 1H), 2.49 -2.42 (m, 1H), 2.31 - 2.03 (m, 2H), 2.02 - 1.21 (m, 6H), 1.19 - 0.96 (m, 9H). | 580.1 |
| 180. | 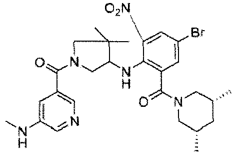 | ¹H NMR (400 MHz, DMSO-*d*₆): δ 8.35 - 8.15 (m, 1H), 8.07 - 7.92 (m, 1H), 7.91 - 7.46 (m, 3H), 7.03 - 6.76 (m, 1H), 6.27 - 5.96 (m, 1H), 4.59 - 4.19 (m, 1H), 4.06 - 3.47 (m, 4H), 3.21 -2.99 (m, 2H), 2.78 - 2.65 (m, 3H), 2.64 - 2.53 (m, 1H), 2.34 - 2.10 (m, 1H), 2.06 - 1.34 (m, 4H), 1.19 - 0.57 (m, 12H). | 587.2 |
| 181. | 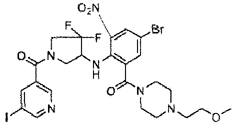 | ¹H NMR (400 MHz, DMSO-(*d*₆): δ 8.94 (s, 1H), 8.72 (s, 1H), 8.42 - 8.21 (m, 2H), 7.85 - 7.65 (m, 1H), 7.58 (brs, 0.5H), 6.96 (brs, 0.5H), 4.51 - 3.90 (m, 4H), 3.89 -3.39 (m, 6H), 3.26 -2.95 (m, 5H), 2.64 - 2.54 (m, 1H), 2.46 - 1.83 (m, 4H). | 723.0 |
| 182. | 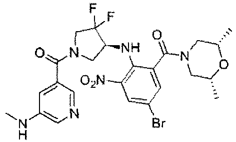 | ¹H NMR (400 MHz, DMSO-*d*₆): δ 8.27 (s, 1H), 8.03 (s, 1H), 7.87 (s, 1H), 7.82 - 7.67 (m, 1H), 7.66 - 7.39 (m, 0.5H), 7.16 (brs, 0.3H), 6.92 (brs, 1.2H)_ 6.17 (brs, 1H), 4.51 - 3.41 (m, 8H), 3.18 - 2.78 (m, 3H), 2.72 (*d, J* = 4.8 Hz. 3H), 1.24 - 0.76 (m, 6H). | 597.1 |
| 183. | 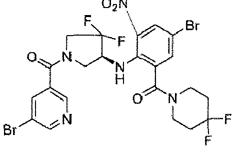 | ¹H NMR (400 MHz, DMSO-*d*₆): δ 8.84 (s, 1H), 8.72 (s, 1H), 8.34 - 8.17 (m, 2H), 7.96 - 7.82 (m, 1H), 7.43 (brs, 0.5H), 6.89 (brs, 0.5H), 4.49 - 3.34 (m, 8H), 3.27 - 2.97 (m, 1H), 2.29 - 1.68 (m, 4H). | 652.0 |
| 184. | 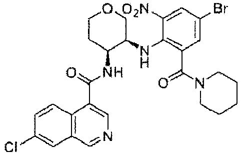 | ¹H NMR (400 MHz, DMSO-*d*₆) δ 9.35 (s, 1H), 8.98 (br, 0.5H), 8.64 (br, 0.5H), 8.42 - 8.04 (m, 5H), 7.79 (s, 1H), 7.61 (d, *J* = 2.5 Hz, 1H), 4.46 (s, 1H), 4.01 - 3.21 (m, 9H), 1.92 - 1.75 (m, 2H), 1.57 -1.41 (m, 6H). | 616.0 |
| 185. | 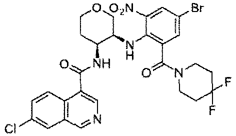 | ¹H NMR (400 MHz, DMSO-*d*₆) δ 9.35 (s, 1H), 8.97 (s, 0.5H), 8.66 (s, 0.5H), 8.43 - 8.06 (m, 5H), 7.81 - 7.77 (m, 2H), 4.42 (s, 1H), 4.10 - 3.38 (m, 9H), 2.07 - 1.74 (m, 6H). | 652.0 |
| 186. | 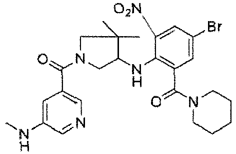 | ¹H NMR (400 MHz, DMSO-*d*₆) δ 8.32 - 8.17 (m, 1H), 7.99 (s, 1H), 7.84 (s, 1H), 7.75 - 7.58 (m, 2H), 6.89 (s, 1H), 6.16 - 6.08 (m, 1H), 3.90 - 3.41 (m, 7H), 3.28 - 3.16 (m, 2H), 2.79 - 2.64 (m, 3H), 1.73 - 1.38 (m, 6H), 1.23 - 0.81 (m, 6H). | 559.2 |
| 187. | 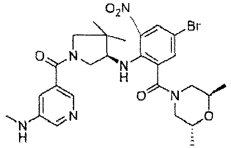 | ¹H NMR (400 MHz, DMSO-*d*₆): δ 8.28 (d, *J* = 2.4 Hz, 0.5H), 8.25 (d, *J* = 2.4 Hz, 0.5H), 7.99 (d, *J* = 2.4 Hz, 1H), 7.86 (s, 0.5H), 7.83 (s, 0.5H), 7.78 - 7.66 (m, 2H), 6.89 (s, 0.5H), 6.86 (s, 0.5H), 6.16 - 6.06 (m, 1H), 4.19 - 3.35 (m, 8H), 3.27 - 2.85 (m, 3H), 2.71 (d, *J* = 4.8 Hz, 3H), 1.25 - 0.79 (m, 12H). | 589.0 |
| 188. | 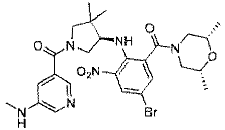 | ¹H NMR (400 MHz, DMSO-*d*₆) δ 8.31 - 8.19 (m, 1H), 8.03 - 7.95 (m, 1H), 7.90 - 7.79 (m, 1H), 7.74 - 7.48 (m, 2H), 6.89 (brs, 1H), 6.12 (brs, 1H), 4.51 - 4.11 (m, 1H), 4.08 - 3.36 (m, 6H), 3.28 - 2.78 (m, 3H), 2.75 - 2.69 (m, 3H), 2.60 - 2.52 (m, 0.4H), 2.45 - 2.35 (m, 0.6H), 1.23 - 0.76 (m, 12H). | 589.2 |
| 189. | 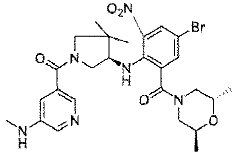 | ¹H NMR (400 MHz, DMSO-*d*₆) δ 8.28 (d, *J* = 2.4 Hz, 0.5H), 8.25 (d, *J* = 2.0 Hz, 0.5H), 7.99 (t, *J* = 2.8 Hz, 1H), 7.86 (brs, 0.5H), 7.84 (brs, 0.5H), 7.72 (d, *J* = 2.0 Hz, 0.5H), 7.69 - 7.57 (m, 1.5H), 6.88 (s, 1H), 6.16 - 6.06 (m, 1H), 4.11 - 3.35 (m, 8H), 3.27 - 2.81 (m, 3H), 2.74 - 2.68 (m, 3H), 1.25 - 0.93 (m, 12H). | 589.2 |
| 190. | 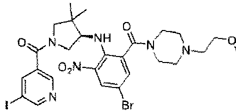 | ¹H NMR (400 MHz, DMSO-*d*₆) δ 8.91 (m, 1H), 8.77 - 8.62 (m, 1H), 8.343 - 8.13 (m, 2H), 7.80 - 7.34 (m, 2H), 4.01 - 3.40 (m, 9H), 3.24 - 2.87 (m, 5H), 2.72 - 2.52 (m, 2.7H), 2.45 - 2.26 (m, 2.7H), 2.07 - 1.80 (m, 0.6H), 1.21 -0.77 (m, 8H). | 715.0 |
| 191. | 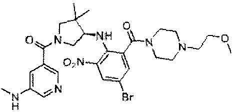 | ¹H NMR (400 MHz, DMSO-*d*₆) δ 8.27 (d, *J* = 2.0 Hz. 0.5H), 8.23 (d, *J* = 2.4 Hz, 0.5H), 7.99 (s, 1H), 7.85 (s, 1H), 7.73 - 7.42 (m, 2H), 6.89 (s, 1H), 6.19 - 6.03 (m, 1H), 3.99 - 3.42 (m, 9H), 3.24 - 2.98 (m, 5H), 2.84 - 2.54 (m, 7H), 2.45 - 2.13 (m, 2H), 1.18 - 0.84 (m, 6H). | 618.2 |
| 192. | 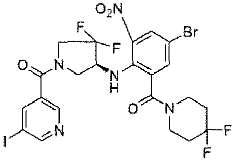 | ¹H NMR (400 MHz, DMSO-*d*₆): δ 8.94 (s, 1H), 8.71 (m, 1H), 8.41 - 8.20 (m, 2H), 7.93 (s, 0.4H), 7.88 (s, 0.6H), 7.44 (s, 0.6H), 6.90 (s, 0.4H), 4.50 - 3.38 (m, 8H), 3.24 - 2.95 (m, 1H), 2.27 - 1.72 (m, 4H). | 699.8 |
| 193. | 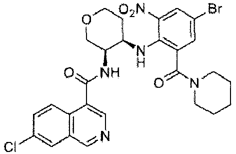 | ¹H NMR (400 MHz, DMSO-*d*₆) δ 9.40 (s, 1H), 9.04 (br, 0.5H), 8.83 (br, 0.5H), 8.69 - 8.62 (m, 1H), 8.35 (s, 114), 8.26 (d, *J* = 2.5 Hz, 1H), 8.08 - 7.83 (m, 3H), 7.65 (s, 1H), 4.42 (br, 1H), 3.92 - 3.91 (m, 3H), 3.68 (br, 2H), 3.54 - 3.20 (m, 4H), 2.09 - 2.01 (m, 1H), 1.75 - 1.49 (m, 7H). | 616.1 |
| 194. | 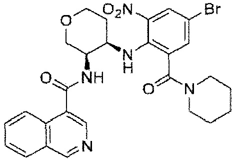 | ¹H NMR (400 MHz, DMSO-*d₆*): δ 9.40 (s, 1H), 8.99 (d, *J* = 8.8 Hz, 0.5H), 8.78 (d, *J* = 6.8 Hz, 0.5H), 8.64 (s, 0.5H), 8.58 (s, 0.5H), 8.26 (d, *J* = 2.4 Hz, 1H), 8.19 (d, *J* = 8.0 Hz, 1H), 8.10 - 7.84 (m, 2H), 7.80 (t, *J* = 7.2 Hz, 1H), 7.73 (t, *J* = 7.2 Hz, 1H), 7.65 (s, 1H), 4.43 (s, 1H), 4.01 - 3.82 (m, 3H), 3.68 (s, 2H), 3.57 - 3.33 (m, 3H), 3.20 (brs, 1H), 2.19 - 1.92 (m, 1H), 1.83 - 1.39 (m, 7H). | 582.0 |
| 195. | 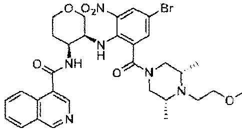 | ¹H NMR (400 MHz, DMSO-(*d*₆): δ 9.37 (s, 1H), 9.04 - 8.84 (m, 0.5H), 8.72 - 8.45 (m, 0.5H), 8.41 - 7.87 (m, 5H), 7.83 - 7.51 (m, 3H), 4.62 - 3.43 (m, 7H), 3.28 - 3.01 (m, 7H), 2.98 - 2.56 (m, 5H), 2.04 - 1.64 (m, 2H), 1.15 - 0.85 (m, 4H), 0.59 (s, 1H), 0.43 (s, 1H). | 669.2 |
| 196. | 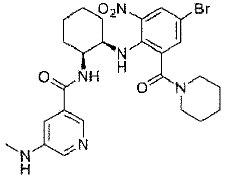 | ¹H NMR (400 MHz, DMSO-*d*₆): δ 8.42 (s, 0.6H), 8.22 (s, 1H), 8.07 - 7.67 (m, 3.4H), 7.55 (s, 1H), 7.05 (s, 1H), 6.07 (brs, 1H), 4.24 (s, 1H), 3.92 - 3.54 (m, 3H), 3.21 - 2.98 (m, 2H), 2.72 (brs, 3H), 1.84 - 1.11 (m, 14H). | 559.2 |
| 197. | 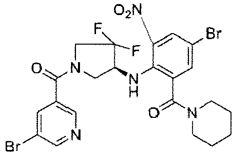 | ¹H NMR (400 MHz, DMSO-(*d₆*): δ 8.85 (s, 1H), 8.72 (s, 1H), 8.24 (brs, 1H), 7.82 - 7.63 (m, 1H), 7.54 (s, 0.5H), 7.16 - 6.83 (m, 0.5H), 4.52 - 3.39 (m, 7H), 3.24 - 2.84 (m, 2H), 1.77 -1.24 (m, 6H). | 616.0 |
| 198. | 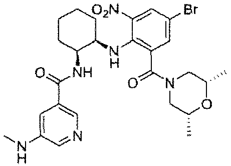 | ¹H NMR (400 MHz, DMSO-d₆): δ 8.59 (d, *J* = 6.4 Hz, 0.3H), 8.39 (d, *J* = 7.6 Hz, 0.3H), 8.30 - 8.16 (m, 1H), 8.15 - 7.73 (m, 3.4H), 7.62 - 7.50 (m, 1H), 7.14 - 6.83 (m, 1H), 6.06 (brs, 1H), 4.52 - 4.03 (m, 2H), 3.95 - 3.40 (m, 3.4H), 3.12 - 2.96 (m, 0.6H), 2.93 - 2.61 (m, 4H), 2.47 - 2.39 (m, 1H), 1.97 - 1.26 (m, 8H), 1.25 - 0.92 (m, 4H), 0.81 (d, *J* = 5.2 Hz, 1H), 0.65 (d, *J* = 4.8 Hz, 1H). | 589.2 |
| 199. | 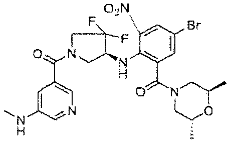 | ¹H NMR (400 MHz, DMSO-*d*₆) δ 8.27 (d, *J* = 2.0 Hz. 1H), 8.03 (d, *J* = 2.8 Hz. 1H), 7.87 (d, *J* = 2.0 Hz. 1H), 7.83 - 7.41 (m, 1.7H), 7.06 - 6.80 (m, 1.3H), 6.17 (brs, 1H), 4.41 - 3.48 (m, 8H), 3.25 -2.79 (m, 3H), 2.72 (d, *J* = 5.2 Hz, 3H), 1.26 - 0.77 (m, 6H). | 597.1 |
| 200. | 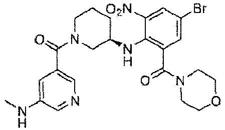 | ¹H NMR (400 MHz, DMSO-*d*₆) δ 8.26 (s, 1H), 8.09 - 7.45 (m, 4H), 6.75 (s, 1H), 6.13 (brs, 1H), 4.11 - 3.41 (m, 11H), 3.25 - 2.88 (m, 3H), 2.70 (brs, 3H), 1.99 - 1.30 (m, 4H). | 547.0 |
| 201. | 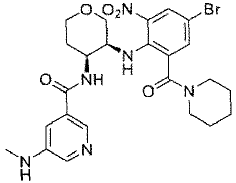 | ¹H NMR (400 MHz, DMSO-*d*₆) δ 8.59 (d, *J* = 5.2 Hz. 1H), 8.23 (s, 1H), 8.20 - 7.85 (m, 3H), 7.54 *(d, J* = 2.0 Hz, 1H), 7.00 (s, 1H), 6.17 - 5.93 (m, 1H), 4.41 - 4.27 (m, 1H), 3.97 - 3.88 (m, 1H), 3.87 - 3.35 (m, 6H), 3.25 - 3.00 (m, 2H), 2.68 (d, *J* = 5.2 Hz. 3H), 2.08 - 1.77 (m, 1H), 1.73 - 1.62 (m, 1H), 1.53 (brs, 3H), 1.33 (brs, 3H). | 561.2 |
| 202. | 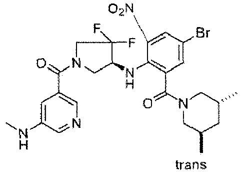 | ¹H NMR (400 MHz, DMSO-*d*₆) δ 8.26 (s, 1H), 8.03 (s, 1H), 7.87 (s, 1H), 7.77 - 7.39 (m, 1.6H), 7.25 (brs, 0.4H), 6.93 (s, 1H), 6.18 (brs, 1H), 4.58 - 4.44 (m, 7H), 3.02 - 2.65 (m, 5H), 2.11 - 1.19 (m, 4H), 1.01 - 0.57 (m, 6H), | 595.1 |
| 203. | 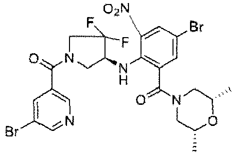 | ¹H NMR (400 MHz, DMSO-*d*₆) & 8.86 (s, 1H), 8.73 (s, 1H), 8.33 - 8.13 (m, 2H), 7.90 - 7.68 (m, 1H), 7.63 -7.33 (m, 0.5H), 7.26 -6.83 (m, 0.5H), 4.58 - 3.50 (m, 8H), 3.14 - 2.56 (m, 3H), 1.22 - 0.81 (m, 6H). | 646.0 |
| 204. | 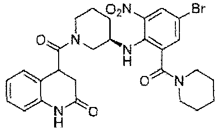 | ¹H NMR (400 MHz, DMSO-d₆) δ 10.05 - 10.03 (m, 1H), 8.23 (s, 1H), 7.90 - 7.81 (m, 1H), 7.59 - 7.58 (m, 1H), 7.16 - 6.85 (m, 4H), 4.68 - 3.24 (m, 11H), 2.03 - 1.44 (m, 11H). | 584.1 |
| 205. | 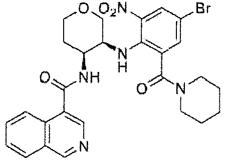 | ¹H NMR (400 MHz, DMSO-*d*₆) δ 9.37 (s, 1H), 8.94 (brs, 0.5H), 8.6.3 (brs, 0.5H), 8.37 (brs, 1H), 8.29 - 7.95 (m, 4H), 7.82 - 7.66 (m, 2H), 7.62 (d, *J* = 2.0 Hz, 1H), 4.53 - 4.40 (m, 1H), 4.10 - 3.45 (m, 7H), 3.21 - 2.95 (m, 2H), 1.98 - 1.83 (m, 1H), 1.82 - 1.67 (m, 1H), 1.66 -1.33 (m, 6H). | 582.0 |
| 206. | 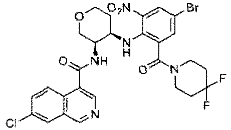 | ¹H NMR (400 MHz, DMSO-*d*₆) δ 9.40 (s, 1H), 9.03 - 8.85 (m, 1H), 8.67 - 8.63 (m, 1H), 8.36 - 8.28 (m, 2H), 8.08 (s, 1H), 7.87 - 7.80 (m, 3H), 4.40 (br, 1H), 4.11 (br, 1H), 3.91 - 3.84 (m, 3H), 3.54 - 3.52 (m, 5H), 2.09 - 1.73 (m, 6H). | 652.0 |
| 207. | 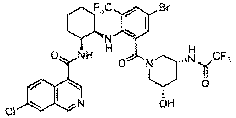 | ¹H NMR (400 MHz, DMSO-*d*₆) δ 9.59 - 9.07 (m, 2H), 8.87 - 8.73 (m, 0.7H), 8.69 - 8.52 (m, 1.3H), 8.37 (brs, 1H), 8.22 - 8.09 (m, 1H), 7.90 - 7.76 (m, 1H), 7.75 - 7.43 (m, 2H), 5.60 - 5.07 (m, 1H), 4.76 - 4.08 (m, 3H), 4.05 - 3.41 (m, 4H), 3.08 - 2.87 (m, 1H), 2.77 - 2.61 (m, 1H), 2.19 - 1.79 (m, 2H), 1.77 - 1.24 (m, 8H). | 764.0 |
| 208. | 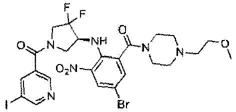 | ¹H NMR (400 MHz, DMSO-*d*₆) δ 8.94 (s, 1H), 8.72 (s, 1H), 8.33 (s, 1H), 8.25 (s, 1H), 7.80 - 7.64 (m, 1H), 7.56 (s, 0.5H), 6.95 (s, 0.5H), 4.46 - 3.44 (m, 9H), 3.23 - 2.84 (m, 5H), 2.65 - 2.55 (m, 1H), 2.45 - 1.77 (m, 5H). | 723.0 |
| 209. | 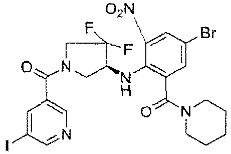 | ¹H NMR (400 MHz, DMSO-*d*₆) δ 8.94 (s, 1H), 8.70 (s, 1H), 8.45 - 8.28 (m, 1H), 8.23 (s, 1H), 7.82 - 7.62 (m, 1H), 7.52 (s, 0.5H), 7.21 - 6.83 (m, 0.5H), 4.48 - 3.39 (m, 7H), 3.22 - 2.83 (m, 2H), 1.79 - 1.27 (m, 6H). | 664.0 |
| 210. | 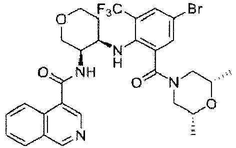 | ¹H NMR (400 MHz, DMSO-*d*₆) δ 9.42 (s, 1H), 9.03 - 8.88 (m, 0.7H), 8.81 - 8.66 (m, 0.3H), 8.61 (d, J = 2.8 Hz. 1H), 8.29 - 8.04 (m, 2H), 7.83 (t, *J* = 7.6 Hz, 1H), 7.79 - 7.67 (m, 2H), 7.66 - 7.53 (m, 1H), 4.82 - 4.23 (m, 3H), 4.08 - 3.35 (m, 7H), 3.26 - 3.09 (m, 1H), 3.01 - 2.53 (m, 2H), 2.18 -1.82 (m, 1H), 1.77 -1.51 (m, 1H), 1.21 - 0.72 (m, 6H). | 635.0 |
| 211. | 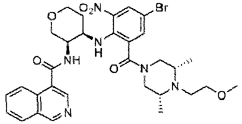 | ¹H NMR (400 MHz, DMSO-*d*₆) δ 9.41 (s, 1H), 9.12 - 8.91 (m, 0.5H), 8.87 - 8.72 (m, 0.5H), 8.70 - 8.51 (m, 1H), 8.28 (d, *J* = 2.0 Hz, 1H), 8.20 (d, *J* = 7.6 Hz, 1H), 8.15 - 7.59 (m, 5H), 4.62 - 4.22 (m, 2H), 4.17 - 3.39 (m, 7H), 3.25 - 3.09 (m, 4H), 3.03 - 2.55 (m, 6H), 2.21 -1.59 (m, 2H), 1.27 -0.78 (m, 6H). | 669.2 |
| 212. | 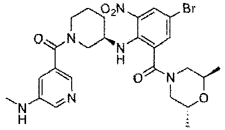 | ¹H NMR (400 MHz, DMSO-*d*₆) δ 8.27 (s, 1H), 8.09 - 7.48 (m, 4H), 6.75 (s, 1 H), 6.14 (brs, 1H), 4.17 - 3.35 (m, 8H), 3.22 - 2.88 (m, 3H), 2.70 (brs, 3H), 1.96 - 1.33 (m, 4H), 1.24 - 0.81 (m, 6H) | 575.1 |
| 213. | 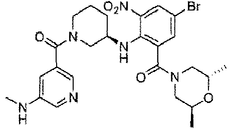 | ¹H NMR (400 MHz, DMSO-*d*₆) δ 8.26 (s, 1H), 8.05 - 7.37 (m, 4H), 6.75 (s, 1H), 6.15 (brs, 1H), 4.15 - 3.36 (m, 8H), 3.27 - 2.80 (m, 3H), 2.70 (brs, 3H), 1.95 - 1.29 (m, 4H), 1.25 - 0.86 (m, 6H). | 575.1 |
| 214. | 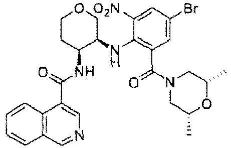 | ¹H NMR (400 MHz, DMSO-*d*₆) δ 9.38 - 9.36 (m, 1H), 9.07 - 8.57 (m, 1H), 8.39 - 7.92 (m, 5H), 7.81 - 7.65 (m, 3H), 4.55 - 3.45 (m, 10H), 3.31 - 3.21 (m, 1H), 2.95 - 2.67 (m, 1H), 1.94 -1.76 (m, 2H), 1.16 - 0.96 (m, 4H), 0.66 - 0.56 (m, 2H). | 612.2 |
| 215. | 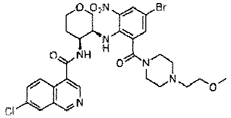 | ¹H NMR (400 MHz, DMSO-*d*₆) δ 9.90 (brs, 1H), 9.36 (s, 1H), 9.00 (s, 0.5H), 8.66 (s, 0.5H), 8.52 - 7.57 (m, 7H), 4.74 - 4.19 (m, 3H), 4.13 - 3.45 (m, 12H), 3.30 - 2.84 (m, 6H), 1.99 - 1.60 (m, 2H). | 675.1 |
| 216. | 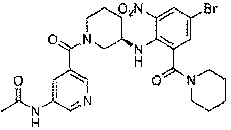 | ¹H NMR (400 MHz, DMSO-*d*₆) δ 10.31 (s, 1H), 8.71 (s, 1H), 8.30 - 7.92 (m, 3H), 7.90 - 7.32 (m, 2H), 4.02 - 3.39 (m, 7H), 3.23 - 3.05 (m, 2H), 2.09 (s, 3H), 1.93 - 1.85 (m, 1H), 1.81 -1.25 (m, 9H). | 573.1 |
| 217. | 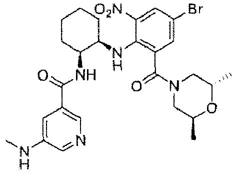 | ¹H NMR (400 MHz, DMSO-*d*₆) δ 8.46 (s, 0.75H), 8.24 (d, *J* = 2.8 Hz, 1H), 8.16 (s, 0.75H), 8.08 - 7.71 (m, 2.5H), 7.59 (d, *J* = 2.4 Hz, 1H), 6.92(s, 1H),6.12 - 5.98 (m, 1H), 4.25 - 3.38 (m, 6H), 3.23 - 3.08 (m, 1H), 3.02 - 2.82 (m, 1H), 2.67 (d, *J* = 3.2 Hz. 3H), 1.95 - 1.27 (m, 8H), 1.18 (d, *J* = 6.4 Hz. 3H), 1.04 - 0.70 (m, 3H). | 589.2 |
| 218. | 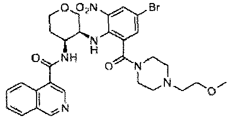 | ¹H NMR (400 MHz, DMSO-*d*₆ and D₂O) δ 9.41 (s, 1H), 8.53 - 8.07 (m, 3H), 8.01 - 7.75 (m, 3.5H), 4.88 - 3.65 (m, 11H), 3.49 - 2.92 (m, 10H), 2.00 - 1.65 (m, 2H). | 641.1 |
| 219. | 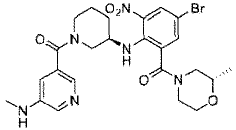 | ¹H NMR (400 MHz, DMSO-*d*₆) δ 8.26 (s, 1H), 8.03 - 7.52 (m, 4H), 6.74 (s, 1H), 6.15 (brs, 1H), 4.43 - 3.36 (m, 8H), 3.24 - 2.78 (m, 3H), 2.77 - 2.56 (m, 4H), 1.99 - 1.29 (m, 4H), 1.14 (d, *J* = 5.6 Hz, 1.5H), 1.01 (brs, 1.5H), | 561.2 |
| 220. | 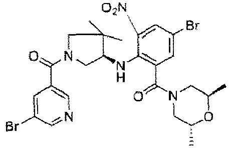 | ¹H NMR (400 MHz, DMSO-*d*₆) δ 8.82 (*d, J* = 2.4 Hz, 1H), 8.71 (d, *J* = 2.0 Hz, 0.5H), 8.67 (d, *J* = 2.0 Hz, 0.5H), 8.28 (d, *J* = 2.4 Hz, 0.5H), 8.26 - 8.21 (m, 1H), 8.17 (s, 0.5H), 7.82 - 7.35 (m 2H), 4.15 - 3.36 (m, 9H), 3.28 - 2.95 (m, 2H), 1.27 - 0.84 (m, 11H). | 638.0 |
| 221. | 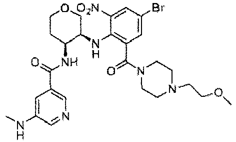 | ¹H NMR (400 MHz, DMSO-*d*₆) δ 8.54 (s, 0.6H), 8.22 - 7.99 (m, 4.4H), 7.54 (s, 1H), 6.96 (s, 1H), 6.06 (brs, 1H), 4.41 - 4.25 (m, 1H), 3.97 - 3.72 (m, 3H), 3.66 - 3.45 (m, 4H), 3.39 (brs, 2H), 3.26 - 3.08 (m, 5H), 2.68 (d, *J* = 5.2 Hz, 3H), 2.46 - 2.38 (m, 4H), 2.36 - 2.24 (m, 2H), 2.04 - 1.79 (m, 1H), 1.74 - 1.57 (m, 1H). | 620.2 |
| 222. | 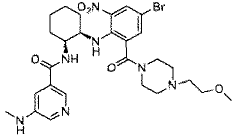 | ¹H NMR (400 MHz, DMSO-*d*₆) δ 8.47 - 8.10 (m, 2H), 8.08 - 7.75 (m, 3H), 7.69 (s, 1H), 7.02 (s, 1H), 6.16 (brs, 1H), 4.70 - 4.06 (m, 2H), 3.98 - 3.45 (m, 6H), 3.27 - 2.80 (m, 9H), 2.72 (brs, 3H), 1.88 - 1.28 (m, 8H). | 618.4 |
| 223. | 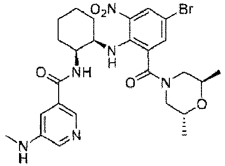 | ¹H NMR (400 MHz, DMSO-*d*₆) δ 8.55 (s, 0.5H), 8.26 (d, *J* = 1.6 Hz, 1H), 8.18 - 7.71 (m, 3.5H), 7.59 (s, 1H), 7.19 - 6.91 (m, 1H), 6.06 (brs, 1H), 4.31 - 3.61 (m, 5H), 3.24 - 2.84 (m, 2H), 2.71 (d, *J* = 5.2 Hz, 3H), 2.67 - 2.53 (m, 1H), 1.90 - 0.49 (m, 14H). | 589.2 |
| 224. | 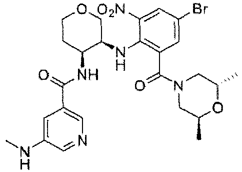 | ¹H NMR (400 MHz, DMSO-*d*₆) δ 8.57 (s, 1H), 8.40 - 8.13 (m, 2H), 7.99 (d, *J* = 2.8 Hz. 1H), 7.85 (d, *J* = 2.0 Hz, 1H), 7.59 (d, *J* = 2.4 Hz, 1H), 6.85 (s, 1H), 6.07 (brs, 1H), 4.40 - 4.23 (m, 1H), 4. 13 - 3.77 (m, 5H), 3.69 - 3.35 (m, 4H), 3.24 - 3.17 (m, 1H), 3.00 - 2.90 (m, 1H), 2.65 (d, *J* = 5.2 Hz. 3H), 2.07 - 1.82 (m, 1H), 1.76 - 1.64 (m, 1H), 1.16 (d, *J* = 6.4 Hz, 3H), 1.02 - 0.70 (m, 3H). | 591.2 |
| 225. | 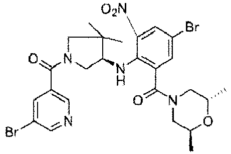 | ¹H NMR (400 MHz, DMSO-*d*₆) δ 8.84 - 8.78 (m, 1H), 8.74 - 8.67 (m, 1H), 8.29 (d, *J* = 2.4 Hz, 0.5H), 8.27 - 8.16 (m, 1.5H), 7.72 (d, *J* = 2.0 Hz, 0.5H), 7.70 - 7.60 (m, 1H), 7.56 (d, *J* = 9.6 Hz, 0.5H), 4.15 - 3.34 (m, 9H), 3.22 - 2.75 (m, 2H), 1.30 - 0.76 (m, 12H). | 638.0 |
| 226. | 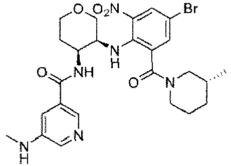 | ¹H NMR (400 MHz, DMSO-*d*₆) δ 8.71 - 8.49 (m, 1H), 8.33 - 7.77 (m, 4H), 7.53 (s, 1H), 7.03 (s, 0.5H), 6.96 (s, 0.5H), 6.19 - 5.93 (m, 1H), 4.47 -4.11 (m, 2H), 4.00 - 3.40 (m, 5H), 3.29 - 3.13 (m, 1H), 3.02 - 2.60 (m, 4.5H), 2.41 - 2.31 (m, 0.5H), 2.07 - 0.97 (m, 7H), 0.88 (d, *J* = 6.4 Hz, 2H), 0.48 (d, *J* = 5.6 Hz, 1H). | 575.0 |
| 227. | 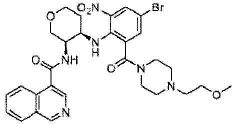 | ¹H NMR (400 MHz, DMSO-*d*₆) δ 9.41 (s, 1H), 9.01 (d, *J* = 6.8 Hz, 0.5H), 8.80 (d, *J* = 6.4 Hz, 0.5H), 8.65 (s, 0.5H), 8.58 (s, 0.5H), 8.28 (s, 1H), 8.21 (s, 0.5H), 8.19 (s, 0.5H), 8.10 - 7.94 (m, 1.5H), 7.89 (d, *J* = 8.0 Hz, 0.5H), 7.81 (t, *J* = 7.2 Hz, 1H), 7.74 (t, *J* = 7.2 Hz, 1H), 7.71 - 7.62 (m, 1H), 4.45 (s, 1H), 4.03 - 3.74 (m, 4H), 3.66 - 3.47 (m, 6H), 3.29 - 3.13 (m, 4H), 2.79 - 2.52 (m, 4H), 2.48 - 2.23 (m, 2H), 2.18 - 1.95 (m, 1H), 1.85 - 1.67 (m, 1H). | 641.1 |
| 228. | 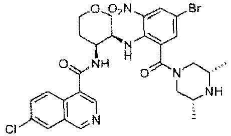 | ¹H NMR (400 MHz, DMSO-*d*₆) δ 9.43 - 9.25 (m, 1H), 9.11 - 8.82 (m, 0.5H), 8.77 - 8.49 (m, 0.5H), 8.45 - 7.93 (m, 5H), 7.86 - 7.72 (m, 1H), 7.69 - 7.49 (m, 1H), 4.57 - 3.43 (m, 7H), 3.21 - 2.96 (m, 1H), 2.80 -2.54 (m, 2H), 2.357 - 2.11 (m, 2H), 2.02 - 1.48 (m, 3H), 1.07 - 0.40 (m, 6H). | 645.2 |
| 229. | 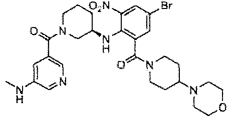 | ¹H NMR (400 MHz, DMSO-*d*₆) δ 8.25 (s, 1H), 8.07 - 7.42 (m, 4H), 6.74 (s, 1H), 6.14 (brs, 1H), 4.39 (s, 1H), 4.19 - 3.55 (m, 8H), 3.22 - 2.77 (m, 4H), 2.70 (brs, 3H), 2.49 - 2.20 (m, 5H), 1.97 - 1.37 (m, 8H). | 630.2 |
| 230. | 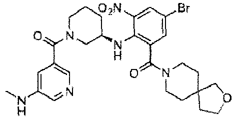 | ¹H NMR (400 MHz, DMSO-*d*₆) δ 8.25 (s, 1H), 8.07 - 7.41 (m, 4H), 6.75 (s, 1H), 6.14 (brs, 1H), 4.13 - 3.41 (m, 10H), 3.25 - 3.09 (m, 3H), 2.70 (brs, 3H), 1.99 - 1.39 (m, 10H). | 601.2 |
| 231. | 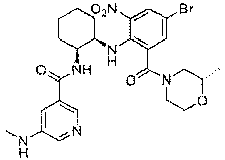 | ¹H NMR (400 MHz, DMSO-*d*₆) δ 8.59 - 8.31 (m, 1H), 8.27 - 8.21 (m, 1H), 8.14 - 7.76 (m, 3H), 7.60 (s, 1H), 7.12 -6.81 (m, 1H), 6.06 (brs, 1H), 4.45 - 4.09 (m, 2H), 4.01 - 3.38 (m, 4H), 3.20 - 2.77 (m, 2H), 2.76 - 2.51 (m, 4H), 1.97 - 1.24 (m, 8H), 1.20 - 0.74 (m, 3H). | 575.3 |
| 232. | 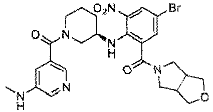 | ¹H NMR (400 MHz, DMSO-*d*₆) δ 8.25 (s, 1H), 8.05 - 7.54 (m, 4H), 6.75 (s, 1H), 6.12 (brs, 1H), 3.93 - 3.43 (m, 10H), 3.25 - 3.17 (m, 2H), 3.06 - 2.83 (m,2H), 2.69 (brs, 3H), 1.93 - 1.35 (m, 5H). | 573.2 |
| 233. | 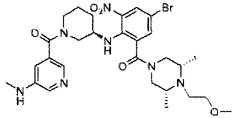 | ¹H NMR (400 MHz, DMSO-*d*₆) δ 8.25 (s, 1H), 8.08 - 7.39 (m, 4H), 6.73 (s, 1H), 6.14 (brs, 1H), 4.27 (s, 1H), 4.00 - 3.32 (m, 6H), 3.27 - 2.98 (m, 5H), 2.90 - 2.56 (m, 9H), 2.02 - 1.33 (m, 4H), 1.08 (d, *J* = 4.4 Hz, 3H), 0.93 (br, 3H), | 632.4 |
| 234. | 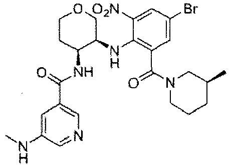 | ¹H NMR (400 MHz, DMSO-*d*₆) δ 8.73 - 8.49 (m, 0.7H), 8.31 - 7.77 (m, 4.3H), 7.62 - 7.46 (m, 1H), 7.09 - 6.86 (m, 1H), 6.19 - 5.95 (m, 1H), 4.46 - 4.04 (m, 2H), 4.01 - 3.39 (m, 5H), 3.26 - 3.16 (m, 1H), 3.12 - 2.83 (m, 1H), 2.68 (d, *J* = 4.4 Hz. 3H), 2.59 - 2.51 (m, 0.4H), 2.47 - 2.41 (m, 0.2H), 2.38 - 2.28 (m, 0.6H), 2.10 - 1.02 (m, 7H), 0.98 - 0.77 (m, 2H), 0.31 (d, *J* = 6.4 Hz. 1H), | 575.1 |
| 235. | 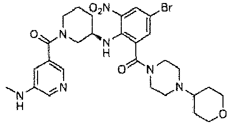 | ¹H NMR (400 MHz, DMSO-*d*₆) δ 8.26 (s, 1H), 8.10 - 7.33 (m, 4H), 6.74 (s, 1H), 6.14 (brs, 1H), 4.00 - 3.46 (m, 7H), 3.24 - 2.89 (m, 6H), 2.78 - 2.55 (m, 4H), 2.45 - 2.19 (m, 4H), 2.0 1 - 1.82 (m, 1H), 1.79 - 1.43 (m, 7H). | 630.4 |
| 236. | 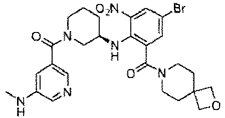 | ¹H NMR (400 MHz, DMSO-*d*₆) δ 8.25 (s, 1H), 8.06 - 7.40 (m, 4H), 6.74 (s, 1H), 6.15 (brs, 1H), 4.34 (brs, 4H), 3.99 - 3.41 (m, 5H), 3.23 - 2.86 (m, 4H), 2.70 (brs, 3H), 1.96 - 1.30 (m, 8H). | 587.2 |
| 237. | 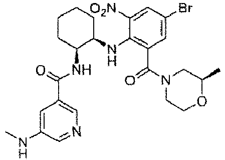 | ¹H NMR (400 MHz, DMSO-*d*₆) δ 8.59 (d, *J* = 7.6 Hz. 0.3H), 8.36 (*d, J* = 8.0 Hz, 0.3H), 8.30 - 8.18 (m, 1H), 8.15 - 7.72 (m, 3.4H), 7.66 - 7.50 (m, 1H), 7.14 - 6.83 (m, 1H), 6.07 (brs, 1H), 4.47 - 4.09 (m, 2H), 4.02 - 3.80 (m, 1H), 3.74 - 3.43 (m, 4H), 3.12 - 2.82 (m, 1H), 2.70 (d, J = 4.4 Hz, 3H), 2.62 - 2.51 (m, 1H), 1.87 - 1.28 (m, 8H), 1.21 - 0.92 (m, 2H), 0.64 (d, *J* = 5.5 Hz, 1H). | 575.3 |
| 238. | 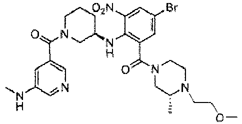 | ¹H NMR (400 MHz, DMSO-*d*₆) δ 8.26 (s, 1H), 8.06 - 7.41 (m, 4H), 6.75 (s, 1H), 6.15 (brs, 1H), 4.45 - 3.40 (m, 7H), 3.26 - 309 (m, 5H), 2.99 - 2.58 (m, 7H), 2.43 - 2.12 (m, 3H), 1.99 - 1.39 (m, 4H), 1.04 (brs, 1.5H), 0.90 (brs, 1.5H). | 618.2 |
| 239. | 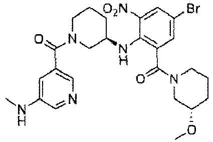 | ¹H NMR (400 MHz, DMSO-*d*₆) δ 8.26 (s, 1H), 8.11 - 7.24 (m, 4H), 6.86 - 6.52 (m, 1H), 6.25 - 5.79 (m, 1H), 4.30 - 3.40 (m, 6H), 3.31 - 2.87 (m, 7H), 2.70 (brs, 3H), 1.94 - 1.27 (m, 8H). | 575.1 |
| 240. | 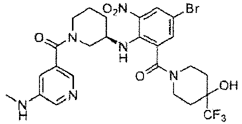 | ¹H NMR (400 MHz, DMSO-*d*₆) δ 8.27 (s, 1H), 8.03 - 7.38 (m, 4H), 6.90 - 6.51 (m, 1H), 6.26 - 5.87 (m, 2H), 4.46 (s, 1H), 4.14 - 3.35 (m, 5H), 3.27 - 2.81 (m, 3H), 2.70 (brs, 3H), 2.00 - 1.35 (m, 8H). | 629.0 |
| 241. | 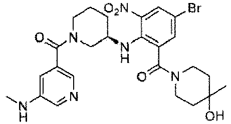 | ¹H NMR (400 MHz, DMSO-*d*₆) δ 8.24 (s, 1H), 8.05 - 7.35 (m, 4H), 6.75 (s, 1H), 6.14 (brs, 1H), 4.46 (s, 1H), 4.29 - 3.33 (m, 6H), 3.28 - 2.86 (m, 3H), 2.70 (brs, 3H), 1.99 - 1.23 (m, 8H), 1.16 (s, 3H). | 575.2 |
| 242. | 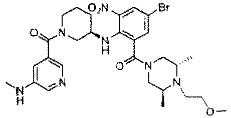 | ¹H NMR (400 MHz, DMSO-*d*₆) δ 8.25 (s, 1H), 8. 11 - 7.17 (m, 4H), 6.76 (s, 1H), 6.15 (brs, 1H), 4.21-3.44 (m, 7H), 3.27 - 3.11 (m, 5H), 3.08 - 2.60 (m, 8H), 2.47 - 2.36 (m, 1H), 1.91 -1.35 (m, 4H), 1.14 - 0.67 (m, 6H). | 632.2 |
| 243. | 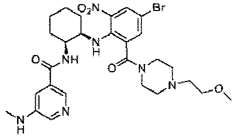 | ¹H NMR (400 MHz, DMSO-*d*₆) δ 8.49 - 8.08 (m, 2H), 8.07 - 7.75 (m, 3H), 7.69 (s, 1H), 7.02 (s, 1H),6.16 (brs, 1H), 4.70 - 4.06 (m, 2H), 3.98 - 3.45 (m, 6H), 3.27 - 2.80 (m, 9H), 2.72 (brs, 3H), 1.88 - 1.28 (m, 8H). | 618.4 |
| 244. | 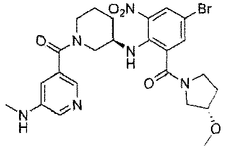 | ¹H NMR (400 MHz, DMSO-*d*₆) δ 8.26 (s, 1H), 8.10 - 7.42 (m, 4H), 6.75 (s, 1H), 6.14 (brs, 1H), 4.12 - 3.53 (m, 8H), 3.29 - 2.97 (m, 5H), 2.70 (brs, 3H), 2.07 - 1.43 (m, 6H). | 561.2 |
| 245. | 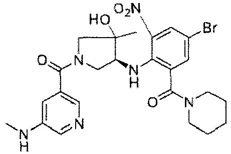 | ¹H NMR (400 MHz, DMSO-*d*₆) δ 8.31 - 8.16 (m, 0.9H), 8.00 (d, *J* = 2.8 Hz. 0.4H), 7.98 (d, *J* = 2.8 Hz, 0.5H), 7.84 (s, 0.4H), 7.79 (d*, J* = 1.6 Hz, 0.5H), 7.74 - 7.48 (m, 2.4H), 6.88 (s, 0.41H), 6.83 (s, 0.5H), 6.21 - 6.05 (m, 0.9H), 5.50 (s, 0.9H), 4.00 - 3.40 (m, 6H), 3.23 - 2.88 (m, 3H), 2.72 (d, *J* = 5.2 Hz, 1.4H), 2.69 (d, *J* = 5.2 Hz. 1.6H), 1.77 - 1.07 (m, 9H). | 561.0 |
| 246. | 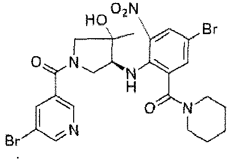 | ¹H NMR (400 MHz, DMSO-*d*₆) δ 8.86 - 8.77 (m, 1H), 8.69 (d, *J* = 1.6 Hz, 0.5H), 8.67 (d, *J* = 1.6 Hz, 0.5H), 8.26 (d, *J* = 2.8 Hz, 0.5H), 8.22 (d, *J* = 2.4 Hz, 0.5H), 8.21 - 8.12 (m, 1H), 7.78 - 7.50 (m, 2H), 5.65 - 5.30 (m, 1H), 4.06 - 3.35 (m, 7H), 3.22 - 2.75 (m, 2H), 1.72 - 1.11 (m, 9H). | 610.0 |
| 247. | 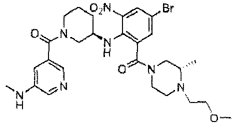 | ¹H NMR (400 MHz, DMSO-*d*₆) δ 8.26 (s, 1H), 8.08 - 7.39 (m, 4H), 6.88 - 6.54 (m, 1H), 6.15 (brs, 1H), 4.30 - 3.36 (m, 7H), 3.26 - 3.11 (m, 5H), 3.05 - 2.56 (m, 7H), 2.45 - 2.08 (m, 3H), 1.93 - 1.38 (m, 4H), 1.10 - 0.78 (m, 3H). | 618.4 |
| 248. | 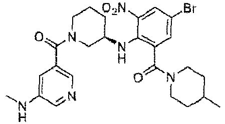 | ¹H NMR (400 MHz, DMSO-*d*₆) δ 8.23 (s, 1H), 8.07 - 7.30 (m, 4H), 6.72 (s, 1H), 6.14 (brs, 1H), 4.40 (s, 1H), 4.13 - 3.42 (m, 4H), 3.23 - 2.97 (m, 2H), 2.96 - 2.56 (m, 5H), 2.00 - 1.39 (m, 7H), 1.28 - 0.87 (m, 5H). | 559.4 |
| 249. | 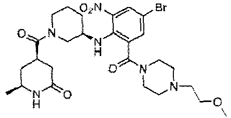 | ¹H NMR (400 MHz, DMSO-*d*₆) δ 8.24 (d, *J* = 2.4 Hz. 1H), 7.85 - 7.32 (m, 3H), 4.18 - 3.38 (m, 9H), 3.23 - 2.74 (m, 9H), 2.70 - 2.56 (m, 1H), 2.41 - 2.02 (m, 4H), 1.99 - 1.16 (m, 7H), 1.09 (d, *J* = 6.0 Hz, 3H). | 609.2 |
| 250. | 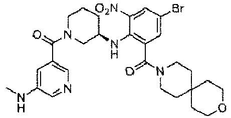 | ¹H NMR (400 MHz, DMSO-*d*₆) δ 8.25 (d, *J* = 2.4 Hz. 1H), 8.09 - 7.34 (m, 4H), 6.75 (s, 1H), 6.15 (brs, 1H), 4.23 - 3.46 (m, 9H), 3.27 - 2.81 (m, 4H), 2.70 (brs, 3H), 2.02 - 1.05 (m, 12H). | 615.2 |
| 251. | 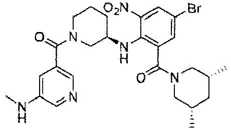 | ¹H NMR (400 MHz, DMSO-*d*₆) δ 8.25 (s, 1H), 8.07 - 7.24 (m, 4H), 6.73 (s, 1H), 6.36 - 5.85 (m, 1H), 4.67 - 4.17 (m, 1H), 4.14 - 3.49 m, 4H), 3.26 -3.01 (m, 2H), 2.98 - 2.56 (m, 5H), 2.22 - 2.06 (m, 1H), 2.02 - 1.26 (m, 7H), 1.04 - 0.54 (m, 6H). | 573.2 |
| 252. | 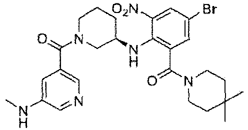 | ¹H NMR (400 MHz, DMSO-*d*₆) δ 8.25 (s, 1H), 8.07 - 7.21 (m, 4H), 6.76 (s, 1H), 6.15 (brs, 1H), 4.31 - 3.50 (m, 6H), 3.18 - 2.78 (m, 3H), 2.70 (brs, 3H), 2.08 - 1.27 (m, 8H), 0.97 (s, 6H). | 573.2 |
| 253. | 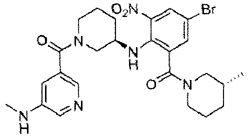 | ¹H NMR (400 MHz, DMSO-*d*₆) δ 8.24 (s, 1H), 8.07 - 7.40 (m, 4H), 6.74 (s, 1H), 6.16 (brs, 1H), 4.49 - 4.12 (m, 1H), 4.08 - 3.37 (m, 4H), 3.28 - 2.91 (m, 2H), 2.89 - 2.55 (m, 4H), 2.45 - 2.17 (m, 1H), 2.03 - 1.25 (m, 8H), 1.21 - 0.97 (m, 1H), 0.96 - 0.62 (m, 3H). | 559.3 |
| 254. | 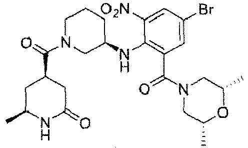 | ¹H NMR (400 MHz, DMSO-*d*₆) δ 8.25 (s, 1H), 8.16 - 7.29 (m, 3H), 4.81 - 3.60 (m, 10H), 3.24 - 3.00 (ny 2H), 2.97 - 2.64 (m, 2H), 2.47 - 2.33 (m, 1H), 2.27 - 1.42 (m, 6H), 1.33 - 0.89 (m, 9H). | 580.2 |
| 255. | 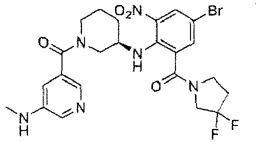 | ¹H NMR (400 MHz, DMSO-*d*₆) δ 8.29 (s, 1H), 8.10 - 7.48 (m, 4H), 6.75 (s, 1H), 6.13 (brs, 1H), 4.14 - 3.43 (m, 7H), 3.23 - 2.83 (m, 2H), 2.70 (brs, 3H), 2.46 - 2.22 (m, 2H), 1.96 - 1.31 (m, 4H). | 567.0 |
| 256. | 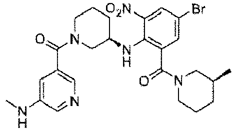 | ¹H NMR (400 MHz, DMSO-*d*₆) δ 8.24 (s, 1H), 8.04 - 7.40 (m, 4H), 6.73 (s, 1H), 6.15 (s, 1H), 4.83 - 4.18 (m, 1H), 4.00 - 3.37 (m, 5H), 3.25 - 2.76 (m, 3H), 2.70 (s, 3H) , 2.01 - 1.26 (m, 8H), 1.20 - 1.04 (m, 1H), 0.98 - 0.56 (m, 3H). | 559.2 |
| 257. | 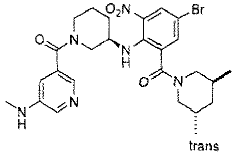 | ¹H NMR (400 MHz, DMSO-*d*₆) δ 8.25 (s, 1H), 8.07 - 7.22 (m, 4H), 6.76 (s, 1H), 6.15 (brs, 1H), 4.20 - 3.40 (m, 5H), 3.23 - 2.86 (m, 4H), 2.70 (brs, 3H), 2.03 - 1.25 (m, 8H), 1.19 - 0.61 (m, 6H). | 573.2 |
| 258. | 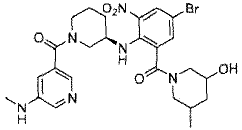 | ¹H NMR (400 MHz, DMSO-*d*₆) δ 8.25 (s, 1H), 8.11 - 7.19 (m, 4H), 6.88 - 6.49 (m, 1H), 6.15 (brs, 1H), 5.09 (s, 0.5H), 4.94 (s, 0.5H), 4.65 - 4.23 (m, 1H), 4.14 - 3.43 (m, 5H), 3.23 - 2.95 (m, 2H), 2.84 - 2.53 (m, 4H), 2.49 -2.19 (m, 1H), 2.06 -1.31 (m, 6H), 1.13 - 0.58 (m, 4H). | 575.4 |
| 259. | 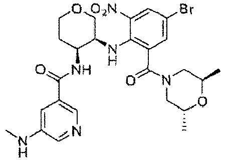 | ¹H NMR (400 MHz, DMSO-*d*₆) δ 8.68 (s, 1H), 8.27 (d, *J* = 2.4 Hz, 1H), 8.24 - 7.74 (m, 3H), 7.58 (d, *J* = 2.0 Hz, 1H), 7.05 (s, 1H), 6.06 (brs, 1H), 4.43 - 4.22 (m, 1H), 4.19 - 3.40 (m, 8H), 3.21 - 2.57 (m, 6H), 2.13 - 1.73 (m, 1H), 1.68 - 1.59 (m, 1H), 1.30 -0.87 (m, 4H), 0.61 (brs, 2H). | 591.3 |
| 260. | 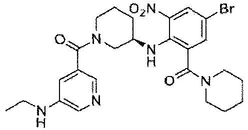 | ¹H NMR (400 MHz, DMSO-*d*₆) δ 8.25 (s, 1H), 8.11 - 7.32 (m, 4H), 6.77 (s, 1H), 6.10 (brs, 1H), 4.15 - 3.41 (m, 6H), 3.25 - 2.81 (m, 5H), 2.01 - 1.84 (m, 1H), 1.82 - 1.28 (m, 9H), 1.17 (brs, 3H). | 560.4 |
| 261. | 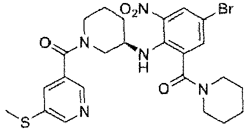 | ¹H NMR (400 MHz, DMSO-*d*₆) δ 8.68 - 8.40 (m, 1H), 8.37 - 8.09 (m, 2H), 8.02 - 7.33 (m, 3H), 4.17 - 3.39 (m, 6H), 3.27 - 2.84 (m, 3H), 2.56 (s, 3H), 2.02 - 1.84 (m, 1H), 1.83 - 1.27 (m, 9H). | 562.2 |
| 262. | 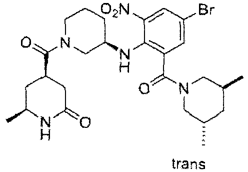 | ¹H NMR (400 MHz, DMSO-*d*₆) δ 8.24 (s, 1H), 7.90 - 7.29 (m, 3H), 4.02 - 3.39 (m, 6H), 3.22 - 2.69 (m, 5H), 2.24 - 1.15 (m, 12H), 1.13 - 1.02 (m, 3H), 1.01 - 0.66 (m, 6H). | 578.5 |
| 263. | 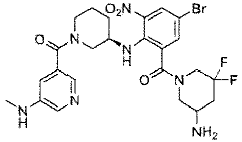 | ¹H NMR (400 MHz, DMSO-*d*₆) δ 8.52 - 8.18 (m, 1H), 8.10 - 7.12 (m, 4H), 6.96 - 6.56 (m, 1H), 6.32 - 5.89 (m, 1H), 4.74 - 4.24 (m, 1H), 4.18 - 3.43 (m, 7H), 3.22 - 2.60 (m, 7H), 2.32 (brs, 1H), 1.94 - 1.34 (m, 5H). | 596.4 |
| 264. | 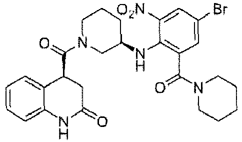 | ¹H NMR (400 MHz, DMSO-*d*₆) δ 10.04 - 10.2 (m, 1H), 8.23 - 8.17 (m, 1H), 7.88 - 7.79 (m, 1H), 7.60 - 7.58 (m, 1H), 7.16 - 6.85 (m, 4H), 4.68 - 3.31 (m, 8H), 3.24 - 3.22 (m, 3H), 2.07 - 1.24 (m, 11H). | 584.1 |
| 265. | 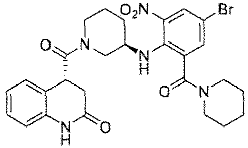 | ¹H NMR (400 MHz, DMSO-*d*₆) δ 10.04 - 10.2 (m, 1H), 8.23 - 8.17 (m, 1H), 7.88 - 7.79 (m, 1H), 7.60 - 7.58 (m, 1H), 7.16 - 6.85 (m, 4H), 4.68 - 3.31 (m, 8H), 3.24 - 3.22 (m, 3H), 2.07 - 1.24 (m, 11H). | 584.1 |
| 266. | 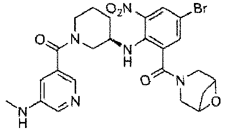 | ¹H NMR (400 MHz, DMSO-*d*₆) δ 8.27 (s, 1H), 7.96 - 7.58 (m, 4H), 6.75 (brs, 1H), 6.13 (brs, 1H), 4.66 (s, 1H), 4.51 (s, 1H), 3.86 - 3.53 (m, 6H), 3.10 - 2.80 (m, 3H), 2.67 (s, 3H), 1.96 - 1.43 (m, 6H). | 559.2 |
| 267. | 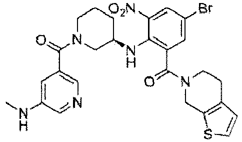 | ¹H NMR (400 MHz, DMSO-*d*₆) δ 8.28 (s, 1H), 8.09 - 7.46 (m, 4H), 7.45 - 7.28 (m, 1H), 6.97 - 6.81 (m, 1H), 6.73 (s, 1H), 6.12 (brs, 1H), 5.12 - 3.36 (m, 7.5H), 3.25 - 2.92 (m, 1.5H), 2.90 - 2.57 (m, 5H), 1.90 - 0.79 (m, 4H). | 599.3 |
| 268. | 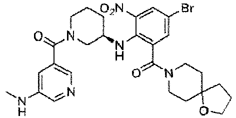 | ¹H NMR (400 MHz, DMSO-*d*₆) δ 8.24 (s, 1H), 8.09 - 7.38 (m, 4H), 6.75 (s, 1H), 6.14 (s, 1H), 4.26 - 3.34 (m, 9H), 3.28 - 2.99 (m, 2H), 2.70 (s, 3H), 2.00 - 1.80 (m, 3H), 1.78 - 1.24 (m, 9H). | 601.2 |
| 269. | 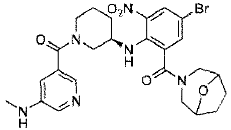 | ¹H NMR (400 MHz, DMSO-*d*₆) δ 8.26 (s, 1H), 8.05 - 7.40 (m, 4H), 6.93 - 6.54 (m, 1 H), 6.30 - 5.86 (m, 1H), 4.42 (brs, 1H), 4.30 - 3.37 (m, 6H), 3.26 - 2.81 (m, 4H), 2.70 (s, 3H), 2.07 - 1.27 (m, 8H). | 573.2 |
| 270. | 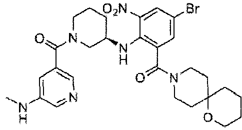 | ¹H NMR (400 MHz, DMSO-*d*₆) δ 8.24 (s, 1H), 8.05 - 7.41 (m, 4H), 6.75 (s, 1H), 6.14 (brs, 1H), 4.28 - 3.40 (m, 7H), 3.25 - 2.93 (m, 4H), 2.70 (brs, 3H), 2.01 - 1.23 (m, 14H). | 615.2 |
| 271. | 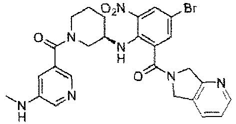 | ¹H NMR (400 MHz, DMSO-*d*₆) δ 8.54 -8.42 (m, 1H), 8.32 (s, 1H), 8.05 - 7.66 (m, 4H), 7.65 - 7.50 (m, 1H), 7.40 - 7.26 (m, 1H), 6.70 (s, 1H), 6.12 (brs, 1H), 5.00 - 4.33 (m, 4H), 4.03 - 3.39 (m, 4H), 3.25 - 3.07 (m, 1H), 2.69 (brs, 3H), 1.90 - 1.66 (m, 2H), 1.64 - 1.27 (m, 2H). | 580.2 |
| 272. | 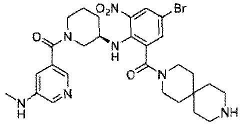 | ¹H NMR (400 MHz, DMSO-*d*₆) δ 8.39 (s, 1H), 8.11 - 7.35 (m, 4H), 6.75 (s, 1H), 6.16 (brs, 1H), 1.90 - 3.87 (m, 5H), 3.32 - 3.00 (m, 5H), 2.89 (brs, 4H), 2.70 (brs, 3H), 1.99 - 1.22 (m, 12H). | 614.0 |
| 273. | 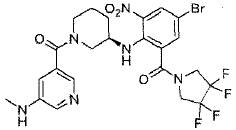 | ¹H NMR (400 MHz, DMSO-*d*₆) δ 8.32 (s, 1H), 7.98 (s, 1H), 7.84 (s, 1H), 7.77 - 7.53 (m, 2H), 6.75 (s, 1H), 6.15 (brs, 1H), 4.53 - 3.92 (m, 4H), 3.89 - 3.42 (m, 5H), 2.69 (brs, 3H), 1.87 (brs, 1H), 1.76 - 1.30 (m, 3H). | 603.0 |
| 274. | 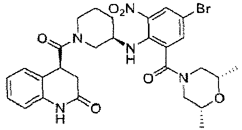 | ¹H NMR (400 MHz, DMSO-*d*₆) δ 10.05 (s, 1H), 8.27 - 8.23 (m, 1H), 7.91 - 7.64 (m, 2H), 7.18 - 7.14 (m, 1H), 7.03 - 6.85 (m, 3H), 4.50 - 3.32 (m, 11H), 2.86 - 2.85 (m, 1H), 2.50 - 2.33 (m, 2H), 2.04 - 1.46 (m, 4H), 1.16 (s, 3H), 1.00 (s, 3H). | 614.1 |
| 275. | 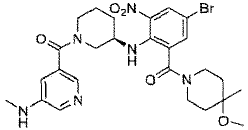 | ¹H NMR (400 MHz, DMSO-*d*₆) δ 8.25 (s, 1H), 8.09 - 7.36 (m, 4H), 6.76 (s, 1H), 6.15 (brs, 1H), 4.38 - 3.41 (m, 5H), 3.26 - 2.95 (m, 7H), 2.70 (s, 3H), 2.05 - 1.24 (m, 8H), 1.26 (s, 3H). | 589.2 |
| 276. | 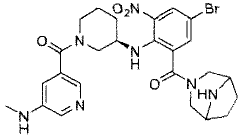 | ¹H NMR (400 MHz, DMSO-*d*₆) δ 8.25 (s, 1H), 8.06 - 7.33 (m, 4H), 6.96 - 6.51 (m, 1H), 6.30 -5.85 (m, 1H), 4.30 - 3.39 (m, 6H), 3.29 - 2.77 (m, 6H), 2.70 (brs, 3H), 2.07 - 1.22 (m, 8H). | 572.1 |
| 277. | 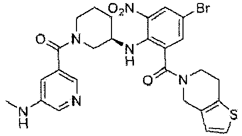 | ¹H NMR (400 MHz, DMSO-*d*₆) δ 8.29 (s, 1H), 8.08 -7.47 (m, 4H), 7.39 (d, *J* = 5.2 Hz, 0.5H), 7.32 (s, 0.5H), 6.97 (d, *J* = 4.8 Hz, 0.5H), 6.87 - 6.81 (m, 1.5H), 6.14 (brs, 1H), 4.93 - 4.32 (m, 2H), 4.30 - 3.36 (m, 5H), 3.28 - 2.77 (m, 4H), 2.70 (s, 3H), 1.89 - 1.17 (m, 4H). | 599.3 |
| 278. | 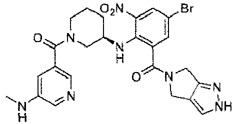 | ¹H NMR (400 MHz, DMSO-*d*₆) δ 12.77 (s, 1H), 8.30 (s, 1H), 8.08 - 7.37 (m, 5H), 6.70 (s, 1H), 6.27 - 5.94 (m, 1H), 4.86 - 4.03 (m, 4H), 3.97 - 3.41 (m, 4H), 3.24 - 3.06 (m, 1H), 2.69 (brs, 3H), 1.93 - 1.28 (m, 4H). | 569.2 |
| 279. | 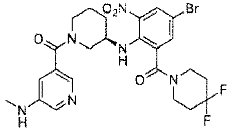 | ¹H NMR (400 MHz, DMSO-*d*₆) δ 8.27 (s, 1H), 8.09 - 7.48 (m, 4H), 6.76 (s, 1H), 6.15 (s, 1H), 4.20 - 3.34 (m, 7H), 3.29 - 2.93 (m, 2H), 2.70 (s, 3H), 2.30 - 1.79 (m, 5H), 1.77 - 1.30 (m, 3H). | 581.2 |
| 280. | 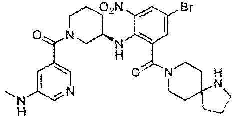 | ¹H NMR (400 MHz, DMSO-*d*₆) δ 8.25 (s, 1H), 8.11 - 7.32 (m, 4H), 6.76 (s, 1H), 6.16 (brs, 1H), 4.74 - 3.90 (m, 4H), 3.54 - 3.07 (m, 6H), 2.95 (brs, 2H), 2.70 (brs, 3H), 2.07 - 1.22 (m, 12H). | 600.2 |
| 281. | 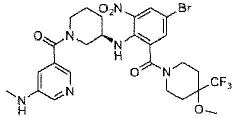 | ¹H NMR (400 MHz, DMSO-*d*₆) δ 8.27 (s, 1H), 8.14 - 7.38 (m, 4H), 6.92 -6.56 (m, 1H), 6.15 (brs, 1H), 4.73 - 3.51 (m, 9H), 3.30 - 2.82 (m, 3H), 2.71 (brs, 3H), 2.29 - 1.29 (m, 8H). | 643.2 |
| 282. | 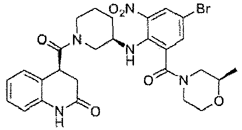 | ¹H NMR (400 MHz, DMSO-*d*₆) δ 10.03 (s, 1H), 8.27 - 8.23 (m, 1H), 7.90 - 7.66 (m, 2H), 7.16 - 7.14 (m, 1H), 7.00 - 6.85 (m, 3H), 4.50 - 3.32 (m, 11H), 2.89 - 2.88 (m, 1H), 2.56 - 2.49 (m, 3H), 1.90 - 1.46 (m, 4H), 1.15 - 1.03 (m, 3H). | 600.2 |
| 283. | 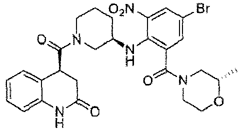 | ¹H NMR (400 MHz, DMSO-*d*₆) δ 10.04 (s, 1H), 8.27 - 8.23 (m, 1H), 7.81 - 7.66 (m, 2H), 7.18 -7.14 (m, 1H), 6.99 - 6.85 (m, 3H), 4.51 - 3.32 (m, 11H), 2.88 - 2.86 (m, 1H), 2.51 - 2.49 (m, 3H), 2.07 - 1.44 (m, 4H), 1.23 - 1.00 (m, 3H). | 600.2 |
| 284. | 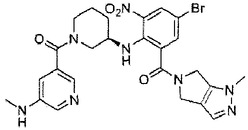 | ¹H NMR (400 MHz, DMSO-*d*₆) δ 8.30 (s, 1H), 8.04 - 7.48 (m, 4H), 7.28 (s, 0.5H), 7.17 (s, 0.5H), 6.79 - 6.57 (m, 1H), 6.10 (brs, 1H), 4.86 - 4.18 (m, 4H), 3.96 - 3.41 (m, 7H), 3.23 - 3.07 (m, 1H), 2.69 (brs, 3H), 1.91 - 1.30 (m, 4H). | 583.1 |
| 285. | 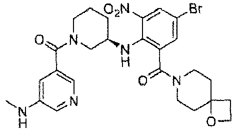 | ¹H NMR (400 MHz, DMSO-*d*₆) δ 8.25 (s, 1H), 8.08 - 7.41 (m, 4H), 6.75 (s, 1H), 6.13 (brs, 1H), 4.63 - 4.28 (m, 2H), 4.22 - 3.41 (m, 6H), 3.27 - 2.99 (m, 3H), 2.70 (brs, 3H), 2.44 - 2.19 (m, 2H), 2.09 - 1.28 (m, 8H). | 587.4 |
| 286. | 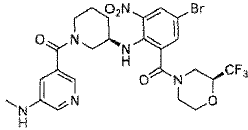 | ¹H NMR (400 MHz, DMSO-*d*₆) δ 8.29 (s, 1H), 8.11 - 1.17 (m, 4H), 6.75 (s, 1H), 6.14 (brs, 1H), 4.71 - 4.17 (m, 2H), 4.16 - 3.34 (m, 7H), 3.26 - 2.84 (m, 3H), 2.70 (brs, 3H), 2.01 - 1.32 (m, 4H). | 615.0 |
| 287. | 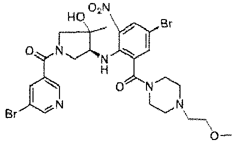 | ¹H NMR (400 MHz, DMSO-*d*₆) δ 8.89 - 8.74 (m, 1H), 8.69 (d, *J* = 1.6 Hz, 0.5H), 8.67 (d, *J* = 1.6 Hz, 0.5H), 8.26 (d, *J* = 2.4 Hz, 0.5H), 8.23 (d, *J* = 2.4 Hz, 0.51H), 8.21 - 8.12 (m, 1H), 7.80 - 7.44 (m, 2H), 5.51 (s, 1H), 4.08 - 3.61 (m, 3H), 3.59 - 3.37 (m, 7H), 3.26 - 3.15 (m, 4H), 2.69 - 2.51 (m, 3H), 2.49 - 2.16 (m, 3H), 1.35 (s, 1.4H), 1.22 (s, 1.6H). | 669.0 |
| 288. | 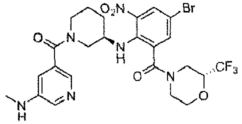 | ¹H NMR (400 MHz, DMSO-*d*₆) δ 8.28 (s, 1H), 8.09 - 7.48 (m, 4H), 6.75 (s, 1H), 6.15 (brs, 1H), 4.65 - 4.17 (m, 2H), 4.16 - 3.37 (m, 7H), 3.24 - 2.82 (m, 3H), 2.70 (brs, 3H), 2.05 - 1.29 (m, 4H). | 615.0 |
| 289. | 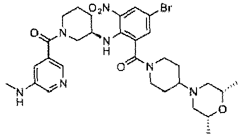 | ¹H NMR (400 MHz, DMSO-*d*₆) δ 8.25 (s, 1H), 8.10 - 7.36 (m, 4H), 6.74 (s, 1H), 6.14 (brs, 1H), 4.41 (s, 1H), 4.16 - 3.35 (m, 7H), 3.28 - 2.93 (m, 2H), 2.90 - 2.57 (m, 6H), 2.46 - 2.13 (m, 1H), 2.05 - 1.20 (m, 10H), 1.03 (brs, 6H). | 658.6 |
| 290. | 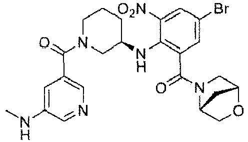 | ¹H NMR (400 MHz, DMSO-*d*₆) δ 8.26 (s, 1H), 7.97 (s, 1H), 7.75 - 7.70 (m, 3H), 6.76 (s, 1H), 6.14 (brs, 1H), 4.90 - 4.27 (m, 2H), 3.77 - 3.06 (m, 9H), 2.70 (s, 3H), 1.99 - 1.45 (m, 6H). | 559.2 |
| 291. | 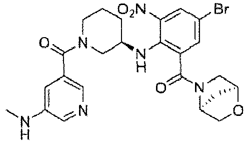 | ¹H NMR (400 MHz, DMSO-*d*₆) δ 8.27 (s, 1H), 7.97 (s, 1H), 7.74 - 7.70 (m, 3H), 6.75 (brs, 1H), 6.13 (brs, 1H), 4.87 - 4.23 (m, 2H), 3.78 - 3.31 (m, 9H), 2.70 (s, 3H), 1.94 - 1.45 (m, 6H). | 559.2 |
| 292. | 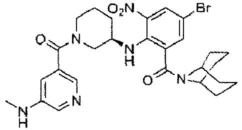 | ¹H NMR (400 MHz, DMSO-*d*₆) δ 8.24 (s, 1H), 7.98 - 7.61 (m, 4H), 6.79 - 6.70 (m, 1H), 6.16 - 5.97 (m, 1H), 4.69 (br, 1H), 4.00 - 3.48 (m, 5H), 3.21 (s, 1H), 2.71 - 2.67 (m, 3H), 2.07 - 1.35 (t, *J* = 64.7 Hz, 16H). | 585.3 |
| 293. | 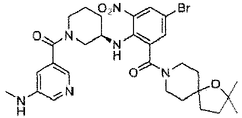 | ¹H NMR (400 MHz, DMSO-*d*₆) δ 8.24 (s, 1H), 8.05 - 7.31 (m, 4H), 6.75 (s, 1H), 6.24 - 5.89 (m, 1H), 4.34 - 3.36 (m, 6H), 3.27 - 2.94 (m, 3H), 2.70 (brs, 3H), 2.00 - 1.31 (m, 12H), 1.24 - 1.09 (m, 6H). | 629.2 |
| 294. | 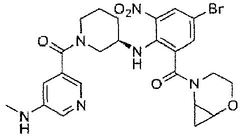 | ¹H NMR (400 MHz, DMSO-*d*₆) δ 8.27 (s, 1H), 8.06 - 7.49 (m, 4H), 6.76 (s, 1H), 6.31 - 5.92 (m, 1H), 4.17 - 3.36 (m, 9H), 3.27 - 2.85 (m, 2H), 2.70 (brs, 3H)_ 2.02 - 1.38 (m, 4H), 1.01 -0.36 (m, 2H). | 559.0 |
| 295. | 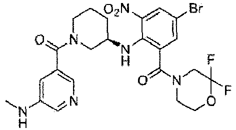 | ¹H NMR (400 MHz, DMSO-*d*₆) δ 8.31 (s, 1H), 8.16 - 7.44 (m, 4H), 6.75 (s, 1H), 6.15 (brs, 1H), 4.79 - 3.49 (m, 10H), 3.27 - 3.09 (m, 1H), 2.70 (brs, 3H), 2.00 - 1.46 (m, 4H). | 583.0 |
| 296. | 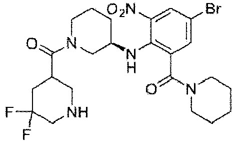 | ¹H NMR (400 MHz, DMSO-*d*₆) δ 8.32 (s, 1H), 8.00 - 7.41 (m, 2H), 4.59 - 3.69 (m, 5H), 3.25 -2.58 (m, 9H), 2.43 - 2.21 (m, 1H), 2.16 - 1.79 (m, 3H), 1.77 - 1.27 (m, 9H). | 558.2 |
| 297. | 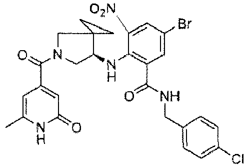 | ¹H NMR (400 MHz, DMSO-*d*₆) δ 11.73 (s, 1H), 9.43 (t, *J* = 5.8 Hz, 0.5H), 9.36 (t, *J* = 5.8 Hz, 0.5H), 8.23 (d, *J* = 2.4 Hz, 0.5H), 8.18 (d, *J* = 2.4 Hz. 0.5H), 8.06 (d, *J* = 7.6 Hz, 0.5H), 8.01 (d, *J* = 8.0 Hz. 0.5H), 7.86 (d, *J* = 2.4 Hz, 0.5H), 7.83 (d, *J* = 2.4 Hz, 0.5H), 7.46 - 7.30 (m, 4H), 6.08 (s, 0.5H), 5.99 (s, 0.5H), 5.91 (s, 0.5H), 5.65 (s, 0.5H), 4.46 - 4.29 (m, 2H), 3.69 - 3.52 (m, 1.5H), 3.44 (dd, *J* = 11.4, 4.2Hz, 0.5H), 3.39 - 3.13 (m, 1H), 3.30 - 3.21 (m, 1H), 3.17 (d, *J* = 12.4 Hz, 0.5H), 3.12 (d, *J* = 10.8 Hz, 0.5H), 2.15 (s, 1.5H), 2.13 (s, 1.5H), 0.74 - 0.56 (m, 3H), 0.55 - 0.43 (m, 1H). | 614.0 |
| 298. | 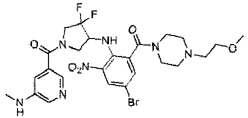 | ¹H NMR (400 MHz, DMSO-*d*₆) δ 8.26 (s, 1H), 8.03 (d, *J* = 2.4, 1H), 7.87 (s, 1H), 7.81 - 7.50 (m, 1.6H), 7.00 (brs, 0.4H), 6.92 (s, 1H), 6.17 (brs, 1H), 4.53 - 3.36 (m, 9H), 3.30 - 2.92 (m, 5H), 2.72 (d, *J* = 5.2, 3H), 2.65 - 2.51 (m, 2H), 2.46 - 1.85 (m, 4H). | 626.0 |
| 299. | 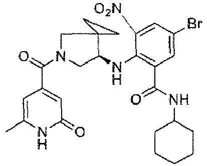 | ¹H NMR (400 MHz, DMSO-*d*₆) δ 11.74 (s, 1H), 8.73 (d, *J* = 8.0 Hz. 0.5H), 8.64 (d, *J* = 7.6 Hz. 0.5H), 8.22 (d, *J* = 2.8 Hz, 0.5H), 8.17 (d, *J* = 2.4 Hz, 0.5H), 8.08 (d, *J* = 6.8 Hz, 0.5H), 8.01 (d, *J* = 8.0 Hz. 0.5H), 7.73 (d, *J* = 2.4 Hz, 0.5H), 7.68 (d, *J* = 2.4 Hz. 0.5H), 6.09 (s, 0.5H), 6.03 (s, 0.5H), 5.92 (s, 0.5H), 5.75 (s, 0.5H), 3.73 - 3.52 (m, 4.5H), 3.39 - 3.36 (m, 0.5H), 3.27 (d, *J* = 12.4 Hz, 0.5H), 3.19 (d, *J* = 10.8 Hz, 0.5H), 2.17 (s, 1.5H), 2.14 (s, 1.5H), 1.89 - 1.65 (m, 4H), 1.64 - 1.52 (m, 1H), 1.37 - 1.16 (m, 5H), 0.80 - 0.61 (m, 4H). | 572.1 |
| 300. | 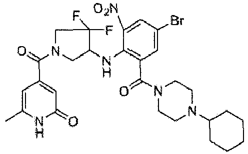 | ¹H NMR (400 MHz, DMSO-*d*₆) δ 11.82 (s, 1H), 8.25 (d, *J* = 2.4 Hz, 1H), 7.81 - 7.67 (m, 1H), 7.61 (s, 0.5H), 6.92 (s, 0.5H), 6.24 - 6.12 (m, 1H), 6.00 (s, 1H), 4.45 - 4.16 (m, 1H), 4.13 - 3.38 (m, 6H), 3.29 - 2.90 (m, 2H), 2.65 - 2.55 (m, 1H), 2.46 - 2.09 (m, 7H), 1.81 - 1.51 (m, 5H), 1.28 - 0.98 (m, 5H). | 651.1 |
| 301. | 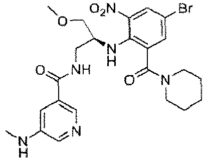 | ¹H NMR (400 MHz, DMSO-*d*₆) δ 8.70 (s, 0.5H), 8.27 (s, 0.5H), 8.21 - 7.92 (m, 3H), 7.71 (d, *J* = 10.0 Hz, 0.5H), 7.62 - 7.38 (m, 1.5H), 7.11 (s, 0.5H), 7.05 (s, 0.5H), 6.19 - 5.95 (m, 1H), 3.97 - 3.36 (m, 7H), 3.30 - 3.05 (m, 5H), 2.71 (d, *J* = 5.2 Hz, 3H), 1.83 - 1.29 (m, 6H) | 549.2 |
| 302. | 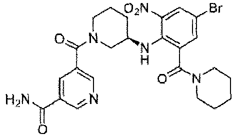 | ¹H NMR (400 MHz, DMSO-*d*₆) δ 9.08 (s, 1H), 8.66 (s, 1H), 8.35 - 8.05 (m, 3H), 7.96 - 7.36 (m, 3H), 4.02 - 3.51 (m, 4H), 3.49 - 3.37 (m, 3H), 3.21 - 3.00 (m, 2H), 2.05 -1.24 (m, 10H). | 559.0 |
| 303. | 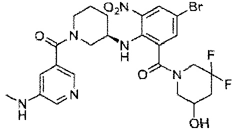 | ¹H NMR (400 MHz, DMSO-*d*₆) δ 8.29 (s, 1H), 8.16 - 7.37 (m, 4H), 6.94 - 6.56 (m, 1H), 6.29 - 5.88 (m, 1H), 5.61 - 4.92 (m, 1H), 4.94 - 3.37 (m, 8H), 3.22 - 2.80 (m, 2H), 2.70 (brs, 3H), 2.46 - 2.20 (m, 1H), 2.16 - 1.35 (m, 5H), | 597.0 |
| 304. | 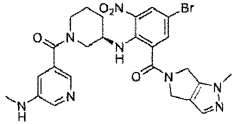 | ¹H NMR (400 MHz, DMSO-*d*₆) δ 8.30 (s, 1H), 8.04 - 7.48 (m, 4H), 7.28 (s, 0.5H), 7.17 (s, 0.5H), 6.79 - 6.57 (m, 1H), 6.10 (brs, 1H), 4.86 - 4.18 (m, 4H), 3.96 - 3.41 (m, 7H), 3.23 - 3.07 (m, 1H), 2.69 (brs, 3H), 1.91 - 1.30 (m, 4H). | 583.1 |
| 305. | 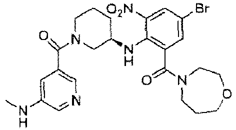 | ¹H NMR (400 MHz, DMSO-*d*₆) δ 8.25 (s, 1H), 8.08 - 7.79 (m, 2H), 7.77 - 7.47 (m, 2H), 6.90 - 6.57 (m, 1H), 6.28 - 5.94 (m, 1H), 4.08 - 3.41 (m, 11H), 3.28 - 2.94 (m, 2H), 2.70 (brs, 3H), 1.99 - 1.34 (m, 6H). | 561.0 |
| 306. | 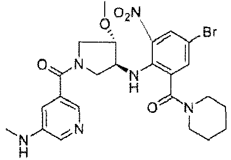 | ¹H NMR (400 MHz, DMSO-*d*₆) δ 8.23 (s, 0.5H), 8.20 (s, 0.5H), 8.01 (s, 0.5H), 7.99 (s, 0.5H), 7.86 (s, 0.5H), 7.79 (s, 0.5H), 7.67 (s, 0.5H), 7.60 (s, 0.5H), 7.51 - 7.35 (m, 1H), 7.00 - 6.76 (m, 1H), 6.13 (brs, 1H), 4.32 - 3.55 (m, 8H), 3.29 - 2.87 (m, 5H), 2.81 - 2.62 (m, 3H), 1.76 -1.18 (m, 6H). | 561.0 |
| 307. | 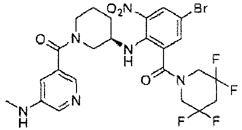 | ¹H NMR (400 MHz, DMSO-*d*₆) δ 8.31 (s, 1H), 8.13 - 7.84 (m, 2H), 7.81 - 7.47 (m, 2H), 6.95 - 6.60 (m, 1H), 6.32 - 5.85 (m, 1H), 4.52 - 3.44 (m, 7H), 3.26 - 2.81 m, 4H), 2.70 (brs, 3H), 1.93 - 1.44 (m, 4H). | 617.0 |
| 308. | 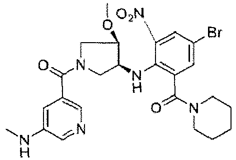 | ¹H NMR (400 MHz, DMSO-*d*₆) δ 8.49 - 8.12 (m, 2H), 8.00 (s, 1H), 7.92 - 7.76 (m, 1H), 7.67 (d, *J* = 13.2 Hz, 0.4H), 7.58 (d, *J* = 15.2 Hz, 0.6H), 6.98 - 6.77 (m, 1H), 6.13 (brs, 1H), 4.31 - 3.38 (m, 9H), 3.31 - 3.14 (m, 3H), 3.07 -2.84 (m, 1H), 2.73 -2.65 (m, 3H), 1.75 - 1.03 (m, 6H). | 56 L2 |
| 309. | 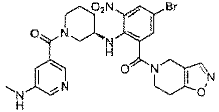 | ¹H NMR (400 MHz, DMSO-*d*₆) δ 8.57 (s, 0.5H), 8.36 (s, 0.5H), 8.29 (s, 1H), 8.10 - 7.34 (m, 4H), 6.74 (s, 1H), 6.14 (brs, 1H), 5.00 - 3.48 (m, 7H), 3.23 - 2.79 (m, 4H), 2.70 (brs, 3H), 1.97 - 1.28 (m, 4H). | 584.0 |
| 310. | 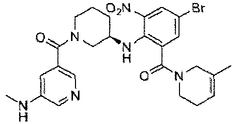 | ¹H NMR (400 MHz, DMSO-*d*₆) δ 8.26 (s, 1H), 8.11 - 7.27 (m, 4H), 6.74 (s, 1H), 6.15 (brs, 1H), 5.80 - 5.31 (m, 1H), 4.50 - 3.44 (m, 7H), 3.26 - 3.05 (m, 2H), 2.70 (brs, 3H), 2.29 - 1.25 (m, 9H). | 557.1 |
| 311. | 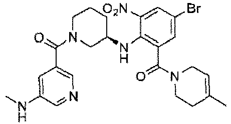 | ¹H NMR (400 MHz, DMSO-*d*₆) δ 8.26 (s, 1H), 8.08 - 7.23 (m, 4H), 6.76 (s, 1H), 6.15 (brs, 1H), 5.61 - 5.02 (m, 1H), 4.45 - 3.49 (m, 6H), 3.28 - 3.01 (m, 2H), 2.96 - 2.78 (m, 1H), 2.70 (brs, 3H), 2.27 - 1.13 (m, 9H). | 557.2 |
| 312. | 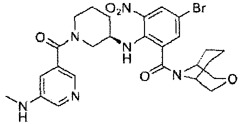 | ¹H NMR (400 MHz, DMSO-*d*₆) δ 8.27 (s, 1H), 7.98 - 7.78 (m, 4H), 6.79 - 6.71 (m, 1H), 6.15 (s, 1H), 4.44 (br. 1H), 3.98 - 3.32 (m, 10H), 2.70 (s, 3H), 1.86 - 1.36 (m, 10H). | 587.0 |
| 313. | 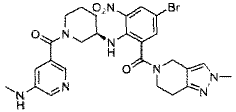 | ¹H NMR (400 MHz, DMSO-*d*₆) δ 8.27 (s, 1H), 8.10 - 7.22 (m, 5H), 6.89 - 6.50 (m, 1H), 6.11 (brs, 1H), 4.92 - 3.48 (m, 10H), 3.17 - 2.91 (m, 2H), 2.86 - 2.59 (m, 5H), 1.99 - 1.27 (m, 4H). | 597.1 |
| 314. | 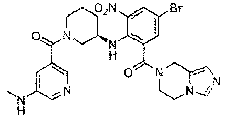 | ¹H NMR (400 MHz, DMSO-*d*₆) δ 8.30 (s, 1H), 8.07 - 7.48 (m, 5H), 6.83 (s, 0.5H) , 6.79 - 6.56 (m, 1.5H), 6.13 (brs, 1H), 5.27 - 4.30 (m, 2H), 4.20 - 3.52 (m, 7H), 3.15 - 2.82 (m, 2H), 2.70 (brs, 3H) , 1.90 - 1.32 (m, 4H). | 583.0 |
| 315. | 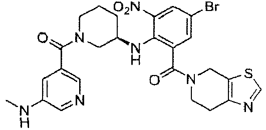 | ¹H NMR (400 MHz, DMSO-*d*₆) δ 9.01 (s, 0.5H), 8.97 (s, 0.5H), 8.29 (s, 1H), 8.08 - 7.38 (m, 4H), 6.76 (s, 1H), 6.13 (brs, 1H), 5.21 - 3.49 (m, 7H), 3.24 - 2.76 (m, 4H), 2.70 (brs, 3H), 1.99 - 1.08 (m, 4H). | 600.0 |
| 316. | 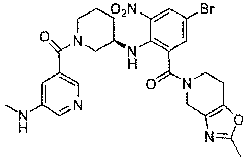 | ¹H NMR (400 MHz, DMSO-*d*₆) δ 8.29 (s, 1H), 8.08 - 7.51 (m, 4H), 6.74 (s, 1H), 6.15 (brs, 1H), 4.67 - 4.16 (m, 2H), 4.02 - 3.34 (m, 5H), 3.29 - 2.99 (m, 2H), 2.93 - 2.56 (m, 5H), 2.39 (s, 1.6H), 2.35 (s, 1.4H), 1.83 - 1.01 (m, 4H). | 598.2 |
| 317. | 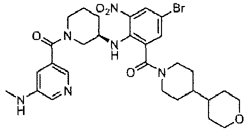 | ¹H NMR (400 MHz, DMSO-*d*₆) δ 8.26 (s, 1H), 8.10 - 7.34 (m, 4H), 6.92 - 6.57 (m, 1H), 6.34 - 5.90 (m, 1H), 4.79 - 4.26 (m, 1H), 4.23 - 3.48 (m, 6H), 2.28 - 2.83 (m, 5H), 2.70 (brs, 4H), 2.02 - 1.42 (m, 8H), 1.39 - 1.26 (m, 2H), 1.22 - 0.92 (m, 4H). | 629.2 |
| 318. | 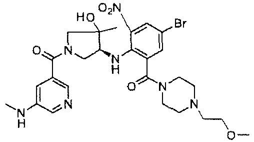 | ¹H NMR (400 MHz, DMSO-*d*₆) δ 8.26 (d, *J* = 2.4 Hz, 0.5H), 8.23 (d, *J* = 2.0 Hz, 0.5H), 8.03 - 7.92 (m, 1H), 7.84 (s, 0.5H), 7.79 (s, 0.5H), 7.73 - 7.52 (m, 2H), 6.88 (s, 0.5H), 6.83 (s, 0.5H), 6.18 - 6.04 (m, 1H), 5.49 (s, 1H), 4.07 - 3.62 (m, 3H), 3.60 - 3.35 (m, 7H), 3.27 - 3.02 (m, 4H), 2.96 - 2.59 (m, 4H), 2.57 - 2.51 (m, 2H), 2.45 - 2.11 (m, 3H), 1.35 (s, 1.5H), 1.23 (s, 1.5H). | 620.1 |
| 319. | 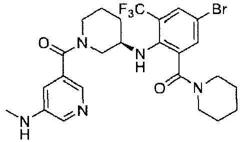 | ¹H NMR (400 MHz, DMSO-*d*₆) δ 8.05 - 7.83 (m, 1H), 7.80 - 7.60 (m, 2H), 7.51 (s, 1H), 6.74 (s, 1H), 6.28 - 5.86 (m, 1H), 4.77 - 4.30 (m, 1H), 4.22 - 3.38 (m, 4H), 3.27 - 2.80 (m, 5H), 2.69 (brs, 3H), 1.96 - 1.14 (m, 10H). | 568.2 |
| 320. | 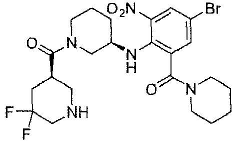 | ¹H NMR (400 MHz, DMSO-*d*₆) δ 8.23 (d, *J* = 2.0 Hz, 1H), 7.92 - 7.65 (m, 1H), 7.63 - 7.55 (m, 1H), 4.03 - 3.39 (m, 6H), 3.29 - 3.09 (m, 3H), 3.05 - 2.53 (m, 5H), 2.40 - 2.18 (m, 1H), 2.15 - 1.81 (m, 3H), 1.78 -1.29 (m, 9H) | 558.0 |
| 321. | 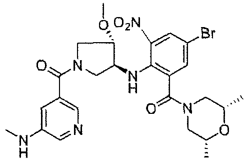 | ¹H NMR (400 MHz, DMSO-*d*₆) δ 8.36 - 8.16 (m, 1H), 8.08 - 7.94 (m, 1H), 7.91 - 7.59 (m, 2H), 7.56 - 7.22 (m, 1H), 6.96 - 6.75 (m, 1H), 6.23 - 6.06 (m, 1H), 4.48 - 4.19 (m, 1H), 4.14 - 3.39 (m, 9H), 3.29 - 3.01 (m, 4H), 2.99 - 2.65 (m, 4H), 1.25 - 0.81 (m, 6H). | 591.2 |
| 322. | 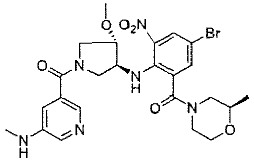 | ¹H NMR (400 MHz, DMSO-*d*₆) δ 8.32 - 8.15 (m, 1H), 8.06 - 7.92 (m, 1H), 7.90 - 7.60 (m, 2H), 7.55 - 7.29 (m, 1H), 6.95 - 6.75 (m, 1H), 6.21 - 6.04 (m, 1H), 4.45 - 4.13 (m, 1H), 4.11 - 3.36 (m, 9H), 3.28 - 2.82 (m, 5H), 2.73 - 2.54 (m, 4H), 1.25 - 0.86 (m, 3H). | 577.2 |
| 323. | 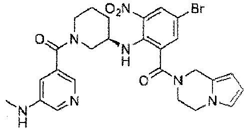 | ¹H NMR (400 MHz, DMSO-*d*₆) δ 8.29 (s, 1H), 8.08 - 7.44 (m, 4H), 6.89 - 6.53 (m, 2H), 6.30 - 5.62 (m, 3H), 5.09 - 4.36 (m, 2H), 4.33 - 3.41 (m, 8H), 3.23 - 2.94 (m, 1H), 2.70 (brs, 3H), 2.05 - 0.98 (m, 4H). | 582.0 |

| **Example** | **Structural Formula** | ¹HNMR | MS (ESI) ' M/Z [M+H]⁺ |
|---|---|---|---|
| 324. | 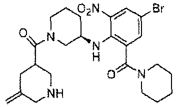 | ¹H NMR (400 MHz, DMSO-*d*₆) δ 8.33 - 8.13 (m, 2H), 8.00 - 7.66 (m, 1H), 7.65 - 7.53 (m, 1H), 4.84 - 4.51 (m, 2H), 4.21 - 3.63 (m, 3H), 3.36 - 2.70 (m, 11H), 2.67 - 2.55 (m, 0.7H), 2.36 - 2.10 (m, 2.3H), 2.02 - 1.31 (m, 10H). | 534.2 |
| 325. | 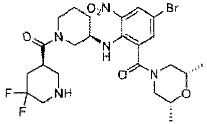 | ¹H NMR (400 MHz, DMSO-*d*₆) δ 8.26 (d, *J* = 2.0 Hz. 1H), 7.97 - 7.55 (m, 2H), 4.48 - 4.23 (m, 1H), 3.97 - 3.32 (m, 7H), 3.28 - 3.13 (m, 1H), 3.12 - 2.58 (m, 6H), 2.47 - 2.18 (m, 2H), 2.17 - 1.24 (m, 6H), 1.15 (d, , *J* = 6.0 Hz, 3H), 1.01 (brs, 3H). | 588.2 |
| 326. | 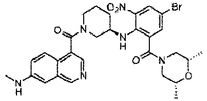 | ¹H NMR (400 MHz, DMSO-*d*₆) δ 9.14 - 8.80 (m, 1H), 8.30 (s, 0.6H), 8.18 - 7.59 (m, 2.7H), 7.56 - 6.94 (m, 2.3H), 6.92 - 6.61 (m, 1H), 6.58 - 6.27 (m, 1H), 4.59 - 3.35 (m, 7H), 3.28 - 2.90 (m, 3H), 2.80 (d, *J* = 4.8 Hz. 3H), 2.45 - 2.11 (m, 1H), 2.08 - 1.33 (m, 4H), 1.18 - 0.88 (m, 6H). | 625.0 |
| 327. | 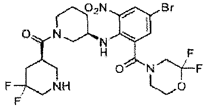 | ¹HNMR (400 MHz, MeOD-d₄) δ 8.45 - 8.36 (m, 1H), 7.68 - 7.59 (m, 1H), 4.32 - 3.60 (m, 9H), 3.13 - 3.00 (m, 3H), 2.91 - 2.69 (m, 1.5H), 2.62 - 2.37 (m, 1.5H), 2.31 -1.89 (m, 4H), 1.88 - 1.55 (m, 3H). | 596.0 |
| 328. | 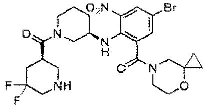 | ¹H NMR (400 MHz, DMSO-*d*₆) δ 8.41 - 8.24 (m, 1H), 8.15 - 7.44 (m, 2H), 4.14 - 3.41 (m, 9H), 3.29 - 3.10 (m, 2H), 3.08 - 2.56 (m, 5H), 2.49 - 1.40 (m, 8H), 0.92 - 0.30 (m, 4H). | 586.0 |
| . 329. | 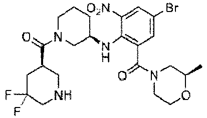 | ¹H NMR (400 MHz, DMSO-*d*₆) δ 8.26 (s, 1H), 7.97 - 7.58 (m, 2H), 4.42 - 4.14 (m, 1H), 4.10 - 3.39 (m, 7H), 3.27 - 3.12 (m, 2H), 3.09 - 2.55 (m, 7H), 2.41 - 2.19 (m, 1H), 2.17 - 1.85 (m, 3H), 1.80 - 1.33 (m, 3H), 1.15 (d, *J* = 6.0 Hz, 1.5H), 1.02 (d, *J* = 4.8 Hz, 1.5H). | 574.2 |
| 330. | 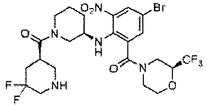 | ¹H NMR (400 MHz, DMSO-*d*₆) δ 8.27 (d, *J* = 2.0 Hz. 1H), 7.93 - 7.52 (m, 2H), 4.70 - 3.61 (m, 8H), 3.14 - 2.91 (m, 5H), 2.90 - 2.80 (m, 2H), 2.79 - 2.69 (m, 2H), 2.29 - 2.17 (m, 1H), 2.13 - 1.80 (m, 3H), 1.75 - 1.34 (m, 3H). | 628.0 |
| 331. | 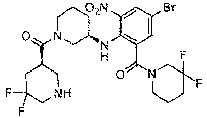 | ¹H NMR (400 MHz, DMSO-*d*₆) δ 8.28 (s, 1H), 8.01 - 7.46 (m, 2H), 4.66 - 3.50 (m, 7H), 3.19 - 2.67 (m, 8H), 2.22 - 1.93 (m, 4H), 1. 90 - 1.35 (m, 6H). | 594.0 |
| 332. | 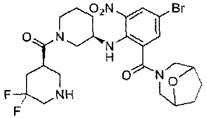 | ¹H NMR (400 MHz, DMSO) δ 8.26 (d, *J* = 2.4 Hz. 1H), 8.00 - 7.53 (m, 2H), 4.52 - 4.37 (m, 1H), 4.32 - 4. 15 (m, 1H), 4.14 - 3.94 (m, 1H), 3.91 - 3.49 (m, 4H), 3.11 - 2.81 (m, 6H), 2.80 - 2.68 (m, 1H), 2.39 - 2.21 (m, 1H), 2.17 - 1.41 (m, 10H). | 586.4 |
| 333. | 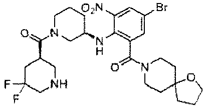 | ¹H NMR (400 MHz, DMSO-*d*₆) δ 8.24 (s, 1H), 7.98 - 7.49 (m, 2H), 4.23 - 3.38 (m, 8H), 3.28 - 2.81 (m, 6H), 2.80 - 2.54 (m, 2H), 2.44 - 2.21 (m, 1H), 2.16 - 1.80 (m, 5H), 1.78 - 1.32 (m, 9H). | 614.2 |
| 334. | 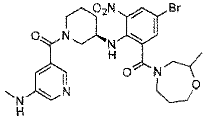 | ¹H NMR (400 MHz, DMSO-*d*₆) δ 8.25 (s, 1H), 8.10 - 7.82 (m, 2H), 7.79 - 7.38 (m, 2H), 6.91 - 6.55 (m, 1H), 6.29 - 5.92 (m, 1H), 4.27 - 3.37 (m, 10H), 3.25 - 2.99 (m, 2H), 2.81 - 2.60 (m, 3H), 1.96 - 1.26 (m, 6H), 1.22 - 0.70 (m, 3H). | 575.2 |
| 335. | 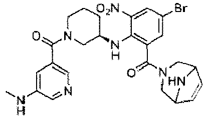 | ¹H NMR (400 MHz, DMSO-*d*₆) δ 8.22 (s, 1H), 8.05 - 7.13 (m, 4H), 6.95 -6.55 (m, 1H), 6.37 - 5.89 (m, 3H), 4.32 - 3.39 (m, 7H), 3.29 - 2.80 (m, 5H), 2.70 (s, 3H), 2.04 -1.31 (m, 4H). | 570.0 |
| 336. | 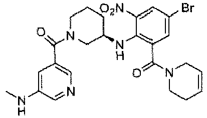 | ¹H NMR (400 MHz, DMSO-*d*₆) δ 8.27 (s, 1H), 8.05 - 7.45 (m, 4H), 6.75 (s, 1H), 6.16 (s, 1H), 5.96 - 5.57 (m, 2H), 4.27 - 3.48 (m, 7H), 3.29 - 3.13 (m, 2H), 2.70 (s, 3H), 2.27 - 1.37 (m, 6H). | 543.0 |
| 337. | 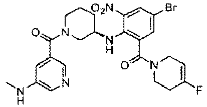 | ¹H NMR (400 MHz, DMSO-*d*₆) δ 8.27 (s, 1H), 8.10 - 7.40 (m, 4H), 6.75 (s, 1H), 6.14 (s, 1H), 5.54 - 5.06 (m, 1H), 4.42 - 3.34 (m, 8H), 3.28 - 3.01 (m, 1H), 2.70 (s, 3H), 2.46 - 2.17 (m, 2H), 1.96 - 1.28 (m, 4H). | 561.05 |
| 338. | 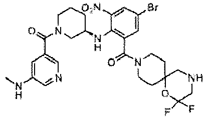 | ¹H NMR (400 MHz, DMSO-*d*₆) δ 8.25 (s, 1H), 8.08 - 7.37 (m, 4H), 6.76 (s, 1H), 6.15 (s, 1H), 4.32 - 3.33 (m, 6H), 3.29 - 2.88 (m, 6H), 2.83 - 2.59 (m, 5H), 2.13 - 1.30 (m, 8H). | 652.1 |
| 339. | 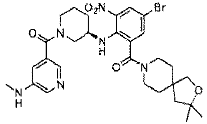 | ¹H NMR (400 MHz, DMSO-*d*₆) δ 8.25 (s, 1H), 8.07 - 7.41 (m, 4H), 6.76 (s, 1H), 6.15 (s, 1H), 4.27 - 3.44 (m, 8H), 3.28 - 3.05 (m, 3H), 2.70 (s, 3H), 1.98 - 1.32 (m, 10H), 1.31 - 0.92 (m, 6H). | 629.2 |
| 340. | 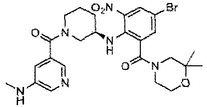 | ¹H NMR (400 MHz, DMSO-*d*₆) δ 8.27 (s, 1H), 8.10 - 7.36 (m, 4H), 6.93 -6.51 (m, 1H), 6.16 (brs, 1H), 4.06 - 3.35 (m, 7H), 3.29 - 2.79 (m, 4H), 2.70 (brs, 3H), 1.97 - 1.31 (m, 4H), 1.20 (brs, 3H), 1.07 (brs, 3H). | 575.2 |
| 341. | 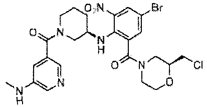 | ¹H NMR (400 MHz, DMSO-*d*₆) δ 8.27 (s, 1H), 8.10 - 7.34 (m, 4H), 6.91 - 6.44 (m, 1H), 4.62 - 4.12 (m, 1H), 4.09 - 3.37 (m, 10H), 3.32 - 2.88 (m, 3H), 2.87 - 2.68 (m, 3H), 2.00 - 1.38 (m, 5H). | 595.1 |
| 342. | 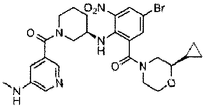 | ¹H NMR (400 MHz, DMSO-*d*₆) δ 8.26 (s, 1H), 8.08 - 7.38 (m, 4H), 6.88 -6.44 (m, 1H), 4.51 - 4.12 (m, 1H), 4.09 - 3.36 (m, 6H), 3.34 - 3.06 (m, 3H), 3.05 - 2.67 (m, 5H), 1.97 - 1.29 (m, 4H), 0.98 - 0.63 (m, 1H), 0.57 - 0.18 (m, 3.6H), 0.13 - 0.01 (m, 0.4H). | 587.1 |
| 343. | 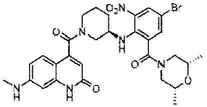 | ¹H NMR (400 MHz, DMSO-*d*₆) δ 11.46 (s, 1H), 8.45 - 8.25 (m, 1H), 7.97 - 7.63 (m, 2H), 6.98 - 6.93 (m, 1H), 6.62 - 6.42 (m, 2H), 6.30 - 6.22 (m, 1H), 6.02 - 5.70 (m, 1H),4.44 - 4.41 (m, 1H), 4.10 - 3.58 (m, 5H), 3.21 - 2.88 (m, 3H), 2.71 - 2.67 (m, 3H), 1.91 - 1.42 (m, 3H), 1.24 - 0.97 (m, 9H). | 641.2 |
| 344. | 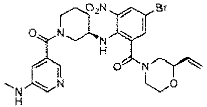 | ¹H NMR (400 MHz, DMSO-*d*₆) δ 8.27 (s, 1H), 8.10 - 7.39 (m, 4H), 6.75 (s, 1H), 6.15 (s, 1H), 5.97 - 5.52 (m, 1H), 5.36 (d, *J* = 17.6 Hz, 0.5H), 5.31 - 5.19 (m, 1H), 5.15 (d, *J* = 10.8 Hz. 0. 5H), 4.52 - 4.16 (m, 1H), 4.15 - 3.37 (m, 7H), 3.30 - 2.85 (m, 3H), 2.70 (s, 4H), 2.04 - 1.30 (m, 4H). | 573.4 |
| 345. | 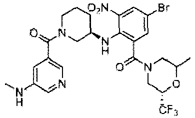 | ¹H NMR (400 MHz, DMSO-*d*₆) δ 8.29 (s, 1H), 8.09 - 7.46 (m, 4H), 6.75 (s, 1H), 6.15 (s, 1H), 4.70 - 4.19 (m, 2H), 4.18 - 3.34 (m, 6H), 3.29 - 3.05 (m, 1.5H), 3.04 - 2.79 (m, 1.5H), 2.70 (brs, 3H), 2.00 - 1.34 (m, 4H), 1.29 - 0.96 (m, 3H). | 629.2 |
| 346. | 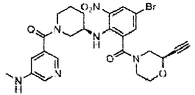 | ¹H NMR (400 MHz, DMSO-*d*₆) δ 8.28 (s, 1H), 8.14 - 7.37 (m, 4H), 6.89 - 6.49 (m, 1H), 6.26 - 5.92 (m, 1H), 4.80 - 4.32 (m, 1H), 4.31 - 3.32 (m, 10H), 3.30 - 2.94 (m, 2H), 2.71 (s, 3H), 2.02 - 1.31 (m, 4H), | 571.4 |
| 347. | 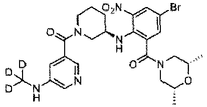 | ¹H NMR (400 MHz, DMSO-*d*₆) δ 8.26 (s, 1H), . 8.09 - 7.40 (m, 4H), 6.73 (s, 1H), 6.12 (brs, 1H), 4.59 - 4.14 (m, 1H), 4.13 - 3.32 (m, 6.6H), 3.27 - 3.09 (m, 1.4H), 2.95 - 2.69 (m, 1H), 2.49 - 2.35 (m, 1H), 2.01 - 1.28 (m, 4H), 1.15 (brs, 3H), 1.02 (brs, 3H). | 578.0 |
| 348. | 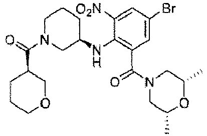 | ¹H NMR (400 MHz, DMSO-*d*₆) δ 8.25 (s, 1H), 7.96 - 7.53 (m, 2H), 4.49 - 4.22 (m, 1H), 4.02 - 3.35 (m, 9H), 3.29 - 2.97 (m, 3H), 2.93 - 2.72 (m, 2H), 2.49 - 2.37 (m, 1H), 2.03 - 1.26 (m, 8H), 1.15 (d, *J* = 5.6 Hz. 3H), 1.02 (brs, 3H). | 553.2 |
| 349. | 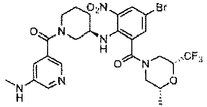 | ¹H NMR (400 MHz, CDCl₃) δ 8.36 (s, 1H), 8.27 - 7.66 (m, 2.7H), 7.63 - 7.34 (m, 1.3H), 7.05 (s, 1H), 4.78 - 3.04 (m, 11H), 2.92 (s, 3H), 2.08 - 1.41 (m, 4H), 1.38 - 1.02 (m, 3H). | 629.0 |
| 350. | 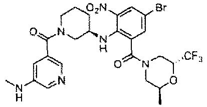 | ¹H NMR (400 MHz, CDCl₃) δ 8.38 (s, 1H), 8.23 - 7.81 (m, 2.3H), 7.80 - 7.34 (m, 1.7H), 7.15 - 6.96 (m, 1H), 4.98 - 3.06 (m, 10H), 2.92 (s, 3H), 2.73 - 2.46 (m, 1H), 2.12 - 1.41 (m, 4H), 1.40 - 1.15 (m, 3H). | 629.0 |
| 351. | 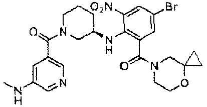 | ¹H NMR (400 MHz, DMSO-*d*₆) δ 8.37 - 8.15 (m, 1H), 8.10 - 7.35 (m, 4H), 6.92 - 6.48 (m, 1H), 6.15 (brs, 1H), 4.28 - 3.35 (m, 8.5H), 3.27 - 2.91 (m, 1.5H), 2.70 (brs, 3H), 2.05 - 1.30 (m, 4H), 0.93 - 0.21 (m, 4H), | 573.0 |
| 352. | 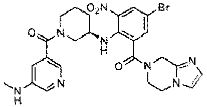 | ¹H NMR (400 MHz, DMSO-*d*₆) δ 8.30 (s, 1H), 8.05 - 7.49 (m, 4H), 7.25 - 7.05 (m, 1H), 6.91 (s, 0.6H), 6.88 (s, 0.4H), 6.74 (s, 1H), 6.14 (s, 1H), 5.08 - 4.40 (m, 2H), 4.38 - 3.39 (m, 8H), 3.24 - 3.00 (m, 1H), 2.70 (s, 3H), 1.91 - 1.30 (m, 4H), | 583.4 |
| 353. | 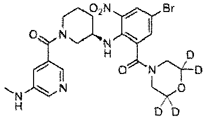 | ¹H NMR (400 MHz, CDCl₃) δ 8.34 (s, 1H), 8.25 - 7.77 (m, 2.7H), 7.48 (s, 1.3H), 7.00 (s, 1H), 4.04 - 3.10 (m, 9H), 2.91 (s, 3H), 2.23 - 1.76 (m, 3H), 1.56 - 1.38 (m, 1H). | 550.6 |
| 354. | 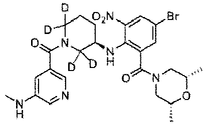 | ¹H NMR (400 MHz, DMSO-*d*₆) δ 8.26 (s, 1H), 8.07 - 7.38 (m, 4H), 6.74 (s, 1H), 6.16 (s, 1H), 4.38 (s, 1H), 3.97 - 3.36 (m, 4H), 3.29 - 3.17 (m, 0.5H), 2.96 - 2.77 (m, 1H), 2.70 (s, 3H), 2.47 - 2.39 (m, 0.5H), 2.01 - 1.29 (m, 4H), 1.15 (brs, 3H), 1.03 (brs, 3H). | 579.0 |
| 355. | 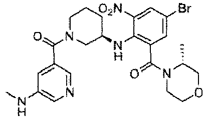 | ¹H NMR (400 MHz, DMSO-*d*₆) δ 8.26 (s, 1H), 8.08 - 7.32 (m, 4H), 6.75 (s, 1H), 6.16 (s, 1H), 4.64 - 3.36 (m, 9.5H), 43.28 - 2.84 (m, 2.5H), 2.70 (brs, 3H), 2.08 - 1.07 (m, 7H). | 561.1 |
| 356. | 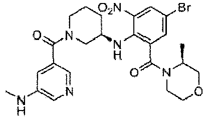 | ¹H NMR (400 MHz, DMSO-*d*₆) *δ* 8.26 (s, 1H), 8.04 - 7.29 (m, 4H), 6.89 -6.50 (m, 1H), 6.28 - 5.91 (m, 1H), 4.64 - 3.34 (m, 10H), 3.29 - 3.09 (m, 2H), 2.70 (brs, 3H), 2.01 - 1.11 (m, 7H). | 560.9 |
| 357. | 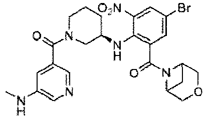 | ¹H NMR (400 MHz, DMSO-*d*₆) δ 9.40 (s, 0.6H), 9.29 (s, 0.4H), 8.55 - 7.56 (m, 7H), 6.16 (s, 0.6H), 5.85 (s, 0.4H), 4.47 - 3.38 (m, 6H), 3.26 - 2.85 (m, 3H), 2.07 - 1.38 (m, 10H). | 588.6 |
| 358. | 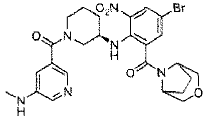 | ¹H NMR (400 MHz, DMSO-*d*₆) δ 8.27 (s, 1H), 8.10 - 7.36 (m, 4H), 6.75 (s, 1H), 6.38 - 5.83 (m, 1H), 4.75 - 4.36 (m, 1H), 4.18 - 3.37 (m, 9H), 3.24 - 3.06 (m, 1H), 2.70 (s, 3H), 2.11 - 1.30 (m, 8H). | 573.0 |
| 359. | 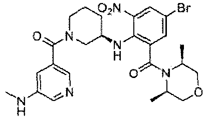 | ¹H NMR (400 MHz, DMSO-*d*₆) δ 8.27 (s, 1H), 8.11 - 7.40 (m, 4H), 6.91 - 6.46 (m, 1H), 6.33 - 5.85 (m, 1H), 4.38 (s, 1H), 4.13 - 3.46 (m, 8H), 3.28 - 2.92 (m, 2H), 2.70 (s, 3H), 2.13 - 1.43 (m, 4H), 1.42 - 1.03 (m, 6H). | 575.2 |
| 360. | 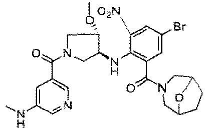 | ¹H NMR (400 MHz, DMSO-*d*₆) δ 8.33 - 8.10 (m, 1H), 8.08 - 7.92 (m, 1H), 7.90 - 7.57 (m, 2.3H), 7.46 - 7.10 (m, 0.7H), 7.01 -6.68 (m, 1H), 6.25 - 6.04 (m, 1H), 4.53 - 3.38 (m, 9H), 3.32 - 2.83 (m, 6H), 2.79 - 2.61 (m, 3H), 2.00 - 1.34 (m, 4H). | 589.2 |
| 361. | 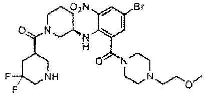 | ¹H NMR (400 MHz, DMSO-*d₆*) δ 8.25 (s, 1H), 7.92 - 7.67 (m, 1H), 7.67 - 7.55 (m, 1H), 4.08 - 3.40 (m, 8H), 3.26 - 2.71 (m, 13H), 2.27 - 1.30 (m, 11H). | 617.2 |
| 362. | 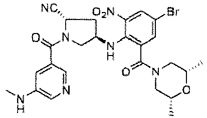 | ¹H NMR (400 MHz, DMSO-*d₆*) δ 8.34 - 8.14 (m, 1H), 8.11 - 7.95 (m, 1H), 7.94 - 7.77 (m, 1H), 7.74 - 7.58 (m, 1H), 7.53 - 7.26 (m, 1H), 6.99 - 6.78 (m, 1H), 6.23 (s, 1H), 5.12 - 4.86 (m, 1H), 4.53 - 3.40 (m, 6.5H), 3.30 - 3.08 (m, 1.5H), 3.00 - 2.67 (m, 4H), 2.66 - 2.53 (m, 1H), 2.45 - 2.35 (m, 1H), 1.11 (brs, 3H), 1.06-0.84 (m. 3H). | 586 |
| 364. | 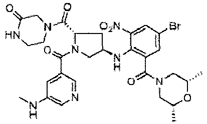 | ¹H NMR (400 MHz, DMSO-*d₆*) δ 8.34 - 7.40 (m, 5.4H), 6.89 -6.73 (m, 0.6H), 6.72 -6.53 (m, 0.4H), 6.27 - 5.93 (m, 0.6H), 5.19 - 4.73 (m, 1H), 4.42 - 4.09 (m, 2H), 4.06 - 3.45 (m, 7H), 3.26 - 2.66 (m, 8H), 2.24 - 1.63 (m, 4H), 1.30 - 0.81 (m, 6H). | 687.0 |
| 365. | 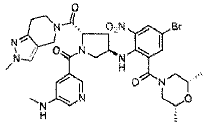 | ¹H NMR (400 MHz, DMSO-*d₆*) *δ* 8.30 - 8.12 (m, 1H), 7.98 (s, 0.6H), 7.89 - 7.14 (m, 4.4H), 6.87 - 6.75 (m, 0.6H), 6.66 - 6.44 (m, 0.4H), 6.23 - 6.11 (m, 0.6H), 6.04 - 5.88 (m, 0.4H), 5.27 - 5.05 (m, 0.6H), 4.96 - 4.81 (m, 0.4H), 4.76 - 3.40 (m, 14H), 3.29 - 3.10 (m, 1H), 2.99 - 2.65 (m, 4H), 2.45 -1.81 (m, 4H), 1.30 - 0.69 (m, 6H). | 724.0 |
| 366. | 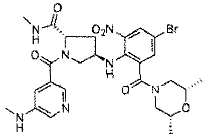 | ¹H NMR (400 MHz, DMSO-*d₆*) δ 8.24 (s, 0.3H), 8.20 (s, 0.7H), 8.02 - 7.75 (m, 2.7H), 7.71 - 7.47 (m, 2H), 6.91 - 6.77 (m, 0.7H), 6.72 - 6.63 (m, 0.3H), 6.24 - 6.08 (m, 0.7H), 4.60 - 4.12 (m, 2.5H), 4.08 - 3.37 (m, 5H), 3.24 - 2.97 (m, 1H), 2.92 - 2.57 (m, 6.5H), 2.30 - 1.86 (m, 3H), 1.23 - 0.85 (m, 6H). | 618.1 |
| 367. | 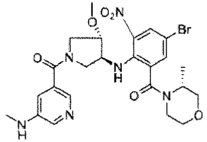 | ¹H NMR (400 MHz, DMSO-*d*₆) δ 8.32 - 8.15 (m, 1H), 8.06 - 7.92 (m, 1H), 7.90 - 7.51 (m, 2H), 7.49 - 7.13 (m, 1H), 7.00 - 6.70 (m, 1H), 6.29 - 6.00 (m, 1H), 4.68 - 3.38 (m, 11H), 3.30 - 2.83 (m, 5H), 2.79 - 2.62 (m, 3H), 1.46 - 0.96 (m, 3H). | 577.0 |
| 368. | 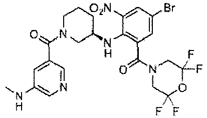 | ¹H NMR (400 MHz, DMSO-*d*₆) δ 8.35 (s, 1H), 8.10 - 7.81 (m, 2H), 7.79 - 7.44 (m, 2H), 6.75 (s, 1H), 6.28 - 5.89 (m, 1H), 4.80 - 3.94 (m, 4H), 3.92 - 3.35 (m, 4H), 3.29 - 3.11 (m, 1H), 2.70 (s, 3H), 1.94 - 1.65 (m, 2H), 1.63 - 1.32 (m, 2H). | 619.0 |
| 369. | 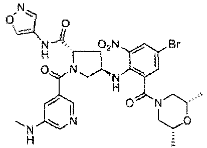 | ¹H NMR (400 MHz, DMSO-*d₆*) δ 10.68 - 10.26 (m, 1H), 9.25 - 8.93 (m, 1H), 8.77 - 8.52 (m, 1H), 8.34 - 8.14 (m, 1H), 8.05 - 7.46 (m, 4H), 6.92 - 6.60 (m, 1H), 6.28 - 6.0 1 (m, 1H), 4.82 - 4.56 (m, 1H), 4.47 - 3.48 (m, 7H), 2.84 - 2.69 (m, 3H), 2.62 - 2.54 (m, 1H), 2.40 - 2.11 (m, 3H), 1.18 - 0.83 (m, 6H). | 671.0 |
| 370. | 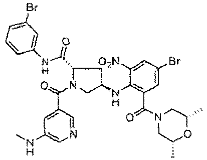 | ¹H NMR (400 MHz, DMSO-*d₆*) *δ* 10.49 - 10.25 (m, 0.7H), 10.12 - 9.97 (m, 0.3H), 8.29 - 8.15 (m, 1H), 8.12 - 7.56 (m, 5H), 7.52 - 7.19 (m, 3H), 6.92 - 6.80 (m, 0.7H), 6.67 (s, 0.3H), 6.26 - 6.13 (m, 0.7H), 6.12 - 6.02 (m, 0.3H), 4.75 - 4.57 (m, 0.8H), 4.52 - 4.43 (m, 6.5H), 3.29 - 3.11 (m, 0.7H), 2.90 - 2.54 (m, 4H), 2.44 - 2.15 (m, 3H), 1.18 - 0.75 (m, 6H). | 757.8 |
| 371. | 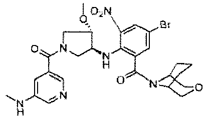 | ¹H NMR (400 MHz, DMSO-*d₆*) δ 8.30 - 8.15 (m, 1H), 8.08 - 7.93 (m, 1H), 7.91 - 7.60 (m, 2H), 7.48 - 7.04 (m, 1H), 7.01 - 6.70 (m, 1H), 6.20 - 6.05 (m, 1H), 4.58 - 4.29 (m, 1H), 4.22 - 3.37 (m, 10H), 3.27 - 2.95 (m, 3H), 2.81 - 2.65 (m, 3H), 2.47 - 2.35 (m, 1H), 2.05 - 1.44 (m, 5H). | 603.0 |
| 372. | 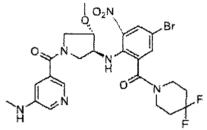 | ¹H NMR (400 MHz, DMSO-*d₆*) δ 8.33 - 8.14 (m, 1H), 8.06 - 7.92 (m, 1H), 7.91 - 7.67 (m, 2H), 7.48 - 7.28 (m, 1H), 6.97 - 6.74 (m, 1H), 6.21 - 6.03 (m, 1H), 4.16 - 3.34 (m, 9H), 3.30 - 3.04 (m, 4H), 2.80 - 2.61 (m, 3H), 2.27 - 1.68 (m, 4H). | 596.9 |
| 373. | 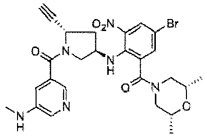 | ¹H NMR (400 MHz, DMSO-*d*₆) δ 8.33 - 8.14 (m, 1H), 8.08 - 7.27 (m, 4H), 6.95 (s, 0.4H), 6.79 (s, 0.6H), 6.16 (brs, 1H), 4.85 (s, 0.6H), 4.63 (s, 0.4H), 4.52 - 3.91 (m, 2H), 3.86 - 3.44 (m, 4H), 3.34 - 3.03 (m, 2H), 2.98 - 2.64 (m, 4H), 2.49 - 2.07 (m, 3H), 1.14 - 0.80 (m, 6H). | 584.6 |
| 374. | 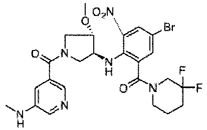 | ¹H NMR (400 MHz, DMSO-*d₆*) δ 8.34 - 8.15 (m, 1H), 8.06 - 7.93 (m, 1H), 7.90 - 7.71 (m, 1H), 7.69 - 7.22 (m, 2H), 6.98 - 6.70 (m, 1H), 6.24 - 6.05 (m, 1H), 4.60 - 3.39 (m, 9H), 3.29 - 2.99 (m, 4H), 2.81 - 2.67 (m, 3H), 2.26 - 1.93 (m, 2H), 1.85 - 1.58 (m, 2H). | 597.0 |
| 375. | 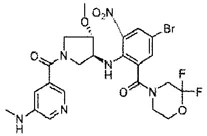 | ¹H NMR (400 MHz, DMSO-*d*₆) δ 8.27 (d, J= 2.0 Hz. 0.5H), 8.23 (d, *J* = 1.2 Hz, 0.5H), 7.98 (d, *J* = 2.4 Hz. 0.5H), 7.96 (d, *J* = 2.4 Hz, 0.5H), 7.87 - 7.57 (m, 2H), 7.39 (brs, 1H), 6.94 - 6.72 (m, 1H), 6.19 - 6.01 (m, 1H), 4.40 - 3.39 (m, 11.5H), 3.28 - 3.09 (m, 3.5H), 2.68 (t, *J* = 6.0 Hz, 3H). | 598.6 |
| 376. | 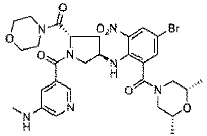 | ¹H NMR (400 MHz, DMSO-*d*₆) δ 8.24 (d, *J* = 2.0 Hz, 0.4H), 8.20 (d, *J* = 2.0 Hz, 0.6H), 7.99 (s, 1H), 7.86 - 7.51 (m, 3H), 6.88 - 6.76 (m, 0.6H), 6.67 (m, 0.4H), 6.24 - 6.07 (m, 1H), 5.15 - 5.00 (m, 0.6H), 4.92 - 4.73 (m, 0.4H), 4.48 - 4.08 (m, 2H), 4.02 - 3.38 (m, 10H), 3.28 - 2.63 (m, 7H), 2.62 - 2.55 (m, 0.4H), 2.46 - 1.97 (m, 2.6H), 1.26 - 0.76 (m, 6H). | 674.0 |
| 377. | 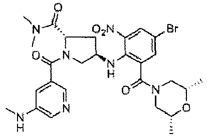 | ¹H NMR (400 MHz, DMSO-*d*₆) δ 8.23 (d, *J* = 2.4 Hz. 0.3H), 8.19 (d, *J* = 2.4 Hz, 0.7H), 8.03 - 7.93 (m, 1H), 7.85 - 7.52 (m, 3H), 6.87 - 6.75 (m, 0.7H), 6.70 - 6.60 (m, 0.3H), 6.23 - 6.06 (m, 1H), 5.15 - 4.95 (m, 0.7H), 4.88 - 4.72 (m, 0.3H), 4.45 - 3.91 (m, 2H), 3.87 - 3.38 (m, 5H), 3.29 - 3.18 (m, 1H), 3.13 - 3.02 (m, 2H), 2.95- 2.57 (m, 8H), 2.45 - 2.35 (m, 1H), 2.23 - 2.01 (m, 1H) , 1.20 - 0.85 (m, 6H). | 632.0 |
| 378. | 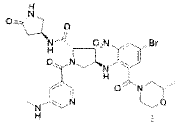 | ¹H NMR (400 MHz, DMSO-*d₆*) δ 8.55 - 8.42 (m, 0.65H), 8.39 - 8.14 (m, 1.35H), 8.05 - 7.92 (m, 1H), 7.89 - 7.51 (m, 4H), 6.90 - 6.77 (m, 0.65H), 6.66 (s, 0.35H), 6.25 - 6.02 (m, 1H), 4.60 - 3.45 (m, 9H), 3.27 - 2.93 (m, 2H), 2.92 - 2.62 (m, 5H), 2.45 - 1.99 (m, 4H), 1.21 - 0.82 (m, 6H). | 686.6 |
| 379. | 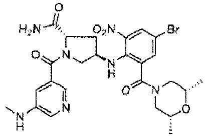 | ¹H NMR (400 MHz, DMSO-*d*₆) δ 8.29 - 8.12 (m, 1H), 8.04 - 7.92 (m, 1H), 7.91 - 7.22 (m, 4H), 7.20 - 6.66 (m, 2H), 6.23 - 6.01 (m, 1H), 4.60 - 3.91 (m, 3H), 3.89 - 3.36 (m, 4.4H), 3.27 - 3.05 (m, 0.6H), 2.95 - 2.64 (m, 4H), 2.62 - 2.54 (m, 0.4H), 2.43 - 2.09 (m, 2.6H), 1.21 -0.79 (m, 6H). | 603.6 |
| 380. | 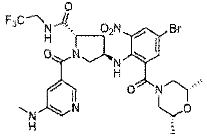 | ¹H NMR (400 MHz, DMSO-*d₆*) δ 8.89 - 8.42 (m, 1H), 8.35 - 8.14 (m, 1H), 8.08 - 7.92 (m, 1H), 7.89 - 7.49 (m, 3H), 6.93 - 6.59 (m, 1H), 6.38 - 5.94 (m, 1H), 4.81 - 3.40 (m, 9H), 3.26 - 2.99 (m, 1H), 2.86 - 2.64 (m, 4H), 2.42 - 2.02 (m, 3H), 1.24 - 0.85 (m, 6H). | 685.8 |
| 381. | 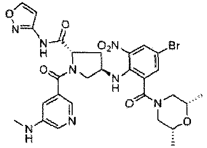 | ¹H NMR (400 MHz, DMSO-*d*₆) δ 11.31 (s, 1H), 8.84 (s, 1H), 8.32 - 8.11 (m, 1H), 8.07 - 7.46 (m, 4H), 7.03 - 6.59 (m, 2H), 6.28 - 6.01 (m, 1H), 4.88 - 4.45 (m, 1H), 4.42 - 3.48 (m, 6H), 2.94 - 2.58 (m, 5H), 2.44 - 2.10 (m, 3H), 1.19 - 0.84 (m, 6H). | 570.8 |
| 382. | 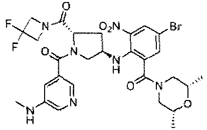 | ¹H NMR (400 MHz, DMSO) δ 8.32 - 8.10 (m, 1H), 7.99 (s, 1H), 7.87 - 7.36 (m, 3H), 6.81 (s, 0.75H), 6.68 (s, 0.25H), 6.17 (s, 1H), 4.99 - 3.47 (m, 11H), 2.98 - 2.63 (m, 8H), 2.42 - 2.12 (m, 3H), 1.20 - 0.81 (m, 6H). | 681.8 |
| 383. | 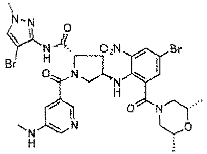 | ¹H NMR (400 MHz, DMSO-*d*₆) δ 9.98 - 9.50 (m, 1H), 8.34 - 8.14 (m, 1H), 8.06 - 7.94 (m, 1H), 7.92 - 7.46 (m, 4H), 6.95 - 6.67 (m, 1H), 6.25 - 5.99 (m, 1H), 4.81 - 4.46 (m, 1H), 4.45 - 3.94 (m, 2H), 3.92 - 3.37 (m, 8H), 3.28 - 3.00 (m, 1H), 2.98 - 2.63 (m, 4H), 2.43 - 2.10 (m, 2H), 1.18 - 0.83 (m, 6H). | 761.6 |
| 385. | 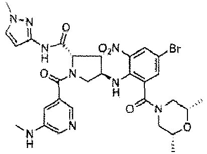 | ¹H NMR (400 MHz, DMSO-*d₆*) δ 10.67 - 10.27 (m, 1H), 8.28 - 8.14 (m, 1H), 8.03 - 7.93 (m, 1H), 7.92 - 7.70 (m, 1H), 7.69 - 7.48 (m, 3H), 6.91 - 6.79 (m, 0.7H), 6.70 - 6.58 (m, 0.3H), 6.51 - 6.29 (m, 1H), 6.21 - 6.02 (m, 1H), 4.80 - 3.80 (m, 4H), 3.74 (s, 2H), 3.69 (s, 1H), 3.66 - 3.38 (m, 3H), 3.25 - 3.15 (m, 1H), 2.88 - 2.51 (m, 4H), 2.46 - 2.04 (m, 3H), 1.19 -0.77 (m, 6H). | 684.0 |
| 386. | 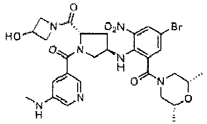 | ¹H NMR (400 MHz, DMSO-*d₆*) δ 8.32 - 8.14 (m, 1H), 8.11 - 7.92 (m, 1H), 7.88 - 7.45 (m, 3H), 6.79 (s, 0.7H), 6.67 (s, 0.3H), 6.30 - 6.04 (m, 1H), 5.88 - 5.56 (m, 1H), 4.69 - 3.36 (m, 12H), 3.27 - 3.01 (m, 1H)_ 2.97 - 2.65 (m, 4H), 2.45 - 1.98 (m, 3H), 1.21 - 0.81 (m, 6H). | 660.0 |
| 387. | 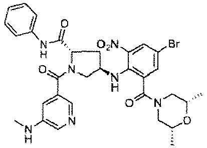 | ¹H NMR (400 MHz, DMSO-*d₆*) δ 10.27 - 10.10 (m, 0.7H), 9.94 - 9.75 (m, 0.3H), 8.31- 8.14 (m, 1H), 8. 07 - 7.52 (m, 5.5H), 7.50 - 7.37 (m, 0.5H), 7.36 - 7.21 (m, 2H), 7.13 - 6.99 (m, 1H), 6.91 - 6.79 (m, 0.7H), 6.68 (s, 0.3H), 6.30 - 6.12 (m, 0.7H), 6.09 - 5.97 (m, 0.3H), 4.71 (s, 0.7H), 4.51 - 3.50 (m, 6.3H), 3.31 - 3.15 (m, 1H), 2.94 - 2.54 (m, 4H), 2.47 - 2.16 (m, 3H), 1.16 - 0.79 (m, 6H). | 679.5 |
| 388. | 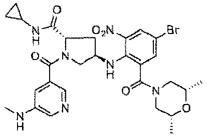 | ¹H NMR (400 MHz, DMSO-*d*₆) δ 8.25 (s, 0.4H), 8.20 (s, 0.6H), 8.07 (s, 0.6H), 8.01 - 7.74 (m, 2H), 7.73 - 7.44 (m, 2.4H), 6.90 - 6.74 (m, 0.6H), 6.67 (s, 0.4H), 6.22 - 6.03 (m, 1H), 4.56 - 3.35 (m, 7H), 3.29 - 3.11 (m, 1H), 2.93 - 2.56 (m, 5H), 2.45 - 2.35 (m, 1H), 2.31 - 1.96 (m, 2H), 1.21 - 0.81 (m, 6H), 0.68 - 0.48 (m, 2H), 0.47 - 0.08 (m, 2H). | 643.6 |
| 389. | 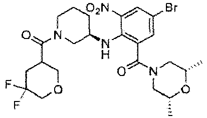 | ¹H NMR (400 MHz, DMSO-*d₆*) δ 9.40 (s, 0.6H), 9.29 (s, 0.4H), 8.55 - 7.56 (m, 7H), 6.16 (s, 0.6H), 5.85 (s, 0.4H), 4.47 - 3.38 (m, 6H), 3.26 -2.85 (m, 3H), 2.07 - 1.38 (m, 10H). | 688.6 |
| 390. | 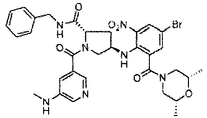 | ¹H NMR (400 MHz, DMSO-*d₆*) δ 8.65 - 8.47 (m, 0.7H), 8.39 - 8.16 (m, 1.3H), 8.06 - 7.93 (m, 1H), 7.92 - 7.54 (m, 3H), 7.38 - 7.17 (m, 4H), 7.07 - 6.92 (m, 0.7H), 6.91 - 6.78 (m, 0.7H), 6.73 (m, 0.3H), 6.23 - 6.04 (m, 1H), 4.73 - 3.43 (m, 9H), 3.24 - 3.07 (m, 1H), 2.91 - 2.65 (m, 4H), 2.47 - 2.04 (m, 3H), 1.32 - 0.82 (m, 6H). | 694.0 |
| 391. | 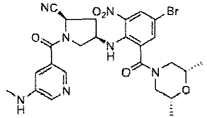 | ¹H NMR (400 MHz, DMSO-*d₆*) δ 8.33 - 8.15 (m, 1H), 8.10 - 7.97 (m, 1H), 7.93 - 7.80 (m, 1H), 7.78 - 7.48 (m, 2H), 7.02 - 6.82 (m, 1H), 6.34 - 6.10 (m, 1H), 5.07 - 4.63 (m, 1H), 4.49 - 3.40 (m, 6H), 3.28 - 2.79 (m, 2H), 2.78 - 2.58 (m, 4H), 2.46 - 2.10 (m, 2H), 1.20 - 0.79 (m, 6H). | 585.7 |
| 392. | 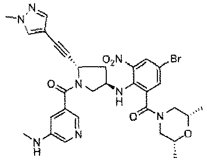 | ¹H NMR (400 MHz, DMSO-*d₆*) δ 8.30 - 8.11 (m, 1H), 8.07 - 7.74 (m, 3H), 7.73 - 7.34 (m, 3H), 6.98 (s, 0.5H), 6.82 (s, 0.5H), 6.25 - 6.03 (m, 1H), 5.19 - 4.70 (m, 1H), 4.51 - 3.98 (m, 2H), 3.95 - 3.35 (m, 8H), 3.29 - 3.06 (m, 1H), 2.96 - 2.61 (m, 4H), 2.43 - 2.21 (m, 2H), 1.13 - 0.81 (m, 6H). | 664.6 |
| 393. | 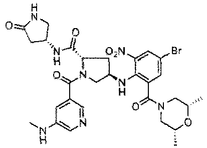 | ¹H NMR (400 MHz, DMSO-*d*₆) δ 8.50 (s, 0.65H), 8.37 - 8.15 (m, 1.35H), 8.03 - 7.93 (m, 1H), 7.87 - 7.50 (m, 4H), 6.90 - 6.76 (m, 0.65H), 6.68 (s, 0.35H), 6.23 - 6.12 (m, 0.65H), 6.11 - 6.0.1 (m, 0.35H), 4.59 - 3.40 (m, 10.35H), 3.28 - 2.94 (m, 1.65H), 2.91 - 2.66 (m, 4H), 2.46 - 1.77 (m, 4H), 1.20 - 0.86 (m, 6H). | 687.0 |
| 394. | 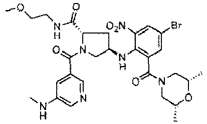 | ¹H NMR (400 MHz, DMSO-*d₆*) δ 8.25 (s, 0.3H), 8.20 (s, 0.7H), 8.15 - 7.88 (m, 2H), 7.87 - 7.48 (m, 3H), 6.90 - 6.75 (m, 0.7H), 6.68 (s, 0.3H), 6.25 - 6.02 (m, 1H), 4.65 - 3.89 (m, 3H), 3.88 - 3.35 (m, 5.5H), 3.29 - 2.93 (m, 6.5H), 2.92 - 2.61 (m, 4H), 2.45 - 1.92 (m, 3H), 1.20 - 0.81 (m, 5H). | 661.6 |
| 395. | 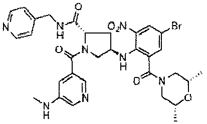 | ¹H NMR (400 MHz, DMSO-*d*₆) δ 8.76 - 8.59 (m, 0.7H), 8.56 - 8.36 (m, 2.3H), 8.26 (s, 0.3H), 8.21 (s, 0.7H), 8.06 - 7.94 (m, 1H), 7.92 - 7.46 (m, 3H), 7.34 - 7.22 (m, 1.4H), 6.95 - 6.67 (m, 1.6H), 6.25 - 6.06 (m, 1H), 4.76 - 4.46 (m, 1H), 4.44 - 3.94 (m,4H), 3.91 - 3.37 (m, 4H), 3.30 - 3.09 (m, 1H), 2.97 - 2.61 (m, 4H), 2.45 - 2.06 (m, 3H), 1.20 - 0.83 (m, 6H). | 694.6 |
| 396. | 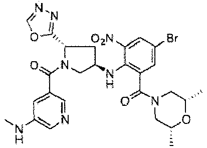 | ¹H NMR (400 MHz, DMSO-*d*₆) δ 9.35 - 9.06 (m, 1H), 8.34 - 8.10 (m, 1H), 8.08 - 7.38 (m, 4H), 6.93 - 6.50 (m, 1H), 6.31 - 6.05 (m, 1H) .5.61 - 5.30 (m, 1H), 4.46 - 4.00 (m, 2H), 3.99 - 3.44 (m, 4H), 3.26 - 3.05 (m, 1H), 2.94 - 2.51 (m, 5H), 2.46 - 2.13 (m, 2H), 1.13 (d, ./= 5.2 Hz, 3H), 0.97 (brs, 3H). | 628.6 |
| 397. | 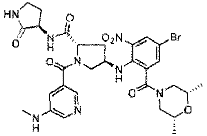 | ¹H NMR (400 MHz, DMSO-*d*₆) δ 8.36 - 8.09 (m, 2H), 8.05 - 7.47 (m, 5H), 6.93 - 6.68 (m, 1H), 6.28 - 5.99 (m, 1H), 4.72 - 3.40 (m, 8H), 3.23 - 2.97 (m, 3H), 2.92 - 2.64 (m, 4H), 2.43 - 1.92 (m, 4H), 1.87 -1.63 (m, 1H), 1.21 - 0.81 (m, 6H). | 686.6 |
| 398. | 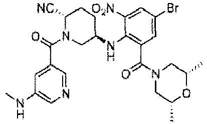 | ¹H NMR (400 MHz, DMSO-*d₆*) δ 8.36 - 7.54 (m, 3H), 7.39 - 6.02 (m, 2H), 5.97 - 4.76 (m, 1H), 4.51 - 3.50 (m, 8H), 2.98 - 2.57 (m, 4H), 2.45 - 2.37 (m, 1H), 2.24 - 1.38 (m, 3H), 1.33 - 0.90 (m, 6H). | 599.6 |
| 399. | 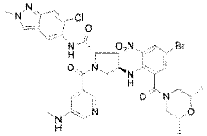 | ¹H NMR (400 MHz, DMSO-*d₆*) δ 9.74 - 9.36 (m, 1H), 8.37 (s, 1H), 8.26 (d, J = 2.0 Hz, 0.3H), 8.21 (s, 0.7H), 8.10 - 7.45 (m, 6H), 6.94 - 6.70 (m, 1H), 6.27 - 6.01 (m, 1H), 5.00 - 4.75 (m, 0.7H), 4.74 - 4.59 (m, 0.3H), 4.47 - 3.73 (m, 6H), 3.69 - 3.35 (m, 4H), 3.28 - 3.09 (m, 1H), 2.97 - 2.61 (m, 4H), 2.45 - 2.14 (m, 2H), 1.10 - 0.63 (m, 6H). | 767.4 |
| 400. | 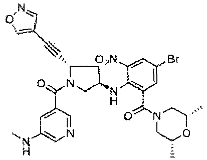 | ¹H NMR (400 MHz, DMSO-*d*₆) δ 9.48 - 9.11 (m, 1H), 8.95 - 8.59 (m, 1H), 8.33 - 8.09 (m, 1H), 7.85 - 7.28 (m, 3H), 7.25 - 6.28 (m, 1H), 5.29 - 4.68 (m, 1H), 4.57 - 3.82 (m, 5H), 3.41 - 3.03 (m, 2H), 3.01 - 2.65 (m, 4H), 2.44 - 2.06 (m, 3H), 1.18 - 0.82 (m, 6H). | 651.8 |
| 401. | 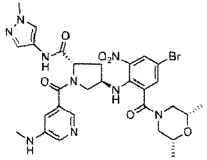 | ¹H NMR (400 MHz, DMSO-*d*₆) δ 10.29 - 9.82 (m, 1H), 8.42 - 8.13 (m, 1H), 8.06 - 7.51 (m, 5H), 7.44 (brs, 0.7H), 7.38 - 7.24 (m, 0.3H), 6.90 -6.76 (m, 0.7H), 6.66 (s, 0.3H), 6.26 - 6.12 (m, 0.7H), 6.11 - 5.99 (m, 0.3H), 4.72 - 4.55 (m, 0.7H), 4.45 - 3.42 (m, 10.3H), 2.88 - 2.68 (m, 3H), 2.63 - 2.55 (m, 1H), 2.44 - 2.09 (m, 3H), 1.17 - 0.81 (m, 6H). | 684.0 |
| 402. | 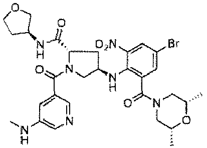 | ¹H NMR (400 MHz, DMSO-*d*₆) δ 8.38 - 7.91 (m, 3H), 7.88 - 7.48 (m, 3H), 6.90 - 6.73 (m, 0.6H), 6.68 (s, 0.4H), 6.23 - 6.01 (m, 1H), 4.68 - 3.41 (m, 13H), 3.27 - 3.07 (m, 0.6H), 3.00 - 2.64 (m, 4.4H), 2.46 - 1.98 (m, 3H), 1.96 - 1.68 (m, 1H), 1.21 -0.76 (m, 6H). | 673.6 |
| 403. | 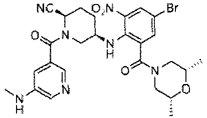 | ¹H NMR (400 MHz, DMSO-*d*₆) δ 8.18 (s, 1H), 8.09 - 7.93 (m, 1H), 7.80 - 7.57 (m, 2H), 7.37 - 6.92 (m, 1H), 6.88 - 6.70 (m, 1H), 6.34 - 6.04 (m, 1H), 5.72 - 5.26 (m, 1H), 4.56 - 3.36 (m, 5H), 3.25 - 2.93 (m, 2H), 2.90 - 2.67 (m, 4H), 2.46 - 2.36 (m, 1H), 2.19 - 1.96 (m, 2H), 1.88 - 1.57 (m, 2H), 1.19 - 1.09 (m, 3H), 1.07 - 0.91 (m, 3H). | 599.6 |
| 404. | 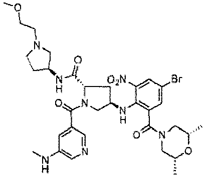 | ¹H NMR (400 MHz, DMSO-*d*₆) δ 8.39 - 8.09 (m, 1.6H), 8.06 - 7.48 (m, 4.4H), 6.91 - 6.75 (m, 0.6H), 6.66 (s, 0.4H), 6.27 - 6.11 (m, 0.6H), 6.11 - 5.99 (m, 0.4H), 4.65 - 3.53 (m, 9H), 3.26 - 3.01 (m, 5H), 2.95 - 2.55 (m, 8H), 2.45 - 1.78 (m, 6.4H), 1.65 - 1.49 (m, 0.6H), 1.19 - 1.09 (m, 3H), 1.07 - 0.88 (m, 3H). | 730.6 |
| 405. | 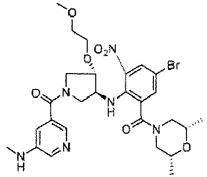 | ¹H NMR (400 MHz, DMSO-*d₆*) δ 8.31 - 8.17 (m, 1H), 8.01 (brs, 1H), 7.91 - 7.30 (m, 3H), 6.97 - 6.78 (m, 1H), 6.23 - 6.06 (m, 1H), 4.52 - 3.47 (m, 12H), 3.29 - 3.01 (m, 5H), 2.97 - 2.65 (m, 4H), 2.61 -2.53 (m, 0.5H), 2.47 - 2.38 (m, 0.5H), 1.19 - 0.88 (m, 6H). | 635.0 |
| 406. | 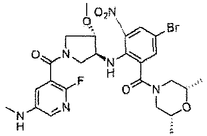 | ¹H NMR (400 MHz, DMSO-*d₆*) δ 8.43 (s, 0.1H), 8.32 - 8.15 (m, 0.9H), 7.81 -7.23 (m, 3H), 7.14 - 6.90 (m, 1H), 6.15 - 5.96 (m, 1H), 4.50 - 4.22 (m, 1H), 4.17 - 3.38 (m, 8H), 3.28 - 3.02 (m, 4H), 3.01 - 2.78 (m, 1H), 2.76 - 2.64 (m, 3H), 2.61 - 2.54 (m, 0.5H), 2.45 - 2.34 (m, 0.5H), 1.21 -0.83 (m, 6H). | 608.9 |
| 407. | 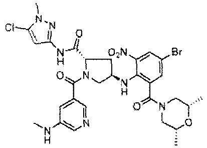 | ¹H NMR (400 MHz, DMSO-*d*₆) δ 10.88 - 10.70 (m, 0.7H), 10.65 -10.45 (m, 0.3H), 8.34 - 8.14 (m, 1H), 8.06 - 7.9f> (m, 0.7H), 7.96 - 7.90 (m, 0.3H), 7.90 - 7.72 (m, 1H), 7.71-7.51 (m, 2H), 6.97 -6.78 (m, 0.7H), 6.75 - 6.38 (m, 1.3H), 6.26 - 6.02 (m, 1H), 4.84 - 4.61 (m, 0.7H), 4.56 - 3.81 (m, 3.3H), 3.79 - 3.40 (m,7H), 2.95 - 2.58 (m, 4H), 2.43 - 2.01 (m, 3H), 1.19 - 0.81 (m, 6H). | 717.8 |
| 408. | 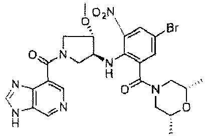 | ¹H NMR (400 MHz, DMSO-*d₆*) δ 13.13 (s, 1H), 9.02 (s, 1H), 8.65 - 8.09 (m, 3H), 7.85 - 7.19 (m, 2H), 4.55 - 3.40 (m, 9H), 3.29 - 3.01 (m, 4H), 2.99 - 2.73 (m, 1H), 2.60 - 2.52 (m, 0.5H), 2.42 - 2.25 (m, 0.5H),1.23 - 0.68 (m, 6H). | 601.9 |
| 409. | 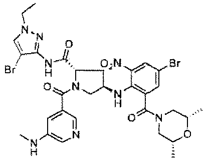 | ¹H NMR (400 MHz, DMSO-*d*₆) δ 10.12 - 9.44 (m, 1H), 8.32 - 8.14 (m, 1H), 8.06 - 7.50 (m, 5H), 6.92 - 6.60 (m, 1H), 6.23 - 6.01 (m, 1H), 4.84 - 4.51 (m, 1H), 4.44 - 3.51 (m, 8H), 3.20 - 3.12 (m, 1H), 2.92 - 2.63 (m, 5H), 2.43 - 2.36 (m, 1H), 2.29 - 2.12 (m, 1H), 1.40 - 1.27 (m, 3H), 1.15 - 0.86 (m, 6H). | 775.8 |
| 410. | 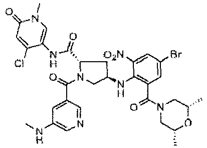 | ¹H NMR (400 MHz, DMSO-*d₆*) δ 9.69 - 9.33 (m, 1H), 8.32 - 8.14 (m, 1H), 8.06 - 7.97 (m, 1H), 7.96 - 7.46 (m, 4H), 6.94 - 6.71 (m, 1H), 6.70 - 6.56 (m, 1H), 6.24 - 6.10 (m, 1H), 4.76 (s, 0.7H), 4.57 (s, 0.3H), 4.45 - 3.55 (m, 5H), 3.53 - 3.43 (m, 5H), 2.94 - 2.62 (m, 4H), 2.47 - 2.15 (m, 3H), 1.15 -0.88 (m, 6H). | 744.4 |
| 411. | 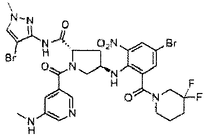 | ¹H NMR (400 MHz, DMSO-*d*₆) δ 10.20 - 9.41 (m, 1H), 8.37 - 8.15 (m, 1H), 8.05 - 7.95 (m, 1H), 7.94 - 7.38 (m, 4H), 6.85 (s, 0.7H), 6.73 (s, 0.3H), 6.28 - 5.98 (m, 1H), 4.82 - 4.53 (m, 1H), 4.21 - 3.69 (m, 7H), 3.59 - 3.45 (m, 2H), 2.79 - 2.63 (m, 3H), 2.47 - 2.35 (m, 1H), 2.29 - 1.48 (m, 6H). | 767.4 |
| 412. | 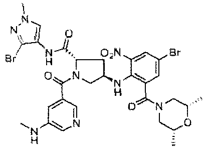 | ¹H NMR (400 MHz, DMSO-*d*₆) δ 9.78 - 9.33 (m, 1H), 8.32 - 8.14 (m, 1H), 8.12 - 7.50 (m, 5H), 6.89 - 6.65 (m, 0.7H), 6.65 (s, 0.3H), 6.23 - 6.12 (m, 0.7H), 6.11 - 6.01 (m, 0.3Σi), 4.94 - 4.54 (m, 1H), 4.43 - 3.92 (m, 2H), 3.90 - 3.72 (m, 4H), 3.70 - 3.38 (m, 3H), 3.29 - 3.16 (m, 1H), 2.92 - 2.65 (m, 3H), 2.60 (d, *J* = 4.8 Hz, 1H), 2.46 - 2.35 (m, 1.7H), 2.87 - 2.07 (m, 1.3H), 1.17 - 0.76 (m, 6H). | 765.4 |
| 413. | 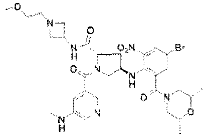 | ¹H NMR (400 MHz, DMSO-*d₆*) δ 8.57 - 8.38 (m, 0.6H), 8.36 - 8.13 (m, 1.4H), 8.05 - 7.90 (m, 1H), 7.87 - 7.50 (m, 3H), 6.89 - 6.73 (m, 0.6H), 6.64 (s,0.4H), 6.26 - 5.99 (m, 1H), 4.58 - 3.35 (m, 12H), 3.29 - 3.14 (m, 5H), 2.96 - 2.63 (m, 6H), 2.60 - 2.53 (m, 1H), 2.46 - 2.40 (m, 1H), 2.34 - 1.96 (m, 2H), 1.20 - 0.86 (m, 6H). | 716.9 |
| 414. | 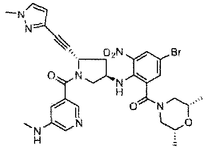 | ¹H NMR (400 MHz, DMSO-*d*₆) δ 8.34 - 8.11 (m, 1H), 8.05 - 7.47 (m, 5H), 7.03 - 6.77 (m, 1H), 6.49 - 6.26 (m, 1H), 6.20 - 6.05 (m, 1H), 5.20 - 4.77 (m, 1H), 4.53 - 3.97 (m, 2H), 3.92 - 3.32 (m, 8H), 3.30 - 3.10 (m, 1H), 2.88 - 2.60 (m, 4H), 2.49 - 2.26 (m, 2H), 1.08 - 0.87 (m, 6H). | 664.9 |
| 415. | 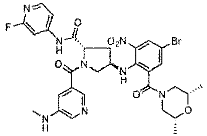 | ¹H NMR (400 MHz, DMSO-*d₆*) *δ* 10.91 (s, 0.8H), 10.58 (s, 0.2H), 8.30 - 8.18 (m, 1H), 817 - 8.06 (m, 1H), 8.05 - 7.96 (m, 0.8H), 7.95 - 7.79 (m, 1H), 7.78 - 7.56 (m, 2.2H), 7.55 - 7.10 (m, 2H), 6.94 - 6.77 (m, 0.8H), 6.65 (s, 0.2H), 6.26 - 6.12 (m, 0.8H), 6.11 - 6.01 (m, 0.2H), 4.79 - 4.60 (m, 0.8H), 4.55 - 3.37 (m, 7H), 3.27 - 3.15 (m, (1.2H), 2.97 - 2.68 (m, 3.2H), 2.64 - 2.54 (m, 0.8H), 2.44 - 2.17 (m, 3H), 1.19 - 0.77 (m, 6H). | 698.9 |
| 416. | 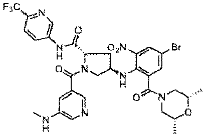 | ¹H NMR (400 MHz, DMSO-*d₆*) δ 11.02 - 10.72 (m, 0.8H), 10.52 (s, 0.2H), 9.09 - 8.85 (m, 0.8H), 8.82 - 8.65 (m, 0.2H), 8.47 - 8.08 (m, 2H), 8.05 - 7.45 (m, 5H), 6.97 - 6.78 (m, 0.8H), 6.68 (s, 0.2H), 6.19 (m, 0.8H), 6.06 (m, 0.2H), 4.86 - 3.39 (m, 8H), 3.28 - 3.07 (m, 1H), 2.93 - 2.58 (m, 4H), 2.40 - 2.17 (m, 2H), 1.16 - 0.78 (m, 6H). | 749.2 |
| 417. | 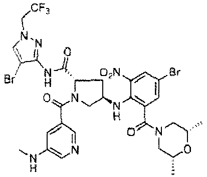 | ¹H NMR (400 MHz, DMSO-*d*₆) δ 10.21 - 9.76 (m, 1H), 8.34 - 8.16 (m, 1H), 8.14 - 7.94 (m, 2H), 7.90 - 7.49 (m, 3H), 6.95 - 6.79 (m, 0.8H), 6.76 - 6.67 (m, 0.2H), 6.24 - 6.12 (m, 0.8H), 6.11 - 6.03 (m, 0.2H), 5.23 - 4.97 (m, 2H), 4.70 (s, 0.8H), 4.57 (s, 0.2Σ3). 4.32 - 3.39 (m, 7H), 3.24 - 3.04 (m, 1H), 2.96 - 2.64 (m, 4H), 2.45 - 2.16 (m, 2H), 1.20 - 0.82 (m, 6H). | 829.8 |
| 418. | 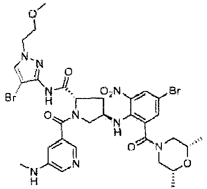 | ¹H NMR (400 MHz, DMSO-*d*₆) δ 10.08 - 9.54 (m, 1H), 8.34 - 8.12 (m, 1H), 8.04 - 7.46 (m, 5H), 6.97 - 6.63 (m, 1H), 6.26 - 6.00 (m, 1H), 4.76 - 4.46 (m, 1H), 4.42 - 3.35 (m, 11H), 3.30 - 3.00 (m, 4H), 2.96 - 2.75 (m, 1H), 2.74 - 2.63 (m, 3H), 2.46 - 2.35 (m, 1H), 2.31 - 2.10 (m, 1H), 1.19 - 0.82 (m, 6H). | 805.4 |
| 419. | 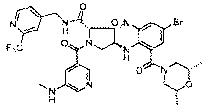 | ¹H NMR (400 MHz, DMSO-*d₆*) δ 8.87 - 8.75 (m, 0.8H), 8.69 (d, *J* = 4.8 Hz, 0.8H), 8.63 (d, *J* = 4.8 Hz, 0.2H), 8.57 - 8.48 (m, 0.2H), 8.26 (s, 0.2H), 8.21 (d, J = 2.0 Hz, 0.8H), 8.05 - 7.94 (m, 1H), 7.90 - 7.53 (m, 4.8,H), 7.16 - 7.03 (m, 0.2H), 6.91 - 6.80 (m, 0.8H), 6.71 (s, 0.2H), 6.24 - 6.08 (m, 1H), 4.76 - 3.95 (m, 5H), 3.93 - 3.72 (m, 1H), 3.70 - 3.40 (m, 3H), 3.26 - 3.10 (s, 1H), 2.90 - 2.64 (m, 4H), 2.48 - 2.14 (m, 3H), 1.16 - 0.86 (m, 6H). | 764.2 |
| 420. | 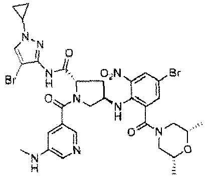 | ¹H NMR (400 MHz, DMSO-*d₆*) δ 10.03 - 9.59 (m, 1H), 8.32 - 8.14 (m, 1H), 8.10 - 7.49 (m, 5H), 6.97 - 6.61 (m, 1H), 6.26 - 5.84 (m, 1H), 4.79 - 4.46 (m, 1H), 4.43 - 3.39 (m, 8H), 3.29 - 3.07 (m, 1H), 2.91 - 2.66 (m, 4H), 2.46 - 2.37 (m, 1H), 2.31 - 2.12 (m, 1H), 1.20 - 0.81 (m, 10H). | 787.4 |
| 421. | 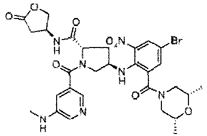 | ¹H NMR (400 MHz, DMSO-*d*₆) δ 8.73 - 8.39 (m, 1H), 8.33 - 8.13 (m, 1H), 8.07 - 7.91 (m, 1H), 7.89 - 7.42 (m, 3H), 6.88 - 6.77 (m, 0.6H), 6.67 (s, 0.4H), 6.28 - 6.03 (m, 1H), 4.67 - 3.39 (m, 10.4H), 3.26 - 3.11 (m, 0.6H), 2.99 - 2.66 (m, 5H), 2.44 - 2.11 (m, 3.4H), 2.04 - 1.91 (m, 0.6H), 1.19 - 1.07 (m, 3H), 1.06 - 0.89 (m, 3H). | 687.8 |
| 422. | 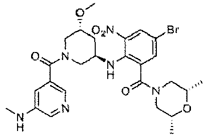 | ¹H NMR (400 MHz, DMSO-*d*₆) δ 8.23 (s, 1H), 7.98 (s, 1H), 7.78 - 7.35 (m, 3H), 6.77 (d, J = 16.8 Hz, 1H), 6.15 (s, 1H), 4.64 - 3.98 (m, 2H), 3.92 - 3.36 (m, 6H), 3.30 - 2.80 (m, 6H), 2.70 (d, *J* = 4.8 Hz. 3H), 2.47 - 1.63 (m, 3H), 1.28 - 0.87 (m, 6H). | 605.2 |
| 423. | 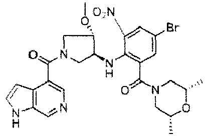 | ¹H NMR (400 MHz, DMSO-*d*₆) δ 11.86 (s, 1H), 8.81 (s, 1H), 8.38 - 7.94 (m, 2H), 7.80 - 7.37 (m, 3H), 6.57 -6.30 (m, 1H), 4.54 - 3.47 (m, 9H), 3.30 - 2.99 (m, 4H), 2.97 - 2.78 (m, 1H), 2.66 - 2.58 (m, 1H), 1.25 - 0.68 (m, 6H). | 601.0 |
| 424. | 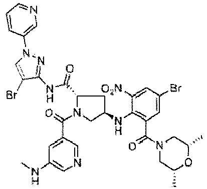 | ¹H NMR (400 MHz, DMSO-*d₆*) δ 10.42 - 9.95 (m, 1H), 9.06 (d, *J* = 2.4 Hz, 0.75H), 9.01 (d, *J* = 2.0 Hz, 0.25H), 8.88 (d, *J* = 2.8 Hz. 0.75H), 8.83 (s, 0.25H), 8.58 - 8.48 (m, 1H), 8.28 - 8.11 (m, 2H), 8.03 - 7.96 (m, 1H), 7.91 - 7.73 (m, 1H), 7.70 - 7.44 (m, 3H), 6.93 - 6.63 (m, 1H), 6.26 - 6.06 (m, 1H), 4.86 - 3.36 (m, 7H), 3.15 - 3.01 (m, 1H), 2.93 - 2.79 (m, 1H), 2.75 - 2.68 (m, 3H), 2.63 -2.55 (m, 1H), 2.43 -2.36 (m, 1H), 2.34 - 2.19 (m, 1H), 1.12 - 0.87 (m, 6H). | 824.0 |
| 425. | 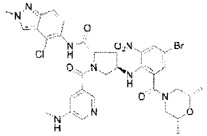 | ¹H NMR (400 MHz, DMSO-*d₆*) δ 9.87 - 9.75 (m, 0.6H), 9.68 - 9.49 (m, 0.4H), 8.45 (s, 0.6H), 8.41 (s, 0.4H), 8.27 (d, J = 2.4 Hz, 0.4H), 8.22 (d, *J* = 2.0 Hz, 0.6H), 8.05 - 7.95 (m, 1H), 7.93 - 7.35 (m, 4.6H), 7.15 - 6.99 (m, 0.4H), 6.93 - 6.70 (m, 1H), 6.24 - 6.08 (m, 1H), 4.85 (brs, 0.6H), 4.67 (brs, 0.4H), 4.53 - 3.97 (m, 5H), 3.95 - 3.38 (m, 4.4H), 3.28 - 3.09 (m, 0.6H), 2.99 - 2.61 (m, 4H), 2.49 - 2.14 (m, 3H), 1.20 - 0.59 (m, 6H). | 768.0 |
| 426. | 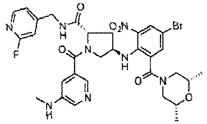 | ¹H NMR (400 MHz,DMSO-*d*₆ ) δ 8.83 - 8.66 (m, 0.75H), 8.58 - 8.40 (m, 0.25H), 8.26 (s, 0.25H), 8.21 (s, 0.75H), 8.16 (d, J= 5.2 Hz, 0.75H), 8.11 (d, *J* = 4.8 Hz, 0.25H), 8.04 - 7.95 (m, 1H), 7.93 - 7.50 (m, 3H), 7.27 (s, 0.75H), 7.08 (s, 0.75H), 6.94 - 6.65 (m, 1.5H), 6.26 - 6.05 (m, 1H), 4.77 - 3.40 (m, 9.25H), 3.23 - 3.04 (m, 0.75H), 2.94 - 2.62 (m, 4H), 2.46 - 2.11 (m, 3H), 1.19 - 0.79 (m, 6H). | 713.1 |
| 427. | 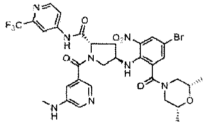 | ¹H NMR (400 MHz, DMSO-*d₆*) δ 11.16 - 10.89 (m, 0.8H), 10.67 (s, 0.2H), 8.71 - 8.57 (m, 1H), 8.47 - 8.16 (m, 1.8H), 8.15 - 7.98 (m, 1H), 7.95 -7.82 (m, 1H), 7.81 - 7.48 (m, 3.2H), 6.97 - 6.81 (m, 0.8H), 6.66 (s, 0.2H), 6.29 - 6.13 (m, 0.8H), 6.11 - 6.00 (m, 0.2H), 4.81 - 4.64 (m, 0.8H), 4.57 - 4.44 (m, 0.2H), 4.43 - 3.36 (m, 6.5H), 3.30 - 3.14 (m, 0.5H), 2.94 - 2.53 (m, 4H), 2.49 - 2.15 (m, 3H), 1.13 - 0.83 (m, 6H). | 749.0 |
| 428. | 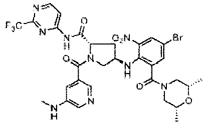 | ¹H NMR (400 MHz, DMSO-*d₆*) δ 11.79 - 11.61 (m, 0.8H), 11.48 - 11.34 (m, 0.2H), 9.00 - 8.84 (m, 1H), 8.40 - 7.46 (m, 6H), 6.99 - 6.77 (m, 0.8H), 6.66 (s, 0.2H), 6.21 (s, 0.8H), 6.10 (s, 0.2H), 4.96 - 4.63 (m, 1H), 4.48 - 3.42 (m, 6.2H), 3.28 - 3.07 (m, 0.8H), 2.90 - 2.68 (m, 3.5H), 3.59 - 2.54 (m, 0.5H), 2.49 - 2.09 (m, 3H), 1.21 - 0.81 (m, 6H). | 750.0 |
| 429. | 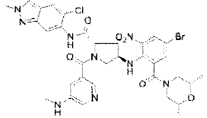 | ¹H NMR (400 MHz, DMSO-*d*₆) δ 9.69 - 9.55 (m, 0.7H), 9.50 - 9.36 (m, 0.3H), 8.32 (s, 0.7H), 8.31 (s, 0.3H), 8.29 - 8.24 (m, 0.3H), 8.22 (d, *J* = 2.4 Hz, 0.7H), 8.05 - 7.96 (m, 1.6H), 7.95 - 7.52 (m, 4.4H), 6.94 - 6.82 (m, 0.7H), 6.75 (s, 0.3H), 6.28 - 6.07 (m, 1H), 4.93 (brs, 0.7H), 4.72 (brs, 0.3H), 4.47 - 3.70 (m, 6H), 3.67 - 3.38 (m, 3.4H), 3.30 - 3.14 (m, 0.6H), 2.92 - 2.61 (m, 4H), 2.48 - 2.21 (m, 3H), 1.08 - 0.66 (m, 6H). | 768.0 |
| 430. | 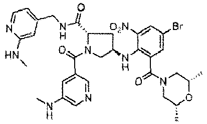 | ¹H NMR (400 MHz, DMSO-*d₆*) δ 8.62 - 8.46 (m, 0.7H), 8.41 - 8.17 (m, 1.3H), 8.04 -7.95 (m, 1H), 7.92 - 7.81 (m, 1.8H), 7.74 - 7.55 (m, 2.2H), 6.90 - 6.78 (m, 0.7H), 6.73 (s, 0.3H), 6.46 - 6.25 (m, 2.5H), 6.22 - 6.02 (m, 1.5H), 4.62 (s, 0.7H), 4.51 - 3.42 (m, 8.6H), 3.28 - 3.11 (m, 0.7H), 3.08 - 2.65 (m, 7H), 2.46 - 2.11 (m, 3H), 1.18 - 0.86 (m, 6H). | 724.0 |
| 431. | 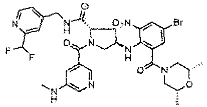 | ¹H NMR (400 MHz, DMSO-*d₆*) δ 8.84 - 8.69 (m, 0.8H), 8.67 - 8.43 (m, 1.2H), 8.26 (d,./= 2.0 Hz, 0.2H), 8.21 (d, *J* = 2.0 Hz, 0.8H), 8.05 - 7.93 (m, 1H), 7.91 - 7.82 (m, 0.8H), 7.80 -7.53 (m, 3H), 7.52 - 7.42 (m, 1H), 7.09 - 6.69 (m, 2.2H), 6.24 - 6.07 (m, 1H), 4.73 - 3.93 (m, 5.2H), 3.91 - 3.37 (m, 4H), 3.27 - 3.06 (m, 0.8H), 2.91 - 2.64 (m, 4H), 2.46 - 2.07 (m, 3H), 1.18 - 0.86 (m, 6H). | 745 |
| 432. | 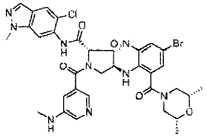 | ¹H NMR (400 MHz, DMSO-*d₆*) δ 9.86 - 9.70 (m, 0.7H), 9.67 - 9.54 (m, 0.3H), 8.27 (d, J= 1.6 Hz. 0.3H), 8.22 (d, .7= 2.0 Hz, 0.7H), 8.20 - 8.05 (m, 0.8H), 8.04 - 7.95 (m, 2.5H), 7.93 - 7.81 (m, 1.2H), 7.80 - 7.54 (m, 2.5H), 6.96 - 6.83 (m, 0.7H), 6.82 - 6.71 (m, 0.3H), 6.29 - 6.06 (m, 1H), 5.14 - 4.90 (m, 0.7H), 4.85 - 4.72 (m, 0.3H), 4.52 - 3.99 (m, 5H), 3.97 - 3.73 (m, 1H), 3.70 - 3.46 (m, 3H), 3.27 - 3.00 (m, 1H), 2.92 - 2.61 (m, 4.5H), 2.45 - 2.16 (m, 2.5H), 1.14 - 0.71 (m, 6H). | 768.0 |
| 433. | 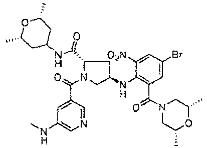 | ¹H NMR (400 MHz, DMSO-*d₆*) δ 8.27 (d, *J* = 2.0 Hz, 0.5H), 8.22 (s, 0.5H), 8.14 - 8.02 (m, 0.5H), 8.01 - 7.96 (m, 0.5H), 7.95 - 7.56 (m, 4H), 6.86 - 6.75 (m, 0.5H), 6.72 - 6.63 (m, 0.5H), 6.22 - 6.13 (m, 0.5H), 6.12 - 6.04 (m, 0.5H), 4.64 (brs, 0.5H), 4.54 - 4.15 (m, 2H), 4.00 (brs, 1H), 3.88 - 3.38 (m, 6.5H), 3.29 - 3.04 (m, 1H), 2.96 - 2.63 (m, 4H), 2.47 - 1.90 (m, 3H), 1.71 - 1.48 (m, 1H), 1.44 - 1.19 (m, 2H), 1.17 - 0.89 (m, 13H). | 716.0 |
| 434. | 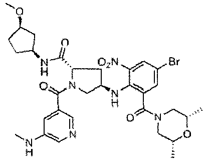 | ¹H NMR (400 MHz, DMSO-*d*₆) δ 8.26 (d, *J* = 2.0 Hz, 0.4H), 8.21 (s, 0.6H), 8.06 - 7.90 (m, 1.6H), 7.86 - 7.53 (m, 3.4H), 6.89 - 6.76 (m, 0.6H), 6.67 (s, 0.4H), 6.24 - 6.12 (m, 0.6H), 6.11 - 6.02 (m, 0.4H), 4.61 - 4.11 (m, 2.6H), 4.09 - 3.90 (m, 1H), 3.88 - 3.35 (m, 6H), 3.27 - 3.06 (m, 3.4H), 2.96 - 2.64 (m, 4H), 2.63 - 2.54 (m, 0.4H), 2.48 - 2.37 (m, 0.6H), 2.34 - 1.96 (m, 3H), 1.91-1.18 (m, 5H), 1.16 - 0.86 (m, 6H). | 702.0 |
| 435. | 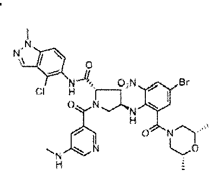 | ¹H NMR (400 MHz, DMSO-*d₆*) δ 9.87 (s, 0.7H), 9.71 - 9.53 (m, 0.3H), 8.27 (d, *J* = 2.4 Hz, 0.3H), 8.22 (d, J= 2.4 Hz, 0.6H), 8.14 - 7.95 (m, 2H), 7.93 - 7.49 (m, 4.7H), 7.30 - 7.12 (m, 0.3H), 6.97 - 6.71 (m, 1H), 6.24 - 6.08 (m, 1H), 4.86 (s, 0.7H), 4.68 (s, 0.3H), 4.48 - 3.98 (m, 5H), 3.96 - 3.74 (m, 1H), 3.70 - 3.38 (m, 3.5H), 3.29 - 3.12 (m, 0.5H), 2.97 - 2.64 (m, 4H), 2.48 - 2.06 (m, 3H), 1.10 - 0.63 (m, 6H). | 768.0 |

### Example 321

### (5-Bromo-2-((3S,4S)-4-methoxy-1-(5-(methylamino)nicotinoyl)pyrrolidin-3-yl)amino)-3-nitrophenyl)((2S,6R)-2,6-dimethylmorpholinyl)methanone

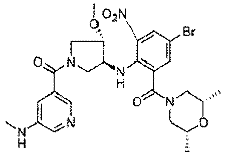

### Reaction route:

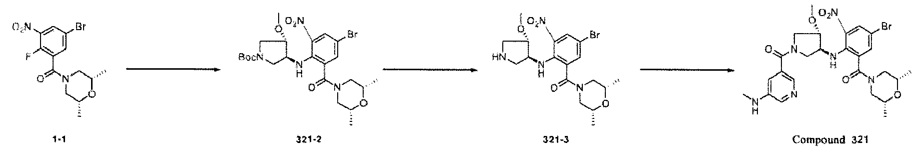

### Operating Steps:

Step A: Compound 1-1 (886 mg, 3.37 mmol), tert-butyl (3*S*, 4*S*)-3-amino-4-methoxypyrrolidin-1-carboxylate (800 mg, 3.70 mmol), and *N*,*N*-diisopropylethylamine (1.3 g, 10.11 mmol) were dissolved in *N*,*N*-dimethylformamide solution (17 mL) at room temperature under the protection of nitrogen. The reaction system was then stirred at 80°C for 1 h.

After the depletion of the raw material as monitored by LCMS, the reaction was quenched by addition of water (60 mL), and the mixture solution was extracted with ethyl acetate (15 mL × 3 times). The organic phases were combined and washed with saturated saline (20 mL × 2 times), then dried with anhydrous sodium sulfate, filtered, and concentrated under reduced pressure. The resulting residue was purified by silica gel column chromatography to obtain Compound 321-2 (1.7 g, 90% yield).
MS (ESI) M/Z: 457.0 [M-99]⁺.

Step B: Compound 321-2 (1.7 g, 3.06 mmol) was dissolved in dichloromethane solution (15 mL) at room temperature under the protection of nitrogen. Subsequently, the above solution was cooled to 0°C, followed by slow and dropwise addition of 4 M of hydrochloric acid-1,4-dioxane solution (7.6 mL). The reaction system was then stirred at room temperature for 1 h.

After the depletion of the raw material as monitored by LCMS, the reaction solution was concentrated by vacuum distillation. Crude Compound 321-3 (1.6 g) was then obtained.

Step C: 5-(Methylamino)nicotinic acid (630 mg, 4.15 mmol) was dissolved in *N,N*-dimethylformamide solution (17 mL) at room temperature under the protection of nitrogen. Subsequently, the above solution was cooled to 0°C, followed by addition of *N,N*-diisopropylethylamine (2.23 g, 17.3 mmol), 2-(7-azabenzotriazolyl)-*N*,*N*,*N'*,*N'*-tetramethyluronium hexafluorophosphate (1.97 g, 5.19 mmol), and Compound 321-3 (1.7 g, 3.46 mmol) in turn. The reaction system was then stirred at 0°C for 30 min.

After the depletion of the raw material as monitored by LCMS, the reaction was quenched by addition of water (60 mL), and the mixture solution was extracted with ethyl acetate (15 mL × 3 times). The organic phases were combined and washed with saturated sodium bicarbonate solution (20 mL × 2 times). Water (30 mL) was then added to the resulting organic phase, and the pH of the mixture solution was adjusted to 4 to 5 with hydrochloric acid. The resulting organic phase was washed with saturated sodium bicarbonate solution (20 mL) and saturated saline (20 mL) in turn. Finally, the washed organic phase was dried with anhydrous sodium sulfate, filtered, and concentrated under reduced pressure, and the resulting residue was purified by silica gel column chromatography to obtain the titled Compound 321 (1.088 g, 53% yield).

MS (ESI) M/Z: 591.2 [M+H]⁺.

¹H NMR (400 MHz. DMSO-*d*₆) δ 8.36 - 8.16 (m. 1H). 8.08 - 7.94 (m. 1H), 7.91 - 7.59 (m. 2H), 7.56 - 7.22 (m, 1H), 6.96 - 6.75 (m, 1H), 6.23 - 6.06 (m. 1H). 4.48 - 4.19 (m. 1H), 4.14 - 3.39 (m, 9H). 3.29 - 3.01 (m, 4H), 2.99 - 2.65 (m, 4H), 1.25 - 0.81 (m, 6H).

### Example 325

### (5-Bromo-2-((R)-1-((R)-5,5-difluoropiperidin-3-carbonyl)piperidin-3-yl)amino)-3-nitrophenyl)((2R,6S)-2,6-dimethylmorpholinyl)methanone

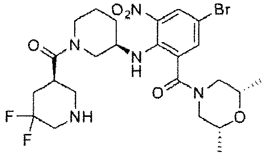

### Reaction route:

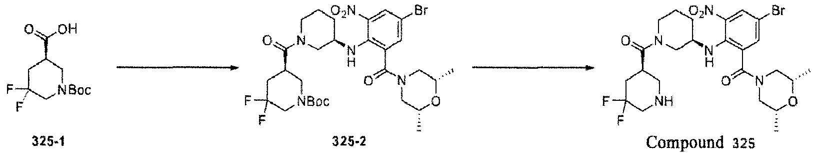

### Operating Steps:

**Step A:** Compound **325-1** (16 mg, 0.06 mmol) and ((5-bromo-3-nitro-2-((*R*)-piperidin-3-yl)amino)phenyl((2*R*,6*S*)-2,6-dimethylmorpholino)methanone (32 mg, 0.72 mmol) were dissolved in *N*,*N*-dimethylformamide (0.6 mL) at 0°C. Subsequently, 2-(7-azabenzotriazolyl)-*N*,*N*,*N'*,*N'*-tetramethyluronium hexafluorophosphate (14.9 mg, 0.04 mmol) and *N,N-*diisopropylethylamine (11.7 mg, 0.09 mmol) were added to the above solution. The reaction system was then stirred at room temperature for 1 h.

After the depletion of the raw material as monitored by LCMS, the reaction solution was quenched by addition of water (20 mL), and the mixture solution was extracted with ethyl acetate (10 mL × 3 times). The organic phases were combined and washed with saturated saline (15 mL × 2 times), then dried with anhydrous sodium sulfate, filtered, and concentrated under reduced pressure. The resulting residue was purified by silica gel column chromatography to obtain Compound **325-2** (36 mg).

MS (ESI) M/Z: 588.2 [M-99].

**Step B:** Compound **325-2** (36 mg, 0.052 mmol) was dissolved in dichloromethane (0.6 mL) at room temperature. Subsequently, 4 M of hydrochloric acid-1,4-dioxane solution (0.6 mL) was added to the above solution. The reaction system was then continued to be stirred for 2 h.

After the depletion of the raw material as monitored by LCMS, the reaction solution was concentrated by vacuum distillation. The pH of the resulting residue was adjusted to 8 with saturated aqueous ammonium bicarbonate solution, which was then purified by preparative high performance liquid chromatography to obtain the titled Compound 325 (21.77 mg).

MS (ESI) M/Z: 588.2 [M+H|⁺.

¹H NMR (400 MHz, DMSO-*d*₆) δ 8.26 (d, *J* = 2.0 Hz, 1H), 7.97 -7.55 (m, 2H), 4.48 -4.23 (m. 1H), 3.97 - 3.32 (m, 7H). 3.28 - 3.13 (m. 1H). 3.12 - 2.58 (m, 6H), 2.47 - 2.18 (m. 2H), 2.17 - 1.24 (m. 6H), 1.15 (d, , *J* = 6.0 Hz, 3H). 1.01 (brs, 3H).

### Example 327

### (5-Bromo-2-((3R)-1-(5,5-difluoropiperidin-3-carbonyl)piperidin-3-yl)amino)-3-nitrophenyl)(2,2-difluoromorpholinyl)methanone

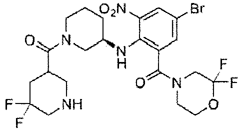

### Reaction route:

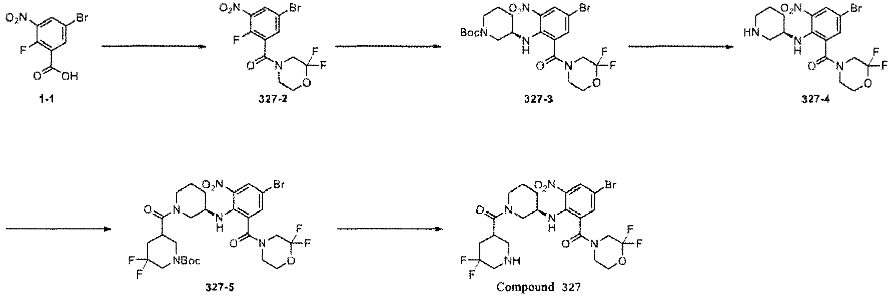

### Operating Steps:

Step A: Compound 1-1 (120 mg, 0.454 mmol) was dissolved in *N*,*N*-dimethylformamide (1 mL) at room temperature. Subsequently, the above solution was cooled to 0°C, followed by addition of 2,2-difluoromorpholine (80.58 mg, 0.505 mmol), 2-(7-azabenzotriazolyl)-*N*,*N*,*N'*,*N'*-tetramethyluronium hexafluorophosphate (288 mg, 0.757 mmol) and *N,N-*diisopropylethylamine (326.37 mg, 2.53 mmol) in turn. The reaction system was then stirred at 0°C for 30 min.

After the depletion of the raw material as monitored by LCMS, the reaction was quenched by addition of water (10 mL), and the mixture solution was extracted with ethyl acetate (10 mL × 3 times). The organic phases were combined and washed with saturated saline (10 mL × 2 times), then dried with anhydrous sodium sulfate, filtered, and concentrated under reduced pressure. The resulting residue was purified by silica gel column chromatography to obtain Compound 327-2 (120 mg).

MS (ESI) M/Z:369.0 [M+H]⁺.

Step B: Compound 327-2 (120 mg, 0.325 mmol) was dissolved in *N*,*N*-dimethylformamide (1 mL) at room temperature under the protection of nitrogen. Subsequently, tert-butyl (R)-3-aminopiperidin-1-carboxylate (78.14 mg, 0.390 mmol) and *N*,*N*-diisopropylethylamine (98 mg, 0.975 mmol) were added in turn to the above reaction solution. The reaction system was then heated to 80°C, and stirred for 4 h.

After the depletion of the raw material as monitored by LCMS, the reaction was quenched by addition of water (15 mL), and the mixture solution was extracted with ethyl acetate (5 mL × 3 times). The organic phases were combined and washed with saturated saline (20 mL × 2 times), then dried with anhydrous sodium sulfate, filtered, and concentrated under reduced pressure. The resulting residue was purified by silica gel column chromatography to obtain Compound 327-3 (180 mg).

MS (ESI) M/Z: 449.0 [M+H]⁺

Step C: Compound 327-3 (180 mg, 0.327 mmol) was dissolved in dichloromethane (4 mL) at room temperature under the protection of nitrogen. Subsequently, 4 M of hydrochloric acid-1,4-dioxane solution (2 mL) was added slowly and dropwise to the above solution. The reaction system was then stirred at room temperature for 4 h.

After the depletion of the raw material as monitored by LCMS, the reaction solution was filtered and concentrated by distillation. Compound 327-4 (100 mg) was then obtained.

MS (ESI) M/Z:451.0 [M+H]⁺.

Step D: Compound 327-4 (10.99 mg, 0.023 mmol) was dissolved in *N*,*N*-dimethylformamide (1 mL) at room temperature. Subsequently, the above solution was cooled to 0°C, followed by addition of 1-(tert-butoxycarbonyl)-5,5-difluoropiperidin-3-carboxylic acid (5 mg, 0.019 mmol), 2-(7-azabenzotriazolyl)-*N*,*N*,*N'*,*N'*-tetramethyluronium hexafluorophosphate (10.7 mg, 0.028 mmol) and *N,N*-diisopropylethylamine (12.18 mg, 0.094 mmol) in turn. The reaction system was then stirred at 0°C for 30 min.

After the depletion of the raw material as monitored by LCMS, the reaction was quenched by addition of water (20 mL), and the mixture solution was extracted with ethyl acetate (5 mL × 3 times). The organic phases were combined and washed with saturated saline (10 mL × 2 times), then dried with anhydrous sodium sulfate, filtered, and concentrated under reduced pressure. The resulting residue was purified by silica gel column chromatography to obtain Compound 327-5 (10 mg).

MS (ESI) M/Z:640.0 [M+H]⁺.

Step E: Compound 327-5 (10 mg, 0.014 mmol) was dissolved in 1,4-dichloromethane (2 mL) at room temperature under the protection of nitrogen. Subsequently, 4 M of hydrochloric acid-1,4-dioxane solution (1 mL) was slowly and dropwise added to the above solution. The reaction system was then stirred at room temperature for 1 h.

After the depletion of the raw material as monitored by LCMS, the reaction solution was concentrated by distillation. The resulting residue was purified by reversed phase column chromatography to obtain the titled Compound 327 (6.14mg, 71.71% yield).

MS (ESI) M/Z: 596.0 [M+H]⁺.

¹HNMR (400 MHz. MeOD-d₄) δ 8.45 - 8.36 (m, 1H), 7.68 - 7.59 (m, 1H), 4.32 - 3.60 (m. 9H). 3.13 - 3.00 (m, 3H). 2.91 - 2.69 (m. 1.5H). 2.62 - 2.37 (m, 1.5H). 2.31 - 1.89 (m, 4H). 1.88 - 1.55 (m. 3H).

### Example 331

### (5-Bromo-2-((3R)-1-(5,5-difluoropiperidin-3-carbonyl)piperidin-3-yl)amino)-3-nitrophenyl)(3,3-difluoropiperidin-1-yl)methanone

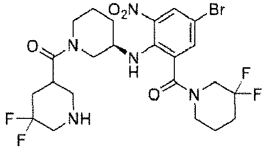

### Reaction route:

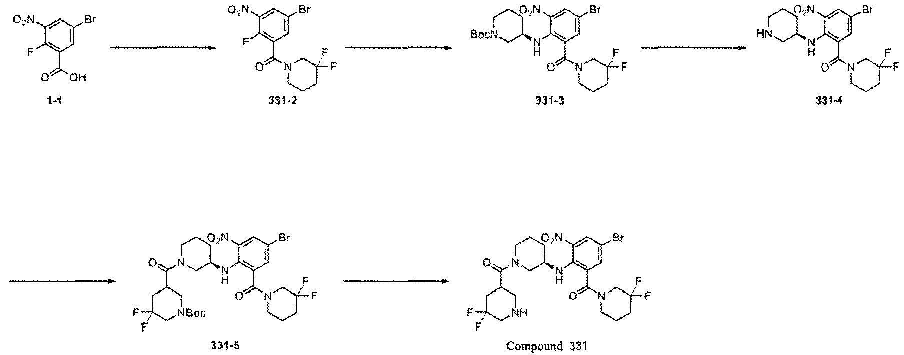

### Operating Steps:

**Step A:** Compound 331-1 (50 mg, 0.19 mmol) was dissolved in *N*,*N*-dimethylformamide (0.95 mL) at room temperature. Subsequently, the above solution was cooled to 0°C, followed by addition of *N*,*N*-diisopropylethylamine (70 mg, 0.57 mmol), 2-(7-azabenzotriazolyl)-*N*,*N*,*N'*,*N'*-tetramethyluronium hexafluorophosphate (101 mg, 0.27 mmol), and 3,3-difluoropyridine (36 mg, 0.23 mmol) in turn. The reaction system was then stirred at 0°C for 10 min.

After the depletion of the raw material as monitored by LCMS, the reaction was quenched by addition of water (5 mL), and the mixture solution was extracted with ethyl acetate (5 mL × 3 times). The organic phases were combined and washed with saturated aqueous sodium chloride solution (10 mL × 3 times). The resulting residue was purified by silica gel column chromatography to obtain Compound 331-2 (51 mg).

MS (ESI) M/Z: 367.0 [M+M]⁺.

**Step B:** Compound 331-2 (51 mg, 0.14 mmol) was dissolved in *N*,*N*-dimethylformamide (0.7 mL) at room temperature. Subsequently, tert-butyl (*R*)-3-aminopiperidin-1-carboxylate (33 mg, 0.17 mmol) and *N,N*-diisopropylethylamine (55 mg, 0.42 mmol) were added in turn to the above solution. The reaction system was then stirred at 80°C for 2 h.

After the depletion of the raw material as monitored by LCMS, the reaction was quenched by addition of water (10 mL), and the mixture solution was extracted with ethyl acetate (5 mL × 3 times). The organic phases were combined and washed with saturated saline (25 mL × 2 times), then dried with anhydrous sodium sulfate, filtered, and concentrated under reduced pressure. The resulting residue was purified by silica gel column chromatography to obtain Compound 331-3 (70 mg).

MS (ESI) M/Z: 447.0 [M-99].

**Step C:** Compound 331-3 (70 mg, 0.13 mmol) was dissolved in dichloromethane (0.33 mL) at room temperature. Subsequently, 4 M of hydrochloric acid-1,4-dioxane solution (0.33 mL) was slowly and dropwise added to the reaction system. The reaction system was then continued to be stirred for 1 h.

After the depletion of the raw material as monitored by LCMS, the reaction system was concentrated under reduced pressure. Compound 331-4 (58mg) was then obtained.

MS (ESI) M/Z: 447.0 [M+H]⁺.

**Step D:** Compound 1-(tert-butoxycarbonyl)-5,5-difluoropiperidin-3-carboxylic acid (5.4 mg, 0.02 mmol) was dissolved in *N,N*-dimethylformamide (0.10 mL) at room temperature. Subsequently, the above solution was cooled to 0°C, followed by addition of *N,N*-diisopropylethylamine (7.7 mg, 0.06 mmol), 2-(7-azabenzotriazolyl)-*N*,*N*,*N'*,*N'-*tetramethyluronium hexafluorophosphate (10 mg, 0.03 mmol), and Compound 1-4 (11 mg, 0.03 mmol) in turn. The reaction system was then continued to be stirred for 10 min.

After the depletion of the raw material as monitored by LCMS, the reaction was quenched by addition of water (10 mL), and the mixture solution was extracted with ethyl acetate (10 mL × 3 times). The organic phases were combined and washed with saturated aqueous sodium chloride solution (30 mL × 2 times), then dried with anhydrous sodium sulfate, filtered, and concentrated under reduced pressure. The resulting residue was purified by silica gel column chromatography to obtain Compound 331-5 (11.8 mg).

MS (ESL) M/Z: 594.0 [M-99].

**Step E:** Compound 331-5 (11.8 mg, 0.02 mmol) was dissolved in dichloromethane (0.1 mL) at room temperature. Subsequently, 4 M of hydrochloric acid-1,4-dioxane solution (0.1 mL) was slowly and dropwise added to the reaction system. The reaction system was then continued to be stirred for 12 h.

After the depletion of the raw material as monitored by LCMS, the reaction solution was concentrated by vacuum distillation. The resulting residue was dissolved in water (1.5 mL), and the the mixture solution was adjusted to pH = 8 with 10%wt aqueous ammonia. The resulting solution was purified by preparative high performance liquid chromatography to obtain the titled Compound 331 (1.76 mg).

MS (ESI) M/Z: 594.0 [M+H]⁺.

¹H NMR (400 MHz, DMSO-*d*₆) δ 8.28 (s, 1H), 8.01 - 7.46 (m, 2H), 4.66 - 3.50 (m, 7H), 3.19 - 2.67 (m. 8H), 2.22 - 1.93 (m. 4H). 1. 90 - 1.35 (m, 6H).

### Example 336

### (R)-(5-Bromo-2-((1-(5-(methylamino)nicotinoyl)piperidin-3-yl)amino)-3-nitrophenyl)(3,6-dihydropyridin-1(2H)-yl)methanone

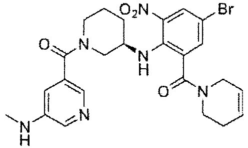

### Reaction route:

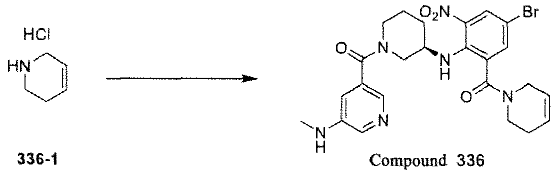

### Operating Steps:

Step A: Compound (*R*)-5-bromo-2-((1-(5-(methylamino)nicotinoyl)piperidin-3-yl)amino)-3-nitrobenzoic acid (40 mg, 0.08 mmol) was dissolved in *N,N*-dimethylformamide (0.5 mL) at room temperature. Subsequently, 2-(7-azabenzotriazolyl)-*N*,*N*,*N'*,*N'*-tetramethyluronium hexafluorophosphate (38 mg, 0.1 mmol), *N*,*N*-diisopropylethylamine (55 mg, 0.42 mmol), and Compound 336-1 (12 mg, 0.1 mmol) were added to the above solution under an ice bath. The reaction system was then stirred under an ice bath for 1 h.

After the depletion of the raw material as monitored by LCMS, the reaction was quenched by addition of water (15 mL). The mixture solution was extracted with ethyl acetate (10 mL × 3 times), and the organic phases were combined, washed with saturated saline (30 mL × 2 times), dried with anhydrous sodium sulfate, filtered, and finally concentrated under reduced pressure. The resulting residue was purified by preparative high performance liquid chromatography to obtain the titled Compound 336 (18.20 mg).

MS (ESI) M/Z: 543.0 [M+H]⁺.

¹H NMR (400 MHz, DMSO-*d*₆) δ 8.27 (s. 1H). 8.05 - 7.45 (m, 4H), 6.75 (s, 1H), 6.16 (s, 1H), 5.96 - 5.57 (m, 2H), 4.27 - 3.48 (m. 7H), 3.29 - 3.13 (m, 2H), 2.70 (s, 3H), 2.27 - 1.37 (m. 6H).

### Example 337

### (R)-(5-Bromo-2-((1-(5-(methylamino)nicotinoyl)piperidin-3-yl)amino)-3-nitrophenyl)(4-fluoro-3,6-dihydropyridin-1(2H)-yl)methanone

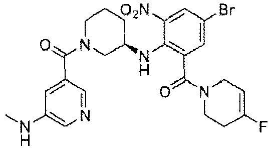

### Reaction route:

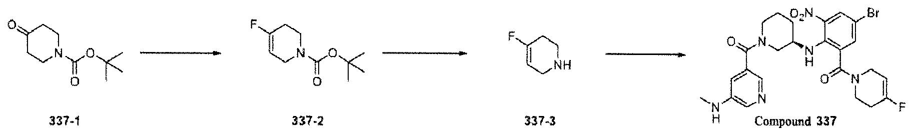

### Operating Steps:

Step A: Triethylamine trihydrofluoride (486 mg, 3.02 mmol) was added to *N*,*N*-dimethylacetamide (5.0 mL) at room temperature. Subsequently, triethylamine (153 mg, 1.51 mmol), (diethylamino)sulfur difluoride tetrafluoroborate (1.03 g, 4.52 mmol), and N-tert-butoxycarbonyl-4-piperidone (300 mg, 1.51 mmol) were added to the above solution. The reaction system was then continued to be stirred for 2 h.

After the depletion of the raw material as monitored by LCMS, the reaction was quenched by addition of saturated aqueous sodium bicarbonate solution (20 mL), and the mixture solution was extracted with ethyl acetate (10 mL × 3 times). The organic phases were combined and washed with saturated saline (10 mL × 2 times), then dried with anhydrous sodium sulfate, filtered, and concentrated under reduced pressure. The resulting residue was purified by silica gel column chromatography to obtain Compound 337-2 (180 mg).

¹H NMR (400 MHz, DMSO.*d₆*) δ 5.31 (d. *J* = 16.0 Hz, 1H), 3.86 - 3.78 (m. 2H). 3.53 - 3.50 (m. 2H), 2.29 - 2.22 (m. 2H), 1.41 (s, 9H).

Step B: Compound 337-2 (80 mg, 0.40 mmol) was dissolved in 1,4-dioxane (1 mL) at room temperature. Subsequently, 4 M of hydrochloric acid-1,4-dioxane solution (2 mL) was slowly and dropwise added to the above solution. The reaction system was then continued to be stirred for 1 h.

After the depletion of the raw material as monitored by LCMS, the reaction solution was concentrated by vacuum distillation. Compound 337-3 (60 mg) was then obtained.

MS (ESI) M/Z: 102.2 [M+H]⁺.

Step C: Compound (*R*)-5-bromo-2-((1-(5-(methylamino)nicotinoyl)piperidin-3-yl)amino)-3-nitrobenzoic acid (30 mg, 0.06 mmol) was dissolved in *N*,*N*-dimethylformamide (1.0 mL) at room temperature. Subsequently, 2-(7-azabenzotriazolyl)-*N*,*N*,*N'*,*N'*-tetramethyluronium hexafluorophosphate (28 mg, 0.07 mmol), *N*',*N*-diisopropylethylamine (16 mg, 0.12 mmol), and Compound 1-3 (10 mg, 0.07 mmol) were added to the above solution. The reaction system was then continued to be stirred for 1 h.

After the depletion of the raw material as monitored by LCMS, the reaction was quenched by addition of water (10 mL), and the mixture solution was extracted with ethyl acetate (5 mL × 3 times). The organic phases were combined and washed with saturated saline (10 mL × 2 times), then dried with anhydrous sodium sulfate, filtered, and concentrated under reduced pressure. The resulting residue was purified by reversed phase column chromatography to obtain the titled Compound 337 (12.14 mg).

MS (ESI) M/Z: 561.05 [M+H]⁺ .

¹H NMR (400 MHz, DMSO₋*d₆*) δ 8.27 (s, 1H), 8.10 - 7.40 (m, 4H), 6.75 (s, 1H), 6.14 (s, 1H). 5.54 - 5.06 (m, 1H), 4.42 - 3.34 (m, 8H), 3.28 - 3.01 (m. 1H), 2.70 (s, 3H), 2.46 - 2.17 (m, 2H). 1.96 - 1.28 (m. 4H).

### Example 341

### (5-Bromo-2-((R)-1-(5-(methylamino)nicotinoyl)piperidin-3-yl)amino)-3-nitrophenyl)((S)-2-(chloromethyl)morpholinyl)methanone

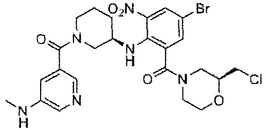

### Reaction route:

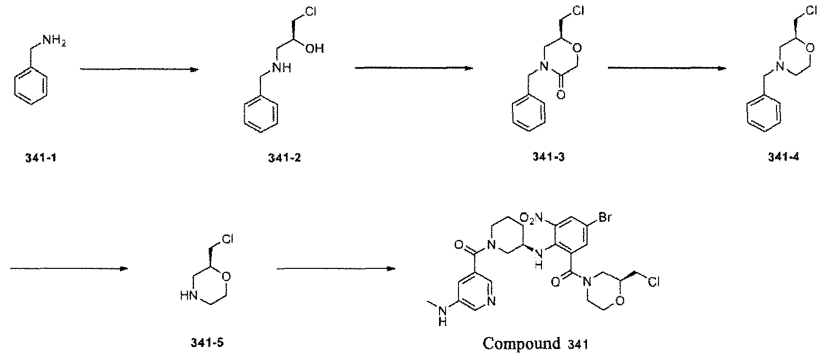

### Operating Steps:

**Step A:** Compound 341-1 (1.12 g, 10.9 mmol) was dissolved in cyclohexane (5 mL) at room temperature under the protection of nitrogen. Subsequently, (S)-2-(chloromethyl)oxirane (1.00 g, 10.9 mmol) was added to the above solution. The reaction system was then continued to be stirred for 16 h.

After the depletion of the raw material as monitored by LCMS, the reaction was quenched by addition of isopropanol (20 mL), and the mixture solution was filtered to obtain Compound 341-2 (800 mg).

MS (ESI) M/Z: 200.2 [M+H]⁺.

**Step B:** Compound 341-2 (300 mg, 3.02 mmol) and sodium hydroxide (600 mg, 30.2 mmol) were dissolved in a mixture of chloroform (3.3 mL) and water (1.3 mL) at room temperature under the protection of nitrogen. Subsequently, the above solution was cooled to 0°C, to which 2-bromoacetyl bromide (800 mg, 8.15 mmol) dissolved in chloroform (0.82 mL) was added, followed by stirring for 1 h. The reaction system was then stirred at room temperature for 2 h.

After the depletion of the raw material as monitored by LCMS, the reaction was quenched by addition of water (10 mL), and the mixture solution was extracted with dichloromethane (10 mL × 2 times). The organic phases were combined and washed with water (20 mL × 3 times), then dried with anhydrous sodium sulfate, filtered, and concentrated under reduced pressure. The resulting residue was purified by silica gel column chromatography to obtain Compound 341-3 (234 mg).

MS (ESI) M/Z: 240.2 [M+H]⁺.

**Step C:** 1 M solution of borane tetrahydrofuran (0.25 mL) was dissolved in tetrahydrofuran (0.4 mL) at room temperature under the protection of nitrogen. Subsequently, compound 341-3 (40 mg, 0.17 mmol) was added to the above solution. The reaction system was then heated to 70°C, and stirred for 2 h.

After the depletion of the raw material as monitored by LCMS, the reaction was quenched by dropwise addition of 0.5M diluted hydrochloric acid (5 mL) at 0°C, and the pH of the mixture solution was adjusted to 10 with saturated sodium bicarbonate solution. The mixture solution was extracted with ethyl acetate (5 mL × 3 times). The organic phases were combined and washed with saturated aqueous sodium chloride solution (15 mL × 2 times), then dried with anhydrous sodium sulfate, filtered, and concentrated under reduced pressure. The resulting residue was purified by silica gel column chromatography to obtain Compound 341-4 (34 mg).

MS (ESI) M/Z: 226.2 [M+H]⁺.

**Step D:** Compound 341-4 (34 mg, 0.15 mmol) and palladium/carbon (7 mg) were dissolved in methanol (2 mL) at room temperature. The reaction system was then subjected to hydrogen replacement and stirred for 2 h.

After the depletion of the raw material as monitored by LCMS, the reaction solution was filtered through diatomaceous earth and the filtrate was concentrated under reduced pressure to obtain Compound 341-5 (22 mg).

MS (ESI) M/Z: 136.2 [M+H]⁺.

**Step E:** Compound (*R*)-5-bromo-2-((1-(5-(metliylamino)nicotinoyl)piperidin-3-yl)amino)-3-nitrobenzoic acid (67 mg, 0.14 mmol) was dissolved in N,N-dimethylformamide (0.70 mL) at room temperature. Subsequently, the above solution was cooled to 0°C, followed by addition of *N*,*N*-diisopropylethylamine (55 mg, 0.42 mmol), 2-(7-azabenzotriazolyl)-*N*,*N*,*N*',*N*'-tetramethyluronium hexafluorophosphate (64 mg, 0.17 mmol), and Compound 1-5 (22.8 mg, 0.17 mmol) in turn. The reaction system was then continued to be stirred for 10 min.

After the depletion of the raw material as monitored by LCMS, the reaction was quenched by addition of water (5 mL), and the mixture solution was extracted with ethyl acetate (5 mL × 3 times). The organic phases were combined and washed with saturated aqueous sodium chloride solution (15 mL × 2 times), then dried with anhydrous sodium sulfate, filtered, and concentrated under reduced pressure. The resulting residue was purified by preparative high performance liquid chromatography to obtain the titled Compound 341 (21.52 mg).

MS (ESI) M/Z: 595.1 [M+H]⁺.

¹H NMR (400 MHz, DMSO-*d*₆) δ 8.27 (s. 1H), 8.10 - 7.34 (m. 4H), 6.91 - 6.44 (m, 1H), 4.62 - 4.12 (m, 1H), 4.09 - 3.37 (m. 10H), 3.32 - 2.88 (m. 3H). 2.87 - 2.68 (m, 3H), 2.00 - 1.38 (m. 5H).

### Example 342

### (5-Bromo-2-((R)-1-(5-(methylamino)nicotinoyl)piperidin-3-yl)amino)-3-nitrophenyl)((R)-2-cyclopropylmorpholinyl)methanone

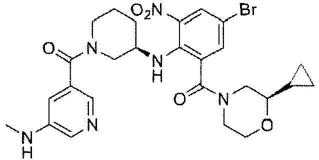

### Reaction route:

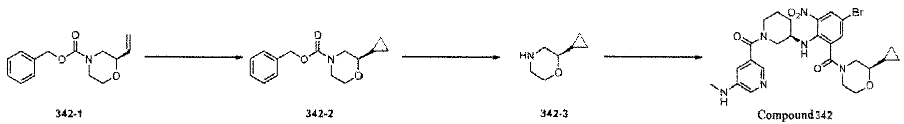

### Operating Steps:

Step A: Compound 342-1 (30 mg, 0.12 mmol) was dissolved in tetrahydrofuran (2.0 mL) at room temperature. Subsequently, palladium acetate (16 mg, 0.08 mmol) was added to the above solution and the reaction system was continued to be stirred for 1 h. The above solution was then cooled to -60°C, followed by dropwise addition of a solution of diazomethane ethyl ether (48 mg, 2.4 mmol). The resultant was heated to 0°C and stirred for 1 h.

After the depletion of the raw material as monitored by LCMS, the reaction was quenched by addition of water (15 mL), and the mixture solution was extracted with dichloromethane (15 mL × 3 times). The organic phases were combined and washed with saturated saline (10 mL), then dried with anhydrous sodium sulfate, filtered, and concentrated under reduced pressure. The resulting residue was purified by silica gel column chromatography to obtain Compound 342-2 (28 mg).

MS (ESI) M/Z: 262.2 [M+H]⁺.

**Step B:** Compound 342-2 (28 mg, 0.11 mmol) was dissolved in methanol (1.25 mL) at room temperature. Subsequently, 10% palladium/carbon (6 mg) was added to the above solution and the reaction system continued to be stirred for 2 hours in a hydrogen atmosphere.

After the depletion of the raw material as monitored by LCMS, the reaction solution was filtered and concentrated under reduced pressure. Compound 342-3 (11 mg) was then obtained.

MS (ESI) M/Z: 128.2 [M+H]⁺.

Step C: Compound 342-3 (11 mg, 0.086 mmol) and (*R*)-5-bromo-2-((1-(5-(methylamino)nicotinoyl)piperidin-3-yl)amino)-3-nitrobenzoic acid (41 mg, 0.086 mmol) were dissolved in *N*,*N*-dimethylformamide (1.0 mL) at room temperature. Subsequently, the above solution was cooled to 0°C, followed by addition of 2-(7-azabenzotriazolyl)-*N*,*N*,*N'*,*N'*-tetramethyluronium hexafluorophosphate (44 mg, 0.115 mmol) and *N*,*N*-diisopropylethylamine (45 mg, 0.345 mmol) in turn. The reaction system was then continued to be stirred for 30 min.

After the depletion of the raw material as monitored by LCMS, the reaction was quenched by addition of water (30 mL), and the mixture solution was extracted with ethyl acetate (6 mL × 3 times). The organic phases were combined and washed with saturated saline (20 mL × 2 times), then dried with anhydrous sodium sulfate, filtered, and concentrated under reduced pressure. The resulting residue was purified by reversed phase column chromatography to obtain the titled Compound 342 (9.5 mg).

MS (ESI) M/Z: 587.1 [M+H]⁺.

¹H NMR (400 MHz, DMSO-*d*₆) δ 8.26 (s. 1H), 8.08 - 7.38 (m. 4H), 6.88 - 6.44 (m, 1H), 4.51 - 4.12 (m, 1H), 4.09 - 3.36 (m, 6H), 3.34 - 3.06 (m, 3H). 3.05 - 2.67 (m. 5H). 1.97 - 1.29 (m. 4H), 0.98 - 0.63 (m. 1H). 0.57 - 0.18 (m. 3.6H), 0.13 - 0.01 (m. 0.4H).

### Example 344

### (5-Bromo-2-((R)-1-(5-(methylamino)nicotinoyl)piperidin-3-yl)amino)-3-nitrophenyl)((R)-2-vinylmorpholinyl)methanone

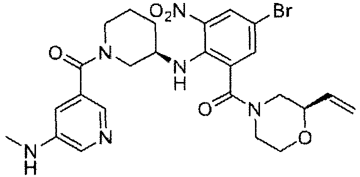

### Reaction route:

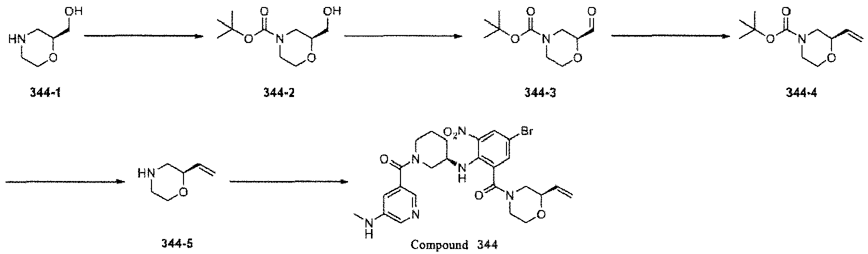

### Operating Steps:

Step A: Compound 344-1 (154 mg, 1.00 mmol) was dissolved in dichloromethane (5 mL) at room temperature. Subsequently, the above solution was cooled to 0°C, followed by addition of triethylamine (202 mg, 2.00 mmol) and di-tert-butyl dicarbonate (327 mg, 1.50 mmol) in turn. The reaction system was then stirred at 25°C for 2 h.

After the depletion of the raw material as monitored by LCMS, the reaction solution was concentrated under reduced pressure. The reaction was quenched by addition of water (30 mL) to the resulting residue, and the mixture solution was extracted with ethyl acetate (15 mL × 3 times). The organic phases were combined and washed with saturated saline (20 mL × 2 times). The washed organic phase was then dried with anhydrous sodium sulfate, filtered, and concentrated under reduced pressure, and the resulting residue was purified by silica gel column chromatography to obtain Compound 344-2 (218 mg).

MS (ESI) M/Z: 118.2 [M-99].

Step B: Compound 344-2 (100 mg, 0.46 mmol) was dissolved in ethyl acetate (2 mL) at room temperature under the protection of nitrogen. Subsequently, the above solution was cooled to -5°C, followed by addition of 2,2,6,6-tetramethylpiperidine oxide (1.0 mg, 0.0046 mmol), sodium bicarbonate (116 mg, 1.38 mmol), and trichloroisocyanuric acid (112 mg, 0.48 mmol) in turn. The reaction system was then continued to be stirred for 2 h.

After the depletion of the raw material as monitored by LCMS, the reaction was quenched by addition of saturated sodium bicarbonate solution (20 mL) and sodium thiosulfate solution (10 mL), and the mixture solution was extracted with ethyl acetate (20 mL × 3 times). The organic phases were combined and washed with saturated saline (10 mL) and sodium thiosulfate solution (10 mL). The washed organic phase was then dried with anhydrous sodium sulfate, filtered, and concentrated under reduced pressure to obtain Compound 344-3 (45 mg).

MS (ESI) M/Z: 160.2 [M-55].

Step C: Compound methyltriphenylphosphonium bromide (114 mg, 0.32 mmol) and potassium bis(trimethylsilyl)amide (64 mg, 0.32 mmol) were dissolved in dry tetrahydrofuran (3 mL) at 0°C, and the above solution was continued to be stirred for 0.5 h. Subsequently, compound 344-3 (45 mg, 0.21 mmol) was added to the above solution with stirring at room temperature for 0.5 h.

After the depletion of the raw material as monitored by LCMS, the reaction was quenched by addition of saturated ammonium chloride solution (20 mL), and the mixture solution was extracted with ethyl acetate (15 mL × 3 times). The organic phases were combined and washed with saturated saline (30 mL). The washed organic phase was then dried with anhydrous sodium sulfate, filtered, and concentrated under reduced pressure, and the resulting residue was purified by silica gel column chromatography to obtain Compound 344-4 (21 mg).

MS (ESI) M/Z: 158.2 [M-55].

¹H NMR (400 MHz, DMSO-*d*₆) δ 5.93 - 5.74 (m, 1H), 5.31 (d, *J* = 17.6 Hz, 1H), 5.1.8 (d, *J* = 10.4 Hz, 1H). 3.96 - 3 .74 (in, 3H), 3.69 (d. *J* = 12.4 Hz, 1H), 3.57 - 3.42 (m, 2H), 2.87 (brs, 1H). 1.41 (s, 9H).

Step D: Compound 344-4 (21 mg, 0.098 mmol) was dissolved in dichloromethane (0.9 mL) at 0°C. Subsequently, 4 M of hydrochloric acid-1,4-dioxane solution (0.49 mL, 1.96 mmol) was added to the above solution. The reaction system was then stirred at room temperature for 2 h.

After the depletion of the raw material as monitored by LCMS, the reaction solution was concentrated under reduced pressure to obtain Compound 344-5 (13 mg).

MS (ESI) M/Z: 114.2 [M+H]⁺.

Step E: Compound 344-5 (13 mg, 0.115 mmol) and (*R*)-5-bromo-2-((1-(5-(methylamino)nicotinoyl)piperidin-3-yl)amino)-3-nitrobenzoic acid (60 mg, 0.126 mmol) were dissolved in *N,N*-dimethylformamide (1.0 mL) at room temperature. Subsequently, the above solution was cooled to 0°C, followed by addition of 2-(7-azabenzotriazolyl)-*N*,*N*,*N'*,*N'*-tetramethyluronium hexafluorophosphate (44 mg, 0.115 mmol) and *N*,*N*-diisopropylethylamine (45 mg, 0.345 mmol) in turn. The reaction system was then continued to be stirred for 30 min.

After the depletion of the raw material as monitored by LCMS, the reaction was quenched by addition of water (30 mL), and the mixture solution was extracted with ethyl acetate (6 mL × 3 times). The organic phases were combined and washed with saturated saline (20 mL × 2 times), then dried with anhydrous sodium sulfate, filtered, and concentrated under reduced pressure. The resulting residue was purified by reversed phase column chromatography to obtain the titled Compound 344 (15.53 mg).

MS (ESI) M/Z: 573.4 [M+H]⁺.

¹H NMR (400 MHz, DMSO-*d*₆) δ 8.27 (s, 1H), 8.10 - 7.39 (m, 4H), 6.75 (s, 1H). 6.15 (s. 1H), 5.97 - 5.52 (m, 1H), 5.36 (d, *J* = 17.6 Hz, 0.5H), 5.31 - 5.19 (in, 1H), 5.15 (d, *J* = 10.8 Hz, 0. 5H). 4.52 - 4.16 (m. 1H), 4.15 - 3.37 (m. 7H), 3.30 - 2.85 (m, 3H), 2.70 (s. 4H). 2.04 - 1.30 (m, 4H).

### Example 345

### (5-Bromo-2-((R)-1-(5-(methylamino)nicotinoyl)piperidin-3-yl)amino)-3-nitrophenyl)((2S,6S)-2-methyl-6-(trifluoromethyl)morpholin)methanone

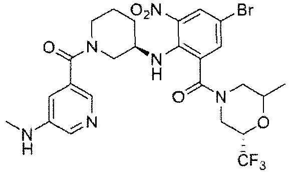

### Reaction route:

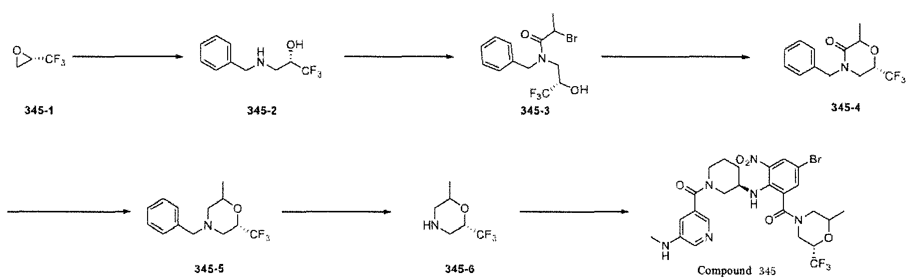

### Operating Steps:

**Step A:** Lithium trifluoromethanesulfonate (73 mg, 0.47 mmol) was dissolved in acetonitrile (11 mL) at -10°C under the protection of nitrogen. Subsequently, compound 345-1 (250 mg, 2.34 mmol) was added to the above solution, and the reaction system was continued to be stirred for 5 min. Compound benzylamine (262 mg, 2.34 mmol) was then added to the above solution. The reaction system was then stirred at room temperature for 16 h.

After the depletion of the raw material as monitored by LCMS, the reaction was quenched by addition of water (40 mL), and the mixture solution was extracted with ethyl acetate (20 mL × 3 times). The organic phases were combined and washed with saturated saline (30 mL × 2 times), then dried with anhydrous sodium sulfate, filtered, and concentrated under reduced pressure. The resulting residue was purified by silica gel column chromatography to obtain Compound 345-2 (200 mg).

MS (ESI) M/Z:220.2 [M+H]⁺.

**Step B:** Compound 345-2 (230 mg, 1.14 mmol) was dissolved in dichloromethane (5 mL) at 0°C under the protection of nitrogen. Subsequently, 2-bromopropionyl chloride (214 mg, 1.25 mmol) and triethylamine (150 mg, 1.48 mmol) were added to the above solution. The reaction system was then continued to be stirred for 2 h.

After the depletion of the raw material as monitored by LCMS, the reaction was quenched by addition of water (20 mL), and the mixture solution was extracted with ethyl acetate (10 mL × 3 times). The organic phases were combined and washed with saturated saline (30 mL × 2 times), then dried with anhydrous sodium sulfate, filtered, and concentrated under reduced pressure. The resulting residue was purified by silica gel column chromatography to obtain Compound 345-3 (202 mg).

MS (ESI) M/Z: 354.0 [M+H]⁺.

**Step C:** Compound 345-3 (202 mg, 0.57 mmol) was dissolved in tetrahydrofuran (3 mL) at 0°C under the protection of nitrogen. Subsequently, sodium hydride (21 mg, 0.85 mmol) was slowly added to the above solution. The reaction system was then continued to be stirred for 1 h.

After the depletion of the raw material as monitored by LCMS, the reaction was quenched by addition of water (20 mL), and the mixture solution was extracted with ethyl acetate (10 mL × 3 times). The organic phases were combined and washed with saturated saline (10 mL × 2 times), then dried with anhydrous sodium sulfate, filtered, and concentrated under reduced pressure. The resulting residue was purified by silica gel column chromatography to obtain Compound 345-4 (150 mg, 96% yield).

MS (ESI) M/Z: 274.2 [M+H]⁺.

**Step D:** Compound 345-4 (150 mg, 0.55 mmol) was dissolved in tetrahydrofuran (2.5 mL) at 0°C under the protection of nitrogen. Subsequently, lithium aluminum hydride (0.4 mL, 1.09 mmol) was added to the above solution. The reaction system was then continued to be stirred for 1 h.

After the depletion of the raw material as monitored by LCMS, sodium sulfate decahydrate (20 mg) was added to the reaction solution, and the resultant was filtered and concentrated under reduced pressure. The resulting residue was purified by silica gel column chromatography to obtain Compound 345-5 (140 mg).

60.2 [M+H]+. MS (ESI) M/Z:260.2 [M+H]⁺

**Step E:** Compound 345-5 (140 mg, 0.54 mmol) was dissolved in methanol (5 mL) at room temperature under the protection of nitrogen. Subsequently, 10% palladium/carbon (32 mg) was added to the above solution. The reaction system was then continued to be stirred for 1 h.

After the depletion of the raw material as monitored by LCMS, the reaction solution was filtered and concentrated under reduced pressure. Compound 345-6 (100 mg) was then obtained.

MS (ESI) M/Z: 170.2 [M+H]⁺.

**Step F:** Compound 345-6 (67 mg, 0.33 mmol) was dissolved in *N,N*-dimethylformamide (2 mL) at room temperature under the protection of nitrogen. Subsequently, the above solution was cooled to 0°C, followed by addition of (*R*)-5-bromo-2-(1-(5-(methylamino)nicotinoyl)piperidin-3-yl)amino)-3-nitrobenzoic acid (130 mg, 0.27 mmol), 2-(7-azabenzotriazolyl)-*N*,*N*,*N'*,*N'*-tetramethyluronium hexafluorophosphate (0.38 mg, 143 mmol) and *N,N*-diisopropylethylamine (174 mg, 1.35 mmol) in turn. The reaction system was then continued to be stirred for 5 min.

After the depletion of the raw material as monitored by LCMS, the reaction solution was concentrated under reduced pressure. The resulting residue was purified by preparative high performance liquid chromatography to obtain the titled Compound 345 (29 mg).

MS (ESI) M/Z: 629.2 [M+H]⁺.

¹H NMR (400 MHz. DMSO-*d*₆) δ 8.29 (s, 1H). 8.09 - 7.46 (m. 4H), 6.75 (s, 1H), 6.15 (s, 1H), 4.70 - 4.19 (m, 2H), 4.18 - 3.34 (m, 6H). 3.29 - 3.05 (m, 1.5H). 3.04 - 2.79 (m, 1.5H), 2.70 (brs, 3H). 2.00 - 1.34 (m, 4H), 1.29 - 0.96 (m. 3H).

### Example 346

### (5-Bromo-2-((R)-1-(5-(methylamino)nicotinoyl)piperidin-3-yl)amino)-3-nitrophenyl)((R)-2-ethynylmorpholinyl)methanone

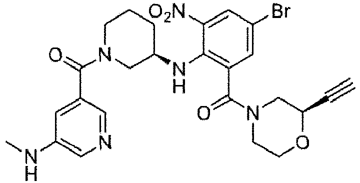

### Reaction route:

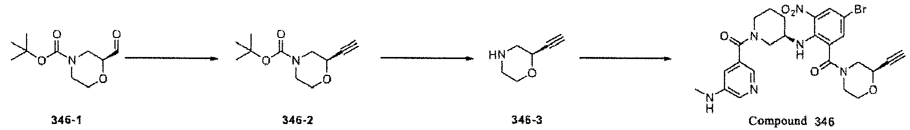

### Operating Steps:

Step A: Compound 346-1 (120 mg, 0.56 mmol) was dissolved in methanol (2.8 mL) at 0°C. Subsequently, potassium carbonate (232 mg, 1.68 mmol) and dimethyl(1-diazo-2-oxopropyl)phosphonate (108 mg, 0.56 mmol) were added to the above solution. The reaction system was then stirred at room temperature for 16 h.

After the depletion of the raw material as monitored by LCMS, the reaction was quenched by addition of water (20 mL), and the mixture solution was extracted with ethyl acetate (15 mL × 3 times). The organic phases were combined, washed with saturated saline (30 mL), then dried with anhydrous sodium sulfate, filtered, and concentrated under reduced pressure. The resulting residue was purified by silica gel column chromatography to obtain Compound 346-2 (48 mg).

MS (ESI) M/Z: 156.2 [M-55].

¹H NMR (400 MHz. DMSO-*d*₆) δ 4.38 - 4.30 (m, 1H), 3.83 - 3.75 (m. 1H), 3.61 - 3.49 (m. 2H), 3.49 - 3.43 (m, 1H). 3.41 - 3.32 (m. 1H). 3.30 - 3.12 (m. 2H). 1.40 (s, 9H).

Step B: Compound 346-2 (48 mg, 0.227 mmol) was dissolved in dichloromethane (1.1 mL) at 0°C. Subsequently, 4 M of hydrochloric acid-1,4-dioxane solution (0.85 mL, 3.41 mmol) was added to the above solution. The reaction system was then stirred at room temperature for 2 h.

After the depletion of the raw material as monitored by LCMS, the reaction solution was concentrated under reduced pressure to obtain Compound 346-3 (30 mg).

MS (ESI) M/Z: 112.2 [M+H]⁺.

Step C: Compound 346-3 (30 mg, 0.27 mmol) and (*R*)-5-bromo-2-((1-(5-(methylamino)nicotinoyl)piperidin-3-yl)amino)-3-nitrobenzoic acid (129 mg, 0.27 mmol) were dissolved in *N*,*N*-dimethylformamide (1.3 mL) at room temperature. Subsequently, the above solution was cooled to 0°C, followed by addition of 2-(7-azabenzotriazolyl)-*N*,*N*,*N'*,*N'*-tetramethyluronium hexafluorophosphate (103 mg, 0.27 mmol) and *N,N*-diisopropylethylamine (105 mg, 0.81 mmol) in turn. The reaction system was then continued to be stirred for 30 min.

After the depletion of the raw material as monitored by LCMS, the reaction was quenched by addition of water (30 mL), and the mixture solution was extracted with ethyl acetate (6 mL × 3 times). The organic phases were combined and washed with saturated saline (20 mL × 2 times), then dried with anhydrous sodium sulfate, filtered, and concentrated under reduced pressure. The resulting residue was purified by reversed phase column chromatography to obtain the titled Compound 346 (28.46 mg).

MS (ESI) M/Z: 571.4 [M+H]⁺.

¹H NMR (400 MHz, DMSO-*d₆*) δ 8.28 (s, 1H), 8.14 - 7.37 (m, 4H), 6.89 - 6.49 (m, 1H). 6.26 - 5.92 (m, 1H), 4.80 - 4.32 (m, 1H), 4.31 - 3.32 (m. 10H). 3.30 - 2.94 (m. 2H). 2.71 (s. 3H). 2.02 - 1.31 (m, 4H),

### Example 353

### (R)-(2-(1-(5-(methylamino)nicotinoyl)piperidin-3-yl)amino)-3-nitrophenyl)(morpholin-2,2,6,6-d₄)methanone

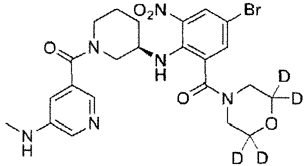

### Reaction route:

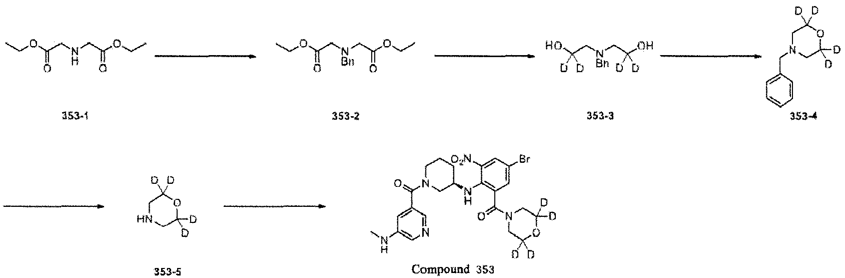

### Operating Steps:

Step A: Compound 353-1 (5.0 g, 26.5 mmol) was dissolved in N,N-dimethylformamide (60 mL) at room temperature under the protection of nitrogen. Subsequently, the above solution was cooled to 0°C, followed by addition of potassium carbonate (11.0 g, 80.5 mmol) and benzyl bromide (5.4 g, 31.7 mmol) in turn. The reaction system was then continued to be stirred for 12 h.

After the depletion of the raw material as monitored by LCMS, the reaction was quenched by addition of water (180 mL), and the mixture solution was extracted with ethyl acetate (50 mL × 3 times). The organic phases were combined and washed with aqueous sodium chloride solution (150 mL × 2 times), then dried with anhydrous sodium sulfate, filtered, and concentrated under reduced pressure. The resulting residue was purified by silica gel column chromatography to obtain Compound 353-2 (6.2 g).

MS (ESI) M/Z: 280.4 [M+H]⁺.

Step B: Compound 353-2 (460 mg, 1.65 mmol) was dissolved in anhydrous tetrahydrofuran (5.5 mL) at room temperature under the protection of nitrogen. Subsequently, the above solution was cooled to 0°C, followed by addition of aluminum lithium deuteride (200.0 mg, 4.94 mmol). The reaction system was then continued to be stirred for 1 h.

After the depletion of the raw material as monitored by LCMS, the reaction was quenched by slow addition of water (25 mL) at 0°C, and the mixture solution was extracted with dichloromethane (8 mL × 3 times). The organic phases were combined and washed with saturated aqueous sodium chloride solution (15 mL × 2 times), then dried with anhydrous sodium sulfate, filtered, and concentrated under reduced pressure. The resulting residue was purified by silica gel column chromatography to obtain Compound 353-3 (200 mg).

MS (ESI) M/Z: 200.4 [M+H]⁺.

Step C: Compound 353-3 (90 mg, 0.45 mmol) was dissolved in concentrated sulfuric acid (1 mL) at room temperature under the protection of nitrogen. Subsequently, the above solution was heated to 150°C and stirred for 1 h.

After the depletion of the raw material as monitored by LCMS, the reaction was quenched by slow and dropwise addition of water (15 mL), and the mixture solution was extracted with ethyl acetate (15 mL × 3 times). The organic phases were combined and washed with aqueous sodium chloride solution (10 mL × 2 times), then dried with anhydrous sodium sulfate, filtered, and concentrated under reduced pressure. The resulting residue was purified by silica gel column chromatography to obtain Compound 353-4 (36 mg).

MS (ESI) M/Z: 182.4 [M+H]⁺

Step D: Compound 353-4 (36 mg, 0.20 mmol) was dissolved in methanol (2 mL) at room temperature. Subsequently, palladium/carbon (20 mg) was added to the above solution. The reaction system was then subjected to hydrogen replacement for three times and stirred for 2 h.

After the depletion of the raw material as monitored by LCMS, the reaction solution was filtered and the filtrate was concentrated under reduced pressure to obtain Compound 353-5 (13 mg).

MS (ESI) M/Z: 92.2 [M+H]⁺.

Step E: Compound 353-5 (9.0 mg, 0.1 mmol) and (*R*)-5-bromo-2-((1-(5-(methylamino)nicotinoyl)piperidin-3-yl)amino)-3-nitrobenzoic acid (40.0 mg, 0.1 mmol) were dissolved in *N*,*N*-dimethylformamide (1.0 mL) at room temperature under the protection of nitrogen. Subsequently, the above solution was cooled to 0°C, followed by addition of 2-(7-azabenzotriazolyl)-*N*,*N*,*N'*,*N'*-tetramethyluronium hexafluorophosphate (45 mg, 0.12 mmol) and *N,N-*diisopropylethylamine (45 mg, 0.33 mmol) in turn. The reaction system was then continued to be stirred for 15 min.

After the depletion of the raw material as monitored by LCMS, the reaction was quenched by addition of water (15 mL), and the mixture solution was extracted with ethyl acetate (5 mL × 3 times). The organic phases were combined and washed with saturated saline (20 mL × 2 times), then dried with anhydrous sodium sulfate, filtered, and concentrated under reduced pressure. The resulting residue was purified by reversed phase column chromatography to obtain the titled Compound 353 (21.3 mg).

MS (ESI) M/Z: 550.6 [M+H]⁺.

¹H NMR (400 MHz. CDCl₃) δ 8.34 (s, 1H), 8.25 - 7.77 (m, 2.7H), 7.48 (s, 1.3H), 7.00 (s. 1H), 4.04 - 3. 10 (in, 9H). 2.91 (s, 3H), 2.23 - 1.76 (m, 3H). 1.56 - 1.38 (m. 1H),

### Example 355

### (5-Bromo-2-((R)-1-(5-(methytamino)nicotinoyi)piperidin-3-yl)amino)-3-nitrophenyl)((R)-3-methylmorpholinyl)methanone

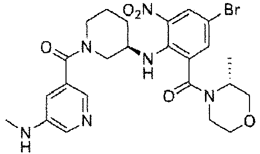

### Reaction route:

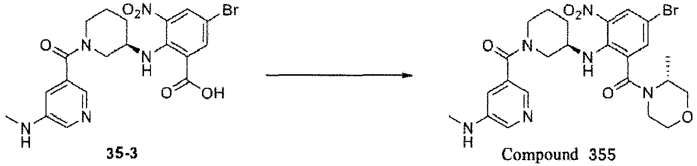

### Operating Steps:

Step A: Compound 35-3 (900 mg, 0.19 mmol) was dissolved in *N*,*N*-dimethylformamide solution (1.0 mL) at room temperature. Subsequently, (R)-3-methylmorpholine hydrochloride (23 mg, 0.23 mmol), *N,N,N',N'*-tetramethyl-O-(7-azabenzotriazol-1-yl)uronium hexafluorophosphate (78 mg, 0.21 mmol), and *N*,*N*-diisopropylethylamine (73 mg, 0.57 mmol) were added in turn to the above solution. The reaction system was then continued to be stirred for 30 min.

After the depletion of the raw material as monitored by LCMS, the reaction was quenched by addition of water (15 mL), and the mixture solution was extracted with ethyl acetate (5 mL × 3 times). The organic phases were combined and washed with saturated saline (15 mL × 2 times), then dried with anhydrous sodium sulfate, filtered, and concentrated under reduced pressure. The resulting residue was purified by preparative high performance liquid chromatography to obtain the titled Compound 355 (43.97 mg).

MS (ESI) M/Z: 561.1 [M+H]⁺.

¹H NMR (400 MHz, DMSO-*d*₆) δ 8.26 (s. 1H), 8.08 - 7.32 (m, 4H), 6.75 (s. 1H), 6.16 (s, 1H), 4.64 - 3.36 (m, 9.5H), 43.28 - 2.84 (m. 2.5H), 2.70 (brs, 3H), 2.08 - 1.07 (m. 7H).

### Example 357

### ((3R)-3-((2-(3-oxa-6-azabicyclo[3.1.1]heptan-6-carbonyl)-4-bromo-6-nitrophenyl)amino)piperidin-1-yl)(5-(methylamino)pyridin-3-yl)methanone

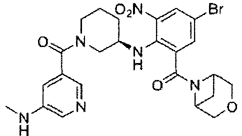

### Reaction route:

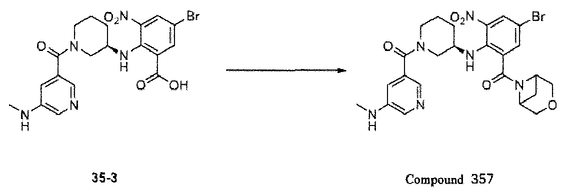

### Operating Steps:

Step A: Compound 35-3 (32 mg, 0.08 mmol) was dissolved in N,N-dimethylformamide (0.34 mL) at room temperature. Subsequently, *N*,*N*-diisopropylethylamine (0.03 mL, 0.20 mmol), 2-(7-azabenzotriazolyl)-*N*,*N*,*N'*,*N'*-tetramethyluronium hexafluorophosphate (28 mg, 0.07 mmol) and 3-oxa-6-azabicyclo[3.1.1]heptan-4-toluenesulfonate (20 mg, 0.07 mmol) were added to the above solution. The reaction system was then stirred at room temperature for 10 min.

After the depletion of the raw material as monitored by LCMS, the reaction was quenched by addition of water (10 mL), and the mixture solution was extracted with ethyl acetate (20 mL × 3 times). The organic phases were combined and washed with saturated aqueous sodium chloride solution (20 mL × 2 times), then dried with anhydrous sodium sulfate, filtered, and concentrated under reduced pressure. The resulting residue was purified by preparative high performance liquid chromatography to obtain the titled Compound 357 (13.87 mg).

MS (ESI) M/Z: 558.9 [M+H]⁺.

¹H NMR (400 MHz, DMSO-*d*₆) δ 8.26 (s. 1H). 7.97 (s, 1H). 7.85 - 7.49 (m, 3H). 6.79 (s. 1H). 6.24 - 5.94 (m. 1H), 4.64 - 3.95 (m, 3H), 3.94 - 3.50 (m. 6H). 3.26 - 2.94 (m, 2H), 2.90 - 2.59 (m, 4H), 2. 10 - 1.38 (m, 5H).

### Example 358

### (5-Bromo-2-((R)-1-(5-(methylamino)nicotinoyl)piperidin-3-yl)amino)-3-nitrophenyl)(3-oxa-8-azabicyclo[3.2.1]octan-8-yl)methanone

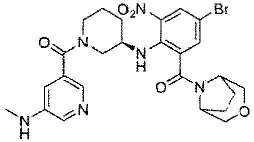

### Reaction route:

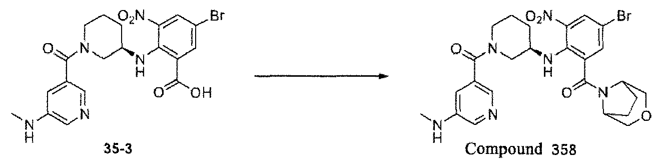

### Operating Steps:

Step A: Compound 3-oxa-8-azabicyclo[3.2.1]octane (11 mg, 0.10 mmol) was dissolved in *N*,*N*-dimethylformamide (0.40 mL) at room temperature. Subsequently, the above solution was cooled to 0°C, followed by addition of *N,N-*diisopropylethylamine (31 mg, 0.24 mmol), 2-(7-azabenzotriazolyl)-*N*,*N*,*N'*,*N'*-tetramethyluronium hexafluorophosphate (38 mg, 0.10 mmol), and Compound 35-3 (40 mg, 0.010 mmol) in turn. The reaction system was then continued to be stirred for 10 min.

After the depletion of the raw material as monitored by LCMS, the reaction was quenched by addition of water (10 mL), and the mixture solution was extracted with ethyl acetate (5 mL × 3 times). The organic phases were combined and washed with saturated aqueous sodium chloride solution (15 mL × 3 times), then dried with anhydrous sodium sulfate, filtered, and concentrated under reduced pressure. The resulting residue was purified by preparative high performance liquid chromatography to obtain the titled Compound 358 (17.28 mg).

MS (ESI) M/Z: 573.0 [M+H]⁻.

¹H NMR (400 MHz, DMSO-*d*₆) δ 8.27 (s. 1H), 8.10 - 7.36 (m, 4H). 6.75 (s, 1H), 6.38 - 5.83 (m. 1H), 4.75 - 4.36 (m. 1H), 4.18 - 3.37 (m, 9H). 3.24 - 3.06 (m. 1H). 2.70 (s, 3H), 2.11 - 1.30 (m. 8H).

### Example 360

### (3S,4S)-3-(2-(8-oxa-3-azabicyclo[3.2.1] octan-3-carbonyl)-4-bromo-6-nitrophenylamino)-4-methoxypyrrolidin-1-yl)5-methylaminopyridine-3-methanone

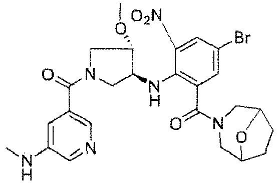

### Reaction route:

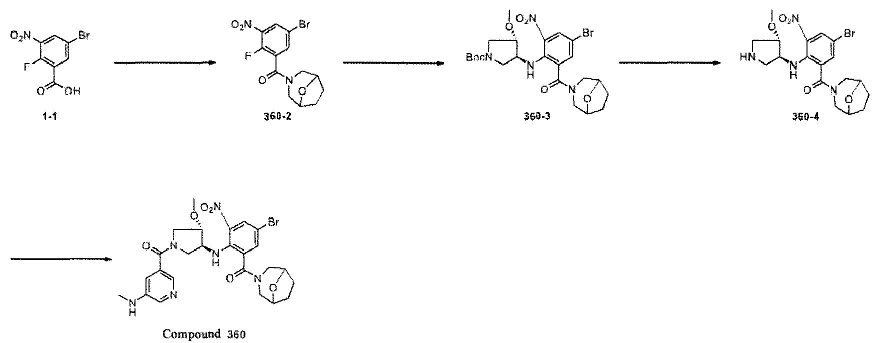

### Operating Steps:

Step A: Compound 1-1 (97.3 mg, 0.37 mmol) was dissolved in N,N-dimethylformamide (2 mL) at room temperature under the protection of nitrogen. Subsequently, *N*,*N*-diisopropylethylamine (0.18 mL, 1.1 mmol), 2-(7-azabenzotriazolyl)-*N*,*N*,*N'*,*N'*-tetramethyluronium hexafluorophosphate (195.7 mg, 0.52 mmol) and 8-oxa-3-azabicyclo[3.2.1]octane (50 mg, 0.44 mmol) were added to the above solution. The reaction system was then continued to be stirred for 20 min.

After the depletion of the raw material as monitored by LCMS, the reaction was quenched by addition of water (6 mL), and the mixture solution was extracted with ethyl acetate (2 mL × 3 times). The organic phases were combined and washed with saturated aqueous sodium chloride solution (6 mL × 2 times), then dried with anhydrous sodium sulfate, filtered, and concentrated under reduced pressure. Compound 360-2 (100.7 mg) was obtained from the resulting residue.

MS (ESI) M/Z: 358.9[M+H]⁺.

Step B: Compound 360-2 (36 mg, 0.1 mmol) was dissolved in *N,N*-dimethylformamide (0.5 mL) at room temperature. Subsequently, *N*,*N*-diisopropylethylamine (0.05 mL, 0.3 mmol) and tert-butyl (3*S*,4*S*)-3-amino-4-methoxypyrrolidin-1-carboxylate (21.7 mg, 0.1 mmol) were added to the above solution. The reaction system was then stirred at 80°C for 2 h.

After the depletion of the raw material as monitored by LCMS, the reaction was quenched by addition of water (2 mL), and the mixture solution was extracted with ethyl acetate (2 mL × 3 times). The organic phases were combined and washed with saturated aqueous sodium chloride solution (6 mL × 2 times), then dried with anhydrous sodium sulfate, filtered, and concentrated under reduced pressure. Compound 360-3 (74 mg) was obtained from the resulting residue.

MS (ESI) M/Z: 455.9[M-99]

Step C: Compound 360-3 (66 mg, 0.11 mmol) was dissolved in dichloromethane (0.6 mL) at room temperature. Subsequently, 4 M of hydrochloric acid-1,4-dioxane solution (0.6 mL) was added to the above solution. The reaction system was then continued to be stirred for 2 h.

After the depletion of the raw material as monitored by LCMS, reaction solution was concentrated under reduced pressure to obtain Compound 360-4 (67 mg).

MS (ESI) M/Z: 455.0[M+H]⁺.

Step D: 5-(Methylamino)nicotinic acid (15 mg, 0.1 mmol) was dissolved in N,N-dimethylformamide (0.5 mL) at room temperature under the protection of nitrogen. Subsequently, *N,N*-diisopropylethylamine (0.08 mL, 0.50 mmol), 2-(7-azabenzotriazolyl)-*N*,*N*,*N'*,*N'*-tetramethyluronium hexafluorophosphate (52.6 mg, 0.14 mmol) and Compound 360-4 (54 mg, 0.12 mmol) were added to the above solution. The reaction system was then continued to be stirred for 20 min.

After the depletion of the raw material as monitored by LCMS, the reaction was quenched by addition of water (2 mL), and the mixture solution was extracted with ethyl acetate (2 mL × 3 times). The organic phases were combined and washed with saturated aqueous sodium chloride solution (6 mL × 2 times), then dried with anhydrous sodium sulfate, filtered, and concentrated under reduced pressure. The crude product was purified by preparative high performance liquid chromatography to obtain the titled Compound 360 (22.37 mg).

MS (ESI) M/Z: 589.2 [M+H]⁺.

¹H NMR (400 MHz, DMSO-*d*₆) δ 8.33 - 8.10 (m. 1H), 8.08 - 7.92 (m, 1H), 7.90 - 7.57 (m, 2.3H). 7.46 - 7.10 (m. 0.7H), 7.01 - 6.68 (m, 1H), 6.25 - 6.04 (m, 1H), 4.53 - 3.38 (m. 9H). 3.32 - 2.83 (m. 6H), 2.79 - 2.61 (m, 3H), 2.00 - 1.34 (m, 4H),

### Example 362

### (2S,4R)-4-(4-bromo-2-(2R,6S)-2,6-dimethylmorpholin-4-carbonyl)-6-nitrophenylamino)-1-(5-(methylamino)nicotinoyl)pyrrolidin-2-carbonitrile

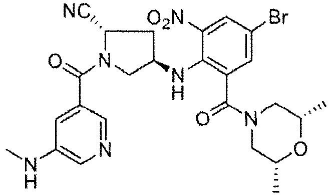

### Reaction route:

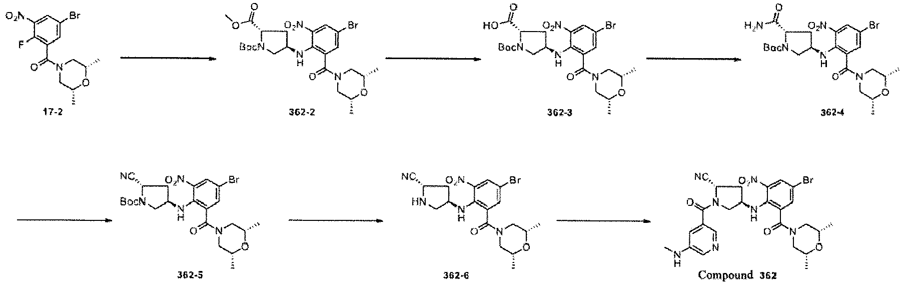

### Operating Steps:

Step A: Compound 17-2 (118 mg, 0.33 mmol) was dissolved in N, N-dimethylformamide (1.5 mL) at room temperature. Subsequently, *N,N*-diisopropylethylamine (0.16 mL, 0.98 mmol) and 2-methyl-2-tert-butyl-4-aminopyrrolidin-1,2-dicarboxylate (80mg, 0.33 mmol) were added to the above solution. The reaction system was then stirred at 80°C for 2 h.

After the depletion of the raw material as monitored by LCMS, the reaction was quenched by addition of water (8 mL), and the mixture solution was extracted with ethyl acetate (2 mL × 3 times). The organic phases were combined and washed with saturated aqueous sodium chloride solution (6 mL × 2 times), then dried with anhydrous sodium sulfate, filtered, and concentrated under reduced pressure. The resulting residue was purified by silica gel column chromatography to obtain Compound 362-2 (163 mg).

MS (ESI) M/Z: 485.0 [M-99].

Step B: Compound 362-2 (58 mg, 0.14 mmol) was dissolved in a mixture of tetrahydrofuran (1.5 mL) and water (0.3 mL) at room temperature. Subsequently, compound lithium hydroxide (23 mg, 0.56 mmol) was added to the above solution. The reaction system was then continued to be stirred for 2 h.

After the depletion of the raw material as monitored by LCMS, the reaction solution was concentrated under reduced pressure to obtain Compound 362-3.

MS (ESI) M/Z: 471.0[M-99].

Step C: Compound 362-3 was dissolved in tetrahydrofuran (1.5 mL) at room temperature. Subsequently, the above solution was cooled to -20°C, followed by addition of isobutyl chloroformate (57 mg, 0.42 mmol) and triethylamine (56.4 mg, 0.56 mmol) in turn with stirring for 30 min. The above solution was then heated to 0°C, followed by addition of aqueous ammonia (48.8 mg, 1.4 mmol). The reaction system was then continued to be stirred for 10 min.

After the depletion of the raw material as monitored by LCMS, the reaction was quenched by addition of water (2 mL), and the mixture solution was extracted with ethyl acetate (2 mL × 3 times). The organic phases were combined and washed with saturated aqueous sodium chloride solution (6 mL × 2 times), then dried with anhydrous sodium sulfate, filtered, and concentrated under reduced pressure. The resulting residue was purified by silica gel column chromatography to obtain Compound 362-4 (129 mg).

MS (ESI) M/Z: 469.9 [M-99]

Step D: Compound 362-4 (129 mg, 0.22 mmol) was dissolved in dichloromethane (1.5 mL) at room temperature under the protection of nitrogen. Subsequently, burgess reagent (523.6 mg, 2.2 mmol) was added to the above solution. The reaction system was then continued to be stirred for 1 h.

After the depletion of the raw material as monitored by LCMS, the reaction was quenched by addition of water (6 mL), and the mixture solution was extracted with ethyl acetate (2 mL × 3 times). The organic phases were combined and washed with saturated aqueous sodium chloride solution (6 mL × 2 times), then dried with anhydrous sodium sulfate, filtered, and concentrated under reduced pressure. The resulting residue was purified by silica gel column chromatography to obtain Compound 362-5 (127 mg).

MS (ESI) M/Z: 451.9 [M-99].

Step E: Compound 362-5 (117 mg, 0.21 mmol) was dissolved in formic acid (3 mL) at room temperature under the protection of nitrogen. The reaction system was then stirred at 0°C for 20 min.

After the depletion of the raw material as monitored by LCMS, aqueous ammonia was added to the reaction solution to adjust to pH = 9 at 0°C. The resulting crude product was purified by preparative high performance liquid chromatography to obtain Compound 362-6 (30 mg).

MS (ESI) M/Z: 452.0 [M+H]⁺.

Step F: 5-(Methylamino)nicotinic acid (8.1 mg, 0.05mmol) was dissolved in *N,N*-dimethylformamide (0.5 mL) at room temperature under the protection of nitrogen. Subsequently, *N*,*N*-diisopropylethylamine (0.04 mL, 0.26 mmol), 2-(7-azabenzotriazolyl)-*N*,*N*,*N'*,*N'*-tetramethyluronium hexafluorophosphate (28.2 mg, 0.07mmol) and Compound 362-6 (30 mg, 0.06 mmol) were added to the above solution. The reaction system was then continued to be stirred for 20 min.

After the depletion of the raw material as monitored by LCMS, the reaction was quenched by addition of water (2 mL), and the mixture solution was extracted with ethyl acetate (2 mL × 3 times). The organic phases were combined and washed with saturated aqueous sodium chloride solution (6 mL × 2 times), then dried with anhydrous sodium sulfate, filtered, and concentrated under reduced pressure. The crude product was purified by preparative high performance liquid chromatography to obtain the titled Compound 362 (7.66 mg).

MS (ESI) M/Z: 586.0 [M+H]⁺.

¹H NMR (400 MHz, DMSO-*d*₆) δ 8.34 - 8.14 (m, 1H), 8.11 - 7.95 (m. 1H), 7.94 - 7.77 (m, 1H), 7.74 - 7.58 (m, 1H), 7.53 - 7.26 (m, 1H), 6.99 - 6.78 (m, 1H), 6.23 (s, 1H). 5.12 - 4.86 (m, 1H). 4.53 - 3.40 (m, 6.5H). 3.30 - 3.08 (m. 1.5H), 3.00 - 2.67 (m. 4H). 2.66 - 2.53 (m, 1H), 2.45 - 2.35 (m, 1H), 1.11 (brs, 3H). 1.06 - 0.84 (m, 3H).

### Example 366

### (2S,4R)-4-(4-bromo-2-(2R,6S)-2,6-dimethylmorpholin-4-carbonyl)-6-nitrophenylamino)-N-methyl-1-(5-(methylamino)nicotinoyl)pyrrolidin-2-formamide

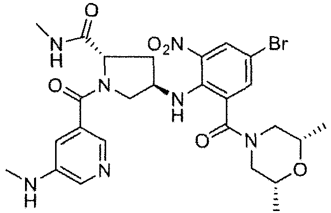

### Reaction route:

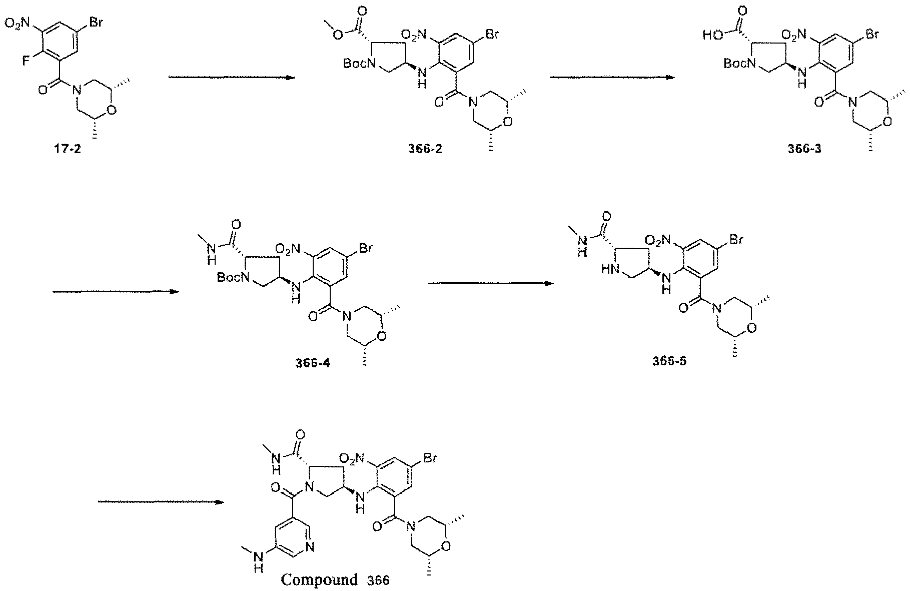

### Operating Steps:

Step A: Compound 17-2 (295 mg, 0.82 mmol) was dissolved in N,N-dimethylformamide (4 mL) at room temperature. Subsequently, *N*,*N*-diisopropylethylamine (0.4 mL, 2.46 mmol) and 2-methyl-2-tert-butyl-4-aminopyrrolidin-1,2-dicarboxylate (200 mg, 0.82 mmol) were added to the above solution. The reaction system was then stirred at 80°C for 2 h.

After the depletion of the raw material as monitored by LCMS, the reaction was quenched by addition of water (6 mL), and the mixture solution was extracted with ethyl acetate (2 mL × 3 times). The organic phases were combined and washed with saturated aqueous sodium chloride solution (6 mL × 2 times), then dried with anhydrous sodium sulfate, filtered, and concentrated under reduced pressure. The resulting residue was purified by silica gel column chromatography to obtain Compound 366-2 (386 mg).

MS (ESI) M/Z: 455.0[M-99]

Step B: Compound 366-2 (40 mg, 0.07 mmol) was dissolved in a mixture of tetrahydrofuran (0.3 mL) and water (0.1 mL) at room temperature. Subsequently, compound lithium hydroxide (5.7 mg, 0.14 mmol) was added to the above solution. The reaction system was then continued to be stirred for 2 h.

After the depletion of the raw material as monitored by LCMS, reaction solution was concentrated under reduced pressure to obtain Compound 366-3 (45 mg).

MS (ESI) M/Z: 470.9[M-99].

Step C: Compound 366-3 (45 mg, 0.08 mmol) was dissolved in N,N-dimethylformamide (0.4 mL) at room temperature under the protection of nitrogen. Subsequently, *N*,*N*-diisopropylethylamine (0.05 mL, 0.03 mmol), 2-(7-azabenzotriazolyl)-*N,N,N',N'*-tetramethyluronium hexafluorophosphate (36.2 mg, 0.09 mmol) and methylamine (3.6 mg, 0.08 mmol) were added to the above solution. The reaction system was then continued to be stirred for 20 min.

After the depletion of the raw material as monitored by LCMS, the reaction was quenched by addition of water (6 mL), and the mixture solution was extracted with ethyl acetate (2 mL × 3 times). The organic phases were combined and washed with saturated aqueous sodium chloride solution (6 mL × 2 times), then dried with anhydrous sodium sulfate, filtered, and concentrated under reduced pressure. The resulting residue was purified by silica gel column chromatography to obtain Compound 366-4 (47 mg).

MS (ESI) M/Z: 484.0 [M-99].

Step D: Compound 366-4 (47 mg, 0.08mmol) was dissolved in dichloromethane (0.4 mL) at room temperature. Subsequently, 4 M of hydrochloric acid-1,4-dioxane solution (0.4 mL) was added to the above solution. The reaction system was then continued to be stirred for 2 h.

After the depletion of the raw material as monitored by LCMS, reaction solution was concentrated under reduced pressure to obtain Compound 366-5 (52 mg).

MS (ESI) M/Z: 484.0 [M+H]⁺.

Step E: 5-(Methylamino)nicotinic acid (12.1 mg, 0.08 mmol) was dissolved in N,N-dimethylformamide (0.5 mL) at room temperature under the protection of nitrogen. Subsequently, *N*,*N*-diisopropylethylamine (0.07 mL, 0.4 mmol), 2-(7-azabenzotriazolyl)-*N*,*N*,*N'*,*N'*-tetramethyluronium hexafluorophosphate (42.5 mg, 0.11 mmol) and Compound 366-5 (52 mg, 0.1 mmol) were added to the above solution. The reaction system was then continued to be stirred for 20 min.

After the depletion of the raw material as monitored by LCMS, the reaction was quenched by addition of water (6 mL), and the mixture solution was extracted with ethyl acetate (2 mL × 3 times). The organic phases were combined and washed with saturated aqueous sodium chloride solution (6 mL × 2 times), then dried with anhydrous sodium sulfate, filtered, and concentrated under reduced pressure to obtain Compound 336 (15.2mg).

MS (ESI) M/Z: 618.1 [M+H]⁺.

¹H NMR (400 MHz, DMSO-*d*₆) δ 8.24 (s, 0.3H), 8.20 (s, 0.7H). 8.02 - 7.75 (m, 2.7H), 7.71 - 7.47 (m, 2H), 6.91 - 6.77 (m, 0.7H), 6.72 - 6.63 (m, 0.3H), 6.24 - 6.08 (m, 0.7H), 4.60 - 4.12 (m, 2.5H), 4.08 - 3.37 (m, 5H). 3.24 - 2.97 (m, 1H). 2.92 - 2.57 (m. 6.5H), 2.30 - 1.86 (m. 3H), 1.23 - 0.85 (m. 6H).

### Example 367

### (5-Bromo-2-((3S,4S)-4-methoxy-1-(5-(methylamino)nicotinoyl)pyrrolidin-3-yl)amino)-3-nitrophenyl)((R)-3-methylmorpholinyl)methanone

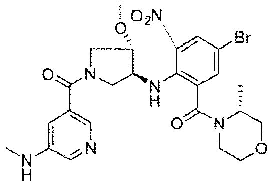

### Reaction route:

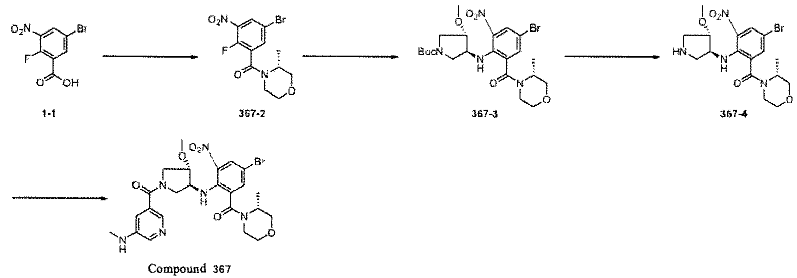

### Operating Steps:

Step A: Compound 1-1 (100 mg, 0.38 mmol) was dissolved in N,N-dimethylformamide (1.9 mL) at room temperature. Subsequently, the above solution was cooled to -10°C, followed by addition of *N*,*N*-diisopropylethylamine (0.19 mL, 1.14 mmol), 2-(7-azabenzotriazolyl)-*N*,*N*,*N'*,*N'*-tetramethyluronium hexafluorophosphate (173 mg, 0.45 mmol) and (R)-3-methylmorpholine (45 mg, 0.45 mmol) in turn. The reaction system was then continued to be stirred for 5 min.

After the depletion of the raw material as monitored by LCMS, the reaction was quenched by addition of water (30 mL), and the mixture solution was extracted with ethyl acetate (20 mL × 3 times). The organic phases were combined and washed with saturated aqueous sodium chloride solution (20 mL × 2 times), then dried with anhydrous sodium sulfate, filtered, and concentrated under reduced pressure. The resulting residue was purified by silica gel column chromatography to obtain Compound 367-2 (56 mg).

MS (ESI) M/Z: 347.0 [M+H]⁺.

Step B: Compound 367-2 (56 mg, 0.16 mmol) was dissolved in N,N-dimethylformamide (0.8 mL) at room temperature. Subsequently, tert-butyl (3*S*,4*S*)-3-amino-4-methoxypyrrolidin-1-carboxylate (35 mg, 0.16 mmol) and *N,N-*diisopropylethylamine (104 mg, 0.81 mmol) were added in turn to the above solution. The reaction system was then stirred at 80°C for 1 h.

After the depletion of the raw material as monitored by LCMS, the reaction was quenched by addition of water (25 mL), and the mixture solution was extracted with ethyl acetate (10 mL × 3 times). The organic phases were combined and washed with saturated aqueous sodium chloride solution (25 mL), then dried with anhydrous sodium sulfate, filtered, and concentrated under reduced pressure. The resulting residue was purified by silica gel column chromatography to obtain Compound 367-3 (60 mg).

MS (ESI) M/Z: 443.0 [M-99]

Step C: Compound 367-3 (60 mg, 0.11 mmol) was dissolved in dichloromethane (1.1 mL) at room temperature. Subsequently, the above solution was cooled to 0°C, followed by slow and dropwise addition of 4 M of hydrochloric acid-1,4-dioxane solution (0.6 mL, 2.21 mmol). The reaction system was then stirred at room temperature for 2 h.

After the depletion of the raw material as monitored by LCMS, the reaction solution was concentrated under reduced pressure. Compound 367-4 (53 mg) was then obtained.

MS (ESI) M/Z: 443.0 [M+H]⁺.

Step D: Compound 367-4 (52 mg, 0.12 mmol) and 5-(methylamino)nicotinic acid (27 mg, 0.18 mmol) was dissolved in N,N-dimethylformamide (0.5 mL) at 0°C. Subsequently, 2-(7-azabenzotriazolyl)-*N*,*N*,*N'*,*N'*-tetramethyluronium hexafluorophosphate (45 mg, 0.12 mmol) and *N*,*N*-diisopropylethylamine (46 mg, 0.35 mmol) were added to the above solution. The reaction system was then continued to be stirred for 20 min.

After the depletion of the raw material as monitored by LCMS, the reaction was quenched by addition of water (25 mL), and the mixture solution was extracted with ethyl acetate (20 mL × 3 times). The organic phases were combined and washed with saturated aqueous sodium chloride solution (20 mL × 2 times), then dried with anhydrous sodium sulfate, filtered, and concentrated under reduced pressure. The resulting residue was purified by preparative high performance liquid chromatography to obtain the titled Compound 367 (26.33 mg).

MS (ESI) M/Z: 577.0 [M+H]⁺.

¹H NMR (400 MHz. DMSO-*d*₆) δ 8.32 - 8.15 (m. 1H), 8.06 - 7.92 (m. 1H), 7.90 - 7.51 (m. 2H), 7.49 - 7.13 (m. 1H), 7.00 - 6.70 (m, 1H), 6.29 - 6.00 (m, 1H), 4.68 - 3.38 (m, 11H), 3.30 - 2.83 (in, 5H). 2.79 - 2.62 (m, 3H), 1.46 - 0.96 (m. 3H).

### Example 368

### (R)-(5-bromo-2-((1-(5-(methylamino)nicotinoyl)piperidin-3-yl)amino)-3-nitrophenyt)(2,2,6,6-tetrafluoromorpholinyl)methanone

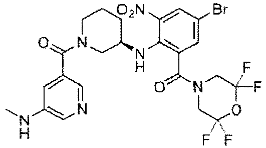

### Reaction route:

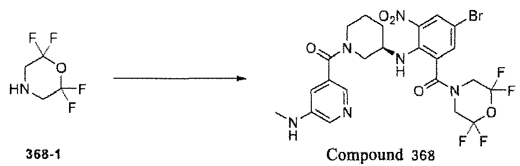

### Operating Steps:

Step A: (*R*)-5-bromo-2-((1-(5-(methylamino)nicotinoyl)piperidin-3-yl)amino)-3-nitrobenzoic acid (54 mg, 0.11 mmol) was dissolved in *N*,*N*-dimethylformamide (0.45 mL) at room temperature. Subsequently, the above solution was cooled to 0°C, followed by addition of *N*,*N*-diisopropylethylamine (34 mg, 0.27 mmol), 2-(7-azabenzotriazolyl)-*N*,*N*,*N'*,*N'-*tetramethyluronium hexafluorophosphate (42 mg, 0.11 mmol) and Compound 368-1 (15 mg, 0.09 mmol) in turn. The reaction system was then stirred at 0°C for 10 min.

After the depletion of the raw material as monitored by LCMS, the reaction was quenched by addition of water (10 mL), and the mixture solution was extracted with ethyl acetate (5 mL × 3 times). The organic phases were combined and washed with saturated aqueous sodium chloride solution (10 mL × 2 times), then dried with anhydrous sodium sulfate, filtered, and concentrated under reduced pressure. The resulting residue was purified by preparative high performance liquid chromatography to obtain the titled Compound 368 (29.07 mg).

MS (ESI) M/Z: 619.0 [M+H]⁺.

¹H NMR (400 MHz. DMSO-*d*₆) δ 8.35 (s. 1H), 8.10 - 7.81 (m, 2H). 7.79 - 7.44 (m, 2H), 6.75 (s. 1H), 6.28 - 5.89 (m. 1H), 4.80 - 3.94 (m. 4H), 3.92 - 3.35 (m. 4H), 3.29 - 3.11 (m. 1H), 2.70 (s, 3H), 1.94 - 1.65 (m, 2H). 1.63 - 1.32 (m. 2H).

### Example 369

### (2S,4R)-4-((4-bromo-2-((2R,6S)-2,6-dimethylmorpholin-4-carbonyl)-6-nitrophenyl)amino)-N-(isoxazol-4-yl)-1-(5-(methylamino)nicotinoyl)pyrrolidin-2-formamide

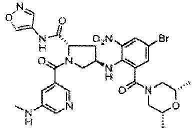

### Reaction route:

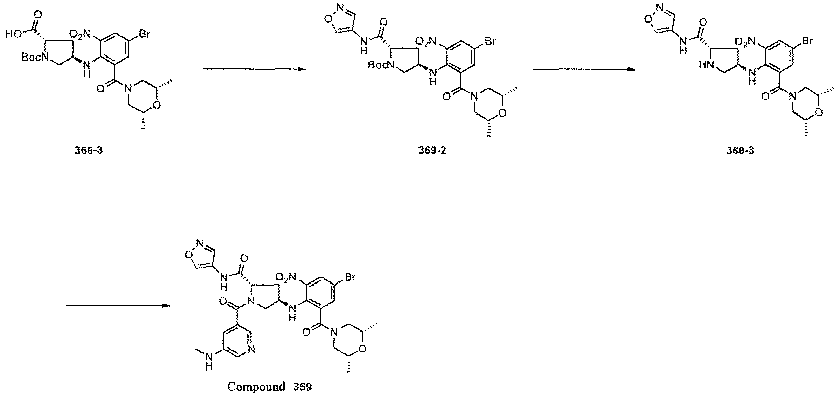

### Operating Steps:

Step A: Compound 366-3 (40 mg, 0.07 mmol) was dissolved in N,N-dimethylformamide (0.5 mL) at room temperature. Subsequently, the above solution was cooled to 0°C, followed by addition of *N,N*-diisopropylethylamine (28 mg, 0.21 mmol), 2-(7-azabenzotriazolyl)-*N*,*N*,*N'*,*N'*-tetramethyluronium hexafluorophosphate (27 mg, 0.07 mmol) and isoxazol-4-amine (11 mg, 0.08 mmol) in turn. The reaction system was then continued to be stirred for 1 h.

After the depletion of the raw material as monitored by LCMS, the reaction was quenched by addition of water (10 mL), and the mixture solution was extracted with ethyl acetate (15 mL × 3 times). The organic phases were combined, washed with saturated aqueous sodium chloride solution (20 mL × 2 times), dried with anhydrous sodium sulfate, filtered and concentrated under reduced pressure. The resulting residue was purified by silica gel column chromatography to obtain Compound 369-2 (50 mg).

MS (ESI) M/Z: 536.8 [M-99].

Step B: Compound 369-2 (50 mg, 0.08 mmol) was dissolved in dichloromethane (0.8 mL) at room temperature. Subsequently, the above solution was cooled to 0°C, followed by slow and dropwise addition of 4 M of hydrochloric acid-1,4-dioxane solution (0.8 mL). The reaction system was then stirred at room temperature for 30 min.

After the depletion of the raw material as monitored by LCMS, the reaction solution was concentrated by vacuum distillation. Compound 369-3 (50 mg) was then obtained.

MS (ESI) M/Z: 536.8 [M+H]⁺.

Step C: 5-(Methylamino)nicotinic acid (11 mg, 0.07 mmol) was dissolved in N,N-dimethylformamide (0.5 mL) at room temperature. Subsequently, the above solution was cooled to 0°C, followed by addition of *N*,*N*-diisopropylethylamine (47 mg, 0.36 mmol), 2-(7-azabenzotriazolyl)-*N*,*N*,*N'*,*N'*-tetramethyluronium hexafluorophosphate (41 mg, 0.11 mmol) and Compound 369-3 (50 mg, 0.09 mmol) in turn. The reaction system was then continued to be stirred for 1 h.

After the depletion of the raw material as monitored by LCMS, the reaction was quenched by addition of water (10 mL), and the mixture solution was extracted with ethyl acetate (15 mL × 3 times). The organic phases were combined, washed with saturated aqueous sodium chloride solution (20 mL × 2 times), dried with anhydrous sodium sulfate, filtered and concentrated under reduced pressure. The resulting residue was purified by preparative high performance liquid chromatography to obtain the titled Compound 369 (10.89 mg).

MS (ESI) M/Z: 671.0 [M+H]⁺.

¹H NMR (400 MHz, DMSO-*d*₆) δ 10.68 - 10.26 (m, 1H). 9.25 - 8.93 (m, 1H), 8.77 - 8.52 (m, 1H), 8.34 - 8.14 (m, 1H), 8.05 - 7.46 (m, 4H), 6.92 - 6.60 (m, 1H), 6.28 - 6.01 (m. 1H), 4.82 - 4.56 (m. 1H), 4.47 - 3.48 (m, 7H). 2.84 - 2.69 (m, 3H), 2.62 - 2.54 (m, 1H), 2.40 - 2.11 (m. 3H). 1.18 - 0.83 (m. 6H).

### Example 371

### ((3S,4S)-3-(2-(1R,5S)-3-oxa-9-azabicyclo[3.3.1]nonan-9-carbonyl)-4-bromo-6-nitrophenyl)amino)-4-methoxypyrrolidin-1-yl)(5-(methylamino)pyridin-3-yl)methanone

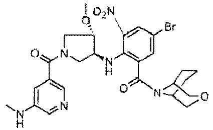

### Reaction route:

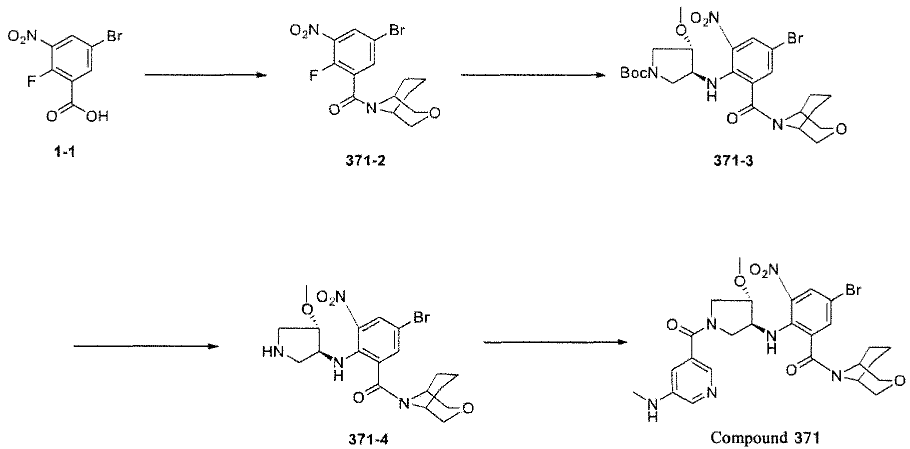

### Operating Steps:

Step A: Compound 1-1 (21.65 g, 82.32 mmol) was dissolved in *N*,*N*-dimethylformamide solution (400 mL) at room temperature. Subsequently, the above solution was cooled to -10°C, followed by addition of 2-(7-azabenzotriazolyl)-*N*,*N*,*N'*,*N'*-tetramethyluronium hexafluorophosphate (43.79 mg, 115.25 mmol), *N*,*N*-diisopropylethylamine (31.86 mg, 246.96 mmol) and (1*R*,5*S*)-3-oxa-9-azabicyclo[3.3.1]nonane hydrochloride (16.2 g, 98.78 mmol) in turn. The reaction system was then stirred at 0°C for 20 min.

After the depletion of the raw material as monitored by LCMS, the reaction was quenched by addition of water (1.2 mL), and the mixture solution was extracted with ethyl acetate (400 mL × 3 times). The organic phases were combined and washed with saturated saline (100 mL × 2 times), then dried with anhydrous sodium sulfate, filtered, and concentrated under reduced pressure. The resulting residue was purified by silica gel column chromatography to obtain Compound 371-2 (29 g).

MS (ESI) M/Z: 372.9 [M+H]⁺.

Step B: Compound 371-2 (53 mg, 0.14 mmol) was dissolved in N,N-dimethylformamide solution (1 mL) at room temperature under the protection of nitrogen. Subsequently, tert-butyl (3*S*,4*S*)-3-amino-4-methoxypyrrolidin-1-carboxylate (37 mg, 0.17 mmol) and *N*,*N*-diisopropylethylamine (54 mg, 0.42 mmol) were added in turn to the above solution. The reaction system was then stirred at 80°C for 1 h.

After the depletion of the raw material as monitored by LCMS, the reaction was quenched by addition of water (20 mL), and the mixture solution was extracted with ethyl acetate (9 mL × 3 times). The organic phases were combined and washed with saturated saline (10 mL × 2 times), then dried with anhydrous sodium sulfate, filtered, and concentrated under reduced pressure. The resulting residue was purified by silica gel column chromatography to obtain Compound 371-3 (60 mg).

MS (ESI) M/Z: 469.0 [M-99].

Step C: Compound 371-3 (60 mg, 0.11 mmol) was dissolved in dichloromethane (0.5 mL) at room temperature under the protection of nitrogen. Subsequently, the above solution was cooled to 0°C, followed by slow addition of 4 M of hydrochloric acid-1,4-dioxane solution (0.27 mL). The reaction system was then stirred at room temperature for 1 h.

After the depletion of the raw material as monitored by LCMS, the reaction solution was concentrated under reduced pressure. Compound 371-4 (50 mg) was then obtained.

MS (ESI) M/Z: 469.0 [M+H]⁺.

Step D: 5-(Methylamino)nicotinic acid (19 mg, 0.12 mmol) was dissolved in *N*,*N*-dimethylformamide solution (1 mL) at room temperature. Subsequently, the above solution was cooled to 0°C, followed by addition of 2-(7-azabenzotriazolyl)-*N*,*N*,*N'*,*N'*-tetramethyluronium hexafluorophosphate (75 mg, 0.15 mmol), *N,N*-diisopropylethylamine (85 mg, 0.5 mmol) and Compound 371-4 (50 mg, 0.10 mmol) in turn. The reaction system was then continued to be stirred for 30 min.

After the depletion of the raw material as monitored by LCMS, the reaction was quenched by addition of water (20 mL), and the mixture solution was extracted with ethyl acetate (9 mL × 3 times). The organic phases were combined and washed with saturated saline (10 mL × 2 times), then dried with anhydrous sodium sulfate, filtered, and concentrated under reduced pressure. The resulting residue was purified by preparative high performance liquid chromatography to obtain the titled Compound 371 (6.45 mg).

MS (ESI) M/Z:603.0 [M+H]⁺.

¹H NMR (400 MHz, DMSO-*d*₆) δ 8.30 - 8.15 (m, 1H), 8.08 - 7.93 (m, 1H), 7.91 - 7.60 (m, 2H). 7.48 - 7.04 (m, 1H), 7.01 - 6.70 (m, 1H), 6.20 - 6.05 (m. 1H), 4.58 - 4.29 (m. 1H), 4.22 - 3.37 (m, 10H). 3.27 - 2.95 (m, 3H). 2.81 - 2.65 (m. 3H), 2.47 - 2.35 (m. 1H). 2.05 - 1.44 (m, 5H).

### Example 374

### (5-Bromo-2-((3S,4S)-4-methoxy-1-(5-(methylamino)nicotinoyl)pyrrolidin-3-yl)amino)-3-nitrophenyl)(3,3-difluoropiperidin-1-yl)methanone

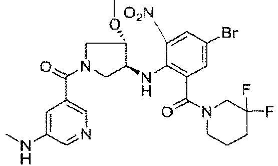

### Reaction route:

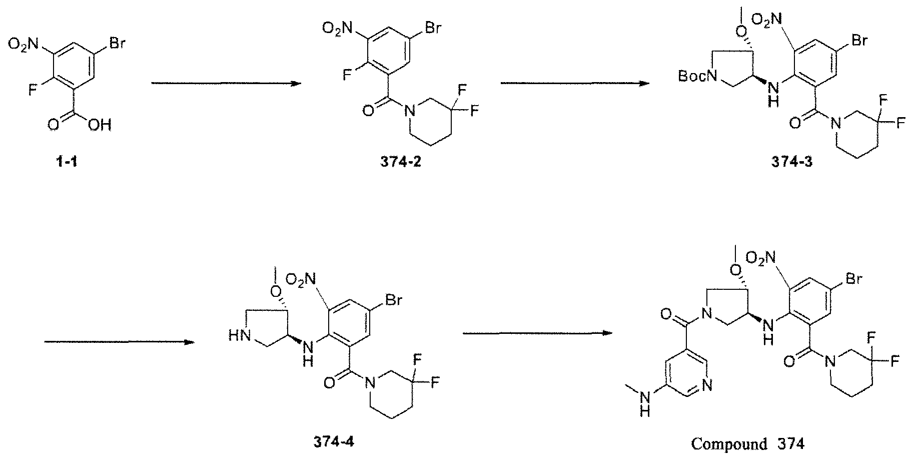

### Operating Steps:

Step A: Compound 1-1 (80 mg, 0.30 mmol) was dissolved in N,N-dimethylformamide (1.5 mL) at room temperature. Subsequently, the above solution was cooled to 0°C, followed by addition of *N,N*-diisopropylethylamine (117 mg, 0.90 mmol), 2-(7-azabenzotriazolyl)-*N,N,N',N'-*tetramethyluronium hexafluorophosphate (160 mg, 0.42 mmol), and 3,3-difluoropyridine (58 mg, 0.37 mmol) in turn. The reaction system was then stirred at 0°C for 10 min.

After the depletion of the raw material as monitored by LCMS, the reaction was quenched by addition of water (10 mL), and the mixture solution was extracted with ethyl acetate (10 mL × 3 times). The organic phases were combined and washed with saturated aqueous sodium chloride solution (30 mL × 2 times), then dried with anhydrous sodium sulfate, filtered, and concentrated under reduced pressure. The resulting residue was purified by silica gel column chromatography to obtain Compound 374-2 (75 mg).

MS (ESI) M/Z: 366.8 [M+H]⁺.

Step B: Compound 374-2 (42 mg, 0.12mmol) was dissolved in N,N-dimethylformamide (0.6 mL) at room temperature. Subsequently, tert-butyl (3*S*,4*S*)-3-amino-4-methoxypyrrolidin-1-carboxylate (30 mg, 0.14 mmol) and *N,N-*diisopropylethylamine (46 mg, 0.36 mmol) were added in turn to the above solution. The reaction system was then stirred at 80°C for 2 h.

After the depletion of the raw material as monitored by LCMS, the reaction was quenched by addition of water (10 mL), and the mixture solution was extracted with ethyl acetate (5 mL × 3 times). The organic phases were combined and washed with saturated saline (25 mL × 2 times), then dried with anhydrous sodium sulfate, filtered, and concentrated under reduced pressure. The resulting residue was purified by silica gel column chromatography to obtain Compound 374-3 (60 mg).

MS (ESI) M/Z: 462.8 [M-99].

Step C: Compound 374-3 (60 mg, 0.11 mmol) was dissolved in dichloromethane (0.28 mL) at room temperature. Subsequently, 4 M of hydrochloric acid-1,4-dioxane solution (0.28 mL) was slowly and dropwise added to the reaction system. The reaction system was then continued to be stirred for 2 h.

After the depletion of the raw material as monitored by LCMS, the reaction system was concentrated under reduced pressure. Compound 374-4 (50mg) was then obtained.

MS (ESI) M/Z: 463.0 [M+H]⁺.

Step D: 5-(Methylamino)nicotinic acid (19 mg, 0.13 mmol) was dissolved in N,N-dimethylformamide (0.55 mL) at room temperature. Subsequently, the above solution was cooled to 0°C, followed by addition of *N*,*N*-diisopropylethylamine (43 mg, 0.33 mmol), 2-(7-azabenzotriazolyl)-*N*,*N*,*N'*,*N'*-tetramethyluronium hexafluorophosphate (49 mg, 0.13 mmol) and Compound 374-4 (50 mg, 0.11 mmol) in turn. The reaction system was then stirred at 0°C for 10 min.

After the depletion of the raw material as monitored by LCMS, the reaction was quenched by addition of water (10 mL), and the mixture solution was extracted with ethyl acetate (10 mL × 3 times). The organic phases were combined and washed with saturated aqueous sodium chloride solution (30 mL × 2 times), then dried with anhydrous sodium sulfate, filtered, and concentrated under reduced pressure. The resulting residue was purified by preparative high performance liquid chromatography to obtain the titled Compound 374 (11.98 mg).

MS (ESI) M/Z: 597.0 [M+H]⁺.

¹H NMR (400 MHz, DMSO-*d*₆) δ 8.34 - 8.15 (m. 1H), 8.06 - 7.93 (m, 1H), 7.90 - 7.71 (m, 1H), 7.69 - 7.22 (m, 2H), 6.98 - 6.70 (m, 1H). 6.24 - 6.05 (m. 1H), 4.60 - 3.39 (m. 9H), 3.29 - 2.99 (m, 4H), 2.81 - 2.67 (m, 3H), 2.26 - 1.93 (m, 2H), 1.85 - 1.58 (m. 2H).

### Example 379

### (2S,4R)-4-((4-bromo-2-((2R,6S)-2,6-dimethylmorpholin-4-carbonyl)-6-nitrophenyl)amino)-1-(5-(methylamino)nicotinoyl)pyrrolidin-2-formamide

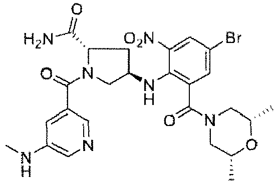

### Reaction route:

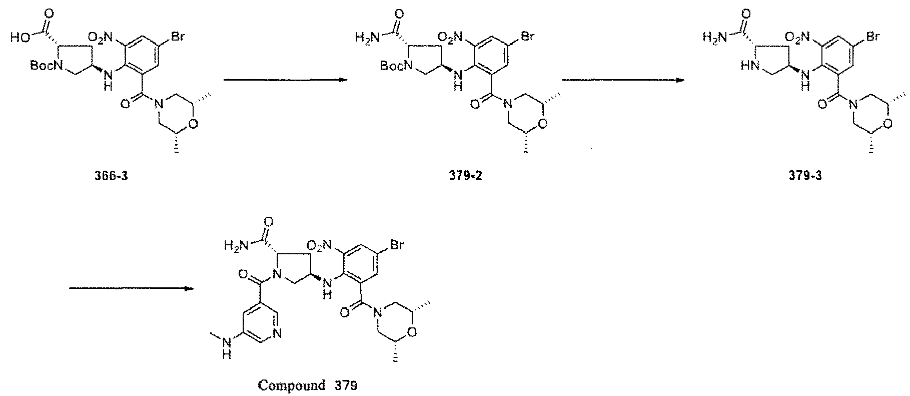

### Operating Steps:

Step A: Compound 366-3 (44 mg, 0.08 mmol) was dissolved in tetrahydrofuran (0.5 mL) at room temperature. Subsequently, the above solution was cooled to -20°C, followed by addition of isobutyl chloroformate (16 mg, 0.12 mmol) and triethylamine (15 mg, 0.15 mmol). The reaction system was then continued to be stirred for 30 min. The above solution was then heated to 0°C, followed by addition of aqueous ammonia (13 mg, 0.39 mmol). The reaction system was then continued to be stirred for 10 min.

After the depletion of the raw material as monitored by LCMS, the pH was adjusted to 4 by dropwise addition of 1 M aqueous hydrochloric acid solution to the reaction solution, which was heated to room temperature. Water (3 mL) was then added to the above solution, and the mixture solution was extracted with ethyl acetate (3 mL × 3 times). The organic phases were combined and washed with saturated aqueous sodium chloride solution (15 mL), then dried with anhydrous sodium sulfate, filtered, and concentrated under reduced pressure. The resulting residue was purified by silica gel column chromatography to obtain Compound 379-2 (23 mg).

MS (ESI) M/Z: 469.9[M-99].

Step B: Compound 379-2 (23 mg, 0.04 mmol) was dissolved in dichloromethane (0.4 mL) at room temperature. Subsequently, 4 M of hydrochloric acid-1,4-dioxane solution (0.4 mL) was added to the above solution. The reaction system was then continued to be stirred for 2 h.

After the depletion of the raw material as monitored by LCMS, the reaction solution was concentrated by vacuum distillation. Compound 379-3 (52 mg) was then obtained.

MS (ESI) M/Z: 470.0 [M+H]⁺.

Step C: 5-(Methylamino)nicotinic acid (16.7 mg, 0.11 mmol) was dissolved in N,N-dimethylformamide (0.5 mL) at room temperature. Subsequently, *N,N*-diisopropylethylamine (0.09 mL, 0.55 mmol), 2-(7-azabenzotriazolyl)-*N,N,N',N'-*tetramethyluronium hexafluorophosphate (58.5 mg, 0.15 mmol) and Compound 379-3 (52 mg, 0.11 mmol) were added in turn to the above solution. The reaction system was then continued to be stirred for 20 min.

After the depletion of the raw material as monitored by LCMS, the reaction was quenched by addition of water (10 mL), and the mixture solution was extracted with ethyl acetate (10 mL × 3 times). The organic phases were combined and washed with saturated aqueous sodium chloride solution (10 mL × 2 times), then dried with anhydrous sodium sulfate, filtered, and concentrated under reduced pressure. The resulting residue was purified by preparative high performance liquid chromatography to obtain the titled Compound 379 (13.09 mg).

MS (ESI) M/Z: 603.6 [M+H]+.

¹H NMR (400 MHz, DMSO-*d*₆) δ 8.29 - 8.12 (m, 1H), 8.04 - 7.92 (m, 1H), 7.91 - 7.22 (m, 4H), 7.20 - 6.66 (m, 2H), 6.23 - 6.01 (m, 1H), 4.60 - 3.91 (m, 3H), 3.89 - 3.36 (m, 4.4H), 3.27 - 3.05 (m. 0.6H), 2.95 - 2.64 (m, 4H). 2.62 - 2.54 (m, 0.4H), 2.43 - 2.09 (m. 2.6H). 1.21 - 0.79 (m. 6H).

### Example 383

### (2S,4R)-N-(4-bromo-1-methyl-1H-pyrazol-3-yl)-4-((4-bromo-2-((2R,6S)-2,6-dimethylmorpholin-4-carbonyl)-6-nitrophenyl)amino)-1-(5-(methylamino)nicotinoyl)pyrrolidin-2-formamide

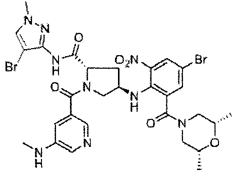

### Reaction route:

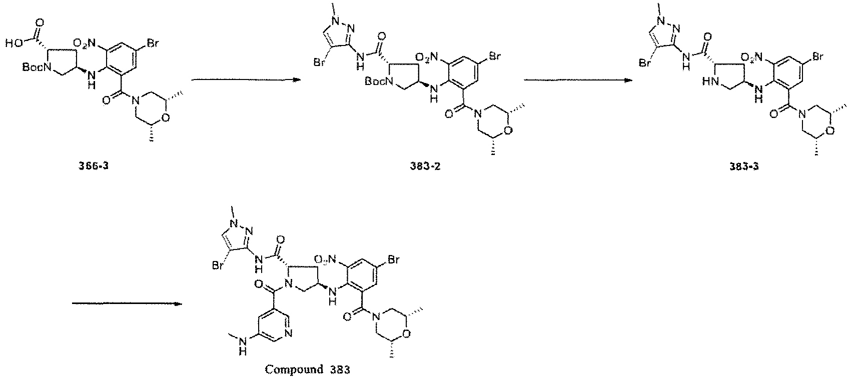

### Operating Steps:

Step A: Compound 366-3 (77 mg, 0.135 mmol) was dissolved in *N*,*N*-dimethylformamide (1 mL) at room temperature under the protection of nitrogen. Subsequently, the above solution was cooled to 0°C, followed by addition of 4-bromo-1-methyl-1*H*-pyrazol-3-amine (23 mg, 0.135 mmol), 2-(7-azabenzotriazolyl)-*N,N,N',N*'-tetramethyluronium hexafluorophosphate (61 mg, 0.16 mmol), and *N*,*N*-diisopropylethylamine (52 mg, 0.40 mmol) in turn. The reaction system was then stirred at 40°C for 3 h.

After the depletion of the raw material as monitored by LCMS, the reaction was quenched by addition of water (25 mL), and the mixture solution was extracted with ethyl acetate (5 mL × 3 times). The organic phases were combined and washed with saturated saline (20 mL × 2 times), then dried with anhydrous sodium sulfate, filtered, and concentrated under reduced pressure. The resulting residue was purified by silica gel column chromatography to obtain Compound 383-2 (45 mg).

MS (ESI) M/Z: 627.8 [M-99]

Step B: Compound 383-2 (45 mg, 0.06 mmol) was dissolved in dichloromethane (0.5 mL) at room temperature under the protection of nitrogen. Subsequently, 4 M of hydrochloric acid-1,4-dioxane solution (0.25 mL) was added to the above solution, and the reaction system was continued to be stirred for 2 h.

After the depletion of the raw material as monitored by LCMS, the reaction solution was concentrated by vacuum distillation. Compound 383-3 (42 mg) was then obtained.

MS (ESI) M/Z: 627.5 [M+H]⁺.

Step C: Compound 383-3 (42 mg, 0.06 mmol) was dissolved in *N,N*-dimethylformamide (0.5 mL) at room temperature under the protection of nitrogen. Subsequently, the above solution was cooled to 0°C, followed by addition of 5-(methylamino)nicotinic acid (10 mg, 0.06 mmol), 2-(7-azabenzotriazolyl)-*N,N,N',N*'-tetramethyluronium hexafluorophosphate (30 mg, 0.08 mmol), and *N,N*-diisopropylethylamine (26 mg, 0.20 mmol) in turn. The reaction system was then continued to be stirred for 30 min.

After the depletion of the raw material as monitored by LCMS, the reaction was quenched by addition of water (15 mL), and the mixture solution was extracted with ethyl acetate (5 mL × 3 times). The organic phases were combined and washed with saturated saline (20 mL × 2 times), then dried with anhydrous sodium sulfate, filtered, and concentrated under reduced pressure. The resulting residue was purified by reversed phase column chromatography to obtain the titled Compound 383 (15.8 mg).

MS (ESI) M/Z: 761.6 [M+H]⁺.

¹H NMR (400 MHz, DMSO-*d*₆) δ 9.98 - 9.50 (m, 1H), 8.34 - 8.14 (m, 1H), 8.06 - 7.94 (m, 1H), 7.92 - 7.46 (m. 4H). 6.95 - 6.67 (m. 1H), 6.25 - 5.99 (m, 1H), 4.81 - 4.46 (m, 1H). 4.45 - 3.94 (m. 2H). 3.92 - 3.37 (m. 8H). 3.28 - 3.00 (m, 1H), 2.98 - 2.63 (m, 4H), 2.43 - 2.10 (in, 2H). 1.18 - 0.83 (m. 6H).

### Example 405

### (5-Bromo-2-((3S,4S)-4-(2-methoxyethoxy)-1-(5-(methylamino)nicotinoyl)pyrrolidin-3-yl)amino)-3-nitrophenyl)((2S,6R)-2,6-dimethylmorpholinyl)methanone

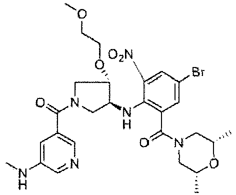

### Reaction route:

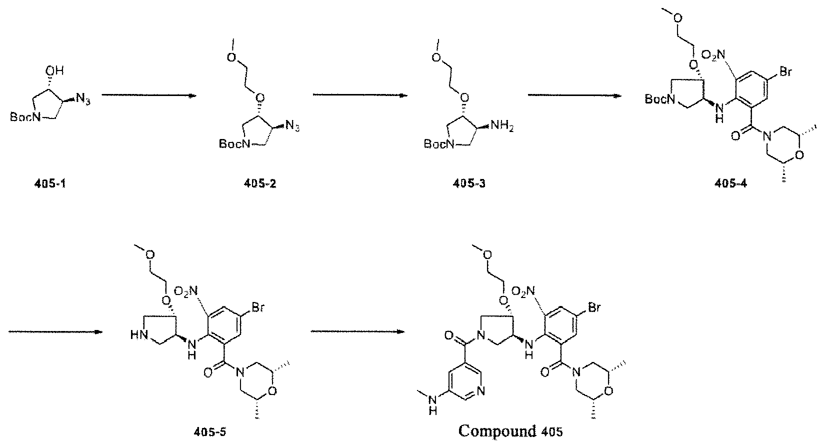

### Operating Steps:

Step A: Compound 405-1 (80 mg, 0.35 mmol) was dissolved in *N*,*N*-dimethylformamide (2 mL) under nitrogen protection at room temperature. Subsequently, the above solution was cooled to 0°C, sodium hydride (28 mg, 0.7 mmol) was slowly added, and the solution was stirred for 30 minutes. Then 1-bromo-2-methoxyethane (72 mg,0.53 mmol) was added to the above solution, and the reaction system was stirred at room temperature for 30 minutes.

After the depletion of the raw material as monitored by LCMS, ice water (35 mL) was added to the reaction solution to quench the reaction. The mixed solution was extracted with ethyl acetate (10 mL × 3 times), and the organic phases were combined. The combined organic phase was washed with saturated saline (15 mL × 2 times), and then dried with anhydrous sodium sulfate, filtered and concentrated under reduced pressure. The resulting residue was purified by silica gel column chromatography to obtain Compound 405-2 (60 mg).

MS (ESI) M/Z: 309.0 [M+23]⁺.

Step B: Compound 405-2 (60 mg, 0.2 mmol) was dissolved in absolute methanol (5 mL) at room temperature. Subsequently, 20%wt platinum dioxide (12 mg) was added to the above solution. Then the reaction system was continued to be stirred for 4 hours under hydrogen protection.

After the depletion of the raw material as monitored by LCMS, the reaction solution was filtered and the filtrate was concentrated under reduced pressure to obtain Compound 405-3 (60 mg, yield 92%).

MS (ESI) M/Z: 161.2 [M-99].

Step C: Compound 405-3 (60 mg, 0.23 mmol) was dissolved in *N*,*N*-dimethylformamide (1 mL) under nitrogen protection at room temperature. Subsequently, (5-bromo-2-fluoro-3-nitrophenyl)((2S,6R)-2,6-dimethylmorpholinyl)methanone (83 mg, 0.23 mmol) and *N*,*N*-diisopropylethylamine (89 mg, 0.69 mmol) were added to the above solution. Then the reaction system was stirred at 80°C for 1 hour.

After the depletion of the raw material as monitored by LCMS, water (20 mL) was added to the reaction solution to quench the reaction. The mixed solution was extracted with ethyl acetate (9 mL × 3 times), and the organic phases were combined. The combined organic phase was washed with saturated saline (10 mL × 2 times), and then dried with anhydrous sodium sulfate, filtered and concentrated under reduced pressure. The resulting residue was purified by silica gel column chromatography to obtain Compound 405-4 (77 mg).

MS (ESI) M/Z: 501.0 [M-99].

Step D: Compound 405-4 (65 mg, 0.11 mmol) was dissolved in dry dichloromethane (0.6 mL) under nitrogen protection at room temperature. Subsequently, the above solution was cooled to 0°C, and 4 M hydrochloric acid-1,4-dioxane solution (0.3 mL) was slowly added. Then the reaction system was stirred at room temperature for 1 hour.

After the depletion of the raw material as monitored by LCMS, the reaction solution was concentrated under reduced pressure to obtain Compound 405-5 (65 mg).

MS (ESI) M/Z: 501.0 [M+H]⁺.

Step E: 5-(methylamino)nicotinic acid (22 mg, 0.15 mmol) was dissolved in *N,N*-dimethylformamide (1 mL) at room temperature. Subsequently, the above solution was cooled to 0°C, and 2-(7-azabenzotriazolyl)-*N*,*N*,*N*',*N*'-tetramethyluronium hexafluorophosphate (68 mg, 0.18 mmol), *N,N*-diisopropylethylamine (77 mg, 0.6 mmol) and Compound 405-5 (65 mg, 0.12 mmol) were successively added. Then the reaction system was continued to be stirred for 30 minutes.

After the depletion of the raw material as monitored by LCMS, water (20 mL) was added to the reaction solution to quench the reaction. The mixed solution was extracted with ethyl acetate (9 mL × 3 times), and the organic phases were combined. The combined organic phase was washed with saturated saline (10 mL × 2 times), and then dried with anhydrous sodium sulfate, filtered and concentrated under reduced pressure. The resulting residue was purified by preparative high performance liquid chromatography to obtain the title Compound 405 (31.68 mg).

MS (ESI) M/Z: 635.0 [M+H]⁺.

¹H NMR (400 MHz, DMSO-*d₆*) δ 8.31 - 8.17 (m, 1H), 8.01 (brs, 1H), 7.91 - 7.30 (m, 3H), 6.97 - 6.78 (m, 1H), 6.23 - 6.06 (m. 1H), 4.52 - 3.47 (m, 12H), 3.29 - 3.01 (m, 5H), 2.97 - 2.65 (m, 4H). 2.61 - 2.53 (m, 0.5H), 2.47 -2.38 (m. 0.5H), 1.19 - 0.88 (m, 6H).

### Example 408

### 2-(3S,4S)-1-(3H-imidazo[4,5-c]pyridin-7-carbonyl)-4-methoxypyrrolidin-3-ylamino)-5-bromo-3-nitrophenyl)((2S,6R)-2,6-dimethylmorpholinyl)methanone

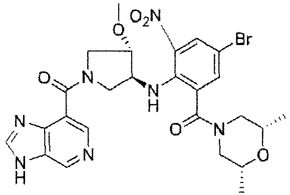

### Reaction route:

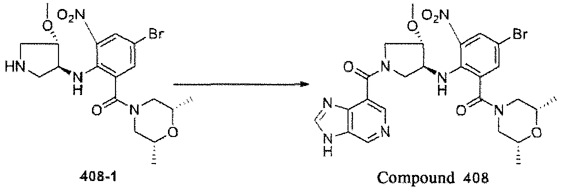

### Operating Steps:

Step A: 3*H*-imidazo[4,5-*C*]pyridin-7-carboxylic acid (8 mg, 0.04 mmol) was dissolved in *N,N*-dimethylformamide (0.5 mL) under nitrogen protection at room temperature. Subsequently, *N,N*-diisopropylethylamine (0.03 mL, 0.16 mmol), 2-(7-azabenzotriazolyl)-*N,N,N',N'-*tetramethyluronium hexafluorophosphate (19 mg, 0.05 mmol) and Compound 408-1 (18.2 mg, 0.04 mmol) were successively added to the above solution. Then the reaction system was continued to be stirred for 20 minutes.

After the depletion of the raw material as monitored by LCMS, water (6 mL) was added to the reaction solution to quench the reaction, the mixed solution was extracted with ethyl acetate (2 mL × 3 times), and the organic phases were combined. The combined organic phase was washed with saturated sodium chloride solution (6 mL × 2 times), then dried with anhydrous sodium sulfate, filtered and concentrated under reduced pressure. The crude product was purified by preparative high performance liquid chromatography to obtain the title Compound 408 (4.01 mg).

MS (ESI) M/Z: 601.9 [M+H]⁺.

¹H NMR (400 MHz, DMSO*-d*₆) δ 13.13 (s. 1H), 9.02 (s. 1H), 8.65 - 8.09 (m, 3H), 7.85 - 7.19 (m, 2H), 4.55 - 3.40 (m, 9H), 3.29 - 3.01 (m, 4H). 2.99 - 2.73 (m, 1H). 2.60 - 2.52 (m. 0.5H), 2.42 - 2.25 (m, 0.5H), 1.23 - 0.68 (m, 6H).

### Example 418

### (2S,4R)-4-((4-bromo-2-((2R,6S)-2,6-dimethylmorpholin-4-carbonyl)-6-nitrophenyl)amino)-1-(5-(methylamino)nicotinoyl)-N-(4-bromo-1-(2-methoxyethyl)-1H-pyrazol-3-yl)pyrrolidine-2-formamide

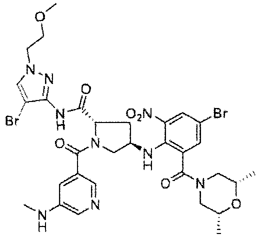

### Reaction route:

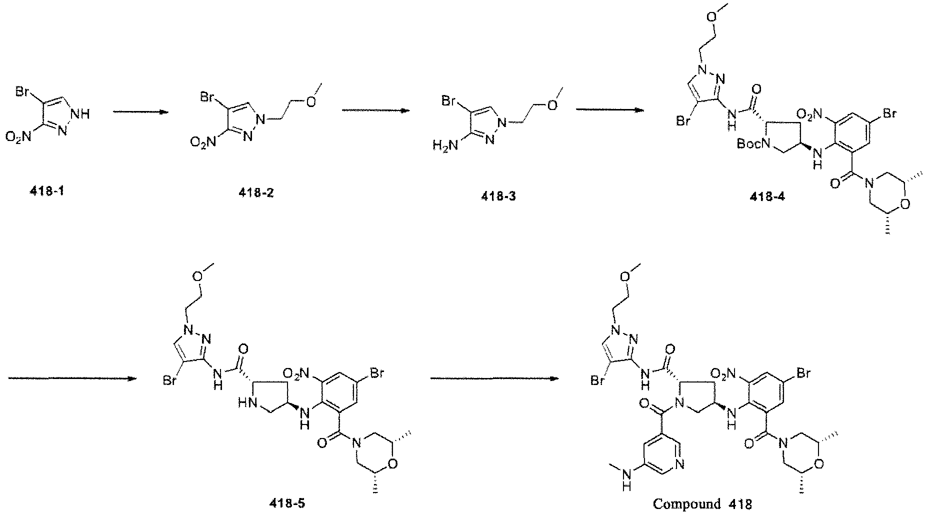

### Operating Steps:

Step A: Compound 418-1 (40 mg, 0.21 mmol) was dissolved in *N*,*N*-dimethylformamide (1 mL) under nitrogen protection at room temperature. Subsequently, 1-bromo-2-methoxyethane (35 mg, 0.25 mmol) and potassium carbonate (145 mg, 1.05 mmol) were successively added to the above solution. Then the reaction system was stirred at 50°C for 1 hour.

After the depletion of the raw material as monitored by LCMS, water (10 mL) was added to the reaction solution to quench the reaction, the mixed solution was extracted with ethyl acetate (15 mL × 3 times), and the organic phases were combined. The combined organic phase was washed with saturated saline (60 mL × 2 times), then dried with anhydrous sodium sulfate, filtered and concentrated under reduced pressure. The resulting residue was purified by silica gel column chromatography to obtain Compound 418-2 (42 mg).

MS (ESI) M/Z: 250.0 [M+H]⁺.

Step B: Compound 418-2 (42 mg, 0.17 mmol) was dissolved in ethanol (0.6 mL) and water (0.3 mL) under nitrogen protection at room temperature. Subsequently, iron powder (48 mg, 0.85 mmol) and ammonium chloride solid (46 mg, 0.85 mmol) were successively added to the above solution. Then the reaction system was stirred at 80°C for 1 hour.

After the depletion of the raw material as monitored by LCMS, the reaction solution was filtered while hot, and the filtrate was concentrated under reduced pressure. Water (5 mL) was added to the resulting residue. The mixed solution was extracted with ethyl acetate (15 mL × 3 times), and the organic phases were combined. The combined organic phase was washed with saturated saline (30 mL × 2 times), then dried with anhydrous sodium sulfate, filtered and concentrated under reduced pressure. Compound 418-3 (42 mg) was obtained.

MS (ESI) M/Z: 220.0 [M+H]⁺.

Step C: (2*S*,4*R*)-4-((4-bromo-2-((2*R*,6*S*)-2,6-dimethylmorpholin-4-carbonyl)-6-nitrophenyl)amino)-1-(tert-butoxycarbonyl)pyrrolidine-2-carboxylic acid (50 mg, 0.09 mmol) was dissolved in *N,N*-dimethylformamide (0.5 mL) under nitrogen protection at room temperature. Subsequently, the above solution was cooled to 0°C, and *N,N-*diisopropylethylamine (57 mg, 0.44 mmol), 2-(7-azabenzotriazolyl)-*N,N,N',N*'-tetramethyluronium hexafluorophosphate (40 mg, 0.11 mmol) and Compound 418-3 (23 mg, 0.11 mmol) were successively added. Then the reaction system was stirred at room temperature for 8 hours.

After the depletion of the raw material as monitored by LCMS, water (10 mL) was added to the reaction solution to quench the reaction. The mixed solution was extracted with ethyl acetate (15 mL × 3 times), and the organic phases were combined. The combined organic phase was washed with saturated saline (30 mL × 2 times), then dried with anhydrous sodium sulfate, filtered and concentrated under reduced pressure. The resulting residue was purified by silica gel column chromatography to obtain Compound 418-4 (45 mg).

MS (ESI) M/Z: 671.8 [M-99].

Step D: Compound 418-4 (45 mg, 0.06 mmol) was dissolved in dichloromethane (0.6 mL) at room temperature. Subsequently, the above solution was cooled to 0°C, and 4 M hydrochloric acid-1,4-dioxane solution (0.6 mL) was added. Then the reaction system was stirred at room temperature for 30 minutes.

After the depletion of the raw material as monitored by LCMS, the reaction solution was distilled and concentrated under reduced pressure. Compound 418-5 (46 mg) was obtained.

MS (ESI) M/Z: 671.8 [M+H]⁺.

Step E: Compound 5-(methylamino)nicotinic acid (13 mg, 0.08 mmol) was dissolved in *N,N*-dimethylformamide (0.5 mL) at room temperature. Subsequently, the above solution was cooled to 0°C, and *N,N*-diisopropylethylamine (45 mg, 0.35 mmol), 2-(7-azabenzotriazolyl)-*N,N,N',N*'-tetramethyluronium hexafluorophosphate (32 mg, 0.08 mmol) and Compound 418-5 (46 mg, 0.07 mmol) were successively added. Then the reaction system was continued to be stirred for 1 hour.

After the depletion of the raw material as monitored by LCMS, water (10 mL) was added to the reaction solution to quench the reaction, the mixed solution was extracted with ethyl acetate (15 mL × 3 times), and the organic phases were combined. The combined organic phase was washed with saturated saline (60 mL × 2 times), then dried with anhydrous sodium sulfate, filtered and concentrated under reduced pressure. The resulting residue was purified by preparative high performance liquid chromatography to obtain the title Compound 418 (16.19 mg).

MS (ESI) M/Z: 805.4 [M+H]⁺.

¹H NMR (400 MHz, DMSO-*d*₆) δ 10.08 - 9.54 (m. 1H), 8.34 - 8.12 (m, 1H), 8.04 - 7.46 (m, 5H), 6.97 - 6.63 (m, 1H), 6.26 - 6.00 (m, 1H), 4.76 - 4.46 (in, 1H), 4.42 - 3.35 (in, 11H), 3.30 - 3.00 (m, 4H), 2.96 - 2.75 (m, 1H), 2.74 - 2.63 (m, 3H), 2.46 - 2.35 (m, 1H). 2.31 - 2.10 (m, 1H), 1.19 - 0.82 (m, 6H).

### Example 419

### (2S,4R)-4-(4-bromo-2-((2R,6S)-2,6-dimethylmorpholin-4-carbonyl)-6-nitrophenyl)amino)-1-(5-(methylamino)nicotinoyl)-N-(2-(trifluoromethyl)pyridin-4-yl)methyl)pyrrolidine-2-formamide

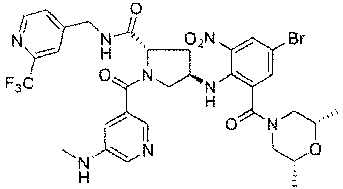

### Reaction route:

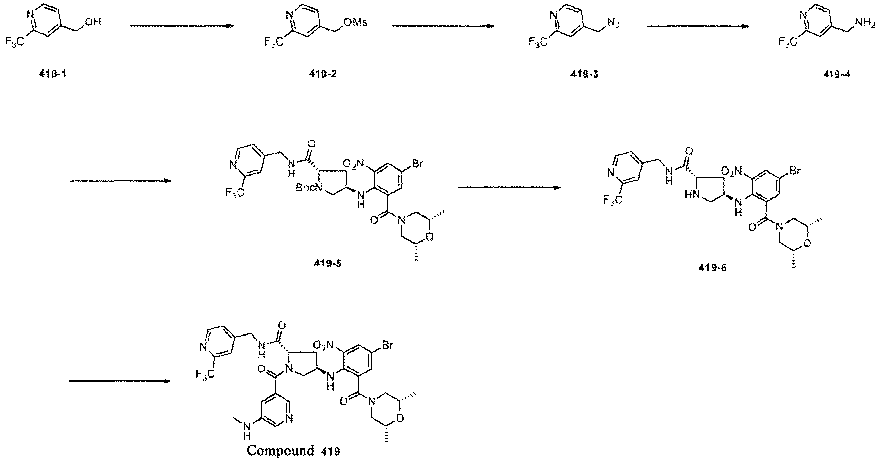

### Operating Steps:

Step A: Compound 419-1 (100 mg, 0.564 mmol) was dissolved in dichloromethane (5 mL) under nitrogen protection at room temperature. Subsequently, the above solution was cooled to 0°C, and triethylamine (114.26 mg, 1.13 mmol) and methanesulfonyl chloride (77.6 mg, 0.677 mmol) were successively added. Then the reaction system was stirred at room temperature for 2 hours.

After the depletion of the raw material as monitored by LCMS, aqueous ammonium chloride solution (20 mL) was added to the reaction solution to quench the reaction. The mixed solution was extracted with dichloromethane (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 419-2 (150 mg).

MS (ESI) M/Z: 256.2 [M+H]⁺.

Step B: Compound 419-2 (140 mg, 0.548 mmol) was dissolved in *N,N*-dimethylformamide (1 mL) under nitrogen protection at room temperature. Subsequently, sodium azide (53.49 mg, 0.822 mmol) was added to the above reaction solution. Then the reaction system was warmed to 80°C and stirred for 4 hours.

After the depletion of the raw material as monitored by LCMS, water (15 mL) was added to the reaction solution to quench the reaction, the mixed solution was extracted with ethyl acetate (5 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 419-3 (100 mg).

MS (ESI) M/Z: 203.1 [M+H]⁺.

Step C: Compound 419-3 (30 mg, 0.148 mmol) was dissolved in tetrahydrofuran (2 mL) under hydrogen protection at room temperature. Subsequently, 10% Palladium/Carbon (10 mg) was added to the above reaction solution. Then the reaction system was stirred at room temperature for 6 hours.

After the depletion of the raw material as monitored by LCMS, the reaction solution was filtered and the filtrate was concentrated under reduced pressure. Compound 419-4 (30 mg) was obtained.

MS (ESI) M/Z: 176.2 [M+H]⁺.

Step D: Compound (2*S*,*4R*)-4-(4-bromo-2-((2*R*,6*S*)-2,6-dimethylmorpholin-4-carbonyl)-6-nitrophenyl)amino)-1-(tert-butoxycarbonyl)pyrrolidine-2-carboxylic acid (60 mg, 0.105 mmol) was dissolved in *N,N*-dimethylformamide (1 mL) at room temperature. Subsequently, the above solution was cooled to 0°C, and Compound 419-4 (18.5 mg, 0.105 mmol), 2-(7-azabenzotriazolyl)-*N*,*N*,*N*',*N*'-tetramethyluronium hexafluorophosphate (59.73 mg, 0.157 mmol) and *N,N-*diisopropylethylamine (54.28 mg, 0.42 mmol) were successively added. Then the reaction system was continued to be stirred for 30 minutes.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 419-5 (70 mg).

MS (ESI) M/Z: 753.3 [M+23]⁺.

Step E: Compound 419-5 (70 mg, 0.096 mmol) was dissolved in dichloromethane (5 mL) under nitrogen protection at room temperature. Subsequently, 4 M hydrochloric acid-1,4-dioxane solution (2 mL) was slowly added dropwise to the solution. Then the reaction system was continued to be stirred for 1 hour.

After the depletion of the raw material as monitored by LCMS, the reaction solution was concentrated under reduced pressure. Compound 419-6 (60 mg, yield 99.35%) was obtained.

Step F: Compound 419-6 (80 mg, 0.127 mmol) was dissolved in *N,N*-dimethylformamide (1 mL) at room temperature. Subsequently, the above solution was cooled to 0°C, and 5-(methylamino)nicotinic acid (19.34 mg, 0.127 mmol), 2-(7-azabenzotriazolyl)-*N,N,N',N'-*tetramethyluronium hexafluorophosphate (72.3 mg, 0.190 mmol) and *N,N-*diisopropylethylamine (82.14 mg, 0.635mmol) were successively added. Then the reaction system was continued to be stirred for 30 minutes.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by reversed phase column chromatography to obtain the title Compound 419 (27.5 mg).

MS (ESI) M/Z: 764.2 [M+H]⁺.

¹H NMR (400 MHz, DMSO-*d₆*) δ 8.87 - 8.75 (m, 0.8H), 8.69 (d, *J* = 4.8 Hz, 0.8H), 8.63 (d, *J* = 4.8 Hz, 0.2H), 8.57 - 8.48 (m, 0.2H), 8.26 (s, 0.2H), 8.21 (d, *J* = 2.0 Hz, 0.8H), 8.05 - 7.94 (in, 1H), 7.90 - 7.53 (m, 4.8H), 7.16 - 7.03 (m, 0.2H), 6.91 - 6.80 (m, 0.8H), 6.71 (s. 0.2H), 6.24 - 6.08 (m, 1H), 4.76 - 3.95 (m, 5H), 3.93 - 3.72 (m, 1H), 3.70 - 3.40 (m. 3H). 3.26 - 3.10 (s. 1H), 2.90 - 2.64 (m, 4H), 2.48 - 2.14 (m, 3H), 1.16 - 0.86 (m, 6H).

### Example 420

### (2S,4R)-N-(4-bromo-1-cyclopropyl-1H-pyrazol-3-yl)-4-(4-bromo-2-(2R,6S)-2,6-dimethylmorpholin-4-carbonyl)-6-nitrophenyl)amino)-1-(5-(methylamino)nicotinoyl)pyrrolidine-2-formamide

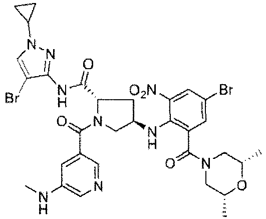

### Reaction route:

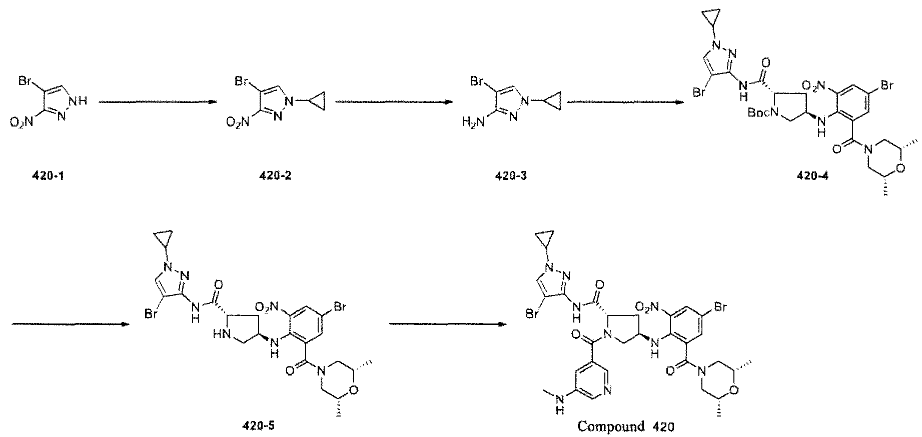

### Operating Steps:

Step A: Compound 420-1 (525 mg, 2.73 mmol), cyclopropylboronic acid (258 mg, 3.0 mmol), pyridine (216 mg, 2.73 mmol) and sodium carbonate (435 mg, 4.1 mmol) were dissolved in dry dioxane (14 mL) under the protection of oxygen at room temperature, and the solution was continued to be stirred for 0.5 h. Subsequently, cupric acetate (498 mg, 2.73 mmol) was added to the above solution and the reaction system was stirred at 70°C for 16 hours.

After the depletion of the raw material as monitored by LCMS, the reaction solution was filtered and the filtrate was concentrated under reduced pressure. The resulting residue was purified by silica gel column chromatography to obtain Compound 420-2 (56 mg).

MS (ESI) M/Z: 232.0 [M+H]⁺.

Step B: Compound 420-2 (56 mg, 0.24 mmol) was dissolved in ethanol (2 mL) and water (2 mL) under nitrogen protection at room temperature. Subsequently, iron powder (68 mg, 1.2 mmol) and ammonium chloride (65 mg, 1.2 mmol) were successively added to the above solution. Then the reaction system was stirred at 70°C for 4 hours.

After the depletion of the raw material as monitored by LCMS, the reaction solution was filtered and the filtrate was concentrated under reduced pressure to obtain Compound 420-3 (45 mg).

MS (ESI) M/Z: 202.0 [M+H]⁺.

Step C: (2*S*,4*R*)-4-(4-bromo-2-(2*R*,6*S*)-2,6-dimethylmorpholin-4-carbonyl)-6-nitrophenyl)amino)-1-(tert-butoxycarbonyl)pyrrolidine-2-carboxylic acid (376 mg, 0.66 mmol) was dissolved in dry dichloromethane (6.5 mL) under nitrogen protection at room temperature. Subsequently, pyridine (43 mg, 0.55 mmol) and thionyl chloride (31 mg, 0.26 mmol) were successively added to the above solution, and the solution was continued to be stirred for 2 hours. Then Compound 420-3 (45 mg, 0.22 mmol) and triethylamine (67 mg, 0.66 mmol) were successively added to the above solution, and the reaction system was stirred for 1 hour.

After the depletion of the raw material as monitored by LCMS, ice water (20 mL) was added to the reaction solution to quench the reaction, the mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 420-4 (24 mg).

MS (ESI) M/Z: 653.8 [M-99].

Step D: Compound 420-4 (24 mg, 0.03 mmol) was dissolved in dry dichloromethane (0.2 mL) under nitrogen protection at room temperature. Subsequently, the above solution was cooled to 0°C, and 4 M hydrochloric acid-1,4-dioxane solution (0.1 mL) was slowly added. Then the reaction system was stirred at room temperature for 2 hours.

After the depletion of the raw material as monitored by LCMS, the reaction solution was concentrated under reduced pressure to obtain Compound 420-5 (24 mg).

MS (ESI) M/Z: 653.8 [M+H]⁺.

Step E: 5-(methylamino)nicotinic acid (7 mg, 0.05 mmol) was dissolved in *N*,*N*-dimethylformamide (0.5 mL) at room temperature. Subsequently, the above solution was cooled to 0°C, and 2-(7-azabenzotriazolyl)-*N,N,N',N'-*tetramethyluronium hexafluorophosphate (23 mg, 0.06 mmol), *N,N*-diisopropylethylamine (26 mg, 0.2 mmol) and Compound 420-5 (24 mg, 0.04 mmol) were successively added. Then the reaction system was continued to be stirred for 30 minutes.

After the depletion of the raw material as monitored by LCMS, ice water (20 mL) was added to the reaction solution to quench the reaction. The mixed solution was extracted with ethyl acetate (9 mL × 3 times), and the organic phases were combined. The combined organic phase was washed with saturated saline (10 mL × 2 times), then dried with anhydrous sodium sulfate, filtered and concentrated under reduced pressure. The resulting residue was purified by preparative high performance liquid chromatography to obtain the title Compound 420 (11.16 mg).

MS (ESI) M/Z: 787.4 [M+H]⁺.

¹H NMR (400 MHz, DMSO-*d₆*) δ 10.03 - 9.59 (m, 1H), 8.32 - 8.14 (m. 1H), 8.10 - 7.49 (m. 5H), 6.97 - 6.61 (m, 1H), 6.26 - 5.84 (m. 1H), 4.79 - 4.46 (m, 1H), 4.43 - 3.39 (m, 8H), 3.29 - 3.07 (m, 1H), 2.91 - 2.66 (m. 4H), 2.46 - 2.37 (m, 1H), 2.31 - 2.12 (m, 1H), 1.20 - 0.81 (m, 10H).

### Example 422

### (5-Bromo-2-(3R,5R)-5-methoxy-1-(5-(methylamino)nicotinoyl)piperidin-3-yl)amino)-3-nitrophenyl)((2S,6R)-2,6-dimethylmorpholinyl)methanone

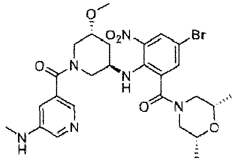

### Reaction route:

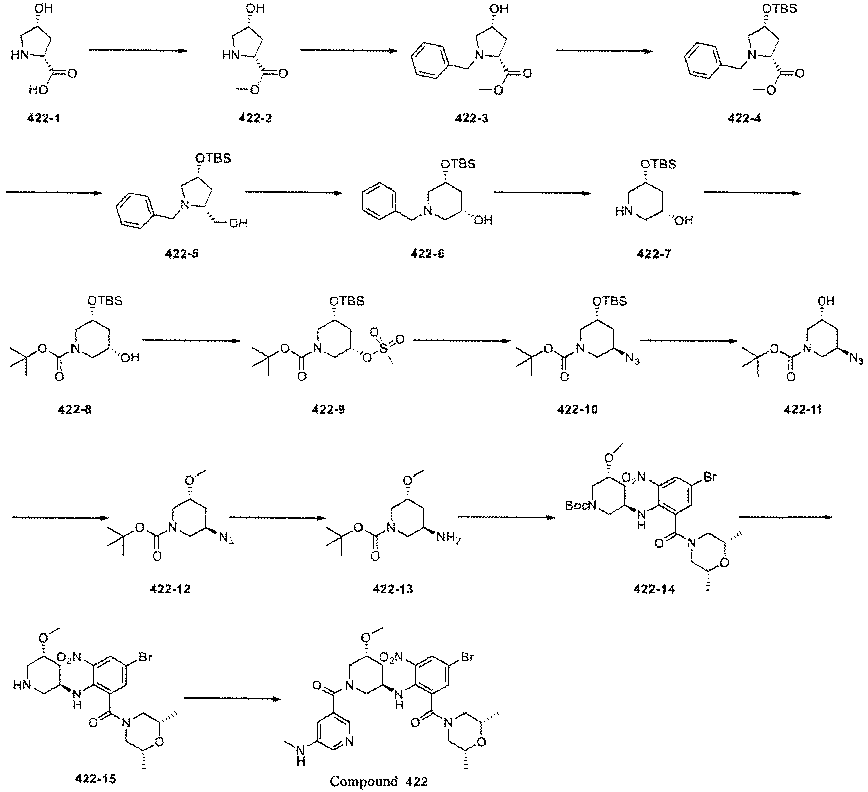

### Operating Steps:

Step A: Compound 422-1 (25 g, 191 mmol) was dissolved in methanol (900 mL) at 0°C. Subsequently, thionyl chloride (80 g, 669 mmol) was slowly added dropwise to the above solution. Then the reaction system was stirred at room temperature for 16 hours.

After the depletion of the raw material as monitored by LCMS, the reaction solution was concentrated under reduced pressure to obtain Compound 422-2 (27 g, yield 97.5%).

MS (ESI) M/Z: 146.0 [M+H]⁺.

Step B: Compound 422-2 (27 g, 186.2 mmol) was dissolved in dichloromethane (480 mL) at room temperature. Subsequently, the above solution was cooled to 0°C, and triethylamine (56.3 g, 558.6 mmol) and benzyl bromide (37.7 g, 223.4 mmol) were successively added. Then the reaction system was stirred at 50°C for 16 hours.

After the depletion of the raw material as monitored by LCMS, the reaction solution was concentrated under reduced pressure, and water (200 mL) was added to the resulting residue to quench the reaction. The mixed solution was extracted with ethyl acetate (200 mL × 3 times), and the organic phases were combined. The combined organic phase was washed with saturated saline (200 mL × 2 times), then dried with anhydrous sodium sulfate, filtered and concentrated under reduced pressure. The resulting residue was purified by silica gel column chromatography to obtain Compound 422-3 (31 g, yield 70.9%).

MS (ESI) M/Z: 236.0 [M+H]⁺.

Step C: Compound 422-3 (31 g, 131.9 mmol) was dissolved in dichloromethane (500 mL) under nitrogen protection at room temperature. Subsequently, the above solution was cooled to 0°C, and triethylamine (39.9 g, 395.7 mmol), tertbutyldimethylsilyl chloride (23.9 g, 158.3 mmol) and 4-dimethylaminopyridine (158.6 mg, 1.3 mmol) were successively added. Then the reaction system was stirred at 50°C for 16 hours.

After the depletion of the raw material as monitored by LCMS, the reaction solution was concentrated under reduced pressure, and water (250 mL) was added to the resulting residue to quench the reaction. The mixed solution was extracted with ethyl acetate (250 mL × 3 times), and the organic phases were combined. The combined organic phase was washed with saturated saline (200 mL × 2 times), then dried with anhydrous sodium sulfate, filtered and concentrated under reduced pressure. The resulting residue was purified by silica gel column chromatography to obtain Compound 422-4 (21 g, yield 45.5%).

MS (ESI) M/Z: 350.0 [M+H]⁺.

Step D: Compound 422-4 (21 g, 60.0 mmol) was dissolved in anhydrous tetrahydrofuran (180 mL) under nitrogen protection at room temperature. Subsequently, the above solution was cooled to 0°C, and lithium borohydride (3.27 g, 150.0 mmol) was slowly added. Then the reaction system was stirred at room temperature for 16 hours.

After the depletion of the raw material as monitored by LCMS, the reaction solution was slowly added to ice water (150 mL) to quench the reaction. The mixed solution was extracted with dichloromethane (150 mL × 3 times), and the organic phases were combined. The combined organic phase was washed with saturated saline (100 mL × 2 times), then dried with anhydrous sodium sulfate, filtered and concentrated under reduced pressure. The resulting residue was purified by silica gel column chromatography to obtain Compound 422-5 (14 g, yield 72.7%).

MS (ESI) M/Z: 322.0 [M+H]⁺.

Step E: Compound 422-5 (14 g, 43.6 mmol) was dissolved in anhydrous tetrahydrofuran (150 mL) under nitrogen protection at room temperature. Subsequently, the above solution was cooled to 0°C, and triethylamine (13.2 g, 130.8 mmol) and trifluoroacetic anhydride (13.7 g, 65.4 mmol) were successively added. Then the reaction system was stirred at 90°C for 16 hours.

After the depletion of the raw material as monitored by LCMS, the reaction solution was slowly added to ice water (150 mL) to quench the reaction. The mixed solution was extracted with dichloromethane (150 mL × 3 times), and the organic phases were combined. The combined organic phase was washed with saturated saline (100 mL × 2 times), then dried with anhydrous sodium sulfate, filtered and concentrated under reduced pressure. The resulting residue was purified by silica gel column chromatography to obtain Compound 422-6 (7.3 g, yield 52.1%).

MS (ESI) M/Z: 322.0 [M+H]⁺.

Step F: Compound 422-6 (850 mg, 2.64 mmol) was dissolved in anhydrous ethanol (13 mL) at room temperature. Subsequently, 10% Palladium/Carbon (85 mg) was added to the above solution. Then the reaction system was subjected to nitrogen replacement for three times, and continued to be stirred for 3 hours.

After the depletion of the raw material as monitored by LCMS, the reaction solution was filtered and concentrated under reduced pressure to obtain Compound 422-7 as crude product (610 mg).

MS (ESI) M/Z: 232.2 [M+H]⁺.

Step G: Compound 422-7 (610 mg, 2.64 mmol) was dissolved in dichloromethane (13 mL) under nitrogen protection at room temperature. Subsequently, the above solution was cooled to 0°C, and triethylamine (799.9 mg, 7.9 mmol) and di-tert-butyl dicarbonate (863.3 g, 3.96 mmol) were successively added. Then the reaction system was stirred at room temperature for 16 hours.

After the depletion of the raw material as monitored by LCMS, the reaction solution was slowly added to ice water (15 mL) to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 422-8 (600 mg, yield 68.6%).

MS (ESI) M/Z: 354.0 [M+23].

Step H: Compound 422-8 (600 mg, 1.81 mmol) was dissolved in dichloromethane (9 mL) under nitrogen protection at room temperature. Subsequently, the above solution was cooled to 0°C, and triethylamine (548.4 mg, 5.4 mmol) and methanesulfonyl chloride (310.0 mg, 2.71 mmol) were successively added. Then the reaction system was stirred at room temperature for 3 hours.

After the depletion of the raw material as monitored by LCMS, the reaction solution was slowly added to ice water (10 mL) to quench the reaction. The mixed solution was extracted with ethyl acetate (6 mL × 3 times), and the organic phases were combined. The combined organic phase was washed with saturated saline (10 mL × 2 times), then dried with anhydrous sodium sulfate, filtered and concentrated under reduced pressure to obtain Compound 422-9 (700 mg, yield 95.1%).

Step I: Compound 422-9 (700 mg, 1.71 mmol) was dissolved in anhydrous *N,N*-dimethylformamide (8 mL) under nitrogen protection at room temperature. Subsequently, sodium azide (330.4 mg, 5.1 mmol) was added to the above solution. Then the reaction system was stirred at 70°C for 16 hours.

After the depletion of the raw material as monitored by LCMS, water (25 mL) was slowly added to the reaction solution to quench the reaction. The mixed solution was extracted with ethyl acetate (10 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 422-10 (320 mg, yield 52.6%).

MS (ESI) M/Z: 257.2 [M-99].

Step J: Compound 422-10 (320 mg, 0.90 mmol) was dissolved in anhydrous tetrahydrofuran (10 mL) at room temperature. Subsequently, the above solution was cooled to 0°C, and 1 M tetrabutylammonium fluoride in tetrahydrofuran solution (0.99 mL, 0.99 mmol) was slowly added. Then the reaction system was stirred at room temperature for 4 hours.

After the depletion of the raw material as monitored by LCMS, water (15 mL) was added to the reaction solution to quench the reaction. The mixed solution was extracted with dichloromethane (15 mL × 3 times), and the organic phases were combined. The combined organic phase was washed with saturated saline (20 mL × 2 times), then dried with anhydrous sodium sulfate, and filtered. The resulting residue was purified by silica gel column chromatography to obtain Compound 422-11 (150 mg, yield 68.8%).

MS (ESI) M/Z: 143.2 [M-99].

Step K: Compound 422-11 (150 mg, 0.62 mmol) was dissolved in methyl iodide (3 mL) at room temperature. Subsequently, silver oxide (214.8 mg, 0.93 mmol) was added to the above solution. Then the reaction system was stirred at 60°C for 16 hours.

After the depletion of the raw material as monitored by LCMS, the reaction solution was filtered and the filtrate was concentrated under reduced pressure to obtain Compound 422-12 (130 mg, yield 81.9%).

MS (ESI) M/Z: 157.2 [M-99].

Step L: Compound 422-12 (130 mg, 0.51 mmol) was dissolved in absolute methanol (5 mL) at room temperature. Subsequently, 10% Palladium/Carbon (25 mg) was added to the above solution, then the reaction system was subjected to nitrogen replacement for three times, and continued to be stirred for 3 hours.

After the depletion of the raw material as monitored by LCMS, the reaction solution was filtered and concentrated under reduced pressure. Compound 422-13 crude product (130 mg) was obtained.

MS (ESI) M/Z: 231.2 [M+H]⁺.

Step M: Compound 422-13 (130 mg, 0.56 mmol) was dissolved in *N,N*-dimethylformamide (2.5 mL) under nitrogen protection at room temperature. Subsequently, (5-bromo-2-fluoro-3-nitrophenyl)((2*S*,6*R*)-2,6-dimethylmorpholinyl)methanone (201 mg, 0.56 mmol) and *N,N*-diisopropylethylamine (216.7 mg, 1.68 mmol) were successively added to the above solution. Then the reaction system was stirred at 80°C for 12 hours.

After the depletion of the raw material as monitored by LCMS, water (20 mL) was added to the reaction solution to quench the reaction. The mixed solution was extracted with ethyl acetate (8 mL × 3 times), and the organic phases were combined. The combined organic phase was washed with saturated sodium chloride solution (15 mL × 2 times), then dried with anhydrous sodium sulfate, filtered and concentrated under reduced pressure. The resulting residue was purified by silica gel column chromatography to obtain Compound 422-14 (95 mg, yield 29.7%).

MS (ESI) M/Z: 471.2 [M-99].

Step N: Compound 422-14 (95 mg, 0.16 mmol) was dissolved in 4 M hydrochloric acid-1,4-dioxane solution (0.4 mL) and dichloromethane (0.8 mL) under nitrogen protection at room temperature. Then the reaction system was stirred for 2 hours.

After the depletion of the raw material as monitored by LCMS, the reaction solution was distilled and concentrated under reduced pressure. Compound 422-15 crude product (78 mg) was obtained.

MS (ESI) M/Z: 471.0 [M+H]⁺.

Step O: Compound 422-15 (78 mg, 0.16 mmol) was dissolved in *N*,*N*-dimethylformamide (1 mL) at room temperature. Subsequently, the above solution was cooled to 0°C, and 5-(methylamino)nicotinic acid (24 mg, 0.16 mmol), 2-(7-azabenzotriazolyl)-*N,N,N',N'-*tetramethyluronium hexafluorophosphate (68.4 mg, 0.18 mmol) and *N,N-*diisopropylethylamine (61.9 mg, 0.48 mmol) were successively added. Then the reaction system was continued to be stirred for 15 minutes.

After the depletion of the raw material as monitored by LCMS, water (15 mL) was added to the reaction solution to quench the reaction. The mixed solution was extracted with ethyl acetate (5 mL × 3 times), and the organic phases were combined. The combined organic phase was washed with saturated saline (20 mL × 2 times), then dried with anhydrous sodium sulfate, filtered and concentrated under reduced pressure. The resulting residue was purified by reversed phase column chromatography to obtain the title Compound 422 (30.2 mg, yield 31.2%).

MS (ESI) M/Z: 605.2 [M+H]⁺.

¹H NMR (400 MHz, DMSO-*d*₆) δ 8.23 (s, 1H), 7.98 (s, 1H), 7.78 - 7.35 (m, 3H). 6.77 (d, *J* = 16.8 Hz, 1H), 6.15 (s, 1H), 4.64 - 3.98 (m, 2H), 3.92 - 3.36 (m. 6H), 3.30 - 2.80 (m, 6H). 2.70 (d. .7= 4.8 Hz, 3H), 2.47 - 1.63 (m, 3H), 1.28 - 0.87 (m, 6H).

### Example 424

### (2S,4R)-N-(4-bromo-1-(pyridin-3-yl)-1H-pyrazol-3-yl)-4-(4-bromo-2-((2R,6S)-2,6-dimethylmorpholin-4-carbonyl)-6-nitrophenyl)amino))-1-(5-(methylamino)nicotinoyl)pyrrolidine-2-formamide

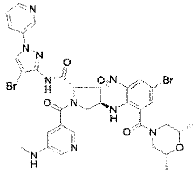

### Reaction route:

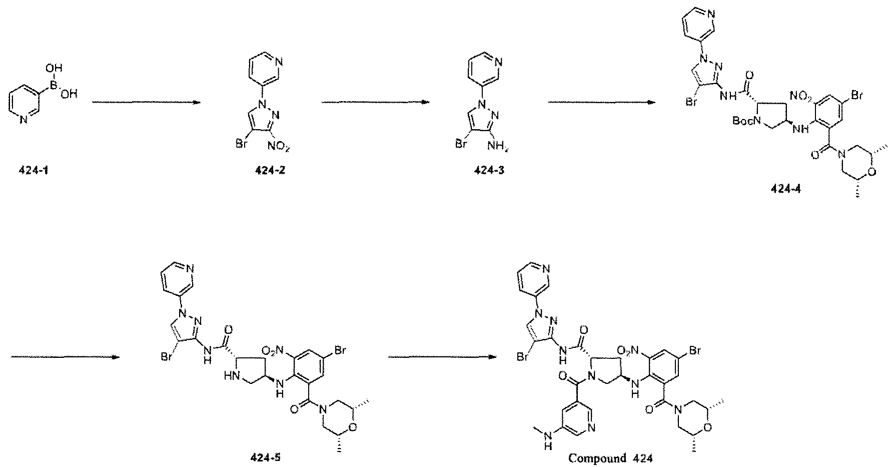

### Operating Steps:

Step A: Compound 424-1 (213 mg, 1.73 mmol), 4-bromo-3-nitro-1*H*-pyrazole (300 mg, 1.57 mmol), sodium carbonate (250 mg, 2.36 mmol) and pyridine (124 mg, 1.57 mmol) were dissolved in 1,4-dioxane (8 mL) at room temperature, and the solution was stirred for 30 minutes. Then cupric acetate (286 mg, 1.57 mmol) was added to the above solution, and the reaction system was stirred at 70°C for 16 hours.

After the depletion of the raw material as monitored by LCMS, water (10 mL) was added to the reaction solution to quench the reaction. The mixed solution was extracted with ethyl acetate (15 mL × 3 times), and the organic phases were combined. The combined organic phase was washed with saturated saline (60 mL × 2 times), then dried with anhydrous sodium sulfate, filtered and concentrated under reduced pressure. The resulting mixture was purified by silica gel column chromatography to obtain Compound 424-2 (110 mg).

MS (ESI) M/Z: 269.0 [M+H]⁺.

Step B: Compound 424-2 (110 mg, 0.41 mmol), iron powder (115 mg, 2.05 mmol) and ammonium chloride solid (110 mg, 2.05 mmol) were dissolved in EtOH/H₂O (v/v, 1/1, 2.1 mL) under nitrogen protection at room temperature. Then the reaction solution was stirred at 90°C for 1 hour.

After the depletion of the raw material as monitored by LCMS, water (10 mL) was added to the reaction solution to quench the reaction. The mixed solution was extracted with ethyl acetate (15 mL × 3 times), and the organic phases were combined. The combined organic phase was washed with saturated saline (60 mL × 2 times), then dried with anhydrous sodium sulfate, filtered and concentrated under reduced pressure to obtain Compound 424-3 (80 mg).

MS (ESI) M/Z: 239.0 [M+H]⁺.

Step C: Compound (2*S*,4*R*)-4-(4-bromo-2-(2*R*,6*S*)-2,6-dimethylmorpholin-4-carbonyl)-6-nitrophenyl)amino)-1-(tert-butoxycarbonyl)pyrrolidine-2-carboxylic acid (80 mg, 0.14 mmol), pyridine (28 mg, 0.35 mmol) and thionyl chloride (20 mg, 0.17 mmol) were dissolved in dichloromethane (0.7 mL) at room temperature, and the solution was stirred for 1 hour. Then Compound 424-3 (40 mg, 0.17 mmol) and triethylamine (43 mg, 1.4 mmol) were successively added to the above solution, and the reaction system was continued to be stirred for 2 hours.

After the depletion of the raw material as monitored by LCMS, water (10 mL) was added to the reaction solution to quench the reaction. The mixed solution was extracted with ethyl acetate (15 mL × 3 times), and the organic phases were combined. The combined organic phase was washed with saturated saline (60 mL × 2 times), then dried with anhydrous sodium sulfate, filtered and concentrated under reduced pressure. The resulting mixture was purified by silica gel column chromatography to obtain Compound 424-4 (40 mg).

MS (ESI) M/Z: 690.9 [M-99].

Step D: Compound 424-4 (40 mg, 0.05 mmol) was dissolved in dichloromethane (0.5 mL) under nitrogen protection at room temperature. Subsequently, 4 M hydrochloric acid-1,4-dioxane solution (0.25 mL) was added to the above solution under an ice-water bath. Then the reaction system was continued to be stirred for 1 hour.

After the depletion of the raw material as monitored by LCMS, the above reaction solution was concentrated under reduced pressure to obtain Compound 424-5 (25 mg).

MS (ESI) M/Z: 691.0 [M+H]⁺.

Step E: Compound 424-5 (8 mg, 0.05 mmol) was dissolved in *N,N*-dimethylformamide (0.5 mL) at room temperature. *N,N*-diisopropylethylamine (26 mg, 0.2 mmol), 2-(7-azabenzotriazolyl)-*N,N,N',N*'-tetramethyluronium hexafluorophosphate (19 mg, 0.05 mmol) and (2*S*,4*R*)-*N*-(4-bromo-1-(pyridin-3-yl)-1*H*-pyrazol-3-yl)-4-(4-bromo-2-(2*R*,6*S*)-2,6-dimethylmorpholin-4-carbonyl)-6-nitrophenyl)amino)pyrrolidine-2-formamide (25 mg, 0.04 mmol) were successively added to the above solution under an ice-water bath. Then the reaction system was continued to be stirred for 1 hour.

After the depletion of the raw material as monitored by LCMS, water (10 mL) was added to the reaction solution to quench the reaction. The mixed solution was extracted with ethyl acetate (15 mL × 3 times), and the organic phases were combined. The combined organic phase was washed with saturated saline (60 mL × 2 times), then dried with anhydrous sodium sulfate, filtered and finally concentrated under reduced pressure. The resulting residue was purified by preparative high performance liquid chromatography to obtain the title Compound 424 (1.86 mg).

MS (ESI) M/Z: 824.0 [M+H]⁺.

¹H NMR (400 MHz, DMSO-*d*₆) δ 10.42 - 9.95 (in, 1H), 9.06 (d, *J* = 2.4 Hz, 0.75H), 9.01 (d, *J* = 2.0 Hz, 0.25H), 8.88 (d, *J* = 2.8 Hz, 0.75H), 8.83 (s. 0.25H), 8.58 - 8.48 (m, 1H), 8.28 - 8.11 (m, 2H), 8.03 - 7.96 (m, 1H). 7.91 - 7.73 (m. 1H). 7.70 - 7.44 (m, 3H), 6.93 - 6.63 (m, 1H). 6.26 - 6.06 (m. 1H). 4.86 - 3.36 (m, 7H), 3.15 - 3.01 (m. 1H). 2.93 - 2.79 (m, 1H), 2.75 - 2.68 (m, 3H), 2.63 - 2.55 (m, 1H), 2.43 -2.36 (m, 1H), 2.34 - 2.19 (m, 1H), 1.12 - 0.87 (m, 6H).

### Example 427

### (2S,4R)-4-((4-bromo-2-((2R,6S)-2,6-dimethylmorpholin-4-carbonyl)-6-nitrophenyl)amino)-1-(5-(methylamino)nicotinoyl)-N-(2-(trifluoromethyl)pyridin-4-yl)pyrrolidine-2-formamide

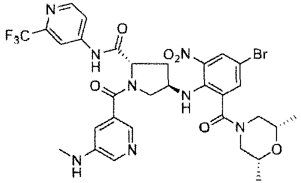

### Reaction route:

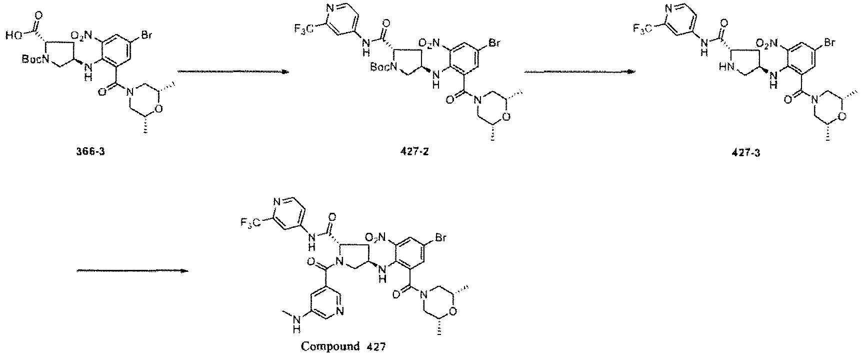

### Operating Steps:

Step A: Compound 366-3 (108 mg, 0.19 mmol) and 2-(trifluoromethyl)pyridin-4-amine (30 mg, 0.19 mmol) were dissolved in dichloromethane (1.5 mL) at 0°C. Subsequently, pyridine (45 mg, 0.57 mmol) and 1-propylphosphonic anhydride (154 mg, 0.29 mmol) were successively added to the above solution. Then the reaction system was stirred at room temperature for 30 minutes.

After the depletion of the raw material as monitored by LCMS, water (40 mL) was added to the reaction solution to quench the reaction. The mixed solution was extracted with ethyl acetate (20 mL × 3 times), and the organic phases were combined. The combined organic phase was washed with saturated sodium chloride solution (20 mL × 2 times), then dried with anhydrous sodium sulfate, filtered and concentrated under reduced pressure. The resulting residue was purified by silica gel column chromatography to obtain Compound 427-2 (100 mg).

MS (ESI) M/Z: 715.0 [M+H]⁺.

Step B: Compound 427-2 (100 mg, 0.14 mmol) was dissolved in dichloromethane (1.4 mL) at room temperature. Subsequently, the above solution was cooled to 0°C, and 4 M hydrochloric acid-1,4-dioxane solution (0.7 mL, 2.80 mmol) was added dropwise. Then the reaction system was stirred at room temperature for 2 hours.

After the depletion of the raw material as monitored by LCMS, the reaction solution was concentrated under reduced pressure. Compound 427-3 (86 mg) was obtained.

MS (ESI) M/Z: 615.0 [M+H]⁺.

Step C: Compound 427-3 (80 mg, 0.13 mmol) and 5-(methylamino)nicotinic acid (24 mg, 0.16 mmol) were dissolved in *N,N*-dimethylformamide (1.3 mL) at 0°C. Subsequently, 2-(7-azabenzotriazolyl)-*N,N,N',N*'-tetramethyluronium hexafluorophosphate (61 mg, 0.16 mmol) and *N,N*-diisopropylethylamine (84 mg, 0.65 mmol) were successively added to the above solution. Then the reaction system was continued to be stirred for 30 minutes.

After the depletion of the raw material as monitored by LCMS, water (40 mL) was added to the reaction solution to quench the reaction. The mixed solution was extracted with ethyl acetate (10 mL × 3 times), and the organic phases were combined. The combined organic phase was washed with saturated sodium chloride solution (20 mL × 2 times), then dried with anhydrous sodium sulfate, filtered and concentrated under reduced pressure. The resulting residue was purified by preparative high performance liquid chromatography to obtain the title Compound 427 (36.66 mg).

MS (ESI) M/Z: 749.0 [M+H]⁺.

¹H NMR (400 MHz, DMSO-*d*₆) δ 11.16 - 10.89 (m, 0.8H), 10.67 (s. 0.2H), 8.71 - 8.57 (m, 1H). 8.47 - 8.16 (m, 1.8H). 8.15 - 7.98 (m. 1H), 7.95 - 7.82 (m, 1H), 7.81 - 7.48 (m, 3.2H), 6.97 - 6.81 (m, 0.8H), 6.66 (s, 0.2H), 6.29 - 6.13 (m, 0.8H), 6.11 - 6.00 (m, 0.2H), 4.81 - 4.64 (m. 0.8H), 4.57 - 4.44 (m, 0.2H), 4.43 - 3.36 (m, 6.5H), 3.30 - 3.14 (m. 0.5H), 2.94 - 2.53 (m. 4H), 2.49 - 2.15 (m, 3H), 1.13 - 0.83 (m, 6H).

### Example 428

### (2S,4R)-4-(4-bromo-2-(2R,6S)-2,6-dimethylmorpholin-4-carbonyl)-6-nitrophenyl)amino)-1-(5-(methylamino)nicotinoyl)-N-(2-(trifluoromethyl)pyrimidin-4-yl)pyrrolidine-2-formamide

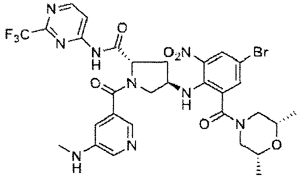

### Reaction route:

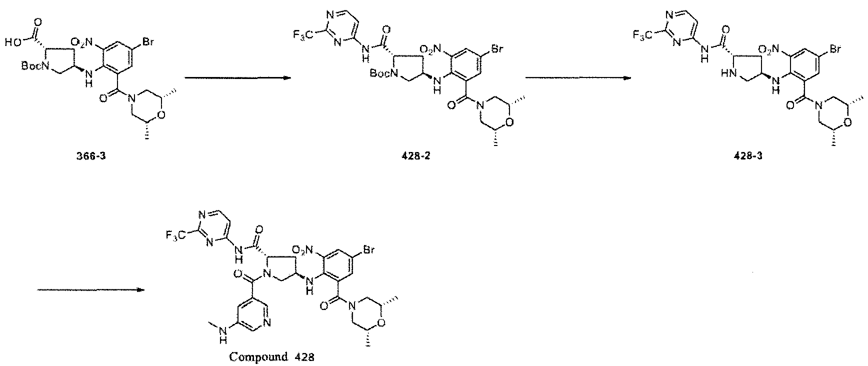

### Operating Steps:

Step A: Compound 366-3 (245 mg, 0.43 mmol), 2-(trifluoromethyl)pyrimidin-4-amine (70 mg, 0.43 mmol) and pyridine (170 mg, 2.15 mmol) were dissolved in dry 1,4-dioxane (2 mL) under nitrogen protection at room temperature. Subsequently, the reaction system solution was cooled to 0°C, and phosphoryl oxychloride (198 mg, 1.29 mmol) was added. Then the reaction system was stirred at room temperature for 0.5 hour.

After the depletion of the raw material as monitored by LCMS, ice water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 428-2 (120 mg).

MS (ESI) M/Z: 616.0 [M-99].

Step B: Compound 428-2 (120 mg, 0.03 mmol) was dissolved in dry dichloromethane (0.8 mL) under nitrogen protection at room temperature. Subsequently, the above solution was cooled to 0°C, and 4 M hydrochloric acid-1,4-dioxane solution (0.4 mL) was slowly added. Then the reaction system was stirred at room temperature for 2 hours.

After the depletion of the raw material as monitored by LCMS, the reaction solution was concentrated under reduced pressure to obtain Compound 428-3 (110 mg).

MS (ESI) M/Z: 616.0 [M+H]⁺.

Step C: 5-(methylamino)nicotinic acid (33 mg, 0.22 mmol) was dissolved in *N,N*-dimethylformamide (4 mL) at room temperature. Subsequently, the above solution was cooled to 0°C, and 2-(7-azabenzotriazolyl)-*N,N,N',N'-*tetramethyluronium hexafluorophosphate (103 mg, 0.27 mmol), *N,N*-diisopropylethylamine (116 mg, 0.9 mmol) and Compound 428-3 (110 mg, 0.18 mmol) were successively added. Then the reaction system was continued to be stirred for 30 minutes.

After the depletion of the raw material as monitored by LCMS, ice water (20 mL) was added to the reaction solution to quench the reaction. The mixed solution was extracted with ethyl acetate (9 mL × 3 times), and the organic phases were combined. The combined organic phase was washed with saturated saline (10 mL × 2 times), then dried with anhydrous sodium sulfate, filtered and concentrated under reduced pressure. The resulting residue was purified by preparative high performance liquid chromatography to obtain the title Compound 428 (43.18 mg).

MS (ESI) M/Z: 750.0 [M+H]⁺.

¹H NMR (400 MHz, DMSO-*d₆*) δ 11.79 - 11.61 (m, 0.8H), 11.48 - 11.34 (m, 0.2H), 9.00 - 8.84 (m. 1H), 8.40 - 7.46 (m, 6H), 6.99 - 6.77 (m, 0.8H), 6.66 (s, 0.2H), 6.21 (s, 0.8H), 6.10 (s, 0.2H), 4.96 - 4.63 (m, 1H), 4.48 - 3.42 (m, 6.2H), 3.28 - 3.07 (m, 0.8H), 2.90 - 2.68 (m, 3.5H), 3.59 - 2.54 (m, 0.5H), 2.49 - 2.09 (m. 3H), 1.21 - 0.81 (m, 6H).

### Example 434

### (2S,4R)-4-((4-bromo-2-((2R,6S)-2,6-dimethylmorpholin-4-carbonyl)-6-nitrophenyl)amino)-N-((1S,3R)-3-methoxycyclopentyl)-1-(5-(methylamino)nicotinoyl)pyrrolidine-2-formamide

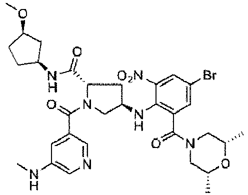

### Reaction route:

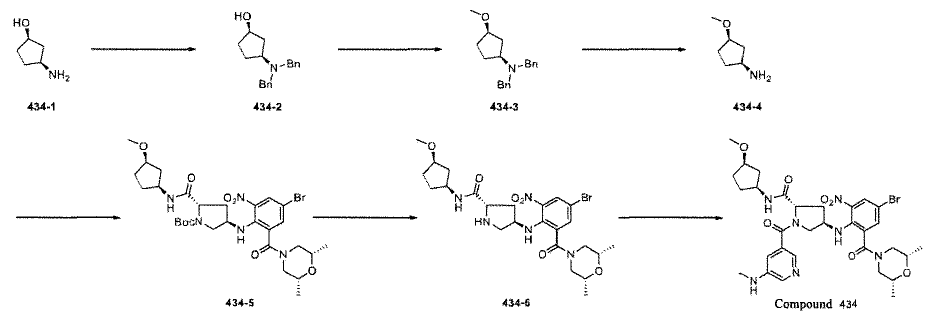

### Operating Steps:

Step A: Compound 434-1 (200 mg, 1.5 mmol) was dissolved in *N,N*-dimethylformamide (10 mL) under nitrogen protection at room temperature. Subsequently, the above solution was cooled to 0°C, sodium hydride (125 mg, 3.1 mmol) was added, and the solution was stirred for 30 minutes. Then benzyl bromide (590 mg, 3.5 mmol) was added to the above reaction solution, and the reaction system was continued to be stirred at room temperature for 16 hours.

After the depletion of the raw material as monitored by LCMS, the reaction solution was added to ice water (50 mL) to quench the reaction. The mixed solution was extracted with ethyl acetate (15 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 434-2 (300 mg).

MS (ESI) M/Z: 282.2 [M+H]⁺.

Step B: Compound 434-2 (320 mg, 1.2 mmol) was dissolved in tetrahydrofuran (10 mL) under nitrogen protection at room temperature. Subsequently, the above solution was cooled to 0°C. Sodium hydride (90 mg, 2.2 mmol) was added, and the solution was stirred for 30 minutes. Then methyl iodide (310 mg, 2.2 mmol) was added to the above reaction solution, and the reaction system was stirred at room temperature for 2 hours.

After the depletion of the raw material as monitored by LCMS, the reaction solution was added dropwise with ice water (40 mL) to quench the reaction. The mixed solution was extracted with dichloromethane (40 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 434-3 (320 mg).

MS (ESI) M/Z: 296.2 [M+H]⁺.

Step C: Compound 434-3 (100 mg, 0.34 mmol) was dissolved in methanol (2.0 mL) at room temperature. Subsequently, 10% Palladium/Carbon (20 mg) was added to the above solution. Then the reaction system was continued to be stirred for 2 hours under hydrogen atmosphere.

After the depletion of the raw material as monitored by LCMS, the reaction solution was filtered and concentrated under reduced pressure to obtain Compound 434-4 (35 mg).

MS (ESI) M/Z: 116.2 [M+H]⁺.

Step D: Compound 434-4 (35 mg, 0.30 mmol) and (2*S*,4*R*)-4-(4-bromo-2-((2*R*,6*S*)-2,6-dimethylmorpholin-4-carbonyl)-6-nitrophenyl)amino)-1-(tert-butoxycarbonyl)pyrrolidine-2-carboxylic acid (171 mg, 0.3 mmol) was dissolved in *N*,*N*-dimethylformamide (3.0 mL) at room temperature. Subsequently, the above solution was cooled to 0°C, and 2-(7-azabenzotriazolyl)-*N*,*N*,*N'*,*N'*-tetramethyluronium hexafluorophosphate (138 mg, 0.36 mmol) and *N,N-*diisopropylethylamine (120 mg, 0.90 mmol) were successively added. Then the reaction system was continued to be stirred for 15 minutes.

After the depletion of the raw material as monitored by LCMS, ice water (40 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 434-5 (124 mg).

MS (ESI) M/Z: 568.0 [M-99].

Step E: Compound 434-5 (122 mg, 0.18 mmol) was dissolved in dry dichloromethane (2 mL) under nitrogen protection at room temperature. Subsequently, the above solution was cooled to 0°C, and 4 M hydrochloric acid-1,4-dioxane solution (0.8 mL) was slowly added. Then the reaction system was stirred at room temperature for 2 hours.

After the depletion of the raw material as monitored by LCMS, the reaction solution was concentrated under reduced pressure to obtain Compound 434-6 (110 mg).

MS (ESI) M/Z: 567.8 [M+H]⁺.

Step F: Compound 434-6 (110 mg, 0.18 mmol) and 5-(methylamino)nicotinic acid (30 mg, 0.20 mmol) were dissolved in *N,N*-dimethylformamide (2.0 mL) at room temperature. Subsequently, the above solution was cooled to 0°C, and 2-(7-azabenzotriazolyl)-*N,N,N',N'-*tetramethyluronium hexafluorophosphate (100 mg, 0.26 mmol) and *N,N-*diisopropylethylamine (85 mg, 0.6 mmol) were successively added. Then the reaction system was continued to be stirred for 15 minutes.

After the depletion of the raw material as monitored by LCMS, water (35 mL) was added to the reaction solution to quench the reaction. The mixed solution was extracted with ethyl acetate (10 mL × 3 times), and the organic phases were combined. The combined organic phase was washed with saturated sodium chloride solution (15 mL × 2 times), then dried with anhydrous sodium sulfate, filtered and concentrated under reduced pressure. The resulting residue was purified by preparative high performance liquid chromatography to obtain the title Compound 434 (15.5 mg).

MS (ESI) M/Z: 702.0 [M+H]⁺.

¹H NMR (400 MHz, DMSO-*d*₆) δ 8.26 (d. *J* = 2.0 Hz, 0.4H), 8.21 (s, 0.6H), 8.06 - 7.90 (m, 1.6H), 7.86 - 7.53 (m, 3.4H), 6.89 - 6.76 (m, 0.6H), 6.67 (s. 0.4H), 6.24 - 6.12 (m, 0.6H), 6.11 - 6.02 (m, 0.4H). 4.61 - 4.11 (m. 2.6H), 4.09 - 3.90 (m, 1H), 3.88 - 3.35 (m, 6H). 3.27 - 3.06 (m, 3.4H), 2.96 - 2.64 (m, 4H), 2.63 - 2.54 (m, 0.4H). 2.48 - 2.37 (m, 0.6H), 2.34 - 1.96 (m, 3H), 1.91- 1.18 (m, 5H), 1.16 - 0.86 (m, 6H).

### Example 436

### (2S,4R)-4-((4-bromo-2-((2R,6S)-2,6-dimethylmorpholin-4-carbonyl)-6-nitrophenyl)amino)-1-(5-(methylamino)nicotinoyl)-N-(6-(trifluoromethyl)pyrimidin-4-yl)pyrrolidine-2-formamide

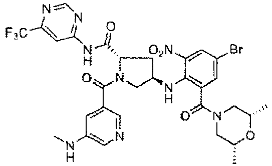

### Reaction route:

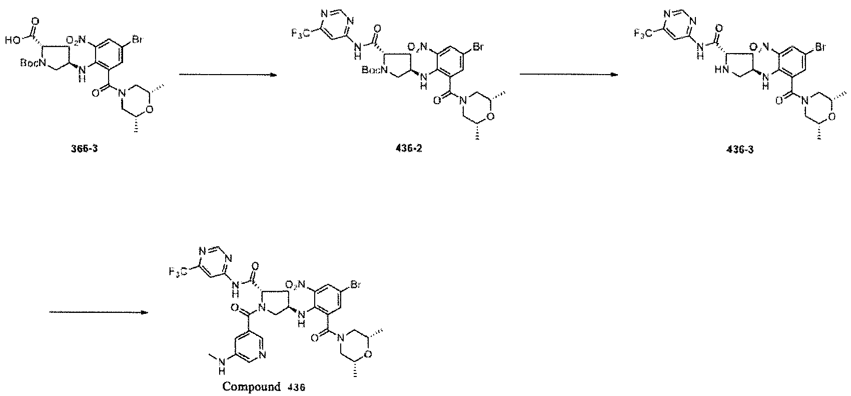

### Operating Steps:

Step A: Compound 366-3 (153.9 mg, 0.27 mmol) and 6-(trifluoromethyl)pyrimidine-4-amine (30 mg, 0.18 mmol) were dissolved in dichloromethane (0.5 mL) under nitrogen protection at room temperature. Subsequently, phosphoryl oxychloride (82.6 mg, 0.54 mmol) and pyridine (71 mg, 0.9 mmol) were successively added to the above solution. Then the reaction system was continued to be stirred for 20 minutes.

After the depletion of the raw material as monitored by LCMS, water (6 mL) was added to the reaction solution to quench the reaction. The mixed solution was extracted with dichloromethane (2 mL × 3 times), and the organic phases were combined. The combined organic phase was washed with saturated sodium chloride solution (6 mL × 2 times), then dried with anhydrous sodium sulfate, filtered and concentrated under reduced pressure. The resulting residue was purified by silica gel column chromatography to obtain Compound 436-2 (124.7 mg, yield 96%).

MS (ESI) M/Z: 616.0 [M-99].

Step B: Compound 436-2 (124.7 mg, 0.17 mmol) was dissolved in dichloromethane (1 mL) at room temperature. Subsequently, 4 M hydrochloric acid-1,4-dioxane solution (1 mL) was added to the above solution. Then the reaction system was continued to be stirred for 2 hours.

After the depletion of the raw material as monitored by LCMS, the reaction solution was concentrated under reduced pressure to obtain Compound 436-3 as crude product.

MS (ESI) M/Z: 616.0 [M+H]⁺.

Step C: 5-(Methylamino)nicotinic acid (40 mg, 0.27 mmol) was dissolved in *N,N*-dimethylformamide (1 mL) under nitrogen protection at room temperature. Subsequently, *N,N*-diisopropylethylamine (0.24 mL, 1.33 mmol), 2-(7-azabenzotriazolyl)-*N,N,N',N*'-tetramethyluronium hexafluorophosphate (140 mg, 0.37 mmol) and Compound 436-3 (164 mg, 0.27 mmol) were successively added to the above solution. Then the reaction system was continued to be stirred for 20 minutes.

After the depletion of the raw material as monitored by LCMS, water (6 mL) was added to the reaction solution to quench the reaction, the mixed solution was extracted with ethyl acetate (2 mL × 3 times), and the organic phases were combined. The combined organic phase was washed with saturated sodium chloride solution (6 mL × 2 times), then dried with anhydrous sodium sulfate, filtered and concentrated under reduced pressure. The crude product was purified by preparative high performance liquid chromatography to obtain the title Compound 436 (53.01 mg, yield 26.6%).

MS (ESI) M/Z: 750.0 [M+H]⁺.

¹H NMR (400 MHz. DMSO-*d*₆) δ 11.978 (s, 0.8H), 11.48 (s, 0.2H), 9.16 (s, 0.8H), 9.10 (s, 0.2H), 8.56 - 8.40 (m, 0.8H), 8.39 - 8.13 (m, 1.2H), 8.05 - 7.99 (m, 0.8H), 7.94 - 7.81 (m, 1H), 7.80 - 7.53 (m, 2.2H), 6.95 - 6.83 (m, 0.8H), 6.65 (s, 0.2H). 6.27 - 6.14 (m, 0.8H), 6.13 - 6.02 (m, 0.2H), 4.87 (s, 0.8H). 4.71 (s, 0.2H). 4.51 - 3.75 (m, 3H), 3.72 - 3.44 (m, 3H). 3.29 - 3.10 (m, 1H), 2.92 - 2.68 (m, 3.4H) . 2.65 - 2.55 (m. 0.6H) . 2.46 - 2.12 (m, 3H) , 1.19 - 0.83 (m, 6H).

### Example 437

### morpholin-4-carbonyl)-6-nitrophenyl)amino)-N-((S)-1-methyl-5-oxopyrrolidin-3-yl)-1-(5-(methylamino)nicotinoyl)pyrrolidine-2-formamide

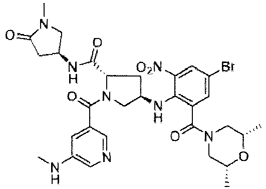

### Reaction route:

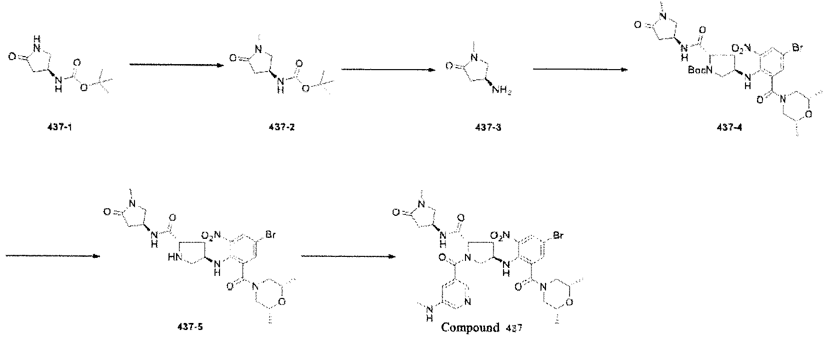

### Operating Steps:

Step A: Compound 437-1 (80 mg, 0.40 mmol) was dissolved in anhydrous *N*,*N*-dimethylformamide (2 mL) under nitrogen protection at 0°C. Subsequently, sodium hydride (36.0 mg, 0.6 mmol) was added to the above solution, and the solution was stirred for 30 minutes. Then methyl iodide (113.6 mg, 0.80 mmol) was added to the above solution, then the solution was warmed to room temperature, and continued to be stirred for 2 hours.

After the depletion of the raw material as monitored by LCMS, water (20 mL) was added to the reaction solution to quench the reaction. The mixed solution was extracted with ethyl acetate (8 mL × 3 times), and the organic phases were combined. The combined organic phase was washed with saturated sodium chloride solution (10 mL × 2 times), then dried with anhydrous sodium sulfate, filtered and concentrated under reduced pressure. The resulting residue was purified by silica gel column chromatography to obtain Compound 437-2 (45 mg, yield 52.5%).

MS (ESI) M/Z: 215.0 [M+H]⁺.

Step B: Compound 437-2 (45 mg, 0.21 mmol) was dissolved in 4 M hydrochloric acid-1,4-dioxane solution (0.5 mL) and dichloromethane (1.0 mL) under nitrogen protection at room temperature. Then the reaction system was continued to be stirred for 2 hours.

After the depletion of the raw material as monitored by LCMS, the reaction solution was distilled and concentrated under reduced pressure. Compound 437-3 crude product (24 mg) was obtained.

MS (ESI) M/Z: 115.2 [M+H]⁺.

Step C: Compound 437-3 (24 mg, 0.21 mmol) and (2*S*,4*R*)-4-(4-bromo-2-(2*R*,6*S*)-2,6-dimethylmorpholin-4-carbonyl)-6-nitrophenyl)amino)-1-(tert-butoxycarbonyl)pyrrolidine-2-carboxylic acid (123.9 mg, 0.21 mmol) was dissolved in *N,N-*dimethylformamide (2.0 mL) under nitrogen protection at room temperature. Subsequently, the above solution was cooled to 0°C, and 2-(7-azabenzotriazolyl)-*N,N,N',N*'-tetramethyluronium hexafluorophosphate (117.8 mg, 0.31 mmol) and *N,N-*diisopropylethylamine (81.3 mg, 0.63 mmol) were successively added. Then the reaction system was stirred at 0°C for 15 minutes.

After the depletion of the raw material as monitored by LCMS, ice water (30 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 437-4 (110 mg, yield 78.6%).

MS (ESI) M/Z: 567.0 [M-99].

Step D: Compound 437-4 (55 mg, 0.08 mmol) was dissolved in 4 M hydrochloric acid-1,4-dioxane solution (1.0 mL) and dichloromethane (2.0 mL) under nitrogen protection at room temperature. Then the reaction system was stirred at room temperature for 2 hours.

After the depletion of the raw material as monitored by LCMS, the reaction solution was concentrated under reduced pressure to obtain Compound 437-5 (44 mg, yield 97.1%).

MS (ESI) M/Z: 567.0 [M+H]⁺.

Step E: Compound 437-5 (44 mg, 0.08 mmol) and 5-(methylamino)nicotinic acid (12.0 mg, 0.08 mmol) were dissolved in *N,N*-dimethylformamide (1.0 mL) at room temperature. Subsequently, the above solution was cooled to 0°C, and 2-(7-azabenzotriazolyl)-*N,N,N',N'-*tetramethyluronium hexafluorophosphate (38 mg, 0.10 mmol) and *N,N*-diisopropylethylamine (31.0 mg, 0.24 mmol) were successively added. Then the reaction system was stirred at 0°C for 15 minutes.

After the depletion of the raw material as monitored by LCMS, water (25 mL) was added to the reaction solution to quench the reaction. The mixed solution was extracted with ethyl acetate (10 mL × 3 times), and the organic phases were combined. The combined organic phase was washed with saturated sodium chloride solution (15 mL × 2 times), then dried with anhydrous sodium sulfate, filtered and concentrated under reduced pressure. The resulting residue was purified by preparative high performance liquid chromatography to obtain the title Compound 437 (25.3 mg, yield 45.2%).

MS (ESI) M/Z: 701.0 [M+H]⁺.

¹H NMR (400 MHz, DMSO-*d*₆) δ 8.58 - 8.41 (m, 0.7H), 8.38 - 8.13 (m, 1.3H), 8.07 - 7.94 (m, 1H), 7.90 - 7.52 (m. 3H), 6.93 - 6.77 (m. 0.65H), 6.68 (s. 0.35H), 6.27 - 6.06 (m. 1H), 4.58 - 3.93 (m. 4H), 3.91 - 3.38 (m, 5.7H), 3.29 - 3.02 (m, 1.3H), 3.01 - 2.59 (m, 8H), 2.48 - 1.99 (m, 3.7H). 1.86 - 1.68 (m, 0.3H). 1.24 - 1.10 (m, 3H), 1.09 - 0.83 (m. 3H).

### Example 438

### (2S,4R)-N-(4-bromo-1-(2,6-dimethyltetrahydro-2H-pyran-4-yl)-1H-pyrazol-3-yl)-4-((4-bromo-2-((2R,6S)-2,6-dimethylmorpholin-4-carbonyl)-6-nitrophenyl)amino))-1-(5-(methylamino)nicotinoyl)pyrrolidine-2-formamide

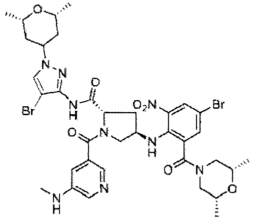

### Reaction route:

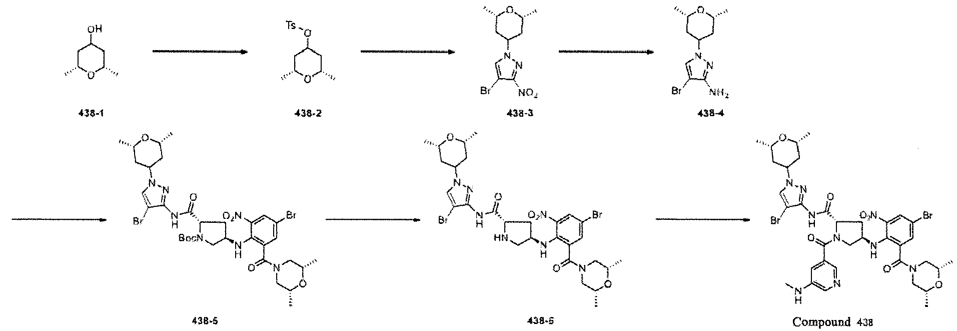

### Operating Steps:

Step A: Compound 438-1 (500 mg, 3.8 mmol) was dissolved in dichloromethane (5 mL) under nitrogen protection at 0°C. Subsequently, pyridine (1.2 g, 15.2 mmol), tosyl chloride (1.45 g, 7.60 mmol) and 4-dimethylaminopyridine (46 mg, 0.38 mmol) were successively added to the above solution. Then the reaction system was stirred at room temperature for 2 hours.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 438-2 (500 mg, yield 46%).

MS (ESI) M/Z: 307.0 [M+23]⁺.

Step B: Compound 438-2 (200 mg, 0.65 mmol) was dissolved in *N,N*-dimethylformamide (3.3 mL) under nitrogen protection at room temperature. Subsequently, the above solution was cooled to 0°C, and 1,4-dibromo-3-nitro-*1H*-pyrazole (78 mg, 0.41 mmol) and potassium carbonate (113 mg, 0.82 mmol) were successively added. Then the reaction system was stirred at 80°C for 16 hours.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography (elution: dichloromethane/methanol = 15/1) to obtain Compound 438-3 (60 mg, yield 30%)

MS (ESI) M/Z: 304.0 [M+H]⁺.

Step C: Compound 438-3 (60 mg, 0.20 mmol) was dissolved in ethanol (3 mL) and water (3 mL) under nitrogen protection at room temperature. Subsequently, iron powder (56 mg, 1 mmol) and ammonium chloride (56 mg, 1 mmol) were added to the above solution. Then the reaction system was stirred at 90°C for 1 hour.

After the depletion of the raw material as monitored by LCMS, the reaction solution was filtered while hot and concentrated under reduced pressure. The resulting residue was purified by silica gel column chromatography to obtain Compound 438-4 (40 mg, yield 73%).

MS (ESI) M/Z: 274.4 [M-99].

Step D: Compound 438-4 (40 mg, 0.15 mmol) was dissolved in *N,N*-dimethylformamide (1 mL) at room temperature. Subsequently, the above solution was cooled to 0°C, and (2S,4R)-4-(4-bromo-2-(2R,6S)-2,6-dimethylmorpholin-4-carbonyl)-6-nitrophenyl)amino)-1-(tert-butoxycarbonyl)pyrrolidine-2-carboxylic acid (86 mg, 0.15 mmol), 2-(7-azabenzotriazolyl)-*N,N,N',N'-*tetramethyluronium hexafluorophosphate (76 mg, 0.20 mmol) and *N,N*-diisopropylethylamine (97 mg, 0.75 mmol) were successively added. Then the reaction system was continued to be stirred for 5 minutes.

After the depletion of the raw material as monitored by LCMS, water (20 mL) was added to the reaction solution to quench the reaction, the mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 438-5 (70 mg, yield 64%).

MS (ESI) M/Z: 728.8 [M+H]⁺.

Step E: Compound 438-5 (70 mg, 0.80 mmol) was dissolved in 4 M hydrochloric acid-1,4-dioxane solution (0.4 mL) under nitrogen protection at room temperature. Then the reaction system was continued to be stirred for 1 hour.

After the depletion of the raw material as monitored by LCMS, the reaction solution was distilled and concentrated under reduced pressure. Compound 438-6 (60 mg, yield 73%) was obtained.

Step F: Compound 438-6 (60 mg, 0.10 mmol) was dissolved in *N*,*N*-dimethylformamide (1 mL) at room temperature. Subsequently, the above solution was cooled to 0°C, and 5-methylaminonicotinic acid (11 mg, 0.07 mmol), 2-(7-azabenzotriazol)-*N*,*N*,*N'*,*N'*-tetramethyluronium hexafluorophosphate (37 mg, 0.10 mmol) and *N,N*-diisopropylethylamine (36 mg, 0.28 mmol) were successively added. Then the reaction system was continued to be stirred for 5 minutes.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by preparative high performance liquid chromatography to obtain the title Compound 438 (13.44 mg, yield 19%).

MS (ESI) M/Z: 682.0 [M+H]⁺.

¹H NMR (400 MHz, DMSO-*d₆*) δ 10.23 - 9.42 (m, 1H), 8.33 - 7.38 (m. 6H), 6.98 - 6.59 (m, 1H), 6.36 - 5.96 (m. 1H). 4.88 - 3.41 (m, 10H), 3.25 - 3.04 (m. 1H), 2.97 - 2.61 (m. 4H). 2.46 - 2.11 (m, 5H), 1.67 - 1.40 (m, 2H), 1.21 - 0.82 (m, 12H).

### Example 439

### (5-Bromo-2-(((3S,4S)-4-methoxy-1-(3-methyl-1H-pyrazolo[3,4-c]pyridin-4-carbonyl)pyrrolidin-3-yl)amino)-3-nitrophenyl)((2S,6R)-2,6-dimethylmorpholinyl)methanone

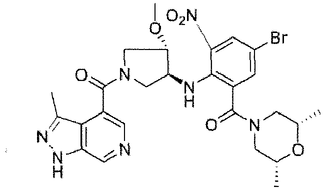

### Reaction route:

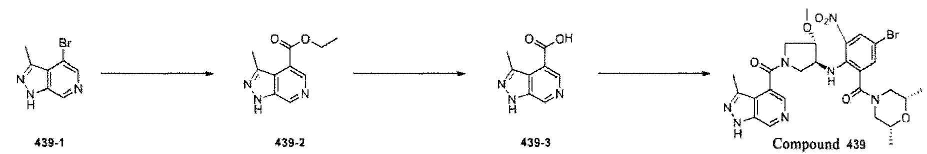

### Operating Steps:

Step A: Compound 439-1 (1 g, 4.7 mmol) was dissolved in ethanol (30 mL) at room temperature. Subsequently, triethylamine (6.5 mL, 47 mmol) and [1,1'-bis(diphenylphosphino)ferrocene]dichloropalladium (575 mg, 0.7 mmol) were successively added to the above solution. Then the reaction system was stirred at 85°C and 30 MPa pressure for 20 hours under hydrogen protection.

After the depletion of the raw material as monitored by LCMS, the reaction solution was filtered and washed with methanol (20 mL × 3 times). The filtrate was purified by silica gel column chromatography to obtain product 439-2 (808 mg, yield 83%).

MS (ESI) M/Z: 206.0 [M+H]⁺.

Step B: Compound 439-2 (868 mg, 4.21 mmol) was dissolved in tetrahydrofuran (10 mL) under nitrogen protection at room temperature. Subsequently, lithium hydroxide (530 mg, 12.6 mmol) was dissolved in water (10 mL) and added to the above solution. Then the reaction system was continued to be stirred for 4 hour.

After the depletion of the raw material as monitored by LCMS, dilute hydrochloric acid solution was added dropwise to the reaction solution to adjust the pH value to 4 at 0°C. Subsequently, the solution was concentrated under reduced pressure. Methanol (2 mL) and water (15 mL) were added to the resulting residue, and the mixture was filtered to obtain Compound 439-3 (702 mg, yield 96%).

MS (ESI) M/Z: 178.2 [M+H]⁺.

Step C: Compound 439-3 (60 mg, 0.33 mmol) and (5-bromo-2-((3*S*,4*S*)-4-methoxypyrrolidin-3-yl)amino-3-nitrophenyl)((2*S*,6*R*)-2,6-dimethylmorpholinyl)methanone (103 mg, 0.23 mmol) were dissolved in acetonitrile (1.6 mL) under nitrogen protection at room temperature. Subsequently, *N*-methylimidazole (94 mg, 1.15mmol) and tetramethylchloroformamidinium hexafluorophosphate (92 mg, 0.33mmol) were successively added to the above solution. Then the reaction system was continued to be stirred for 1 hour.

After the depletion of the raw material as monitored by LCMS, water (10 mL) was added to the reaction solution to quench the reaction. The mixed solution was extracted with ethyl acetate (10 mL × 2 times), and the organic phases were combined. The combined organic phase was washed with saturated sodium chloride solution (20 mL × 2 times), then dried with anhydrous sodium sulfate, filtered and concentrated under reduced pressure. The resulting residue was purified by preparative high performance liquid chromatography to obtain the title Compound 439 (55.39 mg, yield 47%).

MS (ESI) M/Z: 616.0 [M+H]⁺.

¹H NMR (400 MHz, DMSO-*d*₆) δ 13.50 (s, 1H), 9.13 - 8.94 (m, 1H). 8.33 - 8.00 (m. 2H). 7.80 - 7.31 (m, 2H), 4.52 - 4.34 (m, 0.4H), 4.29 - 3.42 (m, 7.6H), 3.31 - 3.24 (m, 2H), 3.24 - 3.02 (in, 2.7H), 2.99 - 2.70 (in, 1.3H), 2.61 - 2.54 (m. 0.3H), 2.45 - 2.30 (m. 3.7H), 1.23 - 0.93 (m, 5H). 0.89 - 0.71 (m, 1H),

### Example 440

### ((3S,4S)-3-((4-bromo-2-(2-methyl-2H-tetrazol-5-yl)-6-nitrophenyl)amino)-4-methoxypyrrolidin-1-yl)(5-(methylamino)pyridin-3-yl)methanone

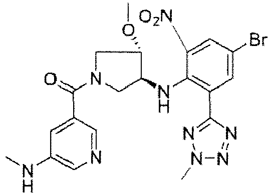

### Reaction route:

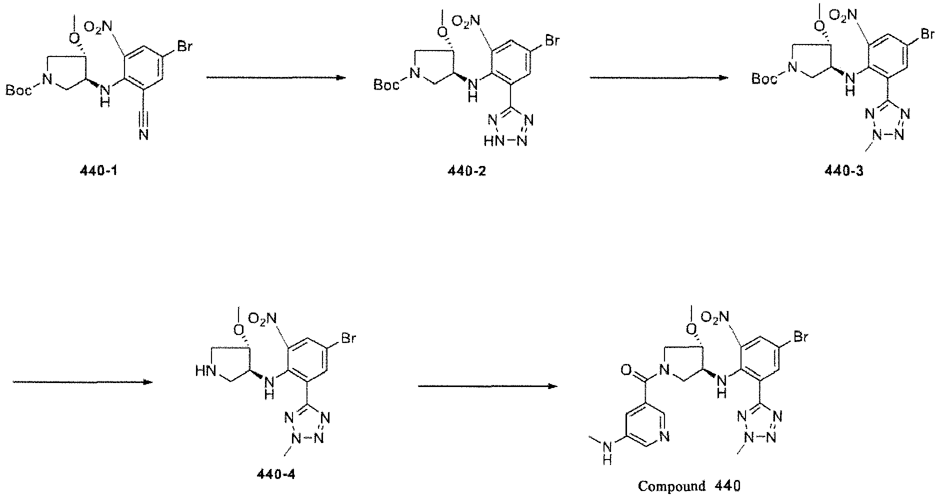

### Operating Steps:

Step A: Compound 440-1 (1.5 g, 3.41 mmol) was dissolved in *N*,*N*-dimethylformamide (17 mL) at room temperature. Subsequently, sodium azide (443 mg, 6.82 mmol) was added to the above solution. Then the reaction system was stirred at 80°C for 16 hours.

After the depletion of the raw material as monitored by LCMS, water (20 mL) was added to the reaction solution to quench the reaction, the mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 440-2 (1 g, yield 60%).

MS (ESI) M/Z: 384.40 [M-99].

Step B: Compound 440-2 (40 mg, 0.08 mmol) was dissolved in *N*,*N*-dimethylformamide (1 mL) under nitrogen protection at room temperature. Subsequently, the above solution was cooled to 0°C, and methyl iodide (12 mg, 0.08 mmol) and potassium carbonate (22 mg, 0.16 mmol) were successively added. Then the reaction system was stirred at room temperature for 2 hours.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 440-3 (30 mg, yield 75%).

MS (ESI) M/Z: 398.5 [M-99].

Step C: Compound 440-3 (40 mg, 0.08 mmol) was dissolved in 4 M hydrochloric acid-1,4-dioxane solution (0.4 mL) under nitrogen protection at room temperature. Then the reaction system was continued to be stirred for 1 hour.

After the depletion of the raw material as monitored by LCMS, the reaction solution was distilled and concentrated under reduced pressure to obtain Compound 440-4 (30 mg, yield 94%).

MS (ESI) M/Z: 398.4 [M+H]⁺.

Step E: Compound 440-4 (30 mg, 0.08 mmol) was dissolved in *N*,*N*-dimethylformamide (1 mL) at room temperature. Subsequently, the above solution was cooled to 0°C, and 5-methylaminonicotinic acid (12 mg, 0.08 mmol), 2-(7-azabenzotriazol)-N,N,N',N'-tetramethyluronium hexafluorophosphate (43 mg, 0.11 mmol) and *N,N*-diisopropylethylamine (45 mg, 0.32 mmol) were successively added. Then the reaction system was continued to be stirred for 5 minutes.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by preparative high performance liquid chromatography to obtain the title Compound 440 (10.45 mg, yield 25%).

MS (ESI) M/Z: 531.8 [M+H]⁺.

1H NMR (400 MHz, DMSO-*d₆*) δ 8.32 - 8.15 (m, 2H), 8.05 - 7.93 (m, 1H), δ 7.77 (d. *J* = 1.6 Hz. 0.45H). 7.75 (d, *J* = 1.6 Hz. 0.55H). 7.40 (d, *J* = 7.6 Hz. 0.45H), 7.28 (d. *J* = 8.8 Hz. 0.55H), 6.84 (t. *J* = 2.0 Hz, 0.45H), 6.80 (t, *J* = 2.0 Hz, 0.55H), 6.17 - 6.06 (m, 1H). 4.49 (s, 1.6H). 4.47 (s, 1.4H), 3.89 - 3.82 (m, 0.6H), 3.81 - 3.46 (m, 4.2H). 3.43 - 3.35 (m, 1.2H), 3.22 (s, 1.6H), 3.12 (m, 1.4H), 2.70 (d. *J* = 4.8 Hz, 3H).

### Example 441

### (2S,4R)-4-((4-bromo-2-((2R,6S)-2,6-dimethylmorpholin-4-carbonyl)-6-nitrophenyl)amino)-N-(6-chloropyrimidin-4-yl)-1-(5-(methylamino)nicotinoyl)pyrrolidine-2-formamide

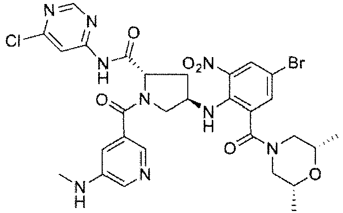

### Reaction route:

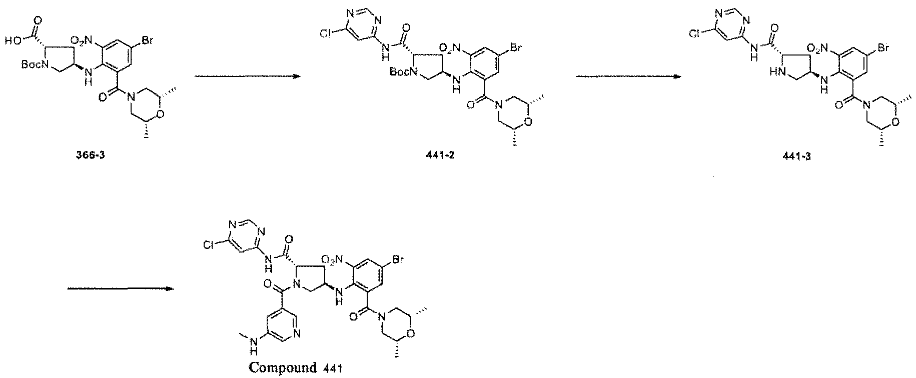

### Operating Steps:

Step A: Compound 366-3 (150 mg, 0.26 mmol) and 6-chloropyrimidin-4-amine (31.02 mg, 0.17 mmol) were dissolved in dichloromethane (1 mL) under nitrogen protection at room temperature. Subsequently, phosphoryl oxychloride (78 mg, 0.51 mmol) and pyridine (0.07 mL, 0.85 mmol) were successively added to the above solution. Then the reaction system was continued to be stirred for 20 minutes.

After the depletion of the raw material as monitored by LCMS, water (10 mL) was added to the reaction solution to quench the reaction. The mixed solution was extracted with dichloromethane (10 mL × 3 times), and the organic phases were combined. The combined organic phase was washed with saturated sodium chloride solution (15 mL × 2 times), then dried with anhydrous sodium sulfate, filtered and concentrated under reduced pressure. The resulting residue was purified by silica gel column chromatography to obtain Compound 441-2 (110 mg, yield 95%).

MS (ESI) M/Z: 583.0 [M-99].

Step B: Compound 441-2 (110 mg, 0.16 mmol) was dissolved in dichloromethane (1 mL) at room temperature. Subsequently, 4 M hydrochloric acid-1,4-dioxane solution (1 mL) was added to the above solution. Then the reaction system was continued to be stirred for 2 hours.

After the depletion of the raw material as monitored by LCMS, the reaction solution was concentrated under reduced pressure to obtain Compound 441-3 (90 mg, yield 96%).

MS (ESI) M/Z: 583.0 [M+H]⁺.

Step C: 5-(Methylamino)nicotinic acid (15.6 mg, 0.1 mmol) was dissolved in *N,N*-dimethylformamide (0.5 mL) at room temperature. Subsequently, *N,N*-diisopropylethylamine (0.07 mL, 0.43 mmol), 2-(7-azabenzotriazolyl)-*N,N*,*N',N'-*tetramethyluronium hexafluorophosphate (45 mg, 0.12 mmol) and Compound 441-1 (50 mg, 0.09 mmol) were successively added to the above solution. Then the reaction system was continued to be stirred for 20 minutes.

After the depletion of the raw material as monitored by LCMS, water (6 mL) was added to the reaction solution to quench the reaction. The mixed solution was extracted with ethyl acetate (2 mL × 3 times), and the organic phases were combined. The combined organic phase was washed with saturated sodium chloride solution (6 mL × 2 times), then dried with anhydrous sodium sulfate, filtered and concentrated under reduced pressure. The crude product was purified by preparative high performance liquid chromatography to obtain the title Compound 441 (26.63 mg, yield 43.3%).

MS (ESI) M/Z: 716.2 [M+H]⁺.

¹H NMR (400 MHz. DMSO-*d*₆) δ 11.39 (s, 1H), 8.81 (s, 0.8H), 8.74 (s, 0.2H), 8.32 - 7.72 (m, 4H), 7.70 - 7.56 (m, 2H). 6.94 - 6.81 (m. 0.8H). 6.65 (s, 0.2H) , 6.27 - 6.15 (m, 0.8H), 6.13 - 6.05 (s, 0.2H) , 4.84 (s, 0.8H), 4.72 - 4.55 (m, 0.2H), 4.45 - 3.39 (m, 6.8H), 3.26 - 3.17 (m, 0.2H), 2.91 - 2.69 (m, 3H). 2.65 - 2.54 (m, 1H), 2.47 - 2.16 (m, 3H). 1.19 - 0.82 (m, 6H).

### Example 442

### (2S,4R)-4-((4-bromo-2-((2R,6S)-2,6-dimethylmorpholin-4-carbonyl)-6-nitrophenyl)amino)-N-(2-chloropyrimidin-4-yl)-1-(5-(methytamino)nicotinoyl)pyrrolidine-2-formamide

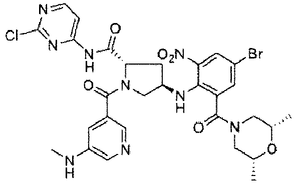

### Reaction route:

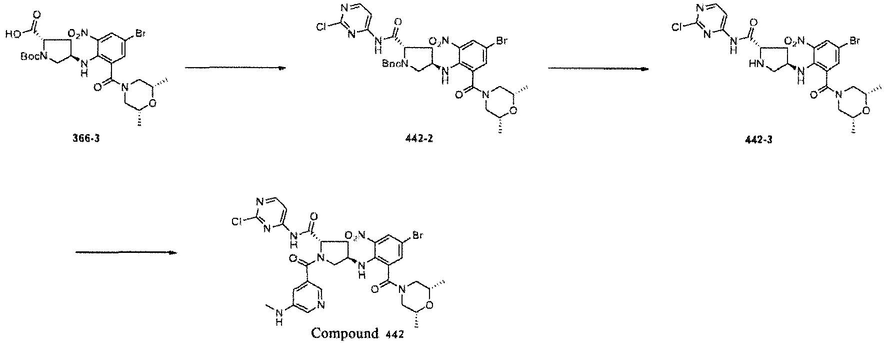

### Operating Steps:

Step A: Compound 366-3 (150 mg, 0.26 mmol) and 2-chloropyrimidin-4-amine (31.02 mg, 0.17 mmol) were dissolved in dichloromethane (1 mL) under nitrogen protection at room temperature. Subsequently, phosphoryl oxychloride (78 mg, 0.51 mmol) and pyridine (0.07 mL, 0.85 mmol) were successively added to the above solution. Then the reaction system was continued to be stirred for 20 minutes.

After the depletion of the raw material as monitored by LCMS, water (10 mL) was added to the reaction solution to quench the reaction. The mixed solution was extracted with dichloromethane (15 mL × 3 times), and the organic phases were combined. The combined organic phase was washed with saturated sodium chloride solution (20 mL × 2 times), then dried with anhydrous sodium sulfate, filtered and concentrated under reduced pressure. The resulting residue was purified by silica gel column chromatography to obtain Compound 442-2 (76 mg, yield 66%).

MS (ESI) M/Z: 583.0 [M-99].

Step B: Compound 442-2 (76 mg, 0.13 mmol) was dissolved in dichloromethane (0.65 mL) at room temperature. Subsequently, 4 M hydrochloric acid-1,4-dioxane solution (0.65 mL) was added to the above solution. Then the reaction system was continued to be stirred for 2 hours.

After the depletion of the raw material as monitored by LCMS, the reaction solution was concentrated under reduced pressure to obtain Compound 442-3 as crude product (80 mg).

MS (ESI) M/Z: 583.0 [M+H]⁺.

Step C: 5-(Methylamino)nicotinic acid (31.4 mg, 0.21 mmol) was dissolved in *N,N*-dimethylformamide (1 mL) at room temperature. Subsequently, *N,N*-diisopropylethylamine (0.18 mL, 1.05 mmol), 2-(7-azabenzotriazolyl)-*N,N,N',N'-*tetramethyluronium hexafluorophosphate (110 mg, 0.29 mmol) and Compound 442-3 (80 mg, 0.13 mmol) were successively added to the above solution. Then the reaction system was continued to be stirred for 20 minutes.

After the depletion of the raw material as monitored by LCMS, water (6 mL) was added to the reaction solution to quench the reaction. The mixed solution was extracted with ethyl acetate (2 mL × 3 times), and the organic phases were combined. The combined organic phase was washed with saturated sodium chloride solution (6 mL × 2 times), then dried with anhydrous sodium sulfate, filtered and concentrated under reduced pressure. The crude product was purified by preparative high performance liquid chromatography to obtain the title Compound 442 (4.31 mg, yield 4.6%).

MS (ESI) M/Z: 716.2 [M+H]⁺.

¹H NMR (400 MHz, DMSO-*d*₆) δ 11.45 (s, 1H), 8.64 (d, *J* = 5.6 Hz, 1H), 8.31 - 7.80 (m, 3.8H), 7.70 - 7.53 (in, 2.2H), 6.96 - 6.79 (m, 0.8H), 6.65 (s, 0.2H), 6.19 (s. 0.8H), 6.08 (s. 0.2H), 4.90 - 4.52 (m, 1H), 4.45 - 3.42 (m, 6H), 3.23 - 3.02 (m, 1H), 2.91 - 2.69 (m, 3.5H), 2.63 - 2.55 (m, 0.5H). 2.46 - 2.16 (m, 3H), 1.16 - 0.84 (m, 6H).

### Example 443

### (5-Bromo-2-((3S,4S)-4-methoxy-1-(5-((methyl-d3)amino)nicotinoyl)pyrrolidin-3-yl)amino)-3-nitrophenyl)((2R,6S)-2,6-dimethylmorpholinyl)methanone

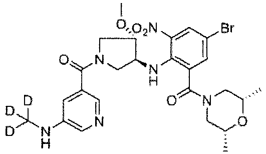

### Reaction route:

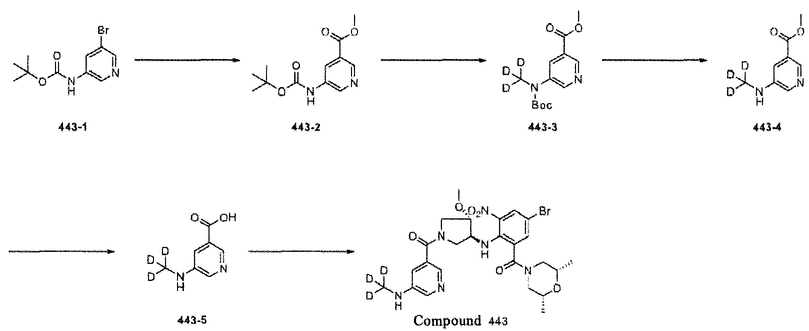

### Operating Steps:

Step A: Compound 443-1 (1 g, 45.58 mmol), triethylamine (3.72 g, 36.8 mmol) and [1,1'-bis(diphenylphosphino)ferrocene]dichloropalladium complex with dichloromethane (446 mg, 0.55 mmol) were dissolved in methanol (18 mL) under nitrogen protection at room temperature. Then the reaction system was subjected to carbon monoxide replacement for three times, and stirred at 90°C for 16 hours.

After the depletion of the raw material as monitored by LCMS, the reaction solution was concentrated under reduced pressure, and water (40 mL) was added to the resulting residue to quench the reaction. The mixed solution was extracted with ethyl acetate (30 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 443-2 (600 mg, yield 65%).

MS (ESI) M/Z: 253.2 [M+H]⁺.

Step B: Compound 443-2 (600 mg, 2.38 mmol) was dissolved in anhydrous tetrahydrofuran (12 mL) under nitrogen protection at room temperature. Subsequently, the above solution was cooled to 0°C. Sodium hydride (190 mg, 4.76 mmol) was added, and the solution was stirred for 30 minutes. Then deuterated iodomethane (521 mg, 3.57 mmol) was added. Then the reaction system was stirred at room temperature for 1 hour.

After the depletion of the raw material as monitored by LCMS, the above reaction solution was slowly added dropwise to ice water (40 mL) to quench the reaction. The mixed solution was extracted with ethyl acetate (20 mL × 3 times), and the organic phases were combined. The combined organic phase was washed with saturated saline (15 mL × 2 times), then dried with anhydrous sodium sulfate, filtered and concentrated under reduced pressure. The resulting residue was purified by silica gel column chromatography to obtain Compound 443-3 (359 mg, yield 56%).

MS (ESI) M/Z: 270.2 [M+H]⁺.

Step C: Compound 443-3 (120 mg, 0.13 mmol) was dissolved in dry dichloromethane solution (2 mL) under nitrogen protection at room temperature. Subsequently, the above solution was cooled to 0°C, and 4 M hydrochloric acid-1,4-dioxane solution (1.1 mL) was slowly added. Then the reaction system was stirred at room temperature for 1 hour.

After the depletion of the raw material as monitored by LCMS, the reaction solution was distilled and concentrated under reduced pressure to obtain Compound 443-4 as crude product (76 mg).

MS (ESI) M/Z: 170.2 [M+H]⁺.

Step D: Compound 443-4 (76 mg, 0.45 mmol) and lithium hydroxide (95 mg, 2.25 mmol) were dissolved in the solution of tetrahydrofuran (1 mL), absolute methanol (0.5 mL) and water (0.25 mL) at room temperature. Then the reaction system was continued to be stirred for 2 hours.

After the depletion of the raw material as monitored by LCMS, the reaction solution was cooled to 0°C, then 1 M hydrochloric acid solution was used to adjust the pH value to 3. The reaction solution was concentrated under reduced pressure to obtain Compound 443-5 (39 mg, yield 56%).

MS (ESI) M/Z: 156.2 [M+H]⁺.

Step E: Compound 443-5 (39 mg, 0.25 mmol) was dissolved in *N*,*N*-dimethylformamide (1 mL) at room temperature. Subsequently, the above solution was cooled to 0°C, and 2-(7-azabenzotriazolyl)-*N,N,N',N*'-tetramethyluronium hexafluorophosphate (120 mg, 0.32 mmol), *N,N*-diisopropylethylamine (135 mg, 1.1 mmol) and (5-bromo-2-((3*S*,4*S*)-4-methoxypyrrolidin-3-yl)amino-3-nitrophenyl)((2*S*,6*R*)-2,6-dimethylmorpholinyl)methanone (95 mg, 0.21 mmol) were successively added. Then the reaction system was continued to be stirred for 30 minutes.

After the depletion of the raw material as monitored by LCMS, water (35 mL) was added to the reaction solution to quench the reaction. The mixed solution was extracted with ethyl acetate (10 mL × 3 times), and the organic phases were combined. The combined organic phase was washed with saturated saline (15 mL × 2 times), then dried with anhydrous sodium sulfate, filtered and concentrated under reduced pressure. The resulting residue was purified by preparative high performance liquid chromatography to obtain the title Compound 443 (40.75 mg, yield 33%).

MS (ESI) M/Z: 594.2 [M+H]⁺.

¹H NMR (400 MHz, DMSO-*d₆*) δ 8.29 - 8.18 (m, 1H), 8.05 - 7.92 (m, 1H), 7.90 - 7.60 (m, 2H), 7.56 - 7.24 (m, 1H). 6.95 - 6.76 (m, 1H), 6.17 - 6.03 (m, 1H). 4.49 - 4.20 (m, 1H), 4.15 - 3.37 (m, 8H), 3.29 - 3.04 (m, 4H), 2.99 - 2.69 (m, 1H), 2.62 - 2.51 (m, 0.5H), 2.47 - 2.36 (m, 0.5H), 1.26 - 0.84 (m, 6H).

### Example 444

### (5-Bromo-2-(((3S,4S)-1-(3-chloro-1H-pyrazolo[3,4-c]pyridin-4-carbonyl)-4-methoxypyrrolidin-3-yl)amino)-3-nitrophenyl)((2S,6R)-2,6-dimethylmorpholinyl)methanone

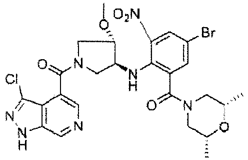

### Reaction route:

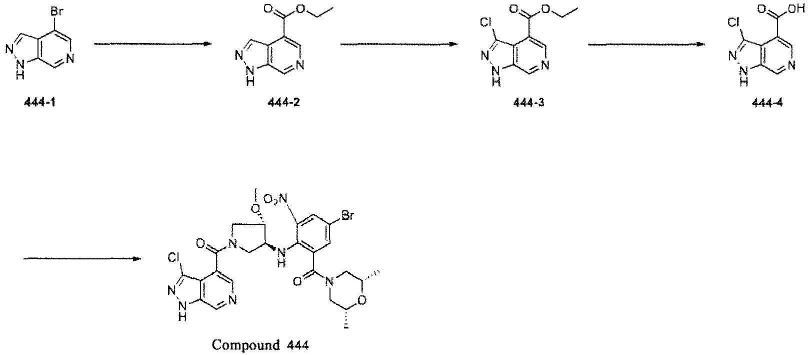

### Operating Steps:

Step A: Compound 444-1 (1.8 g, 9.1 mmol), triethylamine (9.2 g, 91 mmol) and [1,1'-bis(diphenylphosphino)ferrocene]dichloropalladium complex with dichloromethane (1.1 g, 1.36 mmol) were dissolved in ethanol (50 mL) at room temperature. Then the reaction system was subjected to carbon monoxide replacement for three times, and stirred at 90°C for 16 hours.

After the depletion of the raw material as monitored by LCMS, the reaction solution was concentrated under reduced pressure, and water (40 mL) was added to the resulting residue to quench the reaction. The mixed solution was extracted with ethyl acetate (30 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 444-2 (1.1 g, yield 65%).

MS (ESI) M/Z: 192.2 [M+H]⁺.

Step B: Compound 444-2 (800 mg, 4.19 mmol) was dissolved in anhydrous *N,N*-dimethylformamide (21 mL) under nitrogen protection at room temperature. Subsequently, N-chlorosuccinimide (1.1 g, 8.38 mmol) was added to the above solution. Then the reaction system was stirred at 40°C for 2 hours.

After the depletion of the raw material as monitored by LCMS, the above reaction solution was slowly added dropwise to ice water (80 mL) to quench the reaction. The mixed solution was extracted with ethyl acetate (20 mL × 3 times), and the organic phases were combined. The combined organic phase was washed with saturated saline (15 mL × 2 times), then dried with anhydrous sodium sulfate, filtered and concentrated under reduced pressure. The resulting residue was purified by silica gel column chromatography to obtain Compound 444-3 (200 mg, yield 21%).

MS (ESI) M/Z: 226.0 [M+H]⁺.

Step C: Compound 444-3 (35 mg, 0.16 mmol) and lithium hydroxide (33 mg, 0.78 mmol) were dissolved in tetrahydrofuran (12 mL), absolute methanol (5 mL) and water (0.25 mL) at room temperature. Then the reaction system was continued to be stirred for 2 hours.

After the depletion of the raw material as monitored by LCMS, the reaction solution was cooled to 0°C, and then 1 M hydrochloric acid solution was used to adjust the pH value to 3. The reaction solution was concentrated under reduced pressure to obtain Compound 444-4 (30 mg, yield 94%).

MS (ESI) M/Z: 156.2 [M+H]⁺.

Step D: Compound 444-4 (30 mg, 0.25 mmol), (5-bromo-2-((3*S*,4*S*)-4-methoxypyrrolidin-3-yl)amino-3-nitrophenyl)((2*S*,6*R*)-2,6-dimethylmorpholinyl)methanone (57 mg, 0.13 mmol), *N,N,N',N'-*tetramethylchloroformamidinium hexafluorophosphate (110 mg, 0.39 mmol) and N-methylimidazole (53 mg, 0.65 mmol) were dissolved in acetonitrile (1 mL) under nitrogen protection at room temperature. Then the reaction system was stirred at 50°C for 2 hours.

After the depletion of the raw material as monitored by LCMS, water (35 mL) was added to the reaction solution to quench the reaction. The mixed solution was extracted with ethyl acetate (10 mL × 3 times), and the organic phases were combined. The combined organic phase was washed with saturated saline (15 mL × 2 times), then dried with anhydrous sodium sulfate, filtered and concentrated under reduced pressure. The resulting residue was purified by preparative high performance liquid chromatography to obtain the title Compound 444 (40.75 mg, yield 33%).

MS (ESI) M/Z: 636.0 [M+H]⁺.

¹H NMR (400 MHz, DMSO-*d*₆) δ 9.13 (s. 1H). 8.57 - 7.99 (m. 2H), 7.84 - 7.32 (m, 2H), 4.50 - 3.75 (m, 7H), 3.17 - 2.70 (m, 6H), 2.69 - 2.54 (m, 1H), 2.45 - 2.23 (m. 1H), 1.26 - 0.72 (m, 6H).

### Example 445

### (2S,4R)-4-((4-bromo-2-((2R,6S)-2,6-dimethylmorpholin-4-carbonyl)-6-nitrophenyl)amino)-N-(5-bromopyrimidin-2-yl)-1-(5-(methylamino)nicotinoyl)pyrrolidine-2-formamide

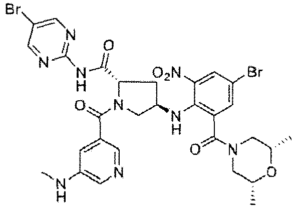

### Reaction route:

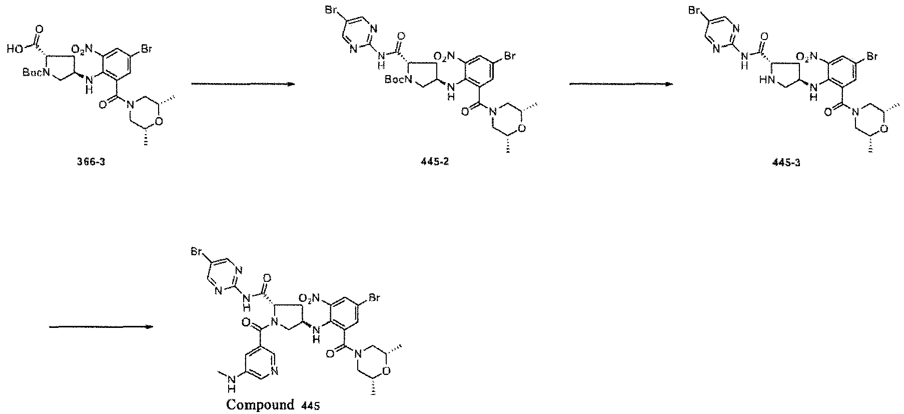

### Operating Steps:

Step A: Compound 366-3 (198 mg, 0.38 mmol) and 5-bromopyrimidin-2-amine (50 mg, 0.29 mmol) were dissolved in dichloromethane (1.45 mL) under nitrogen protection at room temperature. Subsequently, phosphoryl oxychloride (133 mg, 0.87 mmol) and pyridine (0.11 mL, 1.45 mmol) were successively added to the above solution. Then the reaction system was continued to be stirred for 20 minutes.

After the depletion of the raw material as monitored by LCMS, water (6 mL) was added to the reaction solution to quench the reaction. The mixed solution was extracted with dichloromethane (2 mL × 3 times), and the organic phases were combined. The combined organic phase was washed with saturated sodium chloride solution (6 mL × 2 times), then dried with anhydrous sodium sulfate, filtered and concentrated under reduced pressure. The resulting residue was purified by silica gel column chromatography to obtain Compound 445-2 (213 mg, yield 86%).

MS (ESI) M/Z: 627.0 [M-99].

Step B: Compound 445-2 (213 mg, 0.29 mmol) was dissolved in dichloromethane (1.5 mL) at room temperature. Subsequently, 4 M hydrochloric acid-1,4-dioxane solution (1.5 mL) was added to the above solution. Then the reaction system was continued to be stirred for 2 hours.

After the depletion of the raw material as monitored by LCMS, the reaction solution was concentrated under reduced pressure to obtain Compound 445-3 (181 mg, yield 70%).

MS (ESI) M/Z: 627.0 [M+H]⁺.

Step C: 5-(Methylamino)nicotinic acid (52.8 mg, 0.35 mmol) was dissolved in *N,N*-dimethylformamide (1.5 mL) under nitrogen protection at room temperature. Subsequently, *N,N*-diisopropylethylamine (0.26 mL, 1.45 mmol), 2-(7-azabenzotriazolyl)-*N,N,N',N'*-tetramethyluronium hexafluorophosphate (154 mg, 0.41 mmol) and Compound 445-3 (181 mg, 0.29 mmol) were successively added to the above solution. Then the reaction system was continued to be stirred for 20 minutes.

After the depletion of the raw material as monitored by LCMS, water (6 mL) was added to the reaction solution to quench the reaction. The mixed solution was extracted with ethyl acetate (2 mL × 3 times), and the organic phases were combined. The combined organic phase was washed with saturated sodium chloride solution (6 mL × 2 times), then dried with anhydrous sodium sulfate, filtered and concentrated under reduced pressure. The crude product was purified by preparative high performance liquid chromatography to obtain the title Compound 445 (21.06 mg, yield 9.5%).

MS (ESI) M/Z: 762.2 [M+H]⁺.

¹H NMR (400 MHz, DMSO-*d*₆) δ 11.03 (s, 0.7H). 10.79 (m, 0.3H), 8.89 - 8.64 (m, 2H). 8.29 - 8.12 (in, 1H), 8.00 (s, 1H). 7.94 - 7.74 (m. 1.3H), 7.72 - 7.53 (m, 2H), 6.93 - 6.78 (m, 0.7H), 6.66 (s, 0.3H), 6.19 (s, 0.7H). 6.08 (s, 0.3H), 5.05 - 4.55 (m, 1H), 4.43 - 3.41 (m, 6H). 3.28 - 3.00 (m, 1H), 2.92 - 2.56 (m.4H). 2.46 -- 1.99 (m, 3H), 1.14 - 0.74 (m, 6H).

### Example 446

### (5-Bromo-2-((3S,4S)-1-(5-(methylamino)nicotinoyl)-5-(2-methoxyethoxy)piperidin-3-yl)amino)-3-nitrophenyl)((2S,6R)-2,6-dimethylmorpholinyl)methanone

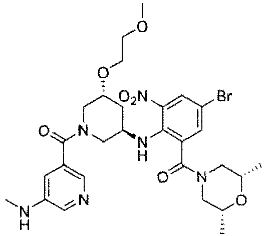

### Reaction route:

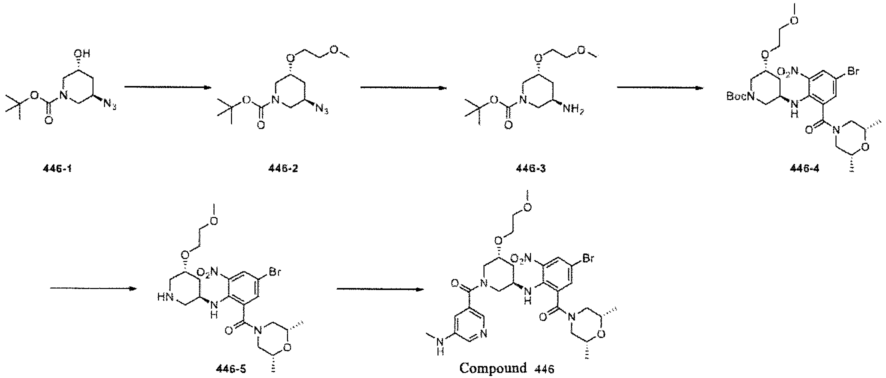

### Operating Steps:

Step A: Compound 446-1 (70 mg, 0.29 mmol) was dissolved in anhydrous *N*,*N*-dimethylformamide (2 mL) under nitrogen protection at 0°C. Subsequently, sodium hydride (17.3 mg, 0.43 mmol) was added to the above solution, and the solution was stirred for 30 minutes. Then 1-bromo-2-methoxyethane (120 mg, 0.87 mmol) was added to the above solution, then the solution was warmed to room temperature, and continued to be stirred for 2 hours.

After the depletion of the raw material as monitored by LCMS, the reaction solution was slowly added dropwise to water (15 mL) to quench the reaction. The mixed solution was extracted with ethyl acetate (5 mL × 3 times), and the organic phases were combined. The combined organic phase was washed with saturated sodium chloride solution (5 mL × 2 times), then dried with anhydrous sodium sulfate, filtered and concentrated under reduced pressure. The resulting residue was purified by silica gel column chromatography to obtain Compound 446-2 (64 mg, yield 73.5%).

MS (ESI) M/Z: 323.2 [M+Na]⁺.

Step B: Compound 446-2 (64 mg, 0.21 mmol) was dissolved in methanol (4 mL) at room temperature. Subsequently, platinum dioxide (12 mg) was added to the above reaction solution. Then the reaction system was stirred for 2 hours under hydrogen atmosphere.

After the depletion of the raw material as monitored by LCMS, the reaction solution was filtered and the filtrate was concentrated under reduced pressure to obtain Compound 446-3 crude product (59 mg).

MS (ESI) M/Z: 275.0 [M+H]⁺.

Step C: Compound 446-3 (59 mg, 0.21 mmol) was dissolved in *N,N*-dimethylformamide (2 mL) under nitrogen protection at room temperature. Subsequently, (5-bromo-2-fluoro-3-nitrophenyl)((2*S*,6*R*)-2,6-dimethylmorpholinyl)methanone (75.6 mg, 0.21 mmol) and *N,N*-diisopropylethylamine (80.0 mg, 0.62 mmol) were successively added to the above solution. Then the reaction system was stirred at 80°C for 12 hours.

After the depletion of the raw material as monitored by LCMS, water (15 mL) was added to the reaction solution to quench the reaction. The mixed solution was extracted with ethyl acetate (5 mL × 3 times), and the organic phases were combined. The combined organic phase was washed with saturated sodium chloride solution (10 mL × 2 times), then dried with anhydrous sodium sulfate, filtered and concentrated under reduced pressure. The resulting residue was purified by silica gel column chromatography to obtain Compound 446-4 (65 mg, yield 50.4%).

MS (ESI) M/Z: 515.1 [M-99].

Step D: Compound 446-4 (65 mg, 0.11 mmol) was dissolved in 4 M hydrochloric acid-1,4-dioxane solution (0.5 mL) and dichloromethane (1.0 mL) under nitrogen protection at room temperature. Then the reaction system was stirred at room temperature for 2 hours.

After the depletion of the raw material as monitored by LCMS, the reaction solution was distilled and concentrated under reduced pressure. Compound 446-5 crude product (59 mg) was obtained.

MS (ESI) M/Z: 515.0 [M+H]⁺.

Step E: Compound 446-5 (59 mg, 0.11 mmol) was dissolved in *N*,*N*-dimethylformamide (1 mL) at room temperature. Subsequently, the above solution was cooled to 0°C, and 5-(methylamino)nicotinic acid (16.5 mg, 0.11 mmol), 2-(7-azabenzotriazolyl)-*N*,*N*,*N'*,*N'*-tetramethyluronium hexafluorophosphate (50.1 mg, 0.13 mmol) and *N,N-*diisopropylethylamine (42.6 mg, 0.33mmol) were successively added. Then the reaction system was stirred at 0°C for 15 minutes.

After the depletion of the raw material as monitored by LCMS, water (20 mL) was added to the reaction solution to quench the reaction. The mixed solution was extracted with ethyl acetate (5 mL × 3 times), and the organic phases were combined. The combined organic phase was washed with saturated saline (20 mL × 2 times), then dried with anhydrous sodium sulfate, filtered and concentrated under reduced pressure. The resulting residue was purified by reversed phase column chromatography to obtain the title Compound 446 (35.7 mg, yield 50.1%).

MS (ESI) M/Z: 649.0 [M+H]⁺.

¹H NMR (400 MHz, DMSO-*d*₆) δ 8.24 (s, 1H), 8.06 - 7.84 (m. 1H), 7.82 - 7.22 (m, 3H), 6.89 - 6.51 (m, 1H), 6.29 - 5.88 (m, 1H), 4.61 - 3.96 (m, 2H). 3.95 - 3.38 (m, 9H), 3.27 - 3.00 (m, 6H). 2.99 - 2.78 (m, 1H). 2.71 (d. *J* = 4.4 Hz, 3H), 2.47 - 2.25 (m, 1H), 2.20 - 1.67 (m, 2H). 1.37 - 0.80 (m, 6H).

### Example 447

### Bromo-2-((3R,5S)-5-(methoxymethyl)-1-(5-methylamino)nicotinoyl)pyrrolidin-3-yl)amino)-3-nitrophenyl)((2R,6S)-2,6-dimethylmorpholinyl)methanone

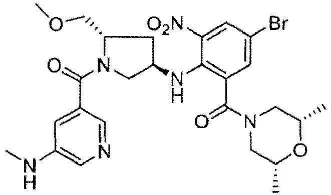

### Reaction route:

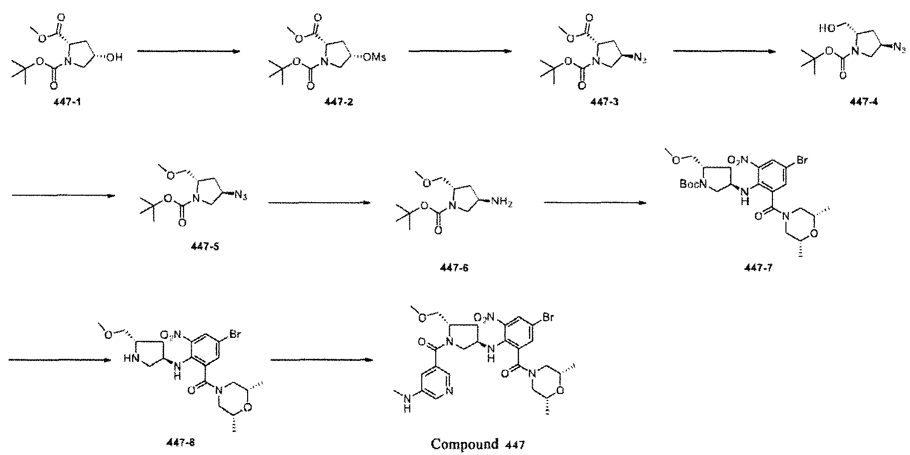

### Operating Steps:

Step A: Compound 447-1 (1 g, 4.08 mmol) was dissolved in dichloromethane (20 mL) under nitrogen protection at 0°C. Subsequently, triethylamine (824 g, 8.16 mmol) and methanesulfonyl chloride (808 g, 6.12 mmol) were successively added to the above solution. Then the reaction system was stirred at room temperature for 2 hours.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. Compound 447-2 (1.2 g, yield 91%) was obtained.

MS (ESI) M/Z: 346.0 [M+23]⁺.

Step B: Compound 447-2 (1.2 g, 3.52 mmol) was dissolved in *N,N*-dimethylformamide (3.3 mL) under nitrogen protection at room temperature. Subsequently, sodium azide (457 mg, 7 mmol) was added to the above solution. Then the reaction system was stirred at 80°C for 16 hours.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 447-3 (700 mg, yield 73%).

MS (ESI) M/Z: 293.1 [M+23]⁺.

Step C: Compound 447-3 (700 mg, 2.58 mmol) was dissolved in tetrahydrofuran (18 mL) under nitrogen protection at 0°C. Subsequently, lithium aluminium hydride (2 mL, 5.16 mmol) was added to the above solution. Then the reaction system was stirred at 0°C for 2 hours.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 447-4 (600 mg, yield 96%).

MS (ESI) M/Z: 265.2 [M+23]⁺.

Step D: Compound 447-4 (600 mg, 2.48 mmol) was dissolved in acetonitrile (10 mL) under nitrogen protection at 0°C. Subsequently, sodium hydride (994 mg, 8.28 mmol) was added to the above solution, and the solution was stirred for 30 minutes. Then methyl iodide (1.18 g, 8.28 mmol) was added to the above solution, and the solution was continued to be stirred for 0.5 hours.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 447-5 (90 mg, yield 13%).

MS (ESI) M/Z: 279.1 [M+23]⁺.

Step E: Compound 447-5 (90 mg, 0.35 mmol) was dissolved in methanol (5 mL) at room temperature. Subsequently, platinum dioxide (9 mg) was added to the above solution. Then the reaction system was stirred for 2 hours under hydrogen atmosphere.

After the depletion of the raw material as monitored by LCMS, the reaction solution was filtered and the filtrate was concentrated under reduced pressure. Compound 447-6 (30 mg, yield 37%) was obtained.

MS (ESI) M/Z: 175.2 [M-55].

Step F: Compound 447-6 (30 mg, 0.13 mmol) was dissolved in *N,N*-dimethylformamide (1 mL) at room temperature. Subsequently, (5-bromo-2-fluoro-3-nitrophenyl)((2*R*,6*S*)-2,6-dimethylmorpholinyl)methanone (47 mg, 0.13 mmol) and *N*,*N*-diisopropylethylamine (50 mg, 0.39 mmol) were added to the above solution. Then the reaction system was stirred at 80°C for 2 hours.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 447-7 (50 mg, yield 67%).

MS (ESI) M/Z: 471.0 [M-99].

Step G: Compound 447-7 (50 mg, 0.09 mmol) was dissolved in 4 M hydrochloric acid-1,4-dioxane solution (0.5 mL) under nitrogen protection at room temperature. Then the reaction system was continued to be stirred for 1 hour.

After the depletion of the raw material as monitored by LCMS, the reaction solution was distilled and concentrated under reduced pressure. Compound 447-8 crude product (50 mg) was obtained.

MS (ESI) M/Z: 471.0 [M+H]⁺.

Step H: Compound 447-8 (50 mg, 0.11 mmol) was dissolved in *N,N*-dimethylformamide (1 mL) at room temperature. Subsequently, the above solution was cooled to 0°C, and 5-methylaminonicotinic acid (16 mg, 0.11 mmol), 2-(7-azabenzotriazol)-*N,N,N',N'*-tetramethyluronium hexafluorophosphate (59 mg, 0.15 mmol) and *N*,*N*-diisopropylethylamine (71 mg, 0.55 mmol) were successively added. Then the reaction system was continued to be stirred for 5 minutes.

After the depletion of the raw material as monitored by LCMS, water (20 mL) was added to the reaction solution to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure. The resulting residue was purified by preparative high performance liquid chromatography to obtain the title Compound 447 (10.99 mg, yield 16%).

MS (ESI) M/Z: 603.2 [M+H]⁺.

1H NMR (400 MHz, DMSO-*d₆*) δ 8.34 - 8.08 (m, 1H). 8.05 - 7.86 (m, 1H), 7.84 - 7.35 (m, 3H), 6.93 - 6.61 (m, 1H), 6.13 (s 1H). 4.52 - 3.45 (m, 8H), 3.30 - 2.91 (m, 1H), 2.90 - 2.65 (m, 4H), 2.44 - 2.32 (m, 1H), 2.25 - 1.96 (m, 2H), 1.27 - 0.81 (m. 6H).

### Example 448

### (5-Bromo-2-(3R,5R)-1-(5-(methylamino)nicotinoyl)-5-((R)-tetrahydrofuran-3-yl)oxy)piperidin-3-amino)-3-nitrophenyl)((2S,6R)-2,6-dimethylmorpholinyl)methanone

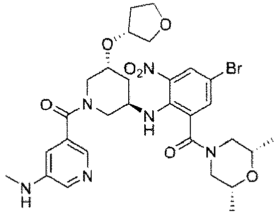

### Reaction route:

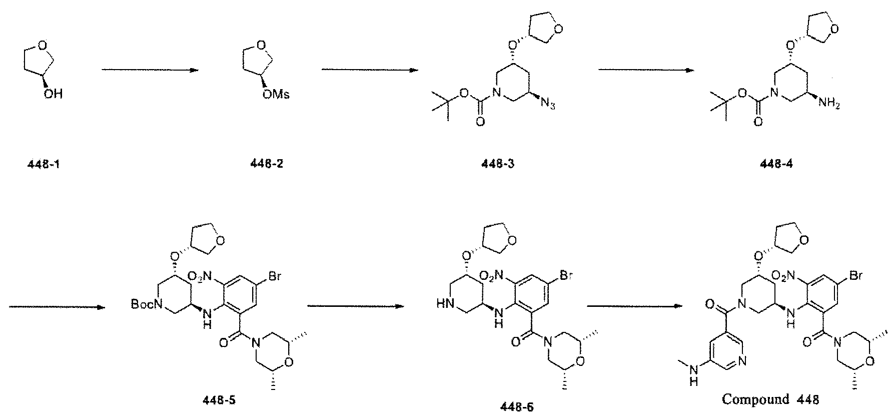

### Operating Steps:

Step A: Compound 448-1 (1 g, 11.4 mmol) was dissolved in dichloromethane solution (25 mL) under nitrogen protection at room temperature. Subsequently, the above solution was cooled to 0°C, and triethylamine (3.45 g, 34.2 mmol) and methanesulfonyl chloride (1.95 g, 17.1 mmol) were successively slowly added. Then the reaction system was stirred at room temperature for 3 hours.

After the depletion of the raw material as monitored by LCMS, the reaction solution was concentrated and added to ice water (25 mL) to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure to obtain Compound 448-2 (1.2 g, yield 63.4%).

¹H NMR (400 MHz, CDCl₃) δ 5.36 - 5.26 (m, 1H), 4.07 - 3.87 (m, 4H), 3.05 (s, 3H), 2.30 - 2.20 (m, 2H).

Step B: Compound 448-2 (760 mg, 4.57 mmol) was dissolved in anhydrous *N,N*-dimethylformamide (20 mL) under nitrogen protection at 0°C. Subsequently, sodium hydride (274 mg, 6.85 mmol) was added to the above solution, and the solution was stirred for 30 minutes. Then tert-butyl (3*R*,5*R*)-3-azido-5-hydroxypiperidine-1-carboxylate (1.1 g, 4.57 mmol) was added to the solution, and the solution was warmed to 70°C, and continued to be stirred for 16 hours.

After the depletion of the raw material as monitored by LCMS, the reaction solution was slowly added dropwise to water (20 mL) to quench the reaction. The mixed solution was extracted with ethyl acetate (15 mL × 3 times), and the organic phases were combined. The combined organic phase was washed with saturated sodium chloride solution (10 mL × 2 times), then dried with anhydrous sodium sulfate, filtered and concentrated under reduced pressure. The resulting residue was purified by silica gel column chromatography to obtain Compound 448-3 (30 mg, yield 2.1%).

MS (ESI) M/Z: 213.0 [M-99].

Step C: Compound 448-3 (30 mg, 0.96 mmol) was dissolved in methanol (2 mL) at room temperature. Subsequently, platinum dioxide (6 mg) was added to the above reaction solution. Then the reaction system was stirred for 2 hours under hydrogen atmosphere.

After the depletion of the raw material as monitored by LCMS, the reaction solution was filtered and the filtrate was concentrated under reduced pressure to obtain Compound 448-4 (25 mg, yield 91.1%).

MS (ESI) M/Z: 231.2 [M-55].

Step D: Compound 448-4 (25 mg, 0.09 mmol) was dissolved in *N*,*N*-dimethylformamide (1 mL) under nitrogen protection at room temperature. Subsequently, (5-bromo-2-fluoro-3-nitrophenyl)((2S,6R)-2,6-dimethylmorpholinyl)methanone (36.1 mg, 0.1 mmol) and *N,N*-diisopropylethylamine (39.0 mg, 0.3 mmol) were successively added to the above solution. Then the reaction system was stirred at 80°C for 12 hours.

After the depletion of the raw material as monitored by LCMS, water (15 mL) was added to the reaction solution to quench the reaction. The mixed solution was extracted with ethyl acetate (5 mL × 3 times), and the organic phases were combined. The combined organic phase was washed with saturated sodium chloride solution (10 mL × 2 times), then dried with anhydrous sodium sulfate, filtered and concentrated under reduced pressure. The resulting residue was purified by silica gel column chromatography to obtain Compound 448-5 (35 mg, yield 62.1%).

MS (ESI) M/Z: 527.2 [M-99].

Step E: Compound 448-5 (35 mg, 0.056 mmol) was dissolved in 4 M hydrochloric acid-1,4-dioxane solution (0.3 mL) and dichloromethane (0.6 mL) under nitrogen protection at room temperature. Then the reaction system was stirred at room temperature for 2 hours.

After the depletion of the raw material as monitored by LCMS, the reaction solution was distilled and concentrated under reduced pressure. Compound 448-6 crude product (32 mg) was obtained.

MS (ESI) M/Z: 527.0 [M+H]⁺.

Step F: Compound 448-6 (32 mg, 0.06 mmol) was dissolved in *N,N*-dimethylformamide (1 mL) at room temperature. Subsequently, the above solution was cooled to 0°C, and 5-(methylamino)nicotinic acid (9.0 mg, 0.06 mmol), 2-(7-azabenzotriazol)-*N,N,N',N'*-tetramethyluronium hexafluorophosphate (26.6 mg, 0.07 mmol) and *N,N*-diisopropylethylamine (23.2 mg, 0.18 mmol) were successively added. Then the reaction system was stirred at 0°C for 15 minutes.

After the depletion of the raw material as monitored by LCMS, water (15 mL) was added to the reaction solution to quench the reaction. The mixed solution was extracted with ethyl acetate (5 mL × 3 times), and the organic phases were combined. The combined organic phase was washed with saturated saline (20 mL × 2 times), then dried with anhydrous sodium sulfate, filtered and concentrated under reduced pressure. The resulting residue was purified by reversed phase column chromatography to obtain the title Compound 448 (14.6 mg, yield 36.8%).

MS (ESI) M/Z: 660.8 [M+H]⁺.

¹H NMR (400 MHz, DMSO-*d*₆) δ 8.32 - 8.07 (m, 1H), 8.03 - 7.84 (m, 1H), 7.82 - 7.18 (m. 3H), 6.95 - 6.65 (m, LH), 6.26 (s, 1H), 4.60 - 3.93 (m, 3H), 3.89 - 3.46 (m, 10H), 3.19 - 2.77 (m, 3H), 2.72 (s, 3H), 2.45 - 2.36 (m, 1H). 2.12 - 1.58 (in, 4H), 1.20 - 1.08 (m. 3H), 1.01 (brs, 3H).

### Example 449

### (5-Bromo-2-(((3S,4S)-4-methoxy-1-(2-methyl-3H-imidazol(4,5-c]pyridin-7-carbonyl)pyrrolidin-3-yl)amino)-3-nitrophenyl)((2S,6R)-2,6-dimethylmorpholinyl)methanone

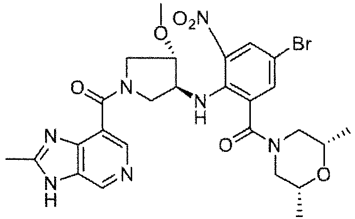

### Reaction route:

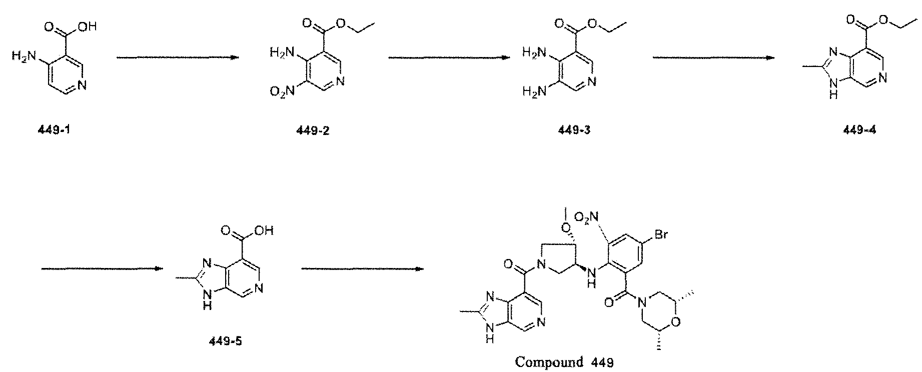

### Operating Steps:

Step A: Compound 449-1 (2.5 g, 18.11 mmol) was dissolved in concentrated sulfuric acid (24 mL) at 0°C. Subsequently, potassium nitrate (1.8 g, 18.11 mmol) was added to the above solution. Then the reaction system was warmed to 30°C, stirred for 3 hours, then warmed to 70°C, and continued to be stirred for 3 hours. Then the reaction system was cooled to 0°C, added with ethanol (9.5mL), warmed to 60°C and stirred for 18 hours.

After the depletion of the raw material as monitored by LCMS, the reaction solution was added dropwise to potassium acetate solution on ice to quench the reaction, then saturated sodium bicarbonate solution was used to adjust the pH value to 7 to 8. The mixed solution was extracted with ethyl acetate (100 mL × 3 times), and the organic phases were combined. The combined organic phase was washed with saturated sodium chloride solution (500 mL × 2 times), then dried with anhydrous sodium sulfate, filtered and concentrated under reduced pressure. The resulting residue was purified by silica gel column chromatography to obtain Compound 449-2 (880 mg, yield 23%).

MS (ESI) M/Z: 212.0 [M+H]⁺.

Step B: Compound 449-2 (880 mg, 4.17 mmol) was dissolved in ethanol (21 mL) at room temperature. Subsequently, 10% Palladium/Carbon (88 mg) was added to the above solution. Then the reaction system was continued to be stirred for 6 hours under hydrogen atmosphere.

After the depletion of the raw material as monitored by LCMS, the reaction solution was filtered and the filtrate was concentrated under reduced pressure to obtain Compound 449-3 (725 mg, yield 95.6%).

MS (ESI) M/Z: 182 [M+H]⁺.

Step C: Compound 449-3 (245 mg, 1.35 mmol) and 1,1,1-triethoxyethane (2.18 g, 13.5 mmol) were dissolved in acetate (2.5 mL) at room temperature. Then the reaction system was stirred at 120°C for 8 hours.

After the depletion of the raw material as monitored by LCMS, the reaction solution was concentrated under reduced pressure. The resulting residue was purified by silica gel column chromatography to obtain Compound 449-4 (231 mg, yield 83%).

MS (ESI) M/Z: 206.0 [M+H]⁺.

Step D: Compound 449-4 (231 mg, 1.13 mmol) was dissolved in the solution of tetrahydrofuran (5.6 mL) and water (2.8 mL) at room temperature. Subsequently, lithium hydroxide (94 mg, 2.25 mmol) was added to the above solution. Then the reaction system was continued to be stirred for 8 hours.

After the depletion of the raw material as monitored by LCMS, the reaction solution was concentrated under reduced pressure to obtain Compound 449-5 (197 mg, yield 98%).

MS (ESI) M/Z: 178.0 [M+H]⁺.

Step E: Compound 449-5 (63 mg, 0.36 mmol) was dissolved in *N,N*-dimethylformamide (1.2 mL) at room temperature. Subsequently, *N*,*N*-diisopropylethylamine (0.13 mL, 0.72 mmol), 2-(7-azabenzotriazolyl)-*N,N,N',N'*-tetramethyluronium hexafluorophosphate (182 mg, 0.48 mmol) and (5-bromo-2-(((3*S*,4*S*)-4-methoxypyrrolidin-3-yl)amino)-3-nitrophenyl)((2*S*,6*R*)-2,6-dimethylmorpholinyl)methanone (109 mg, 0.24 mmol) were successively added to the above solution. Then the reaction system was continued to be stirred for 20 minutes.

After the depletion of the raw material as monitored by LCMS, water (10 mL) was added to the reaction solution to quench the reaction. The mixed solution was extracted with ethyl acetate (3 mL × 3 times), and the organic phases were combined. The combined organic phase was washed with saturated sodium chloride solution (10 mL × 2 times), then dried with anhydrous sodium sulfate, filtered and concentrated under reduced pressure. The crude product was purified by preparative high performance liquid chromatography to obtain the title Compound 449 (4.67 mg, yield 3.2%).

MS (ESI) M/Z: 616.1 [M+H]⁺.

¹H NMR (400 MHz, DMSO-*d*₆) δ 13.13 - 12.63 (m, 1H), 8.94 - 8.74 (m, 1H), 8.53 - 8.12 (m, 2H). 7.83 - 7.25 (m. 2H). 4.54 - 3.51 (m, 7H), 3.31 - 2.76 (m, 6H). 2.66 - 2.53 (m, 3H), 2.45 - 1.70 (m, 1H). 1.30 - 0.63 (m. 6H).

### Example 450

### (2S,4R)-4-((4-bromo-2-((2R,6S)-2,6-dimethylmorpholin-4-carbonyl)-6-nitrophenyl)amino)-1-(5-(methylamino)nicotinoyl)-N-((2-(trifluoromethyl)pyrimidin-4-yl)methyl)pyrrolidine-2-formamide

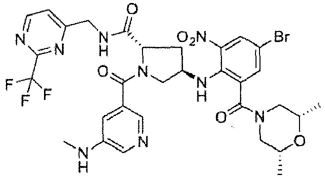

### Reaction route:

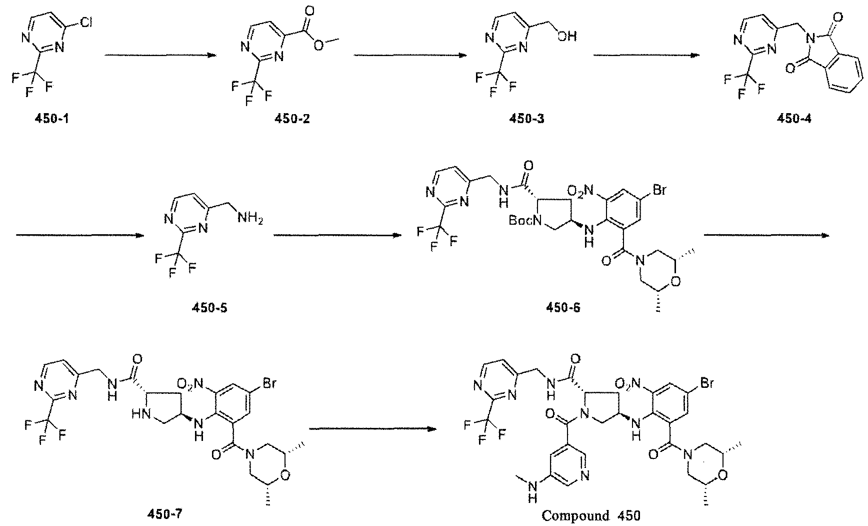

### Operating Steps:

Step A: Compound 450-1 (1.0 g, 5.49 mmol) was dissolved in methanol (50 mL) at room temperature. Subsequently, potassium acetate (808.2 mg, 8.24 mmol) and 1,1'-bis(diphenylphosphino)ferrocene dichloropalladium (4.0 g, 5.49mmol) were successively added to the above solution, and then the reaction system was stirred at 80°C for 16 hours under the atmosphere of carbon monoxide.

After the depletion of the raw material as monitored by LCMS, the reaction solution was filtered and concentrated under reduced pressure. Water (50 mL) was added to the resulting residue to quench the reaction. The mixed solution was extracted with dichloromethane (30 mL × 3 times), and the organic phases were combined. The combined organic phase was washed with saturated saline (40 mL × 2 times), then dried with anhydrous sodium sulfate, filtered and concentrated under reduced pressure. The resulting residue was purified by silica gel column chromatography to obtain Compound 450-2 (370 mg, yield 32.7%).

MS (ESI) M/Z: 207.0 [M+H]⁺.

Step B: Compound 450-2 (370 mg, 1.80 mmol) was dissolved in tetrahydrofuran (10 mL) and ethanol (1 mL) under nitrogen protection at 0°C. Subsequently, sodium borohydride (138 mg, 3.6 mmol) was added to the above solution. Then the reaction system was continued to be stirred for 30 minutes.

After the depletion of the raw material as monitored by LCMS, the reaction solution was slowly added dropwise to water (15 mL) to quench the reaction. The mixed solution was extracted with dichloromethane (20 mL × 3 times), and the organic phases were combined. The combined organic phase was washed with sodium chloride solution (10 mL × 2 times), then dried with anhydrous sodium sulfate, filtered and concentrated under reduced pressure. The resulting residue was purified by silica gel column chromatography to obtain Compound 450-3 (120 mg, yield 37.4%).

MS (ESI) M/Z: 179.0 [M+H]⁺.

Step C: Compound 450-3 (120 mg, 0.67 mmol) was dissolved in anhydrous tetrahydrofuran (3.5 mL) under nitrogen protection at 0°C. Subsequently, phthalimide (191.1 mg, 1.3 mmol), triphenylphosphine (340 mg, 1.3 mmol) and diisopropylazodicarboxylate (203.0 mg, 1.0 mmol) were successively added to the above solution. Then the reaction system was stirred at room temperature for 12 hours.

After the depletion of the raw material as monitored by LCMS, water (10 mL) was added to the reaction solution to quench the reaction. The mixed solution was extracted with dichloromethane (5 mL × 3 times), and the organic phases were combined. The combined organic phase was washed with sodium chloride solution (10 mL × 2 times), then dried with anhydrous sodium sulfate, filtered and concentrated under reduced pressure. The resulting residue was purified by silica gel column chromatography to obtain Compound 450-4 (150 mg, yield 72.9%).

MS (ESI) M/Z: 308.0 [M+H]⁺.

Step D: Compound 450-4 (60 mg, 0.20 mmol) was dissolved in methanol (1.0 mL) under nitrogen protection at room temperature. Subsequently, hydrazine hydrate (50.0 mg, 1.0 mmol) was added to the above solution. Then the reaction system was stirred at 70°C for 1 hour.

After the depletion of the raw material as monitored by LCMS, the reaction solution was distilled and concentrated under reduced pressure. The resulting residue was purified by reversed phase column chromatography to obtain the Compound 450-5 (12 mg, yield 33.8%).

Step E: Compound 450-5 (12 mg, 0.067 mmol) and (2*S*,4*R*)-4-(4-bromo-2-(2*R*,6*S*)-2,6-dimethylmorpholin-4-carbonyl)-6-nitrophenyl)amino)-1-(tert-butoxycarbonyl)pyrrolidine-2-carboxylic acid (41.0 mg, 0.067 mmol) was dissolved in *N*,*N*-dimethylformamide (1.0 mL) at room temperature. Subsequently, the above solution was cooled to 0°C, and 2-(7-azabenzotriazolyl)-*N,N,N',N'-*tetramethyluronium hexafluorophosphate (31.9 mg, 0.084 mmol) and *N,N-*diisopropylethylamine (25.9 mg, 0.20 mmol) were successively added. Then the reaction system was continued to be stirred for 15 minutes.

After the depletion of the raw material as monitored by LCMS, ice water (10 mL) was added to the reaction solution to quench the reaction. The mixed solution was extracted with ethyl acetate (5 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 450-6 (32 mg, yield 65.5%).

MS (ESI) M/Z: 629.5 [M-99].

Step F: Compound 450-6 (32 mg, 0.044 mmol) was dissolved in 4 M hydrochloric acid-1,4-dioxane solution (0.5 mL) and dichloromethane (1.0 mL) under nitrogen protection at room temperature. Then the reaction system was continued to be stirred for 2 hours.

After the depletion of the raw material as monitored by LCMS, the reaction solution was concentrated under reduced pressure to obtain Compound 450-7 (30 mg, yield 108.4%).

MS (ESI) M/Z: 630.0[M+H]⁺.

Step G: Compound 450-7 (30 mg, 0.044 mmol) and 5-(methylamino)nicotinic acid (6.7 mg, 0.044 mmol) were dissolved in *N,N*-dimethylformamide (0.5 mL) at room temperature. Subsequently, the above solution was cooled to 0°C, and 2-(7-azabenzotriazolyl)-*N,N,N',N'*-tetramethyluronium hexafluorophosphate (19.0 mg, 0.05 mmol) and *N,N-*diisopropylethylamine (16.8 mg, 0.13 mmol) were successively added. Then the reaction system was continued to be stirred for 15 minutes.

After the depletion of the raw material as monitored by LCMS, water (15 mL) was added to the reaction solution to quench the reaction. The mixed solution was extracted with ethyl acetate (5 mL × 3 times), and the organic phases were combined. The combined organic phase was washed with saturated sodium chloride solution (10 mL × 2 times), then dried with anhydrous sodium sulfate, filtered and concentrated under reduced pressure. The resulting residue was purified by preparative high performance liquid chromatography to obtain the title Compound 450 (17.2 mg, yield 51.2%).

MS (ESI) M/Z: 763.4 [M+H]⁺.

1H NMR (400 MHz, DMSO-*d*₆) δ 9.17 - 8.75 (m, 2H), 8.35 - 8.16 (m, 1H), 8.10 - 7.94 (m, 1H). 7.94 - 7.45 (m, 4H). 6.98 - 6.80 (m. 0.8H). 6.72 (s. 0.2H), 6.36 - 6.10 (m, 1H), 4.75 - 3.47 (m, 9H), 3.30 - 3.07 (m, 1H), 2.89 - 2.63 (m, 4H), 2.44 - 2.12 (m, 3H), 1.14 - 0.77 (m, 6H).

### Example 451

### (5-Bromo-2-((3S,4S)-4-(methoxy-d₃)-1-(5-((methyl-d₃)amino)nicotinoyl)pyrrolidin-3-yl)amino)-3-nitrophenyl)((2S,6R)-2,6-dimethylmorpholinyl)methanone

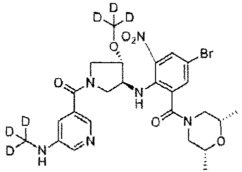

### Reaction route:

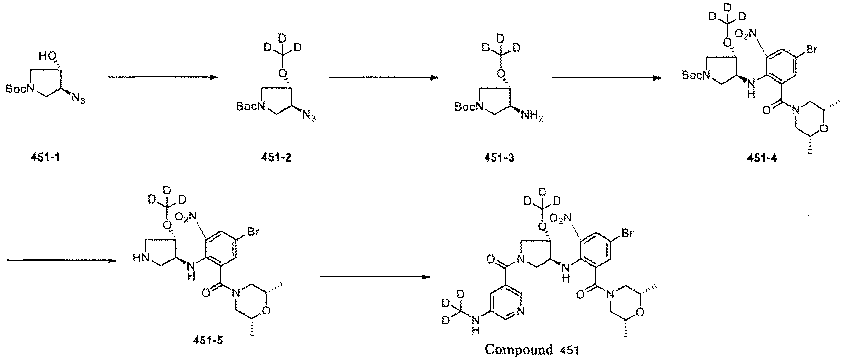

### Operating Steps:

Step A: Compound 451-1 (200 mg, 0.88 mmol) and deuterated iodomethane (1.02 g, 7.04 mmol) were dissolved in acetonitrile (3.0 mL) and water (0.6 mL) at room temperature. Subsequently, silver oxide (600 mg, 2.59 mmol) was added to the above solution. Then the reaction system was continued to be stirred at 60°C for 48 hours.

After the depletion of the raw material as monitored by LCMS, water (25 mL) was added to the reaction solution to quench the reaction. The mixed solution was extracted with ethyl acetate (20 mL × 3 times), and the organic phases were combined. The combined organic phase was washed with saturated sodium chloride solution (20 mL × 2 times), then dried with anhydrous sodium sulfate, filtered and concentrated under reduced pressure. The resulting residue was purified by silica gel column chromatography to obtain Compound 451-2 (165 mg, yield 76.7%).

MS (ESI) M/Z: 146.0 [M-99].

Step B: Compound 451-2 (100 mg, 0.41 mmol) was dissolved in methanol (2.0 mL) at room temperature. Subsequently, platinum dioxide (30 mg) was added to the above solution. Then the reaction system was stirred for 2 hours under hydrogen atmosphere at room temperature.

After the depletion of the raw material as monitored by LCMS, the reaction solution was filtered and the filtrate was concentrated under reduced pressure to obtain Compound 451-3 crude product (100 mg).

MS (ESI) M/Z: 120.2 [M-99].

Step C: Compound 451-3 (100 mg, 0.46 mmol) was dissolved in *N,N*-dimethylformamide (2.3 mL) at room temperature. Subsequently, (5-bromo-2-fluoro-3-nitrophenyl)((2*S*,6*R*)-2,6-dimethylmorpholinyl) methanone (166 mg, 0.46 mmol) and *N,N*-diisopropylethylamine (297 mg, 2.30 mmol) were successively added to the above reaction solution. Then the reaction system was continued to be stirred at 80°C for 1 hour.

After the depletion of the raw material as monitored by LCMS, water (40 mL) was added to the reaction solution to quench the reaction. The mixed solution was extracted with ethyl acetate (10 mL × 3 times), and the organic phases were combined. The combined organic phase was washed with saturated sodium chloride solution (20 mL × 2 times), then dried with anhydrous sodium sulfate, filtered and concentrated under reduced pressure. The resulting residue was purified by silica gel column chromatography to obtain Compound 451-4 (55 mg, yield 22.0%).

MS (ESI) M/Z: 460.0 [M-99].

Step D: Compound 451-4 (55 mg, 0.10 mmol) was dissolved in dichloromethane (1 mL) at room temperature. Subsequently, the above solution was cooled to 0°C, and 4 M hydrochloric acid-1,4-dioxane solution (1 mL) was added dropwise. Then the reaction system was continued to be stirred at room temperature for 2 hours.

After the depletion of the raw material as monitored by LCMS, the reaction solution was concentrated under reduced pressure. Compound 451-5 (45 mg, yield 101%) was obtained.

MS (ESI) M/Z: 460.0 [M+H]⁺.

Step E: Compound 451-5 (38 mg, 0.08 mmol) and 5-((methyl-*d*₃)amino)nicotinic acid (13 mg, 0.08 mmol) were dissolved in *N,N*-dimethylformamide (0.8 mL) at 0°C. Subsequently, 2-(7-azabenzotriazolyl)-*N,N,N',N'*-tetramethyluronium hexafluorophosphate (32 mg, 0.08 mmol) and *N,N*-diisopropylethylamine (32 mg, 0.25 mmol) were successively added to the above reaction solution. Then the reaction system was continued to be stirred for 20 minutes.

After the depletion of the raw material as monitored by LCMS, water (30 mL) was added to the reaction solution to quench the reaction. The mixed solution was extracted with ethyl acetate (10 mL × 3 times), and the organic phases were combined. The combined organic phase was washed with saturated sodium chloride solution (20 mL × 2 times), then dried with anhydrous sodium sulfate, filtered and concentrated under reduced pressure. The resulting residue was purified by preparative high performance liquid chromatography to obtain the title Compound 451 (30.8 mg, yield 62.8%).

MS (ESI) M/Z: 596.8 [M+H]⁺.

¹H NMR (400 MHz, DMSO-*d*₆) δ 8.25 - 8.22 (m, 1H), 8.01 (s, 1H), 7.93 - 7.76 (m. 1H), 7.75 - 7.60 (m, 1H), 7.55 -7.28 (m, 1H), 6.96 - 6.78 (m. 1H), 6.17 (s, 1H). 4.47 - 4.20 (m, 1H), 4.19 - 3.44 (m, 9H), 3.30 - 3.05 (m, 1H), 3.01 - 2.72 (m, 1H). 1.24 - 0.84 (m. 6H).

### Example 452

### (5-Bromo-2-(((3R,5R)-1-(5-(methylamino)nicotinoyl)-5-((S)-tetrahydrofuran-3-yl)oxy)piperidin-3-amino)-3-nitrophenyl)((2S,6R)-2,6-dimethylmorpholinyl)methanone

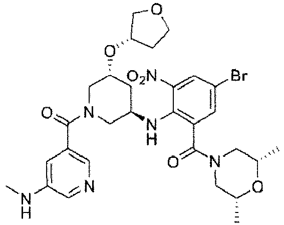

### Reaction route:

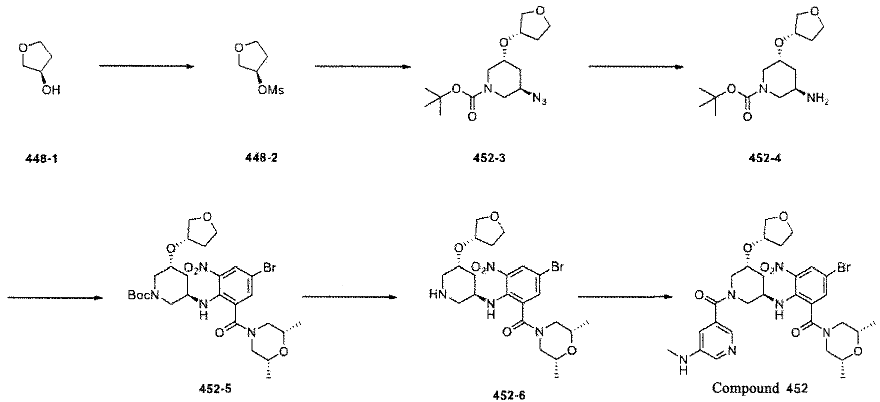

### Operating Steps:

Step A: Compound 448-1 (800 mg, 9.1 mmol) was dissolved in dichloromethane solution (20 mL) under nitrogen protection at room temperature. Subsequently, the above solution was cooled to 0°C, and triethylamine (2.75 g, 27.3 mmol) and methanesulfonyl chloride (1.55 g, 13.65 mmol) were successively slowly added. Then the reaction system was stirred at room temperature for 3 hours.

After the depletion of the raw material as monitored by LCMS, the reaction solution was added to ice water (20 mL) to quench the reaction. The mixed solution was extracted with ethyl acetate (10 mL × 3 times), and the organic phases were combined. The combined organic phase was washed with saturated saline (10 mL × 2 times), then dried with anhydrous sodium sulfate, filtered and concentrated under reduced pressure to obtain Compound 448-2 (1.1 g, yield 72.8%).

Step B: Compound 448-2 (700 mg, 4.21 mmol) was dissolved in anhydrous *N*,*N*-dimethylformamide (20 mL) under nitrogen protection at 0°C. Subsequently, sodium hydride (252.6 mg, 6.32 mmol) was added to the above solution, and the solution was stirred for 30 minutes. Then tert-butyl (3*R*,5*R*)-3-azido-5-hydroxypiperidine-1-carboxylate (1.02 g, 4.21 mmol) was added to the solution, and the solution was warmed to 70°C, and continued to be stirred for 16 hours.

After the depletion of the raw material as monitored by LCMS, water (20 mL) was added to the reaction solution to quench the reaction. The mixed solution was extracted with ethyl acetate (15 mL × 3 times), and the organic phases were combined. The combined organic phase was washed with saturated sodium chloride solution (10 mL × 2 times), then dried with anhydrous sodium sulfate, filtered and concentrated under reduced pressure. The resulting residue was purified by silica gel column chromatography to obtain Compound 452-3 (32 mg, yield 2.4%).

MS (ESI) M/Z: 213.0 [M-99].

Step C: Compound 452-3 (32 mg, 0.1 mmol) was dissolved in methanol (2 mL) at room temperature. Subsequently, platinum dioxide (6 mg) was added to the above reaction solution. Then the reaction system was stirred for 2 hours under hydrogen atmosphere.

After the depletion of the raw material as monitored by LCMS, the reaction solution was filtered and the filtrate was concentrated under reduced pressure to obtain Compound 452-4 (27 mg, yield 94.4%).

MS (ESI) M/Z: 187.0 [M-99]⁺.

Step D: Compound 452-4 (27 mg, 0.09 mmol) was dissolved in *N*,*N*-dimethylformamide (1 mL) under nitrogen protection at room temperature. Subsequently, (5-bromo-2-fluoro-3-nitrophenyl)((2S,6R)-2,6-dimethylmorpholinyl)methanone (36.1 mg, 0.1 mmol) and *N*,*N*-diisopropylethylamine (39.0 mg, 0.3 mmol) were successively added to the above reaction solution. Then the reaction system was stirred at 80°C for 12 hours.

After the depletion of the raw material as monitored by LCMS, water (15 mL) was added to the reaction solution to quench the reaction. The mixed solution was extracted with ethyl acetate (5 mL × 3 times), and the organic phases were combined. The combined organic phase was washed with saturated sodium chloride solution (10 mL × 2 times), then dried with anhydrous sodium sulfate, filtered and concentrated under reduced pressure. The resulting residue was purified by silica gel column chromatography to obtain Compound 452-5 (38 mg, yield 60.7%).

MS (ESI) M/Z: 527.0 [M-99]⁺.

Step E: Compound 452-5 (38 mg, 0.06 mmol) was dissolved in 4 M hydrochloric acid-1,4-dioxane solution (0.3 mL) and dichloromethane (0.6 mL) under nitrogen protection at room temperature. Then the reaction system was stirred at room temperature for 2 hours.

After the depletion of the raw material as monitored by LCMS, the reaction solution was distilled and concentrated under reduced pressure. Compound 452-6 crude product (32 mg) was obtained.

MS (ESI) M/Z: 527.0 [M+H]⁺.

Step F: Compound 452-6 (32 mg, 0.06 mmol) was dissolved in *N,N*-dimethylformamide (1 mL) at room temperature. Subsequently, the above solution was cooled to 0°C, and 5-(methylamino)nicotinic acid (9.0 mg, 0.06 mmol), 2-(7-azabenzotriazol)-*N,N,N',N*'-tetramethyluronium hexafluorophosphate (26.6 mg, 0.07 mmol) and *N*,*N*-diisopropylethylamine (23.2 mg, 0.18 mmol) were successively added. Then the reaction system was stirred at 0°C for 15 minutes.

After the depletion of the raw material as monitored by LCMS, water (15 mL) was added to the reaction solution to quench the reaction. The mixed solution was extracted with ethyl acetate (5 mL × 3 times), and the organic phases were combined. The combined organic phase was washed with saturated saline (20 mL × 2 times), then dried with anhydrous sodium sulfate, filtered and concentrated under reduced pressure. The resulting residue was purified by reversed phase column chromatography to obtain the title Compound 452 (12.5 mg, yield 31.5%).

MS (ESI) M/Z: 660.8 [M+H]⁺.

¹H NMR (400 MHz, DMSO-*d*₆) δ 8.24 (s, 1H). 8.05 - 7.82 (m, 1H), 7.79 - 7.24 (m, 3H), 6.86 - 6.54 (in, 1H), 6.24 - 5.88 (m, 1H), 4.64 - 4.06 (m, 2H), 4.02 - 3.36 (m. 11H), 3.28 - 2.76 (m, 3H). 2.70 (d, *J* = 4.4 Hz. 3H), 2.47 - 2.35 (m. 1H), 2.16 - 1.61 (m, 4H), 1.21 - 1.08 (m, 3H), 1.02 (brs, 3H).

### Example 453

### (2S,4R)-4-((4-bromo-2-((2R,6S)-2,6-dimethylmorpholin-4-carbonyl)-6-nitrophenyl)amino)-N-(-3,3-difluoro-1-(2-methoxyethyl)piperidin-4-yl)-1-(5-(methylamino)nicotinoyl)pyrrolidine-2-formamide

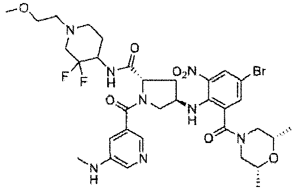

### Reaction route:

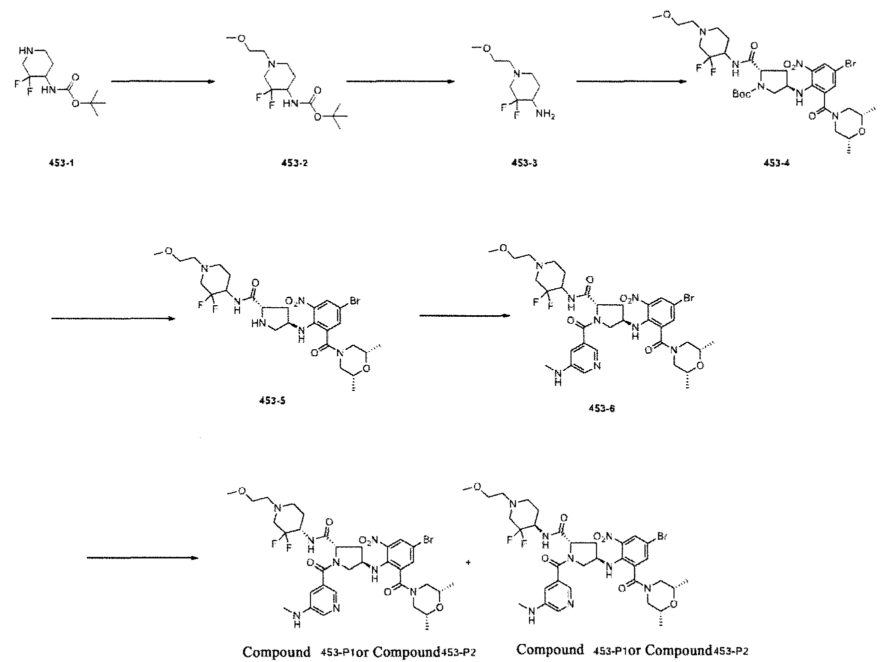

### Operating Steps:

Step A: Compound 453-1 (258 mg, 1.09 mmol) was dissolved in acetonitrile (5.0 mL) at room temperature. Subsequently, potassium carbonate (449 mg, 3.25 mmol) and 1-bromo-2-methoxyethane (362 mg, 2.62 mmol) were added to the above solution. Then the reaction system was stirred at 50°C for 2 hours.

After the depletion of the raw material as monitored by LCMS, water (30 mL) was added to the reaction solution to quench the reaction. The mixed solution was extracted with ethyl acetate (60 mL × 3 times), and the organic phases were combined. The combined organic phase was washed with saturated sodium chloride solution (20 mL × 2 times), then dried with anhydrous sodium sulfate, filtered and concentrated under reduced pressure. The resulting residue was purified by silica gel column chromatography to obtain Compound 453-2 (136 mg, yield 45.8%).

MS (ESI) M/Z: 295.0 [M+H]⁺.

Step B: Compound 453-2 (136 mg, 0.46 mmol) was dissolved in dichloromethane (2.0 mL) at room temperature. Subsequently, the above solution was cooled to 0°C, and 4 M hydrochloric acid-1,4-dioxane solution (2.0 mL) was added dropwise. Then the reaction system was stirred at room temperature for 3 hours.

After the depletion of the raw material as monitored by LCMS, the reaction solution was distilled and concentrated under reduced pressure. Compound 453-3 crude product (140 mg) was obtained.

MS (ESI) M/Z: 195.2 [M+H]⁺.

Step C: Compound 453-3 (140 mg, 0.72 mmol) was dissolved in *N,N*-dimethylformamide (3.6 mL) at 0°C. Subsequently, (2*S*,4*R*)-4-((4-bromo-2-((2*R*,6*S*)-2,6-dimethylmorpholin-4-carbonyl)-6-nitrophenyl)amino)-1-(tert-butoxycarbonyl)pyrrolidine-2-carboxylic acid (400 mg, 0.72 mmol), 2-(7-oxobenzotriazol)-*N*,*N*,*N'*,*N'*-tetramethyluronium hexafluorophosphate (356 mg, 0.94 mmol) and *N*,*N-*diisopropylethylamine (279 mg, 2.2 mmol) were successively added to the above solution. Then the reaction system was stirred at room temperature for 10 minutes.

After the depletion of the raw material as monitored by LCMS, water (30 mL) was added to the reaction solution to quench the reaction. The mixed solution was extracted with ethyl acetate (60 mL × 3 times), and the organic phases were combined. The combined organic phase was washed with saturated sodium chloride solution (20 mL × 2 times), then dried with anhydrous sodium sulfate, filtered and concentrated under reduced pressure. The resulting residue was purified by silica gel column chromatography to obtain Compound 453-4 (125 mg, yield 23%).

MS (ESI) M/Z: 747.1 [M+H]⁺.

Step D: Compound 453-4 (125 mg, 0.17 mmol) was dissolved in dichloromethane (16.0 mL) at room temperature. Subsequently, the above solution was cooled to 0°C, and 4 M hydrochloric acid-1,4-dioxane solution (8.0 mL) was added dropwise. Then the reaction system was stirred at room temperature for 3 hours.

After the depletion of the raw material as monitored by LCMS, the reaction solution was distilled and concentrated under reduced pressure. Compound 453-5 crude product (130 mg) was obtained.

MS (ESI) M/Z: 647.2 [M+H]⁺.

Step E: Compound 453-5 (130 mg, 0.20 mmol) was dissolved in *N*,*N*-dimethylformamide (1.0 mL) at 0°C. Subsequently, 5-(methylamino)nicotinic acid (30.6 mg, 0.20 mmol), 2-(7-azabenzotriazol)-*N*,*N*,*N'*,*N'*-tetramethyluronium hexafluorophosphate (99.3 mg, 0.26 mmol) and *N,N*-diisopropylethylamine (77.8 mg, 0.60 mmol) were successively added to the above solution. Then the reaction system was stirred at room temperature for 10 minutes.

After the depletion of the raw material as monitored by LCMS, water (20 mL) was added to the reaction solution to quench the reaction. The mixed solution was extracted with ethyl acetate (30 mL × 3 times), and the organic phases were combined. The combined organic phase was washed with saturated sodium chloride solution (20 mL × 2 times), then dried with anhydrous sodium sulfate, filtered and concentrated under reduced pressure to obtain Compound 453-6. The resulting residue was separated by chiral SFC, chiral chromatography column: YMC Cellulose-SC (4.6*100mm, 3µm), mobile phase: carbon dioxide/methanol (containing 0.2% ammonia) = 60/40, flow rate: 3 mL/min. The products were collected to obtain Compound 453-P1 (14.72 mg, retention time 2.985 min) and Compound 453-P2 (15.20 mg, retention time 4.548 min).

### Compound 453-P1:

MS (ESI) M/Z: 781.1 [M+H]⁺.

¹H NMR (400 MHz, DMSO-*d*₆) δ 8.37 - 8.14 (m. 1.7H), 8.13 - 7.88 (m, 1.3H), 7.87 - 7.72 (m, 1H), 7.71 - 7.52 (m, 2H), 6.88 - 6.76 (m. 0.7H), 6.65 (s, 0.3H), 6.22 - 6.09 (m, 0.7H). 6.04 - 5.90 (m. 0.3H), 4.77 - 4.54 (m, 0.7H), 4.51 - 3.36 (m. 10.3H). 3.22 - 3.19 (m. 3H), 3.18 - 2.97 (m, 1H), 2.94 - 2.63 (m. 5H), 2.62 - 2.54 (m, 2H), 2.47 - 1.89 (m, 5H), 1.78 - 1.36 (m. 2H). 1.19 - 1.09 (m, 3H), 1.08 - 0.88 (m. 3H).

### Compound 453-P2:

MS (ESI) M/Z: 781.1 [M+H]⁺.

¹H NMR (400 MHz, DMSO-*d*₆) δ 8.36 - 8.14 (m, 1.6H), 8.09 - 7.90 (m, 1.4H), 7.88 - 7.52 (m, 3H), 6.87 -6.75 (m, 0.6H), 6.66 (s, 0.4H), 6.24 - 6.12 (m, 0.6H). 6.11 - 6.01 (m, 0.4H), 4.72 - 4.56 (m, 0.6H), 4.53 - 3.35 (m, 10.4H). 3.25 - 3.18 (m. 3H). 3.17 - 2.97 (m. 1H). 2.94 - 2.64 (m. 5H). 2.62 - 2.53 (m, 2H), 2.45 - 1.92 (m, 5H), 1.79 - 1.36 (m, 2H), 1.18 - 0.88 (m, 6H).

### Example 454

### (5-Bromo-2-(((3S,4S)-4-methoxy-1-(2-methyl-3H-1,2,3-triazole[4,5-c]pyridin-7-carbonyl)pyrrolidin-3-yl)amino)-3-nitrophenyl)((2S,6R)-2,6-dimethylmorpholinyl)methanone

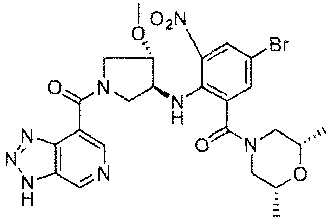

### Reaction route:

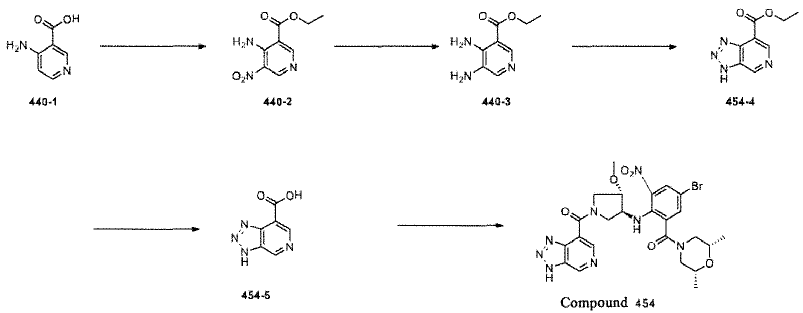

### Operating Steps:

Step A: Compound 440-1 (2.2 g, 15.9 mmol) was dissolved in concentrated sulfuric acid (21 mL) at 0°C. Subsequently, potassium nitrate (1.6 g, 15.9 mmol) was added to the above solution, then the reaction system was warmed to 30°C, and stirred for 3 hours, then warmed to 70°C, and continued to be stirred for 3 hours. Then the reaction system was cooled to 0°C, added with ethanol (8.4 mL), warmed to 60°C and stirred for 18 hours.

After the depletion of the raw material as monitored by LCMS, the reaction solution was added to ice potassium acetate solution to quench the reaction, and then saturated sodium bicarbonate solution was used to adjust the pH value to 7 to 8. The mixed solution was extracted with ethyl acetate (100 mL × 3 times), and the organic phases were combined. The combined organic phase was washed with saturated sodium chloride solution (500 mL × 2 times), then dried with anhydrous sodium sulfate, filtered and concentrated under reduced pressure. The resulting residue was purified by silica gel column chromatography to obtain Compound 440-2 (788 mg, yield 23.5%).

MS (ESI) M/Z: 212.0 [M+H]⁺.

Step B: Compound 440-2 (788 mg, 3.73 mmol) was dissolved in ethanol (18 mL) at room temperature. Subsequently, 10% Palladium/Carbon (78 mg) was added to the above solution. Then the reaction system was continued to be stirred for 6 hours under hydrogen atmosphere.

After the depletion of the raw material as monitored by LCMS, the reaction solution was filtered and the filtrate was concentrated under reduced pressure to obtain Compound 440-3 (672 mg, yield 99%).

MS (ESI) M/Z: 182[M+H]⁺.

Step C: Compound 440-3 (200 mg, 1.1 mmol) was dissolved in hydrochloric acid (6 mL) at room temperature. Subsequently, sodium nitrite (343 mg, 4.97 mmol) was dissolved in water (6 mL) and added dropwise to the above solution. Then the reaction system was stirred at room temperature for 10 minutes.

After the depletion of the raw material as monitored by LCMS, water (36 mL) was added to the reaction solution to dilute, and then saturated sodium bicarbonate solution was used to adjust the pH value to 7 to 8. Then the mixture was freeze-dried under reduced pressure to obtain Compound 454-4 (178 mg, yield 84%).

MS (ESI) M/Z: 193.0 [M+H]⁺.

Step D: Compound 454-4 (70 mg, 0.36 mmol) was dissolved in the solution of tetrahydrofuran (1.8 mL) and water (0.9 mL) at room temperature. Subsequently, lithium hydroxide (31 mg, 0.72 mmol) was added to the above solution. Then the reaction system was continued to be stirred for 8 hours.

After the depletion of the raw material as monitored by LCMS, the reaction solution was concentrated under reduced pressure to obtain Compound 454-5 (46 mg, yield 78%).

MS (ESI) M/Z: 165.2 [M+H]⁺.

Step E: Compound 454-5 (63 mg, 0.36 mmol) and (5-bromo-2-(((3S,4S)-4-methoxypyrrolidin-3-yl)amino-3-nitrophenyl)((2*S*,6*R*)-2,6-dimethylmorpholinyl)methanone (52 mg, 0.23 mmol) were dissolved in acetonitrile (0.6 mL) at room temperature. Subsequently, N-methylimidazole (42.2 mg, 0.57 mmol) and *N*,*N*,*N'*,*N'*-tetramethylchloroformamidinium hexafluorophosphate (48 mg, 0.17 mmol) were successively added to the above solution. Then the reaction system was continued to be stirred for 30 minutes.

After the depletion of the raw material as monitored by LCMS, water (10 mL) was added to the reaction solution to quench the reaction. The mixed solution was extracted with ethyl acetate (3 mL × 3 times), and the organic phases were combined. The combined organic phase was washed with saturated sodium chloride solution (10 mL × 2 times), then dried with anhydrous sodium sulfate, filtered and concentrated under reduced pressure. The crude product was purified by preparative high performance liquid chromatography to obtain the title Compound 454 (23.65 mg, yield 33%).

MS (ESI) M/Z: 603.2 [M+H]⁺.

¹H NMR (400 MHz, DMSO-*d*₆) δ 9.62 - 9.47 (m, 1H), 8.76 - 8.53 (m, 1H), 8.33 - 8.12 (m, 1H), 7.82 - 7.31 (m, 2H). 4.52 - 3.53 (m, 9H). 3.21 - 2.71 (m, 5H), 2.47 - 2.21 (m, 1H), 1.24 - 0.94 (m, 5H), 0.90 - 0.72 (m, 1H).

### Example 455

### (2-((3S,4S)-4-(benzyloxy)-1-(5-(methylamino)nicotinoyl)pyrrolidin-3-yl)amino)-5-bromo-3-nitrophenyl)((2S,6R)-2,6-dimethylmorpholinyl)methanone

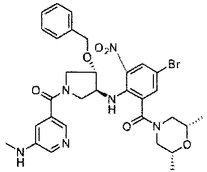

### Reaction route:

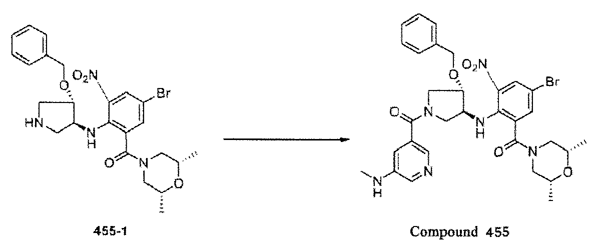

### Operating Steps:

Step A: Compound 455-1 (53.2 mg, 0.1 mmol) was dissolved in *N,N*-dimethylformamide (1 mL) at room temperature. Subsequently, the above solution was cooled to 0°C, and 5-(methylamino)nicotinic acid (15.2 mg, 0.1 mmol), 2-(7-azabenzotriazol)-*N*,*N*,*N'*,*N'*-tetramethyluronium hexafluorophosphate (45 mg, 0.12 mmol) and *N,N*-diisopropylethylamine (39 mg, 0.3 mmol) were successively added. Then the reaction system was stirred at 0°C for 15 minutes.

After the depletion of the raw material as monitored by LCMS, water (15 mL) was added to the reaction solution to quench the reaction. The mixed solution was extracted with ethyl acetate (5 mL × 3 times), and the organic phases were combined. The combined organic phase was washed with saturated saline (20 mL × 2 times), then dried with anhydrous sodium sulfate, filtered and concentrated under reduced pressure. The resulting residue was purified by reversed phase column chromatography. The title Compound 455 (35.6 mg, yield 53.3%) was obtained.

MS (ESI) M/Z: 667.0 [M+H]⁺.

¹H NMR (400 MHz, DMSO-*d*₆) δ 8.31 - 8.16 (m, 1H), 8.00 (s, 1H), 7.91 - 7.76 (m, 1H), 7.75 - 7.61 (m. 1H), 7.60 - 7.02 (m, 6H), 6.94 - 6.79 (m, 1H). 6.15 (s. 1H), 4.69 - 3.38 (m, 11H), 3.27 - 3.01 (m, 1H). 2.95 - 2.65 (m. 4H), 2.47 - 1.82 (m, 1H), 1.18 - 0.77 (m. 6H).

### Example 456

### (5-Bromo-2-(((3S,4S)-4-((6-methoxypyridin-3-yl)methoxy)-1-(5-methylamino)nicotinoyl)pyrrolidin-3-amino)-3-nitrophenyl)((2S,6R)-2,6-dimethylmorpholinyl)methanone

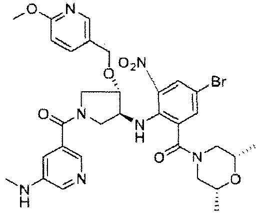

### Reaction route:

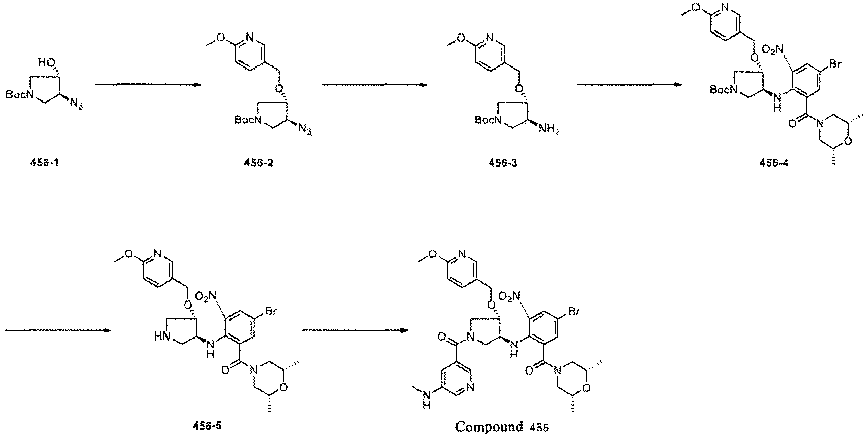

### Operating Steps:

Step A: Compound 456-1 (80 mg, 0.35 mmol) was dissolved in *N*,*N*-dimethylformamide (1.7 mL) under nitrogen protection at room temperature. Subsequently, the above solution was cooled to 0°C, sodium hydride (21.2 mg, 0.53 mmol) was added, and the solution was continued to be stirred for 30 minutes. Then 5-(chloromethyl)-2-methoxypyridine (66 mg, 0.42 mmol) was added to the above solution. The reaction system was warmed to room temperature, and continued to be stirred for 1 hour.

After the depletion of the raw material as monitored by LCMS, water (10 mL) was added to the reaction solution to quench the reaction. The mixed solution was extracted with ethyl acetate (10 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 456-2 (87 mg, yield 71%).

MS (ESI) M/Z: 294.1 [M-55].

Step B: Compound 456-2 (87 mg, 0.25 mmol) was dissolved in tetrahydrofuran (12.5 mL) and water (0.13 mL) under nitrogen protection at room temperature. Subsequently, triphenylphosphine (97 mg, 0.37 mmol) was added to the above solution. Then the reaction system was stirred at room temperature for 48 hours.

After the depletion of the raw material as monitored by LCMS, water (10 mL) was added to the reaction solution to quench the reaction. Six drops of 1 M hydrochloric acid solution was added, and the mixed solution was extracted with ethyl acetate (15 mL × 2 times). Aqueous phase was concentrated under reduced pressure to obtain Compound 456-3 (77 mg, yield 96%).

MS (ESI) M/Z: 268.1 [M-55].

Step C: Compound 456-3 (77 mg, 0.24 mmol) was dissolved in *N*,*N*-dimethylformamide (1.2 mL) under nitrogen protection at room temperature. Subsequently, (5-bromo-2-fluoro-3-nitrophenyl)((2*S*,6*R*)-2,6-dimethylmorpholinyl)methanone (86 mg, 0.24 mmol) and *N*,*N*-diisopropylethylamine (93 mg, 0.72 mmol) were successively added to the above solution. Then the reaction system was stirred at 80°C for 3 hours.

After the depletion of the raw material as monitored by LCMS, water (20 mL) was added to the reaction solution to quench the reaction. The mixed solution was extracted with ethyl acetate (20 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 456-4 (120 mg, yield 70%).

MS (ESI) M/Z: 663.8 [M+H]⁺.

Step D: Compound 456-4 (120 mg, 0.18 mmol) was dissolved in dichloromethane (4.5 mL) under nitrogen protection at room temperature. Subsequently, the above solution was cooled to 0°C, and 4 M hydrochloric acid-1,4-dioxane solution (4.5 mL) was slowly added. Then the reaction system was stirred at room temperature for 1 hour.

After the depletion of the raw material as monitored by LCMS, the reaction solution was distilled and concentrated under reduced pressure. Compound 456-5 crude product (110 mg) was obtained.

MS (ESI) M/Z: 564.0 [M+H]⁺.

Step E: 5-(Methylamino)nicotinic acid (15 mg, 0.10 mmol) was dissolved in *N*,*N*-dimethylformamide (0.5 mL) at room temperature. Subsequently, the above solution was cooled to 0°C, and 2-(7-azabenzotriazolyl)-*N*,*N*,*N'*,*N'-*tetramethyluronium hexafluorophosphate (38 mg, 0.10 mmol), *N*,*N*-diisopropylethylamine (35 mg, 0.27 mmol) and Compound 456-5 (50 mg, 0.09 mmol) were successively added. Then the reaction system was continued to be stirred for 10 minutes.

After the depletion of the raw material as monitored by LCMS, water (10 mL) was added to the reaction solution to quench the reaction. The mixed solution was extracted with ethyl acetate (15 mL × 3 times), and the organic phases were combined. The combined organic phase was washed with saturated saline (20 mL × 2 times), then dried with anhydrous sodium sulfate, filtered and concentrated under reduced pressure. The resulting residue was purified by preparative high performance liquid chromatography to obtain the title Compound 456 (19.69 mg, yield 31%).

MS (ESI) M/Z: 697.5 [M+H]⁺.

¹H NMR (400 MHz, DMSO-*d₆*) *δ* 8.36 - 7.14 (m, 6H), 7.05 - 6.52 (m, 2H). 6.15 (s, 1H), 4.70 - 3.99 (m, 4H), 3.94 - 3.71 (m, 4H), 3.69 - 3.43 (m. 4H), 3.19 - 2.97 (m. 3H), 2.91 - 2.81 (m, 1H), 2.76 - 2.64 (m, 3H), 2.39 - 2.29 (m, 1H), 1.33 - 0.45 (m, 6H).

### Example 457

### (5-Bromo-2-((3S,4S)-1-(5-(methylamino)nicotinoyl)-4-((6-(trifluoromethyl)pyridin-3-yl)methoxy)pyrrolidin-3-amino)-3-nitrophenyl)((2S,6R)-2,6-dimethylmorpholinyl)methanone

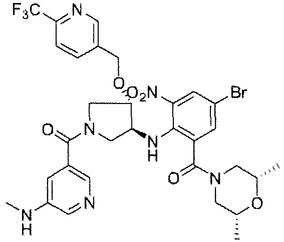

### Reaction route:

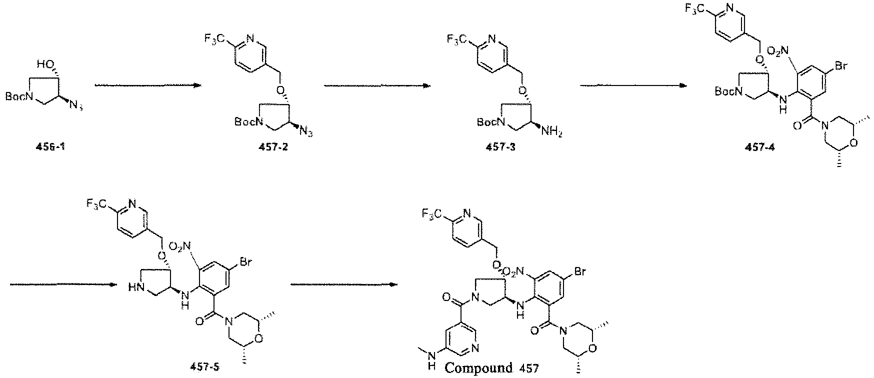

### Operating Steps:

Step A: Compound 456-1 (50 mg, 0.22 mmol) was dissolved in *N,N*-dimethylformamide (1 mL) under nitrogen protection at room temperature. Subsequently, the above solution was cooled to 0°C, sodium hydride (13 mg, 0.33 mmol) was added, and the solution was continued to be stirred for 30 minutes. Then 5-(bromomethyl)-2-(trifluoromethyl)pyridine was added to the above solution. Then the reaction system was stirred at room temperature for 1 hour.

After the depletion of the raw material as monitored by LCMS, water (10 mL) was added to the reaction solution to quench the reaction. The mixed solution was extracted with ethyl acetate (10 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 457-2 (60 mg, yield 70%).

MS (ESI) M/Z: 332.0 [M-55].

Step B: Compound 457-2 (60 mg, 0.16 mmol) was dissolved in the solution of tetrahydrofuran (1 mL) and water (0.1 mL) under nitrogen protection at room temperature. Subsequently, triphenylphosphine (60 mg, 0.23 mmol) was added to the above solution. Then the reaction system was stirred at room temperature for 48 hours.

After the depletion of the raw material as monitored by LCMS, water (2 mL) was added to the reaction solution to quench the reaction. Two drops of 1 M hydrochloric acid solution was added, and the mixed solution was extracted with ethyl acetate (5 mL × 2 times). Then the resulting aqueous phase was concentrated under reduced pressure to obtain Compound 457-3 crude product (60 mg).

MS (ESI) M/Z: 306.0 [M-55].

Step C: Compound 457-3 (60 mg, 0.17 mmol) was dissolved in *N,N*-dimethylformamide (0.7 mL) at room temperature. Subsequently, (5-bromo-2-fluoro-3-nitrophenyl)((2S,6R)-2,6-dimethylmorpholinyl)methanone (60 mg, 0.17 mmol) and *N,N*-diisopropylethylamine (0.14 mL, 0.85 mmol) were successively added to the above solution. Then the reaction system was stirred at 80°C for 5 hours.

After the depletion of the raw material as monitored by LCMS, water (10 mL) was added to the reaction solution to quench the reaction. The mixed solution was extracted with ethyl acetate (10 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 457-4 (60 mg, yield 50%).

MS (ESI) M/Z: 602.0 [M-99].

Step D: Compound 457-4 (36 mg, 0.05 mmol) was dissolved in dichloromethane (0.3 mL) under nitrogen protection at room temperature. Subsequently, the above solution was cooled to 0°C, and 4 M hydrochloric acid-1,4-dioxane solution (0.5 mL) was slowly added. Then the reaction system was stirred at room temperature for 1 hour.

After the depletion of the raw material as monitored by LCMS, the reaction solution was distilled and concentrated under reduced pressure to obtain Compound 457-5 crude product (52 mg).

MS (ESI) M/Z: 602.0 [M+H]⁺.

Step E: 5-(Methylamino)nicotinic acid (6 mg, 0.04 mmol) was dissolved in *N*,*N*-dimethylformamide (0.5 mL) at room temperature. Subsequently, the above solution was cooled to 0°C, and 2-(7-azabenzotriazolyl)-*N*,*N*,*N'*,*N'-*tetramethyluronium hexafluorophosphate (18 mg, 0.05 mmol), *N,N*-diisopropylethylamine (0.05 mL, 0.30 mmol) and Compound 457-5 (25 mg, 0.04 mmol) were successively added. Then the reaction system was continued to be stirred for 10 minutes.

After the depletion of the raw material as monitored by LCMS, water (10 mL) was added to the reaction solution to quench the reaction. The mixed solution was extracted with ethyl acetate (15 mL × 3 times), and the organic phases were combined. The combined organic phase was washed with saturated saline (20 mL × 2 times), then dried with anhydrous sodium sulfate, filtered and concentrated under reduced pressure. The resulting residue was purified by preparative high performance liquid chromatography to obtain the title Compound 457 (4.70 mg, yield 16%).

MS (ESI) M/Z: 734.0 [M+H]⁺.

¹H NMR (400 MHz, DMSO-*d₆*) δ 8.83 - 8.52 (m, 1H), 8.33 - 8.15 (m, 1H), 8.13 - 7.77 (m, 4H), 7.74 - 7.28 (m, 2H), 6.98 - 6.77 (m, 1H). 6.22 (s, 1H), 4.88 - 4.50 (m, 2H). 4.46 - 3.42 (m, 9H), 3.28 - 3.03 (m. 1H), 2.92 - 2.68 (m, 4H), 2.45 - 2.35 (m, 1H), 1.16 - 0.83 (m. 6H).

### Example 458

### Bromo-2-((3S,4S)-1-(5-(methylamino)nicotinoyl)-4-(pyridin-2-ylmethoxy)pyrrolidin-3-yl)amino)-3-nitrophenyl)((2S,6R)-2,6-dimethylmorpholinyl)methanone

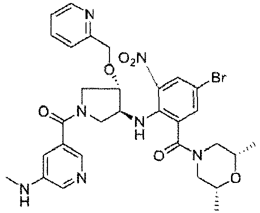

### Reaction route:

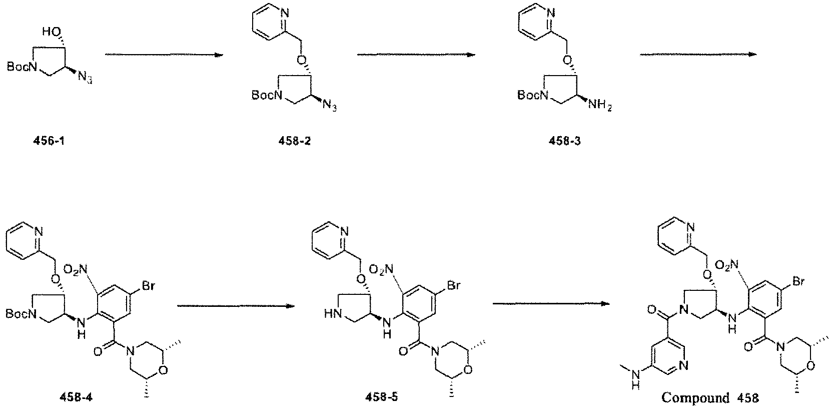

### Operating Steps:

Step A: Compound 456-1 (60.0 mg, 0.26 mmol) was dissolved in *N*,*N*-dimethylformamide (2.0 mL) under nitrogen protection at room temperature. Subsequently, the above solution was cooled to 0°C, 60% sodium hydride (26 mg, 0.65 mmol) was added, and the reaction system was stirred for 30 minutes. Then 2-(chloromethyl)pyridine (49.5 mg, 0.39 mmol) was added to the above reaction system, then the reaction system was warmed to room temperature, and continued to be stirred for 2 hours.

After the depletion of the raw material as monitored by LCMS, the reaction solution was slowly added dropwise to ice water (15 mL) to quench the reaction. The mixed solution was extracted with ethyl acetate (5 mL × 3 times), and the organic phases were combined. The combined organic phase was washed with saturated saline (10 mL × 2 times), then dried with anhydrous sodium sulfate, filtered and concentrated under reduced pressure. The resulting residue was purified by silica gel column chromatography to obtain Compound 458-2 (45 mg, yield 54.2%).

MS (ESI) M/Z: 320.0 [M+H]⁺.

Step B: Compound 458-2 (45.0 mg, 0.14 mmol) was dissolved in the solution of tetrahydrofuran (0.9 mL) and water (0.1 mL) under nitrogen protection at room temperature. Subsequently, triphenylphosphine (55.0 mg, 0.21 mmol) was added to the above solution, and continued to be stirred for 16 hours.

After the depletion of the raw material as monitored by LCMS, the reaction solution was slowly added dropwise to ice water (5 mL) to quench the reaction, the mixed solution was extracted with ethyl acetate (8 mL × 3 times), and the aqueous phases were collected. The collected aqueous phase was concentrated under reduced pressure to obtain Compound 458-3 (28 mg, yield 68.2%).

MS (ESI) M/Z: 294.2 [M+H]⁺.

Step C: Compound 458-3 (28 mg, 0.1 mmol) was dissolved in *N,N*-dimethylformamide (1 mL) under nitrogen protection at room temperature. Subsequently, (5-bromo-2-fluoro-3-nitrophenyl)((2S,6R)-2,6-dimethylmorpholinyl)methanone (36 mg, 0.1 mmol) and *N,N*-diisopropylethylamine (39 mg, 0.3 mmol) were successively added to the above reaction solution. Then the reaction system was stirred at 80°C for 12 hours.

After the depletion of the raw material as monitored by LCMS, water (10 mL) was added to the reaction solution to quench the reaction. The mixed solution was extracted with ethyl acetate (5 mL × 3 times), and the organic phases were combined. The combined organic phase was washed with saturated sodium chloride solution (10 mL × 2 times), then dried with anhydrous sodium sulfate, filtered and concentrated under reduced pressure. The resulting residue was purified by silica gel column chromatography to obtain Compound 458-4 (39 mg, yield 29.7%).

MS (ESI) M/Z: 634.0 [M+H]⁺.

Step D: Compound 458-4 (39 mg, 0.06 mmol) was dissolved in 4 M hydrochloric acid-1,4-dioxane solution (0.6 mL) and dichloromethane (0.6 mL) under nitrogen protection at room temperature. Then the reaction system was stirred at room temperature for 2 hours.

After the depletion of the raw material as monitored by LCMS, the reaction solution was distilled and concentrated under reduced pressure. Compound 458-5 crude product (35 mg) was obtained.

MS (ESI) M/Z: 534.0 [M+H]⁺.

Step E: Compound 458-5 (35 mg, 0.06 mmol) was dissolved in *N*,*N*-dimethylformamide (1 mL) at room temperature. Subsequently, the above solution was cooled to 0°C, and 5-(methylamino)nicotinic acid (9 mg, 0.06 mmol), 2-(7-azabenzotriazol)-*N*,*N*,*N'*,*N'*-tetramethyluronium hexafluorophosphate (31 mg, 0.08 mmol) and *N*,*N*-diisopropylethylamine (31 mg, 0.24 mmol) were successively added. Then the reaction system was stirred at 0°C for 15 minutes.

After the depletion of the raw material as monitored by LCMS, water (15 mL) was added to the reaction solution to quench the reaction. The mixed solution was extracted with ethyl acetate (5 mL × 3 times), and the organic phases were combined. The combined organic phase was washed with saturated saline (20 mL × 2 times), then dried with anhydrous sodium sulfate, filtered and concentrated under reduced pressure. The resulting residue was purified by reversed phase column chromatography to obtain the title Compound 458 (12.3 mg, yield 30.7%).

MS (ESI) M/Z: 667.5 [M+H]⁺.

¹H NMR (400 MHz, DMSO-*d*₆) δ 8.58 - 8.35 (m, 1H), 8.31 - 8.14 (m, 1H), 8.00 (s, 1H), 7.92 - 7.15 (m, 6H), 6.99 - 6.75 (m, 1H), 6.14 (s, 1H), 4.91 - 4.03 (m, 10H), 3.28 - 2.98 (m, 2H), 2.94 - 2.80 (m, 1H), 2.71 (s, 3H), 2.44 - 2.23 (m, 1H), 1.26 - 0.73 (m. 6H).

### Example 459

### (5-Bromo-2-((3S,4S)-4-((5-fluoropyridin-2-yl)methoxy)-1-(5-methylamino)nicotinoyl)pyrrolidin-3-yl)amino)-3-nitrophenyl)((2S,6R)-2,6-dimethylmorpholinyl)methanone

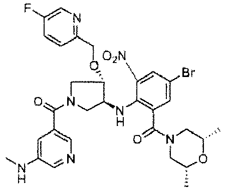

### Reaction route:

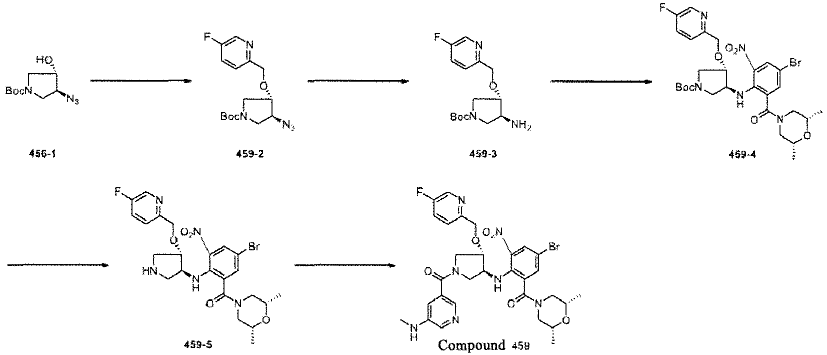

### Operating Steps:

Step A: Compound 456-1 (228 mg, 1.00 mmol) was dissolved in *N,N*-dimethylformamide (5.0 mL) under nitrogen protection at room temperature. Subsequently, the above solution was cooled to 0°C, sodium hydride (80 mg, 2.00 mmol) was added, and the solution was continued to be stirred for 30 minutes. Then 2-(chloromethyl)-5-fluoropyridine (189 mg, 1.30 mmol) was added to the above solution. Then the reaction system was stirred at room temperature for 1 hour.

After the depletion of the raw material as monitored by LCMS, water (40 mL) was added to the reaction solution to quench the reaction. The mixed solution was extracted with ethyl acetate (10 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 459-2 (310 mg, yield 91%).

MS (ESI) M/Z: 282.0 [M-55].

Step B: Compound 459-2 (310 mg, 0.92 mmol) was dissolved in the solution of tetrahydrofuran (4.6 mL) and water (0.5 mL) under nitrogen protection at room temperature. Subsequently, triphenylphosphine (724 mg, 2.76 mmol) was added to the above solution. Then the reaction system was stirred at room temperature for 24 hours.

After the depletion of the raw material as monitored by LCMS, water (20 mL) was added to the reaction solution to quench the reaction. Two drops of 1 M hydrochloric acid solution was added, and the mixed solution was extracted with ethyl acetate (10 mL × 2 times). The aqueous phase was concentrated under reduced pressure to obtain Compound 459-3 crude product (120 mg).

MS (ESI) M/Z: 312.2 [M+H]⁺.

Step C: Compound 459-3 (120 mg, 0.39 mmol) was dissolved in *N,N*-dimethylformamide (1.9 mL) under nitrogen protection at room temperature. Subsequently, (5-bromo-2-fluoro-3-nitrophenyl)((2S,6R)-2,6-dimethylmorpholinyl)methanone (212 mg, 0.59 mmol) and *N*,*N*-diisopropylethylamine (252 mg, 1.95 mmol) were successively added to the above solution. Then the reaction system was stirred at 80°C for 5 hours.

After the depletion of the raw material as monitored by LCMS, water (40 mL) was added to the reaction solution to quench the reaction. The mixed solution was extracted with ethyl acetate (10 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 459-4 (155 mg, yield 62%).

MS (ESI) M/Z: 652.0 [M+H]⁺.

Step D: Compound 459-4 (155 mg, 0.24 mmol) was dissolved in dichloromethane (1.2 mL) under nitrogen protection at room temperature. Subsequently, the above solution was cooled to 0°C, and 4 M hydrochloric acid-1,4-dioxane solution (1.0 mL) was slowly added dropwise. Then the reaction system was stirred at room temperature for 1 hour.

After the depletion of the raw material as monitored by LCMS, the reaction solution was distilled and concentrated under reduced pressure. Compound 459-5 crude product (135 mg) was obtained.

MS (ESI) M/Z: 552.0 [M+H]⁺.

Step E: Compound 459-5 (100 mg, 0.18 mmol) and 5-methylaminonicotinic acid (33 mg, 0.22 mmol) were dissolved in *N*,*N*-dimethylformamide (0.9 mL) at room temperature. Subsequently, the above solution was cooled to 0°C, and 2-(7-azabenzotriazolyl)-*N*,*N*,*N'*,*N'*-tetramethyluronium hexafluorophosphate (68 mg, 0.18 mmol) and *N*,*N*-diisopropylethylamine (116 mg, 0.90 mmol) were successively added. Then the reaction system was continued to be stirred for 10 minutes.

After the depletion of the raw material as monitored by LCMS, water (30 mL) was added to the reaction solution to quench the reaction. The mixed solution was extracted with ethyl acetate (10 mL × 3 times), and the organic phases were combined. The combined organic phase was washed with saturated saline (20 mL × 2 times), then dried with anhydrous sodium sulfate, filtered and concentrated under reduced pressure. The resulting residue was purified by preparative high performance liquid chromatography to obtain the title Compound 459 (30 mg, yield 24%).

MS (ESI) M/Z: 685.4 [M+H]⁺.

¹H NMR (400 MHz, DMSO-*d₆*) δ 8.58 - 8.33 (m, 1H), 8.30 - 8.14 (m. 1H). 8.01 (s, 1H), 7.92 - 7.22 (m, 5H), 6.99 - 6.77 (m, 1H), 6.15 (s, 1H). 4.77 - 3.40 (m, 11H), 3.28 - 3.00 (m, 1H), 2.95 - 2.63 (m, 4H). 2.47 - 2.26 (m. 1H), 1.17 - 0.82 (m, 6H).

### Example 460

### (5-Bromo-2-((3S,4S)-1-(5-(methylamino)nicotinoyl)-4-(oxazol-2-ylmethoxy)pyrrolidin-3-yl)amino)-3-nitrophenyl)((2S,6R)-2,6-dimethylmorpholinyl)methanone

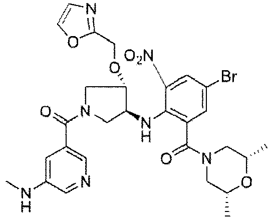

### Reaction route:

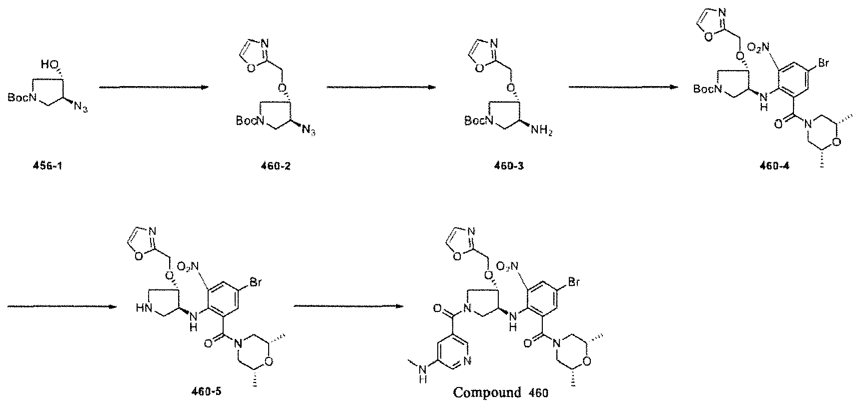

### Operating Steps:

Step A: Compound 456-1 (100 mg, 0.44 mmol) was dissolved in *N,N*-dimethylformamide (2.2 mL) under nitrogen protection at room temperature. Subsequently, the above solution was cooled to 0°C, sodium hydride (26 mg, 0.66 mmol) was added, and the solution was continued to be stirred for 30 minutes. Then 2-chloromethyloxazole (62 mg, 0.53 mmol) was added to the above solution, and the reaction system was stirred at room temperature for 1 hour.

After the depletion of the raw material as monitored by LCMS, water (10 mL) was added to the reaction solution to quench the reaction. The mixed solution was extracted with ethyl acetate (10 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 460-2 (70 mg, yield 51.7%).

MS (ESI) M/Z: 254.0 [M-55].

Step B: Compound 460-2 (70 mg, 0.23 mmol) was dissolved in tetrahydrofuran (1 mL) and water (0.1 mL) at room temperature. Subsequently, triphenylphosphine (89 mg, 0.34 mmol) was added to the above solution. Then the reaction system was continued to be stirred for 48 hours.

After the depletion of the raw material as monitored by LCMS, water (2 mL) was added to the reaction solution to quench the reaction. Two drops of 1 M hydrochloric acid solution was added, and the mixed solution was extracted with ethyl acetate (5mL × 2 times). The aqueous phase was concentrated under reduced pressure to obtain Compound 460-3 (40 mg, yield 62.5%).

MS (ESI) M/Z: 184.2 [M-99].

Step C: Compound 460-3 (40 mg, 0.14 mmol) was dissolved in *N*,*N*-dimethylformamide (0.7 mL) under nitrogen protection at room temperature. Subsequently, (5-bromo-2-fluoro-3-nitrophenyl)((2S,6R)-2,6-dimethylmorpholinyl)methanone (56 mg, 0.16 mmol) and *N*,*N*-diisopropylethylamine (0.07 mL, 0.42 mmol) were successively added to the above solution. Then the reaction system was stirred at 80°C for 5 hours.

After the depletion of the raw material as monitored by LCMS, water (10 mL) was added to the reaction solution to quench the reaction. The mixed solution was extracted with ethyl acetate (10 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 460-4 (60 mg, yield 68.2%).

MS (ESI) M/Z: 524.0 [M-99].

Step D: Compound 460-4 (60 mg, 0.10 mmol) was dissolved in dichloromethane (0.5 mL) under nitrogen protection at room temperature. Subsequently, the above solution was cooled to 0°C, and 4 M hydrochloric acid-1,4-dioxane solution (0.5 mL) was slowly added. Then the reaction system was stirred at room temperature for 1 hour.

After the depletion of the raw material as monitored by LCMS, the reaction solution was distilled and concentrated under reduced pressure. Compound 460-5 crude product (52 mg) was obtained.

MS (ESI) M/Z: 524.0 [M+H]⁺.

Step E: 5-Methylaminonicotinic acid (15 mg, 0.10 mmol) was dissolved in *N,N*-dimethylformamide (0.5 mL) at room temperature. Subsequently, the above solution was cooled to 0°C, and 2-(7-azabenzotriazolyl)-*N*,*N*,*N'*,*N'-*tetramethyluronium hexafluorophosphate (42 mg, 0.11 mmol), *N*,*N*-diisopropylethylamine (0.05 mL, 0.30 mmol) and Compound 460-5 (52 mg, 0.10 mmol) were successively added. Then the reaction system was continued to be stirred for 10 minutes.

After the depletion of the raw material as monitored by LCMS, water (10 mL) was added to the reaction solution to quench the reaction. The mixed solution was extracted with ethyl acetate (15 mL × 3 times), and the organic phases were combined. The combined organic phase was washed with saturated saline (20 mL × 2 times), then dried with anhydrous sodium sulfate, filtered and concentrated under reduced pressure. The resulting residue was purified by preparative high performance liquid chromatography to obtain the title Compound 460 (22.16 mg, yield 33.9%).

MS (ESI) M/Z: 658.0 [M+H]⁺.

¹H NMR (400 MHz, DMSO-*d₆*) δ 8.34 - 8.18 (m, 1H), 8.17 - 7.94 (m. 2H), 7.90 - 7.29 (m, 3H), 7.27 - 7.06 (m. 1H), 6.96 - 6.77 (m, 1H), 6.21 - 6.06 (m. 1H), 4.75 - 4.50 (m, 2H), 4.48 - 3.39 (m, 9H). 3.29 - 3.00 (m, 1H). 2.94 - 2.67 (m, 4H), 2.49 - 2.36 (m, 1H). 1.20 - 0.87 (m. 6H).

### Example 461

### (5-Bromo-2-((3S,4S)-1-(5-(methylamino)nicotinoyl)-4-(thiazot-2-ylmethoxy)pyrrolidin-3-yl)amino)-3-nitrophenyl)((2S,6R)-2,6-dimethylmorpholinyl)methanone

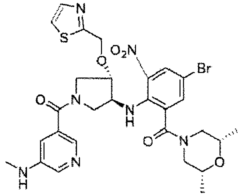

### Reaction route:

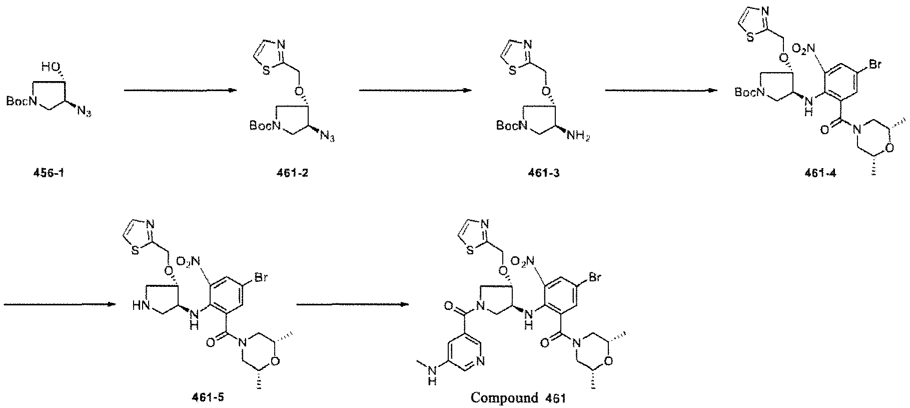

### Operating Steps:

Step A: Compound 456-1 (200 mg, 0.88 mmol) was dissolved in *N,N*-dimethylformamide (4.0 mL) under nitrogen protection at room temperature. Subsequently, the above solution was cooled to 0°C. Sodium hydride (70 mg, 1.76 mmol) was added, and the solution was continued to be stirred for 30 minutes. Then 2-(chloromethyl)thiazole (141 mg, 1.06 mmol) was added to the above solution. Then the reaction system was stirred at room temperature for 1 hour.

After the depletion of the raw material as monitored by LCMS, water (40 mL) was added to the reaction solution to quench the reaction. The mixed solution was extracted with ethyl acetate (10 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 461-2 (95 mg, yield 33%).

MS (ESI) M/Z: 270.0 [M-55].

Step B: Compound 461-2 (95 mg, 0.29 mmol) was dissolved in the solution of tetrahydrofuran (3.1 mL) and water (0.5 mL) under nitrogen protection at room temperature. Subsequently, triphenylphosphine (228 mg, 0.87 mmol) was added to the above solution. Then the reaction system was stirred at room temperature for 24 hours.

After the depletion of the raw material as monitored by LCMS, water (20 mL) was added to the reaction solution to quench the reaction. Two drops of 1 M hydrochloric acid solution was added, and the mixed solution was extracted with ethyl acetate (10 mL × 2 times). The aqueous phase was concentrated under reduced pressure to obtain Compound 461-3 crude product (45 mg).

MS (ESI) M/Z: 300.0 [M+H]⁺.

Step C: Compound 461-3 (40 mg, 0.13 mmol) was dissolved in *N,N*-dimethylformamide (1.9 mL) under nitrogen protection at room temperature. Subsequently, (5-bromo-2-fluoro-3-nitrophenyl)((2*S*,6*R*)-2,6-dimethylmorpholinyl)methanone (58 mg, 0.16 mmol) and *N*,*N*-diisopropylethylamine (50 mg, 0.39 mmol) were successively added to the above solution. Then the reaction system was stirred at 80°C for 5 hours.

After the depletion of the raw material as monitored by LCMS, water (40 mL) was added to the reaction solution to quench the reaction. The mixed solution was extracted with ethyl acetate (10 mL × 3 times), and the organic phases were combined. The combined organic phase was washed with saturated saline (20 mL × 2 times), then dried with anhydrous sodium sulfate, filtered and concentrated under reduced pressure. The resulting residue was purified by silica gel column chromatography to obtain Compound 461-4 (15 mg, yield 18%).

MS (ESI) M/Z: 640.0 [M+H]⁺.

Step D: Compound 461-4 (15 mg, 0.023 mmol) was dissolved in dichloromethane (1.2 mL) under nitrogen protection at room temperature. Subsequently, the above solution was cooled to 0°C, and 4 M hydrochloric acid-1,4-dioxane solution (1.0 mL) was slowly added dropwise. Then the reaction system was stirred at room temperature for 1 hour.

After the depletion of the raw material as monitored by LCMS, the reaction solution was distilled and concentrated under reduced pressure. Compound 461-5 crude product (15 mg) was obtained.

MS (ESI) M/Z: 540.0 [M+H]⁺.

Step E: Compound 461-5 (15 mg, 0.028 mmol) and 5-methylaminonicotinic acid (5 mg, 0.034 mmol) were dissolved in *N*,*N*-dimethylformamide (0.4 mL) at room temperature. Subsequently, the above solution was cooled to 0°C, and 2-(7-azabenzotriazolyl)-*N*,*N*,*N'*,*N'*-tetramethyluronium hexafluorophosphate (21 mg, 0.056 mmol) and *N,N-*diisopropylethylamine (18 mg, 0.14 mmol) were successively added. Then the reaction system was continued to be stirred for 10 minutes.

After the depletion of the raw material as monitored by LCMS, water (30 mL) was added to the reaction solution to quench the reaction. The mixed solution was extracted with ethyl acetate (10 mL × 3 times), and the organic phases were combined. The combined organic phase was washed with saturated saline (20 mL × 2 times), then dried with anhydrous sodium sulfate, filtered and concentrated under reduced pressure. The resulting residue was purified by preparative high performance liquid chromatography to obtain the title Compound 461 (2.7 mg, yield 17%).

MS (ESI) M/Z: 674.0 [M+H]⁺.

¹H NMR (400 MHz, DMSO-*d₆*) δ 8.33 - 8.15 (m, 1H), 8.01 (s, 1H), 7.91 - 7.27 (m, 5H), 6.99 - 6.74 (m, 1H), 6.16 (s, 1H), 5.01 - 4.68 (m, 2H), 4.48 - 3.43 (m. 9H), 3.29 - 3.00 (m. 1H), 2.95 - 2.66 (m, 4H), 2.48 - 1.94 (m, 1H), 1.16 - 0.88 (m, 6H).

### Example 462

### (5-Bromo-2-(((3S,4S)-4-((1-methyl-1H-imidazol-5-yl)methoxy)-1-(5-(methylamino)nicotinoyl) pyrrolidin-3-yl)amino)-3-nitrophenyl)((2S,6R)-2,6-dimethylmorpholinyl)methanone

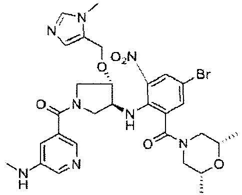

### Reaction route:

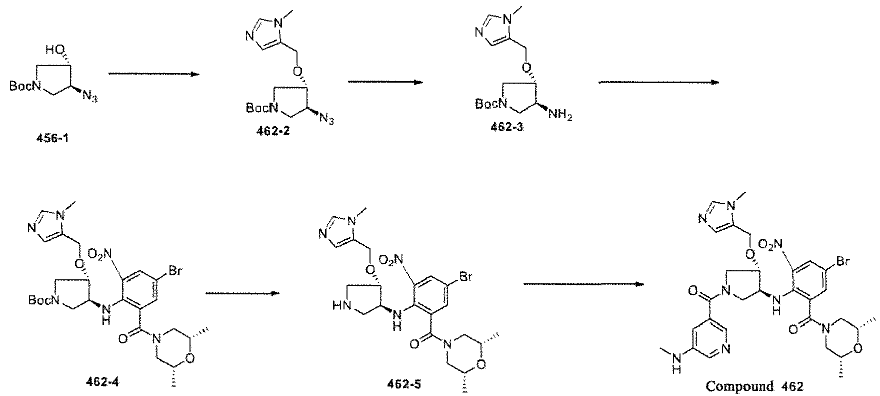

### Operating Steps:

Step A: Compound 456-1 (100 mg, 0.44 mmol) was dissolved in *N,N*-dimethylformamide (2 mL) at room temperature. Subsequently, sodium hydride (26.4 mg, 0.66 mmol) was added to the above solution, and the solution was stirred for 30 minutes. Then 5-(chloromethyl)-1-methyl-1H-imidazole (68.4 mg, 0.58 mmol) was added to the above solution, then the reaction solution was warmed to room temperature, and continued to be stirred for 1 hour.

After the depletion of the raw material as monitored by LCMS, water (10 mL) was added to the reaction solution to quench the reaction. The mixed solution was extracted with ethyl acetate (2 mL × 3 times), and the organic phases were combined. The combined organic phase was washed with saturated sodium chloride solution (6 mL × 2 times), then dried with anhydrous sodium sulfate, filtered and concentrated under reduced pressure. The resulting residue was purified by silica gel column chromatography to obtain Compound 462-2 (76 mg, yield 53%).

MS (ESI) M/Z: 323.0[M+H]⁺.

Step B: Compound 1-2 (76 mg, 0.23 mmol) was dissolved in tetrahydrofuran (2.2 mL) and water (0.25 mL) at room temperature. Subsequently, triphenylphosphine (93.9 mg, 0.35 mmol) was added to the above solution. Then the reaction system was continued to be stirred for 10 hours.

After the depletion of the raw material as monitored by LCMS, 1mol/L hydrochloric acid was used to adjust the pH value of the reaction solution to 5, which was then concentrated under reduced pressure to obtain Compound 462-3 (43 mg, yield 63%).

MS (ESI) M/Z: 297.0 [M+H]⁺.

Step C: Compound 462-3 (43mg, 0.14 mmol) and (S-bromo-2-fluoro-3-nitrophenyl)((2S,6R)-2,6-dimethylmorpholinyl)methanone (52 mg, 0.14 mmol) were dissolved in *N*,*N*-dimethylformamide (0.5 mL) under nitrogen protection at room temperature. Subsequently, *N*,*N*-diisopropylethylamine (0.08 mL, 0.42 mmol) was added to the above solution. Then the reaction system was stirred at 80°C for 1 hour.

After the depletion of the raw material as monitored by LCMS, water (15 mL) was added to the reaction solution to quench the reaction. The mixed solution was extracted with ethyl acetate (10 mL × 3 times), and the organic phases were combined. The combined organic phase was washed with saturated sodium chloride solution (20 mL × 2 times), then dried with anhydrous sodium sulfate, filtered and concentrated under reduced pressure. The resulting residue was purified by silica gel column chromatography to obtain Compound 462-4 (15 mg, yield 16.8%).

MS (ESI) M/Z: 636.8 [M+H]⁺.

Step D: Compound 462-4 (15 mg, 0.03 mmol) was dissolved in dichloromethane (0.5 mL) at room temperature. Subsequently, 4 M hydrochloric acid-1,4-dioxane solution (0.5 mL) was added to the above solution. Then the reaction system was continued to be stirred for 2 hours.

After the depletion of the raw material as monitored by LCMS, the reaction solution was concentrated under reduced pressure to obtain Compound 462-5 crude product (15 mg).

MS (ESI) M/Z: 536.8 [M+H]⁺.

Step E: 5-(Methylamino)nicotinic acid (5 mg, 0.03 mmol) was dissolved in *N*,*N*-dimethylformamide (0.5 mL) at room temperature. Subsequently, *N*,*N*-diisopropylethylamine (0.03 mL, 0.14 mmol), 2-(7-azabenzotriazolyl)-*N*,*N*,*N'*,*N'-*tetramethyluronium hexafluorophosphate (14.8 mg, 0.04 mmol) and Compound 462-5 (15 mg, 0.03 mmol) were successively added to the above solution. Then the reaction system was continued to be stirred for 20 minutes.

After the depletion of the raw material as monitored by LCMS, water (6 mL) was added to the reaction solution to quench the reaction. The mixed solution was extracted with ethyl acetate (2 mL × 3 times), and the organic phases were combined. The combined organic phase was washed with saturated sodium chloride solution (6 mL × 2 times), then dried with anhydrous sodium sulfate, filtered and concentrated under reduced pressure. The crude product was purified by preparative high performance liquid chromatography to obtain the title Compound 462 (5.06mg, yield 25%).

MS (ESI) M/Z: 671.0 [M+H]⁺.

¹H NMR (400 MHz, DMSO-*d*₆) δ 8.31 - 8.15 (m, 1H), 8.08 - 7.94 (m, 1H), 7.91 -7.24 (m, 4H), 6.97 - 6.73 (m, 2H), 6.23 - 6.05 (m, 1H), 4.67 - 3.39 (m, 14H), 3.25 - 2.98 (m. 1H), 2.94 - 2.65 (m, 4H), 2.42 - 2.03 (m, 1H), 1.21 - 0.80 (m, 6H).

### Biological Test and Evaluation

### Test Example 1: Evaluation of the inhibitory effect of the compound of the application on SARS-CoV-2 3CL^{pro}/M^{pro} target

In this experiment, the activity of SARS-CoV-2 3CL^{pro}/M^{pro} protease was detected by fluorescence resonance energy transfer, and the half inhibitory concentration IC₅₀ of the compound on SARS-CoV-2 3CL^{pro}/M^{pro} protease was obtained.

### 1. Experimental materials

Novel coronavirus M^{pro}/3CL^{pro} inhibitor screening kit (P0315M), purchased from Beyotime Biotechnology.

### 2. Experimental method

1) The enzyme solution was prepared with reaction buffer, 49.5 µL enzyme solution was added into each well, and 49.5 µL reaction buffer was added into Min-well.
2) The IC₅₀ of the compound was detected, and the final concentration of the test was starting from 10 µM, 3-fold dilution, 10 concentrations, and each concentration was set up for multiple well testing. The compound to be tested was diluted into a solution of 200-fold final concentration, and 250 nL gradient diluted compound to be tested was added into the 384-well reaction plate by D300e (TECAN) ultramicro sampler. 250 nL 100% DMSO was transferred into both Max-well and Min-well.
3) The reaction plate was incubated on ice for 10 minutes.
4) 250 nL substrate solution was added into each well by D300e (TECAN).
5) The reaction plate was centrifuged at 1000 rpm for 1 min, and the fluorescence signal was read continuously for 30 minutes by Envision microplate reader (PerkinElmer).
6) GraphPad Prism 8 software was used to analyze data and calculate the IC₅₀ of the compound.

### 3. Experimental results

The inhibitory effect of the compound of the application on 3CLpro protease is shown in Table 1. The activity data are divided into four intervals A, B, C and D, the compounds with IC₅₀ ≤ 10 nM are marked with A, the compounds with 10 nM < IC₅₀ ≤ 50 nM are marked with B, the compounds with 50 nM < IC₅₀ C 100 nM are marked with C, and the compounds with 100 nM < IC₅₀ < 200nM are marked with D.

**Table 1 Inhibition on SARS-CoV-2 3CL^{pro}/M^{pro} protease**

| **Example** | IC₅₀ (nM) | **Example** | IC₅₀ (nM) | **Example** | IC₅₀ (nM) |
|---|---|---|---|---|---|
| 1 | B | 2 | A | 3 | A |
| 4 | C | 5 | A | 6 | A |
| 7 | B | 8 | A | 9 | A |
| 10 | B | 11 | C | 12 | B |
| 13 | A | 14 | B | 15 | B |
| 16 | B | 17 | B | 18 | D |
| 19 | B | 20 | B | 21 | B |
| 22 | B | 23 | B | 24 | D |
| 2.5 | B | 26 | B | 27 | B |
| 28 | B | 29 | B | 30 | B |
| 31 | B | 32 | B | 33 | B |
| 34 | B | 35 | B | 36 | B |
| 37 | B | 38 | A | 39 | A |
| 40 | B | 41 | B | 42 | B |
| 43 | B | 44 | A | 45 | B |
| 46 | B | 47 | B | 48 | B |
| 49 | B | 50 | C | 51 | B |
| 52 | B | 53 | C | 54 | B |
| 55 | B | 56 | B | 57 | B |
| 59 | B | 60 | C | 61 | C |
| 62 | B | 63 | B | 64 | B |
| 65 | B | 66 | B | 67 | B |
| 68 | C | 69 | C | 70 | B |
| 71 | B | 72 | A | 73 | B |
| 74 | C | 75 | A | 76 | A |
| 77 | B | 78 | B | 79 | C |
| 80 | B | 81 | B | 82 | B |
| 83 | B | 84 | B | 85 | B |
| 86 | B | 87 | B | 88 | B |
| 89 | B | 90 | B | 91 | B |
| 92 | B | 93 | B | 94 | B |
| 95 | B | 96 | C | 97 | B |
| 98 | B | 99 | D | 100 | B |
| 101 | C | 102 | B | 103 | B |
| 104 | B | 105 | C | 106 | B |
| 107 | B | 108 | B | 109 | B |
| 110 | B | 111 | A | 112 | B |
| 113 | A | 114 | B | 115 | B |
| 116 | A | 117 | B | 118 | B |
| 119 | B | 120 | A | 121 | A |
| 122 | B | 123 | A | 124 | B |
| 125 | A | 126 | B | 127 | B |
| 128 | B | 129 | B | 130 | B |
| 131 | B | 132 | B | 133 | B |
| 134 | B | 135 | B | 136 | A |
| 137 | B | 138 | B | 139 | B |
| 140 | B | 141 | B | 142 | B |
| 143 | B | 144 | B | 145 | B |
| 146 | B | 147 | B | 148 | C |
| 149 | B | 150 | C | 151 | B |
| 152 | B | 153 | B | 154 | C |
| 155 | C | 156 | B | 157 | C |
| 158 | B | 159 | A | 160 | B |
| 161 | B | 162 | B | 163 | B |
| 164 | B | 165 | B | 166 | B |
| 167 | B | 168 | B | 169 | B |
| 170 | B | 171 | B | 172 | B |
| 173 | B | 174 | B | 175 | B |
| 176 | B | 177 | B | 178 | C |
| 179 | C | 180 | C | 181 | B |
| 182 | B | 183 | B | 184 | A |
| 185 | B | 186 | B | 187 | B |
| 188 | B | 189 | B | 190 | B |
| 191 | B | 192 | B | 193 | B |
| 194 | B | 195 | B | 196 | B |
| 197 | B | 198 | B | 199 | C |
| 200 | B | 201 | B | 202 | B |
| 203 | B | 204 | B | 205 | A |
| 206 | C | 207 | B | 208 | B |
| 209 | B | 210 | B | 211 | B |
| 212 | B | 213 | B | 214 | B |
| 215 | B | 216 | B | 217 | C |
| 218 | B | 219 | B | 220 | C |
| 221 | B | 222 | B | 223 | B |
| 224 | B | 225 | A | 226 | B |
| 227 | B | 228 | B | 229 | B |
| 230 | A | 231 | B | 232 | B |
| 233 | C | 234 | B | 235 | B |
| 236 | B | 237 | B | 238 | B |
| 239 | B | 240 | B | 241 | B |
| 242 | B | 243 | B | 244 | B |
| 245 | C | 246 | B | 247 | B |
| 248 | B | 249 | B | 250 | B |
| 251 | C | 252 | B | 253 | C |
| 254 | B | 255 | B | 256 | B |
| 257 | B | 258 | B | 259 | B |
| 260 | B | 261 | B | 262 | B |
| 263 | B | 264 | B | 265 | B |
| 266 | B | 267 | B | 268 | A |
| 269 | B | 270 | B | 271 | B |
| 272 | B | 273 | B | 274 | A |
| 275 | B | 276 | B | 277 | B |
| 278 | B | 279 | B | 280 | B |
| 281 | C | 282 | B | 283 | B |
| 284 | B | 285 | B | 286 | B |
| 287 | B | 288 | B | 289 | B |
| 290 | B | 291 | B | 292 | B |
| 293 | B | 294 | B | 295 | B |
| 296 | B | 297 | B | 298 | B |
| 299 | B | 300 | B | 301 | C |
| 302 | D | 303 | B | 304 | C |
| 305 | B | 306 | B | 307 | B |
| 308 | B | 309 | B | 310 | B |
| 311 | B | 312 | B | 313 | B |
| 314 | B | 315 | B | 316 | B |
| 317 | B | 318 | B | 319 | C |
| 320 | B | 321 | B | 322 | B |
| 323 | B | 324 | D | 325 | B |
| 326 | B | 327 | B | 328 | B |
| 329 | B | 330 | B | 331 | B |
| 332 | B | 333 | B | 334 | B |
| 335 | B | 336 | B | 337 | B |
| 338 | B | 339 | B | 340 | B |
| 341 | B | 342 | B | 343 | B |
| 344 | B | 345 | B | 346 | B |
| 347 | B | 348 | B | 349 | B |
| 350 | C | 351 | B | 352 | B |
| 353 | B | 354 | B | 355 | B |
| 356 | B | 357 | B | 358 | B |
| 359 | B | 360 | B | 361 | B |
| 362 | C | 364 | C | 365 | C |
| 366 | B | 367 | B | 368 | B |
| 369 | C | 370 | C | 371 | B |
| 372 | B | 373 | C | 374 | B |
| 375 | B | 376 | C | 377 | C |
| 378 | B | 379 | B | 380 | B |
| 381 | C | 382 | C | 383 | B |
| 385 | B | 386 | C | 387 | B |
| 388 | C | 389 | C | 390 | B |
| 391 | C | 392 | C | 393 | C |
| 394 | C | 395 | C | 396 | C |
| 397 | C | 398 | B | 399 | C |
| 400 | D | 401 | C | 402 | C |
| 403 | C | 404 | D | 405 | B |
| 406 | C | 407 | C | 408 | B |
| 409 | C | 410 | D | 411 | C |
| 412 | C | 413 | D | 414 | D |
| 415 | C | 416 | D | 417 | C |
| 418 | C | 419 | C | 420 | C |
| 421 | D | 422 | C | 423 | B |
| 424 | C | 425 | D | 426 | D |
| 427 | D | 428 | C | 429 | D |
| 430 | D | 431 | C | 432 | D |
| 433 | D | 434 | D | 435 | C |
| 436 | C | 437 | C | 438 | B |
| 439 | B | 440 | C | 441 | B |
| 442 | B | 443 | B | 444 | B |
| 445 | C | 446 | B | 447 | C |
| 448 | B | 449 | C | 450 | B |
| 451 | B | 452 | C | 453-P1 | C |
| 454 | B | 455 | B | 456 | B |
| 457 | D | 458 | B | 459 | B |
| 460 | B | 461 | B | 462 | B |

Conclusion: It can be seen from Table 1 that the compound of the present application have good inhibitory effect on SARS-CoV-2 3CL^{pro}/M^{pro} protease.

### Test Example 2: Pharmacokinetics in vivo experiment in CD1 mice

Male CD1 mice were used as test animals to study the pharmacokinetics *in vivo* behavior of the compound of the present application after oral administration to male CD1 mice at a dose of 10 mg/kg.

### 1. Experimental protocol

### 1.1 Test article

Control compound and some compounds of the present application.

### 1.2 Test animals

### Animal bBasic information of animals

Species: male CD1 mice (3 mice / group)
Grade: SPF grade
Weight: about 20 to 30 g
Age: 6 to 8 weeks
Source: Vital River

### 1.3 Dose:

The test solution was administered to male CD1 mice by gavage, with a dose of 10 mg/kg and a volume of 10 mL/kg.

Preparation of test solution: The test product was dissolved in solvent (10% DMSO+50% PEG400+40% purified water), and DMSO, PEG400 and purified water were successively added during preparation.

Control group: Compound 2 in patent CN113072497A, with the structural formula as follows:

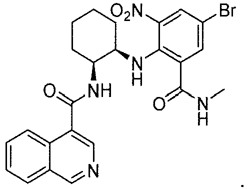

Experimental group: some compounds of the present application.

### 1.5 Sample collection:

Before administration (0 h) and 0.25 h, 0.5 h, 1 h, 2 h, 4 h, 8 h, 24 h after administration, respectively.

According to the set time point of blood collection, about 0.025 to 0.03 mL of whole blood (anticoagulated with EDTA-K2) was collected from the dorsal venous of foot of mice and placed on wet ice. Within 30 min after collection, the blood was centrifuged at 4000 g at 4°C for 5 min, and plasma was separated and transferred into a detection tube.

### 2. Experimental results and analysis

WinNonlin (Phoenix^{™}, version 6.1) was used to calculate pharmacokinetic parameters and the pharmacokinetic parameters are shown in Table 2. The results show that compared with the control group, the compounds of the present application have higher exposure and bioavailability in mice, and have good pharmacokinetic properties.

**Table 2-1 Pharmacokinetic parameters of intravenous administration in CD1 mice.**

| **Compounds** | Dose (mg/kg) | CL (mL/min/kg) | Vss (L/kg) | T_{1/2} (hr) | AUC (hr*ng/m L) |
|---|---|---|---|---|---|
| CN113072497A **Compound** 2 | 1 | 8.8 | 1.73 | 0.185 | 114 |
| **Example** 31 | 1 | 11.6 | 1.56 | NA | 88 |
| **Example** 34 | 1 | 9.4 | 1.02 | 0.114 | 107 |
| **Example** 35 | 1 | 9.12 | 1.28 | 0.135 | 112 |
| **Example** 36 | 1 | 11.2 | 1.70 | 0.136 | 92 |
| **Example** 295 | 1 | 7.4 | 1.06 | 0.127 | 136 |
| **Example** 312 | 1 | 10.6 | 1.38 | 0.128 | 95 |
| **Example** 320 | 1 | 6.3 | 2.1 | 0.36 | 156 |
| **Example** 321 | 1 | 6.2 | 7.1 | 1.6 | 153 |
| **Example** 422 | 1 | 9.82 | 1.32 | 0.106 | 102 |

**Table 2-2 Pharmacokinetic parameters of oral administration in CD1 mice.**

| **Compounds** | Dose (mg/kg) | T_{1/2} (hr) | Cₘₐₓ (ng/mL) | AUC (hr*ng/mL) | F (%) |
|---|---|---|---|---|---|
| CN113072497A **Compound** 2 | 10 | NA | 18.0 | 7.9 | 0.74 |
| **Example** 31 | 10 | 0.25 | 401 | 369 | 42.1 |
| **Example** 34 | 10 | 0.5 | 1021 | 702 | 63 |
| **Example** 3 5 | 10 | NA | 1018 | 668 | 60 |
| **Example** 36 | 10 | NA | 365 | 273 | 29.7 |
| **Example** 295 | 10 | 0.193 | 697 | 494 | 36.3 |
| **Example** 312 | 10 | 0.769 | 594 | 605 | 65.5 |
| **Example** 320 | 10 | 0.437 | 825 | 280 | 27.7 |
| **Example** 321 | 10 | 2.4 | 1640 | 1354 | 84.7 |
| **Example** 422 | 10 | 0.32 | 725 | 628 | 62.6 |

| | | | | | |
|---|---|---|---|---|---|
| Note: NA means no corresponding data. | | | | | |

### Test Example 3: Pharmacokinetics in vivo experiment in cynomolgus macaques

Male cynomolgus macaques were used as test animals to study the pharmacokinetics *in vivo* behavior of the compound of the present application in the plasma of cynomolgus macaques after oral administration at 10 mg/kg.

### 1. Experimental protocol

### 1.1 Test article

Some compounds of the present application.

### 1.2 Test animals

### Basic information of animals

Species: cynomolgus macaque
Grade: conventional grade
Quantity: 18 cynomolgus macaques (male) were used in the drug administration experiment.
Weight: about 2.5 to 4.5 kg
Age: 3 to 5 years old
Source: Huazhen Animal Farm, Conghua City (General Partnership)

### 1.3 Administration:

There are 9 male cynomolgus macaques, 3 cynomolgus macaques/group, 3 groups in total. The animals were fasted for 12 hours before administration, and the test solution was administered by gavage (10 mg/kg), and the administration volume was 2.5 mL/kg.

Preparation of test solution: The test article was dissolved in 10% DMSO+50% PEG400+40% purified water, and DMSO, PEG400 and purified water were successively added during preparation.

### 1.4 Sample collection:

Before administration (0 min) and 0.25 h, 0.5 h, 1 h, 2 h, 4 h, 6 h, 8 h, 12h, 24 h and 48 h after administration (11 points), respectively.

According to the set time point of blood collection, about 1.5 mL of blood was collected from vein of posterior limb of cynomolgus macaque. The blood was placed on wet ice, and centrifuged at 1700 g at 4°C for 10 min within 1.5 h after collection. After centrifugation, the plasma was taken and added into a detection tube. Plasma samples can be stored in a refrigerator below -20°C within 2 hours after centrifugation.

### 2. Experimental results and analysis

WinNonlin 8.1.0.3530 was used to calculate pharmacokinetic parameters. The results show that the compounds of the present application have good pharmacokinetic properties.

**Table 3 Pharmacokinetic parameters of oral administration in cynomolgus macaque.**

| **Compounds** | Dose (mg/kg) | Tₘₐₓ (hr) | T_{1/2} (hr) | Cₘₐₓ (ng/mL) | AUC (hr*ng/mL) |
|---|---|---|---|---|---|
| **Example** 31 | 10 | 1.33 | 0.49 | 232 | 454 |
| **Example** 34 | 10 | 1.33 | 0.584 | 1960 | 4090 |
| **Example** 321 | 10 | 1.17 | NA | 699 | 994 |

| | | | | | |
|---|---|---|---|---|---|
| Note: NA means no corresponding data. | | | | | |

## Claims

1. A compound of formula (I), or an isomer or a pharmaceutically acceptable salt thereof, 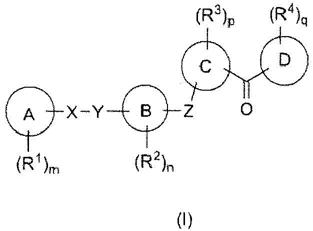
wherein, ring A is selected from the group consisting of C₆₋₁₀ aryl, 5- to 10-membered heteroaryl, C₃₋₁₂ cycloalkyl, and 3- to 12-membered heterocyclyl;
X is selected from the group consisting of -(CH₂)ₛ-C(=O)- and -NH-C(=O)-;
Y is NH or a chemical bond;
ring B is selected from the group consisting of C₃₋₁₂ cycloalkyl, 3- to 12-membered heterocyclyl, and 5- to 10-membered heteroaryl;
Z is selected from the group consisting of -O-, -S-, -CH₂-, -C(=O)-NH- and -(CH₂)ₜNH-;
ring C is selected from the group consisting of C₆₋₁₀ aryl and 5- to 10-membered heteroaryl;
ring D is selected from the group consisting of 3- to 12-membered heterocyclyl and 5- to 6-membered heteroaryl; or ring D is absent;
R¹ is selected from the group consisting of halogen, OH, O, cyano, -NR^{1a}R^{1b}, C₁₋₄ alkyl, C₁₋₄ alkoxy, C₃₋₈ cycloalkyl, C₆₋₁₀ aryl, -NR^{1a}COR^{1c}, -SR^{1d}, and -CONR^{1a}R^{1b}, wherein C₁₋₄ alkyl and C₁₋₄ alkoxy can be further substituted by one or more halogens;
R^{1a}, R^{1b}, R^{1c}, and R^{1d} are each independently selected from the group consisting of H, C₁₋₄ alkyl and halo-C₁₋₄ alkyl, wherein C₁₋₄ alkyl can be substituted by one or more deuteriums;
or, two R¹ linked to the same C atom on ring A can together form =CH₂, =S, or C₃₋₅ cycloalkyl;
R² is selected from the group consisting of deuterium, cyano, C₂₋₄ alkynyl, halogen, C₁₋₄ alkyl, hydroxyl, C₁₋₄ alkoxy, deutero-C₁₋₄ alkoxy, -C(=O)-NR^{2a}R^{2b}, -C₂₋₄ alkynyl-R^{2c}, 5- to 6-membered heteroaryl and -O-(CH₂)_{y}-R^{2d};
R^{2c} is selected from the group consisting of 5- to 6-membered heteroaryl, wherein 5- to 6-membered heteroaryl can be further substituted by C₁₋₄ alkyl;
R^{2a} and R^{2b} are each independently selected from the group consisting of H, C₁₋₄ alkyl, phenyl, 5- to 9-membered heteroaryl, 5- to 6-membered heterocyclyl, and C₃₋₅ cycloalkyl, wherein C₁₋₄ alkyl, phenyl, 5- to 9-membered heteroaryl, 5-to 6-membered heterocyclyl, and C₃₋₅ cycloalkyl can be further substituted by one or more substituents selected from the group consisting of methyl, ethyl, cyclopropyl, halogen, CF₃, -CH₂CF₃, phenyl, methoxy, pyridinyl, pyrimidinyl, tetrahydropyranyl, and -CH₂CH₂OCH₃, wherein pyridinyl, pyrimidinyl, and tetrahydropyranyl can be further substituted by one or more methyl, CF₃, halogen, -NHCH₃, or -CHF₂;
or, R^{2a}, R^{2b} and their linked N atom together form 4- to 9-membered heterocyclyl or 5- to 9-membered heteroaryl, wherein 4- to 9-membered heterocyclyl and 5- to 9-membered heteroaryl can be further substituted by one or more substituents selected from the group consisting of C₁₋₄ alkyl, halogen, hydroxyl, and -CH₂CH₂OCH₃;
R^{2d} is selected from the group consisting of C₁₋₄ alkyl, C₁₋₄ alkoxy, phenyl, 5- to 6-membered heteroaryl and 5- to 6-membered heterocyclyl, wherein phenyl, 5- to 6-membered heteroaryl and 5- to 6-membered heterocyclyl can be further substituted by one or more substituents selected from the group consisting of halogen, C₁₋₄ alkyl, C₁₋₄ alkoxy, and halo-C₁₋₄ alkyl; y is 0, 1, or 2;
R³ is selected from the group consisting of nitro, halogen, hydroxyl, cyano, C₁₋₄ alkyl, halo-C₁₋₄ alkyl, -NR^{3a}R^{3b} and -N(R^{3a}R^{3b}R^{3c})⁺;
R^{3a}, R^{3b} and R^{3c} are each independently selected from the group consisting of H and C₁₋₄ alkyl;
R⁴ is selected from the group consisting of halogen, hydroxyl, C₁₋₄ alkyl, halo-C₁₋₄ alkyl, 3- to 8-membered heterocyclyl, -C(=O)R^{4a}, -NHC(=O)R^{4a}, -(CH₂)ᵣOR^{4b}, -NR^{4c}R^{4d}, C₁₋₄ alkoxy, -(CH₂)ₓ-C₃₋₆ cycloalkyl, -NR^{4a}-(CH₂)ₓ-C₆₋₁₀ aryl, wherein aryl can be further substituted by halogen;
R^{4a}, R^{4b}, R^{4c}, and R^{4d} are each independently selected from the group consisting of H, C₁₋₄ alkyl, halo-C₁₋₄ alkyl, 5- to 6-membered heterocyclyl and C₃₋₆ cycloalkyl;
or R^{4c}, R^{4d} and their linked N atom together form 6-membered heterocyclyl;
x is 0, 1, or 2; r is 0, 1, 2, or 3;
m, n, p, and q are each independently 0, 1, 2, 3, 4, or 5; and
s and t are each independently 0 or 1.

2. The compound of claim 1, or the isomer or the pharmaceutically acceptable salt thereof, 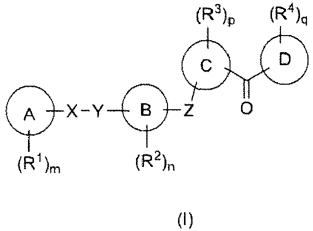
wherein, ring D is 3- to 12-membered heterocyclyl; or ring D is absent;
R¹ is selected from the group consisting of halogen, OH, O, cyano, -NR^{1a}R^{1b}, C₁₋₄ alkyl, C₁₋₄ alkoxy, C₃₋₈ cycloalkyl, C₆₋₁₀ aryl, -NR^{1a}COR^{1c}, -SR^{1d}, and -CONR^{1a}R^{1b}, wherein C₁₋₄ alkyl and C₁₋₄ alkoxy can be further substituted by one or more halogens;
R^{1a}, R^{1b}, R^{1c}, and R^{1d} are each independently selected from the group consisting of H, C₁₋₄ alkyl and halo-C₁₋₄ alkyl,
or, two R¹ linked to the same C atom on ring A can together form =CH₂, =S, or C₃₋₅ cycloalkyl;
R² is selected from the group consisting of halogen, C₁₋₄ alkyl, hydroxyl, and C₁₋₄ alkoxy; and
ring A, ring B, ring C, X, Y, Z, R³, R⁴, m, n, p, and q are each as defined in the compound of formula (I).

3. The compound of claim 1, or the isomer or the pharmaceutically acceptable salt thereof, wherein:
ring D is 3- to 8-membered heterocyclyl; or ring D is absent;
R¹ is selected from the group consisting of halogen, OH, O, cyano, -NR^{1a}R^{1b}, C₁₋₄ alkyl, C₁₋₄ alkoxy, C₃₋₈ cycloalkyl, C₆₋₁₀ aryl, wherein C₁₋₄ alkyl and C₁₋₄ alkoxy can be further substituted by one or more halogens;
R^{1a} and R^{1b} are each independently selected from the group consisting of H and C₁₋₄ alkyl;
R² is selected from the group consisting of halogen and C₁₋₄ alkyl;
R⁴ is selected from the group consisting of halogen, hydroxyl, C₁₋₄ alkyl, halo-C₁₋₄ alkyl, 3- to 8-membered heterocyclyl, -C(=O)R^{4a}, -NHC(=O)R^{4a}, -(CH₂)ᵣOR^{4b}, and -NR^{4c}R^{4d};
R^{4a}, R^{4b}, R^{4c}, and R^{4d} are each independently selected from the group consisting of H, C₁₋₄ alkyl and halo-C₁₋₄ alkyl;
r is 0, 1, 2, or 3;
m, n, p, and q are each independently 0, 1, 2, or 3; and ring A, ring B, ring C, X, Y, Z, and R³ are each as defined in the compound of formula (I).

4. The compound of claim 1 or 2, or the isomer or the pharmaceutically acceptable salt thereof, wherein:
ring A is C₆₋₁₀ aryl, 5- to 10-membered heteroaryl, or 7- to 10-membered heterocyclyl;
ring D is 3- to 8-membered heterocyclyl;
R¹ is selected from the group consisting of halogen, OH, O, cyano, -NR^{1a}R^{1b}, C₁₋₄ alkyl, C₁₋₄ alkoxy, C₃₋₈ cycloalkyl, C₆₋₁₀ aryl, wherein C₁₋₄ alkyl and C₁₋₄ alkoxy can be further substituted by one or more halogens;
R^{1a} and R^{1b} are each independently selected from the group consisting of H and C₁₋₄ alkyl;
R² is selected from the group consisting of halogen and C₁₋₄ alkyl;
R⁴ is selected from the group consisting of halogen, hydroxyl, and C₁₋₄ alkyl;
m, n, p, and q are each independently 0, 1, or 2; and
ring B, ring C, X, Y, Z, and R³ are each as defined in the compound of formula (1).

5. The compound of any one of claims 1 to 4, or the isomer or the pharmaceutically acceptable salt thereof, wherein ring A is selected from the group consisting of pyridinyl, pyrimidinyl, pyrazinyl, isoquinolinyl, cinnolinyl, phthalazinyl, phenyl, imidazolyl, thienyl, isoxazolyl, benzotriazolyl,

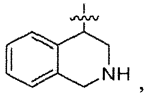

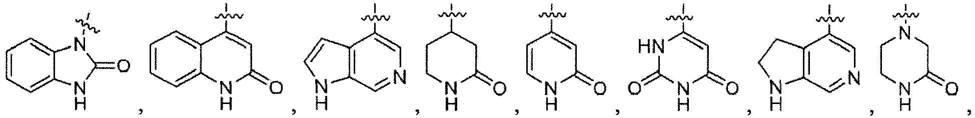

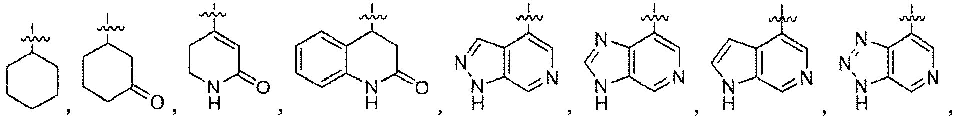

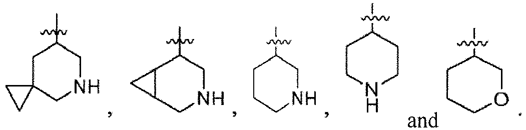

6. The compound of any one of claims 1 to 5, or the isomer or the pharmaceutically acceptable salt thereof, wherein R¹ is selected from the group consisting of F, Cl, Br, I, O, hydroxyl, methyl, methoxy, trifluoromethyl, -NH₂, -NHCH₃, -N(CH₃)₂, 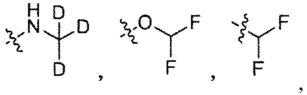 cyano, cyclopropyl, phenyl, -NHCH₂CH₃, -SCH₃, -CONH₂, -NHCOCF₃ and -NHCOCH₃;
or, two R¹ linked to the same C atom on ring A can together form =CH₂, =S, or cyclopropyl; and
m is 0, 1, 2, or 3.

7. The compound of claim 5 or 6, or the isomer or the pharmaceutically acceptable salt thereof, wherein the structural unit 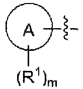 is selected from the group consisting of

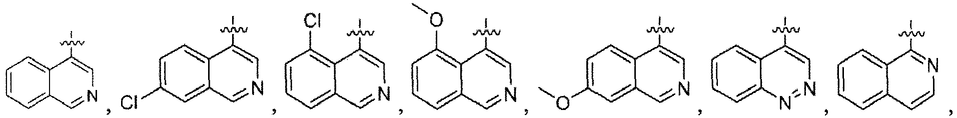

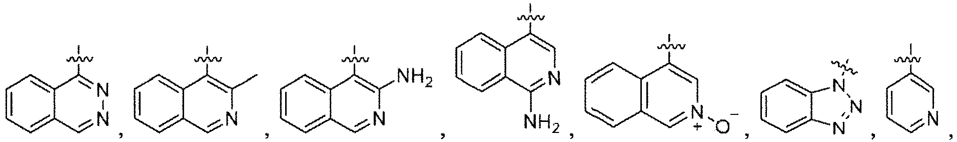

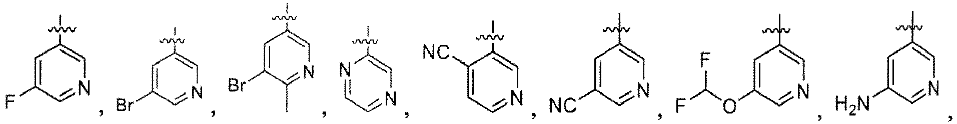

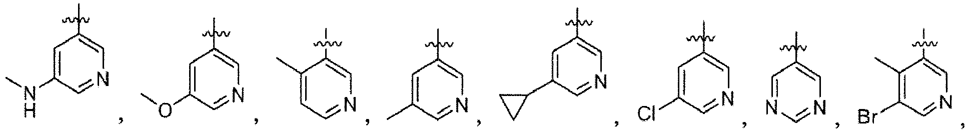

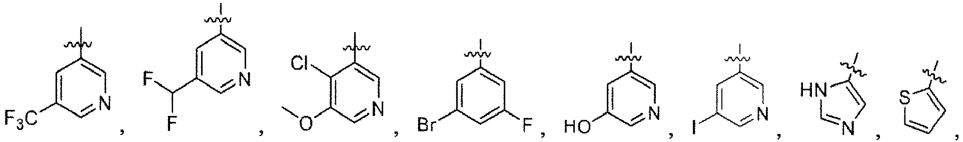

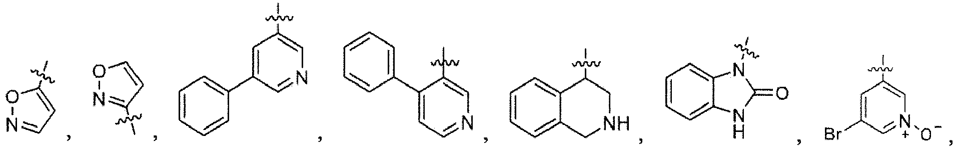

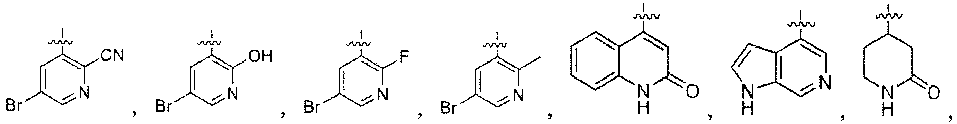

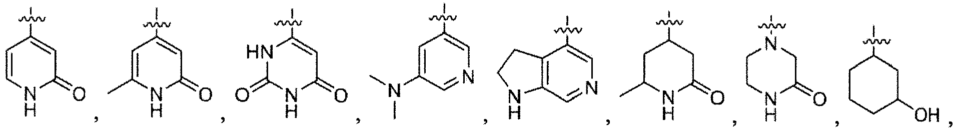

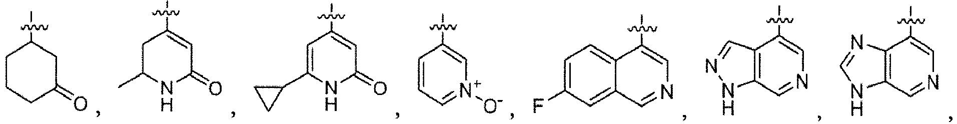

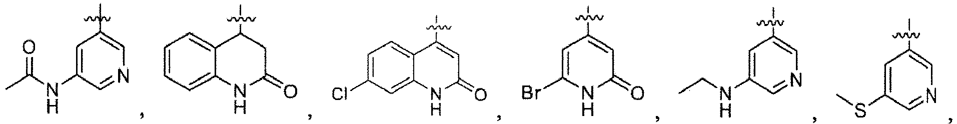

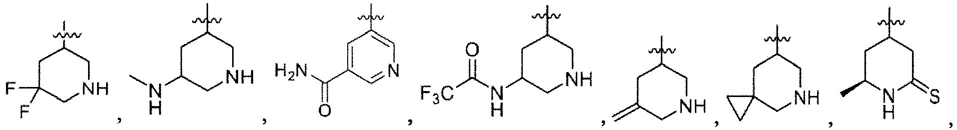

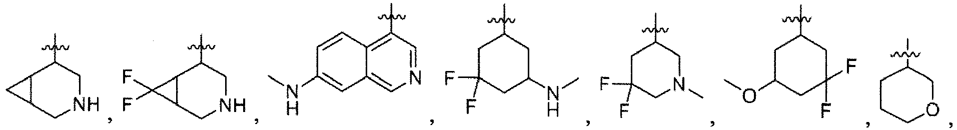

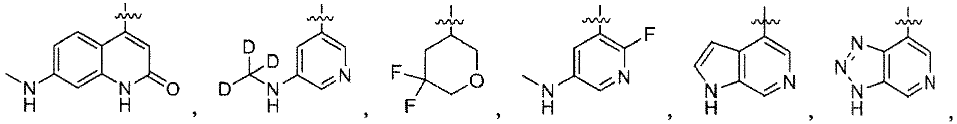

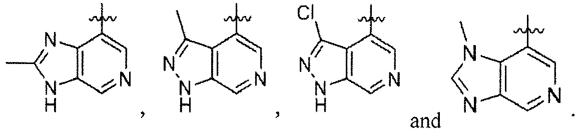

8. The compound of any one of claims 1 to 7, or the isomer or the pharmaceutically acceptable salt thereof, wherein X is selected from the group consisting of -CH₂-C(=O)-, -C(=O)-, and -NH-C(=O)-.

9. The compound of any one of claims 1 to 8, or the isomer or the pharmaceutically acceptable salt thereof, wherein ring B is selected from the group consisting of C₅₋₆ cycloalkyl, 4- to 10-membered heterocyclyl, and 5- to 6-membered heteroaryl;
preferably, ring B is selected from the group consisting of

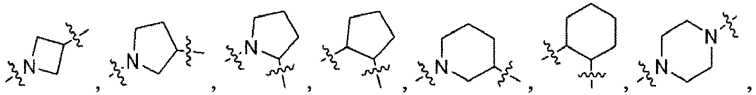

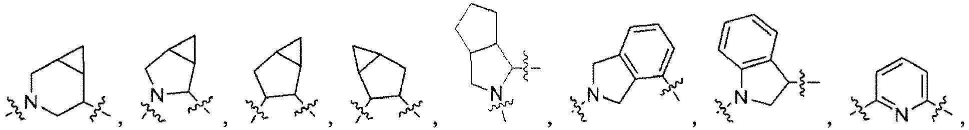

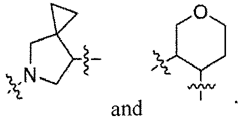

10. The compound of any one of claims 1 to 9, or the isomer or the pharmaceutically acceptable salt thereof, wherein R² is selected from the group consisting of deuterium, cyano, ethynyl, F, methyl, hydroxyl, methoxy, -CONH₂, - CONHCH₃, 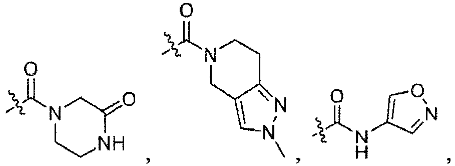 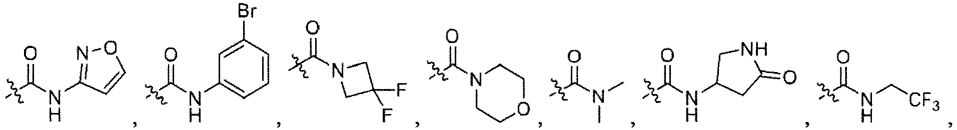 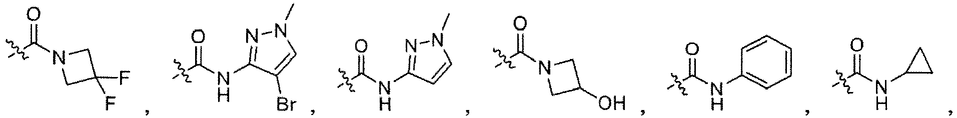 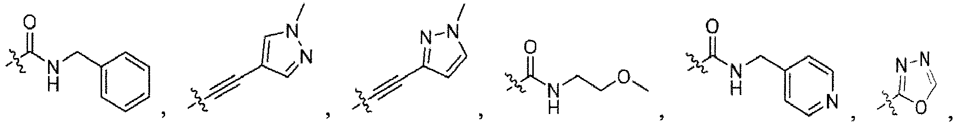 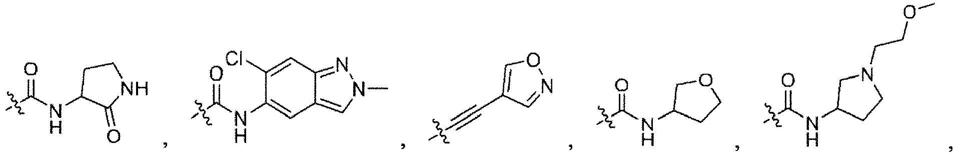 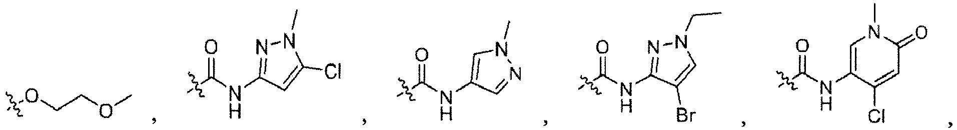 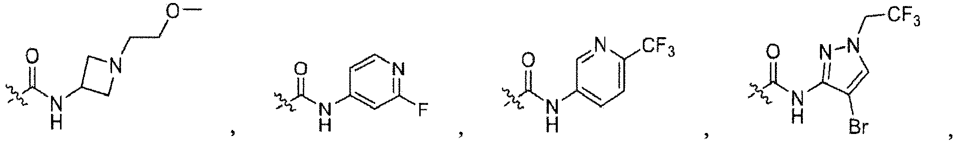 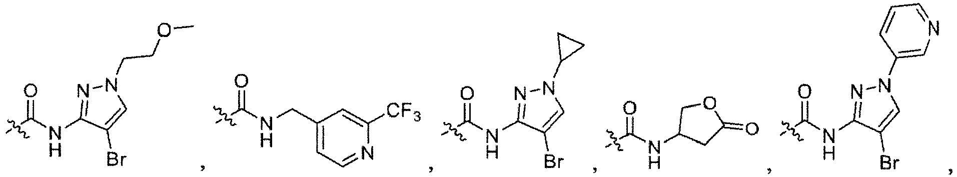 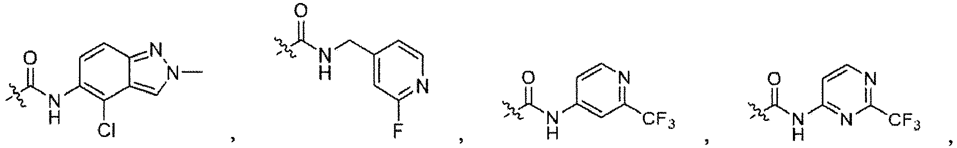 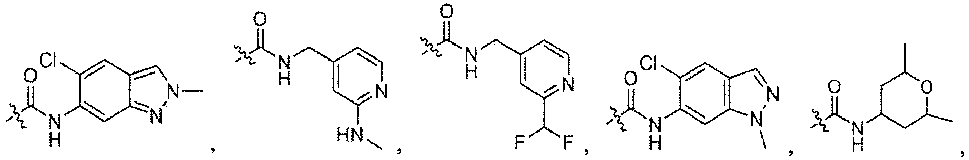 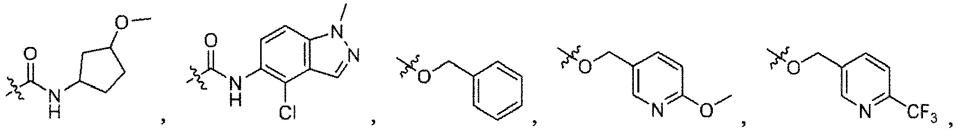 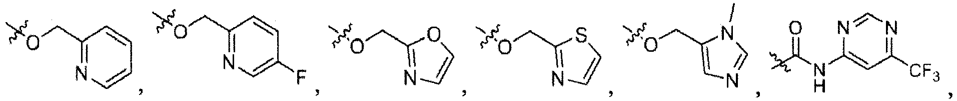 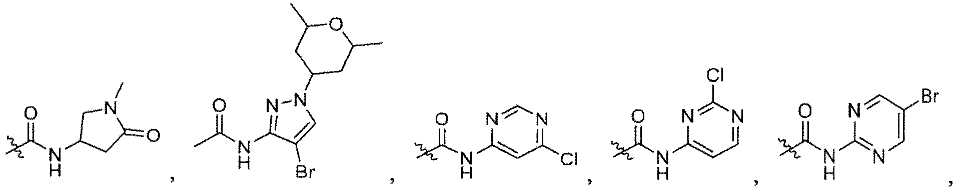 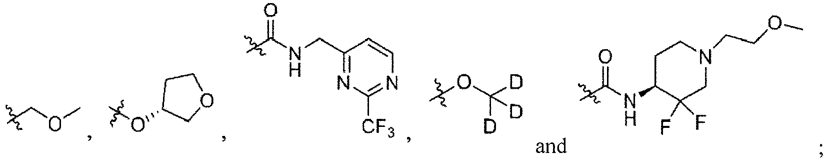 and
n is 0, 1, 2, 3, 4, or 5.

11. The compound of claim 9 or 10, or the isomer or the pharmaceutically acceptable salt thereof, wherein the structural unit 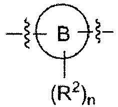 is selected from the group consisting of 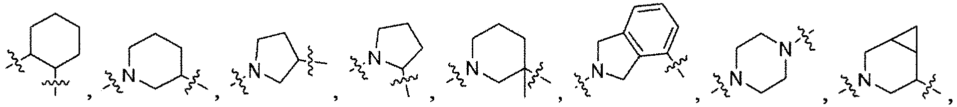 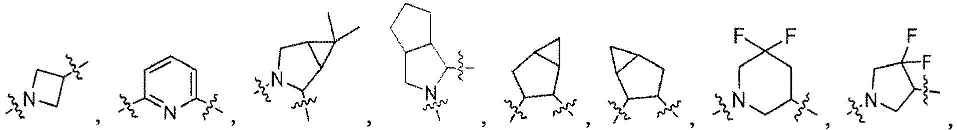 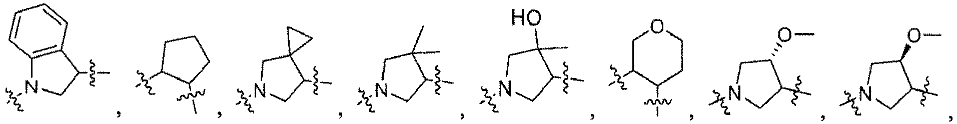 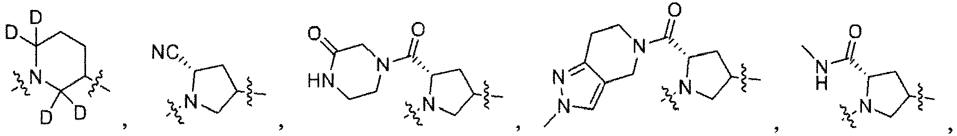 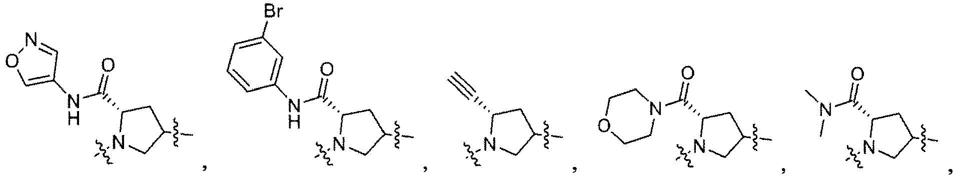 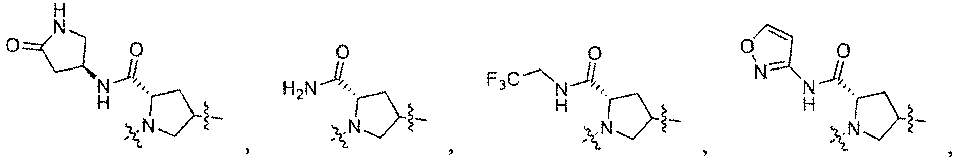 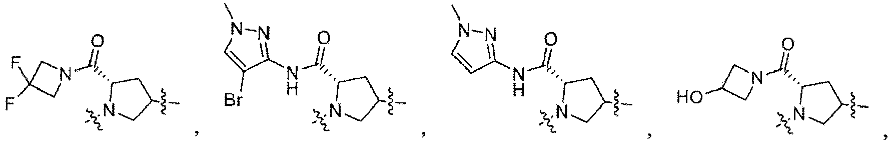 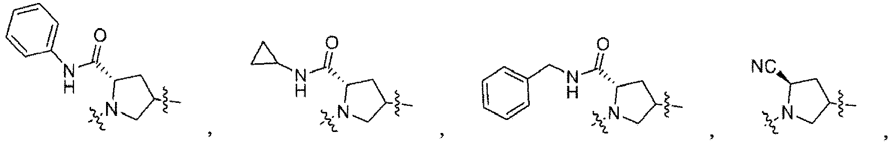 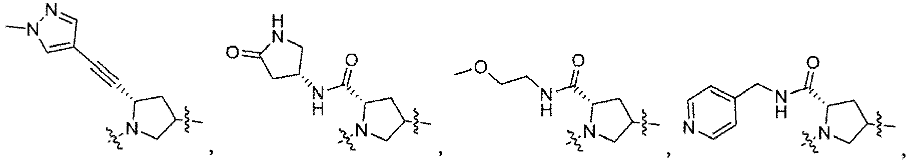 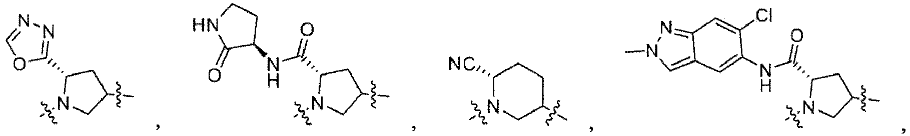 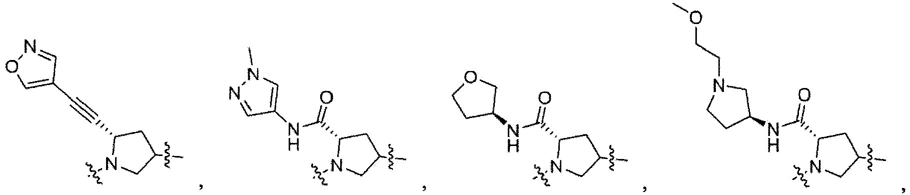 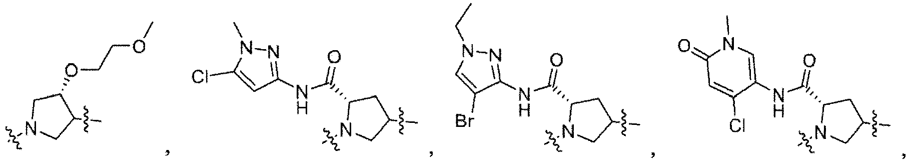 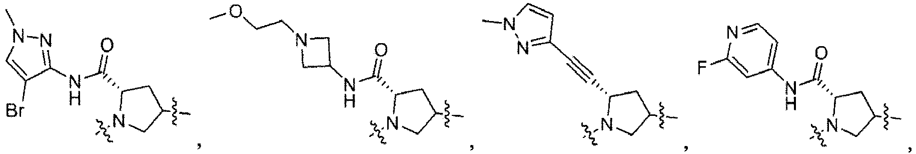 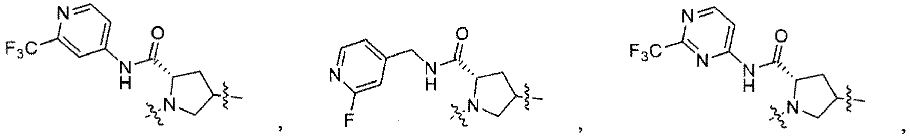 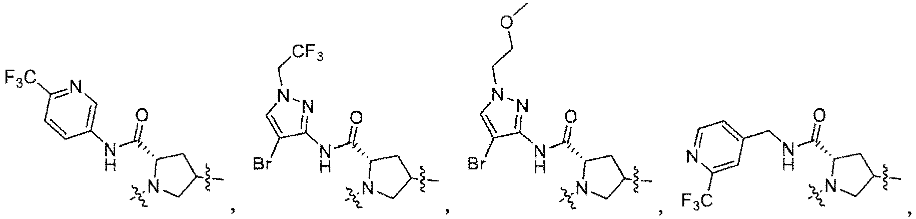 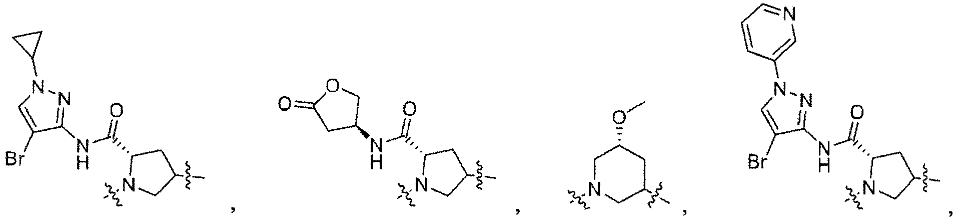 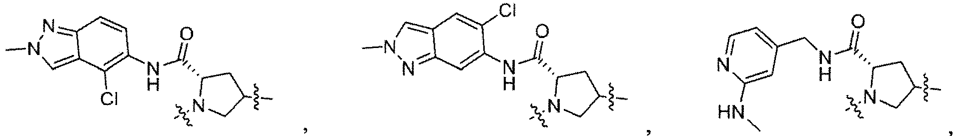 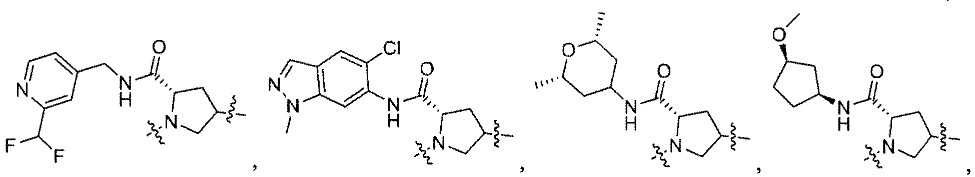 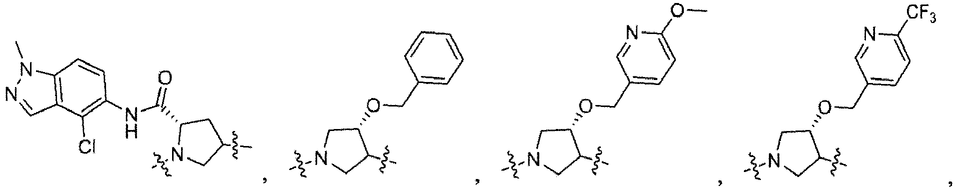 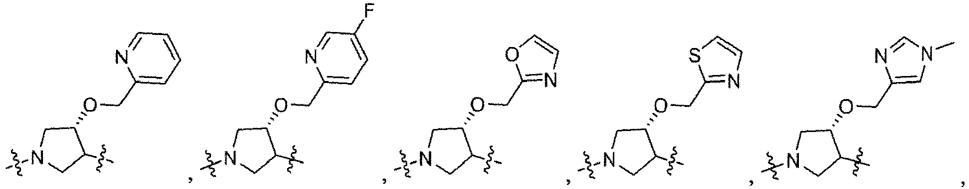 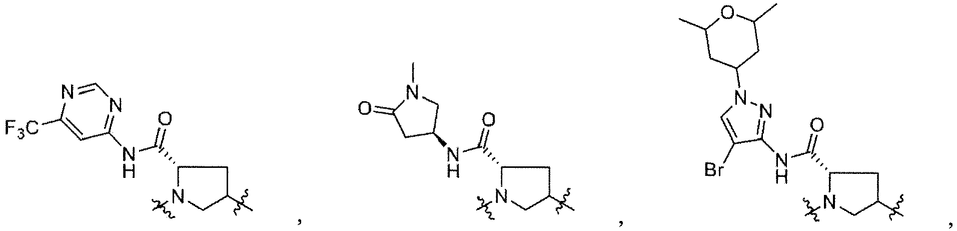 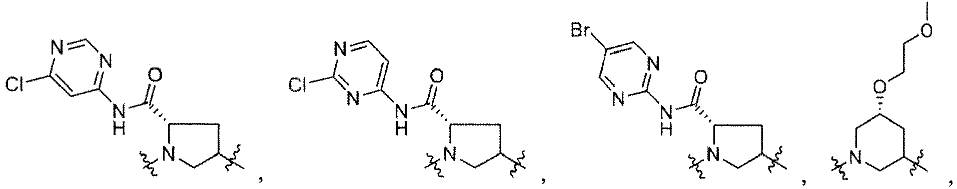 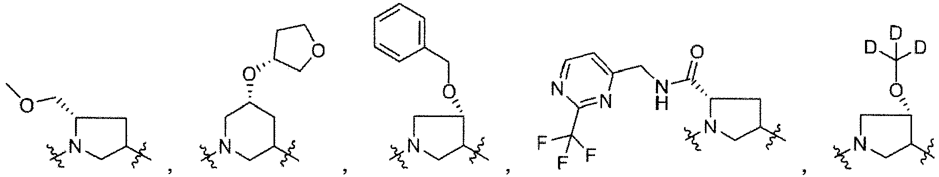 and

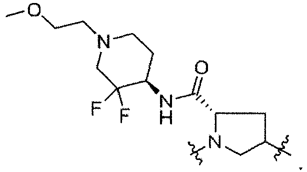

12. The compound of any one of claims 1 to 11, or the isomer or the pharmaceutically acceptable salt thereof, wherein Z is selected from the group consisting of -NH-, -O-, -S-, -CH₂-, -CH₂NH-, and -C(=O)-NH-.

13. The compound of any one of claims 1 to 12, or the isomer or the pharmaceutically acceptable salt thereof, wherein ring C is selected from the group consisting of phenyl, and pyridinyl.

14. The compound of any one of claims 1 to 13, or the isomer or the pharmaceutically acceptable salt thereof, wherein R³ is selected from the group consisting of nitro, Br, Cl, 1, CN, CF₃, and -N(CH₃)₃⁺.

15. The compound of claim 13 or 14, or the isomer or the pharmaceutically acceptable salt thereof, wherein the structural unit 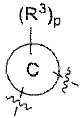 is selected from the group consisting of

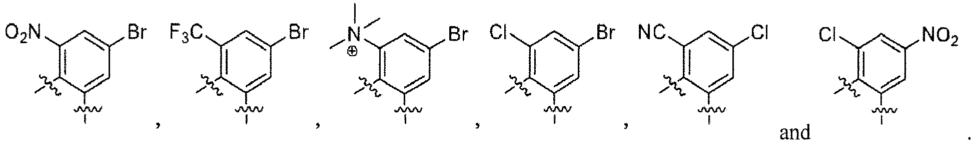

16. The compound of any one of claims 1 to 15, or the isomer or the pharmaceutically acceptable salt thereof, wherein ring D is selected from the group consisting of 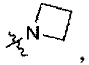 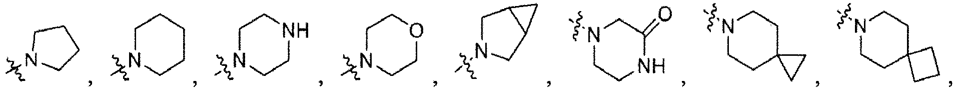 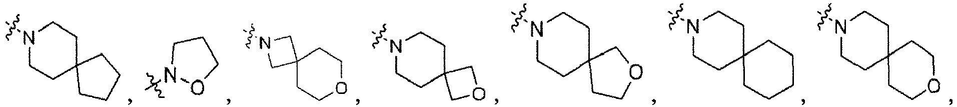 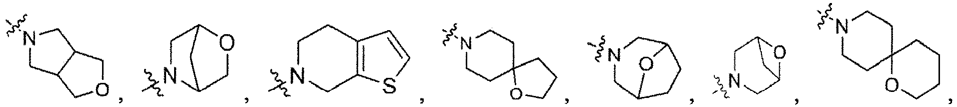 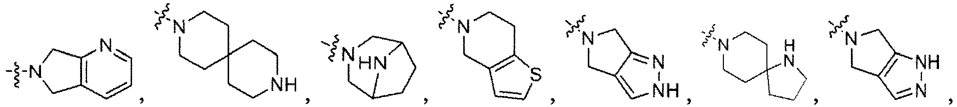 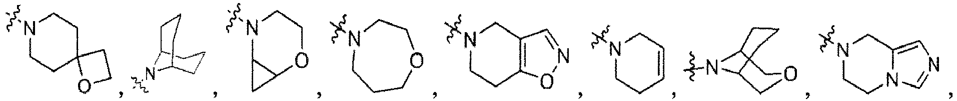 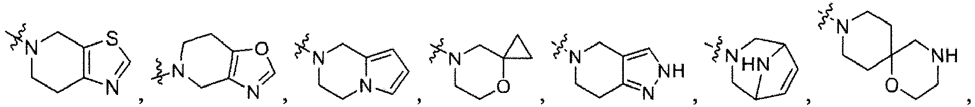 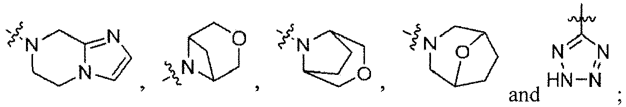 or ring D is absent.

17. The compound of any one of claims 1 to 16, or the isomer or the pharmaceutically acceptable salt thereof, wherein R⁴ is selected from the group consisting of deuterium, F, Cl, methyl, tert-butyl, methoxy, amino, hydroxyl, 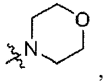 -CH₂CF₃, -CH₂F, -CH₂Cl, vinyl, ethynyl, -CF₃, -CHF₂, 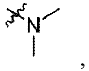 -C(=O)CF₃, -NHC(=O)CF₃, -CH₂CH₂OCH₃, -CH₂CH₂OH, 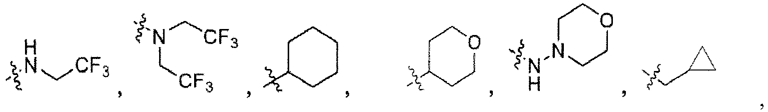 cyclopropyl, 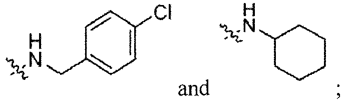 and q is 0, 1, 2, 3, 4, or 5.

18. The compound of claim 16 or 17, or the isomer or the pharmaceutically acceptable salt thereof, wherein the structural unit 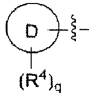 is selected from the group consisting of 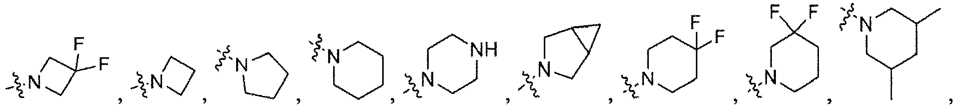 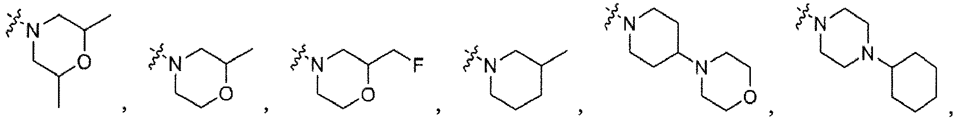 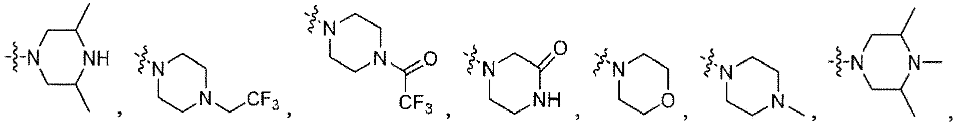 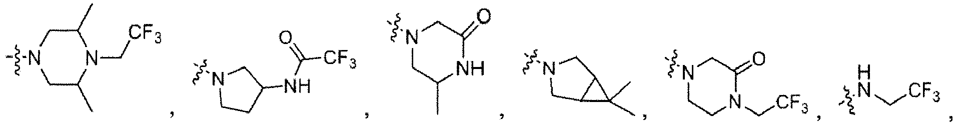 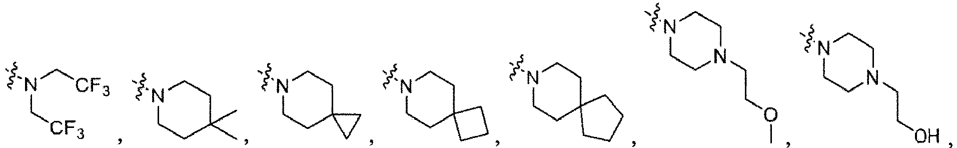 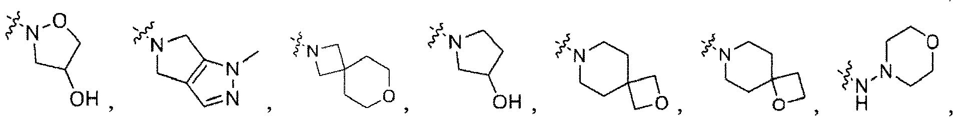 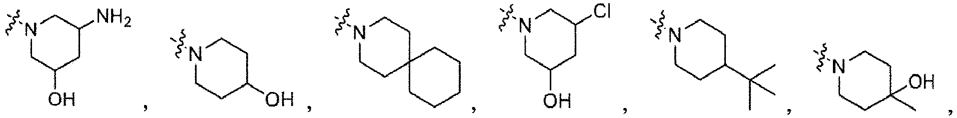 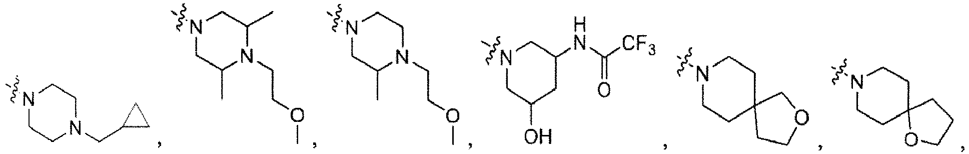 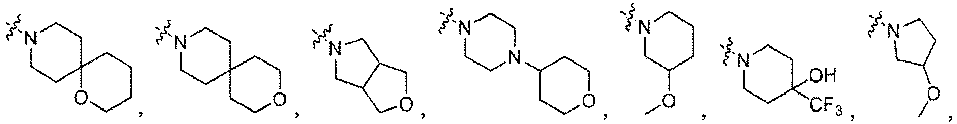 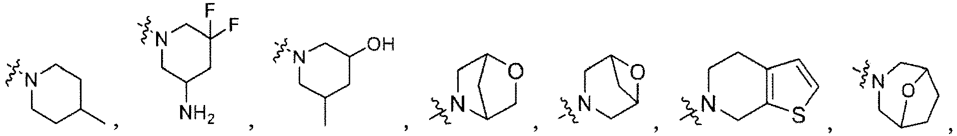 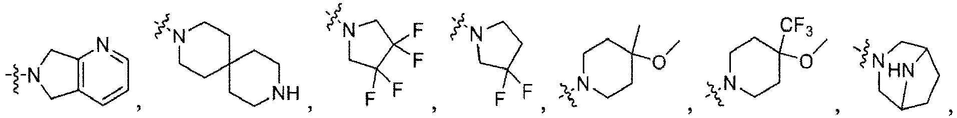 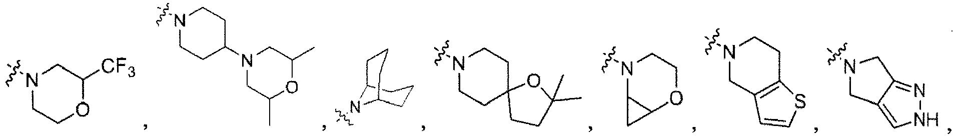 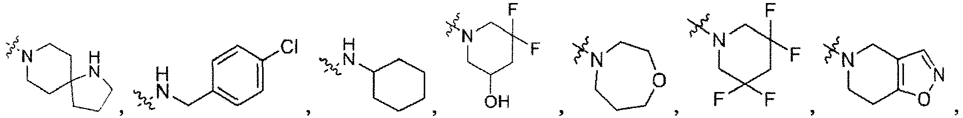 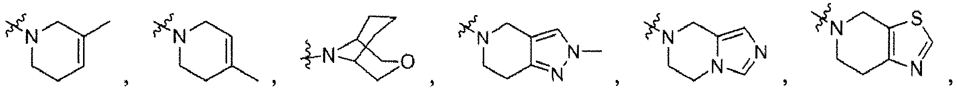 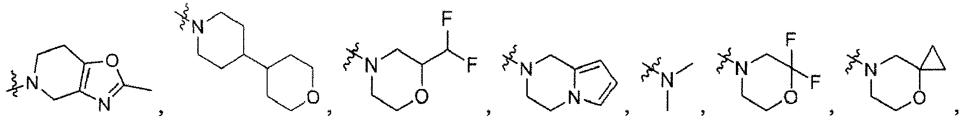 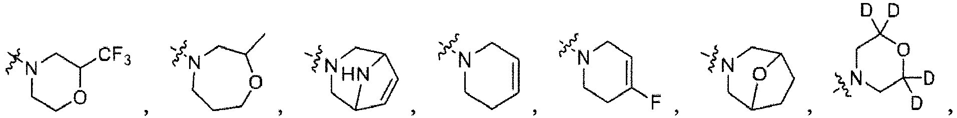 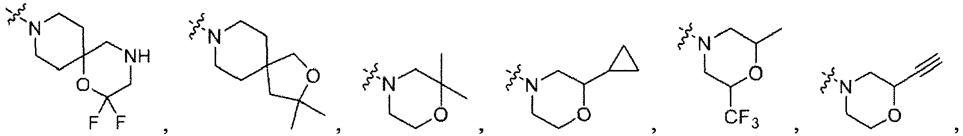 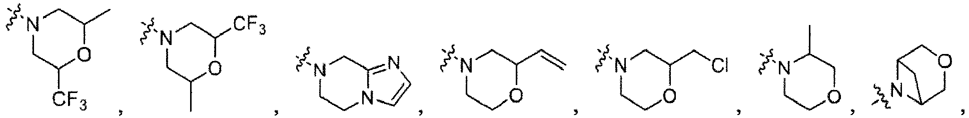 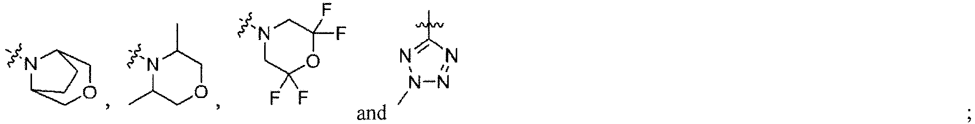 preferably, the structural unit 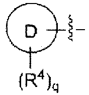 is selected from the group consisting of

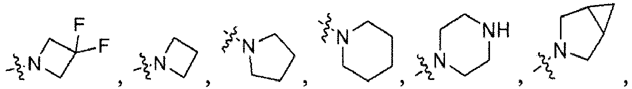

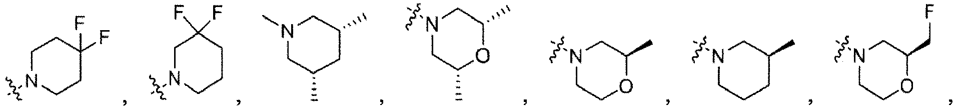

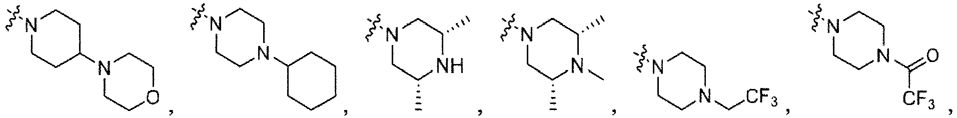

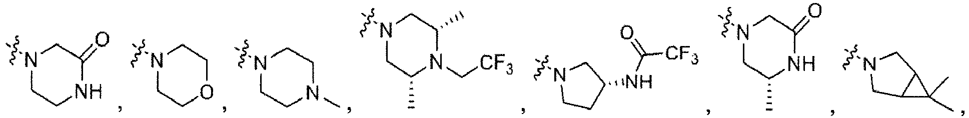

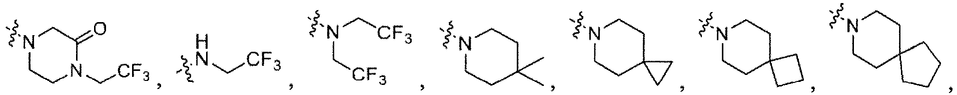

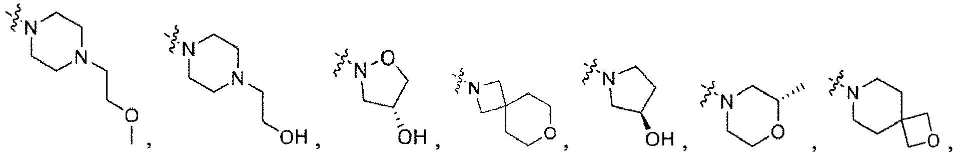

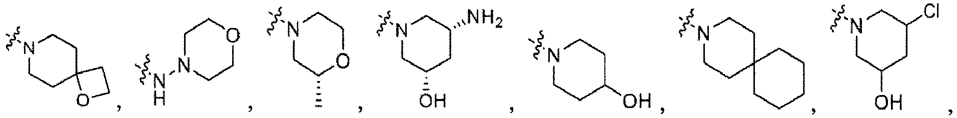

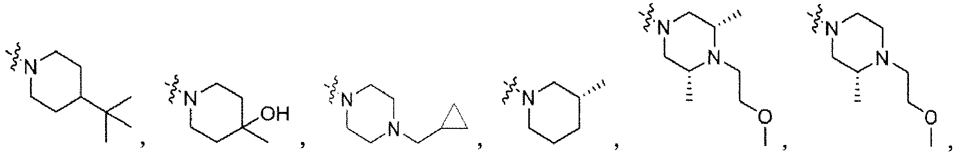

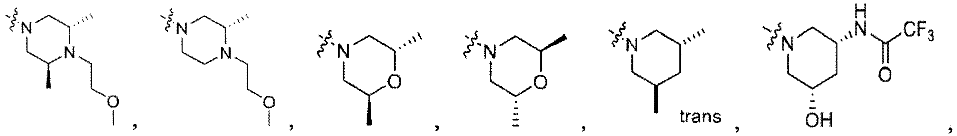

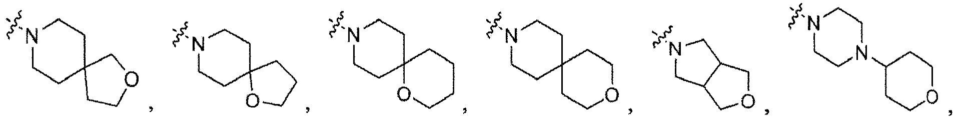

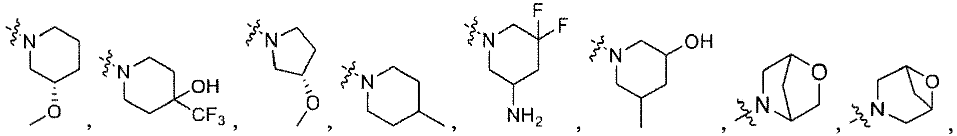

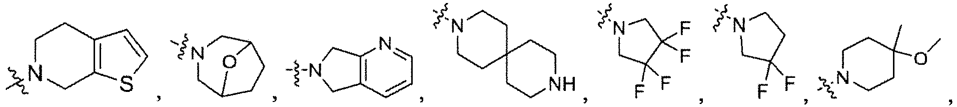

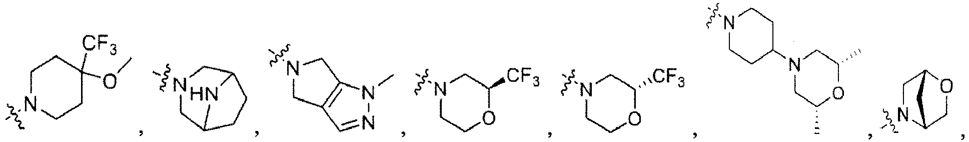

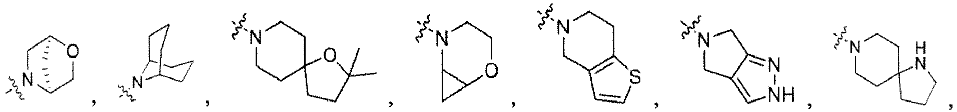

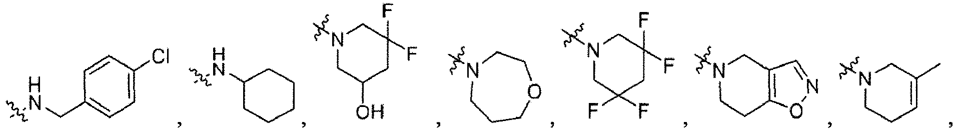

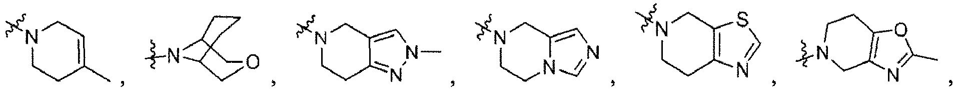

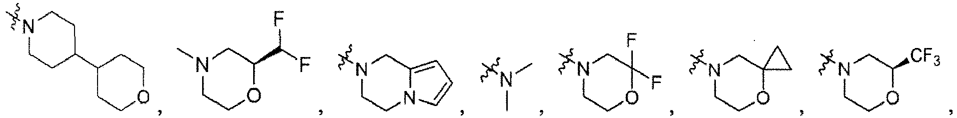

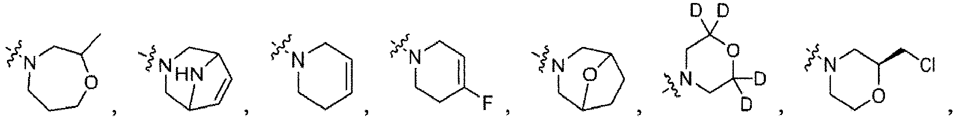

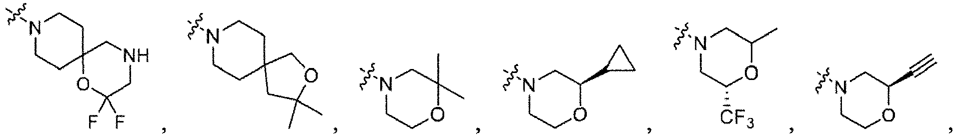

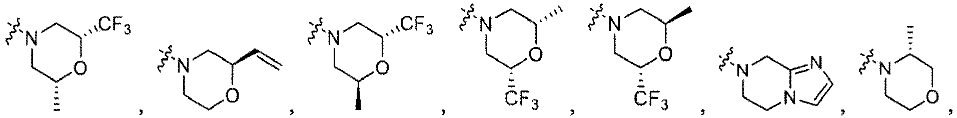

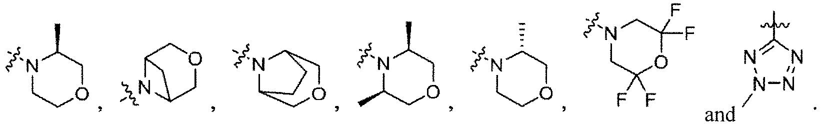

19. The compound of any one of claims 1 to 18, or the isomer or the pharmaceutically acceptable salt thereof, wherein the compound is selected from the group consisting of: 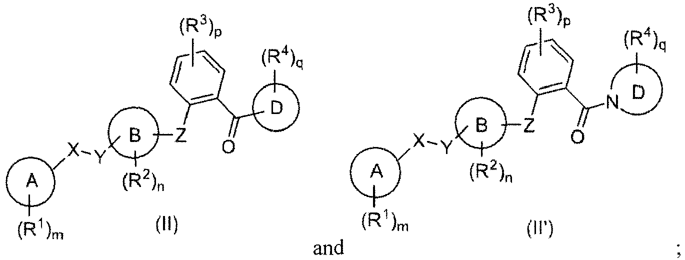 preferably, the compound is selected from the group consisting of: 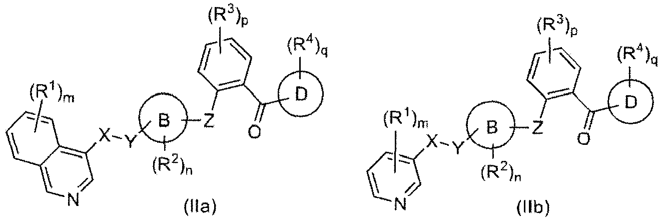 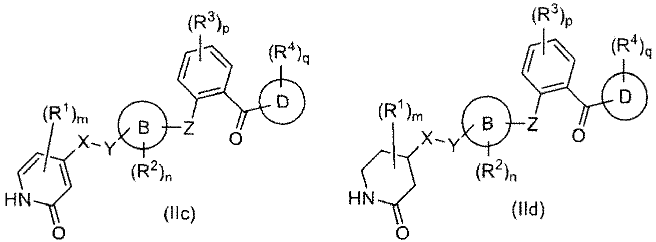 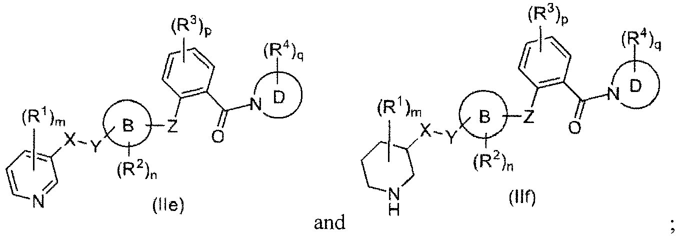 preferably, the compound is selected from the group consisting of: 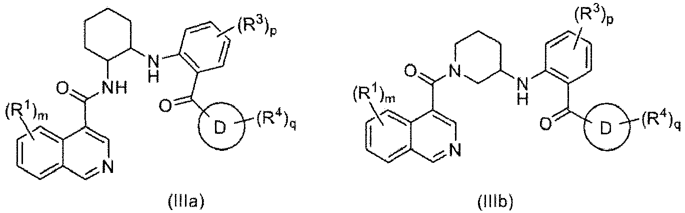 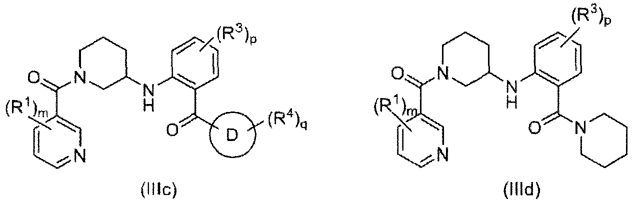 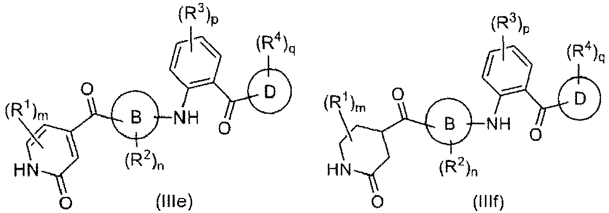 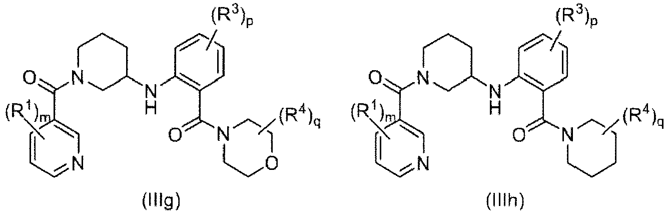 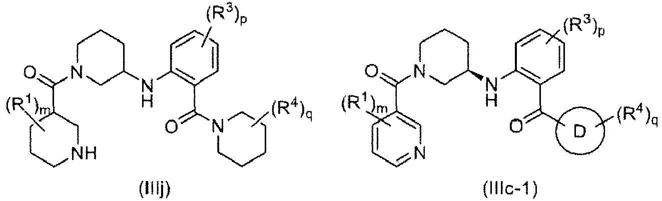 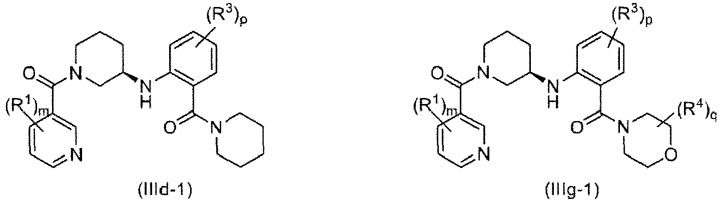 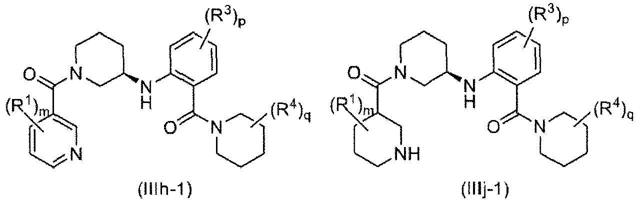 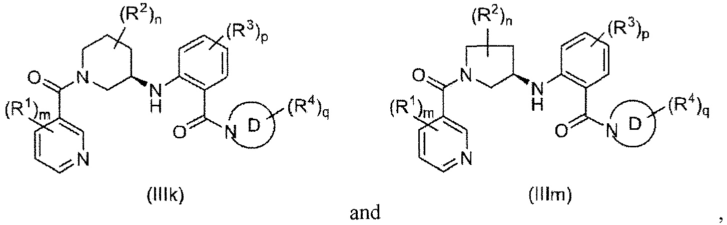 wherein, ring A, ring B, ring D, X, Y, Z, R¹, R², R³, R⁴, m, n, p, and q are each as defined in claims 1 to 18.

20. A compound, or an isomer or a pharmaceutically acceptable salt thereof, wherein the compound is selected from the group consisting of:

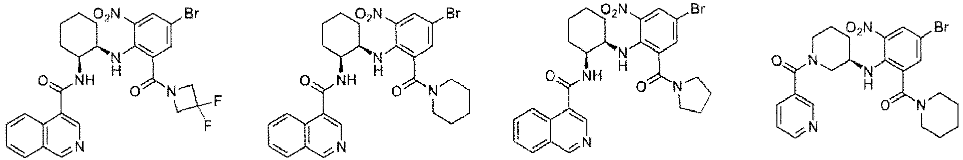

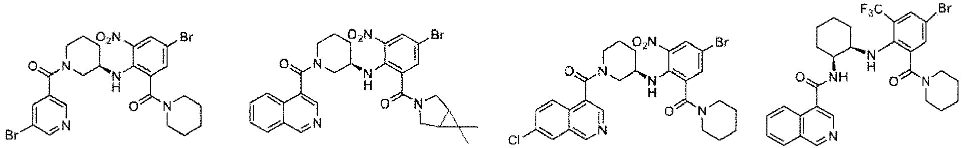

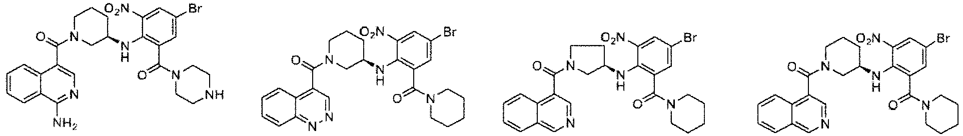

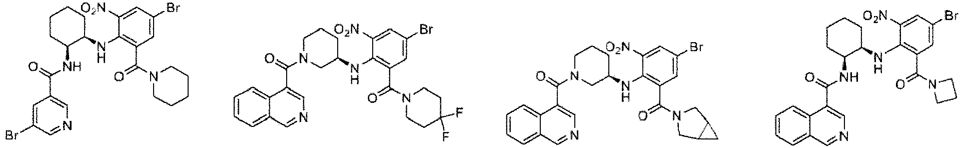

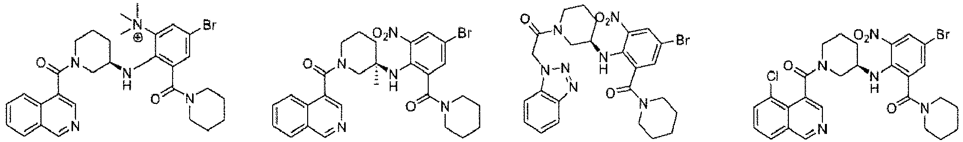

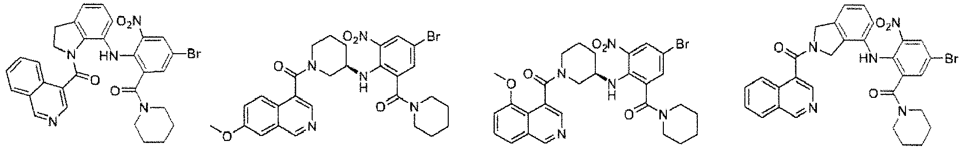

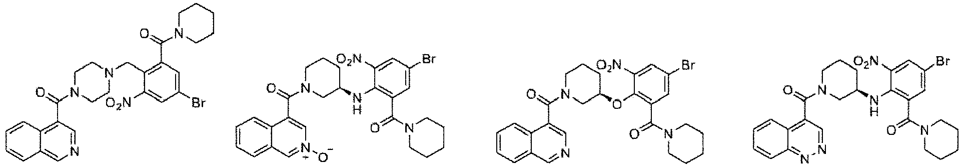

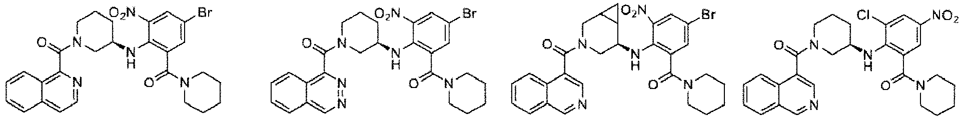

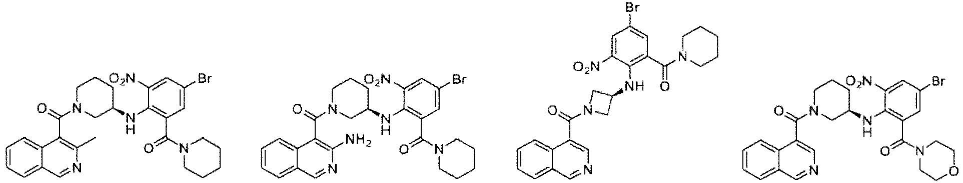

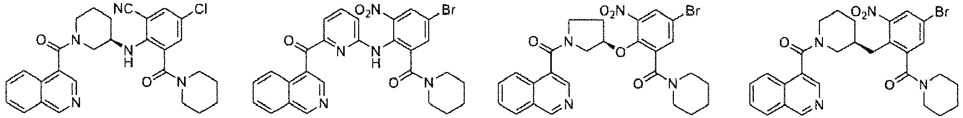

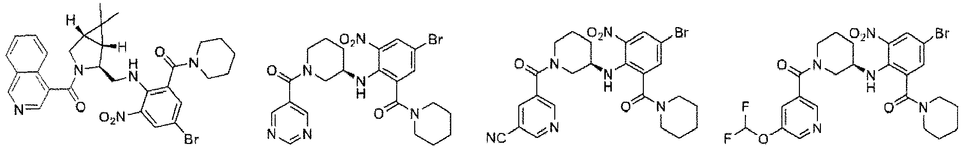

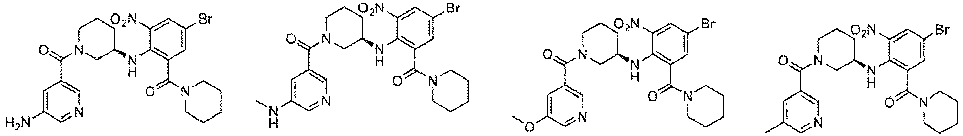

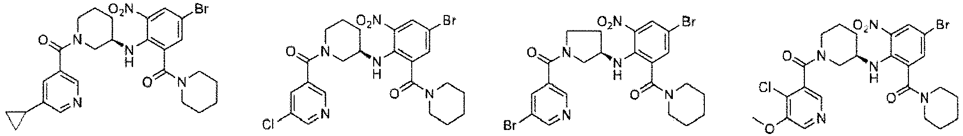

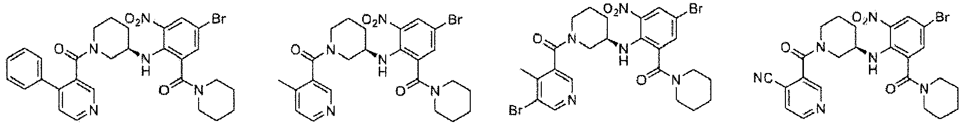

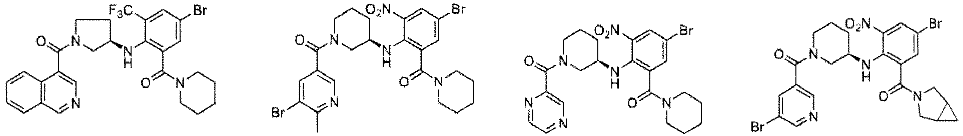

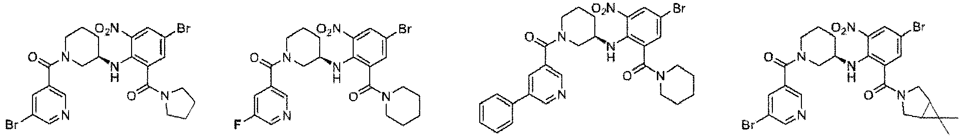

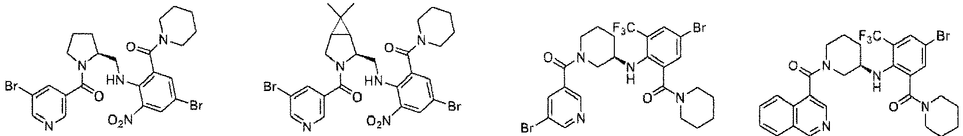

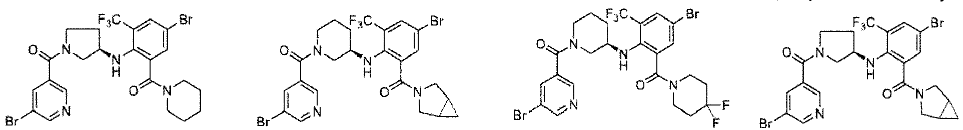

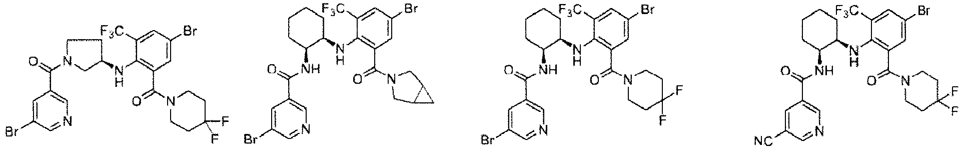

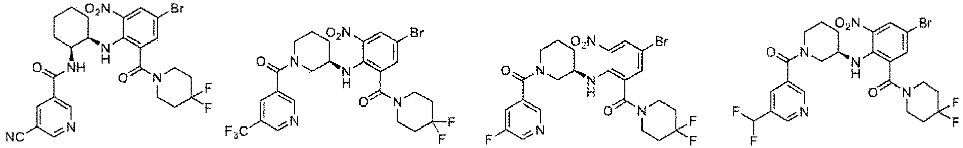

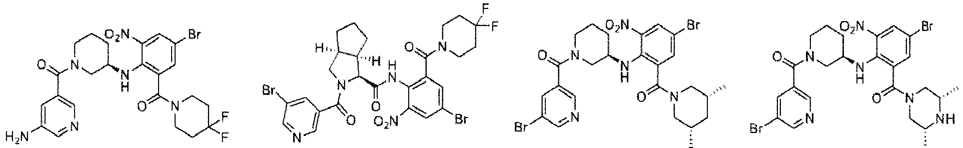

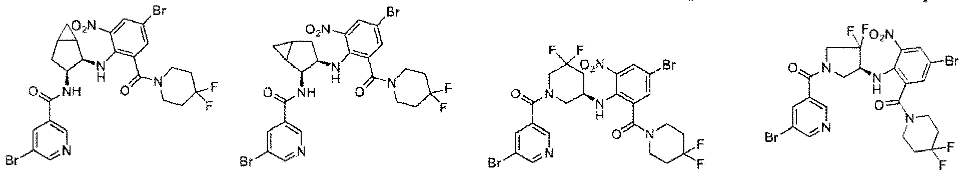

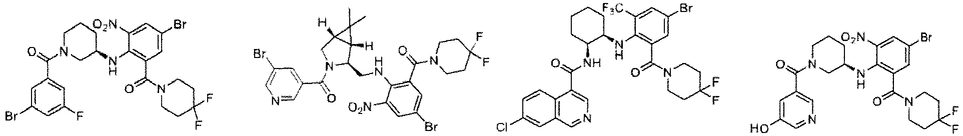

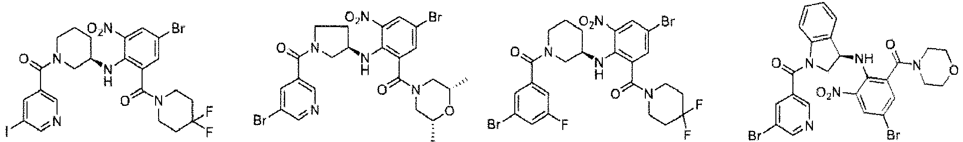

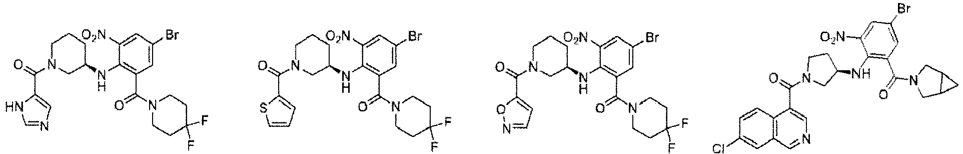

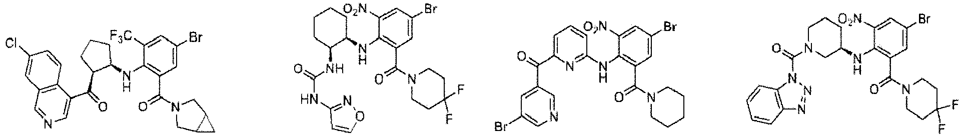

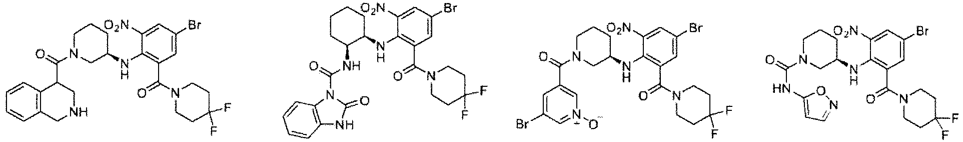

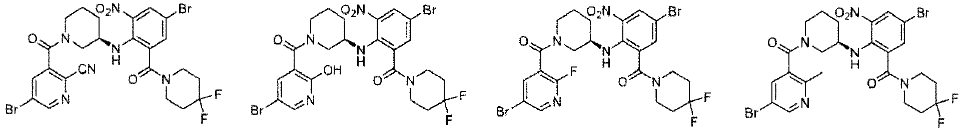

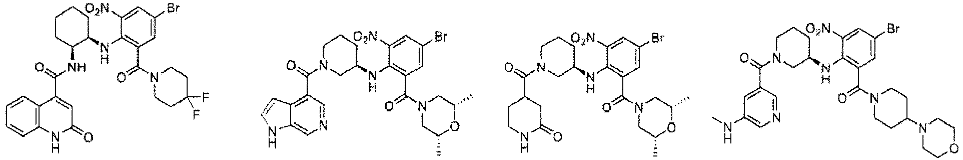

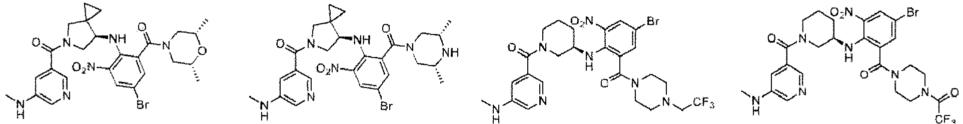

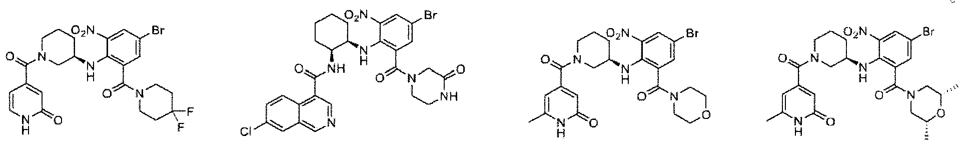

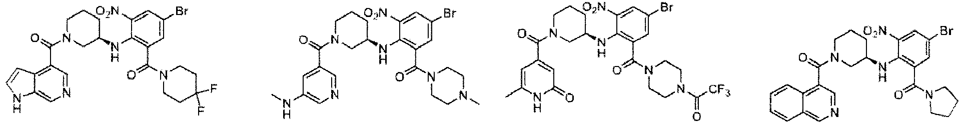

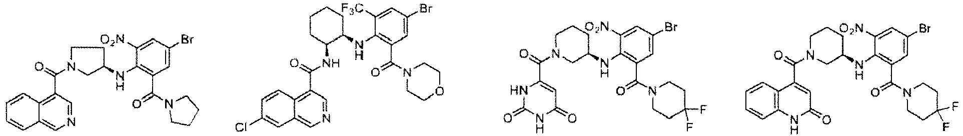

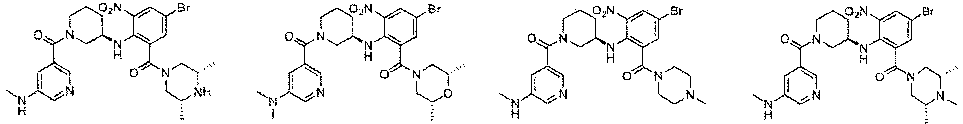

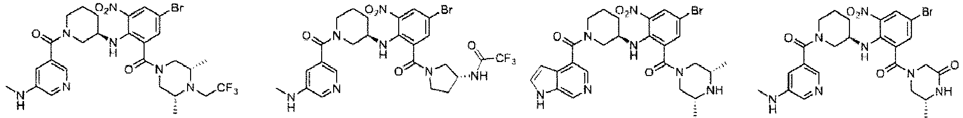

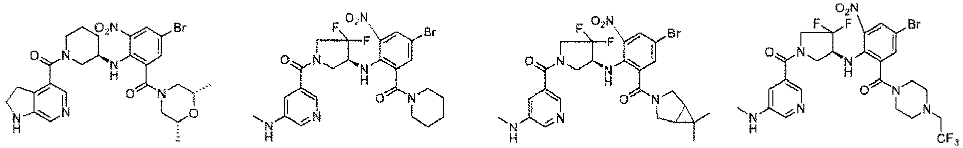

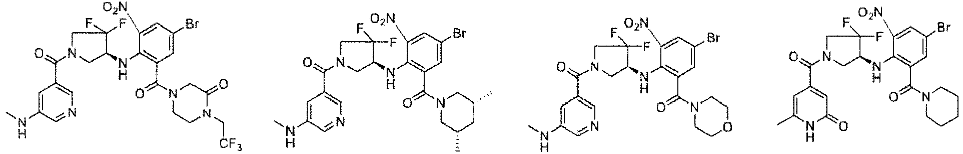

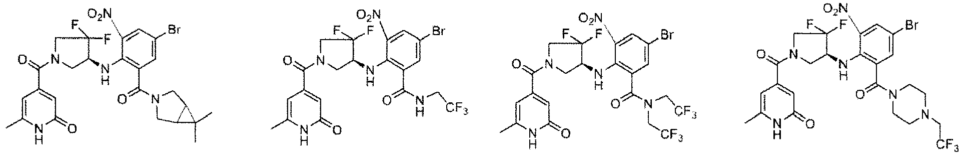

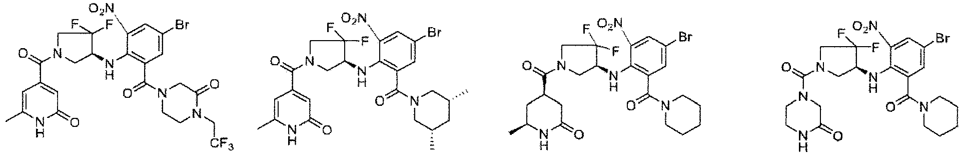

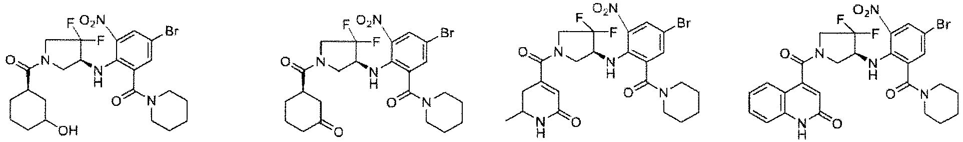

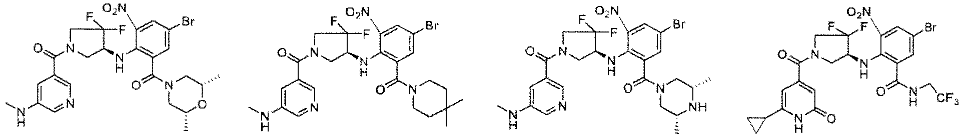

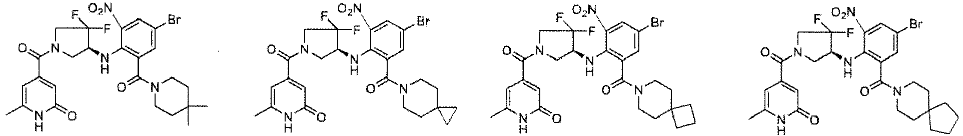

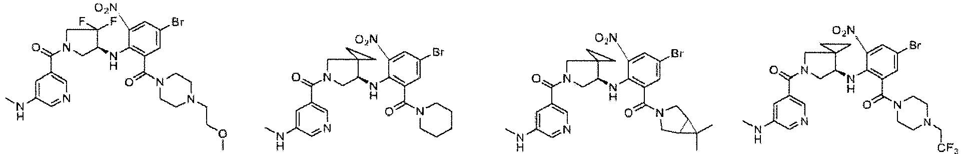

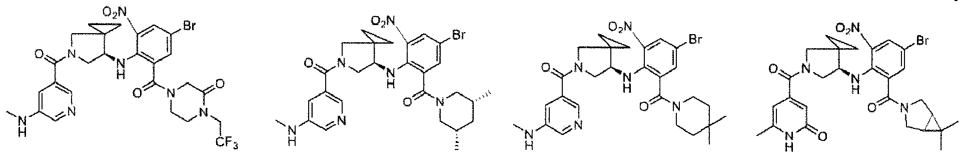

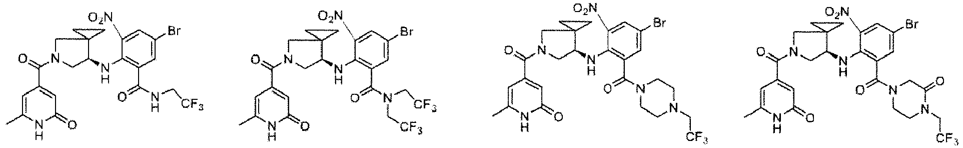

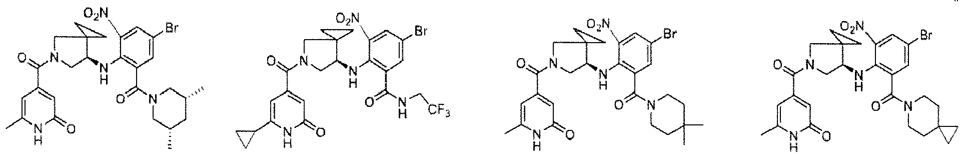

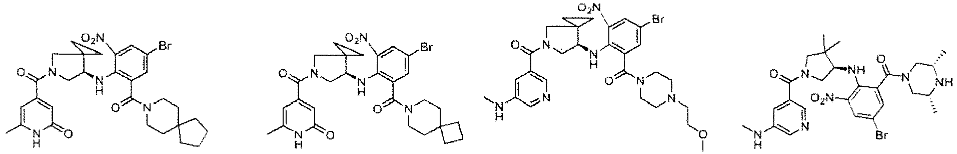

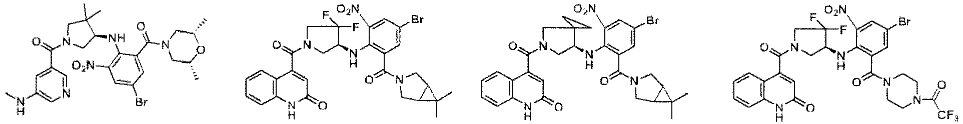

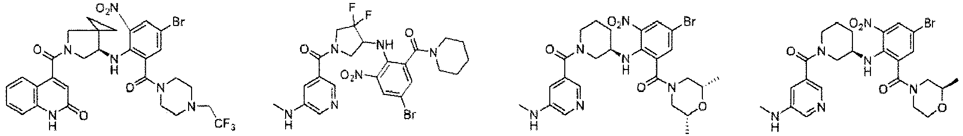

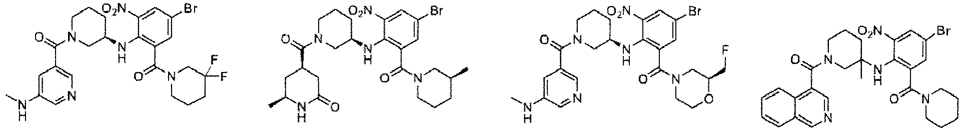

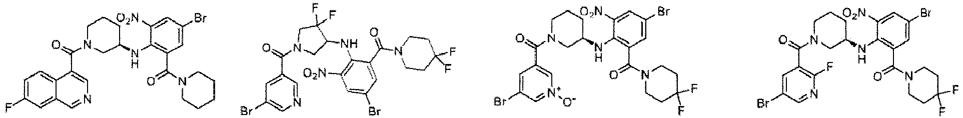

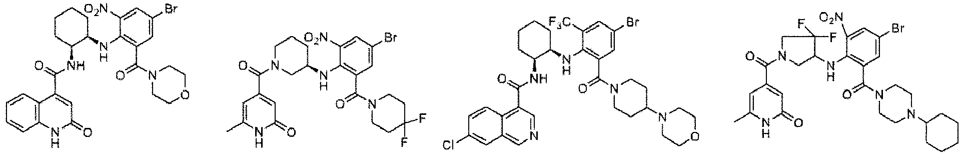

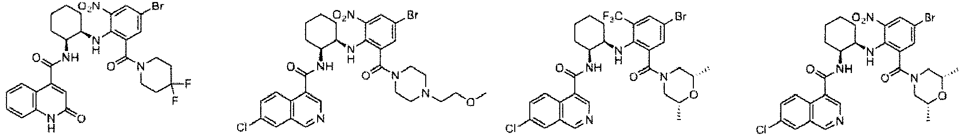

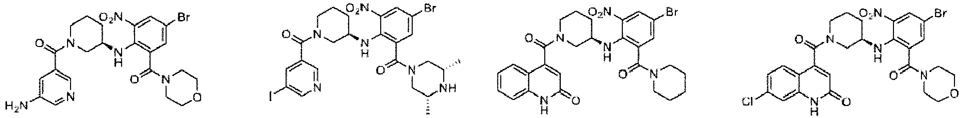

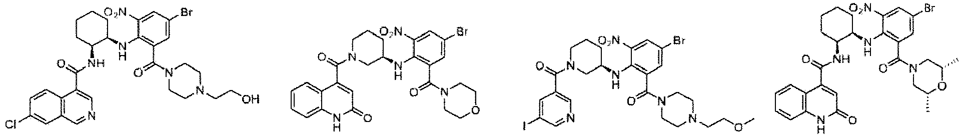

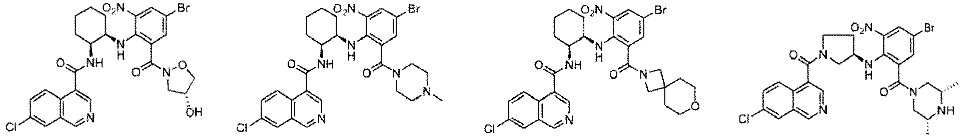

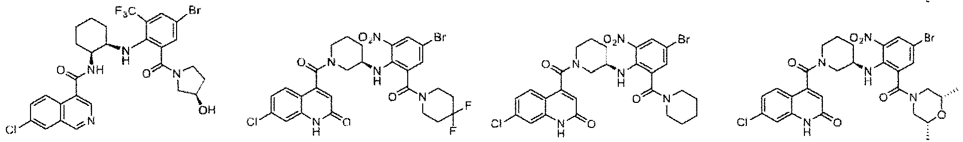

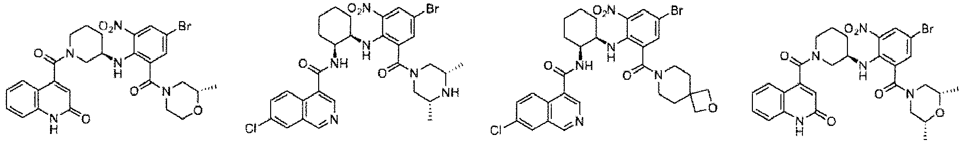

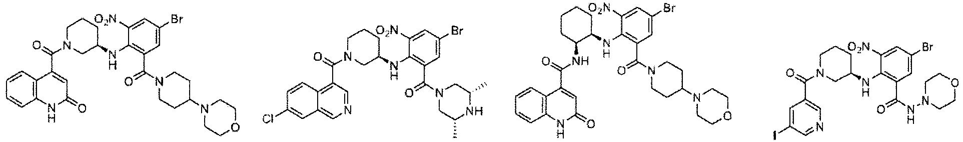

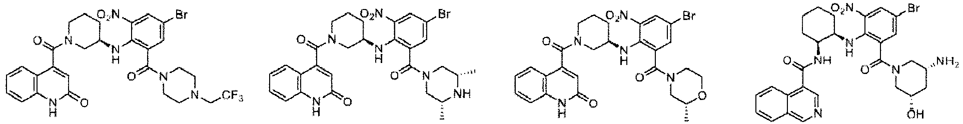

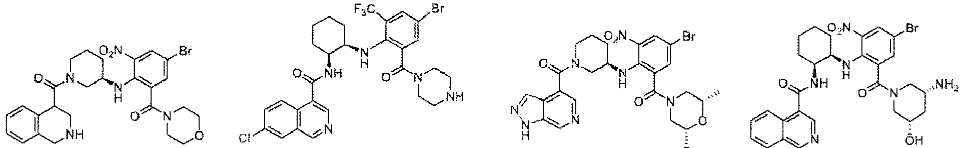

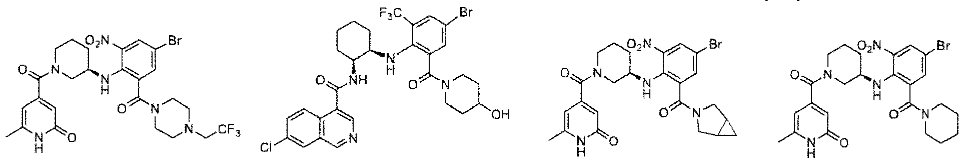

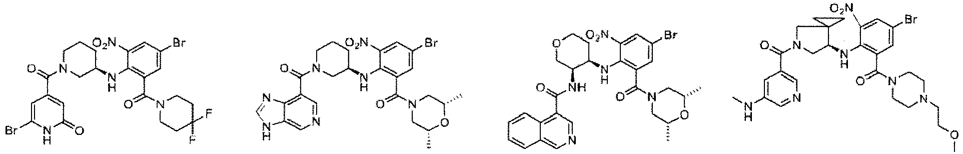

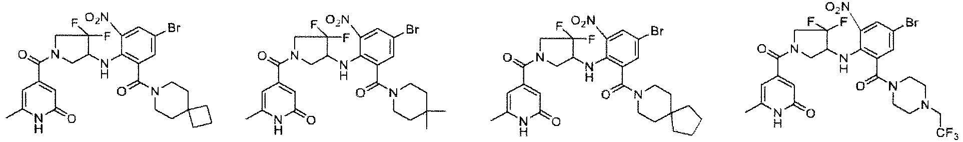

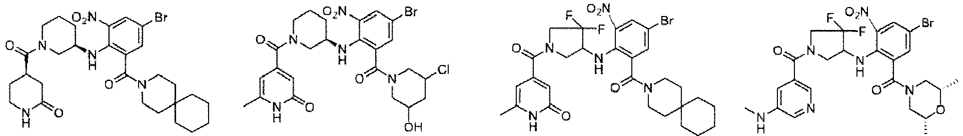

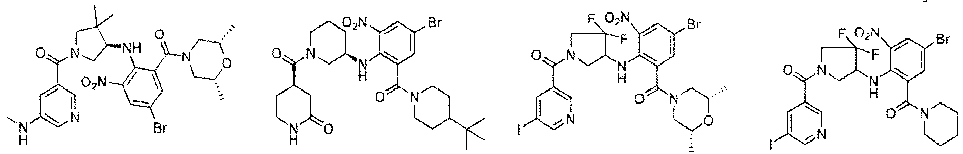

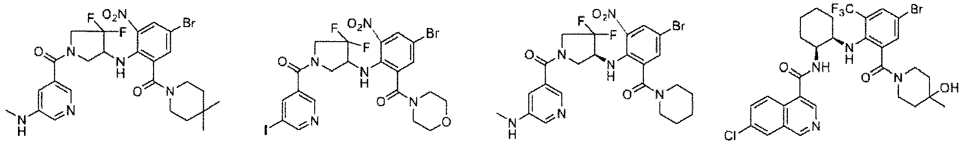

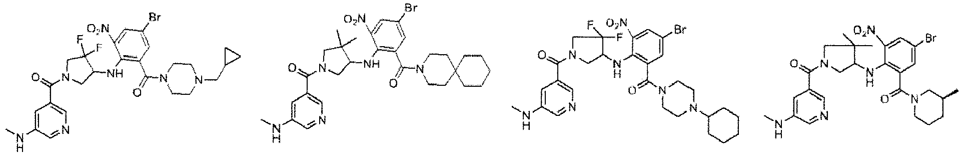

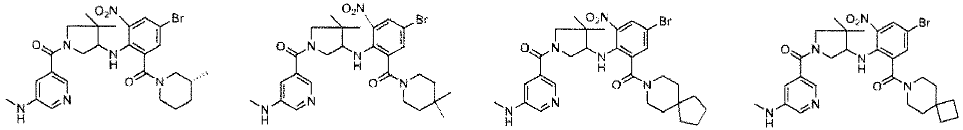

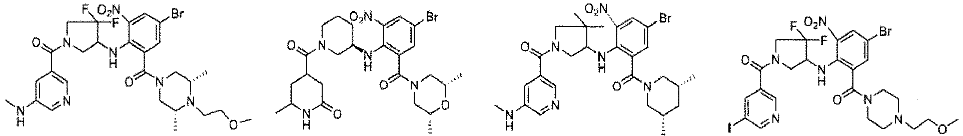

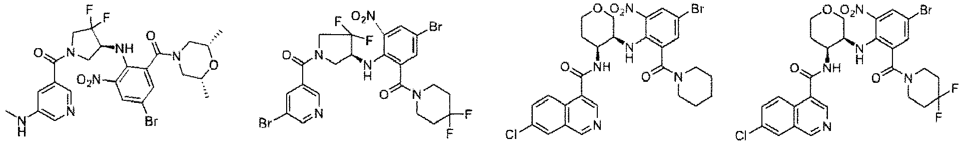

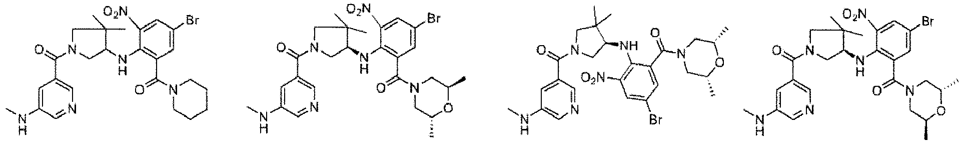

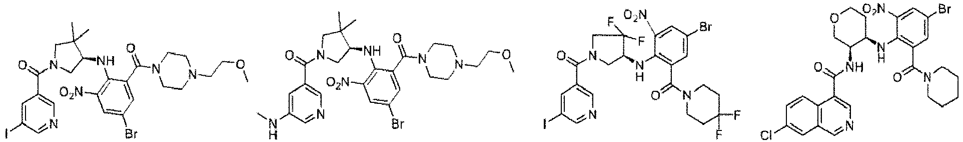

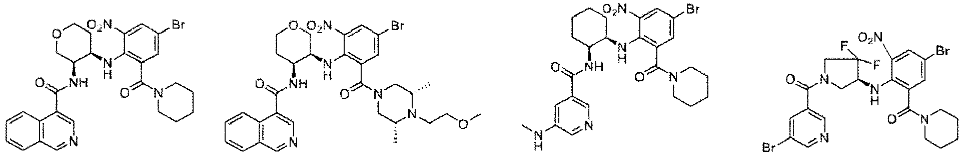

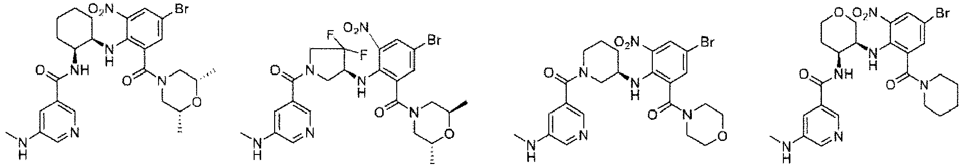

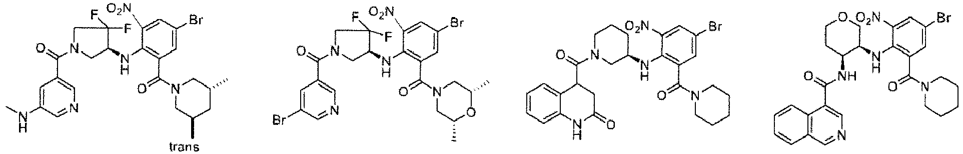

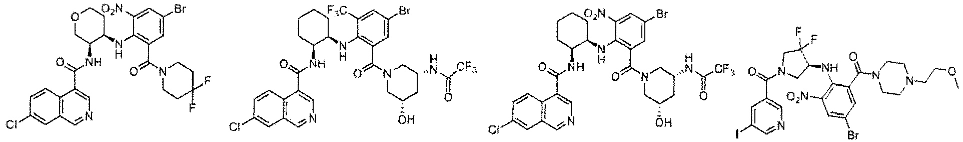

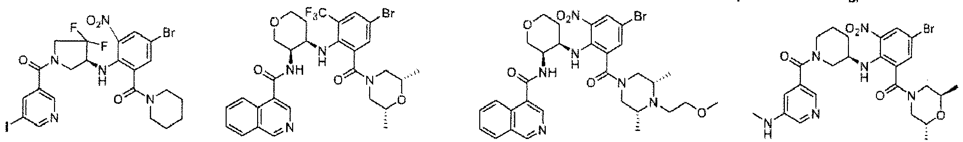

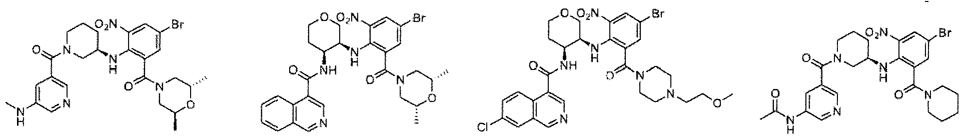

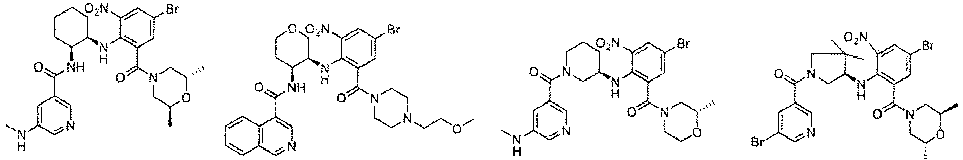

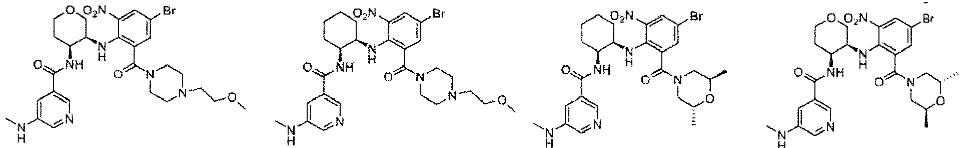

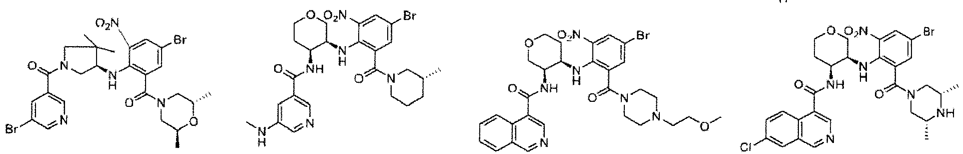

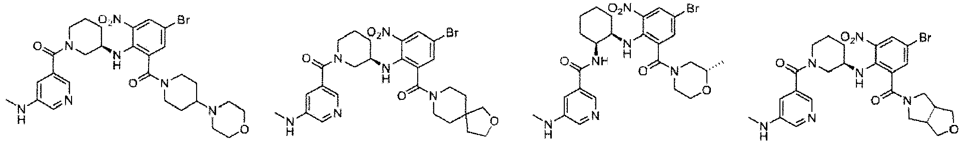

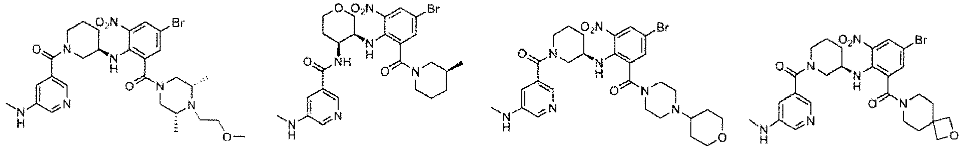

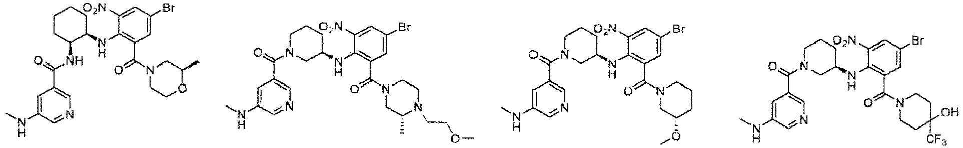

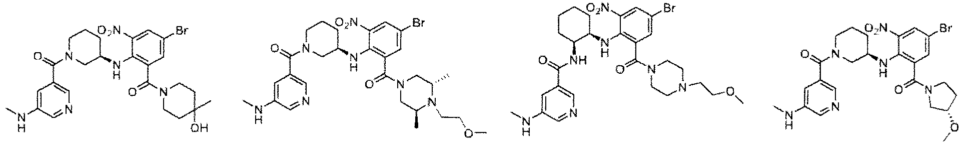

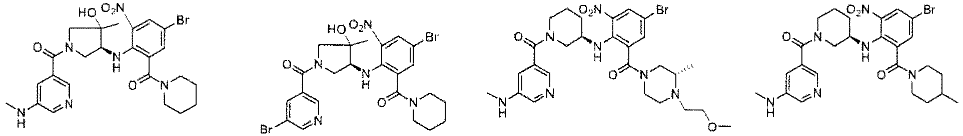

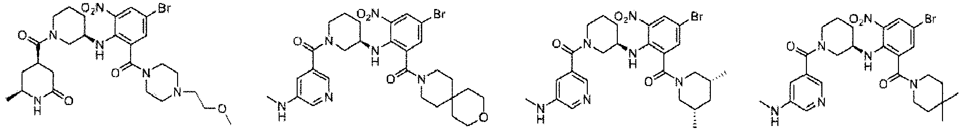

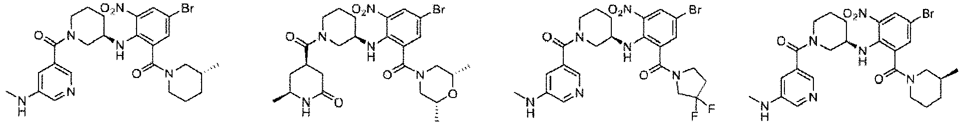

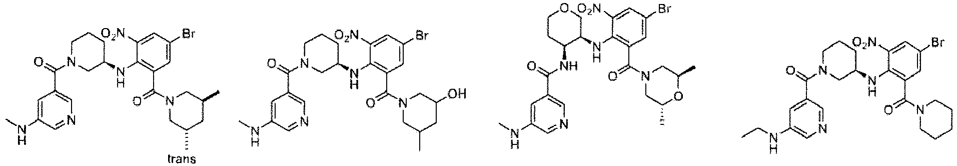

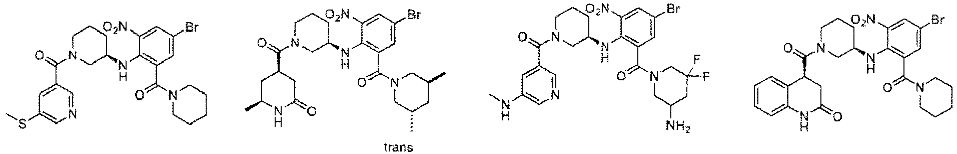

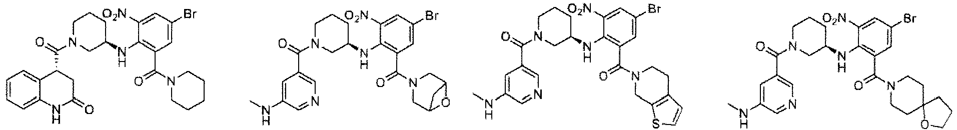

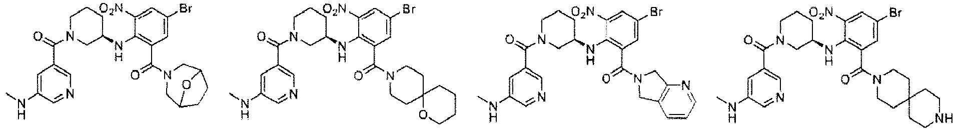

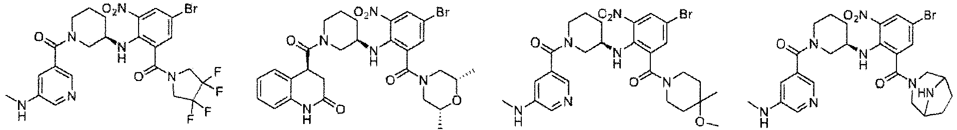

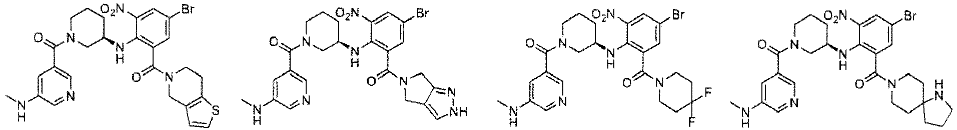

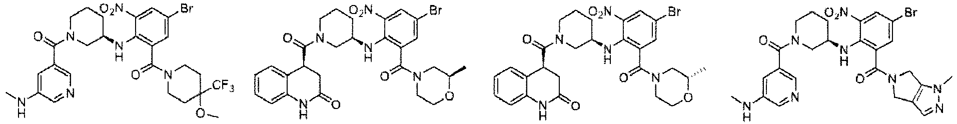

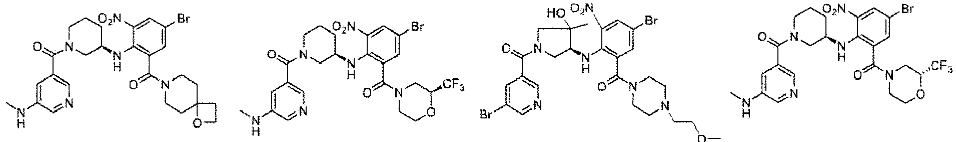

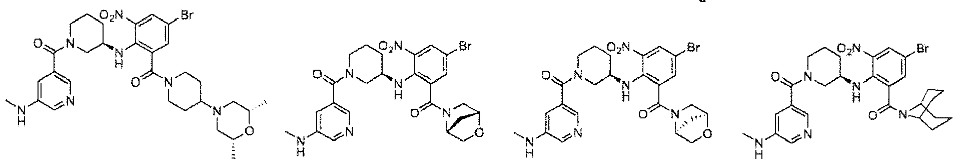

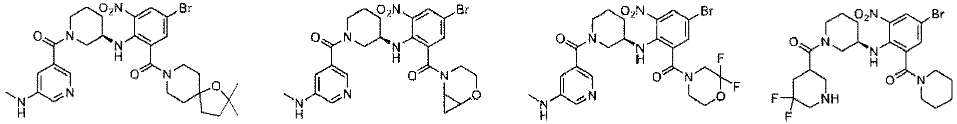

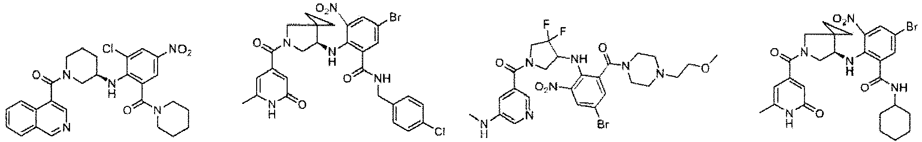

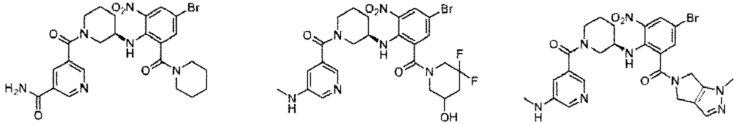

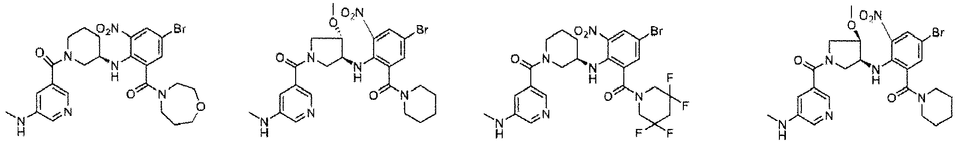

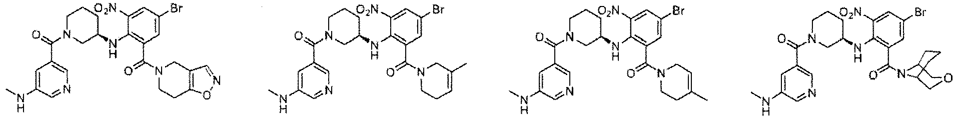

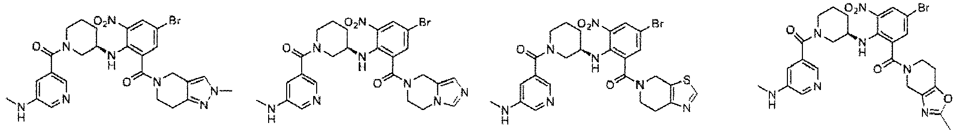

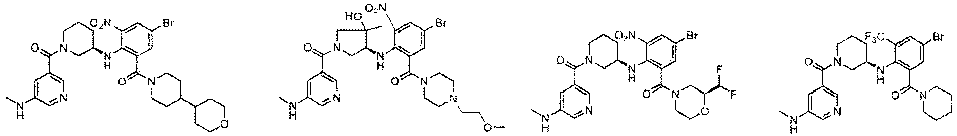

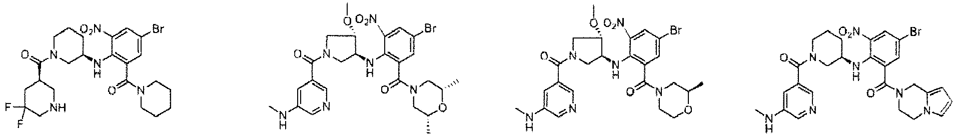

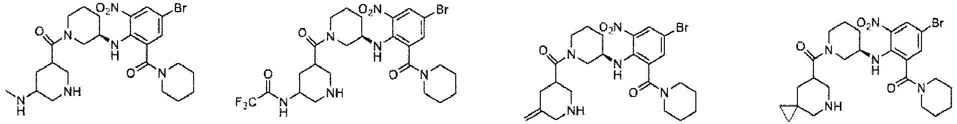

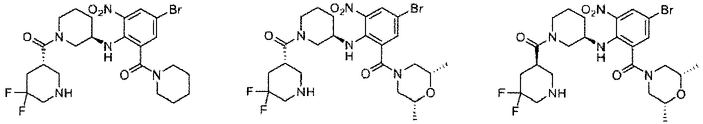

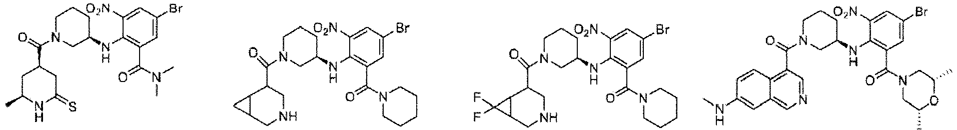

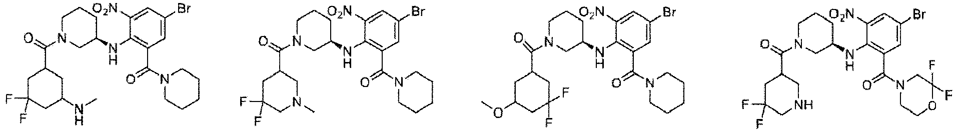

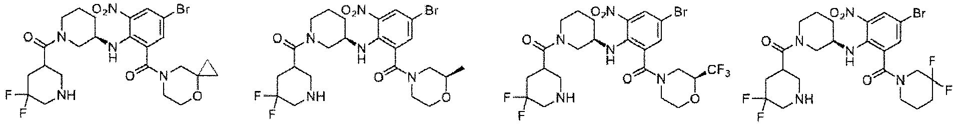

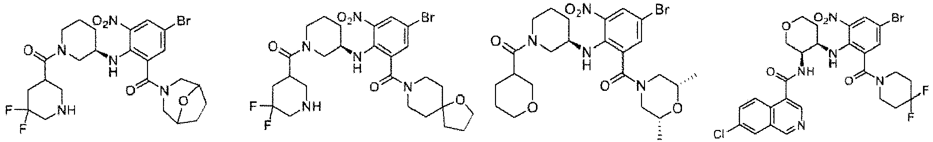

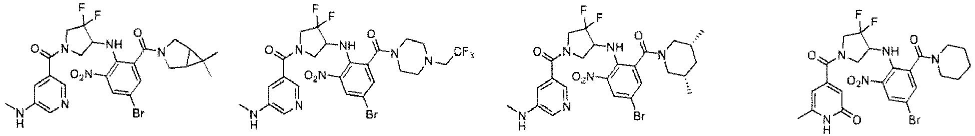

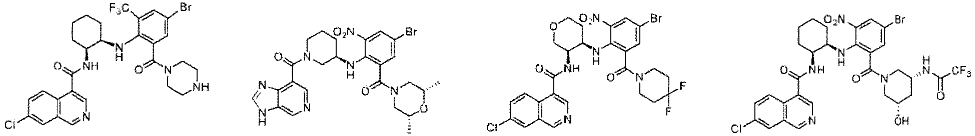

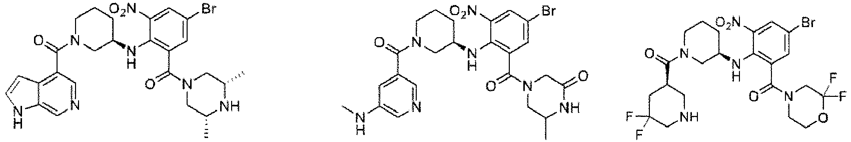

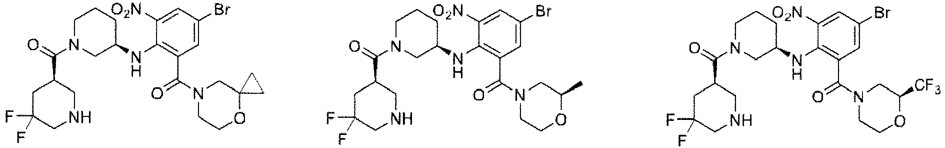

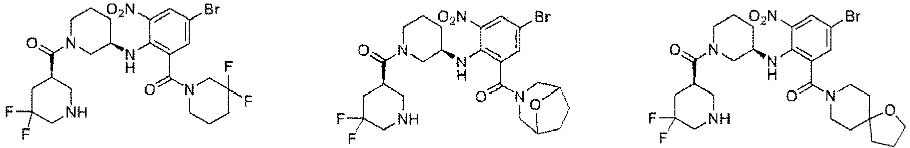

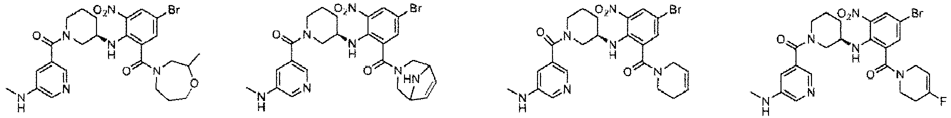

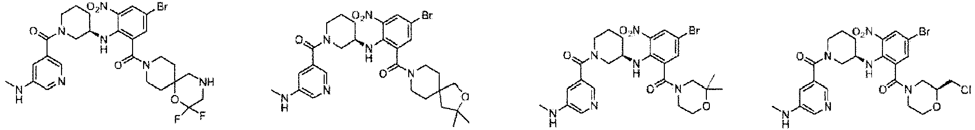

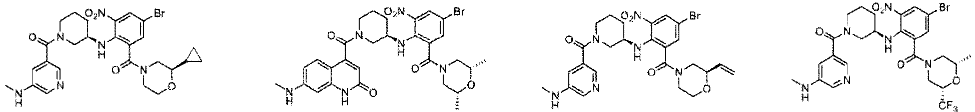

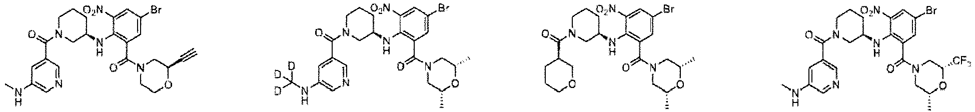

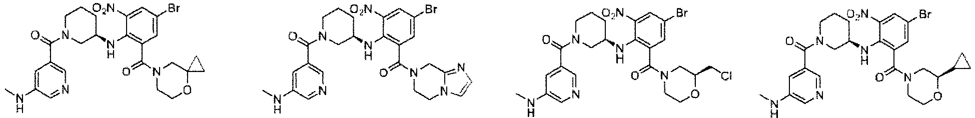

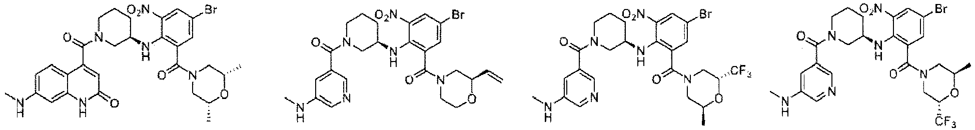

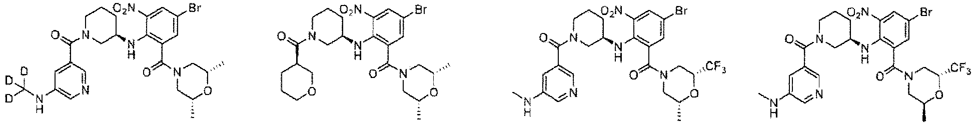

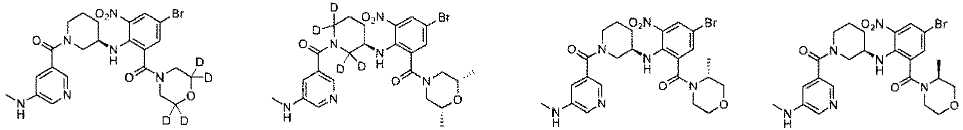

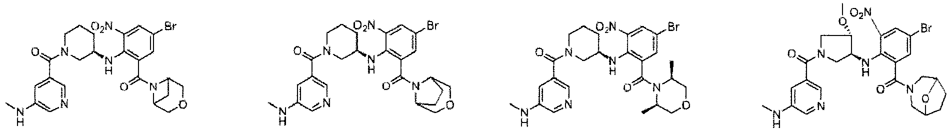

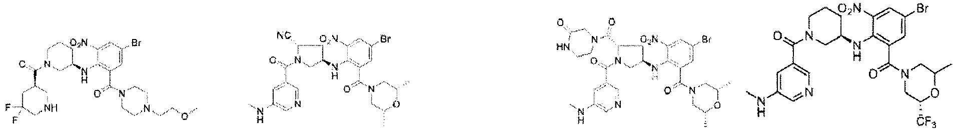

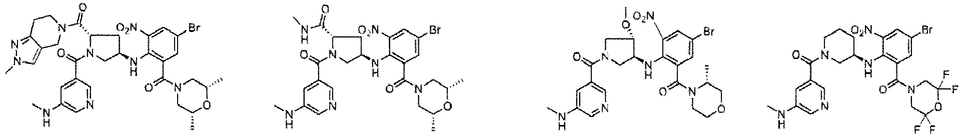

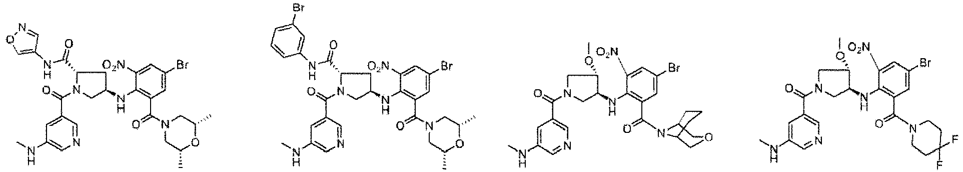

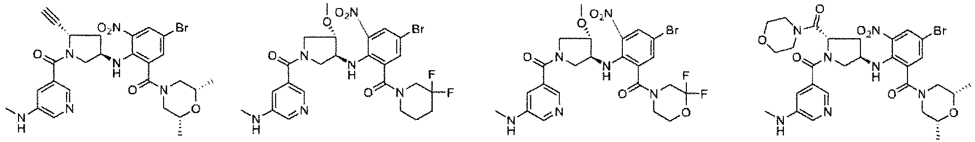

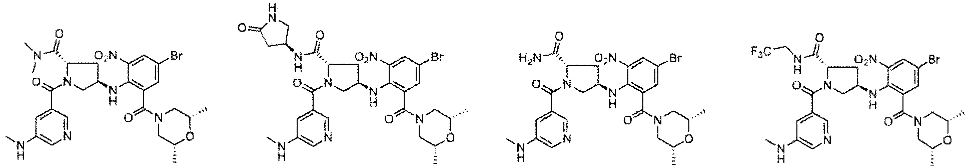

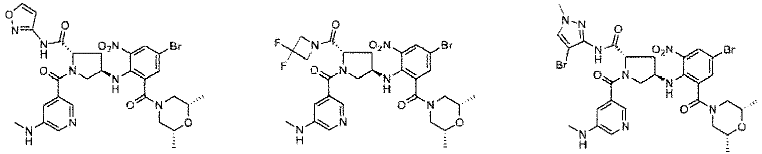

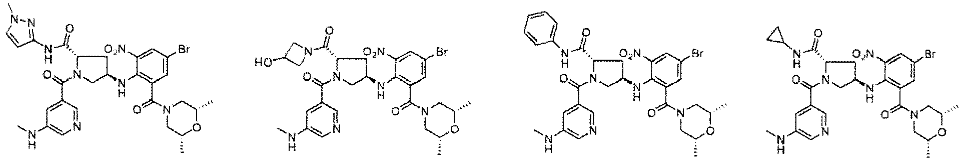

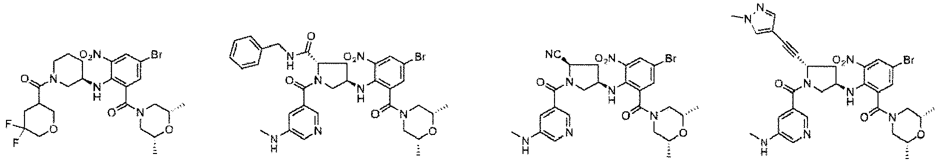

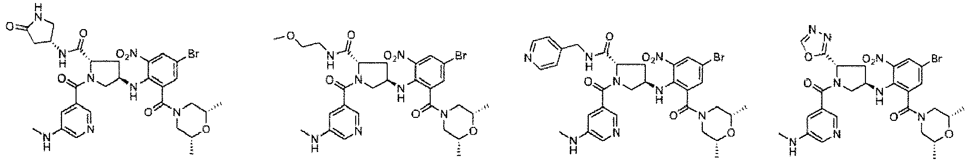

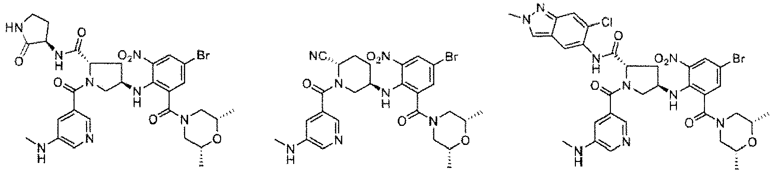

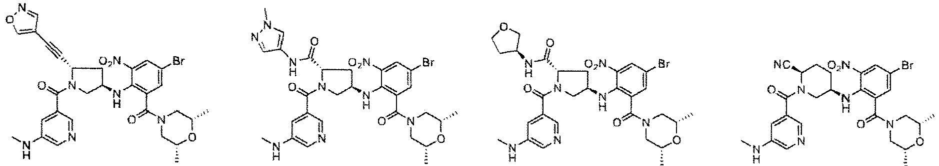

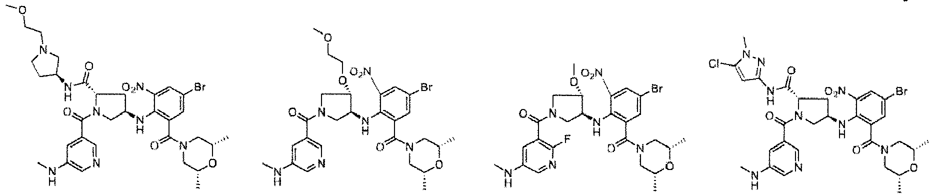

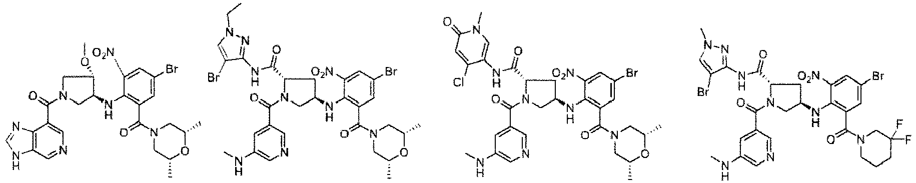

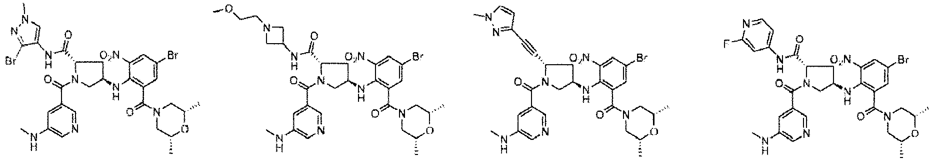

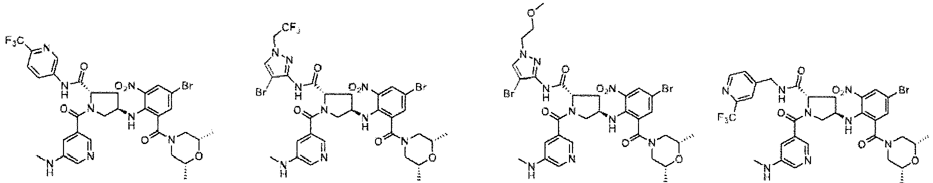

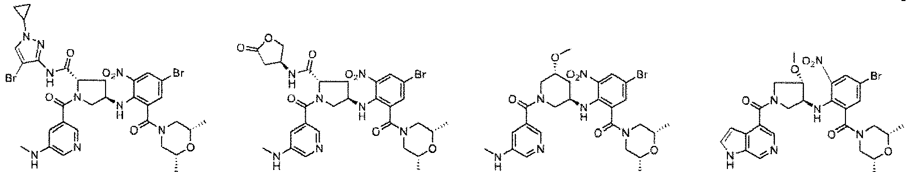

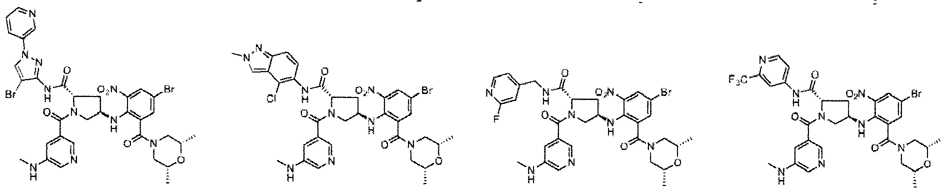

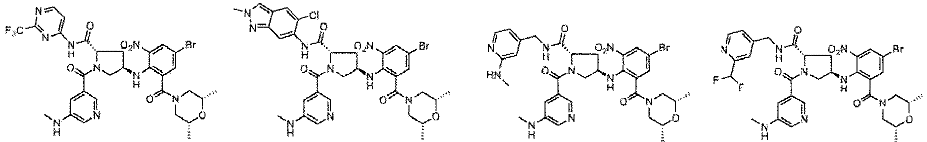

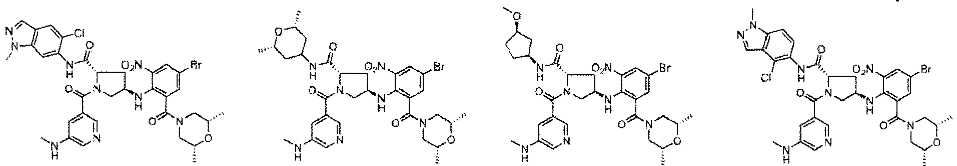

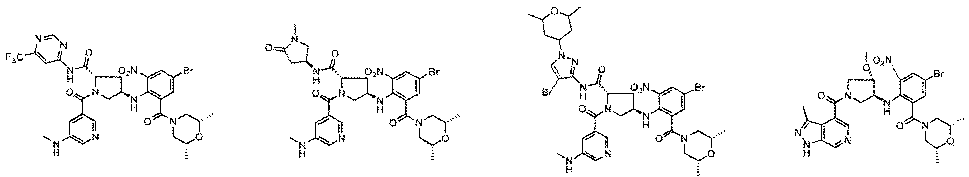

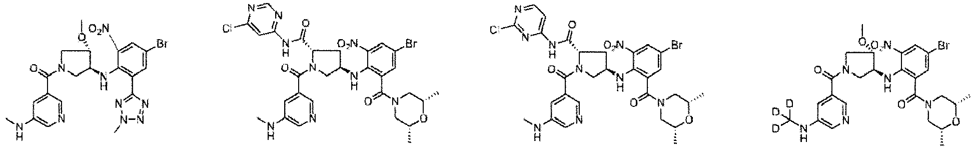

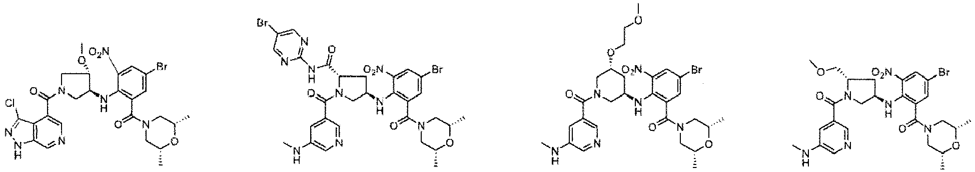

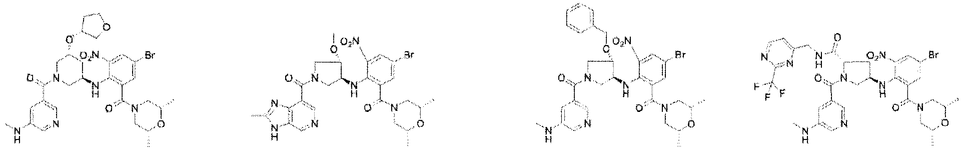

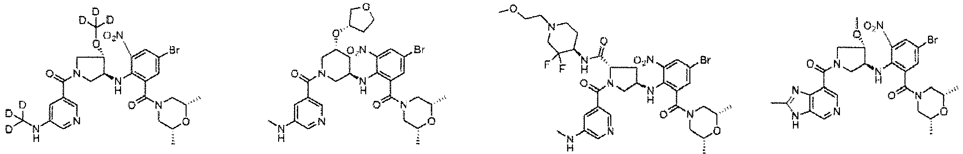

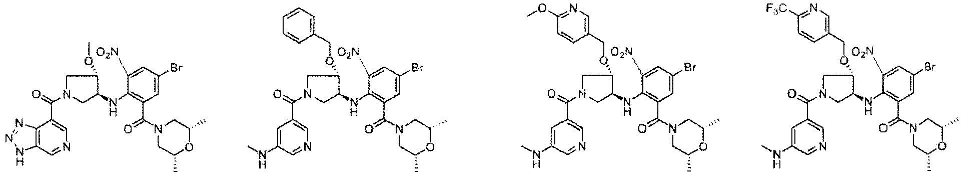

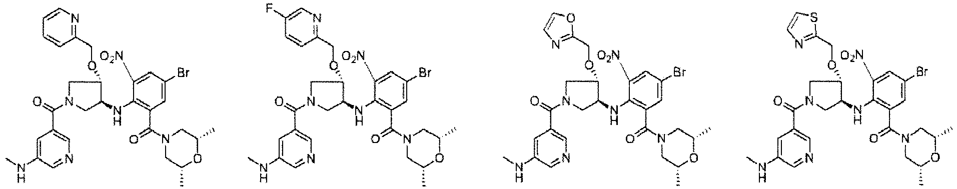

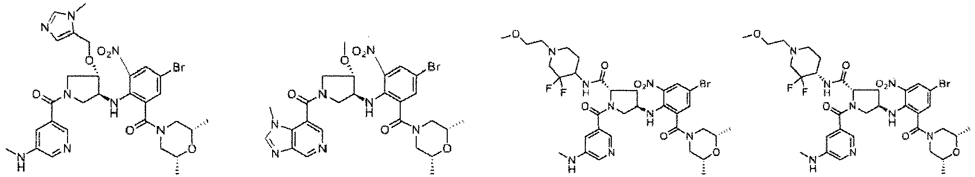

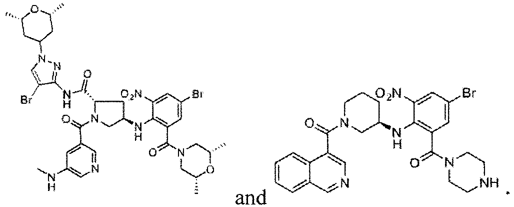

21. A preparation method for the compound of formula (I) of any one of claims 1 to 20, wherein the compound is prepared by condensation reaction between compound of formula (I-A) and compound of formula (I-B) 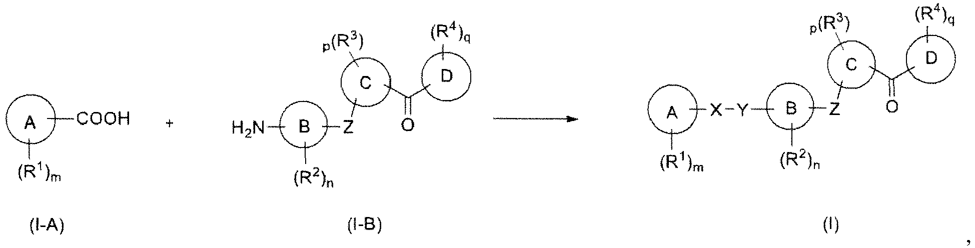 and wherein, ring A, ring B, ring C, ring D, X, Y, Z, R¹, R², R³, R⁴, m, n, p, and q are as defined in formula (I).

22. A pharmaceutical composition, comprising the compound of any one of claims 1 to 20, or the isomer or the pharmaceutically acceptable salt thereof, and a pharmaceutically acceptable carrier, diluent or excipient.

23. Use of the compound of any one of claims 1 to 20, or the isomer or the pharmaceutically acceptable salt thereof and a pharmaceutically acceptable carrier, diluent or excipient, or the pharmaceutical composition of claim 22 in the manufacture of a medicament for treating a disease caused by a coronavirus;
preferably, the disease is a respiratory infectious disease;
preferably, the respiratory infectious disease is severe acute respiratory syndrome; and
preferably, the coronavirus is SARS-CoV-2.
